# EUROPEAN PATENT APPLICATION

(11) **EP 0 810 221 A1**
(43) Date of publication of application: **03.12.1997**
(21) Application number: 96901977.7
(22) Date of filing: 09.02.1996
(51) Int. Cl.: C07D 305/08, C07D 307/22, C07D 309/14, C07H 5/06, C07H 15/18, A61K 31/335, A61K 31/34, A61K 31/35, A61K 31/70

(54) **OXYGEN-CONTAINING HETEROCYCLIC DERIVATIVES**

(30) Priority: 14.02.1995 JP 25398/95; 14.02.1995 JP 25402/95; 12.09.1995 JP 234236/95; 20.10.1995 JP 272432/95
(71) Applicant: MITSUBISHI CHEMICAL CORPORATION, Chiyoda-ku, Tokyo 100 (JP)
(72) Inventor: ANDO, Ryoichi Mitsubishi Chemical Corporation, Aoba-ku, Yokohama-shi Kanagawa 227 (JP); SAKAKI, Toshiro Mitsubishi Chemical Corporation, 2-chome Chiyoda-ku Tokyo 100 (JP); MASUDA, Hirokazu Mitsubishi Chemical Corporation, Aoba-ku, Yokohama-shi Kanagawa 227 (JP); INAKOSHI, Naoto Mitsubishi Chemical Corporation, Aoba-ku, Yokohama-shi Kanagawa 227 (JP); JIKIHARA, Tetsuo Mitsubishi Chemical Corporation, Aoba-ku, Yokohama-shi Kanagawa 227 (JP); FUJIMURA,Yoshiyuki Mitsubishi Chemical Corporation, Aoba-ku, Yokohama-shi Kanagawa 227 (JP); NIWA, Takuro Mitsubishi Chemical Corporation, Aoba-ku, Yokohama-shi Kanagawa 227 (JP); YOSHII, Narihiko Mitsubishi Chemical Corporation, Aoba-ku, Yokohama-shi Kanagawa 227 (JP); TABATA, Reiko Mitsubishi Chemical Corporation, Aoba-ku, Yokohama-shi Kanagawa 227 (JP); SAITO, Ken-Ichi Mitsubishi Chemical Corporation, Aoba-ku, Yokohama-shi Kanagawa 227 (JP); ARITOMO, Keiichi Mitsubishi Chemical Corporation, Aoba-ku, Yokohama-shi Kanagawa 227 (JP)
(74) Representative: Godemeyer, Thomas, Dr.
(86) International application number: JP9600286
(87) International publication number: WO9625408

(57) **Abstract**

An oxygen-containing heterocyclic derivative represented by the following formula (I) and its salt, and a solvate and a hydrate thereof: 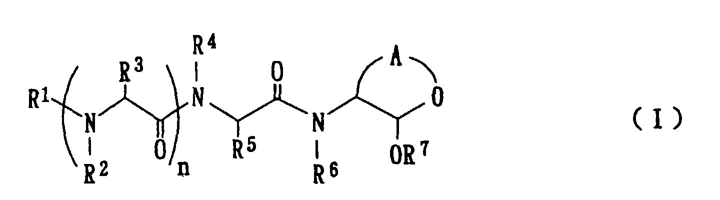 wherein:
- R¹:: hydrogen atom, R⁸-CO-, R⁸-O-CO- or other wherein R⁸ represents C₁-C₂₀ alkyl, aryl or other,
- R², R⁴ and, R⁶:: a hydrogen atom, C₁-C₅ alkyl or other,
- R³ and R⁵:: a hydrogen atom, C₁-C₂₀ alkyl or other,
- R⁷:: a hydrogen atom, R⁹-CO- or other wherein R⁹ represents C₁-C₁₀ alkyl group or other,
- A:: C₁-C₃ alkylene group
- n:: 0 or 1.

The oxygen-containing heterocyclic derivatives of the present invention have potent inhibitory activity against cysteine proteases and are excellent in oral absorbability, tissue distribution, and cellular membrane permeability. Therefore, they are useful as therapeutic medicament for diseases such as cerebral apoplexy, Alzheimer's disease and the like.

## Description

### Technical Field

This invention relates to novel oxygen-containing heterocyclic derivatives.

### Background Art

Functions of cysteine proteases such as papain, cathepsin B, cathepsin H, cathepsin L, calpain, and interleukin-1 β converting enzyme in living bodies have been elucidated, and as the progress, it becomes revealed that abnormal accentuation of these substances is a cause of various types of diseases. There also are increasing numbers of literatures reporting that a cysteine protease inhibitor was found to be effective when applied to an animal model of such disease.

Cysteine proteases such as calpain or cathepsin B are considered to be involved in an early stage of elimination of Z-filaments or other with degradation of muscle fiber proteins in a process of skeletal muscular decay which can be observed in myopathy such as muscular dystrophy and amyotrophia (Metabolism, Vol. 25, Extra Edition, "Highlights of Metabolic Disease," p. 183, 1988). In addition, E-64-d, a cysteine protease inhibitor, was reported to have life prolongation effect on muscular dystrophy hamsters (Journal of Pharmacobio Dynamics, Vol. 10, p. 678, 1987). Accordingly, cysteine protease inhibitors are considered as potential therapeutic medicaments for muscular dystrophy, amyotrophia and the like.

In ischemic diseases such as myocardial infarct and cerebral apoplexy, a major cause of cellular dysfunction after ischemia is active oxygen produced by xanthine oxidase. Some articles suggested that calpain, being activated by an increased concentration of Ca²⁺ during ischemia, partially degrades xanthine dehydrogenase as a precursor of xanthine oxidase to convert it into the oxidase (New England Journal of Medicine, Vol. 312, p. 159, 1985). It is also suggested that the activation of calpain may directly trigger myocardial cellular death and cerebral nerve cellular death (Latest Medicine, Vol. 43, p. 783, 1988). NCO-700, an inhibitor of calpain, was reported to be effective in an animal model of myocardial infarct (Arzneimittel Forschung/Drug Research Vol. 36, p. 190, p. 671, 1986) and E-64-c suppressed the degradation of microtubule-binding proteins after cerebral ischemia (Brain Research, Vol. 526, p. 177, 1990). Therefore, calpain inhibitors are considered as potential therapeutic medicament for ischemic diseases such as myocardial infarct and cerebral apoplexy.

A protein called amyloid deposits in senile plaques that can be uniquely observed in brains of patients suffered from Alzheimer's disease. It is known that the amyloid is synthesized by the degradation of amyloid protein precursor (APP). Some articles suggest that amyloid is produced by abnormal metabolism due to an abnormally accentuated protease to form senile plaques, whilst amyloid is not produced in a normal metabolic process of APP (Scientific American, November, 1991, p. 40). Accordingly, protease inhibitors are expected to be used as therapeutic medicament for Alzheimer's disease.

There is reported that calpain is activated in a head injury model using a rabbit (Neurochemical Research, Vol. 16, p. 483, 1991). A protective effect on axon was observed by the administration of leupeptin as a calpain inhibitor to a head injury model using a rat (Journal of Neurosurgery, Vol. 65, p. 92, 1986). Therefore, inhibitors of calpain are considered to have an improving effect on disturbance of consciousness and movement disorder caused by a head injury.

Myelin binding proteins that exist in dendrites of nerve cells were found to be degraded by calpain (Journal of Neurochemistry, Vol. 47, p. 1007, 1986). Therefore, inhibitors of calpain are considered as effective on diseases caused by demyelination of nerve cells such as multiple sclerosis and neuropathy of peripheral nerve.

In most cases of cataract, it is suggested that crystallin, a water-soluble protein in crystalline lens, is hydrolyzed by the action of a protease, which results in the cloudiness of crystalline lens. In experimental models of cataract and certain types of human cataract, calcium concentrations in crystalline lens are increased (Investigative Ophthalmology & Visual Science, Vol. 28, p. 1702, 1987; Experimental Eye Research, Vol. 34, p. 413, 1982), and calpain exists in major abundance among proteases contained in crystalline lens (Lens and Eye Toxicity Research, Vol. 6, p. 725, 1989). Therefore, abnormal accentuation of calpain is considered to be one of causes of cataract. There is also reported that an inhibitor of calpain, i.e., E-64, was effective in an experimental model of cataract (Investigative Ophthalmology & Visual Science, Vol. 32, p. 533, 1991. Accordingly, inhibitors of calpain are considered as potential therapeutic medicament for cataract.

It is known that neutrophils, which are intimately involved in inflammation, respond to stimulations with chemotactic factors or phorbol esters by causing degranulation or producing superoxide, and this process is considered to be mediated by protein kinase C (PKC). There is reported that calpain has a function to activate PKC, thereby exhibits promoting activity on the degranulation and suppressing activity against the production of superoxide (Journal of Biological Chemistry, Vol. 263, p. 1915, 1988). It has also been reported that concentration of cathepsin B in rat macrophages is 30 to 40-fold higher than that of leucocytes or neutrophils, and moreover, the enzyme concentration of inflammatory macrophages is 6-fold higher than that of ordinary macrophages (Journal of Biochemistry, Vol. 98, p. 87, 1985). Furthermore, it has recently been revealed that the enzyme that catalyzes the conversion of pre-interleukin-1 β into interleukin-1 β (interleukin-1 β converting enzyme) is a cysteine protease (Nature, Vol. 356, p. 768, 1992), which clarifies that the activation process of cysteine protease plays an important role in the formation of inflammation. From these findings, it is considered that inhibitors of cysteine protease can be used as anti-inflammatory agents.

I-type allergic reaction progresses with the mediation of immunoglobulin E (IgE) that is produced by immunization of a living body with an antigen. It has been reported that Estatin A, a cysteine protease inhibitor, specifically suppresses the production of IgE, whereas it does not affect on the production of IgG (The Journal of Antibiotics, Vol. 42, p. 1362, 1989). Accordingly, cysteine protease inhibitors are considered to be usable as anti-allergic agents.

When necrosis of hepatocytes occurs, it is suggested that Ca²⁺ permeability of cell membranes increases due to disturbance of cellular membranes and then intracellular Ca²⁺ concentration is elevated to activate calpain, which leads to degradation of structural proteins or other as substrates of calpain and results in cellular death. Therefore, inhibitors of calpain can be used as therapeutic medicaments for fulminant hepatitis.

Cathepsins such as cathepsin B and cathepsin L participate in degradation of bone collagen in osteoclasts. There is reported that serum calcium concentration and hydroxyproline concentration were lowered when B-64 or Estatin A being an inhibitor of cathepsins was administered to rats whose osteoclasia was accentuated by administering parathormone (Biochemical and Biophysical Research Communication Vol. 125, p. 441, 1984; the Japanese Patent Unexamined Publication (KOKAI) No. (Hei) 2-218610/1990). Therefore, inhibitors of cathepsins are considered as potential therapeutic medicaments for osteoporosis, hypercalcemia and the like.

Substrates of calpain include a class of sexual hormone receptors such as estrogen receptor and androgen receptor. Calpain is known to activate these receptors, and it is suggested that abnormal accentuation of calpain causes diseases that are positively caused by abnormal activation of sexual hormone receptors such as, for example, breast cancer, prostatic cancer, and prostatic hypertrophy. Accordingly, inhibitors of calpain are considered to be useful as therapeutic medicaments for the aforementioned diseases.

It is suggested that receptors of epidermal growth factor (EGF) are activated as cellular tumorigenic transformation proceeds, and it is known that calpain activates the EGF receptors as its substrates. There is also reported that calpain was activated in cells infected with human adult T-cell leukemia virus (ATLV/HTLV-1) (Biochemistry, Vol. 57, p. 1202, 1985). On the other hand, it is suggested that cathepsin B is intimately involved in processes of cancerous metastasis, because cathepsin B promotes collagen degradation, which is an important step of cancerous metastasis, or directly degrades collagen, and it also has close relationship with plasma membranes of neoplastic cells (Tumor Progression and Markers, p. 47, 1982; Journal of Biological Chemistry, Vol. 256, p. 8536, 1984). From these teachings, inhibitors of cysteine protease are considered to be effective in inhibition of cancerous growth and prevention of cancerous metastasis.

Activation of blood platelets triggers agglomeration, which may lead to thrombus formation. There is reported that E-64-d as being an inhibitor of calpain suppressed platelet aggregation caused by thrombin (Thrombosis Research, Vol. 57, p. 847, 1990). Accordingly, inhibitors of calpain can be used as platelet aggregation inhibitors.

As explained above, abnormal accentuation of cysteine protease may become the causes of various kinds of diseases, and some cysteine protease inhibitors are reported to have efficacy in animal models or the like.

However, almost all of known inhibitors are irreversible inhibitors, for example, E-64 (Agricaltural and Biological Chemistry, Vol. 42, p. 529, 1978), E-64-d (Journal of Biochemlstry, Vol. 93, p. 1305, 1983), NCO-700 (the Japanese Patent Unexamined Publication (KOKAI) No. (Sho) 58-126879/1983), epoxysuccinic acid derivatives such as Estatins A and B (The Journal of Antibiotics, Vol. 42, p. 1362, 1989), and α -substituted ketones of peptides whose typical examples include chloromethyl ketones of peptides (Journal of Biochemlstry, Vol. 99, p. 173, 1986) and acyloxymethyl ketones (Biochemistry, Vol. 30, p. 4678, 1991). Irreversible inhibitors are generally considered to have strong toxicity because they are likely to non-specifically react with biogenic components including the target enzymes, and therefore, only a few compounds have been used clinically. As reversible inhibitors, peptidyl aldehydes such as leupeptin (The Journal of Antibiotics, Vol. 22, p. 283, 1969) and calpeptin (Journal of Enzyme Inhibition, Vol. 3, p. 195, 1990) are known. However, they are considered to have problems relating to chemical stability, stability in a living body, cell membrane permeability and the like.

### Disclosure of the Invention

From these reasons, the present inventors conducted researches to obtain cysteine protease inhibitors having excellent oral absorbability, tissue distribution, cell membrane permeability and other. As a result, they achieved the present invention.

The gist of the present invention is oxygen-containing heterocyclic derivatives represented by the following formula (I) and their pharmaceutically acceptable salts, and hydrates and solvates thereof: 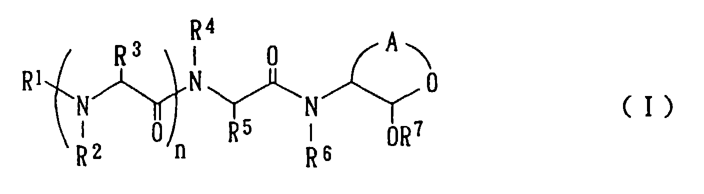 wherein R¹ represents a hydrogen atom, R⁸-CO-, R⁸-O-CO-, R⁸-NH-CO-, or R⁸-SO₂- (R⁸ represents a C₁-C₂₀ alkyl group which may optionally be substituted with one or more substituents selected from the group consisting of a C₃-C₈ cycloalkyl group, a C₃-C₈ cycloalkyloxy group, fluorenyl group, a C₁-C₆ alkoxy group, a C₆-C₁₄ aryl group which may optionally be substituted, a C₆-C₁₄ aryloxy group which may optionally be substituted, a C₆-C₁₄ arylthio group which may optionally be substituted, a C₆-C₁₄ arylsulfonyl group which may optionally be substituted, and a residue of a heterocyclic compound which may optionally be substituted; a C₃-C₈ cycloalkyl group; a C₆-C₁₄ aryl group which may optionally be substituted; a C₂-C₅ alkenyl group which may optionally be substituted with an optionally substituted C₆-C₁₄ aryl group; or a residue of a heterocyclic compound which may optionally be substituted); R², R⁴, and R⁶ independently represent a hydrogen atom, a C₁-C₅ alkyl group, or a C₂-C₆ alkanoyl group; R³ and R⁵ independently represent a hydrogen atom, a C₁-C₂₀ alkyl group which may optionally be substituted with one or more substituents selected from the group consisting of a C₆-C₁₄ aryl group which may optionally be substituted, hydroxyl group, a C₁-C₅ alkoxy group, a C₁-C₅ alkylthio group, and a C₇-C₁₂ aralkyloxy group, or they independently represents a C₆-C₁₄ aryl group which may optionally be substituted; R⁷ represents a hydrogen atom, a C₁-C₅ alkyl group, or R⁹-CO- (R⁹ represents a C₁-C₁₀ alkyl group or a C₆-C₁₂ aryl group which may optionally be substituted); symbol "A" represents a C₁-C₃ alkylene group which may optionally be substituted with a C₁-C₃ alkyl group; and symbol "n" represents 0 or 1.

The present invention will be detailed below.

In the aforementioned general formula (I), examples of the C₁-C₂₀ alkyl group defined by R⁸ include methyl group, ethyl group, propyl group, isopropyl group, butyl group, isobutyl group, sec-butyl group, tert-butyl group, pentyl group, isopentyl group, neopentyl group, tert-pentyl group, hexyl group, isohexyl group, heptyl group, octyl group, nonyl group, decyl group, undecyl group, dodecyl group, tridecyl group, tetradecyl group, pentadecyl group, and octadecyl group. These alkyl groups may have one or more substituents selected from the group consisting of a C₃-C₈ cycloalkyl group such as cyclopropyl group, cyclobutyl group, cyclopentyl group, cyclohexyl group, cycloheptyl group, and cyclooctyl group; a C₃-C₈ cycloalkyloxy group such as cyclopropyloxy group, cyclobutyloxy group, cyclopentyloxy group, cyclohexyloxy group, cycloheptyloxy group, and cyclooctyloxy group; fluorenyl group; a C₁-C₅ alkoxy group such as methoxy group, ethoxy group, propoxy group, isopropoxy group, butoxy group, isobutoxy group, tert-butoxy group, pentyloxy group, and isopentyloxy group; a C₆-C₁₄ aryl group such as phenyl group, naphthyl group, and anthryl group; a C₆-C₁₄ aryloxy group such as phenoxy group and naphthoxy group; a C₆-C₁₄ arylthio group such as phenylthio group and naphthylthio group; a C₆-C₁₄ arylsulfonyl group such as phenylsulfonyl group and naphthylsulfonyl group; and a residue of a heterocyclic compound which contains from 1 to 4 heteroatoms selected from oxygen atom, sulfur atom, or nitrogen atom and has 5 to 10 ring-constituting atoms, and whose examples include furan ring, dihydrofuran ring, tetrahydrofuran ring, pyran ring, dihydropyran ring, tetrahydropyran ring, benzofuran ring, isobenzofuran ring, chromene ring, chroman ring, isochroman ring, thiophene ring, benzothiophene ring, pyrrole ring, pyrroline ring, pyrrolidine ring, imidazole ring, imidazoline ring, imidazolidine ring, pyrazole ring, pyrazoline ring, pyrazolidine ring, triazole ring, tetrazole ring, pyridine ring, pyridine oxide ring, piperidine ring, pyrazine ring, piperazine ring, pyrimidine ring, pyridazine ring, indolizine ring, indole ring, indoline ring, isoindole ring, isoindoline ring, indazole ring, benzimidazole ring, purine ring, quinolizine ring, quinoline ring, phthalazine ring, naphtylidine ring, quinoxaline ring, quinazoline ring, cinnoline ring, pteridine ring, oxazole ring, oxazolidine ring, isoxazole ring, isoxazolidine ring, thiazole ring, thiazolidine ring, isothiazole ring, isothiazolidine ring, dioxane ring, dithian ring, morpholine ring, and thiomorpholine ring. Examples of the C₃-C₈ cycloalkyl group and C₆-C₁₄ aryl group defined by R⁸ include the same groups as those mentioned as the substituents of the aforementioned C₁-C₂₀ alkyl group. Examples of the C₂-C₅ alkenyl group include vinyl group, 1-propenyl group, 2-propenyl group, 1-butenyl group, or 1-pentenyl group. The alkenyl groups may be substituted with a C₆-C₁₄ aryl group such as those explained above as the substituents of the aforementioned C₁-C₂₀ alkyl group. Examples of the residue of a heterocyclic compound include the same groups as those explained as the substituent of the C₁-C₂₀ alkyl group.

The C₁-C₅ alkyl group defined by R², R⁴ and R⁶ may independently be, for example, methyl group, ethyl group, propyl group, isopropyl group, butyl group, isobutyl group, pentyl group, isopentyl group or the like. Examples of the C₂-C₆ alkanoyl group include acetyl group, propionyl group, butyryl group, valeryl group or the like.

Examples of the C₁-C₂₀ alkyl group defined by R³ and R⁵ include independently the same groups as those defined as to R⁸. These alkyl groups may have one or more substituents selected from the group consisting of a C₆-C₁₄ aryl group such as those explained as to R⁸; hydroxyl group; a C₁-C₅ alkoxy group such as those explained above as to R⁸; a C₁-C₅ alkylthio group such as methylthio group, ethylthio group, propylthio group, isopropylthio group, butylthio group, isobutylthio group, tert-butylthio group, pentylthio group, and isopentylthio group; and a C₇-C₁₂ aralkyloxy group such as benzyloxy group, phenylmethoxy group, and naphthylmethoxy group. Examples of the C₆-C₁₄ aryl group include the same groups as those mentioned above.

Examples of the C₁-C₅ alkyl group defined by R⁷ include methyl group, ethyl group, propyl group, isopropyl group, butyl group, isobutyl group, sec-butyl group, tert-butyl group, pentyl group, isopentyl group, neopentyl group, tert-pentyl group or the like.

Examples of the C₁-C₁₀ alkyl group defined by R⁹ include methyl group, ethyl group, propyl group, isopropyl group, butyl group, isobutyl group, sec-butyl group, tert-butyl group, pentyl group, isopentyl group, neopentyl group, tert-pentyl group, hexyl group, isohexyl group, heptyl group, octyl group, nonyl group, decyl group or the like. Examples of the C₆-C₁₂ aryl group include phenyl group, naphthyl group or the like.

Examples of the C₁-C₃ alkylene group defined by the symbol "A" include methylene group, ethylene group, propylene group or the like, and these alkylene groups may have one or two C₁-C₃ alkyl groups such as methyl group, ethyl group and propyl group.

In the aforementioned definition, the aryl group and the heterocyclic residue may further have one or more substituents when they are attached at the end of each functional group, and examples of said substituents include a halogen atom such as fluorine atom, chlorine atom, and bromine atom; a C₁-C₅ alkyl group such as methyl group, ethyl group, propyl group, isopropyl group, butyl group, isobutyl group, sec-butyl group, tert-butyl group, pentyl group, isopentyl group, neopentyl group, and tert-pentyl group; trifluoromethyl group; a C₁-C₅ alkoxy group such as methoxy group, ethoxy group, propoxy group, isopropoxy group, butoxy group, isobutoxy group, tert-butoxy group, pentyloxy group, and isopentyloxy group; a C₁-C₅ alkylenedioxy group such as methylenedioxy group, ethylenedioxy group, and propylenedioxy group; hydroxyl group; nitro group; a C₂-C₆ alkylcarbonyloxy group such as acetoxy group, propionyloxy group, butyryloxy group, and valeryloxy group; carboxyl group; a C₂-C₆ alkoxycarbonyl group such as methoxycarbonyl group, ethoxycarbonyl group, propoxycarbonyl group, isopropoxycarbonyl group, butoxycarbonyl group, isobutoxycarbonyl group, tert-butoxycarbonyl group, and pentyloxycarbonyl group; oxo group; a C₂-C₆ alkylcarbonyl group such as acetyl group, propionyl group, butyryl group, and valeryl group; amino group; a C₁-C₅ monoalkylamino group such as methylamino group, ethylamino group, propylamino group, isopropylamino group, butylamino group, isobutylamino group, tert-butylamino group, pentylamino group, and isopentylamino group; a C₂-C₁₀ dialkylamino group such as dimethylamino group, ethylmethylamino group, diethylamino group, methylpropylamino group, and diisopropylamino group; a C₂-C₆ alkylcarbonylamino group such as acetylamino group, propionylamino group, isopropionylamino group, butyrylamino group, and valerylamino group; carbamoyl group; a C₂-C₆ alkylcarbamoyl group such as methylcarbamoyl group, ethylcarbamoyl group, propylcarbamoyl group, butylcarbamoyl group, tert-butylcarbamoyl group, and pentylcarbamoyl group; and a C₆-C₁₂ aryl group such as phenyl group and naphthyl group.

Among the compounds of the present invention, preferred compounds include those wherein R¹ represents a hydrogen atom, R⁸-CO-, R⁸-O-CO-, R⁸-NH-CO-, or R⁸-SO₂- (R⁸ represents a C₁-C₂₀ alkyl group which may optionally be substituted with one or more substituents selected from the group consisting of a C₃-C₈ cycloalkyl group, fluorenyl group, a C₆-C₁₄ aryl group which may optionally be substituted, a C₆-C₁₄ aryloxy group which may optionally be substituted, a C₆-C₁₄ arylthio group which may optionally be substituted, a C₆-C₁₄ arylsulfonyl group which may optionally be substituted, and a residue of a heterocyclic compound which may optionally be substituted; a C₃-C₈ cycloalkyl group; a C₆-C₁₄ aryl group which may optionally be substituted; a C₂-C₅ alkenyl group which may optionally be substituted with an optionally substituted C₆-C₁₄ aryl group; or a residue of a heterocyclic compound which may optionally be substituted); R², R⁴, and R⁶ independently represent a hydrogen atom, a C₁-C₅ alkyl group, or a C₂-C₆ alkanoyl group; R³ and R⁵ independently represent a hydrogen atom, a C₁-C₂₀ alkyl group which may optionally be substituted with one or more substituents selected from the group consisting of a C₆-C₁₄ aryl group which may optionally be substituted and a C₁-C₅ alkoxy group; R⁷ represents a hydrogen atom, a C₁-C₅ alkyl group, or R⁹-CO- (R⁹ represents a C₁-C₁₀ alkyl group or a C₆-C₁₂ aryl group which may optionally be substituted); symbol "A" represents a C₁-C₃ alkylene group which may optionally be substituted with a C₁-C₃ alkyl group; and symbol "n" represents 0 or 1. Among these compounds, those wherein R², R⁴, and R⁶ independently represent a hydrogen atom or a C₁-C₅ alkyl group are more preferred, and those wherein symbol "n" represents 0 are further preferred. Particularly preferred compounds include:
(1) compounds wherein R¹ represents R⁸-CO- (R⁸ represents a C₁-C₂₀ alkyl group which may optionally be substituted with one or more substituents selected from the group consisting of a C₆-C₁₄ aryl group which may optionally be substituted, a C₆-C₁₄ aryloxy group which may optionally be substituted, a C₆-C₁₄ arylthio group which may optionally be substituted, and a C₆-C₁₄ arylsulfonyl group which may optionally be substituted; a C₆-C₁₄ aryl group which may optionally be substituted; a C₂-C₅ alkenyl group which may optionally be substituted with an optionally substituted C₆-C₁₄ aryl group; or a residue of a heterocyclic compound which may optionally be substituted); R², R⁴, and R⁶ represent hydrogen atoms; R³ and R⁵ independently represent a C₁-C₂₀ alkyl group; R⁷ represents a hydrogen atom or R⁹-CO- (R⁹ represents a C₁-C₁₀ alkyl group); symbol "A" represents a C₁-C₃ alkylene group; and symbol "n" represents 0;
(2) compounds wherein R¹ represents R⁸-O-CO- (R⁸ represents a C₁-C₂₀ alkyl group which may optionally be substituted with one or more substituents selected from the group consisting of a C₃-C₈ cycloalkyl group, fluorenyl group, a C₆-C₁₄ aryl group which may optionally be substituted, and a residue of a heterocyclic compound which may optionally be substituted; a C₃-C₈ cycloalkyl group; or a C₆-C₁₄ aryl group which may optionally be substituted); R², R⁴, and R⁶ represent hydrogen atoms; R³ and R⁵ independently represent a hydrogen atom, or a C₁-C₂₀ alkyl group which may optionally be substituted with one or more substituents selected from the group consisting of a C₆-C₁₄ aryl group which may optionally be substituted and a C₁-C₅ alkoxy group; R⁷ represents a hydrogen atom or R⁹-CO- (R⁹ represents a C₁-C₁₀ alkyl group); symbol "A" represents a C₁-C₃ alkylene group; and symbol "n" represents 0;
(3) compounds wherein R¹ represents R⁸-NH-CO- (R⁸ represents a C₆-C₁₄ aryl group which may optionally be substituted); R², R⁴, and R⁶ represent hydrogen atoms; R³ and R⁵ independently represent a C₁-C₂₀ alkyl group; R⁷ represents a hydrogen atom or R⁹-CO- (R⁹ represents a C₁-C₁₀ alkyl group or a C₆-C₁₂ aryl group which may optionally be substituted); symbol "A" represents a C₁-C₃ alkylene group; and symbol "n" represents 0; and
(4) compounds wherein R¹ represents R⁸-SO₂- (R⁸ represents a C₆-C₁₄ aryl group which may optionally be substituted or a residue of a heterocyclic compound which may optionally be substituted); R², R⁴, and R⁶ represent hydrogen atoms; R³ and R⁵ independently represent a C₁-C₂₀ alkyl group; R⁷ represents a hydrogen atom, a C₁-C₅ alkyl group, or R⁹-CO- (R⁹ represents a C₁-C₁₀ alkyl group or a C₆-C₁₂ aryl group which may optionally be substituted); symbol "A" represents a C₁-C₃ alkylene group which may optionally be substituted with a C₁-C₃ alkyl group; and symbol "n" represents 0.

Among the compounds of the above definition (1), compounds wherein R¹ represents R⁸-CO- (R⁸ represents a C₁-C₂₀ alkyl group which may optionally be substituted with an optionally substituted C₆-C₁₄ aryl group or a C₆-C₁₄ aryl group which may optionally be substituted); R², R⁴, and R⁶ represent hydrogen atoms; R³ and R⁵ independently represent a C₁-C₂₀ alkyl group; R⁷ represents R⁹-CO- (R⁹ represents a C₁-C₁₀ alkyl group); symbol "A" represents a C₁-C₃ alkylene group; and symbol "n" represents 0 are more preferred.

Among the compounds of the above definition (2), compounds wherein R¹ represents R⁸-O-CO- (R⁸ represents a C₁-C₂₀ alkyl group which may optionally be substituted with one or more substituents selected from the group consisting of fluorenyl group and a C₆-C₁₄ aryl group which may optionally be substituted; or a C₃-C₈ cycloalkyl group); R², R⁴, and R⁶ represent hydrogen atoms; R³ and R⁵ independently represent a C₁-C₂₀ alkyl group; R⁷ represents R⁹-CO- (R⁹ represents a C₁-C₁₀ alkyl group); symbol "A" represents a C₁-C₃ alkylene group; and symbol "n" represents 0 are more preferred.

Among the compounds of the above definition (3), compounds wherein R⁷ represents a hydrogen atom are more preferred.

Among the compounds of the above definition (4), compounds wherein R¹ represents R⁸-SO₂- (R⁸ represents a C₆-C₁₄ aryl group which may optionally be substituted); and R⁷ represents a C₁-C₅ alkyl group, or compounds wherein R¹ represents R⁸-SO₂- (R⁸ represents a C₆-C₁₄ aryl group which may optionally be substituted); and R⁷ represents R⁹-CO- (R⁹ represents a C₆-C₁₂ aryl group which may optionally be substituted) are more preferred.

The oxygen-containing heterocyclic derivatives of the present invention represented by the above formula (I) may form pharmaceutically acceptable salts. As specific examples of these salts, where acidic functional groups are attached, examples include metal salts such as lithium salt, sodium salt, potassium salt, magnesium salt, or calcium salt, and ammonium salts such as ammonium salt, methylammonium salt, dimethylammonium salt, trimethylammonium salt, or dicyclohexylammonium salt. Where basic functional groups are attached, examples include inorganic acid salts such as hydrochloride, hydrobromide, sulfate, nitrate, or phosphate, and organic acid salts such as methanesulfonate, benzenesulfonate, p-toluenesulfonate, acetate, propionate, tartrate, fumarate, maleate, malate, oxalate, succinate, citrate, benzoate, mandelate, cinnamate, or lactate. The oxygen-containing heterocyclic derivatives of the present invention represented by the above formula (I) may exist in the form of hydrates or solvates.

Referring to the stereochemistry of asymmetric carbon atoms exist in the oxygen-containing heterocyclic derivatives of the present invention represented by the above formula (I), they may independently be in (R)-, (S)-, or (RS)-configuration.

A compound represented by the following formula (II), which corresponds to the oxygen-containing heterocyclic derivatives of the present invention represented by the above formula (I) wherein R⁷ is a hydrogen atom (R¹, R², R³, R⁴, R⁵, R⁶, A, and n have the same meanings as those defined above), may present under equilibrium between a hydroxyaldehyde derivative represented by the following formula (III) (R¹, R², R³, R⁴, R⁵, R⁶, A, and n have the same meanings as those defined above), especially in the state of a solution. This equilibrium can be demonstrated by the experimental results as explained below. The results of NMR measurements support the chemical structure of the formula (II), whilst differences in content ratio of the stereoisomers derived from the compound of the formula (II) were observed depending on types of solvents, which is attributable to the differences in content ratio of carbon atoms with different configurations that is bound with the hydroxyl group on the lactol ring. The differences in the content ratio of the stereoisomers can be explained by the presence of the equilibrium shown below.

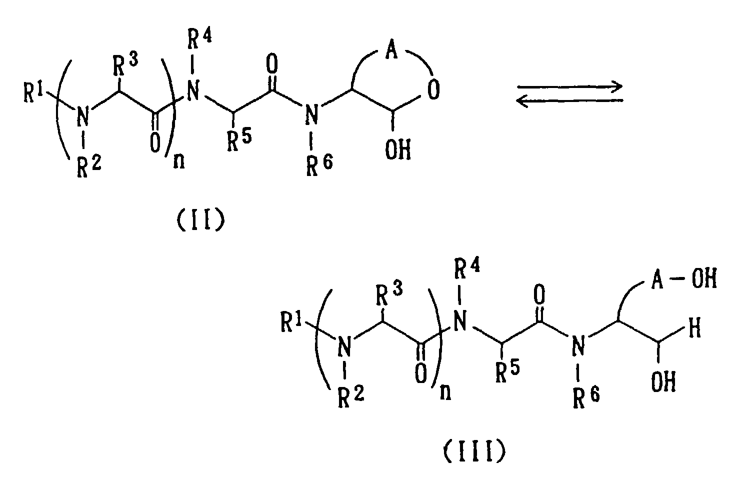

Specific examples of the oxygen-containing heterocyclic derivative of the present invention represented by the above formula (I) include those shown in Table 1 below wherein n is 0 and R⁷ is a hydrogen atom; those shown in Table 2 below wherein n is 0 and R⁷ is other than a hydrogen atom; those shown in Table 3 wherein n is 1 and R⁷ is a hydrogen atom; and those shown in Table 4 wherein n is 1 and R⁷ is other than a hydrogen atom.

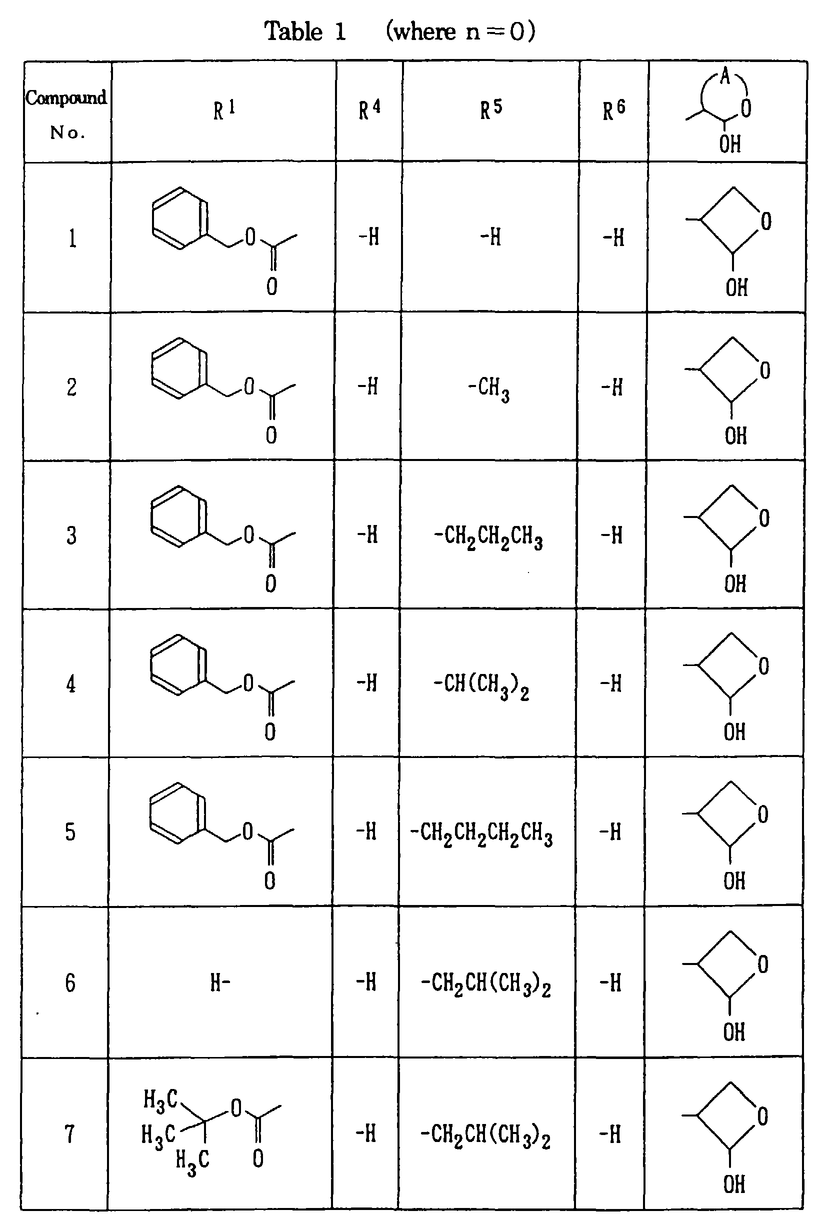

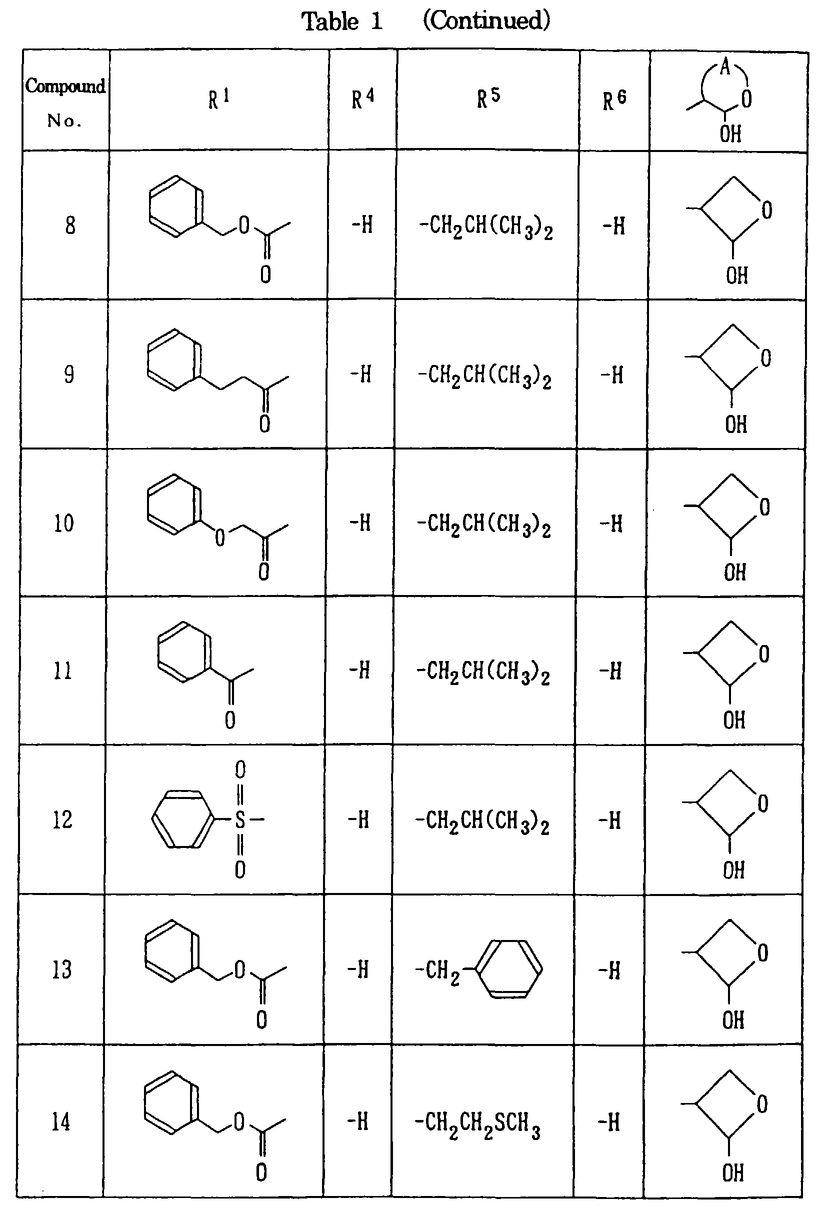

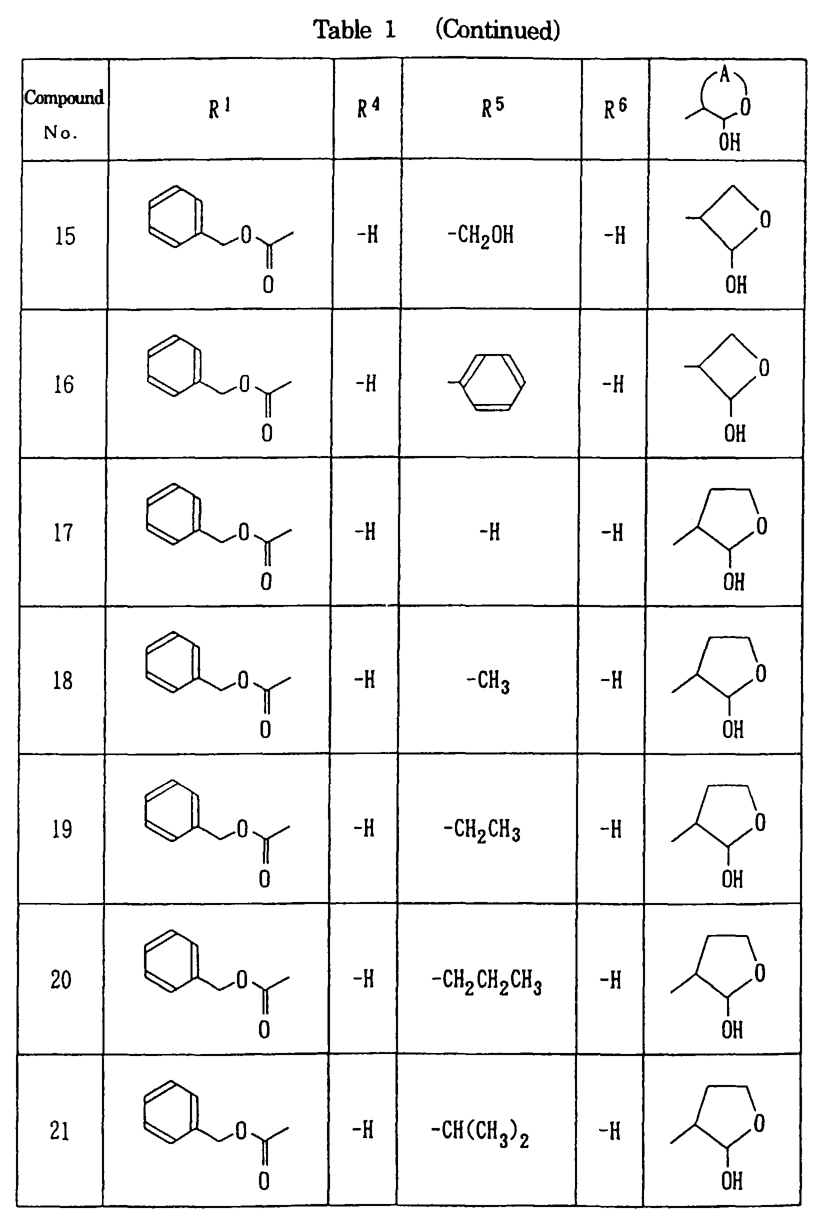

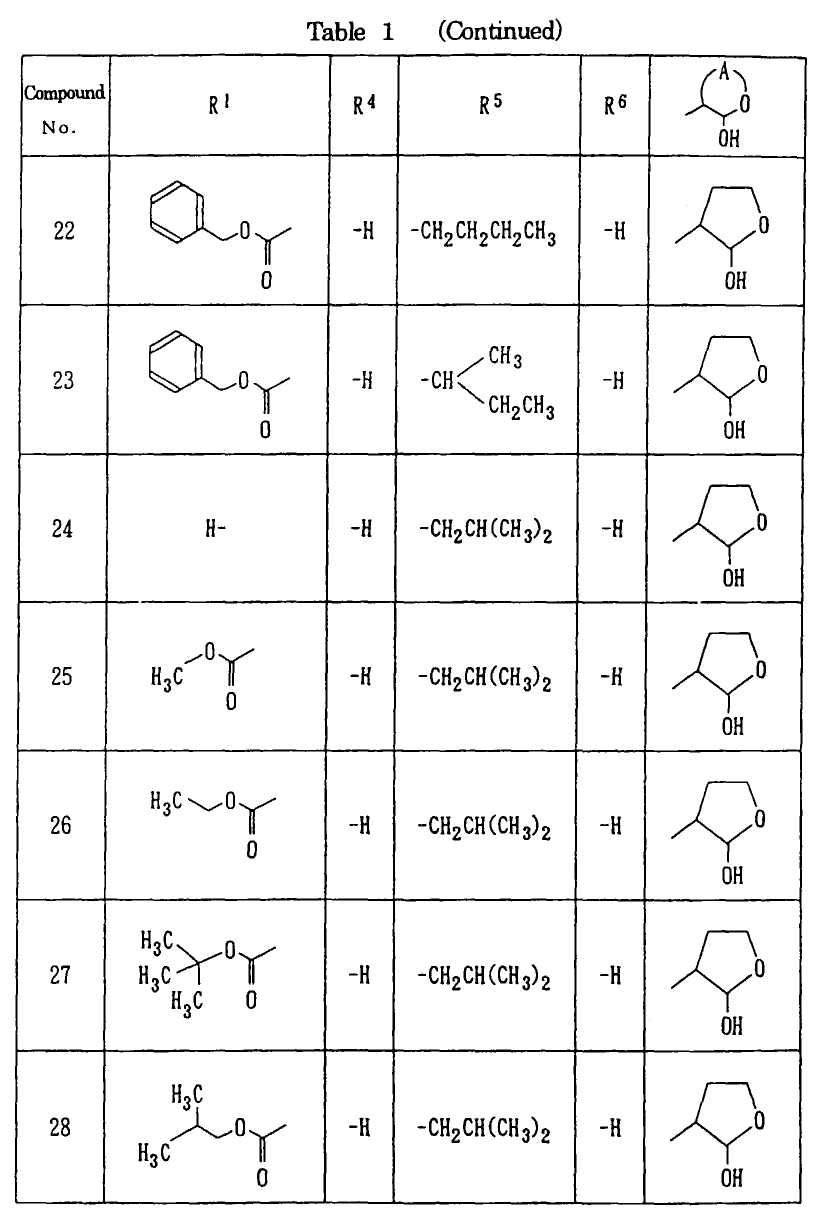

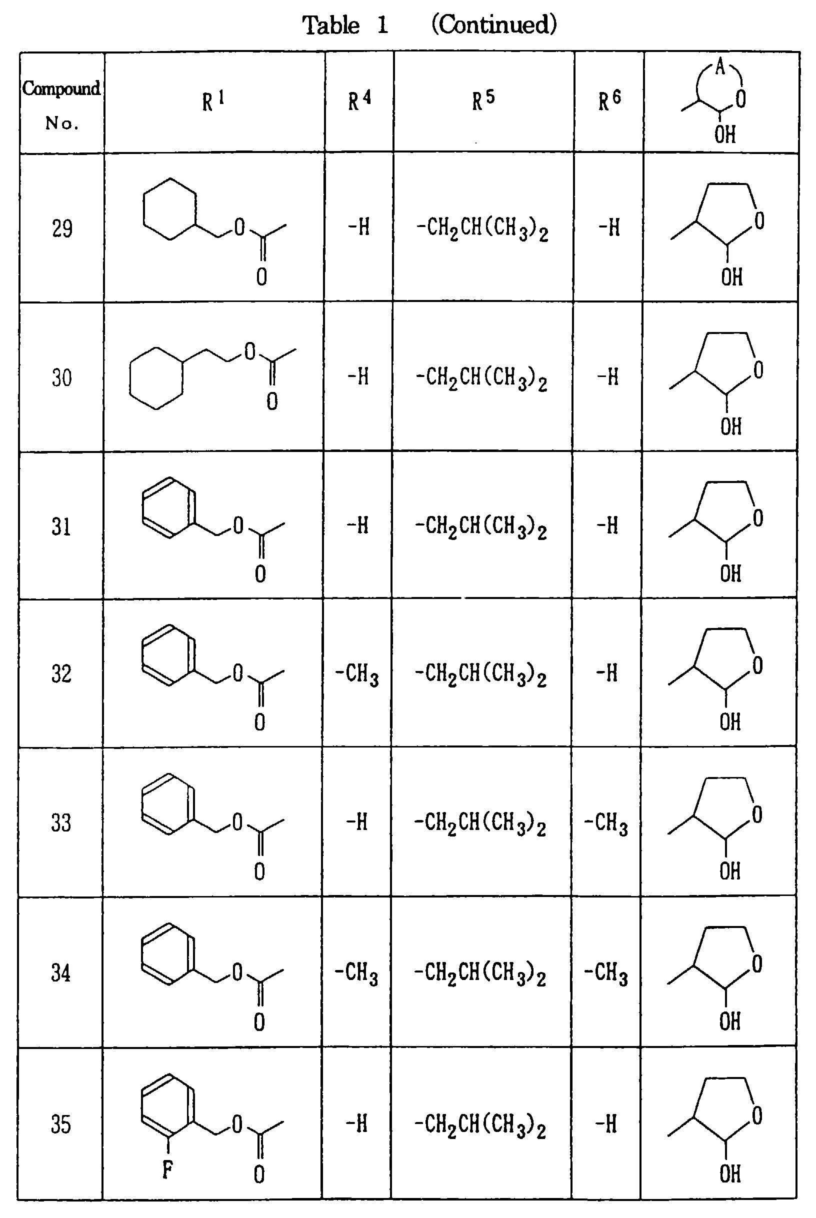

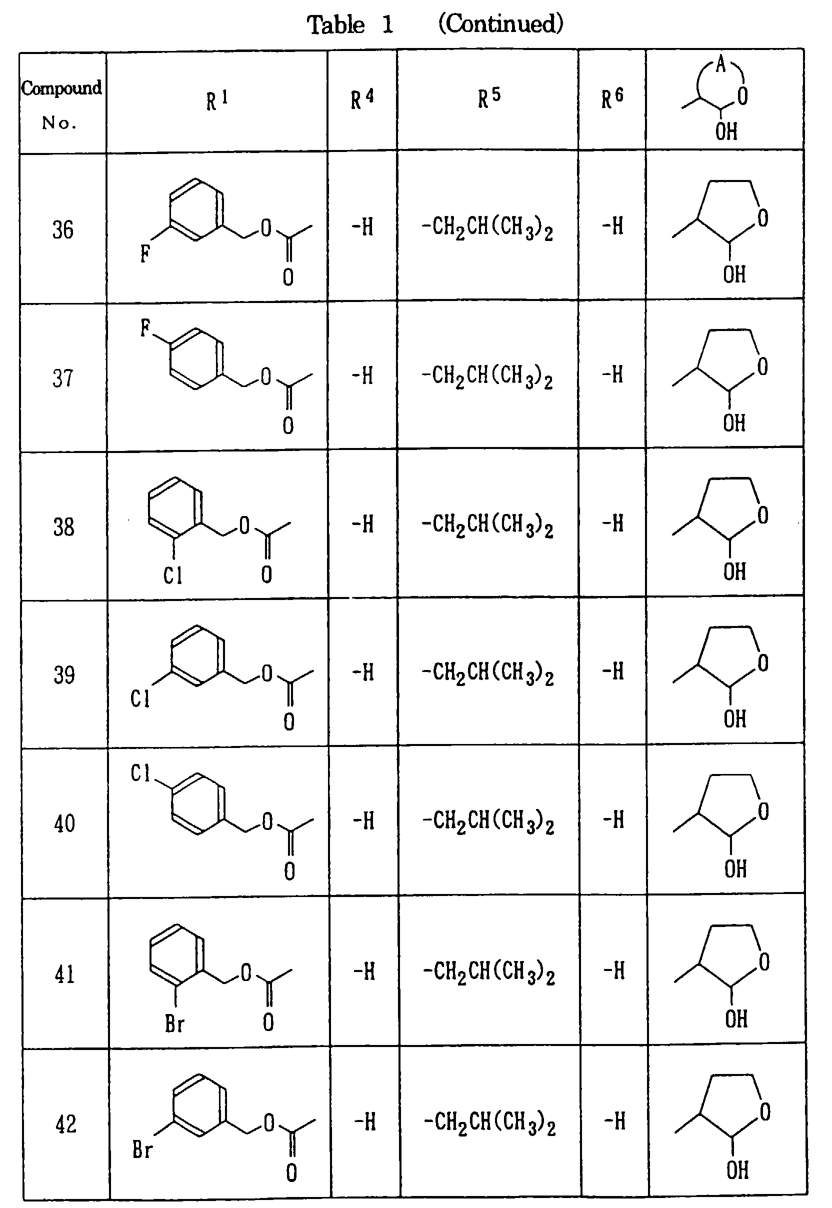

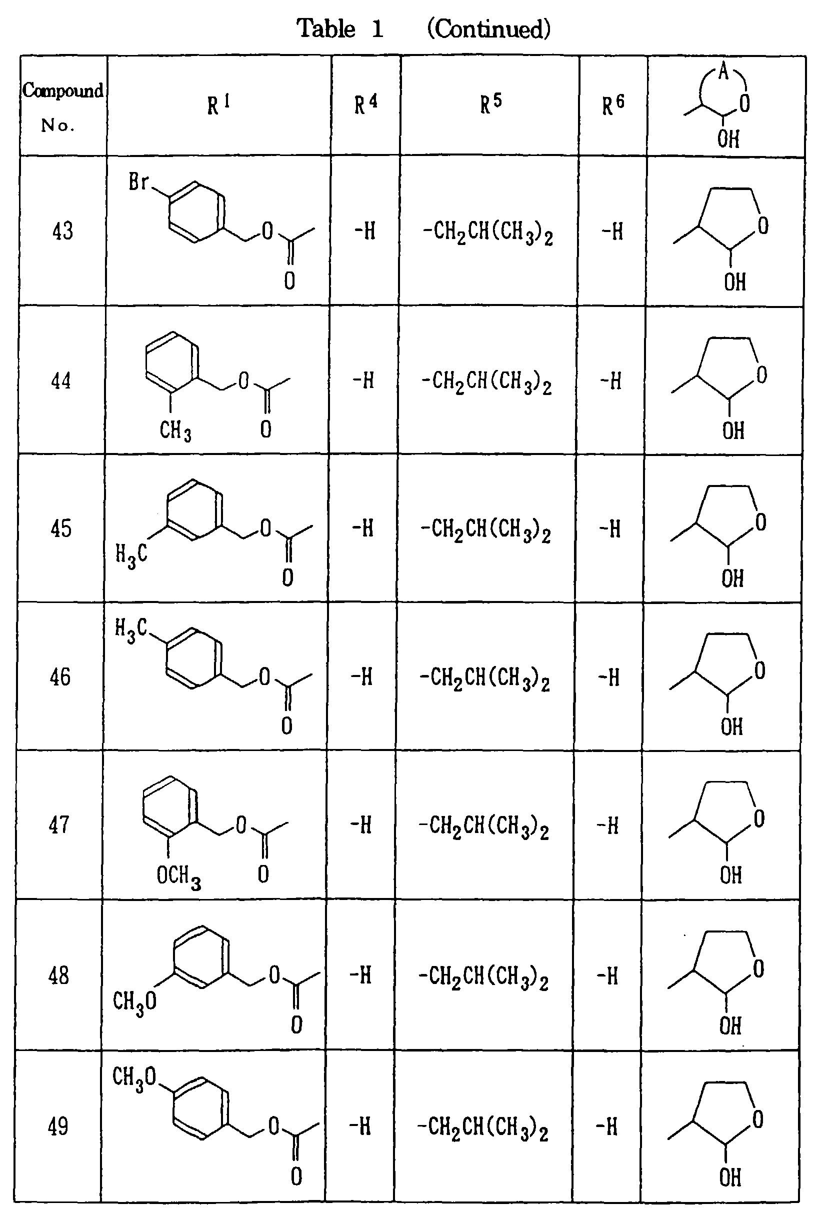

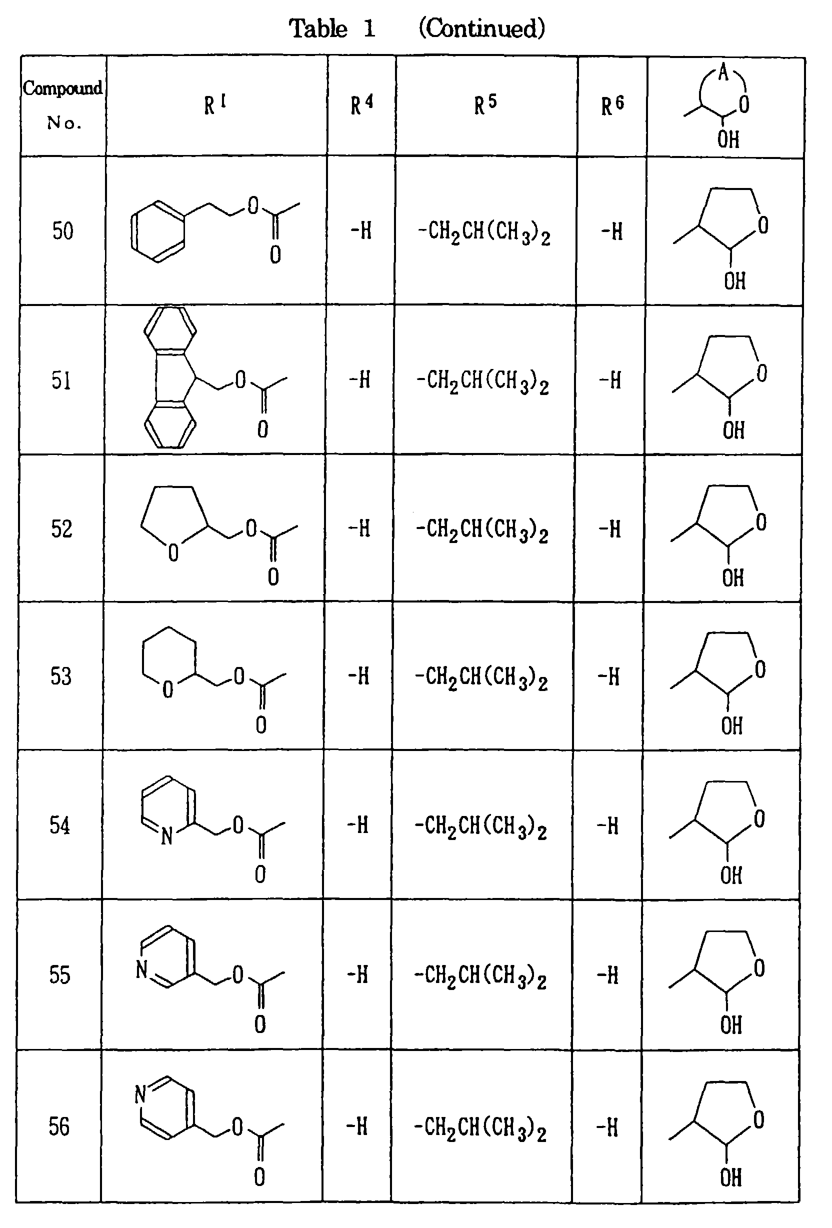

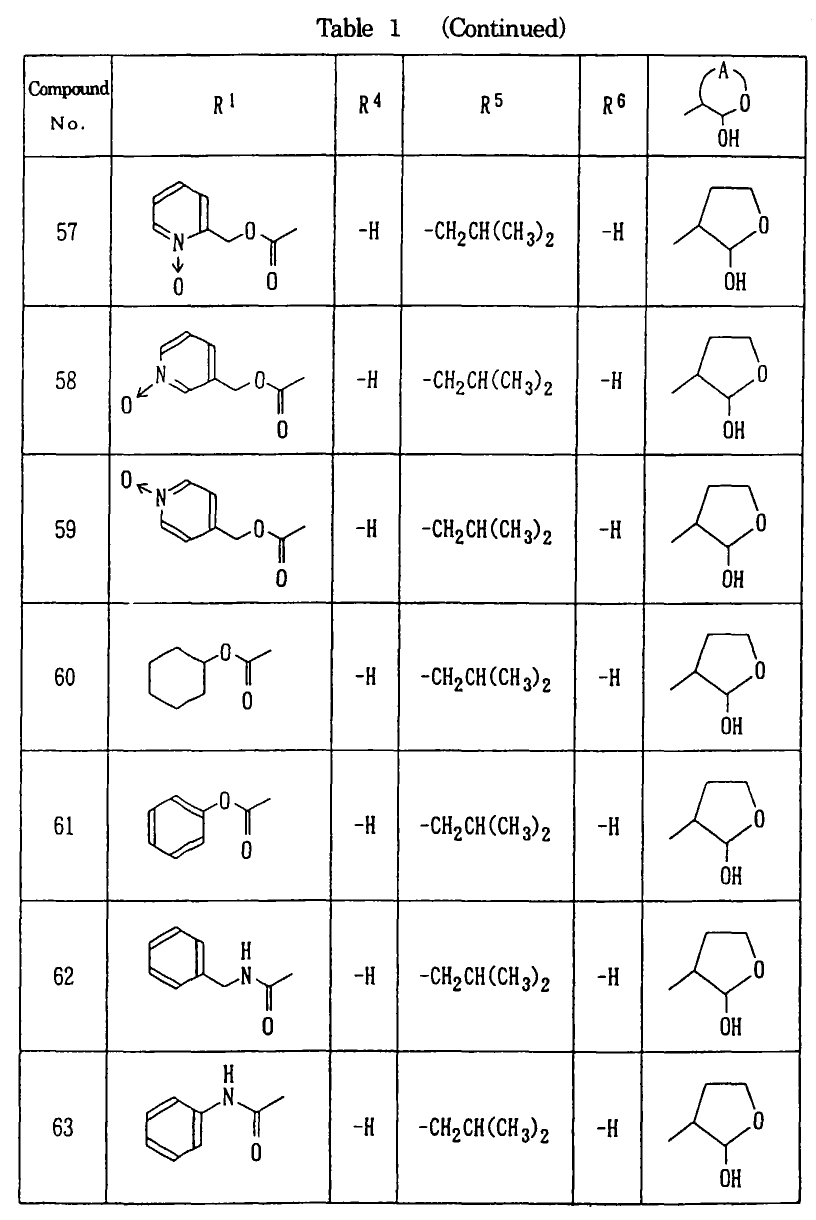

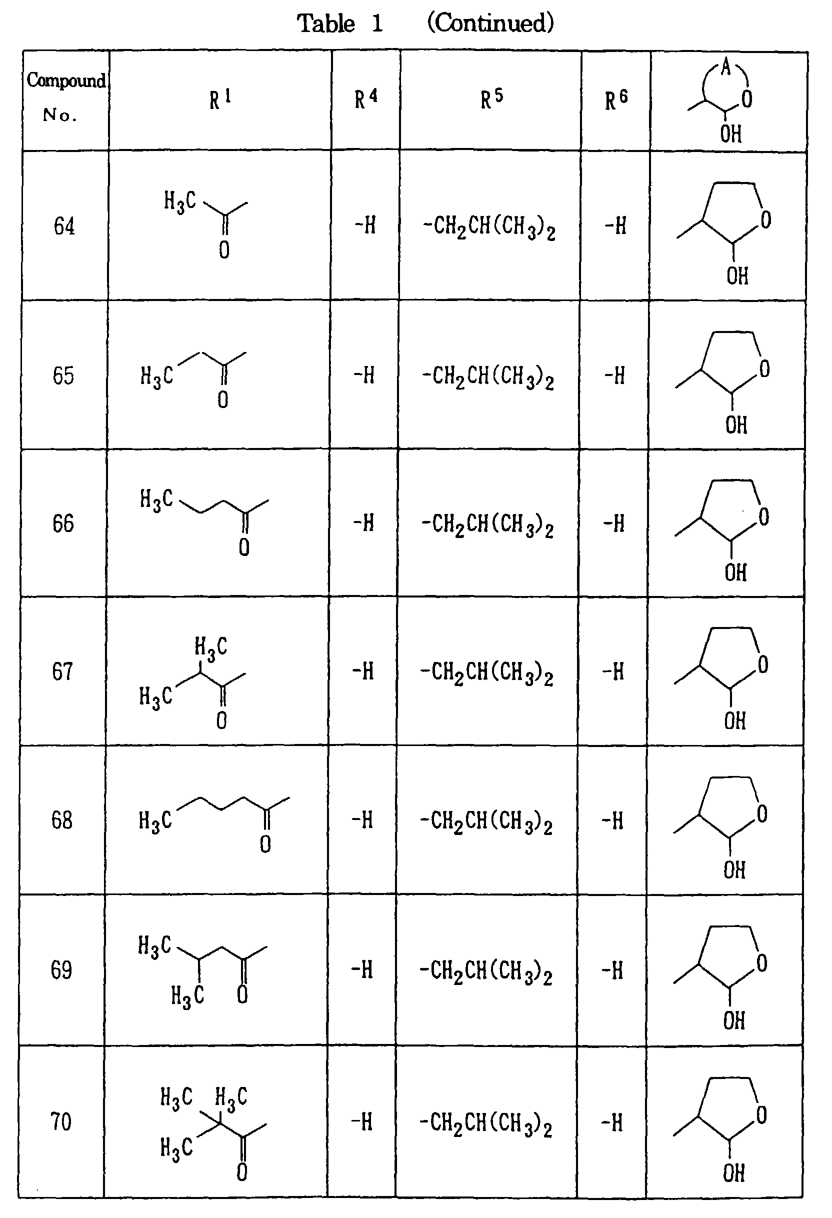

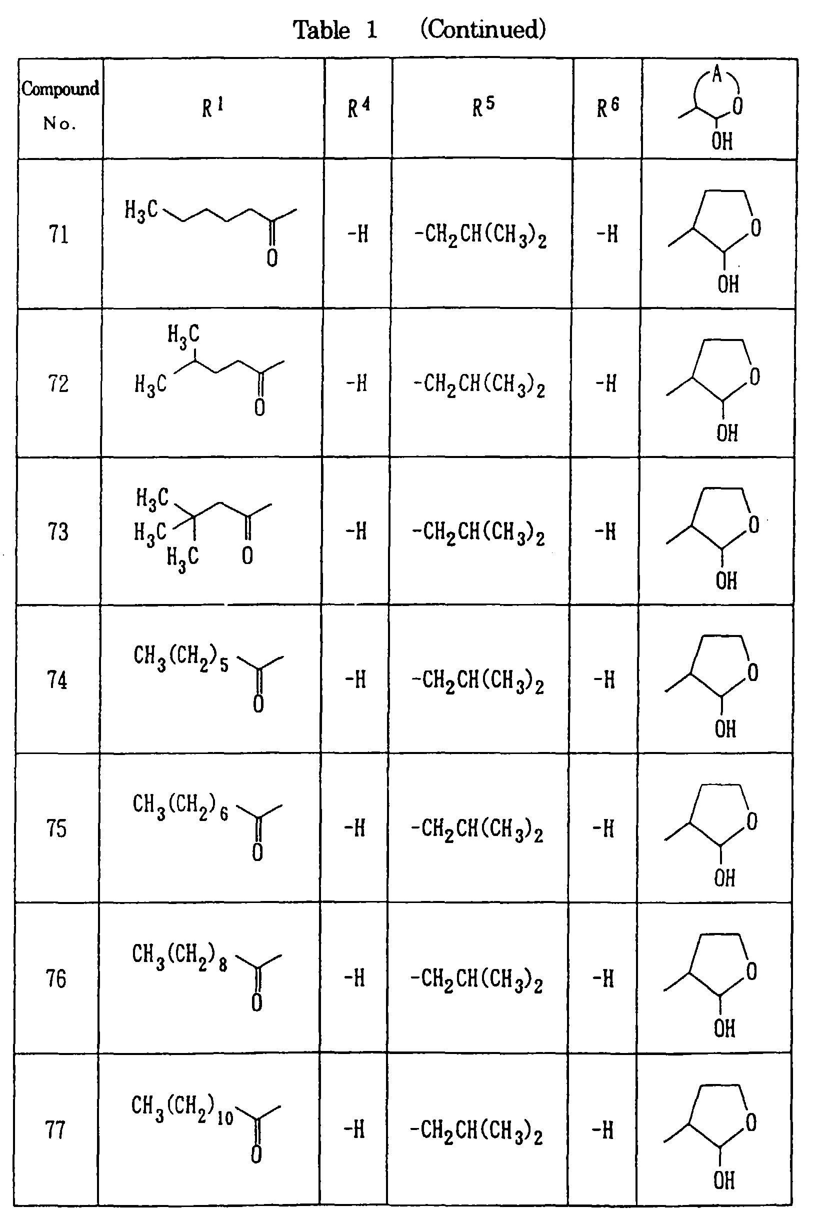

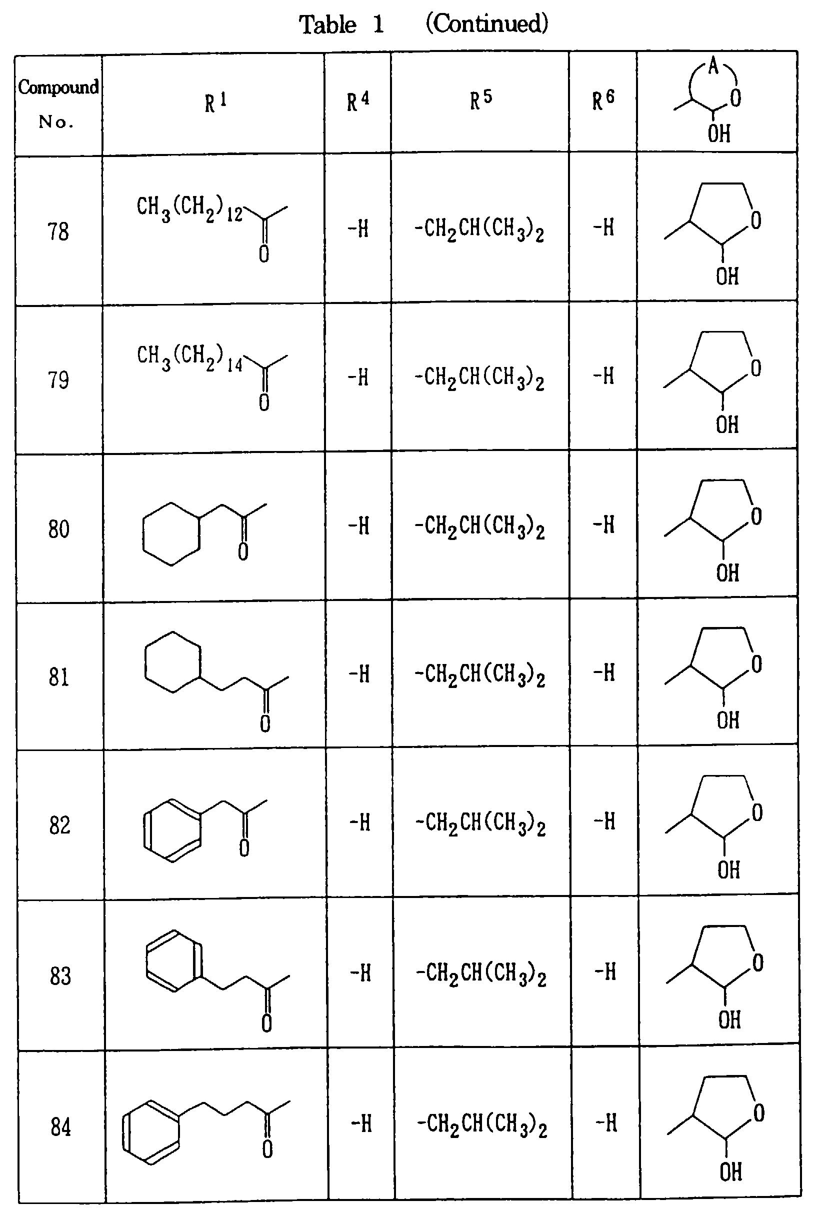

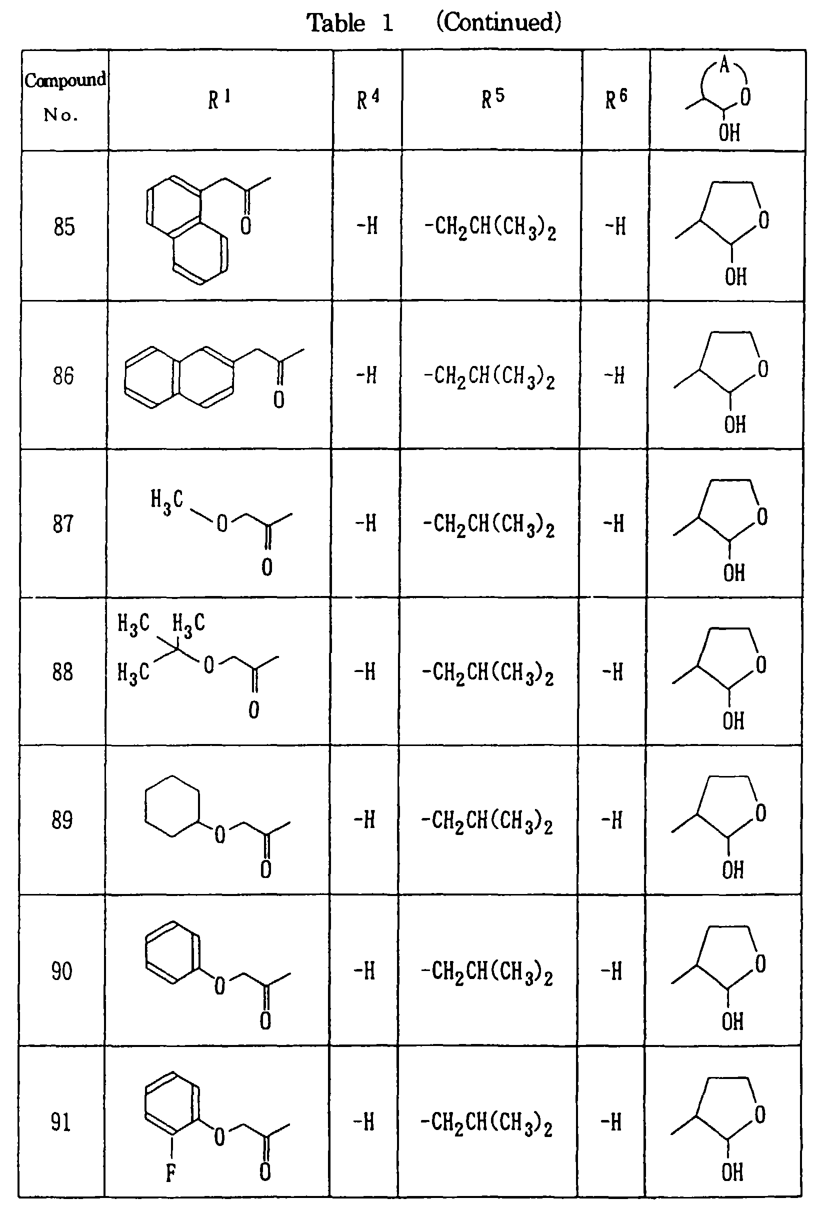

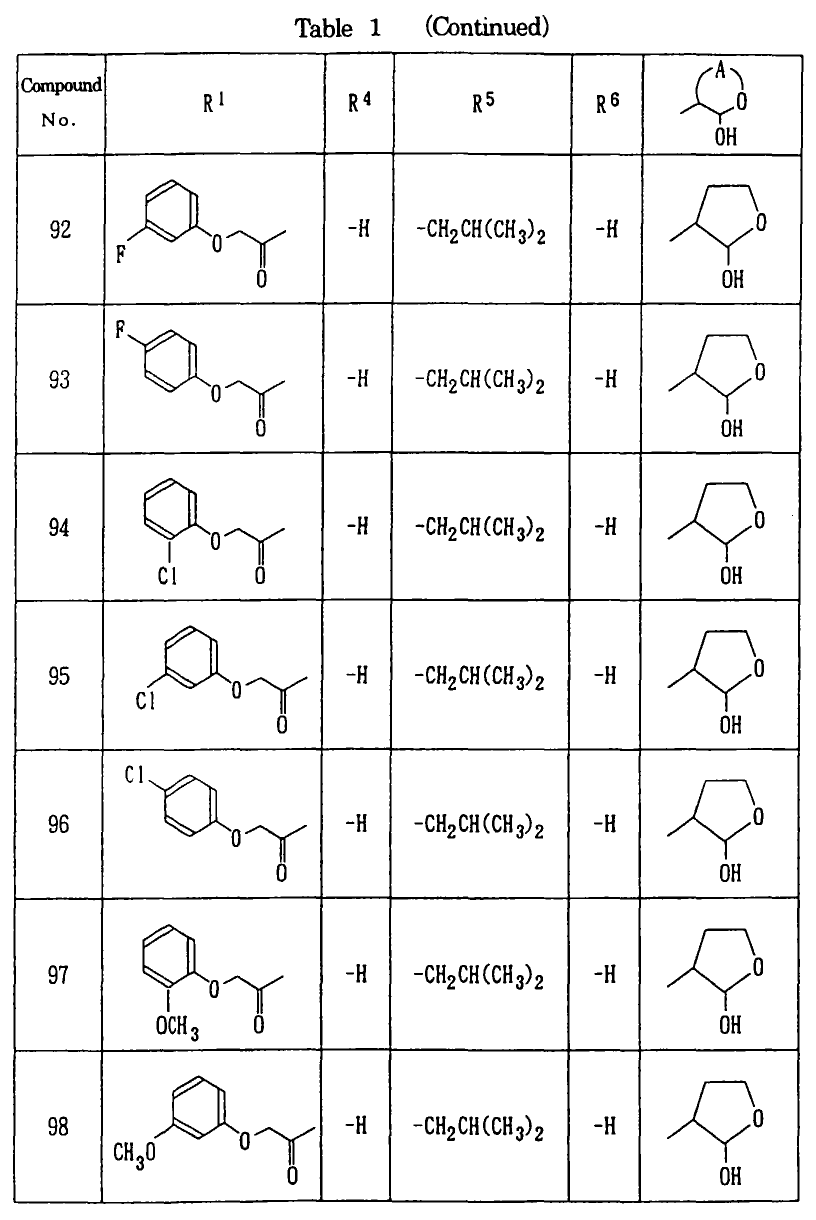

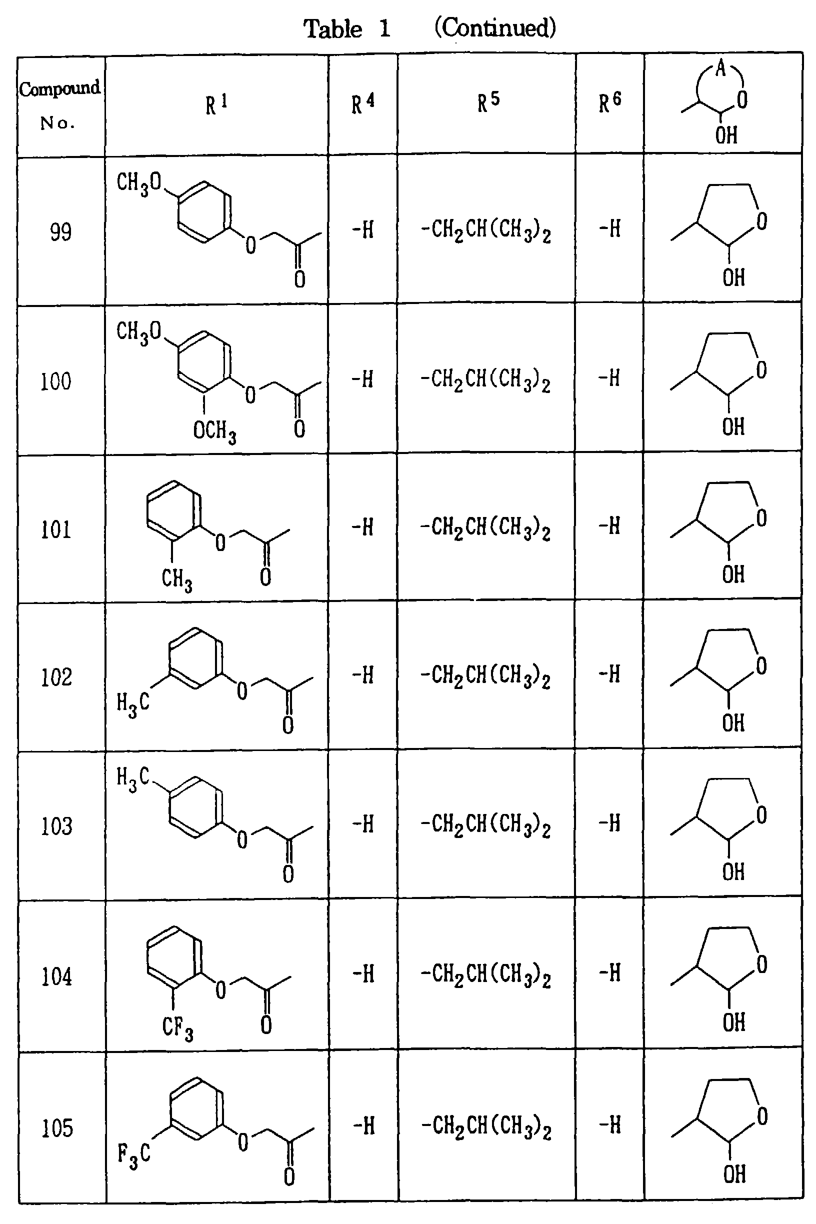

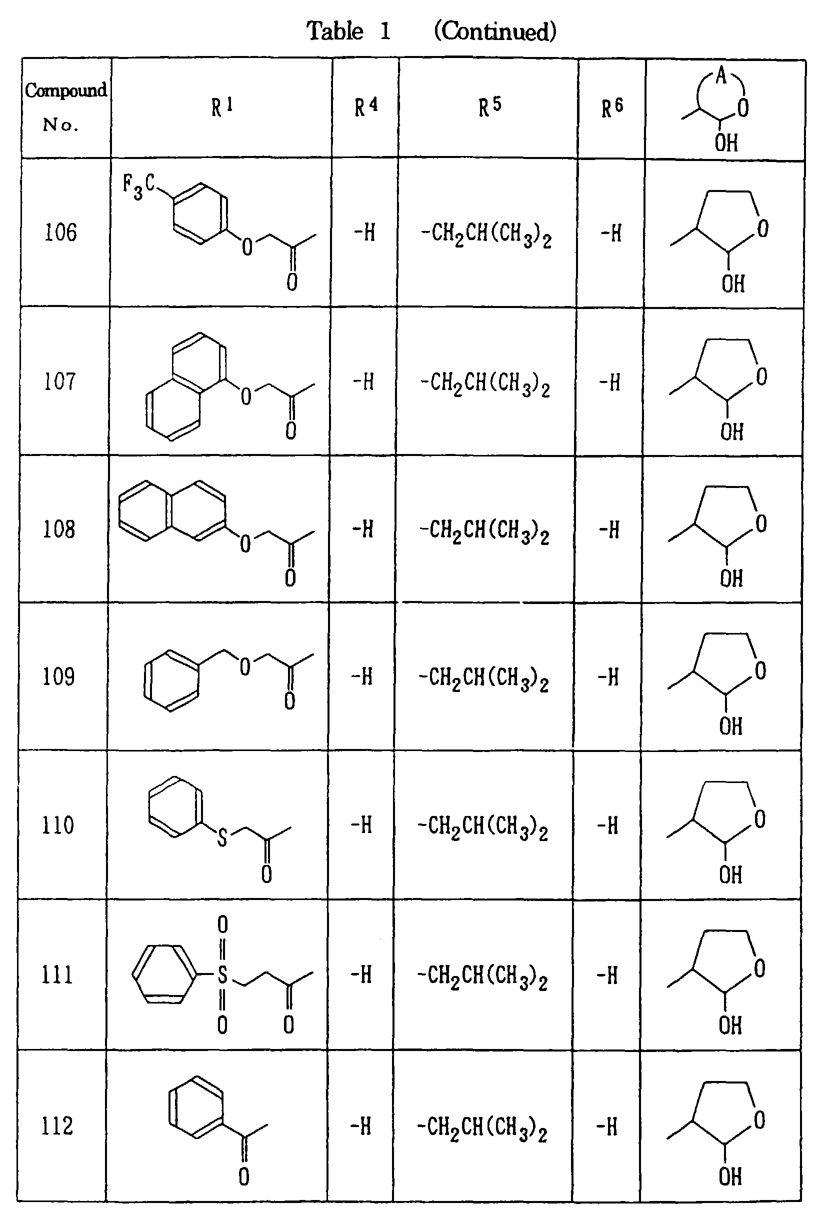

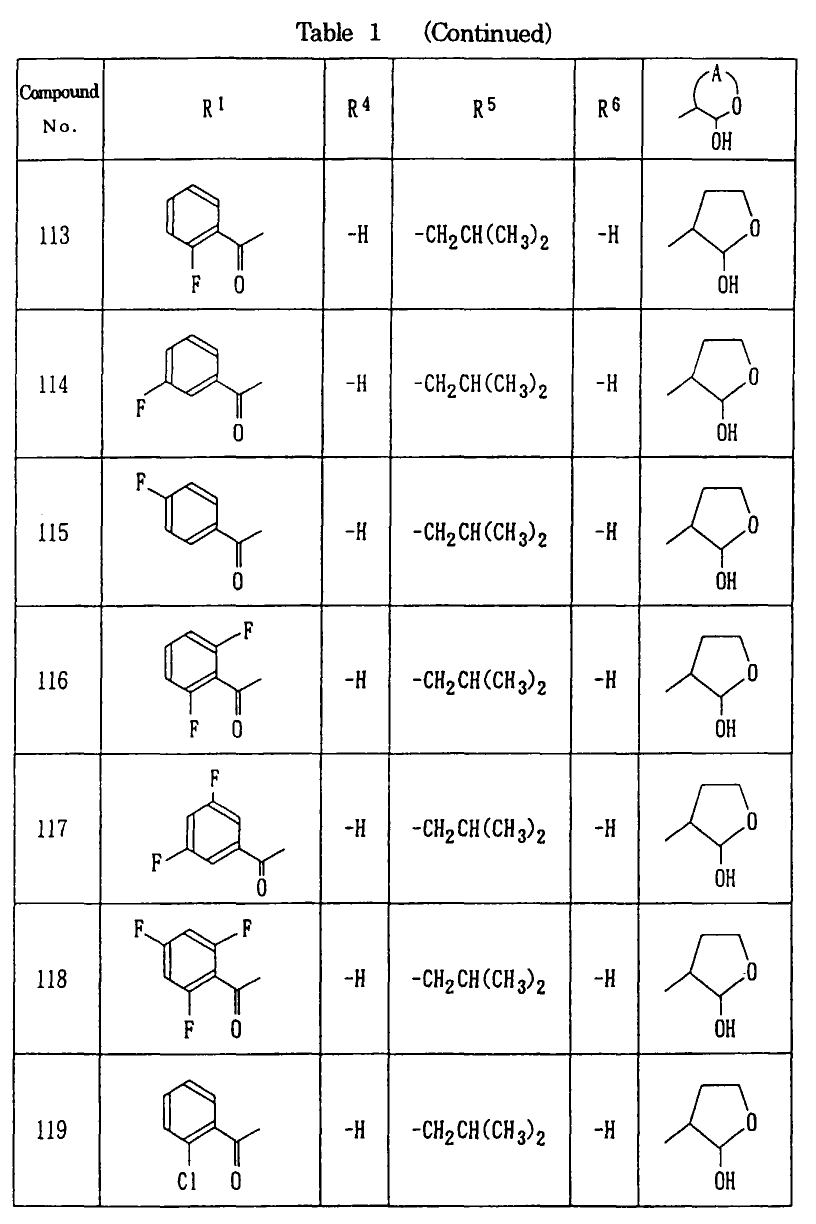

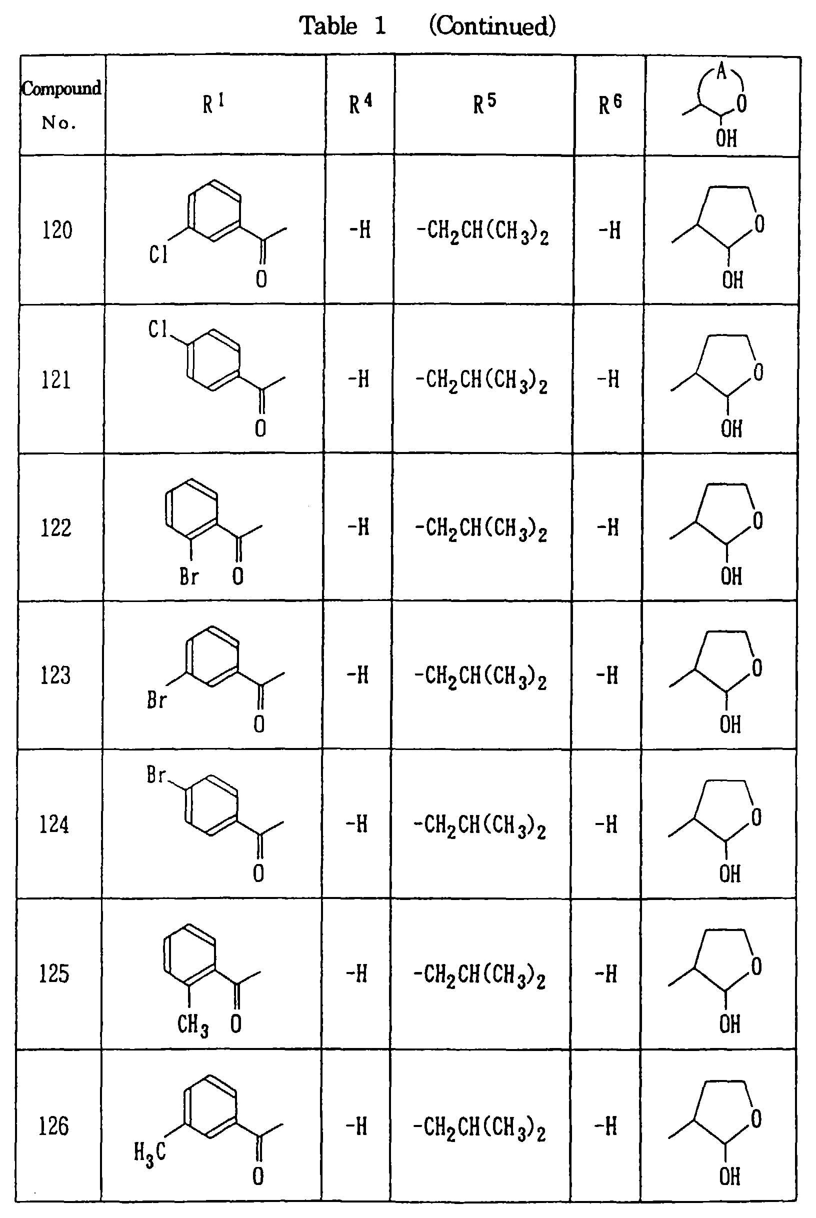

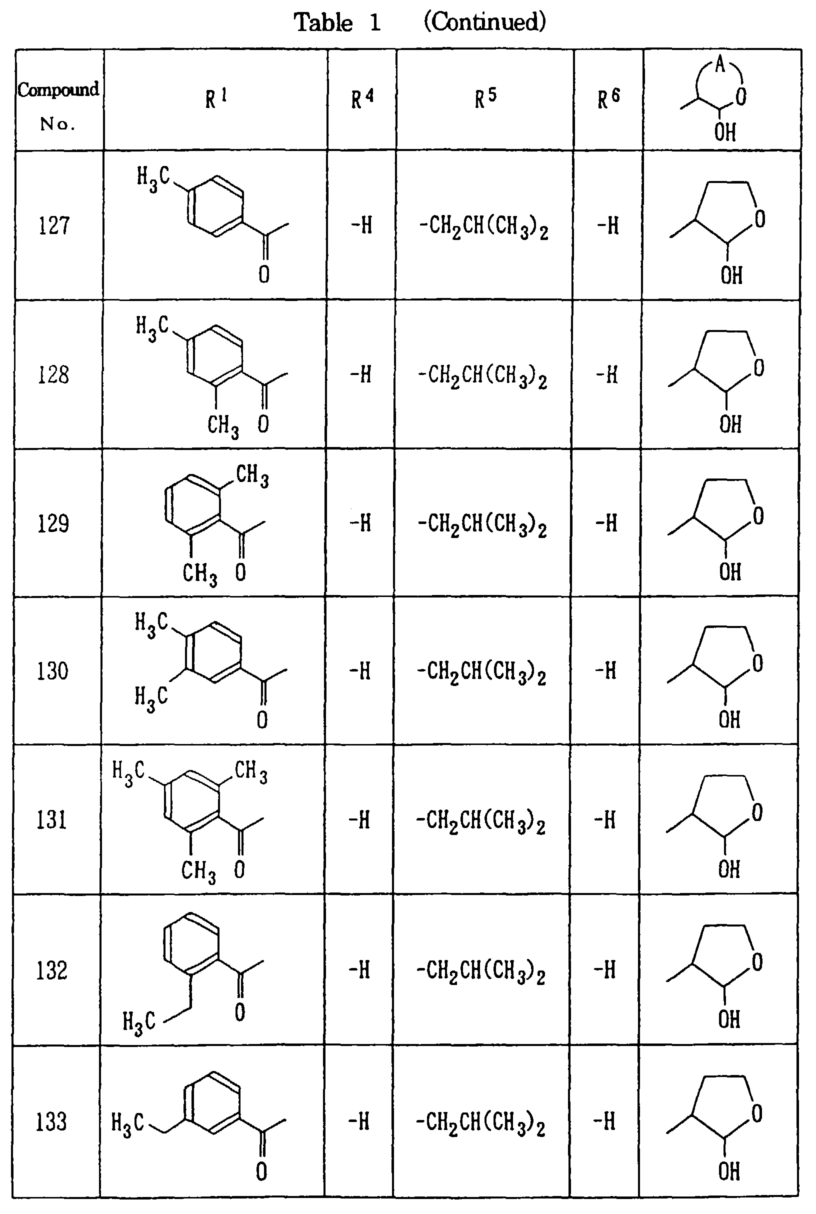

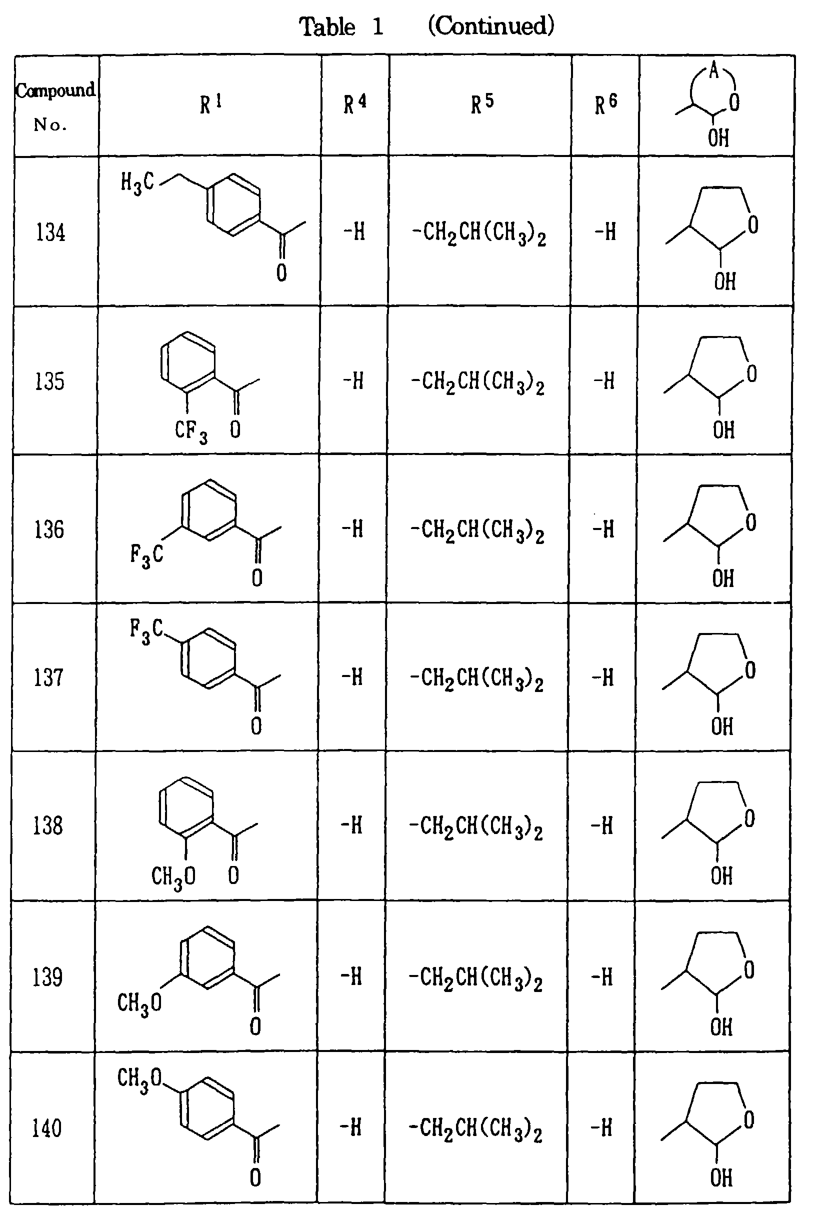

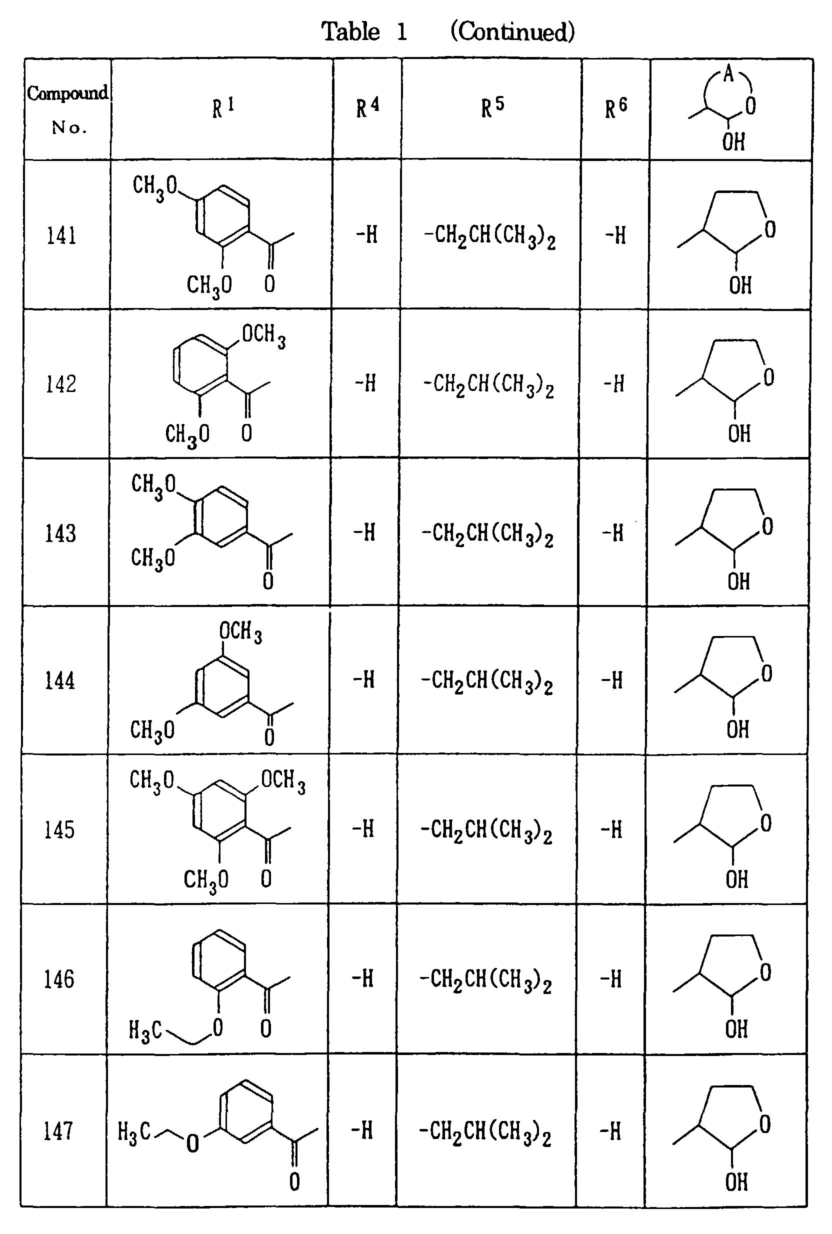

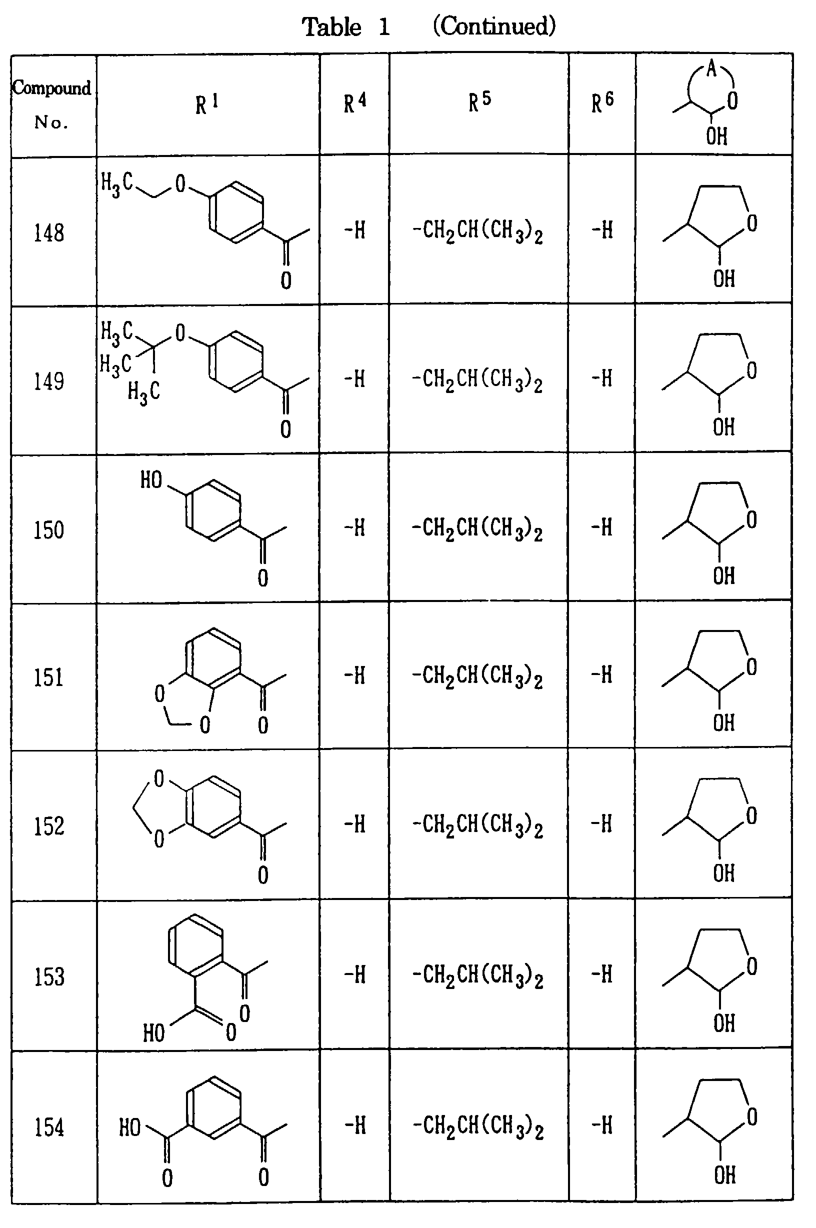

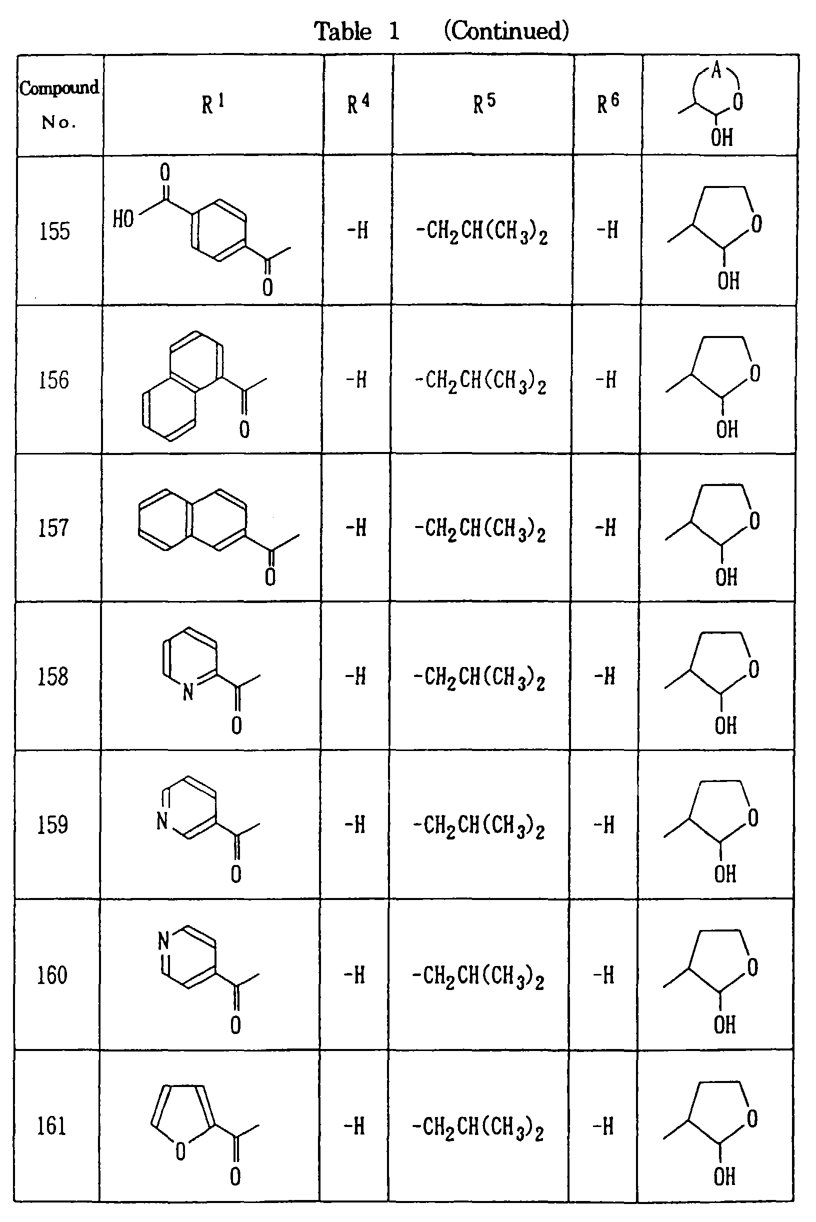

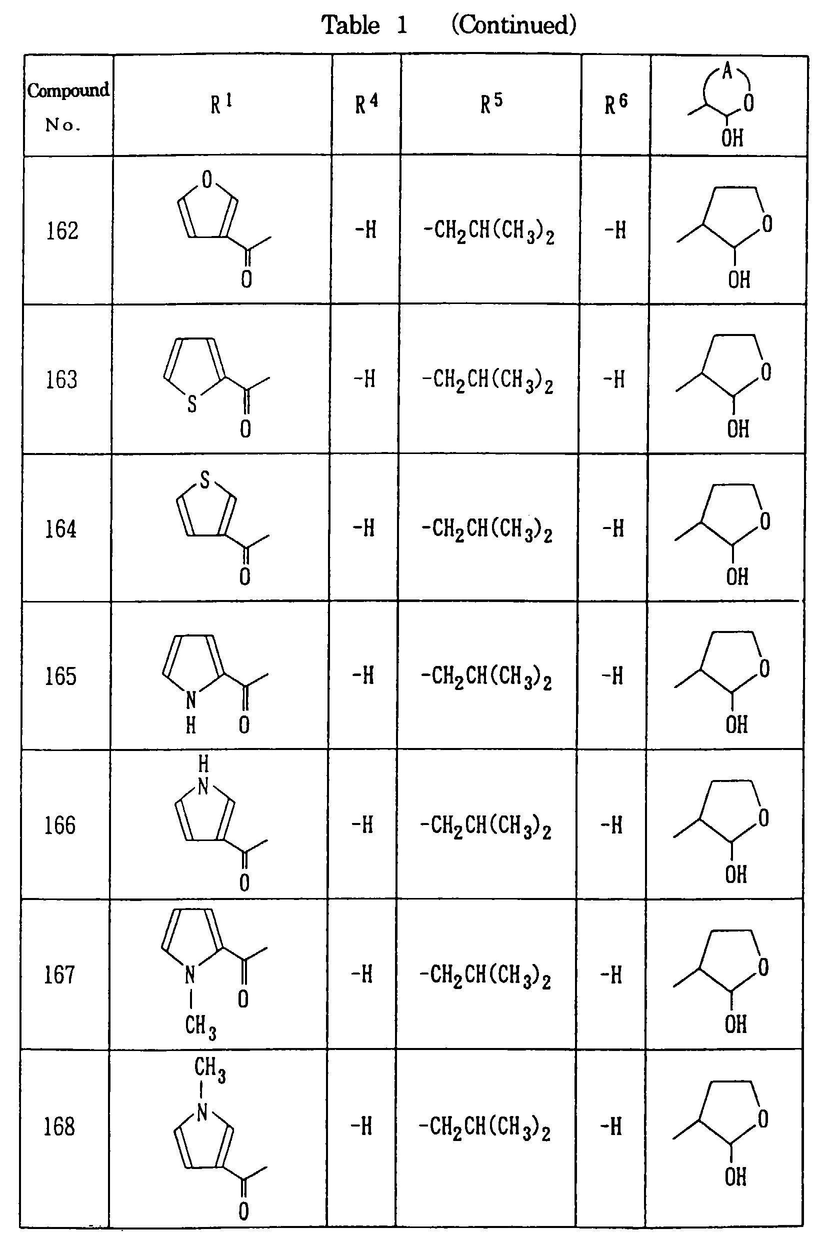

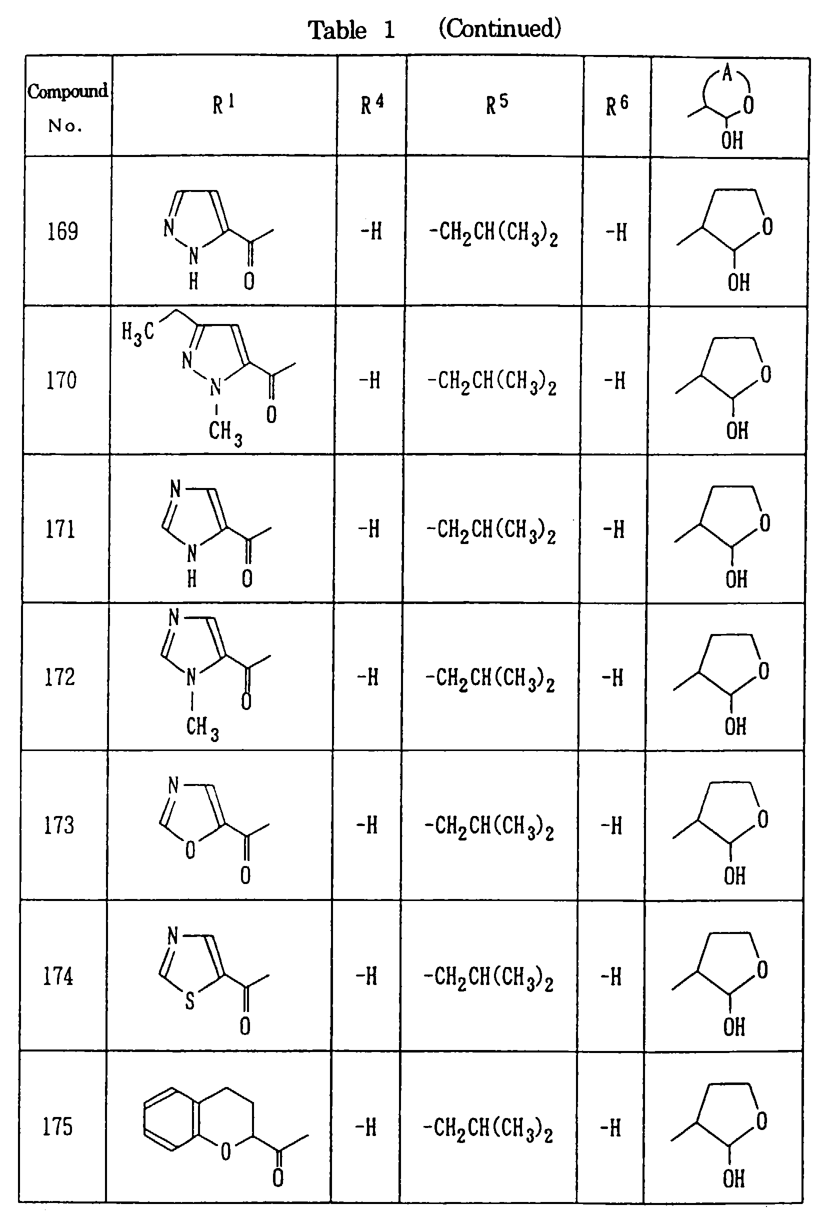

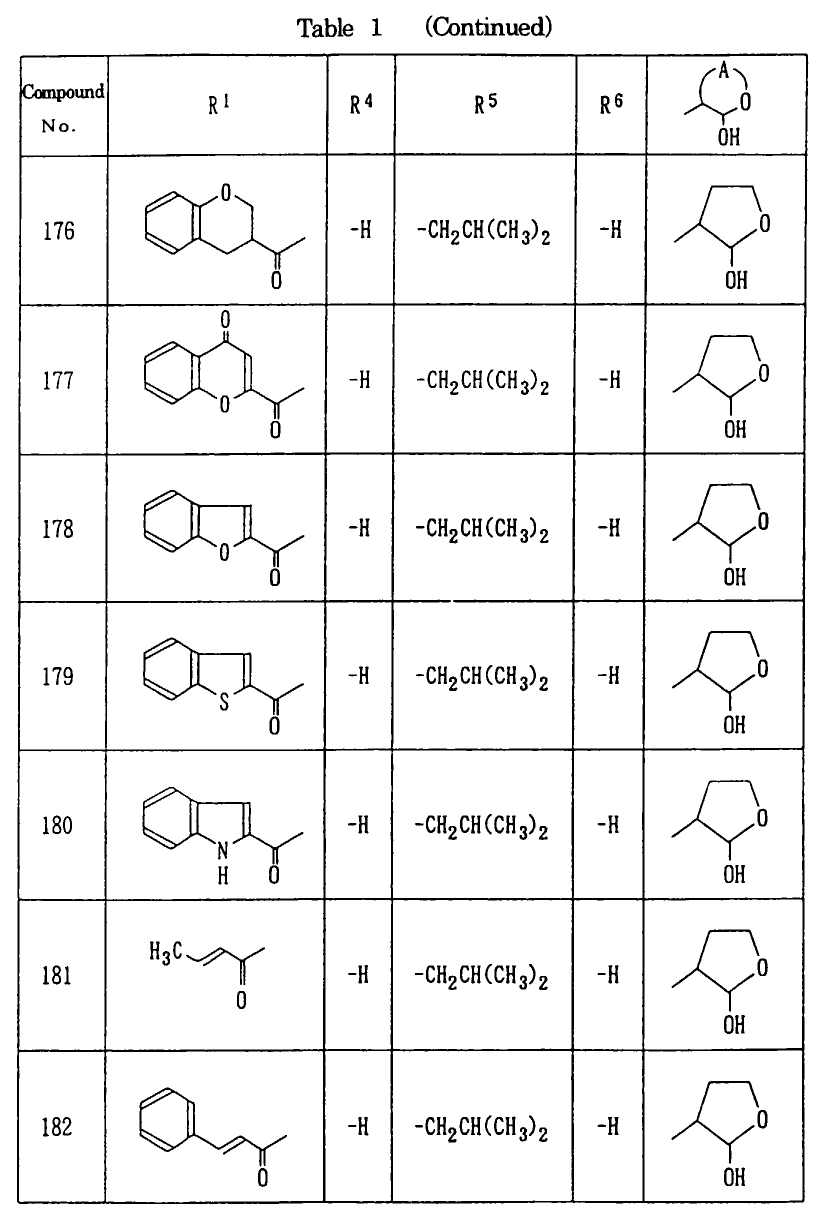

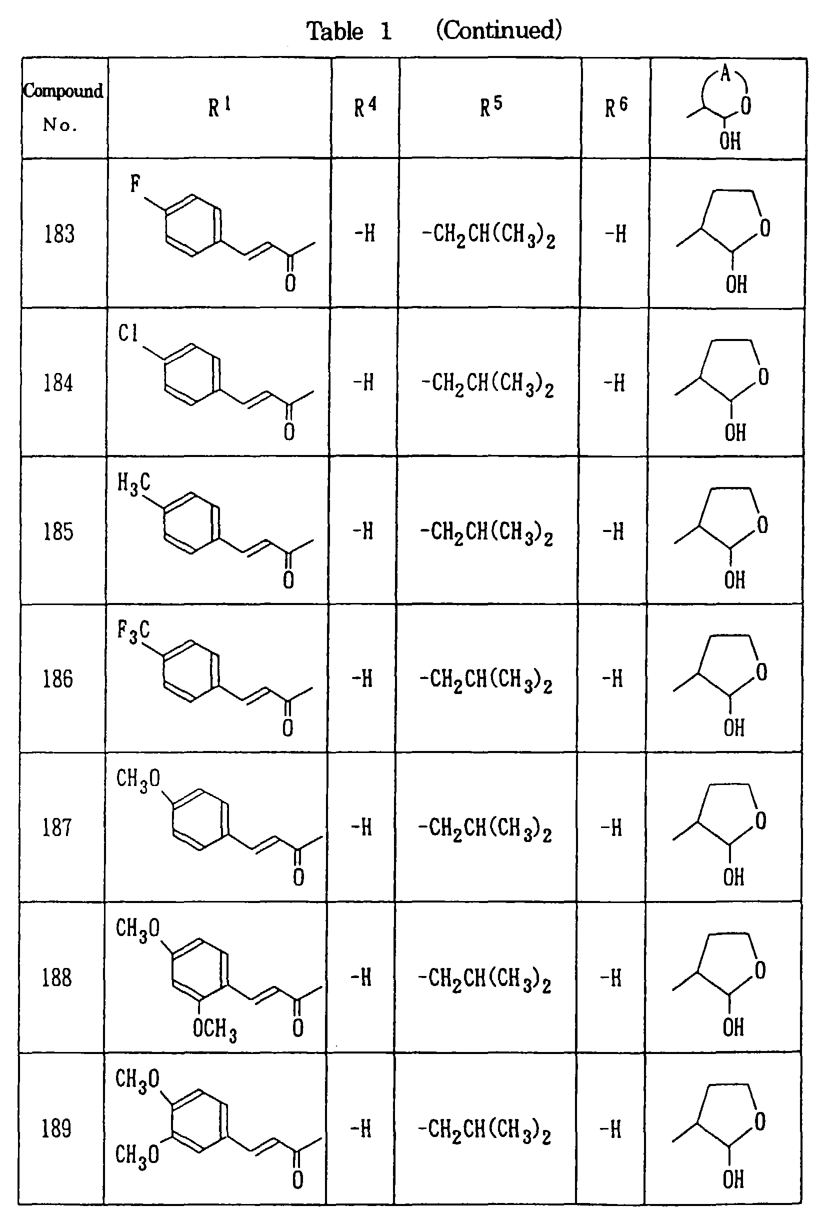

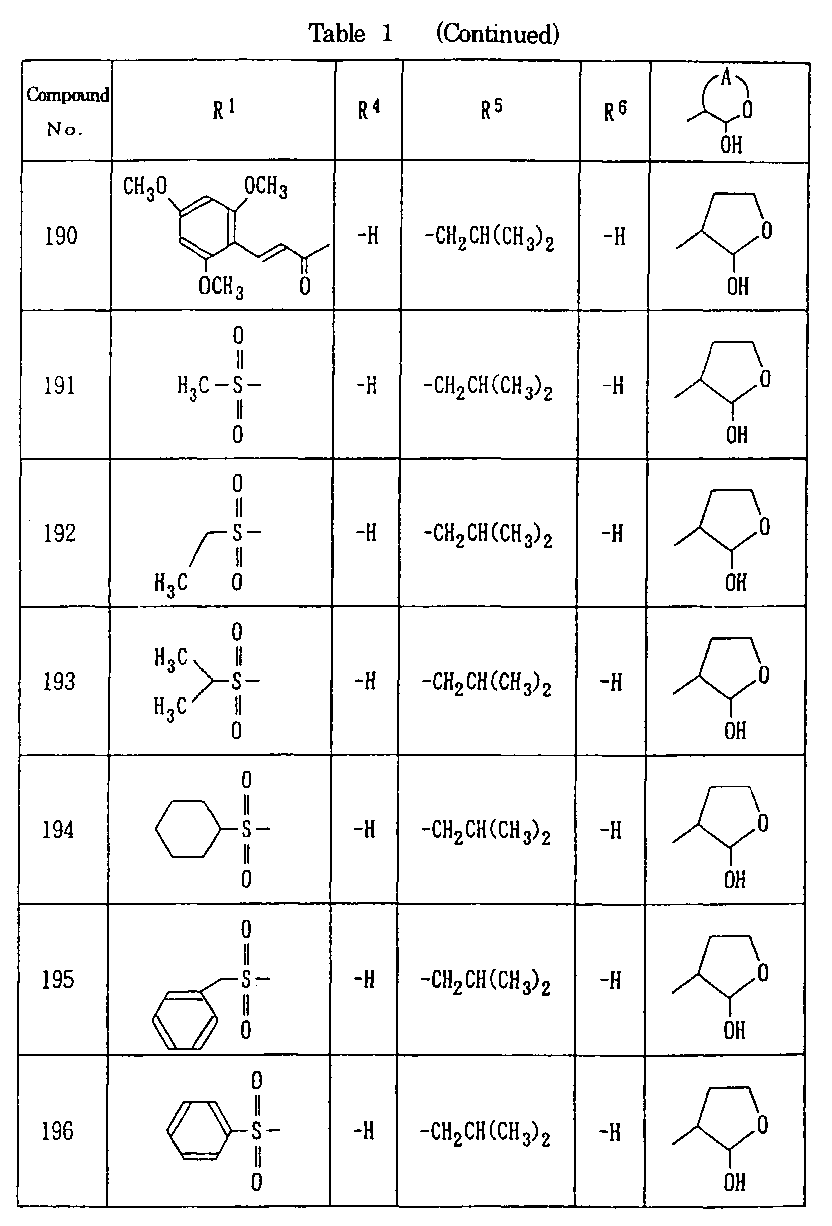

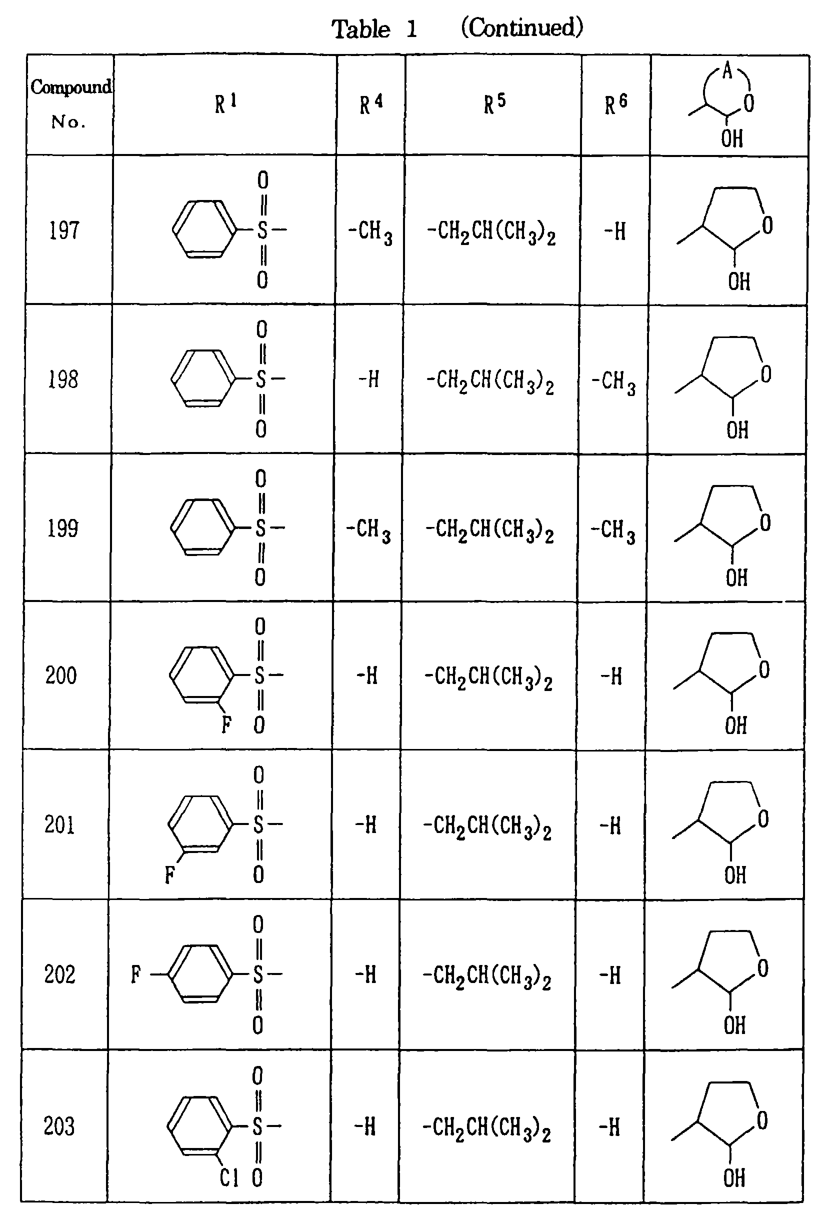

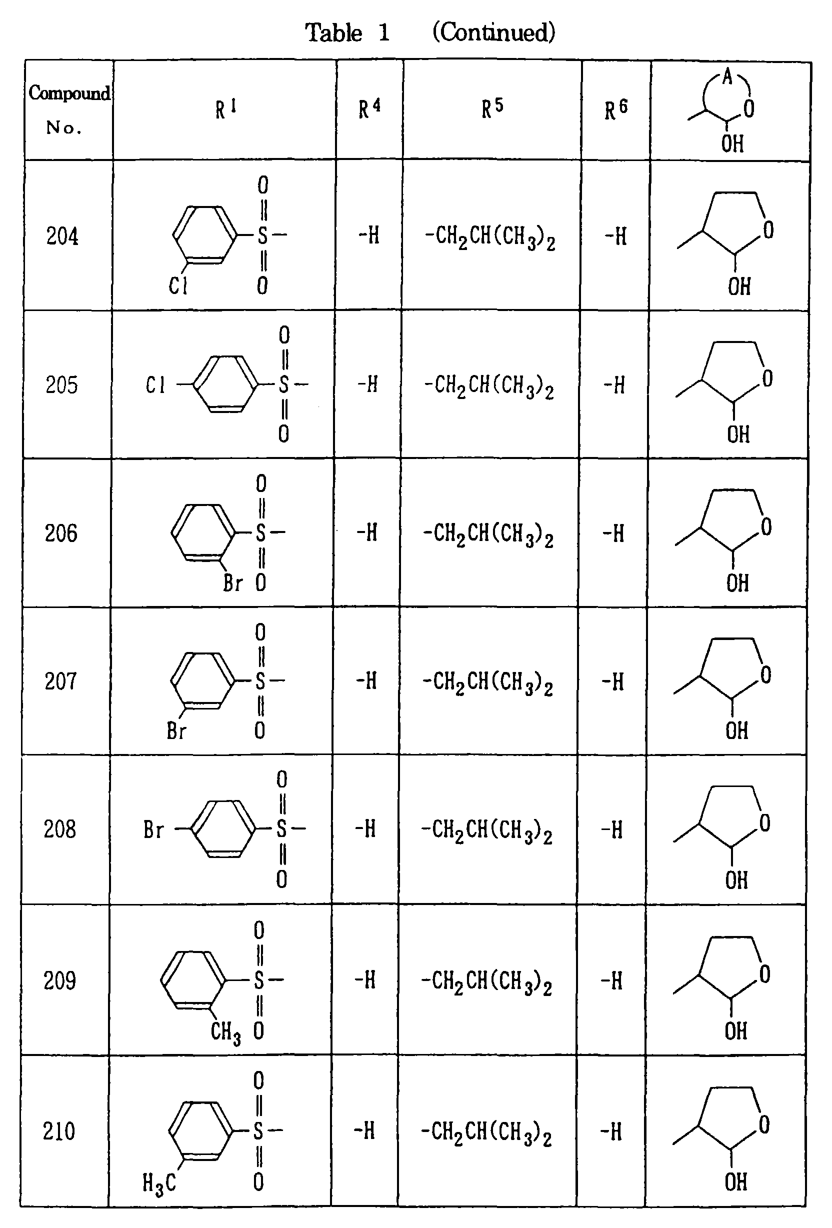

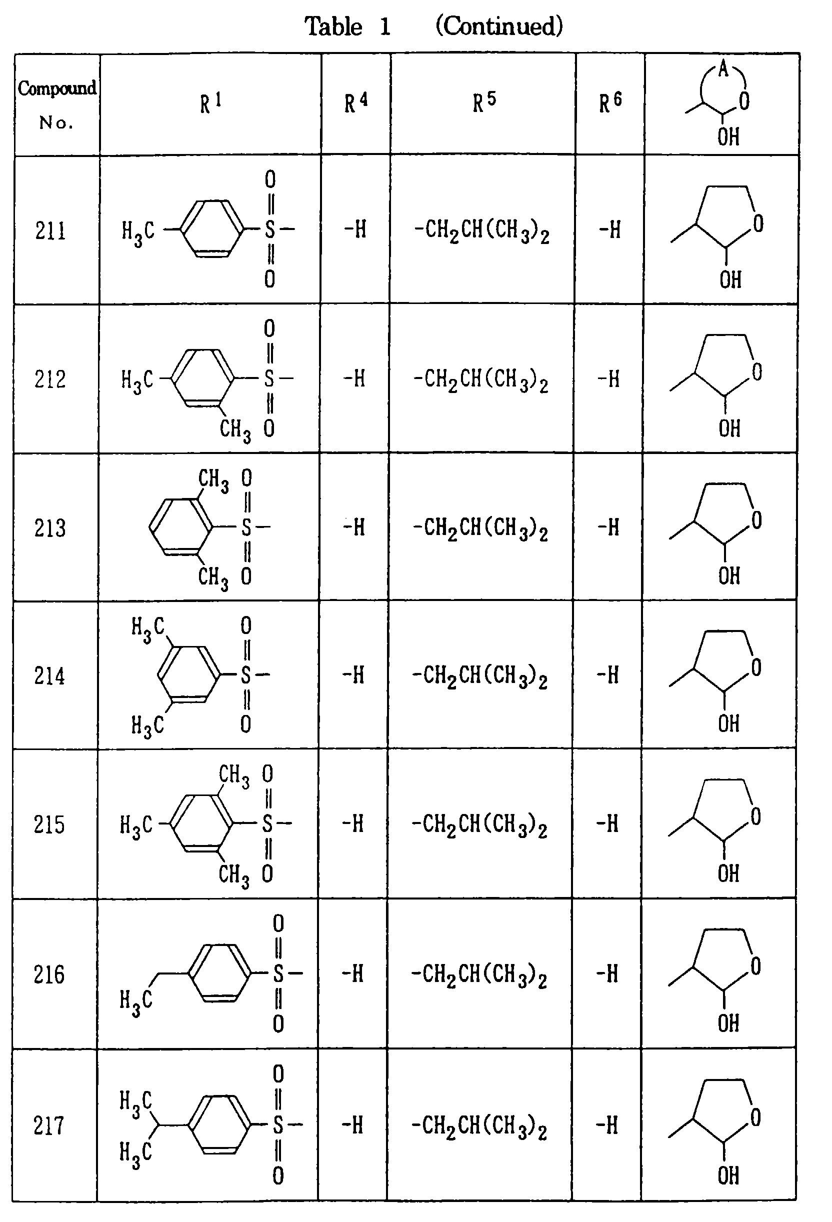

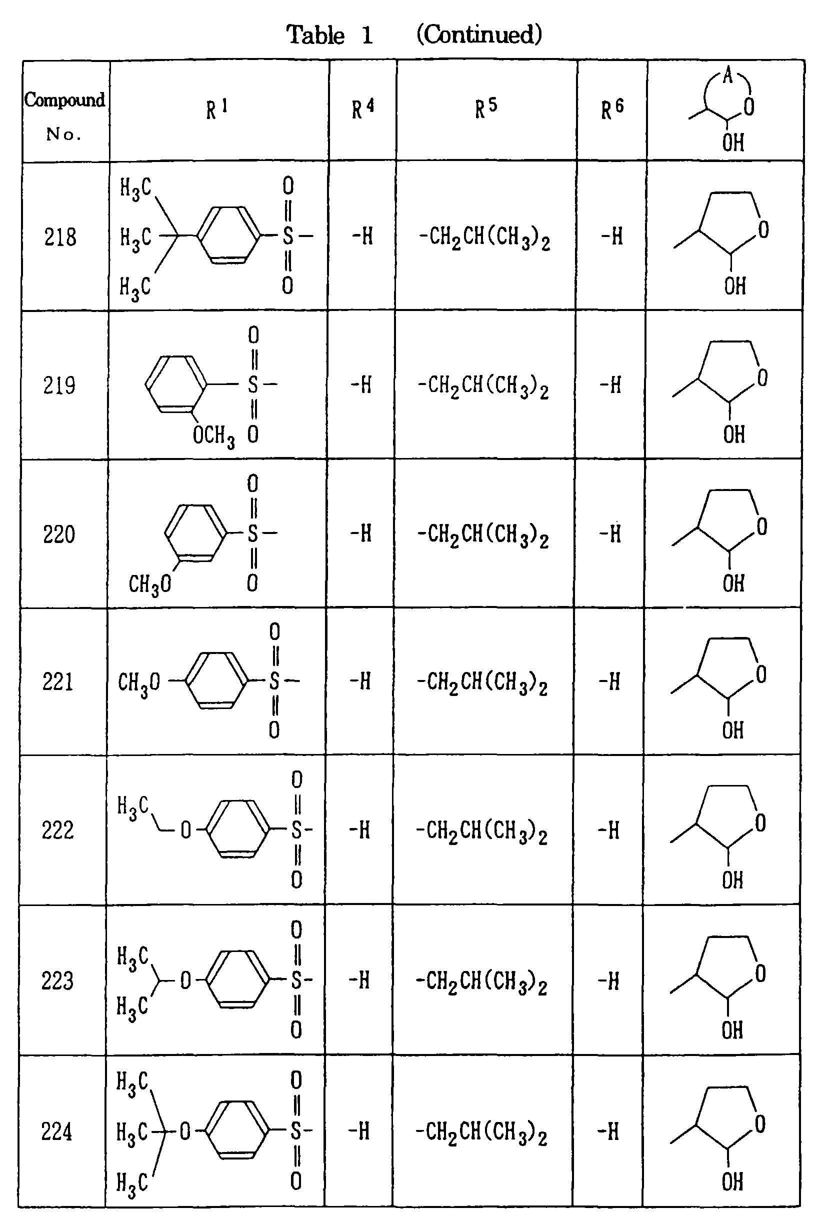

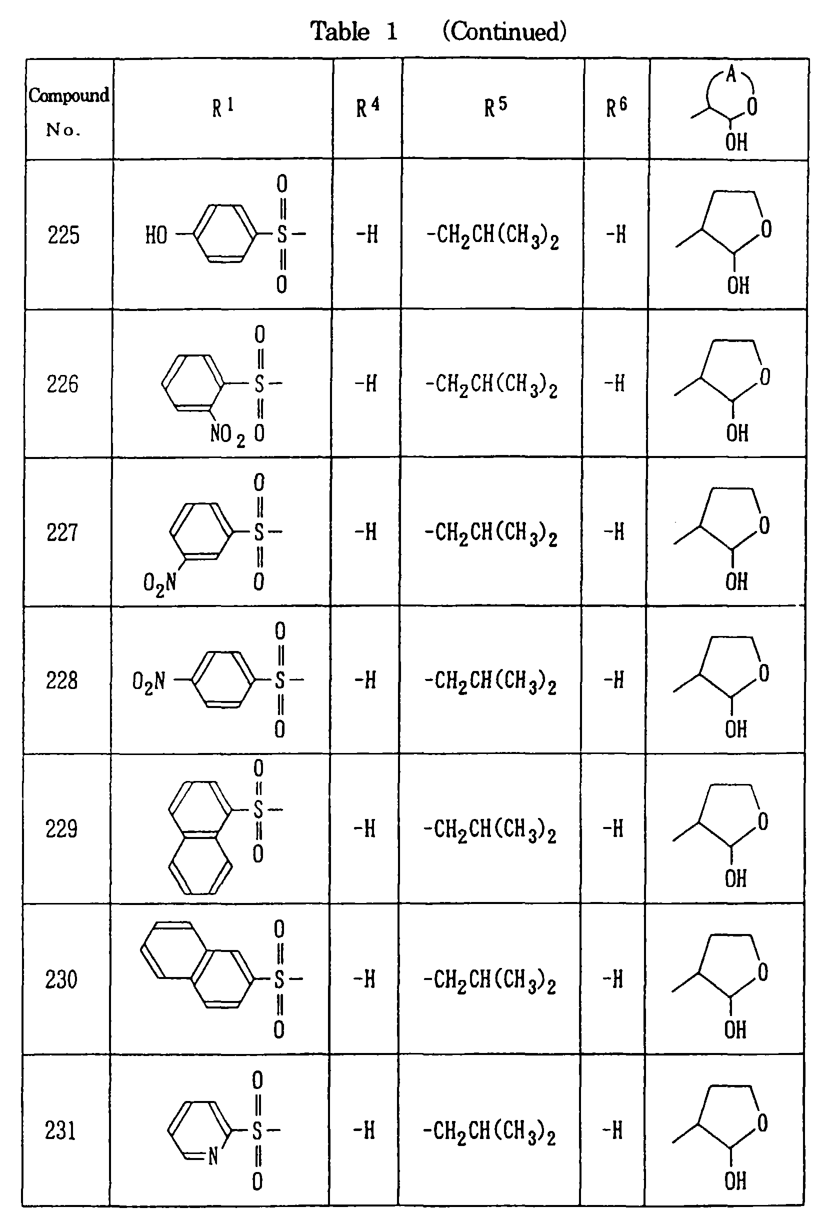

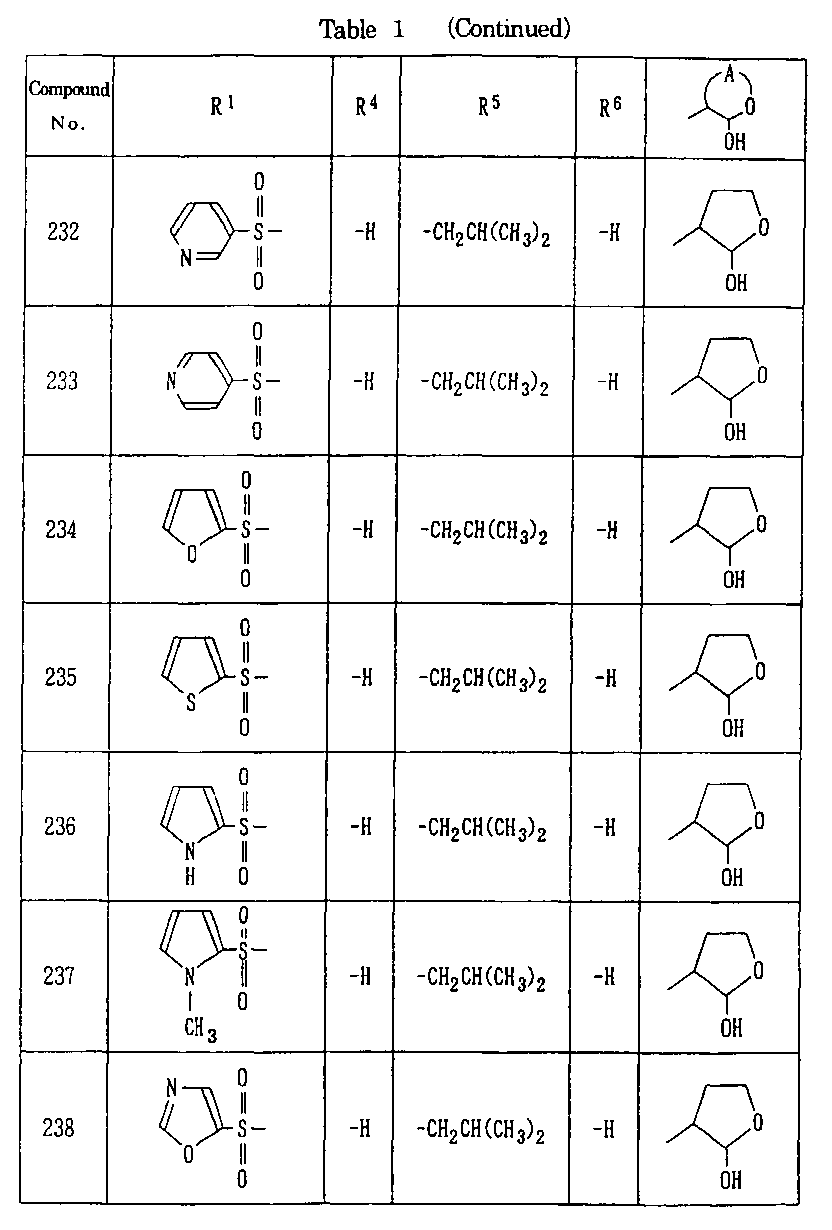

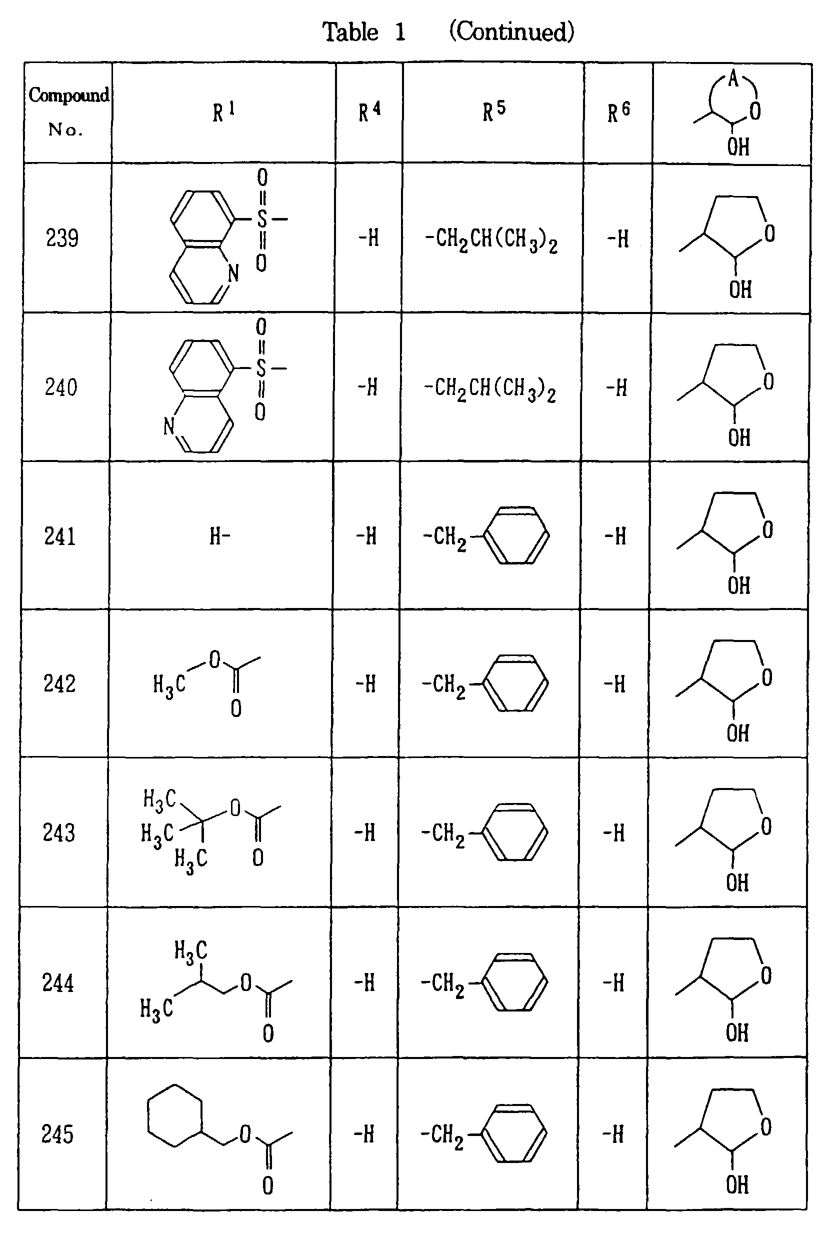

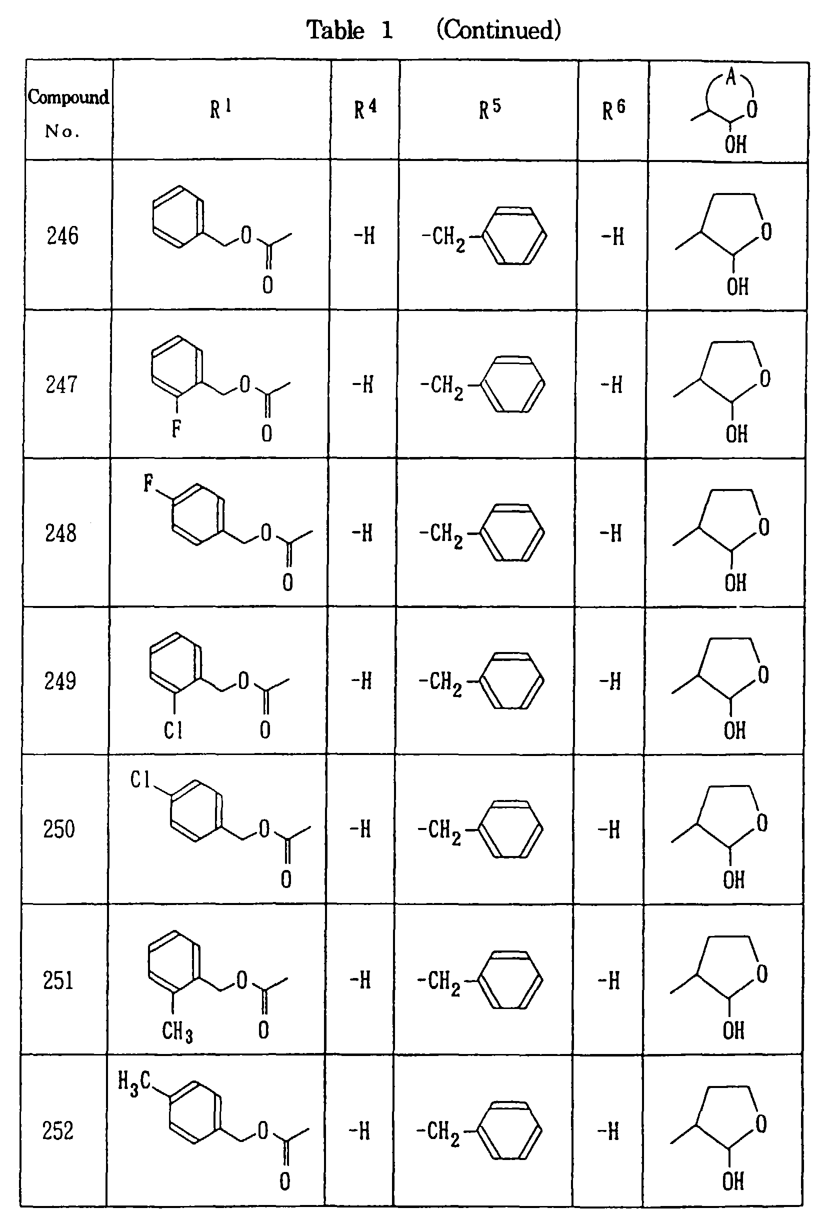

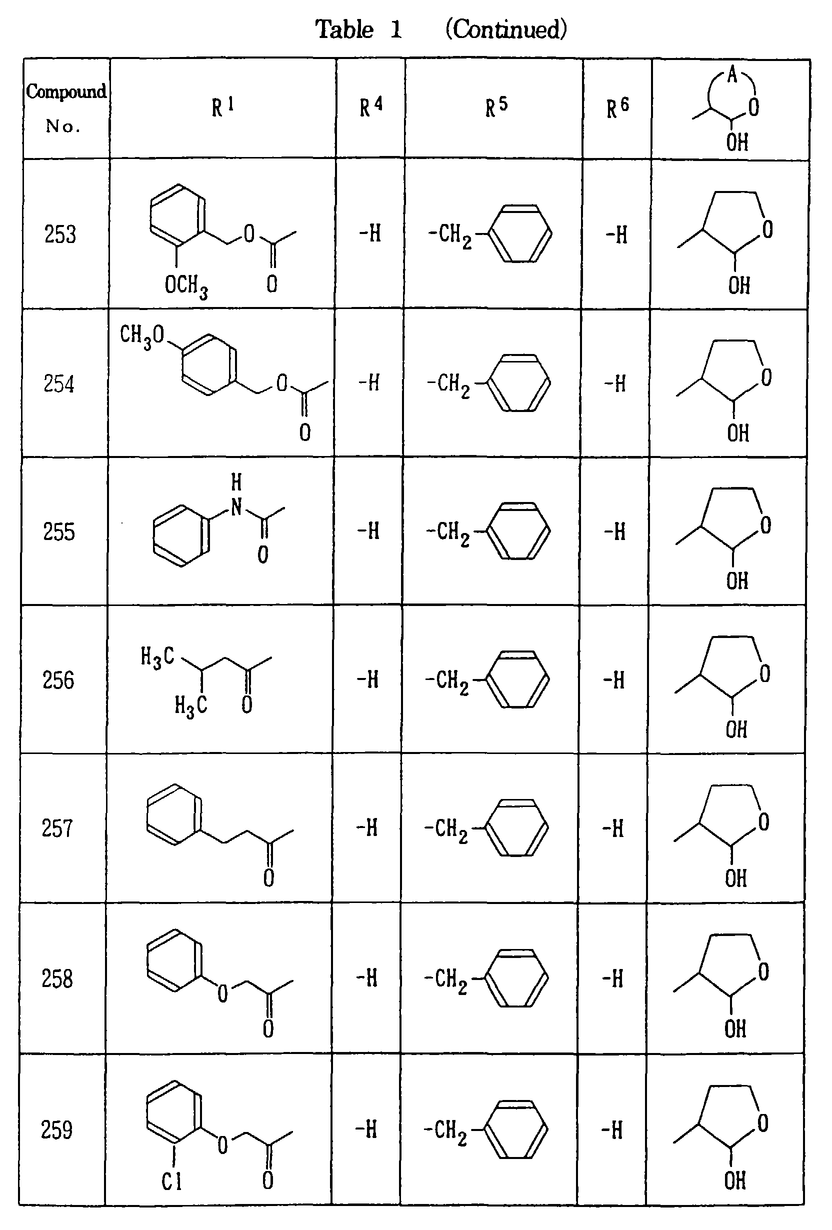

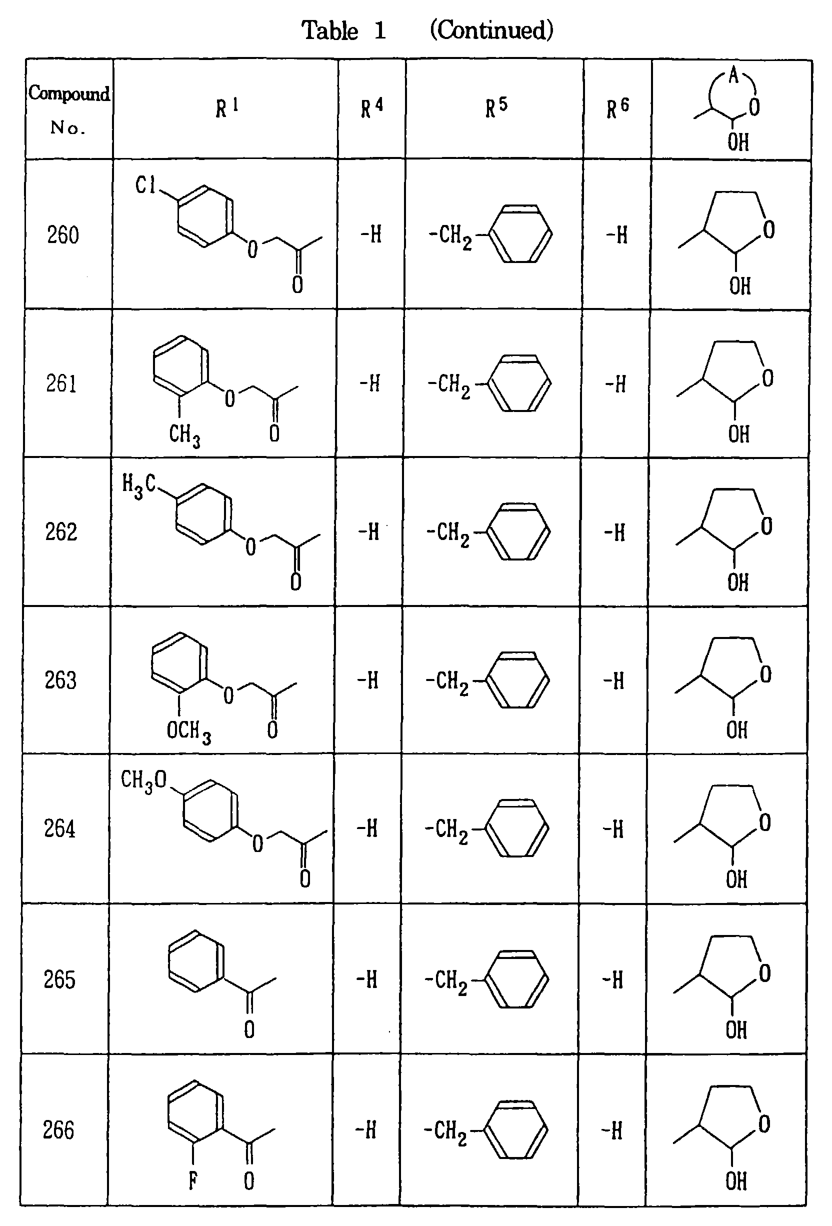

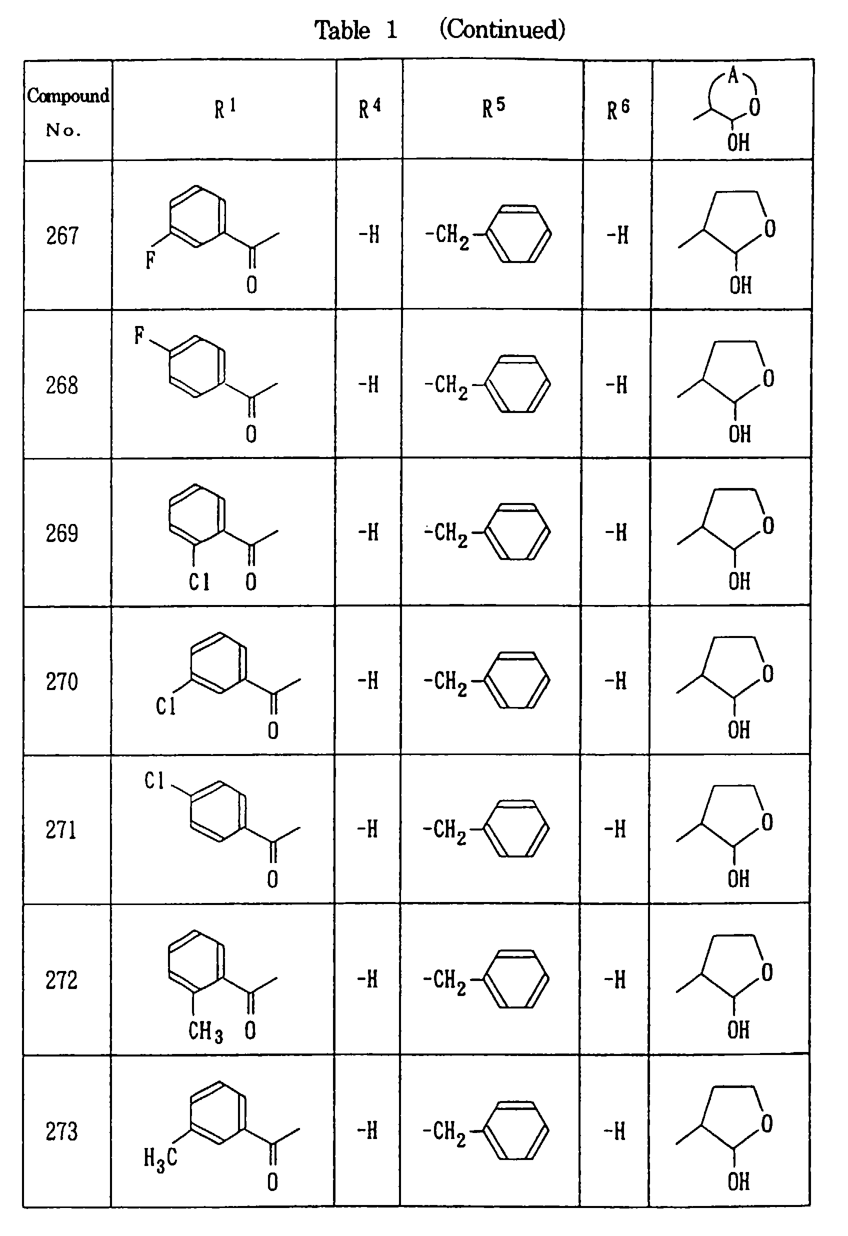

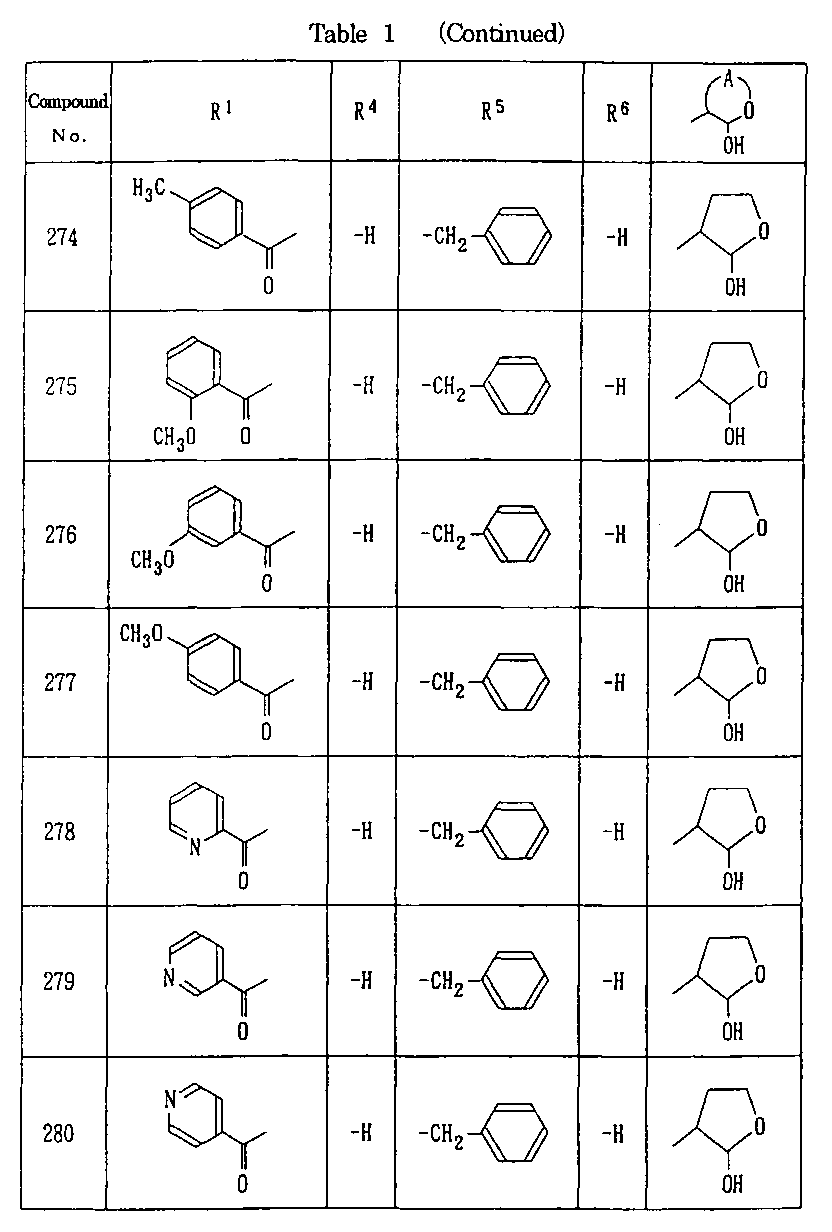

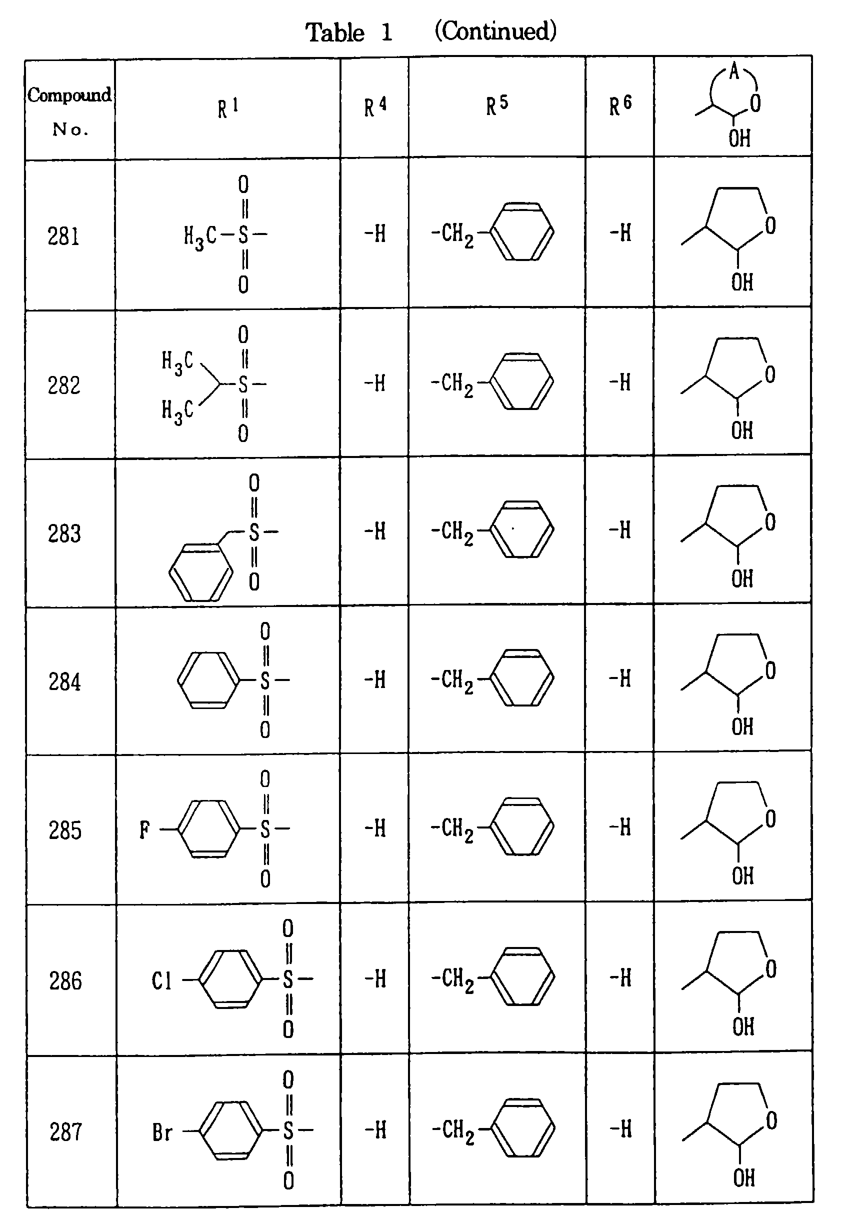

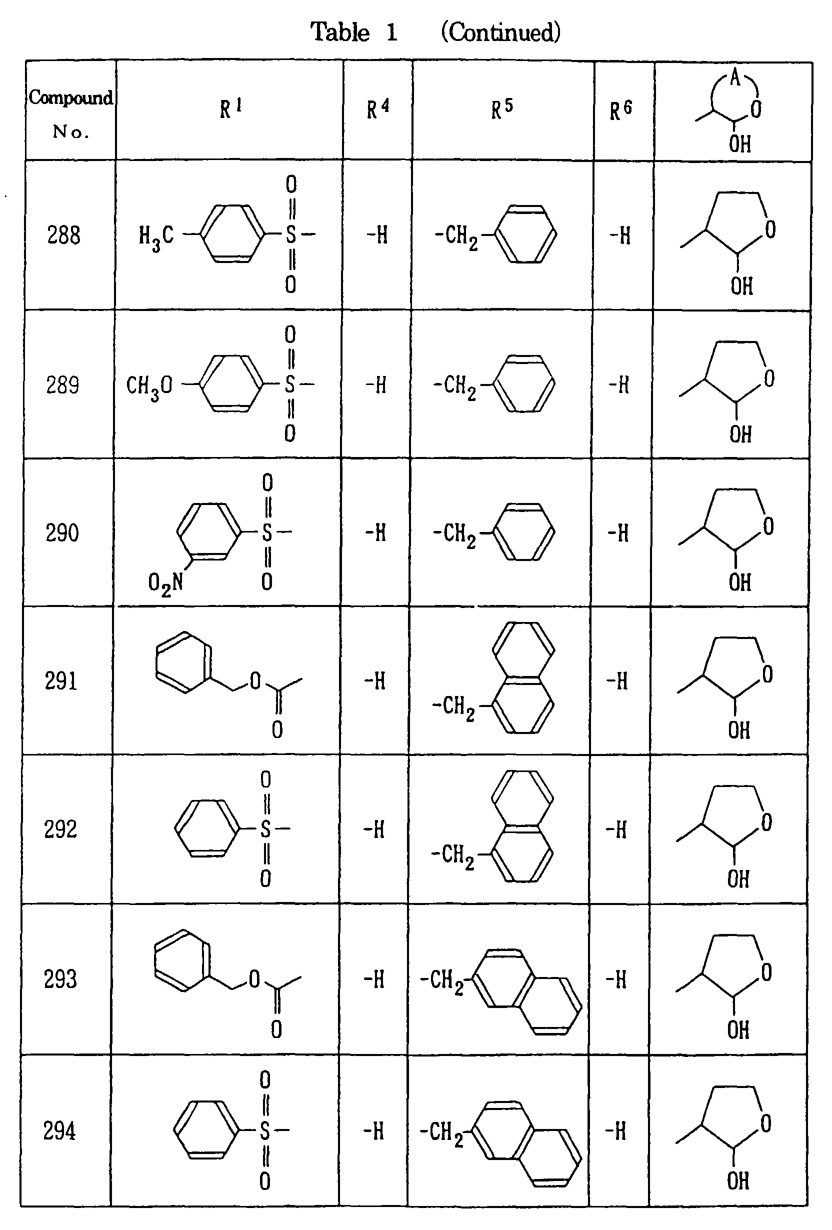

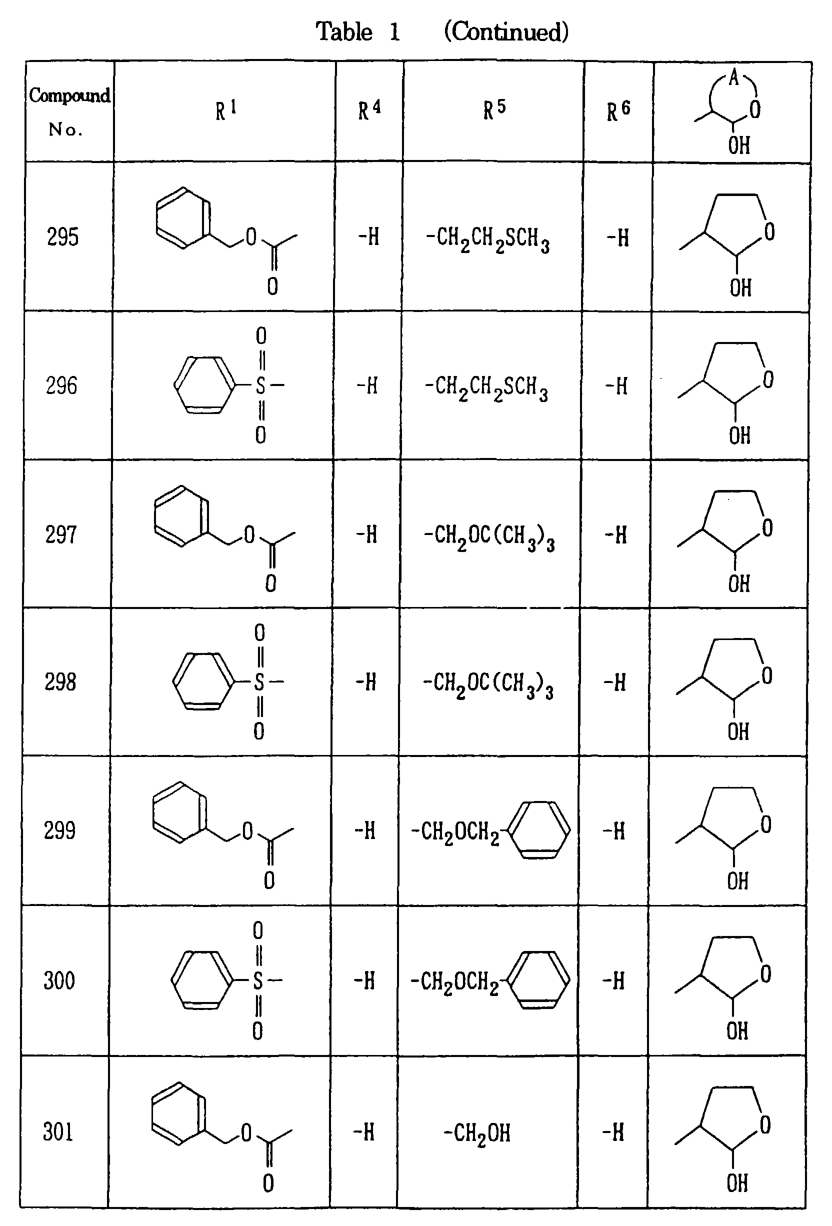

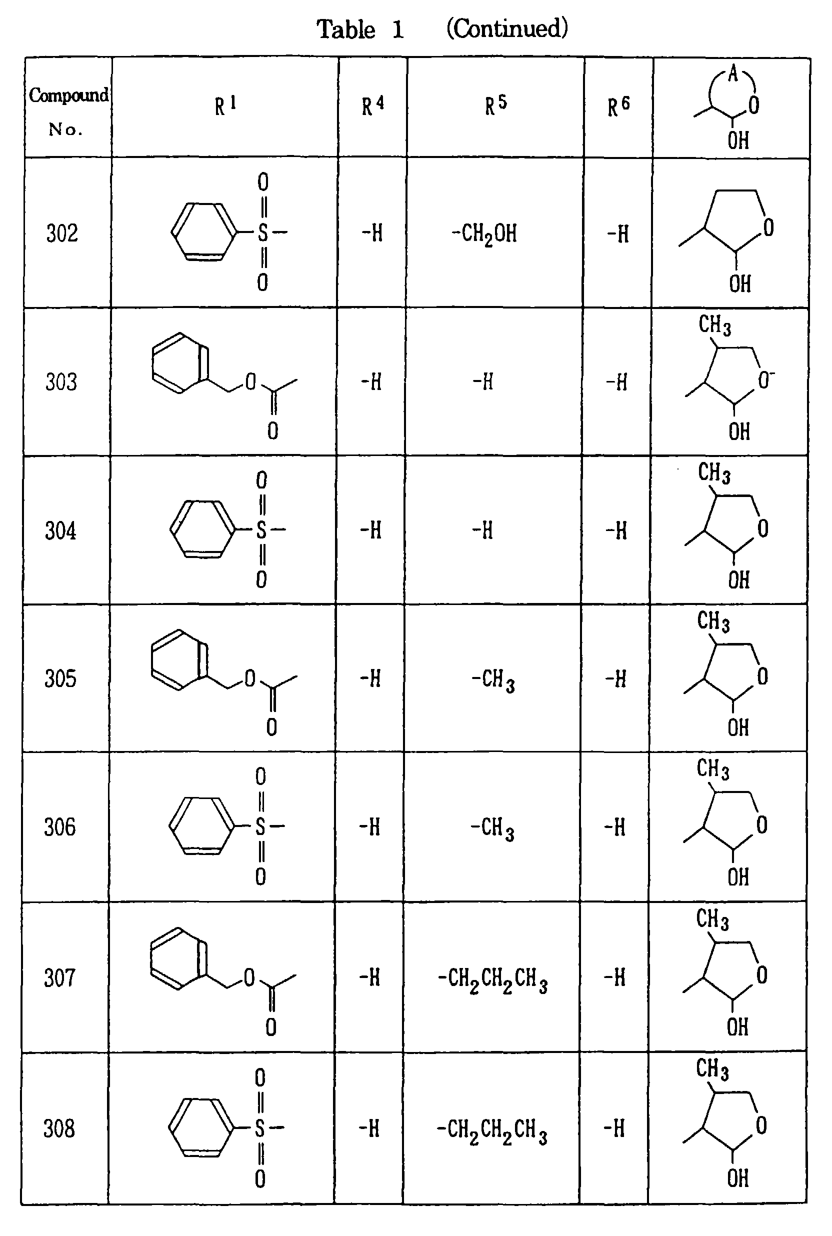

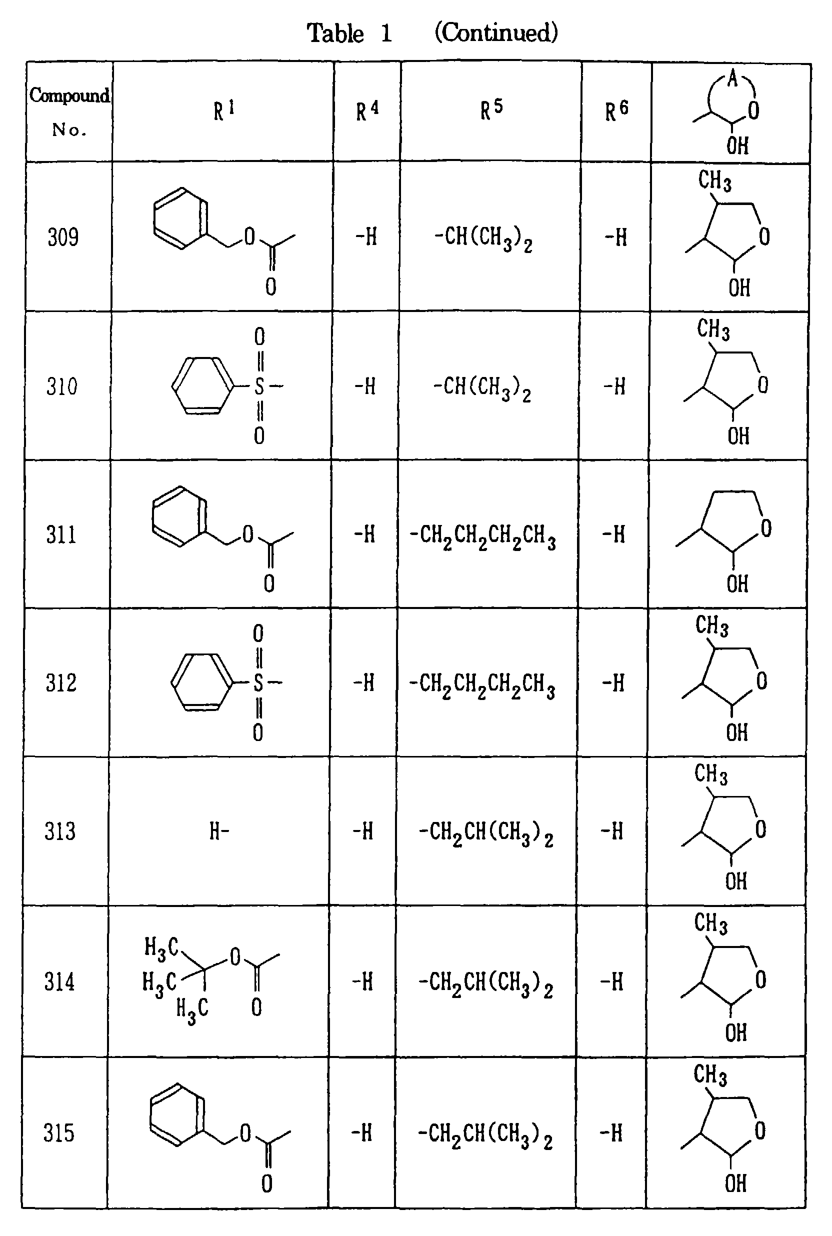

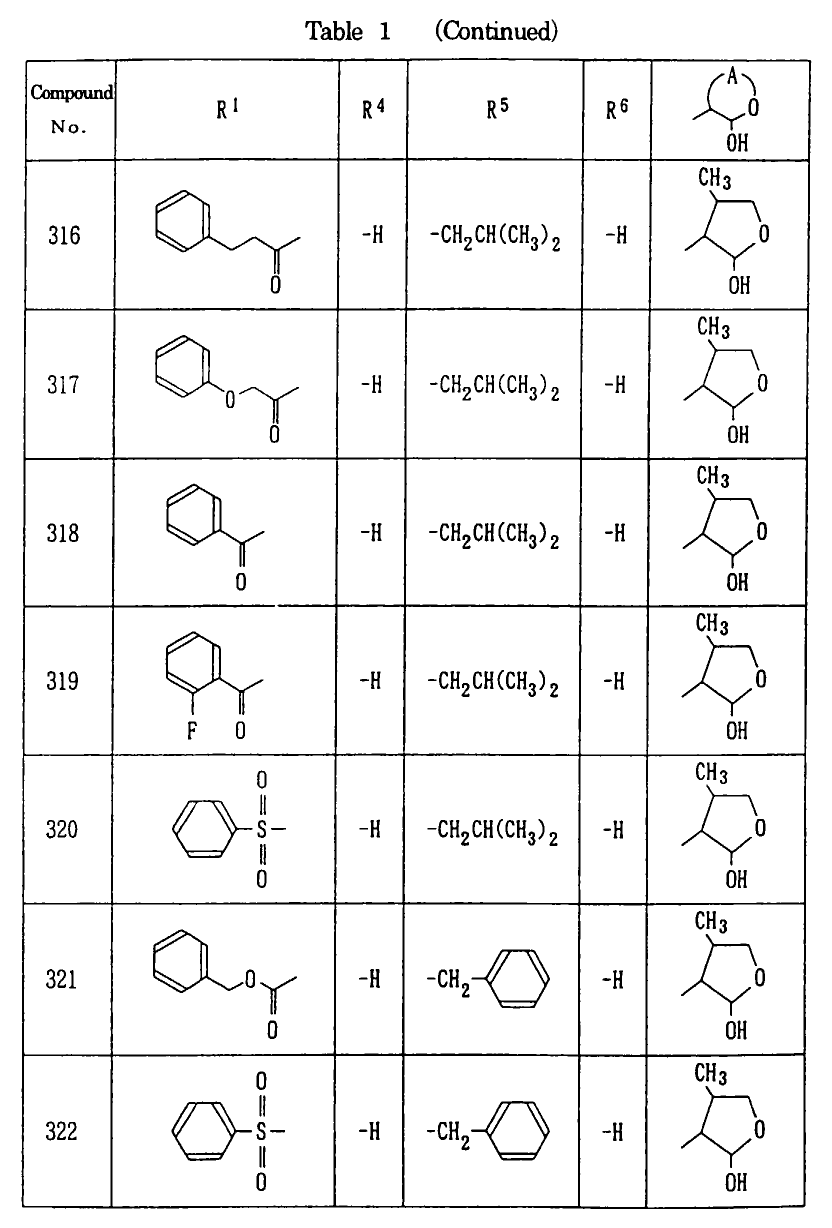

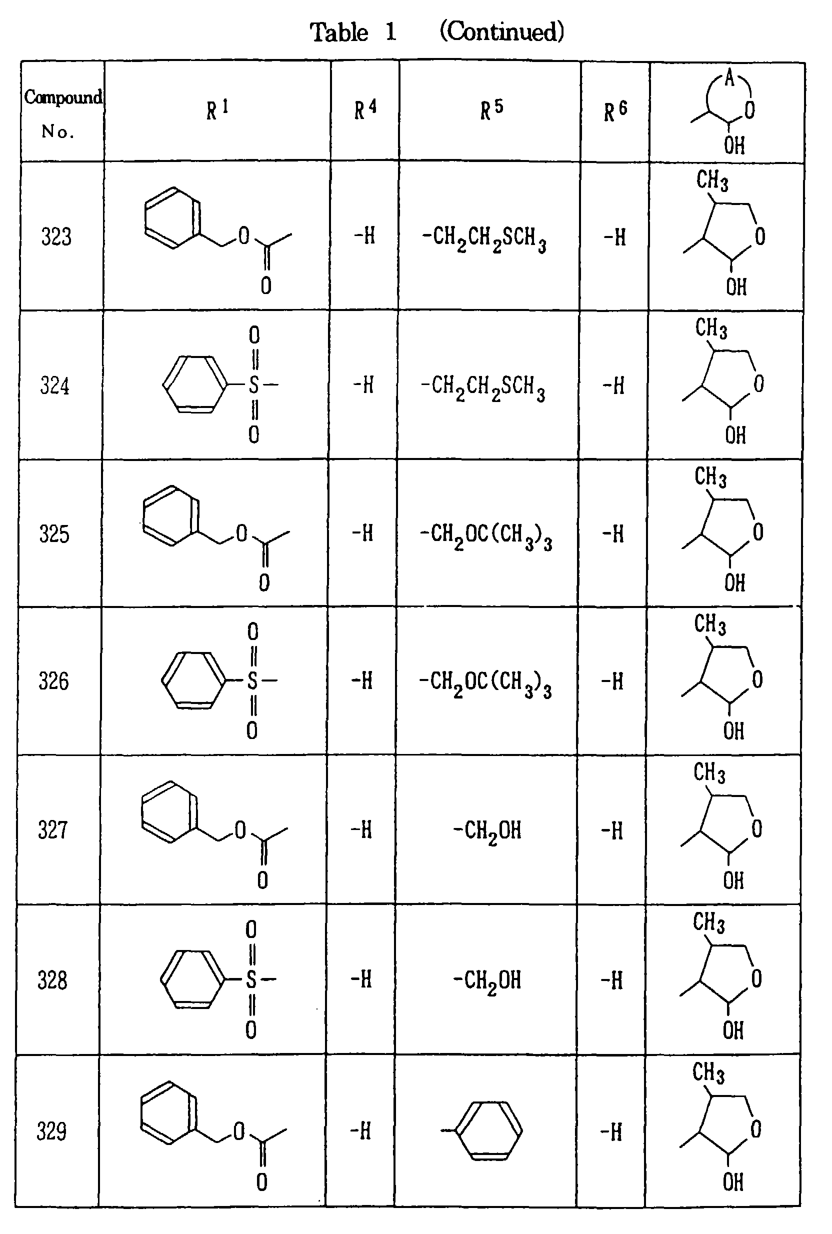

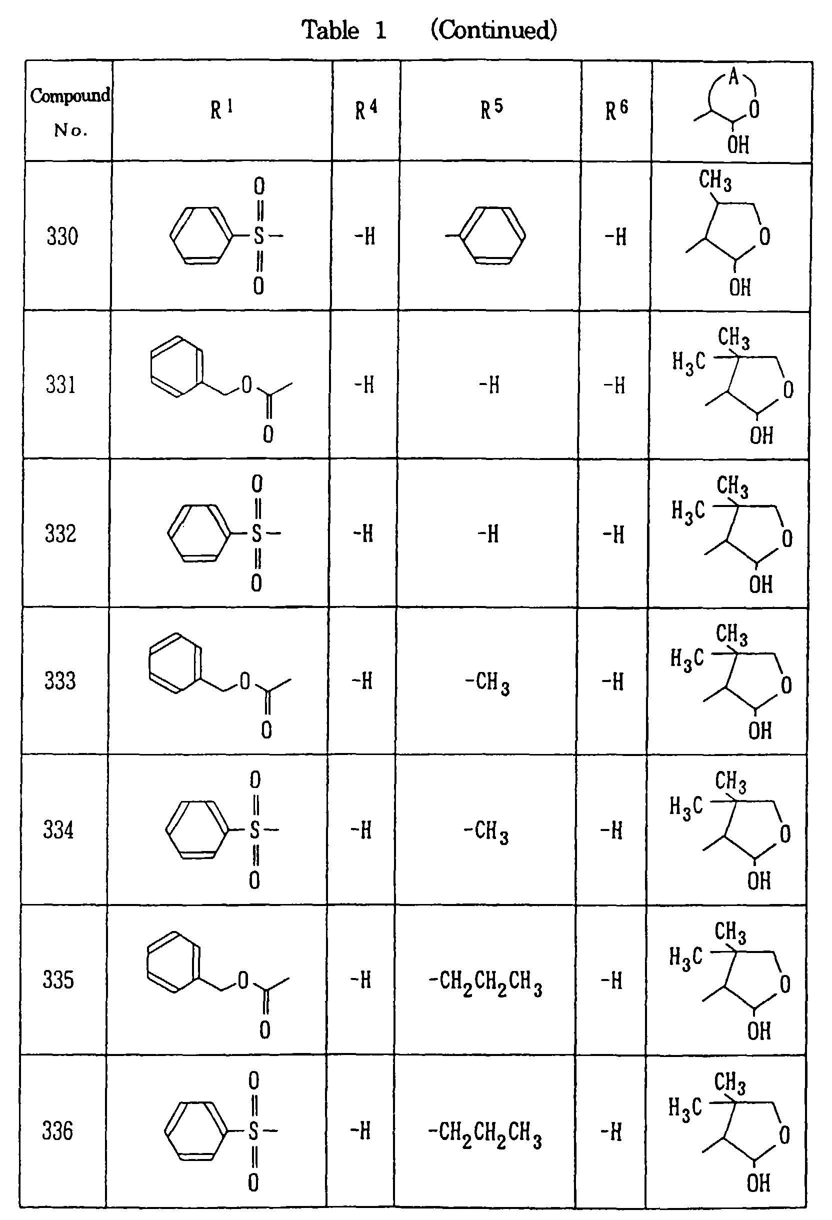

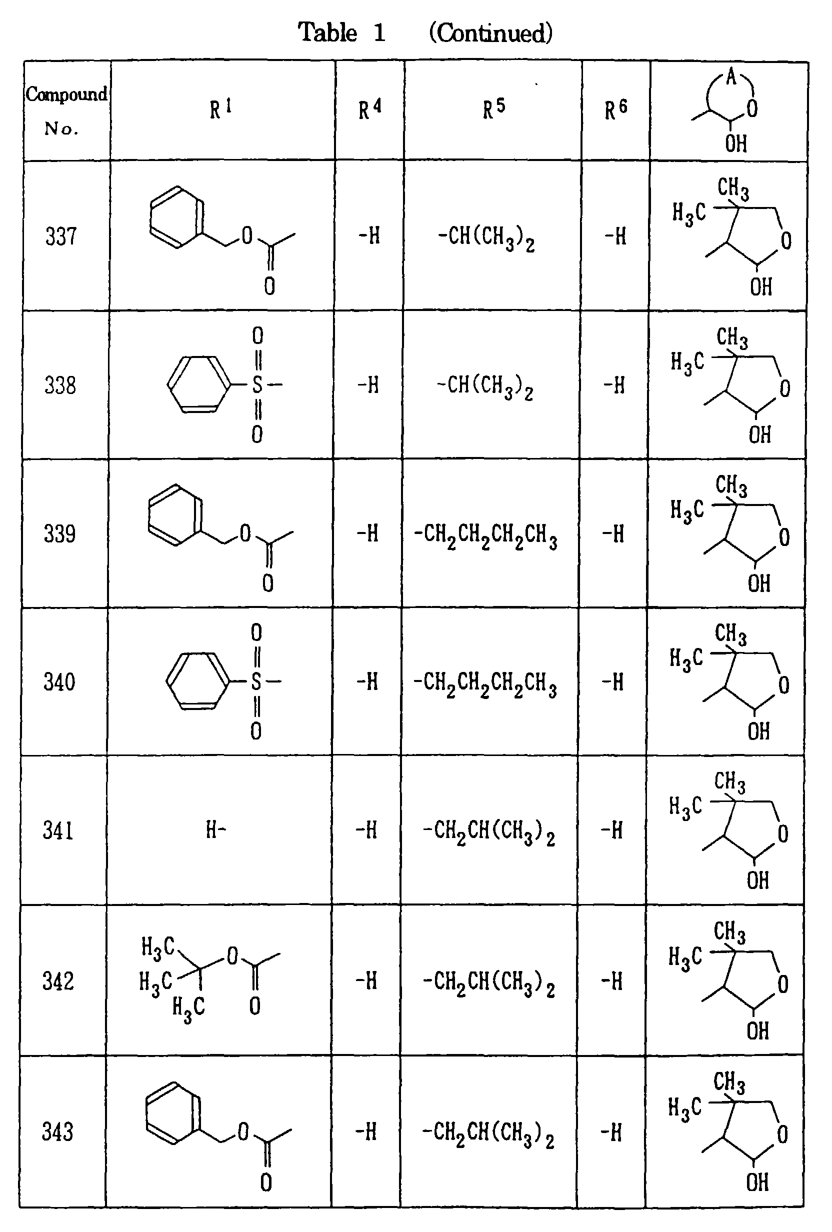

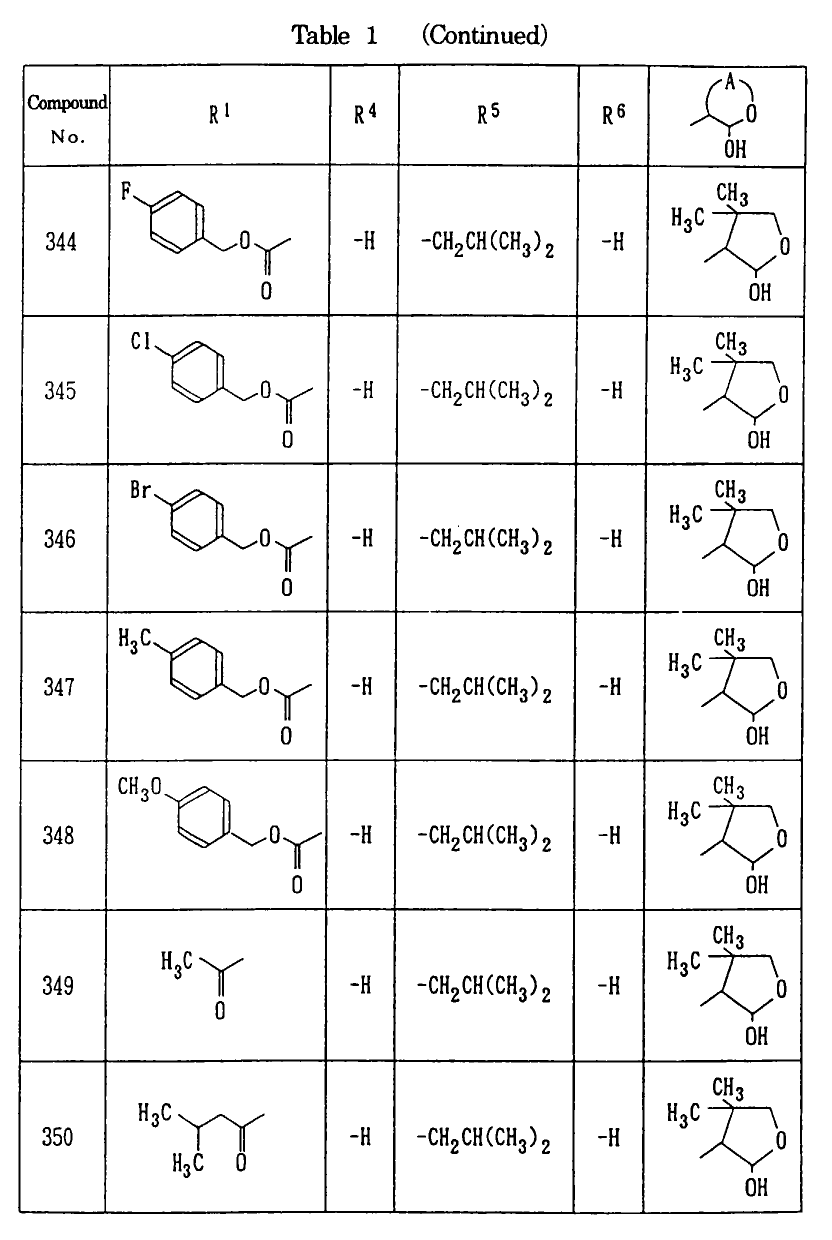

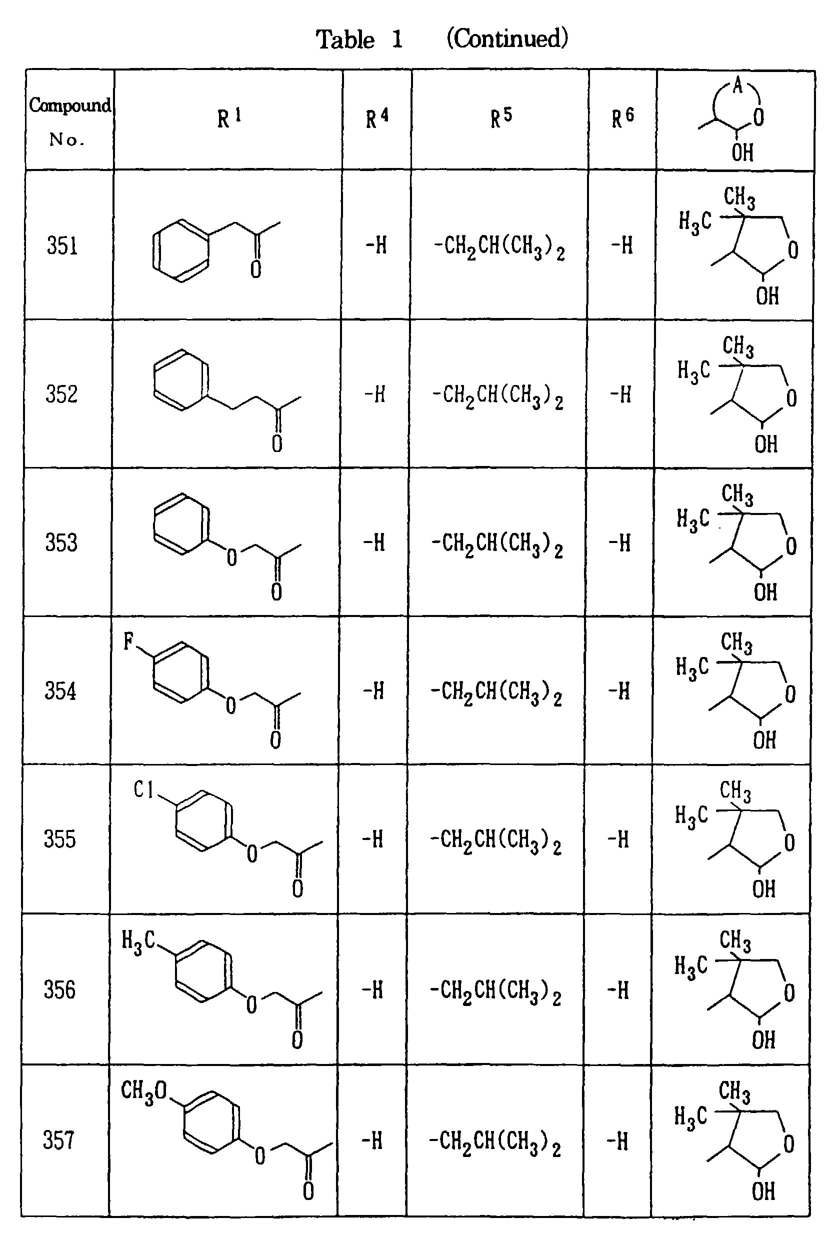

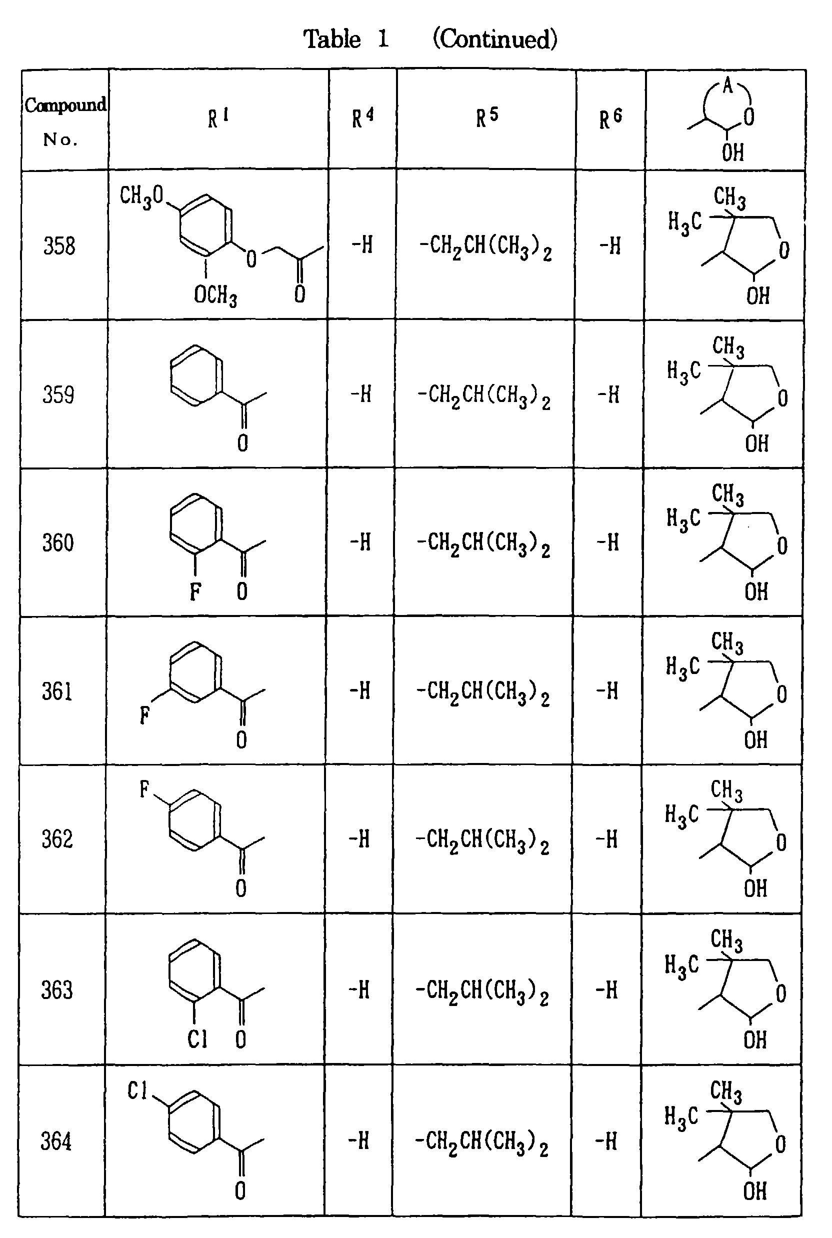

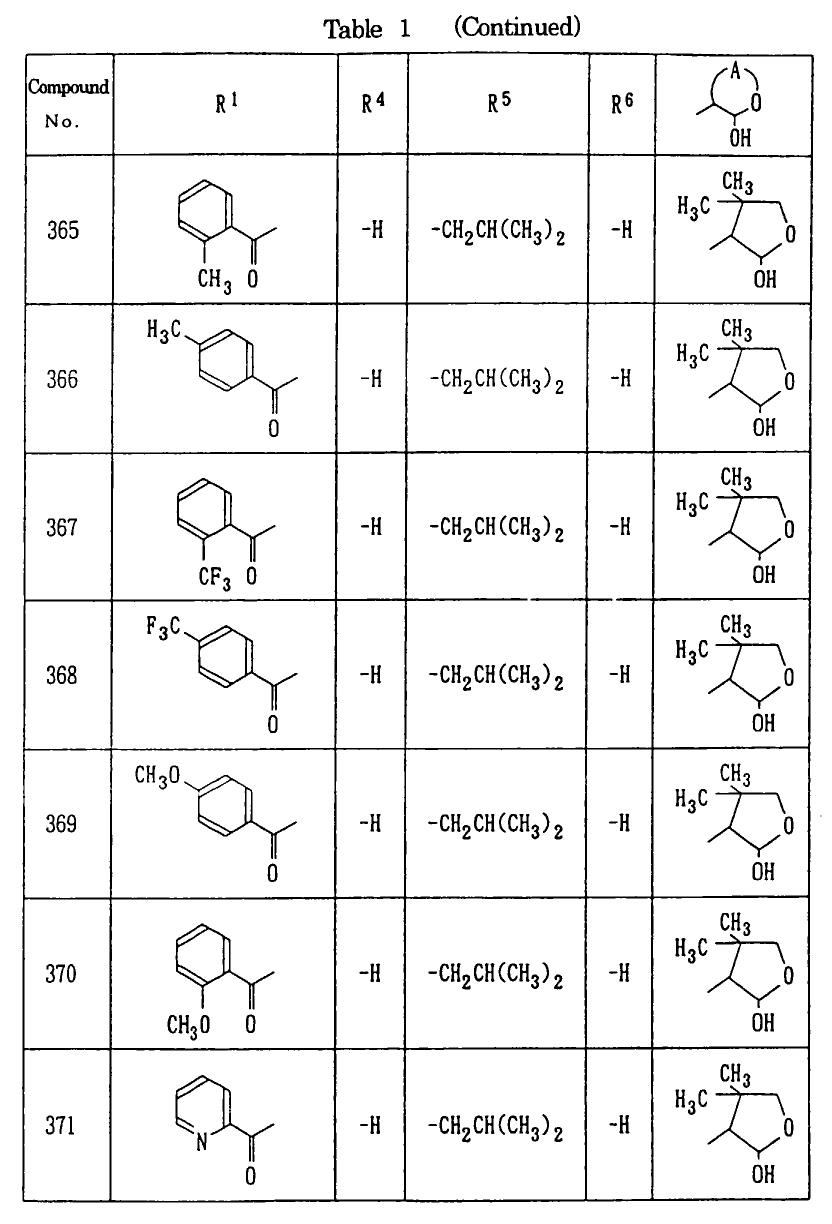

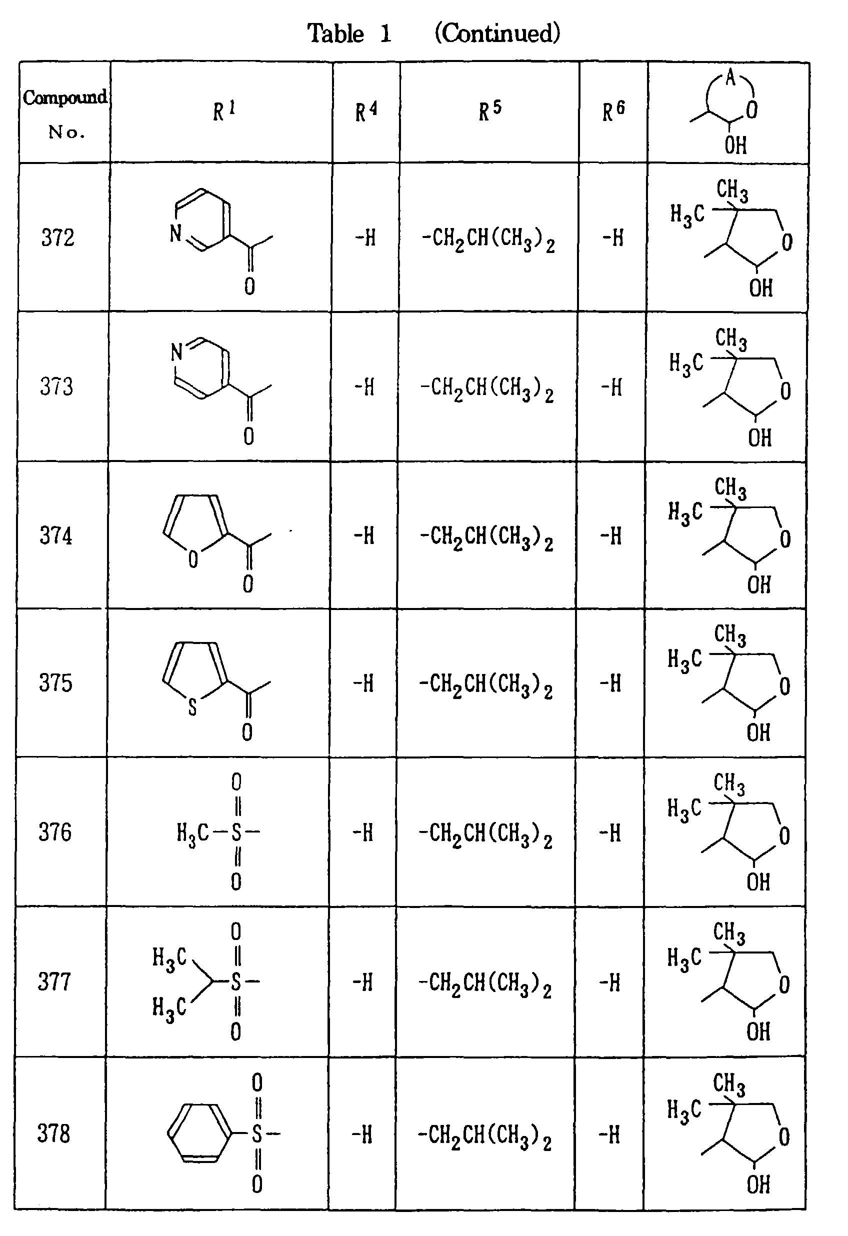

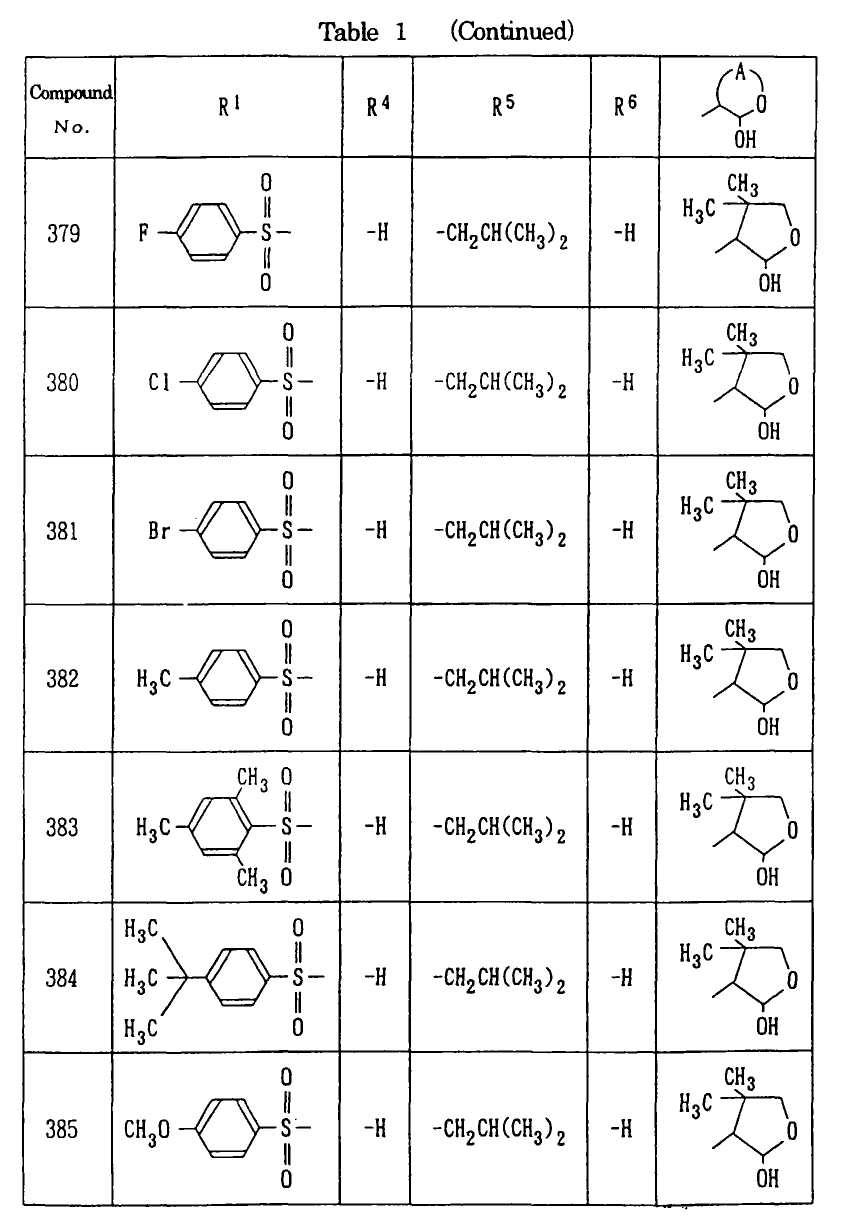

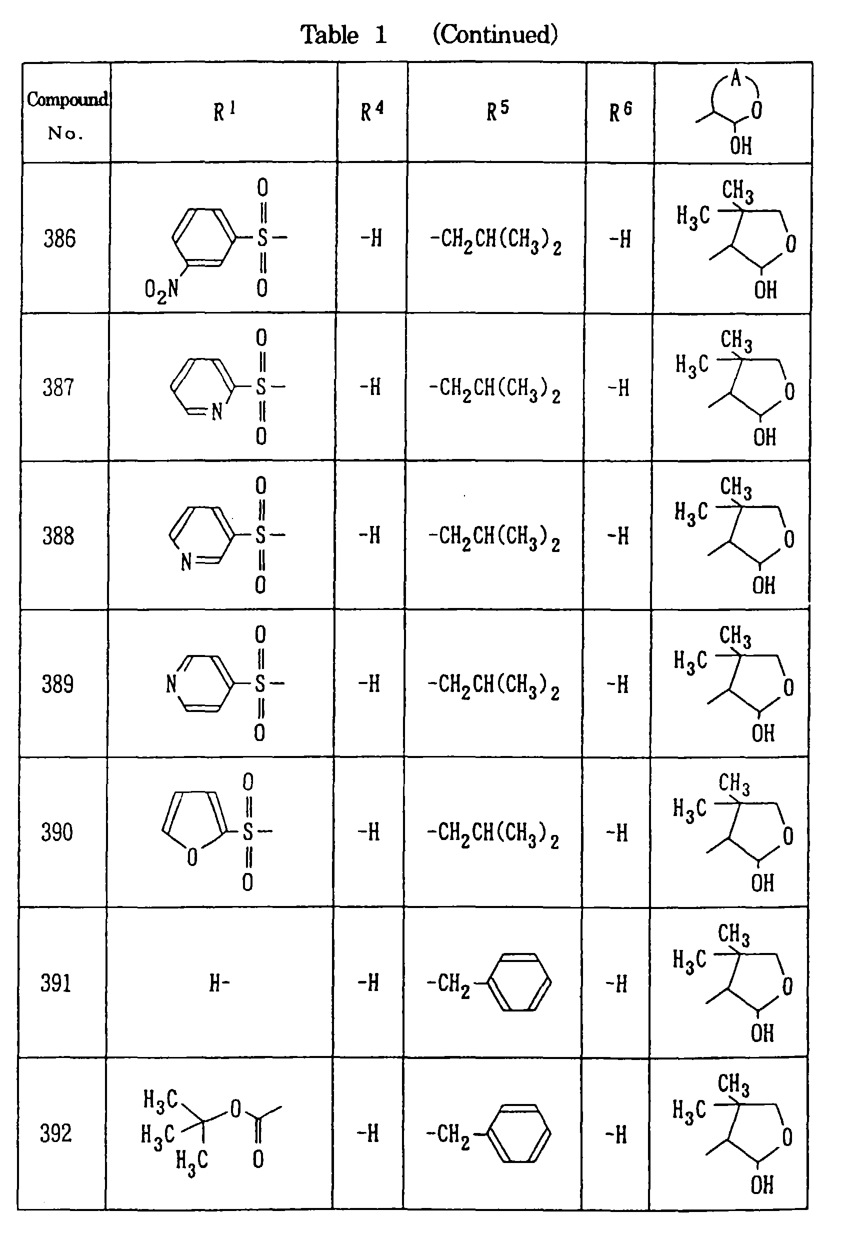

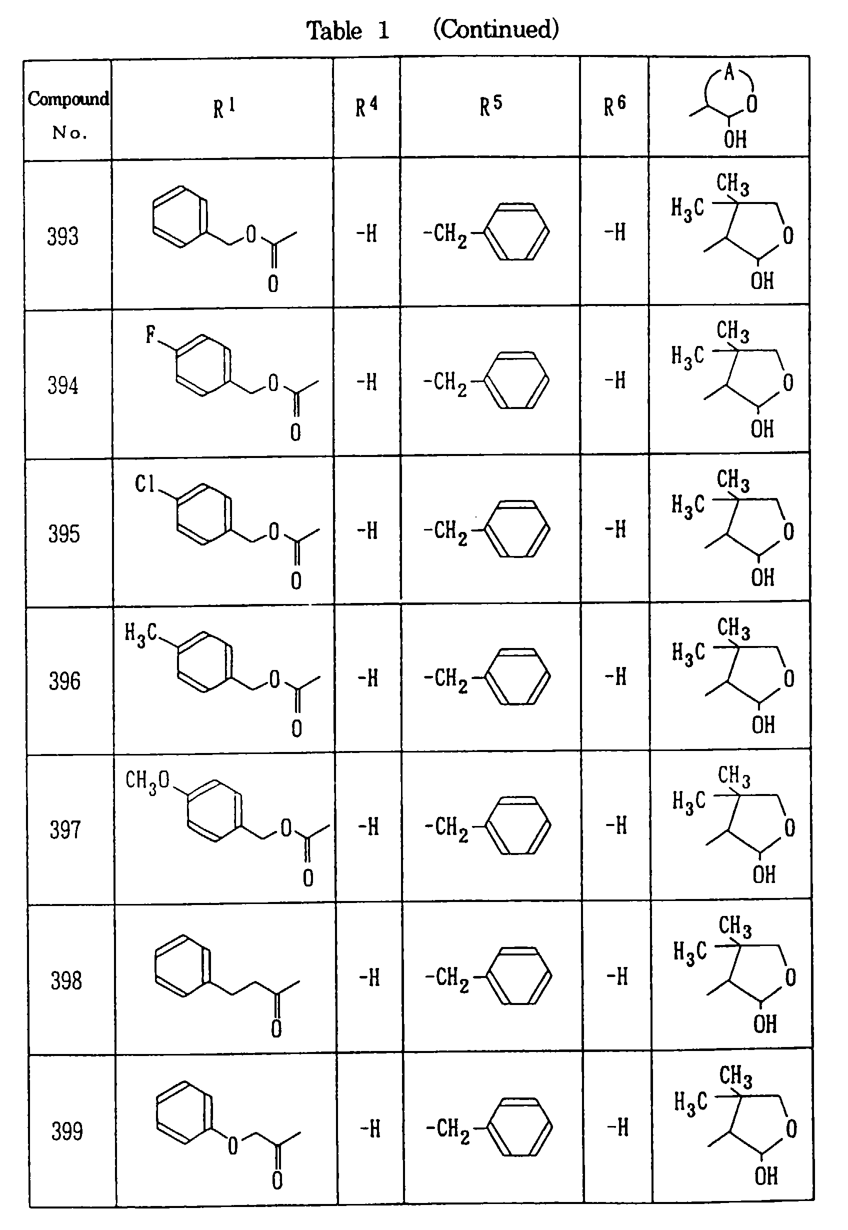

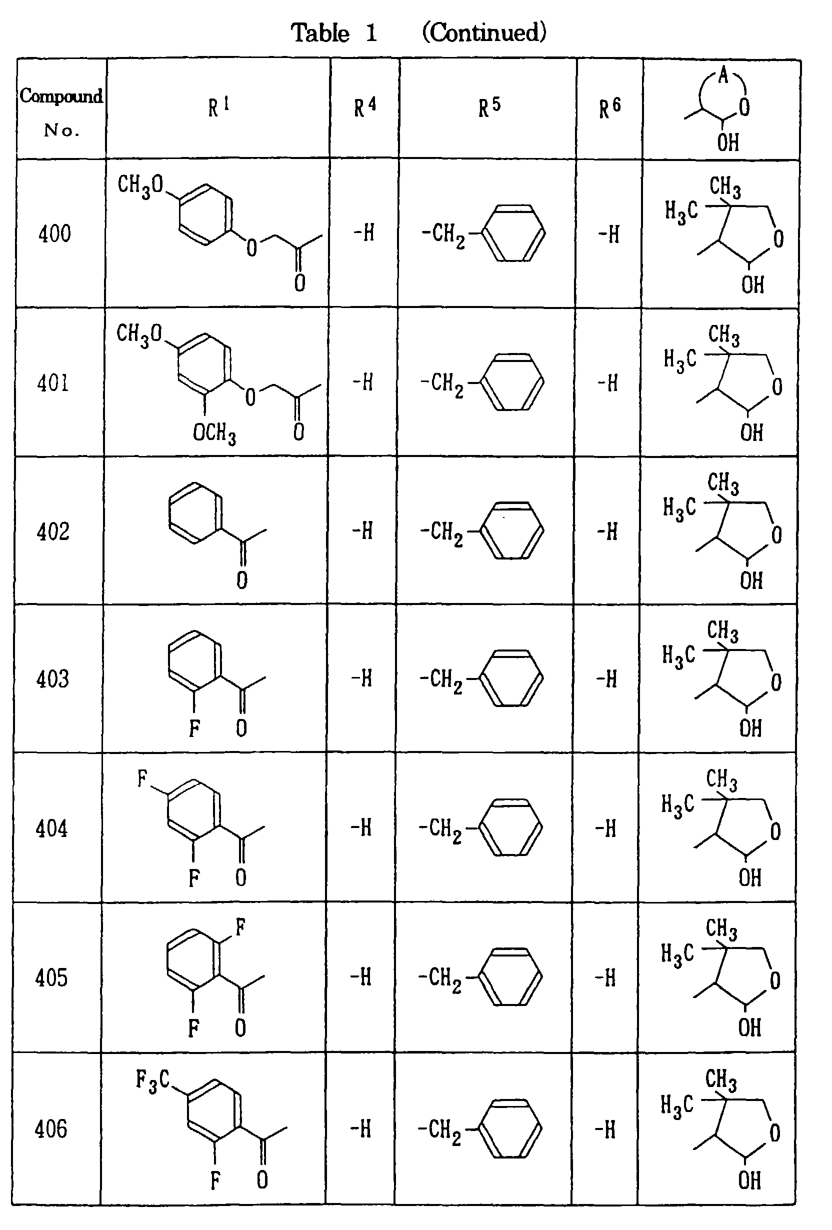

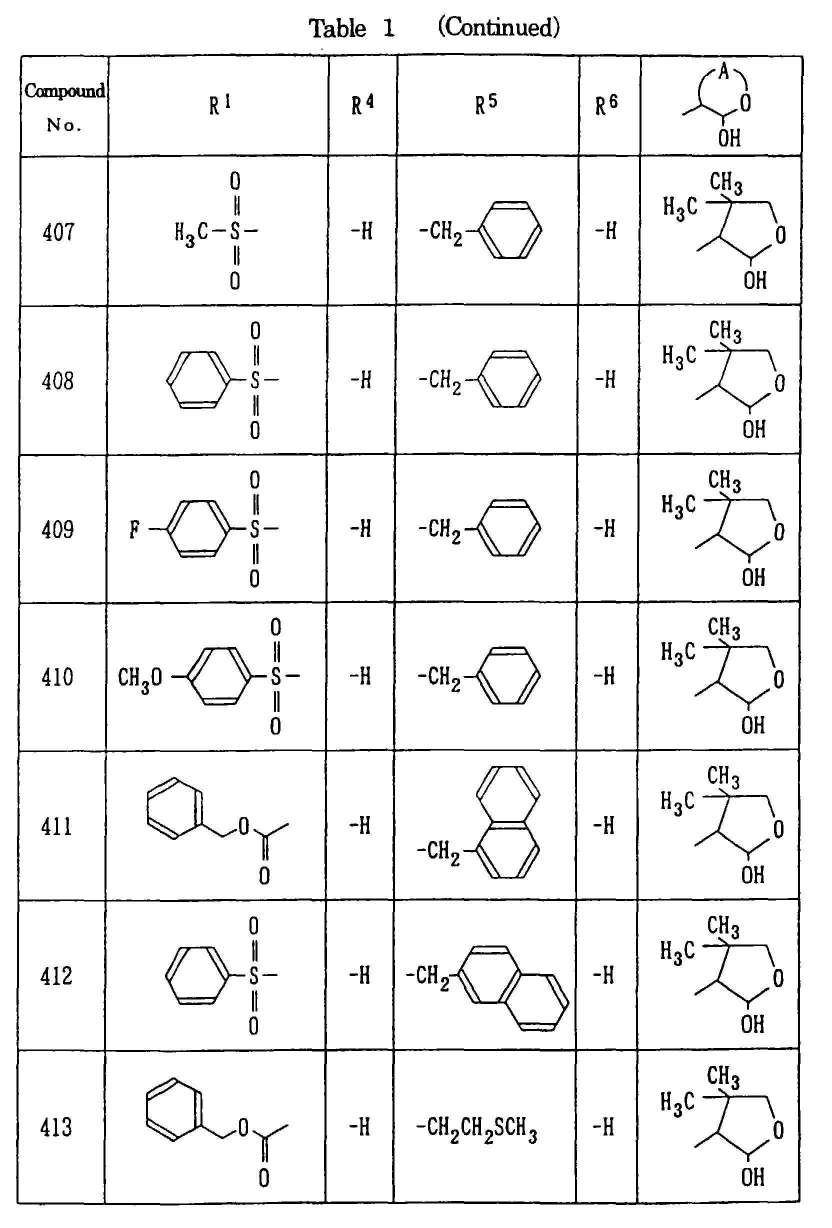

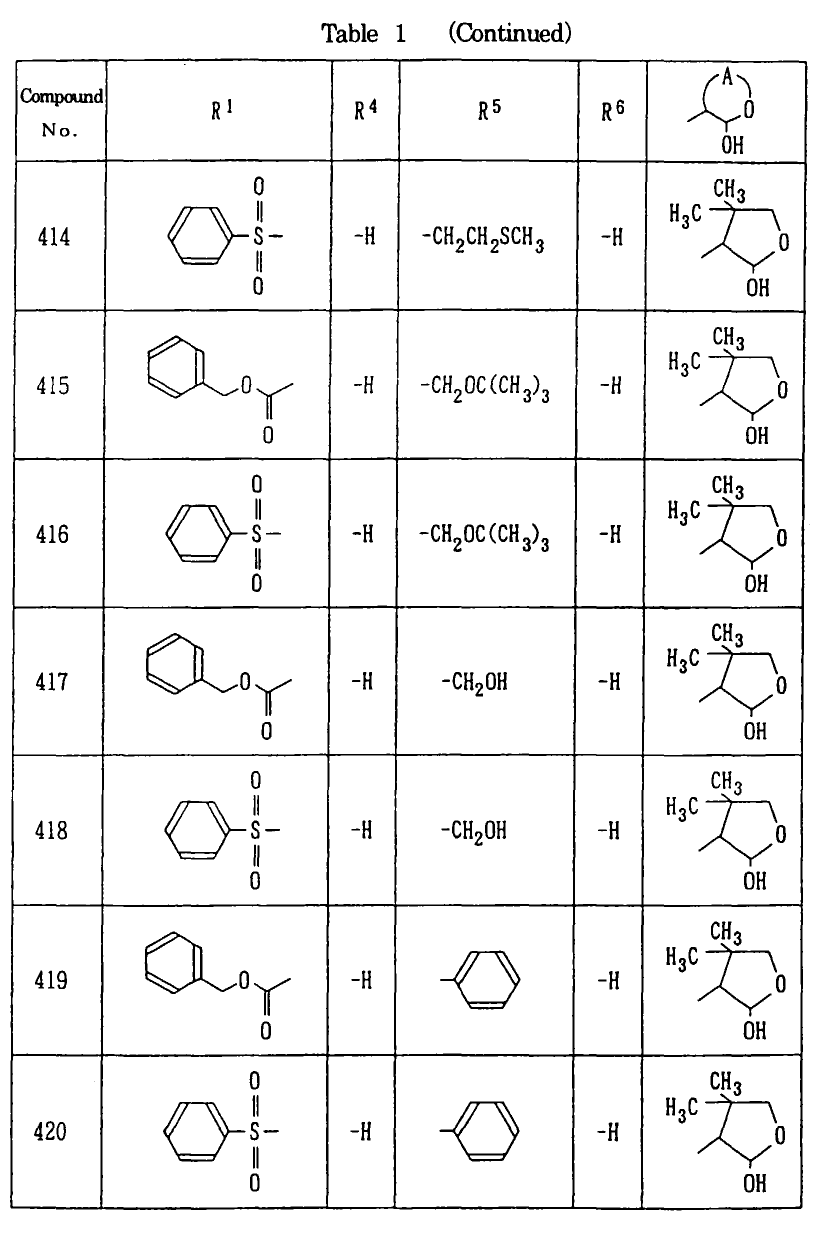

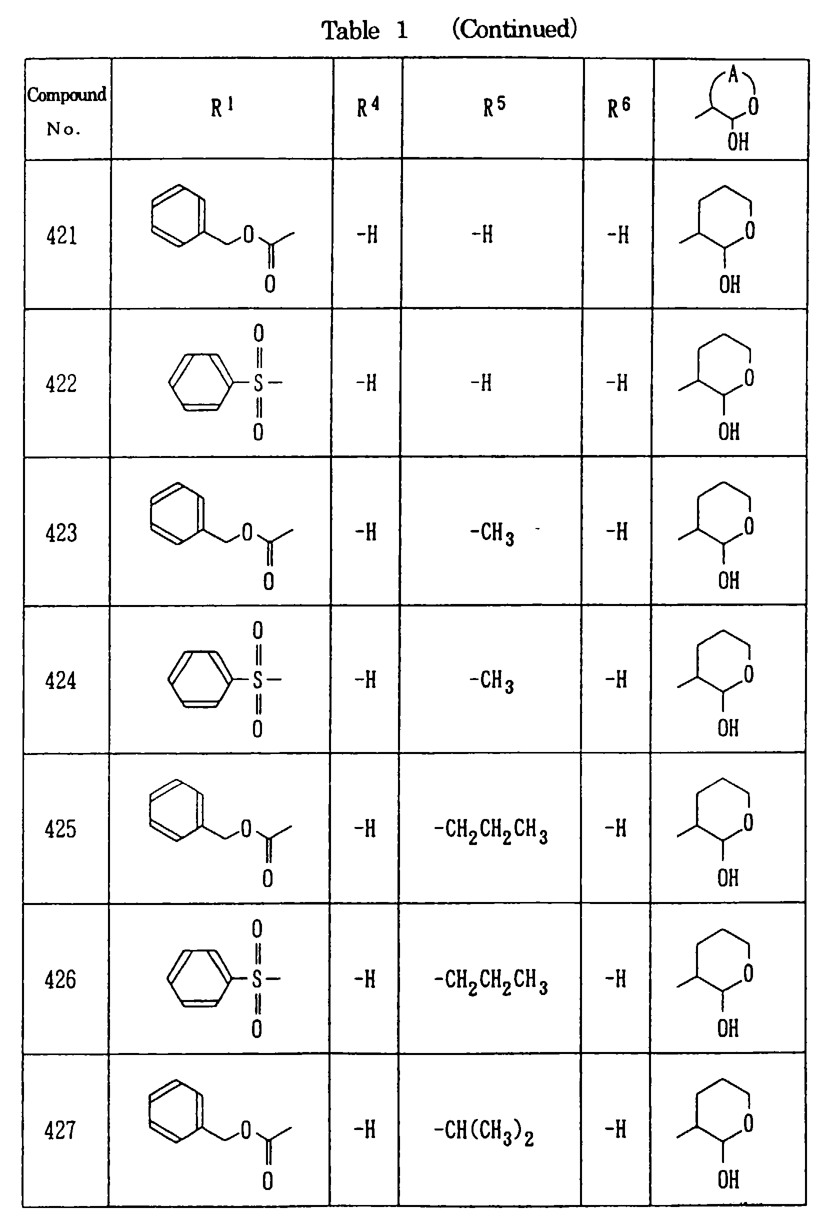

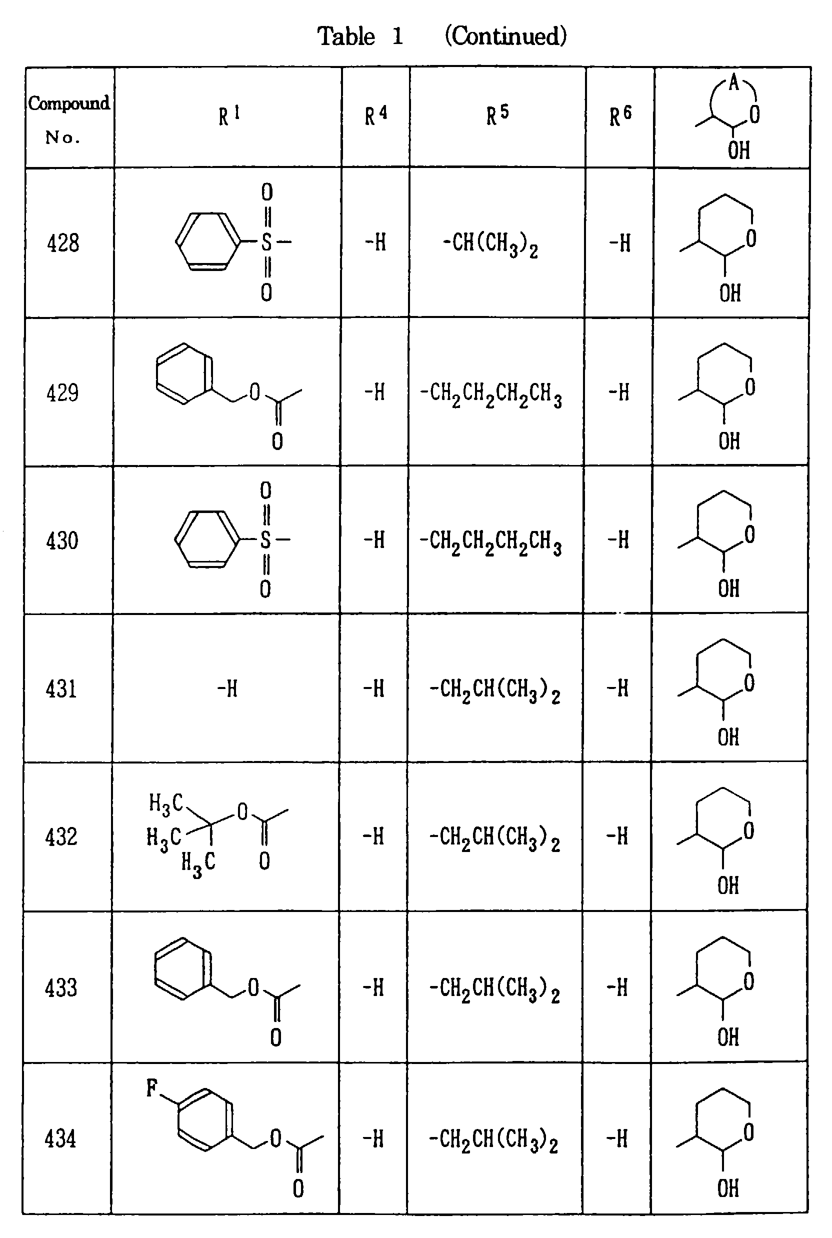

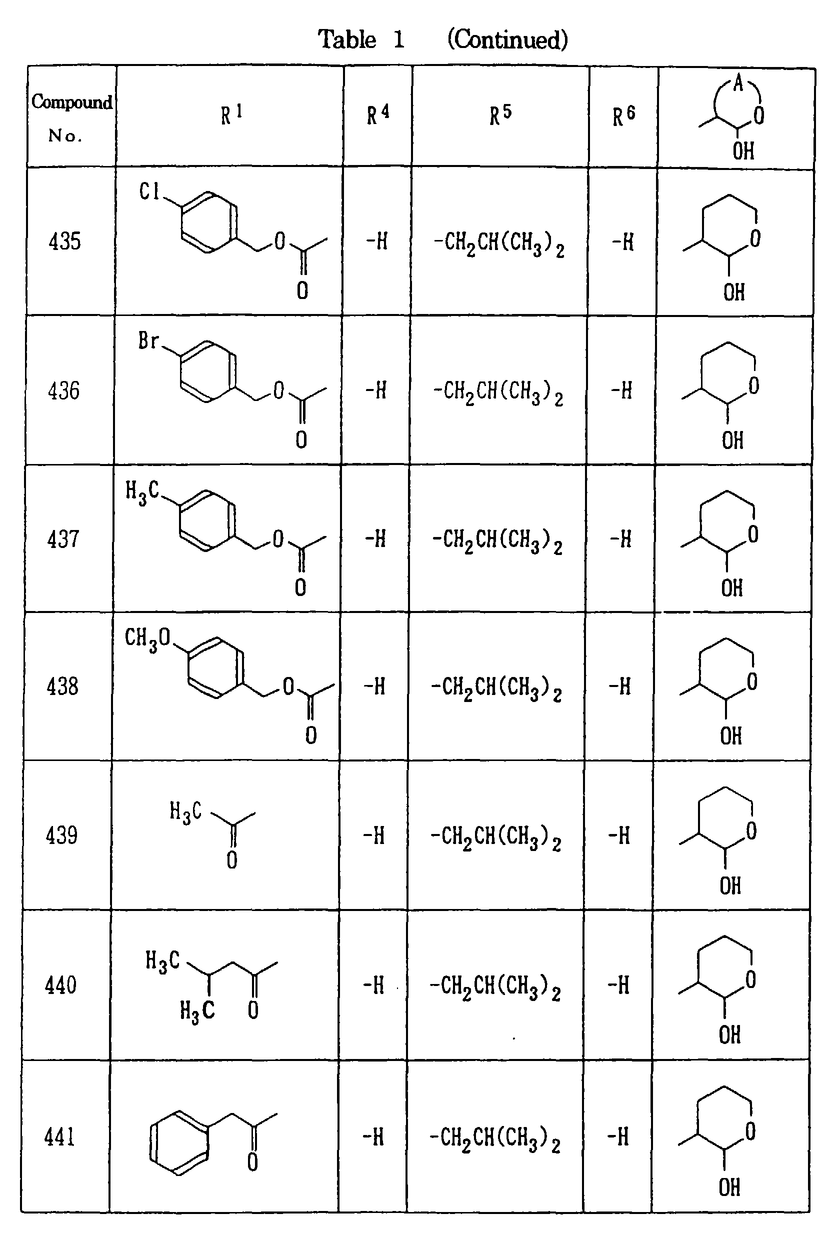

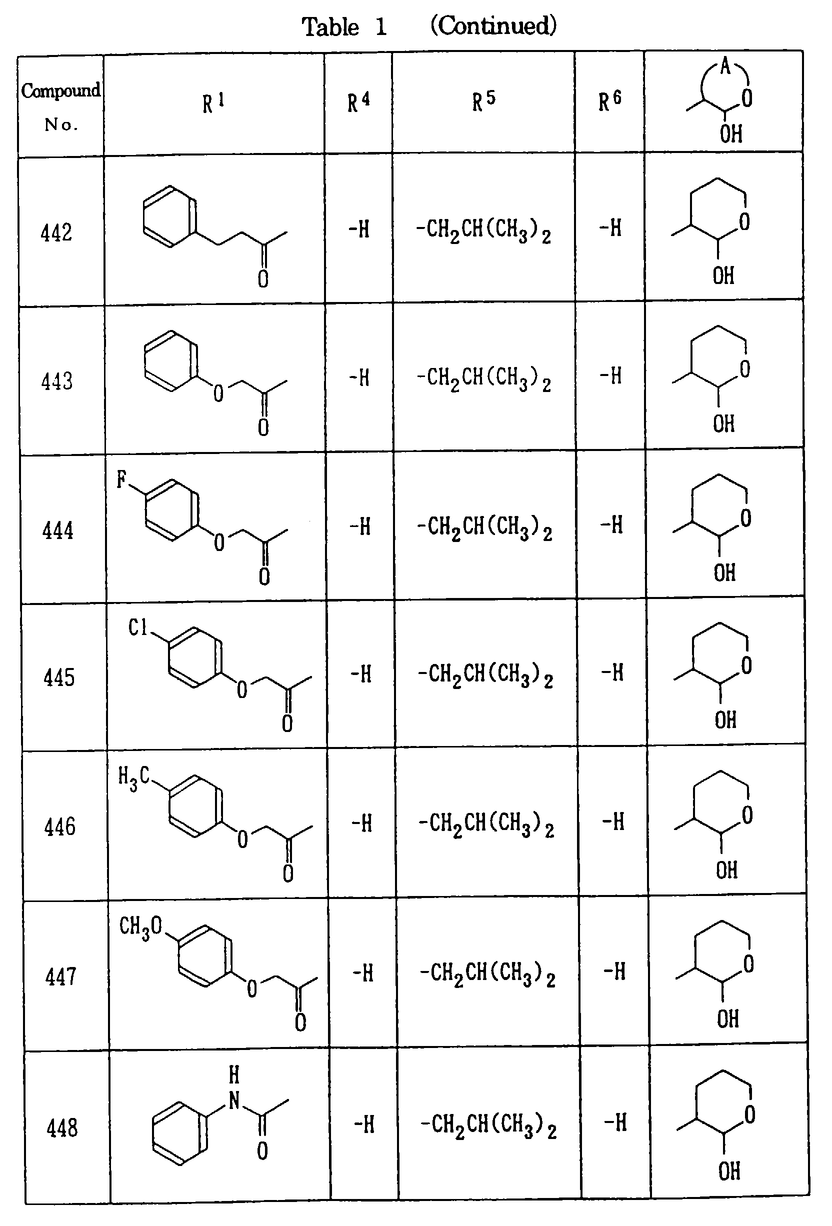

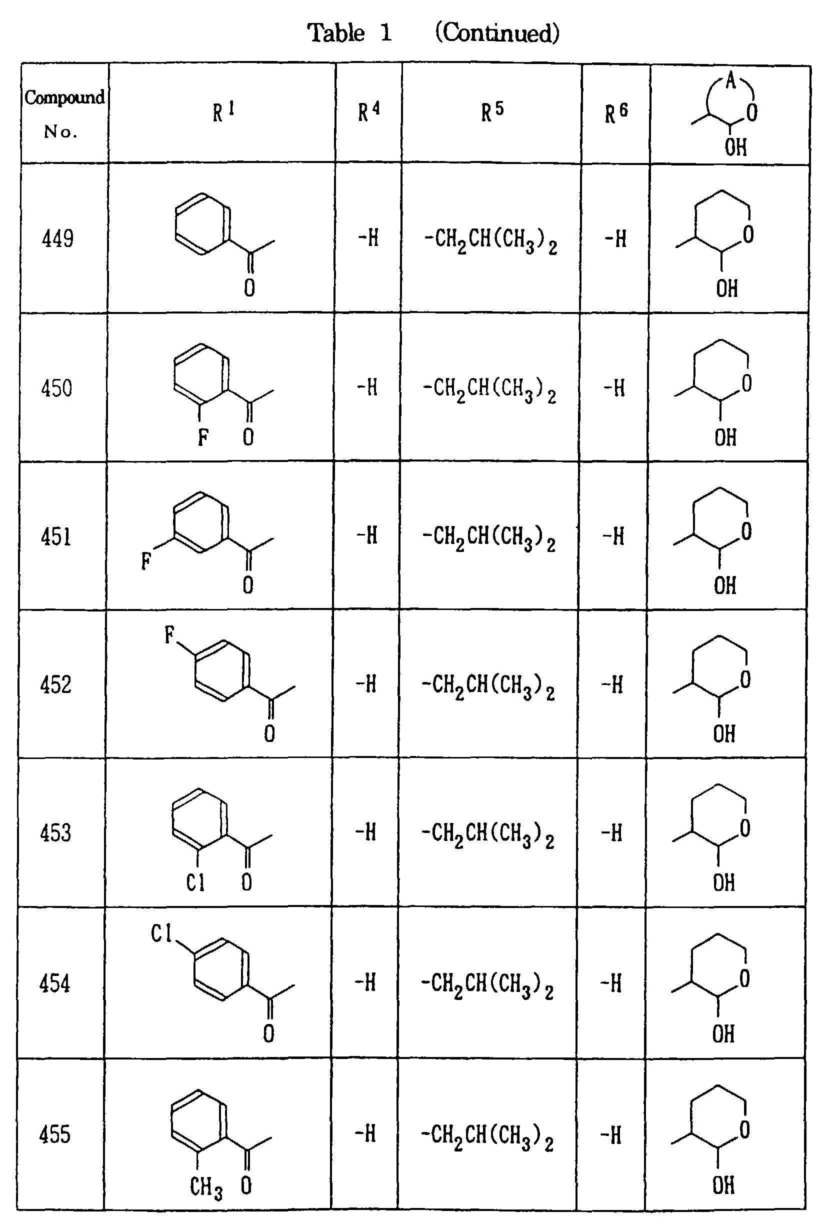

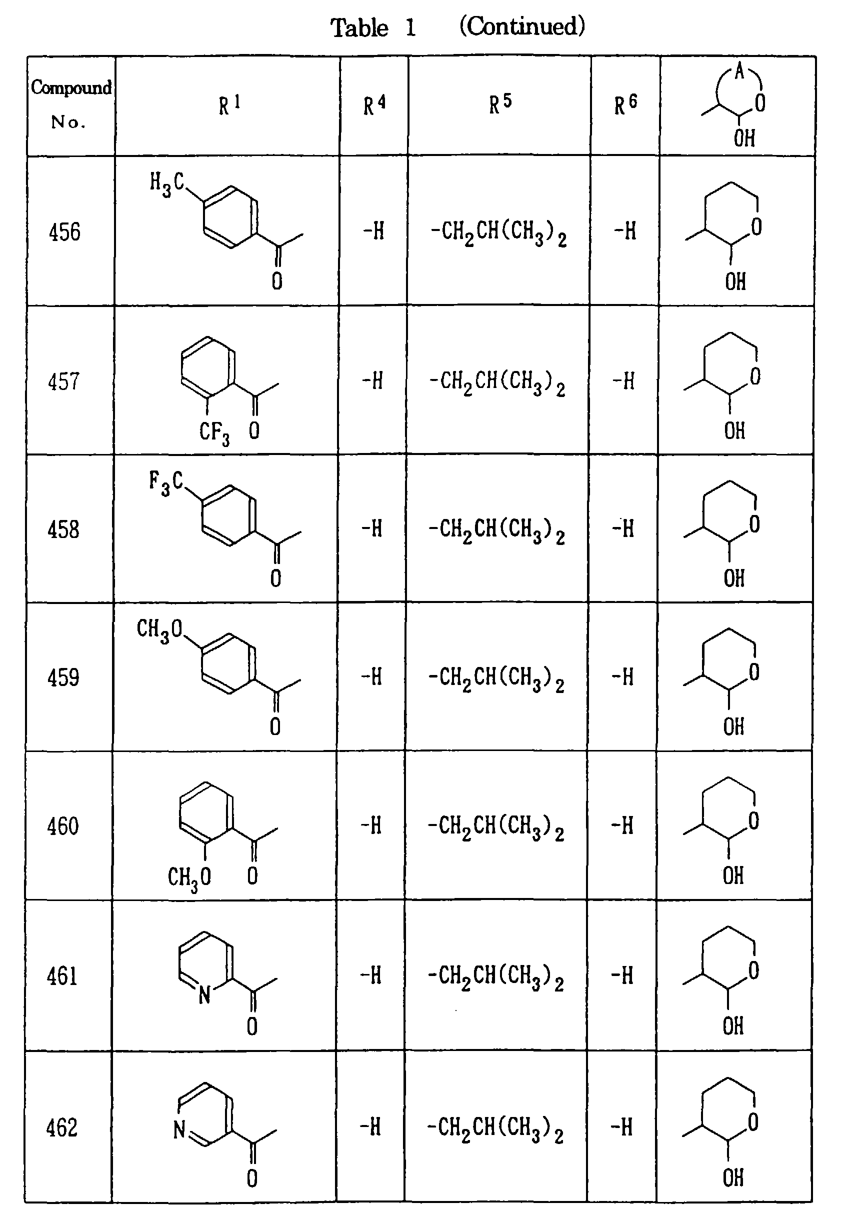

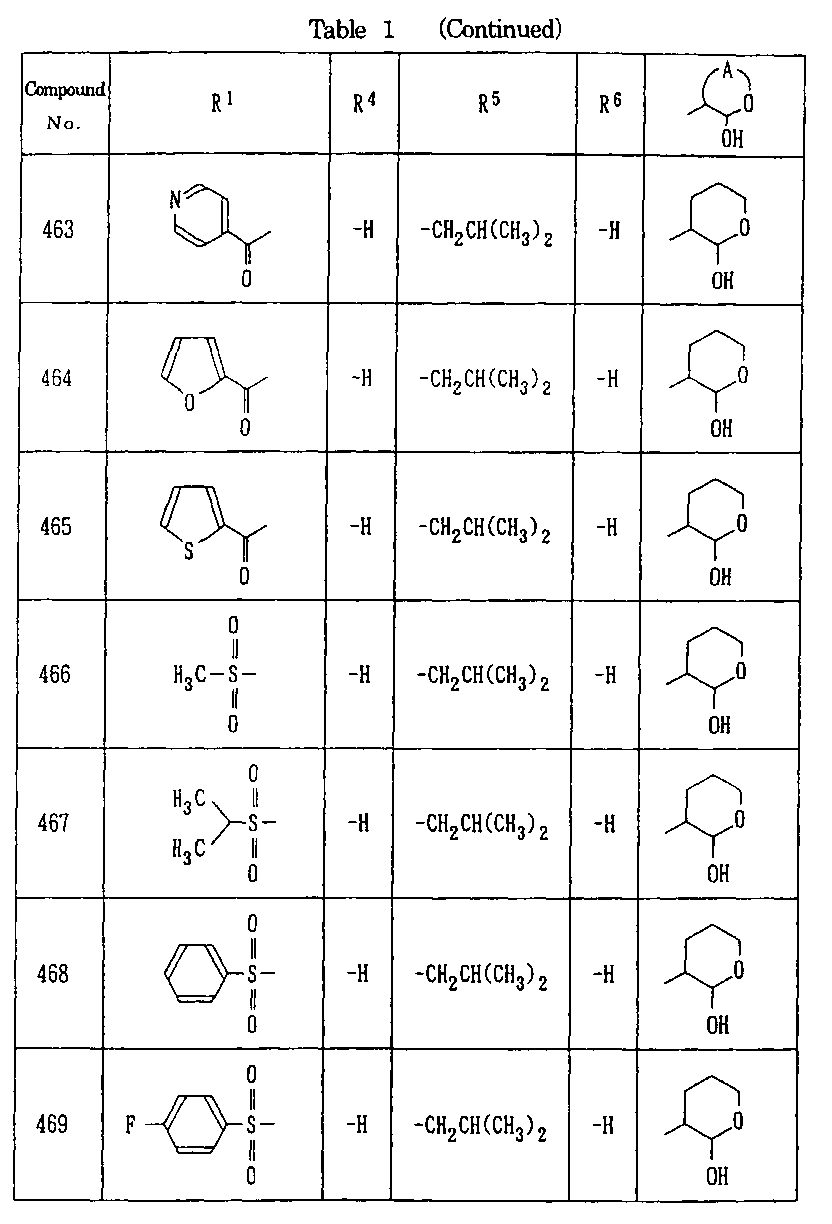

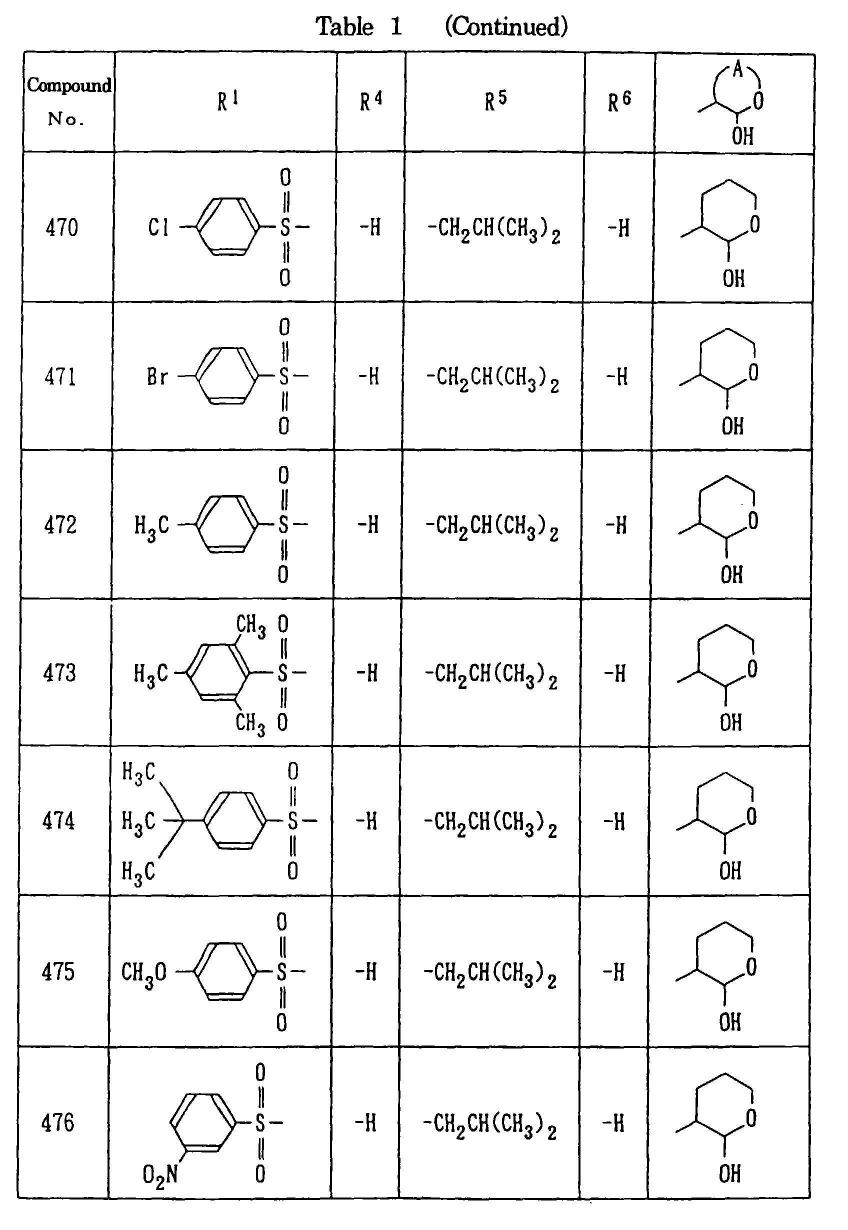

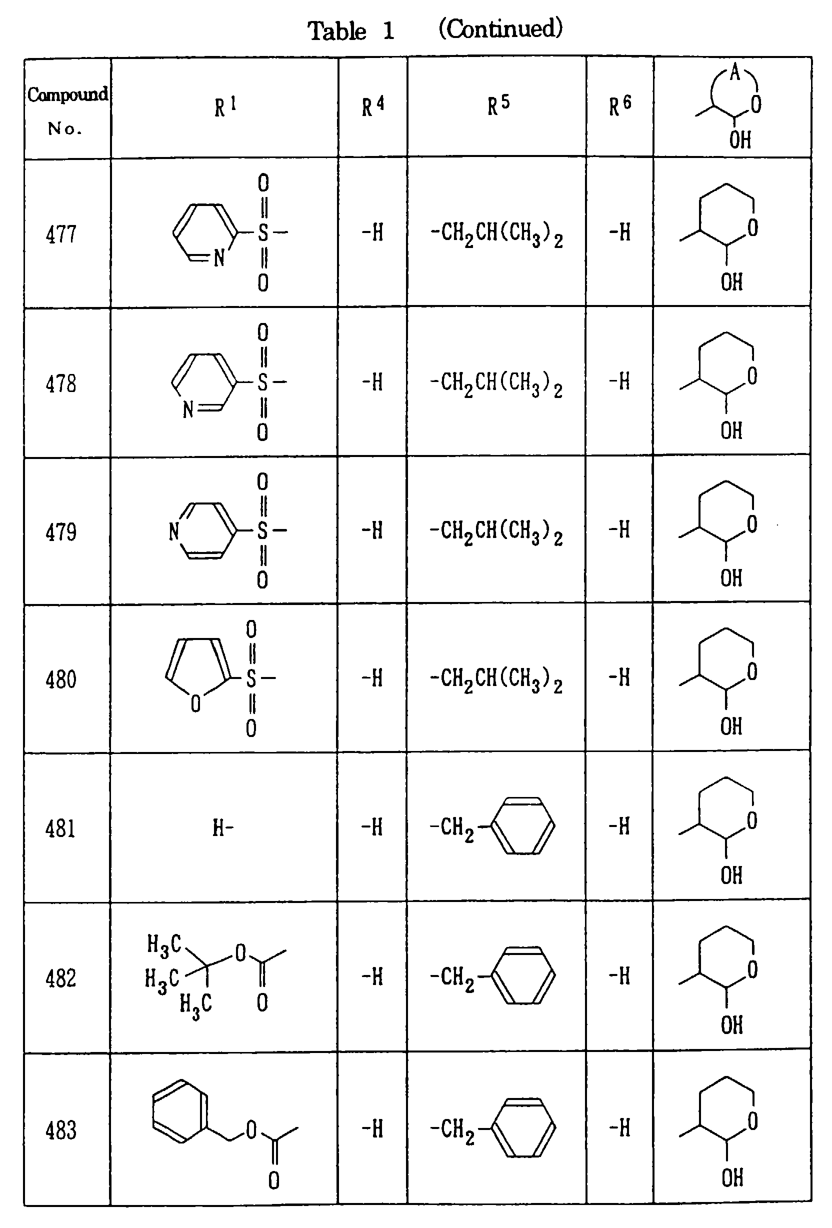

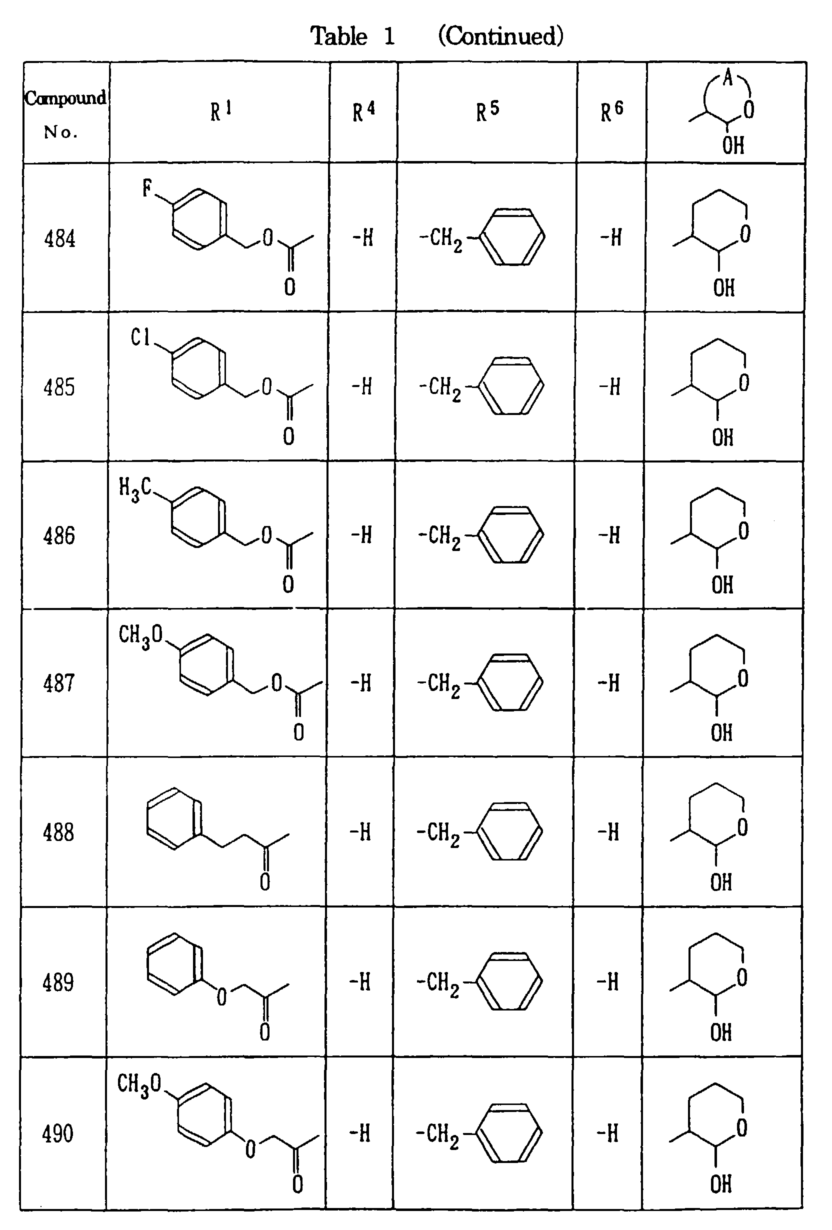

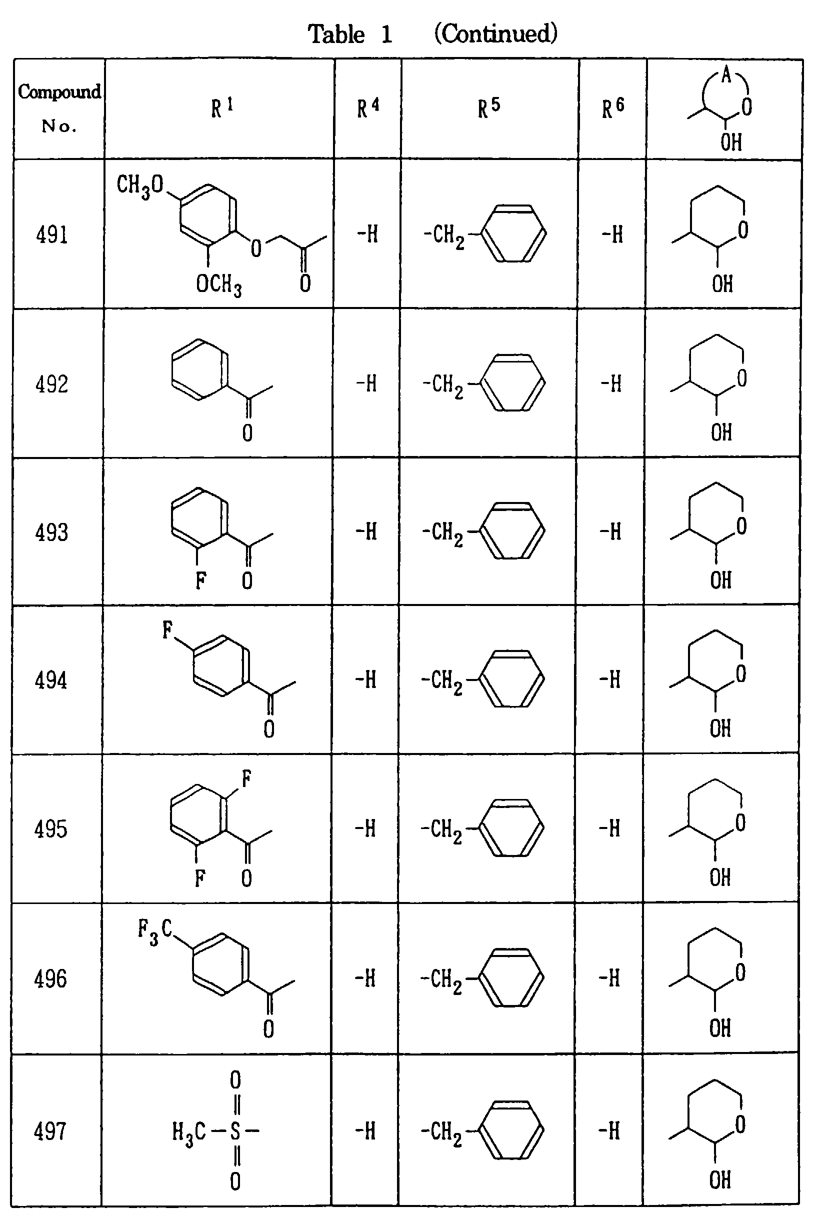

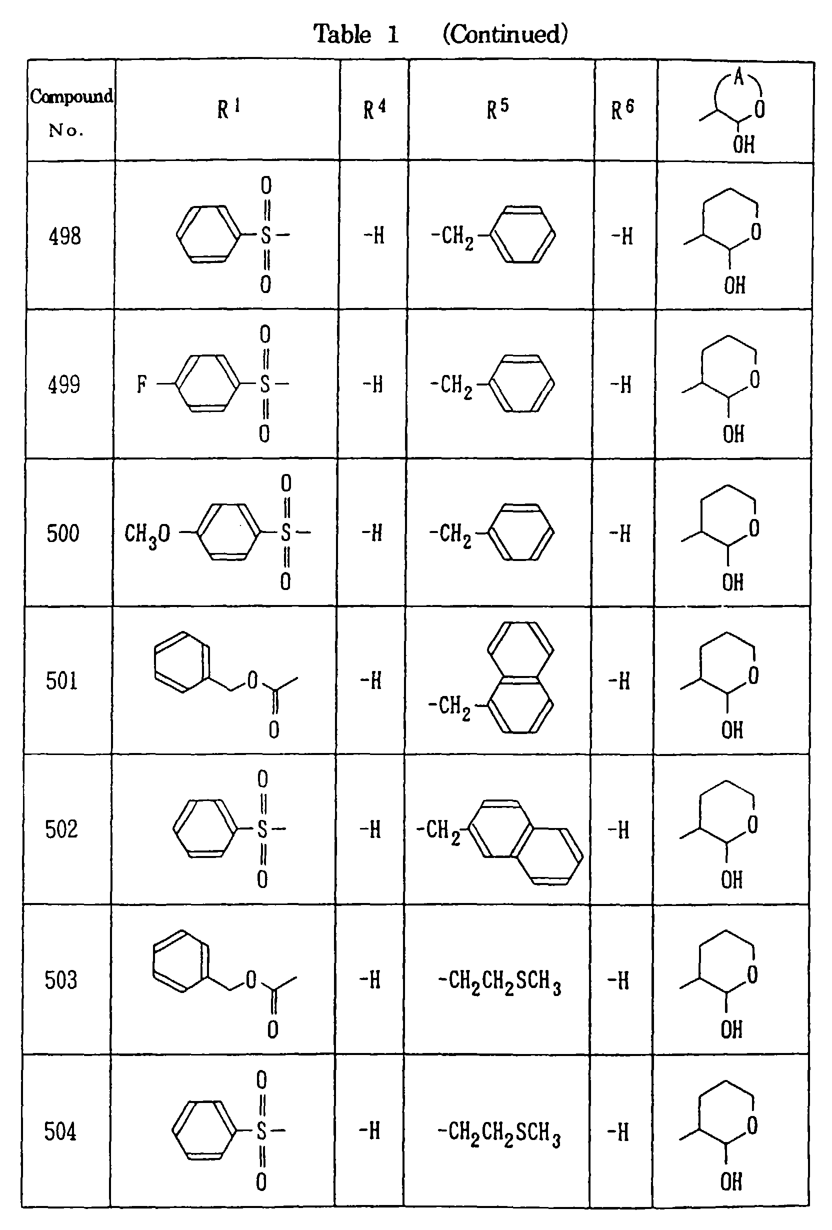

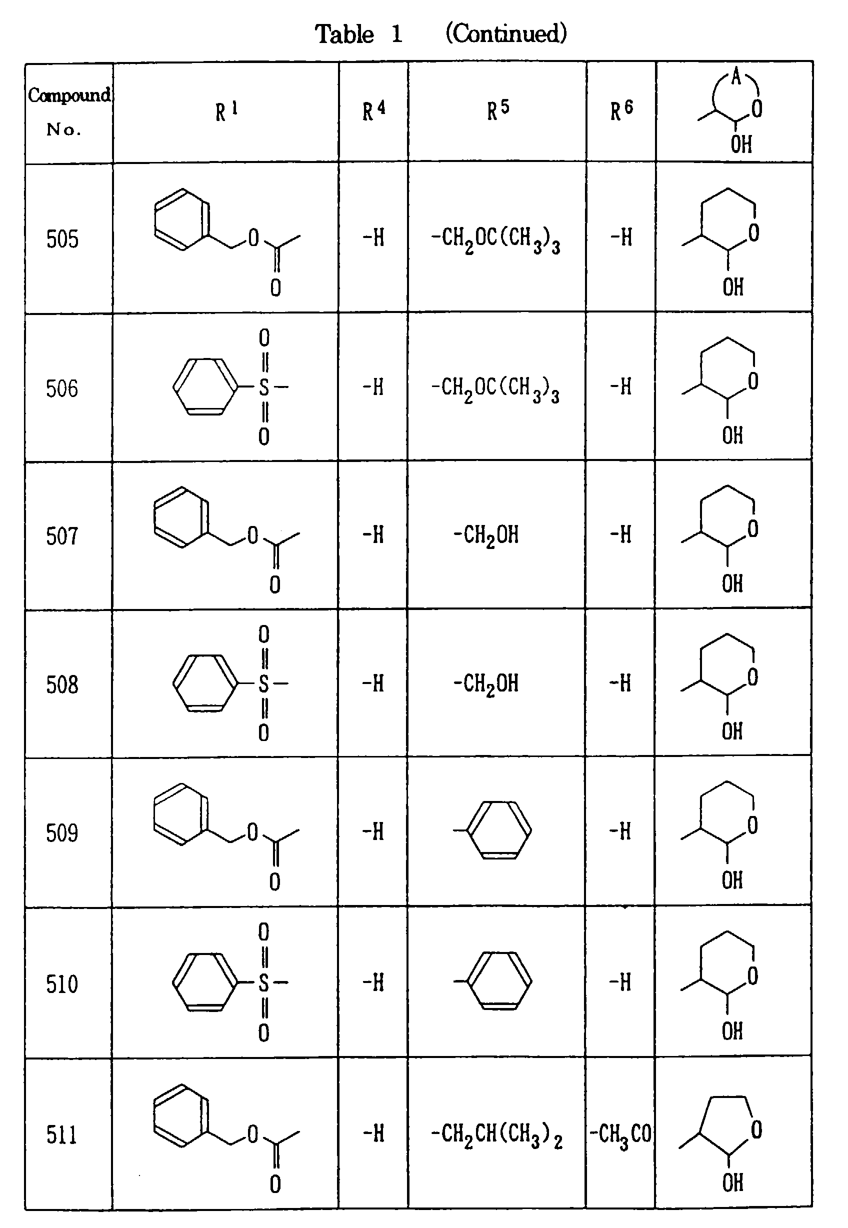

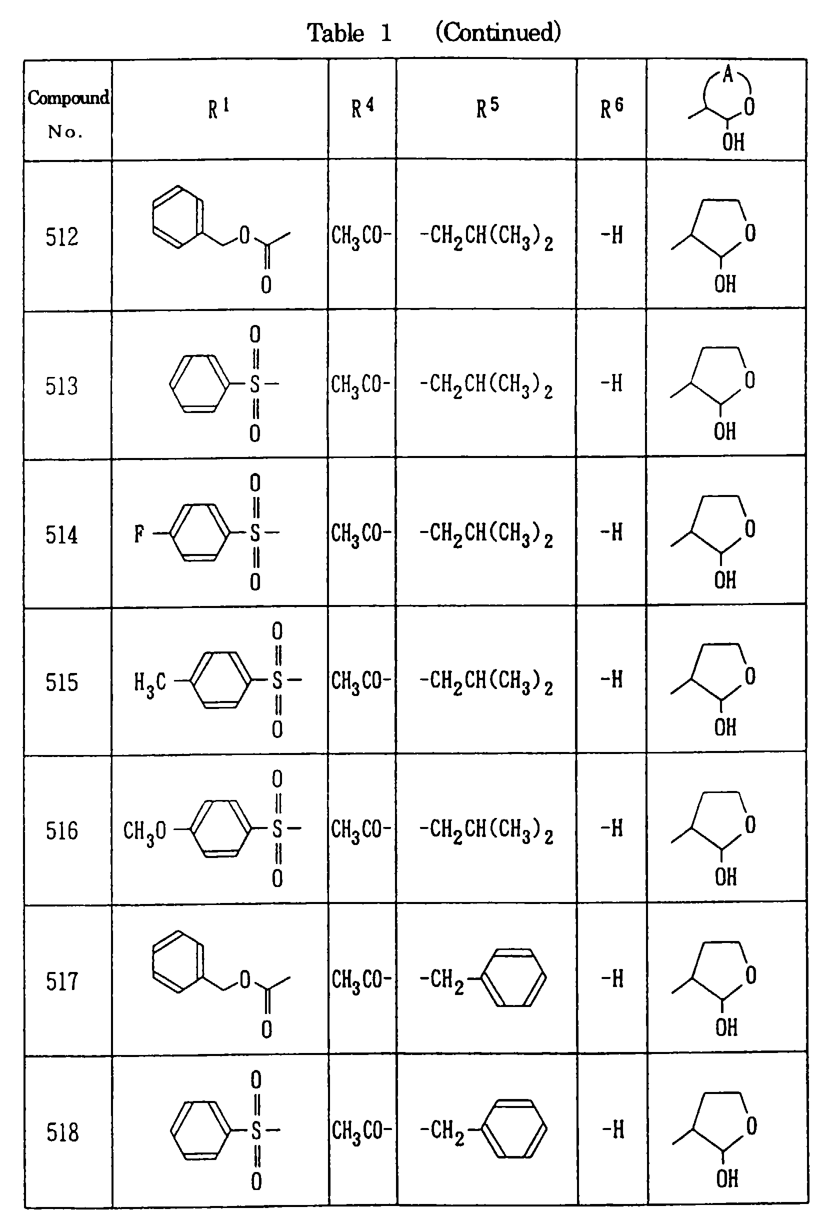

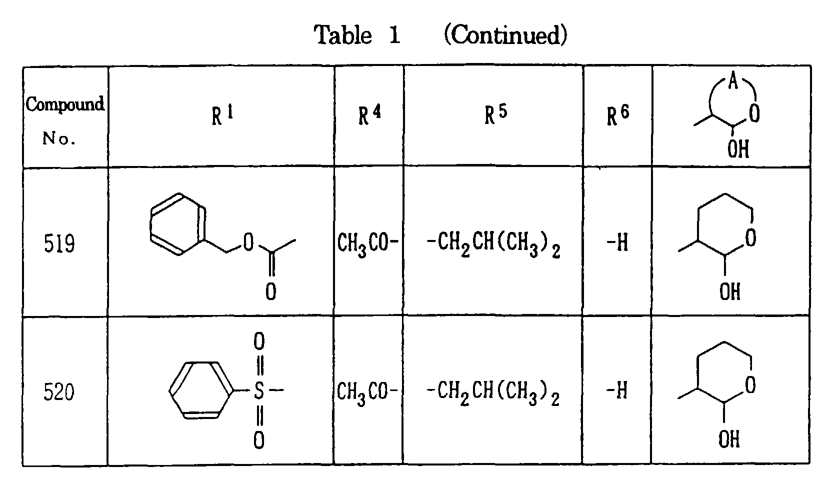

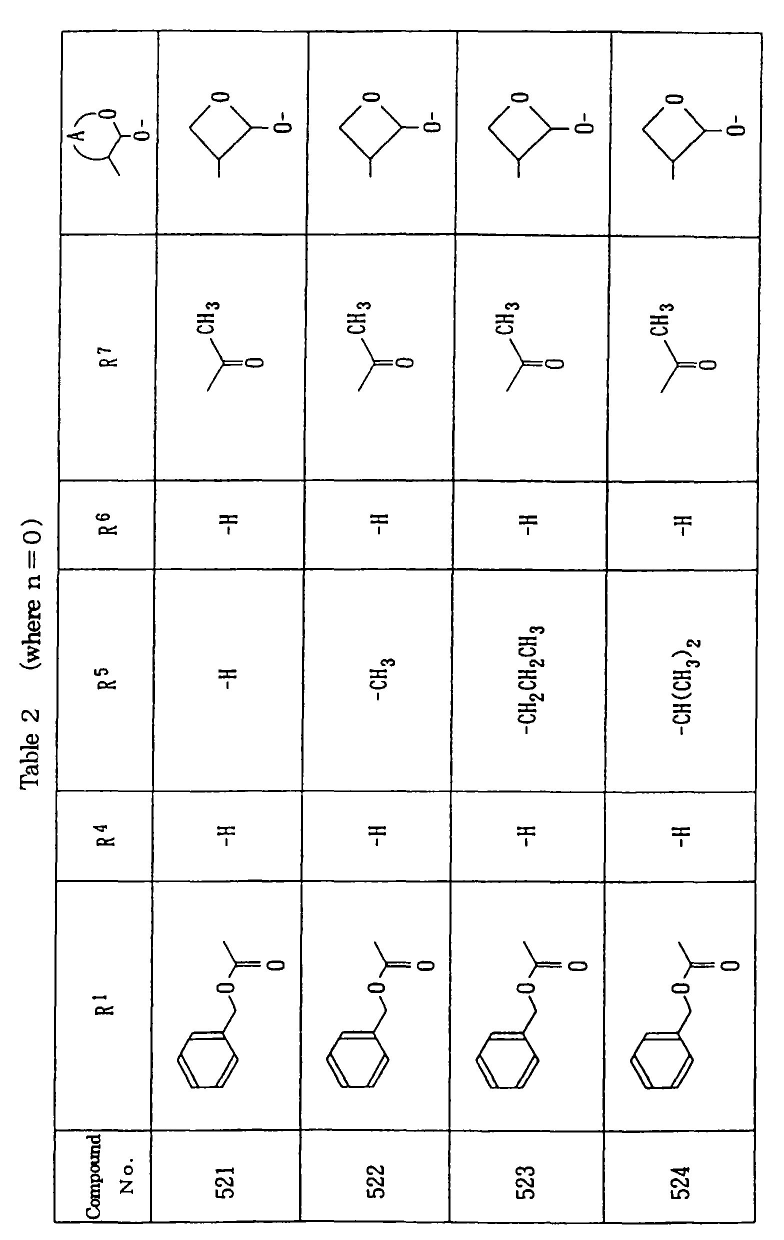

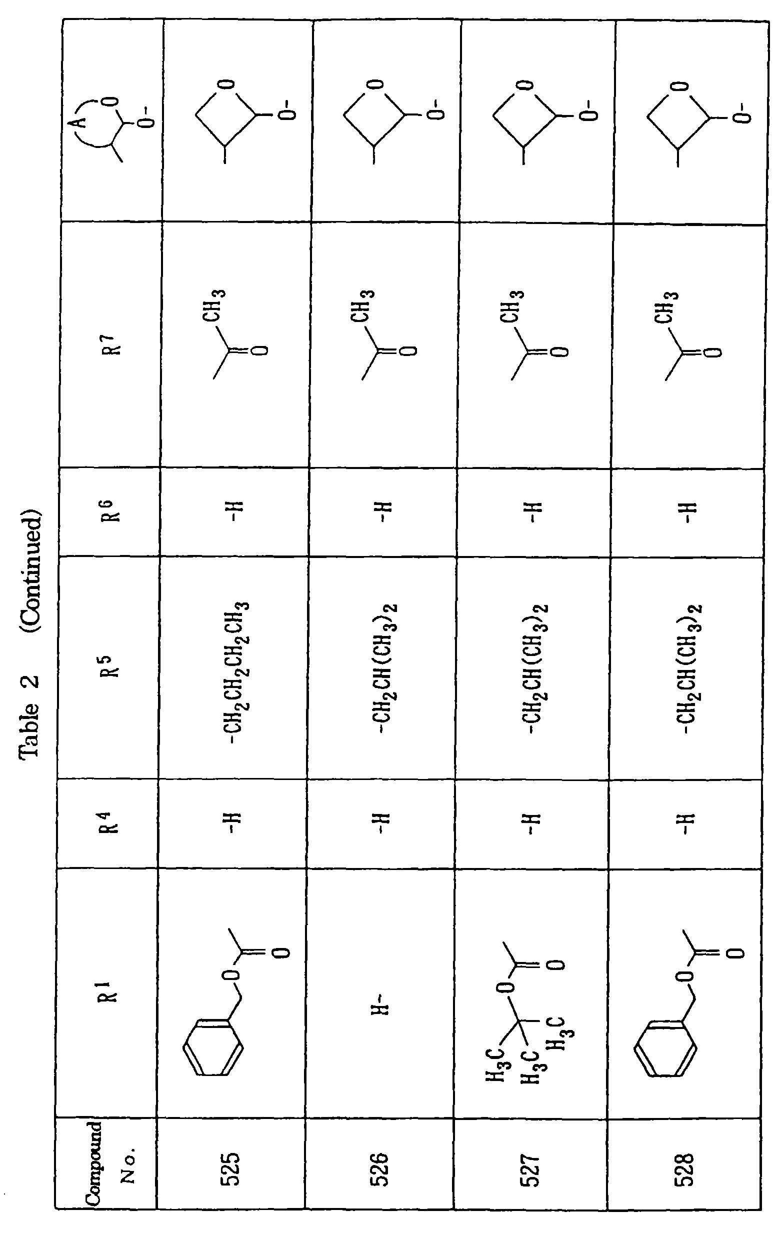

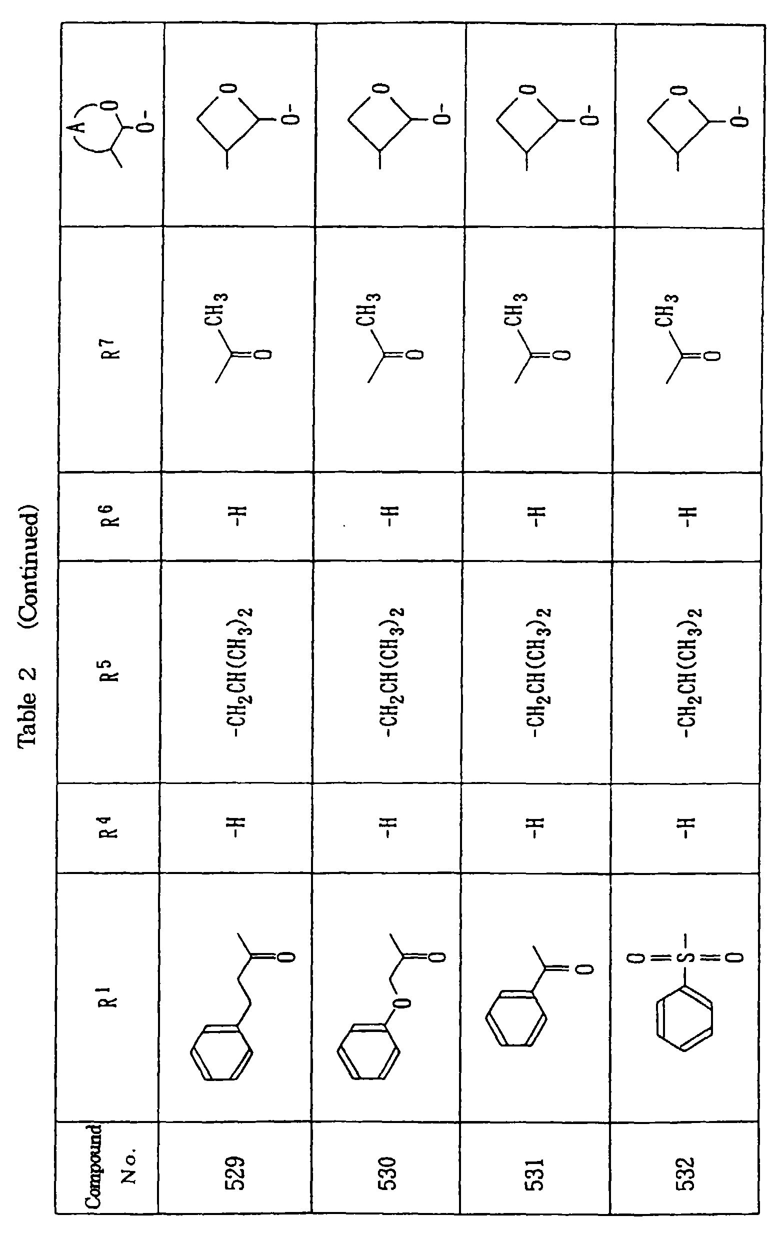

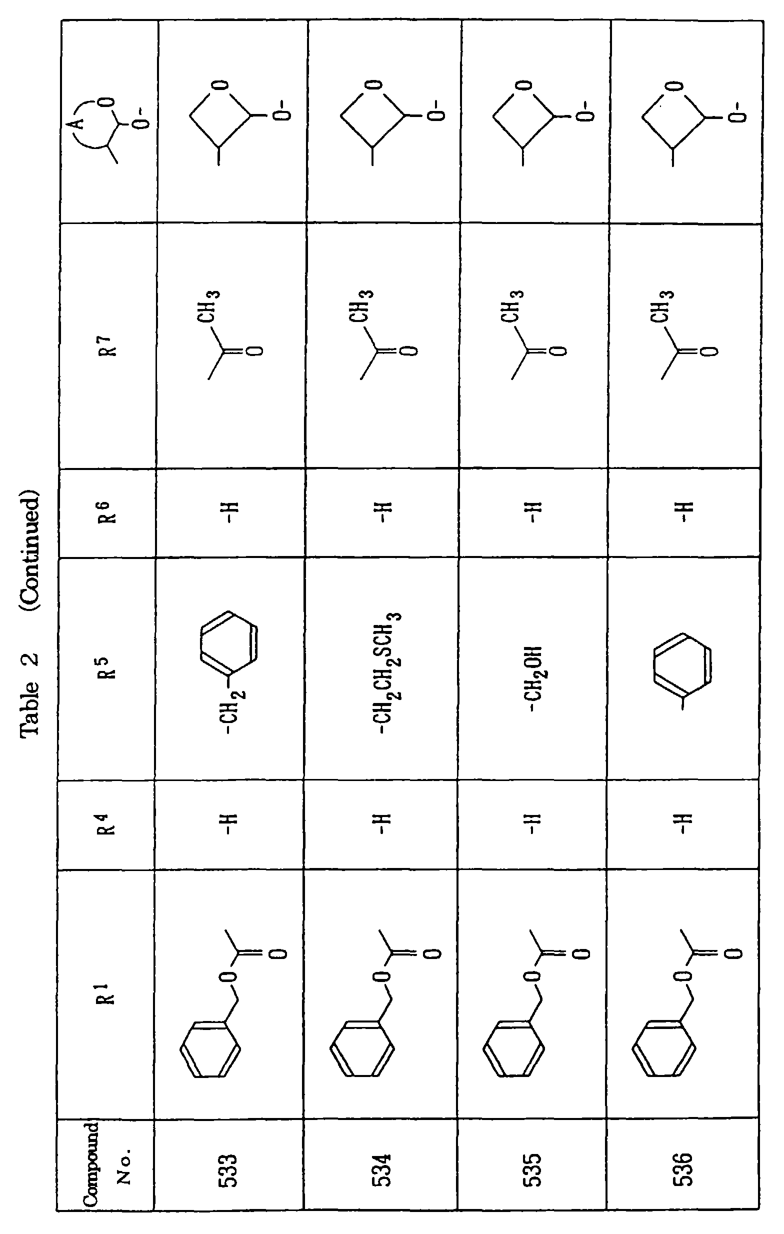

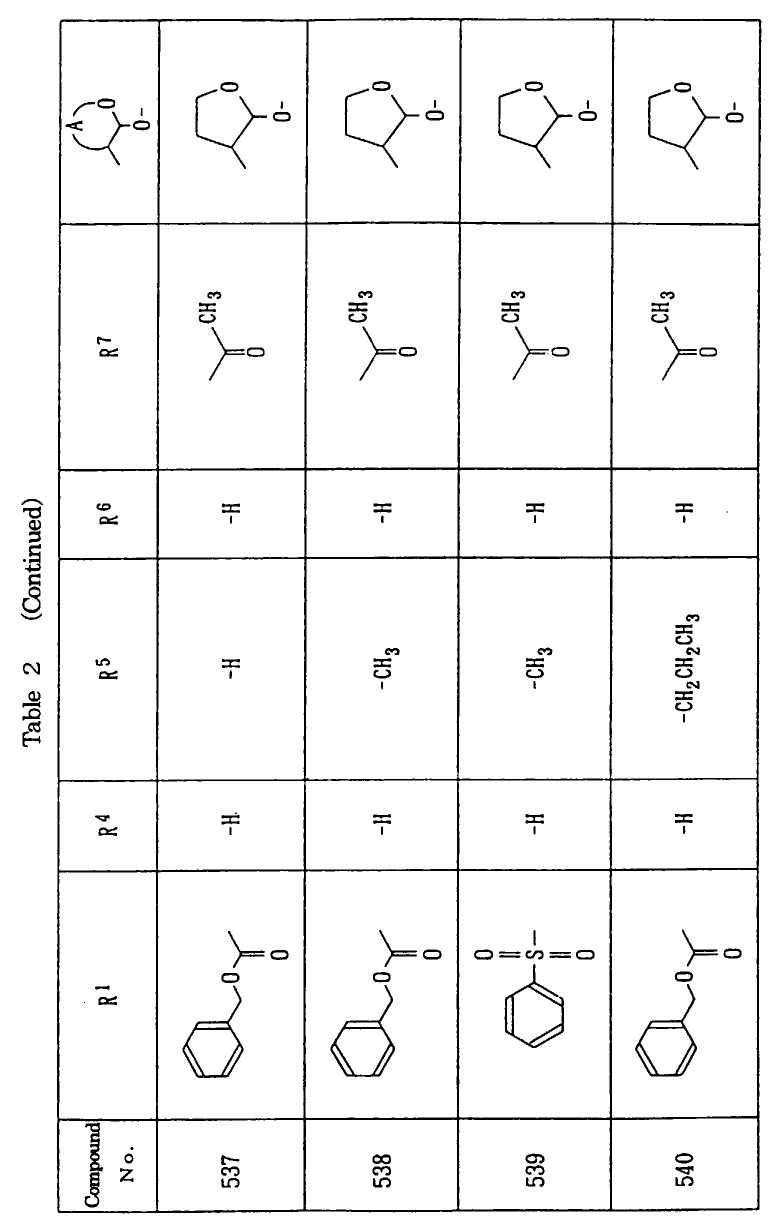

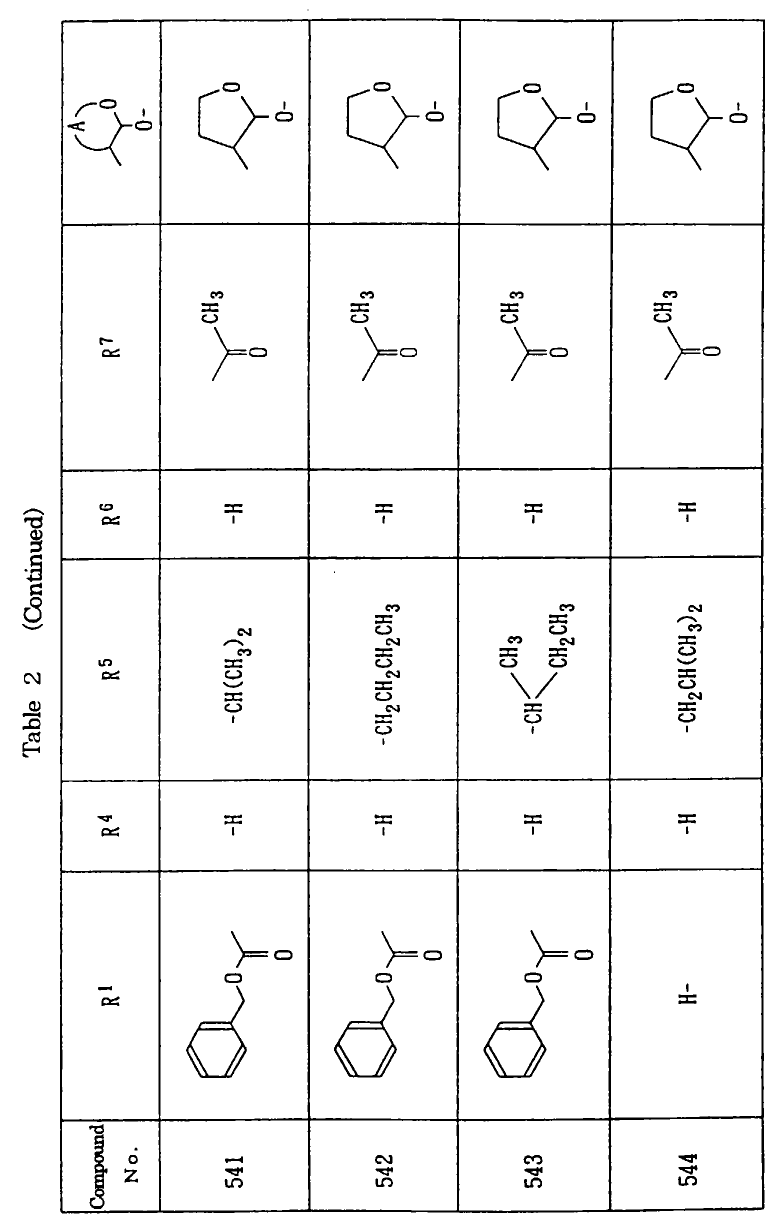

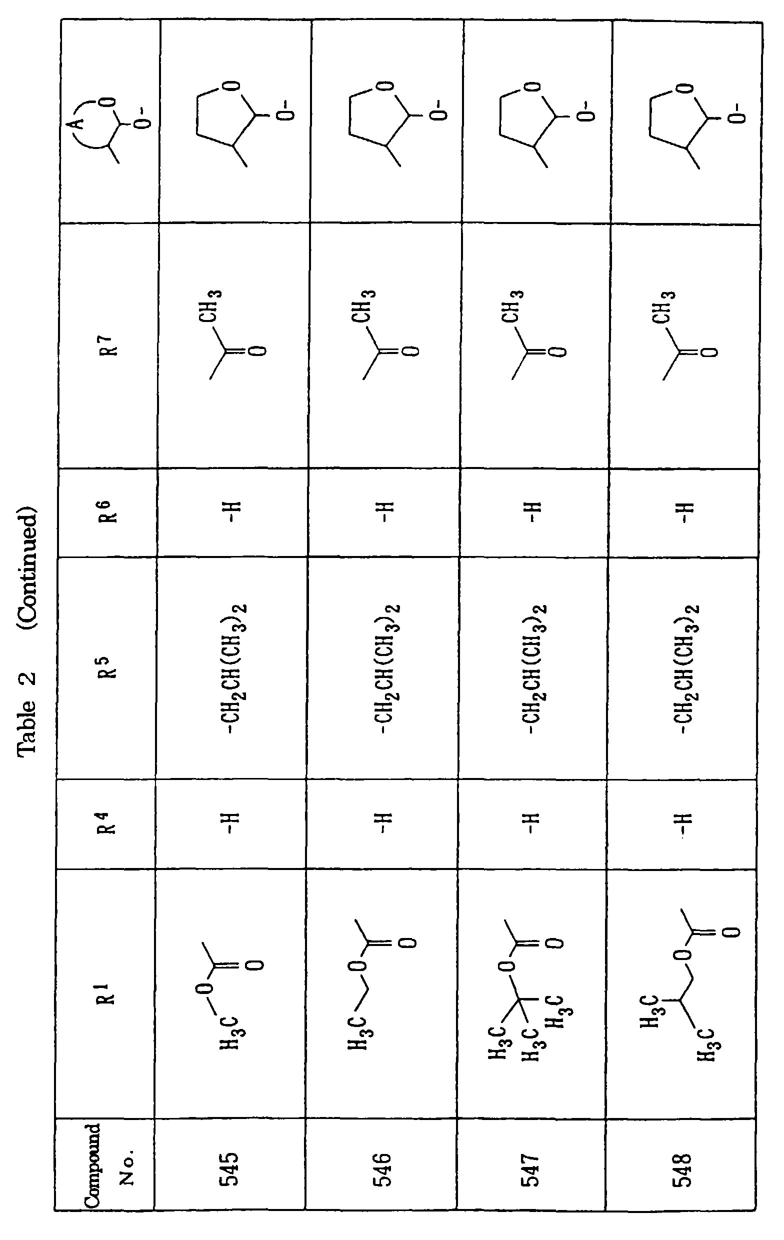

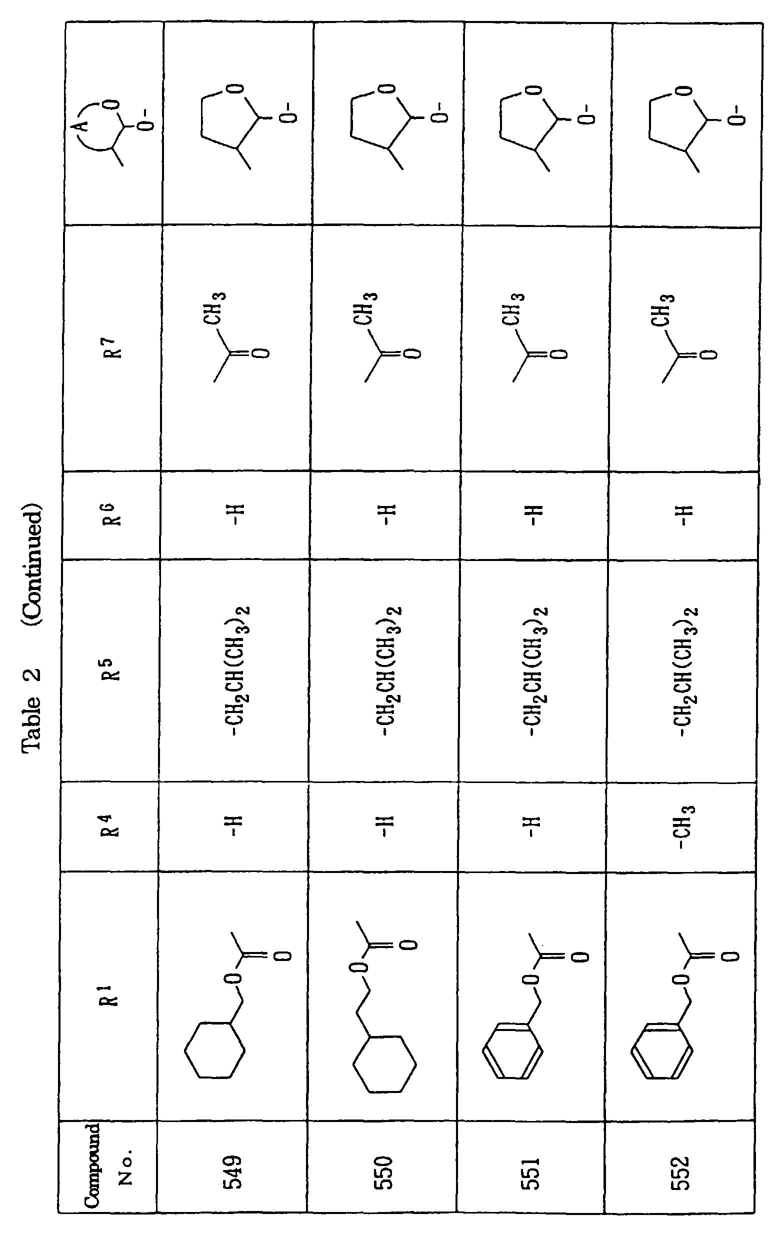

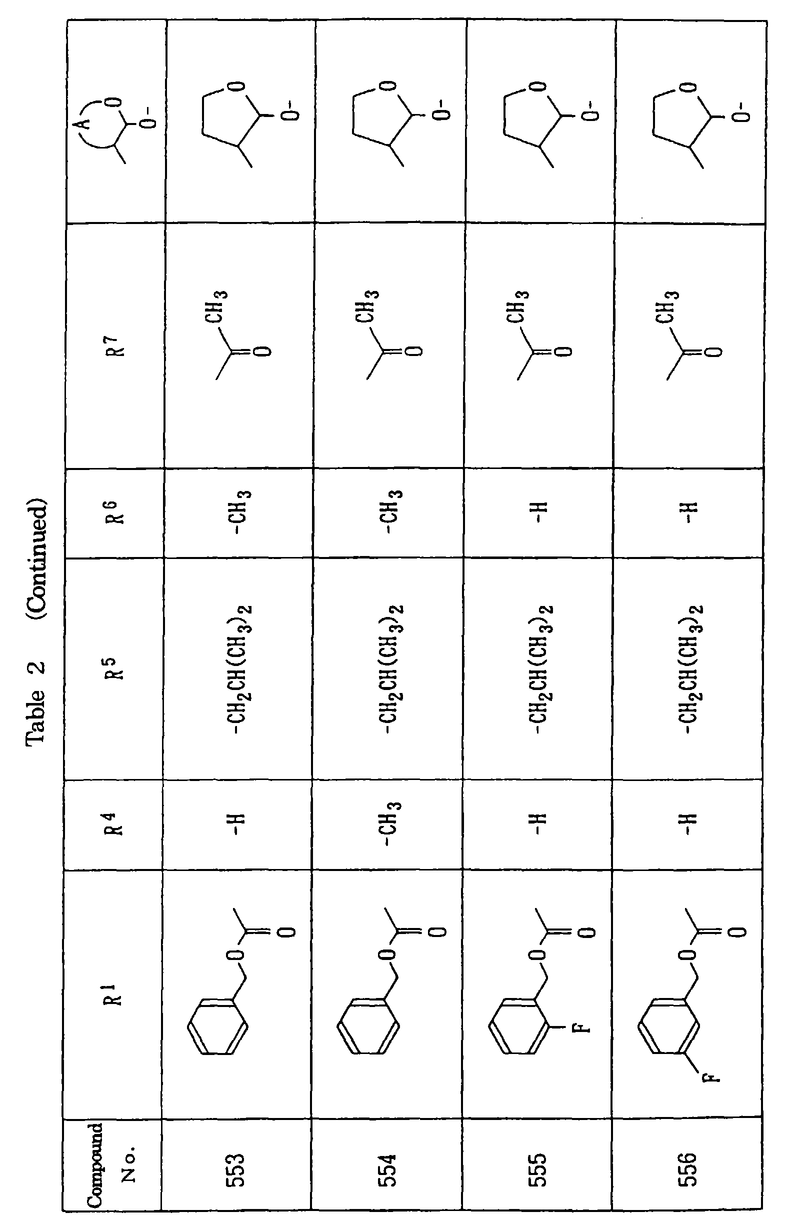

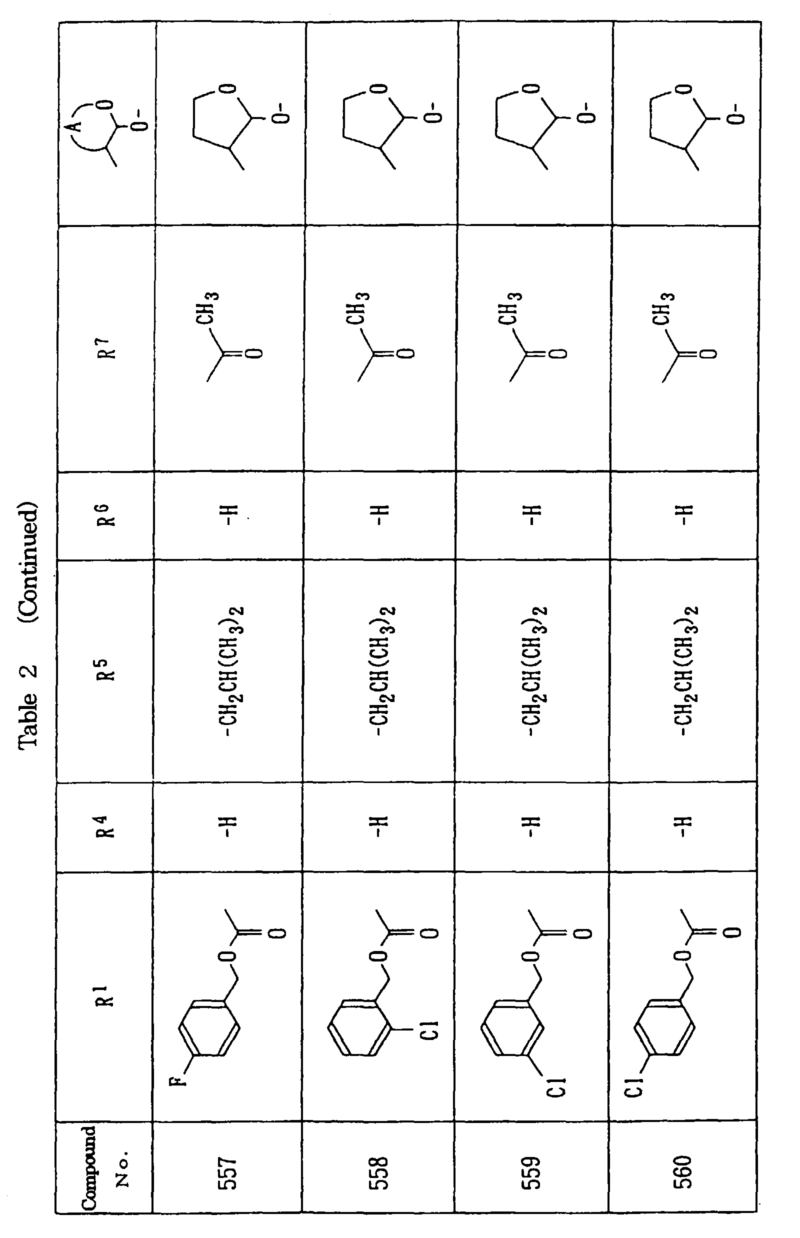

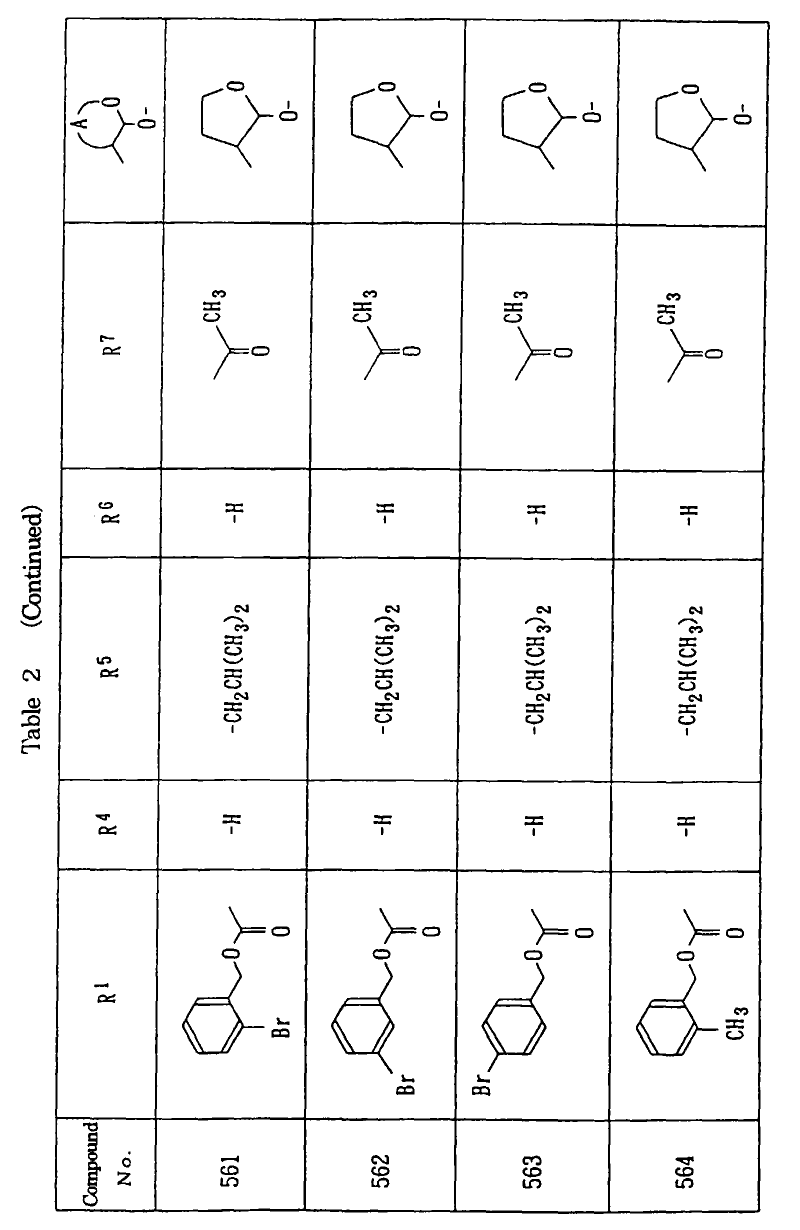

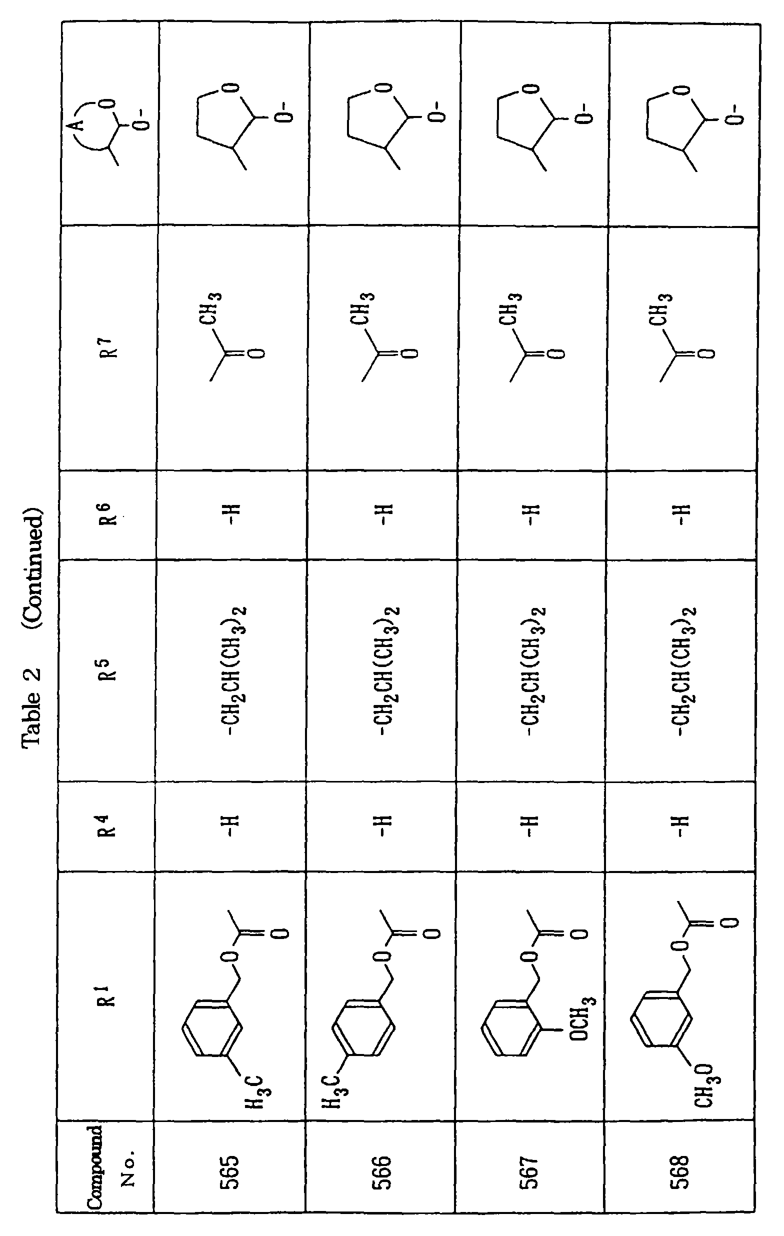

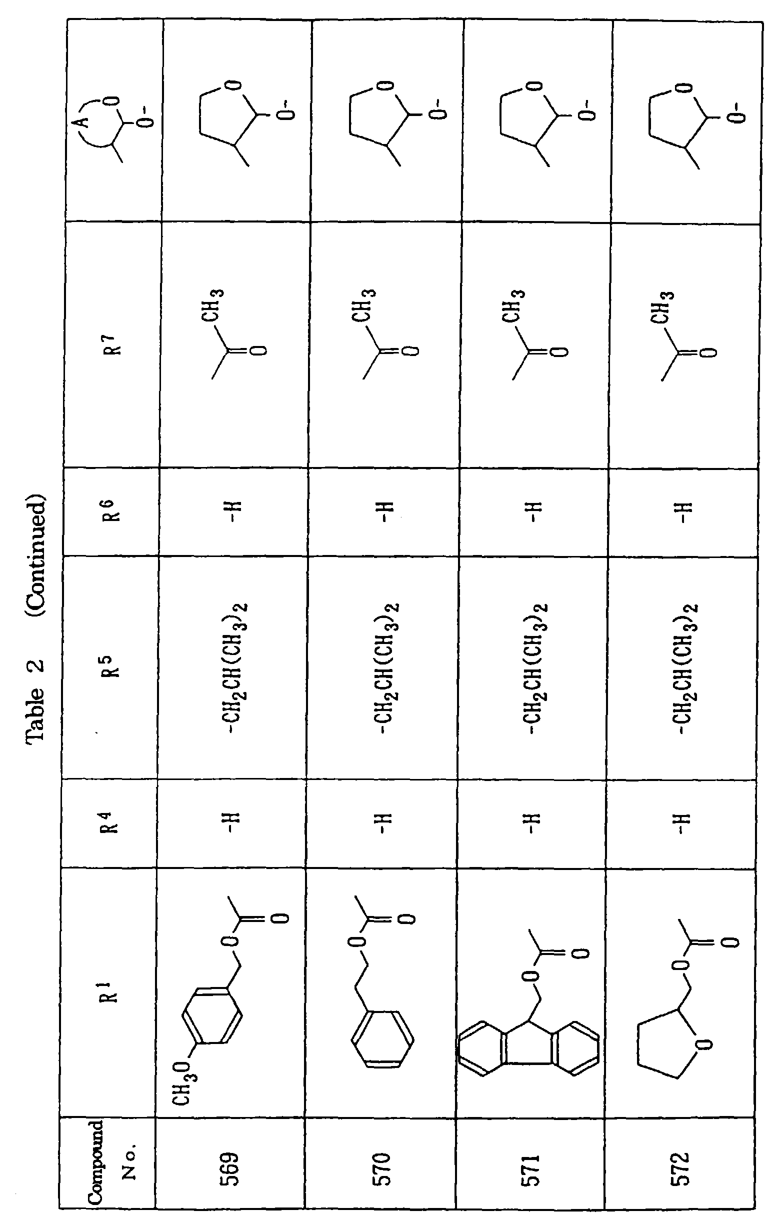

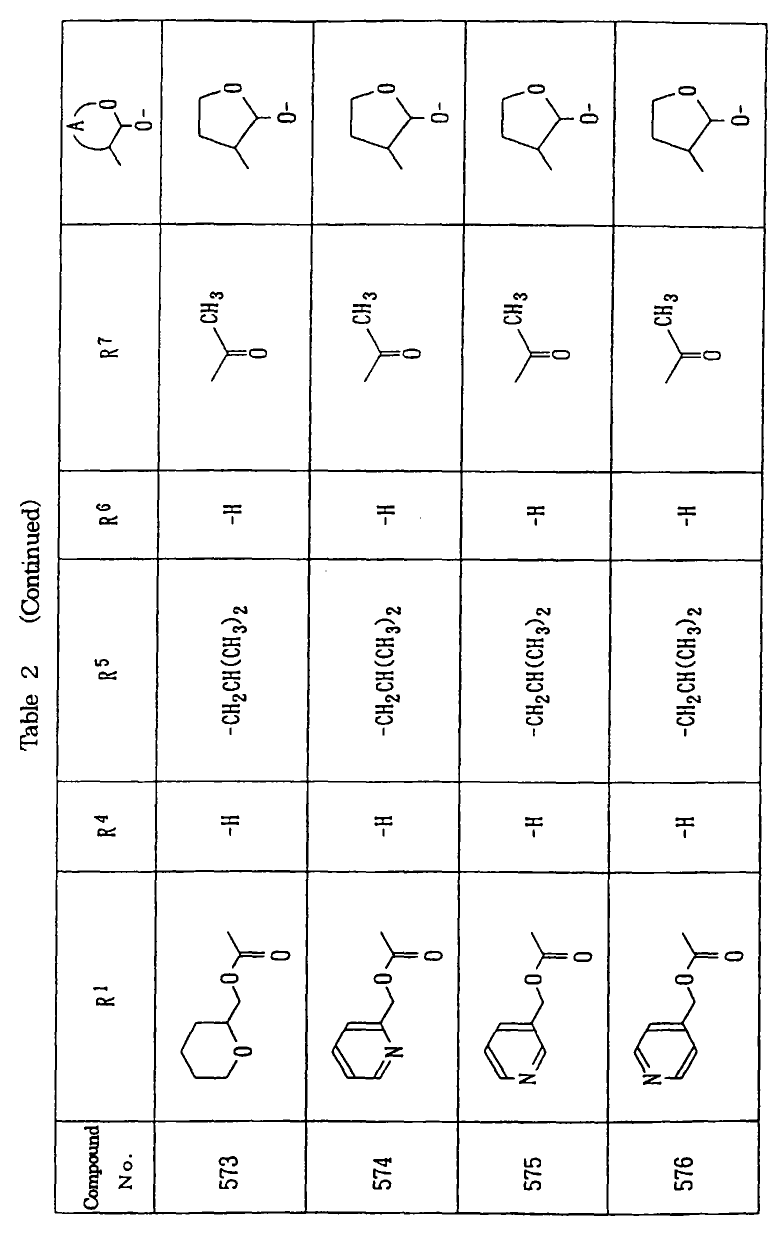

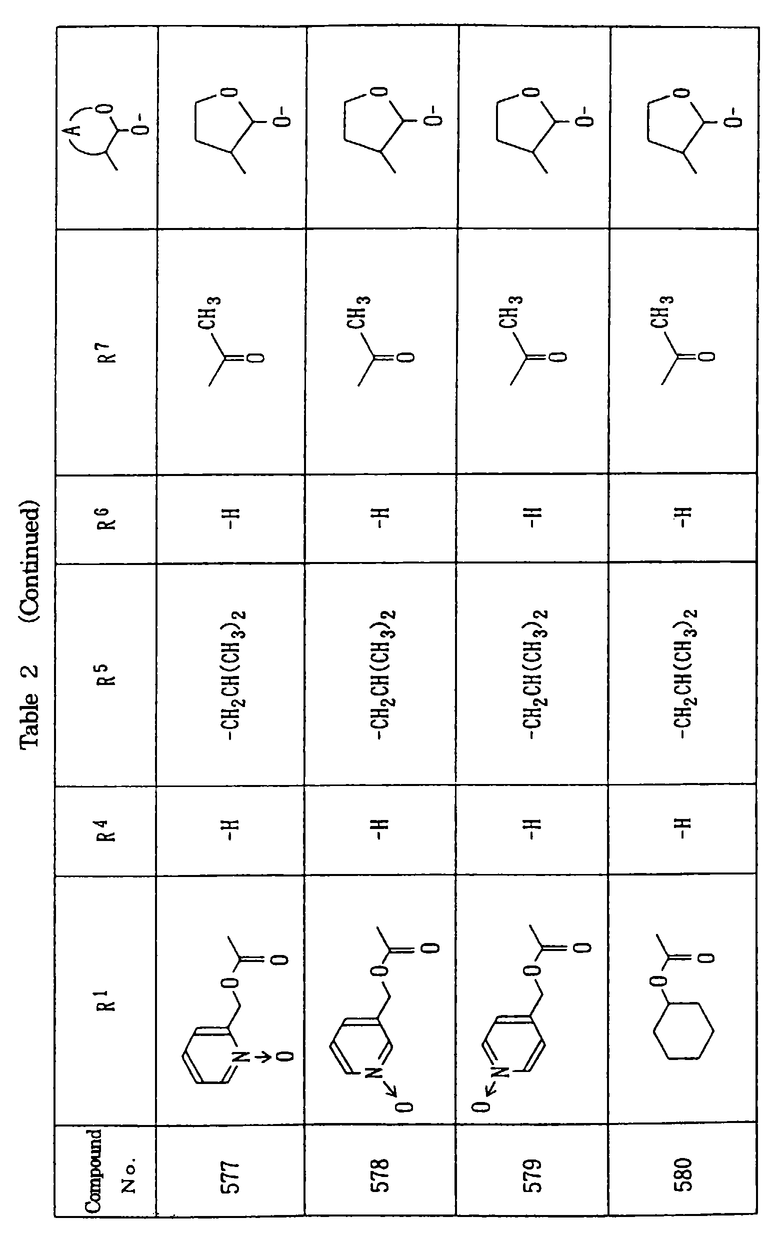

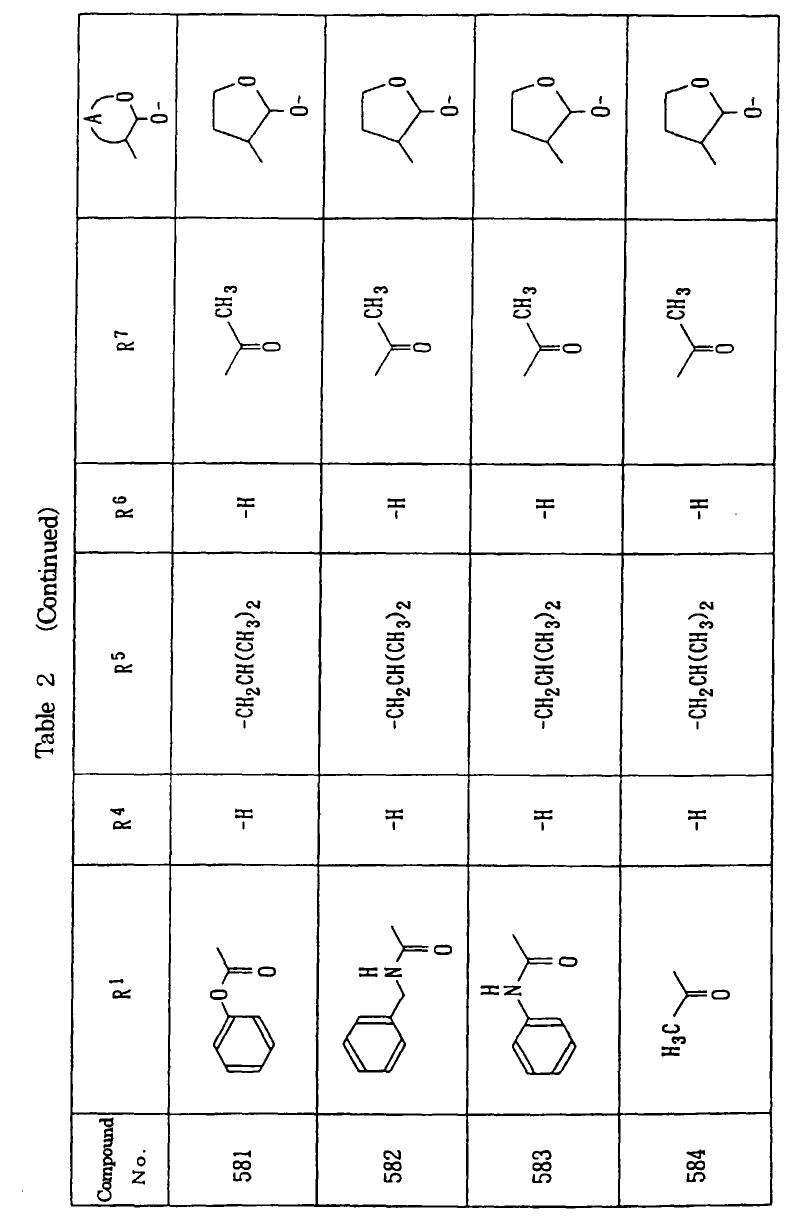

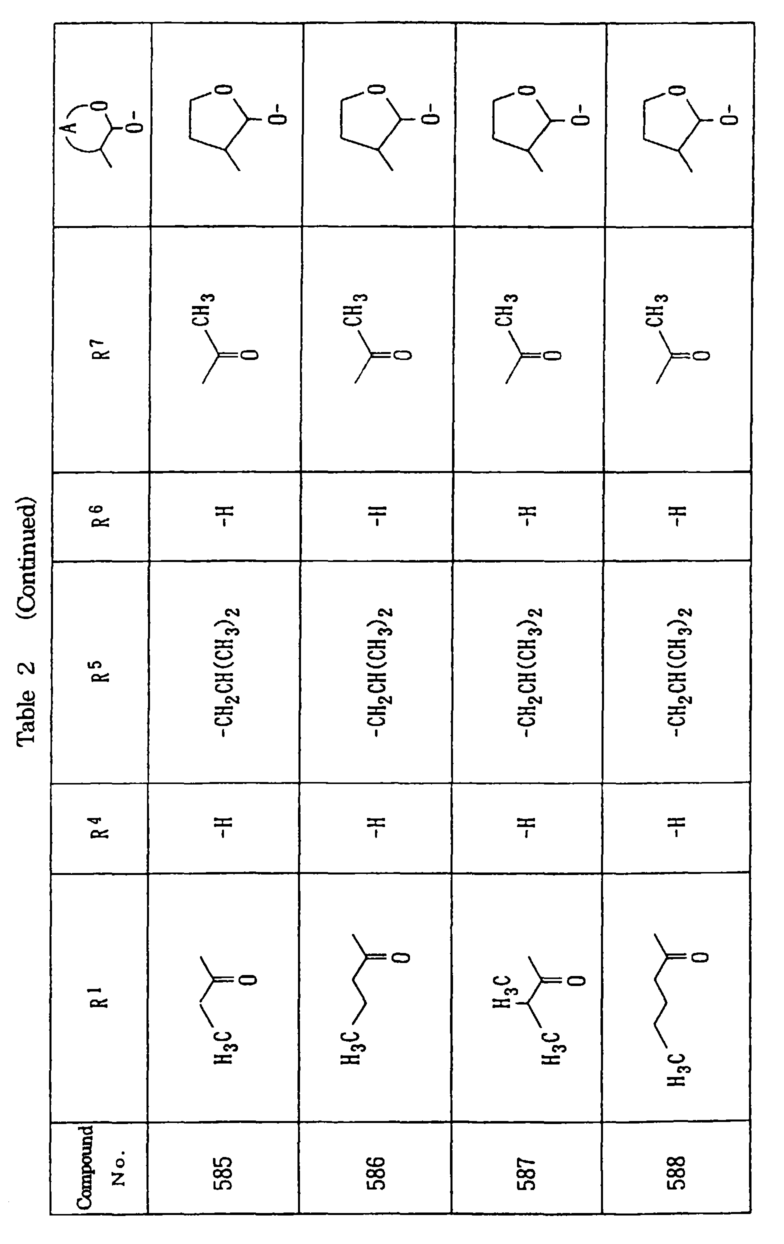

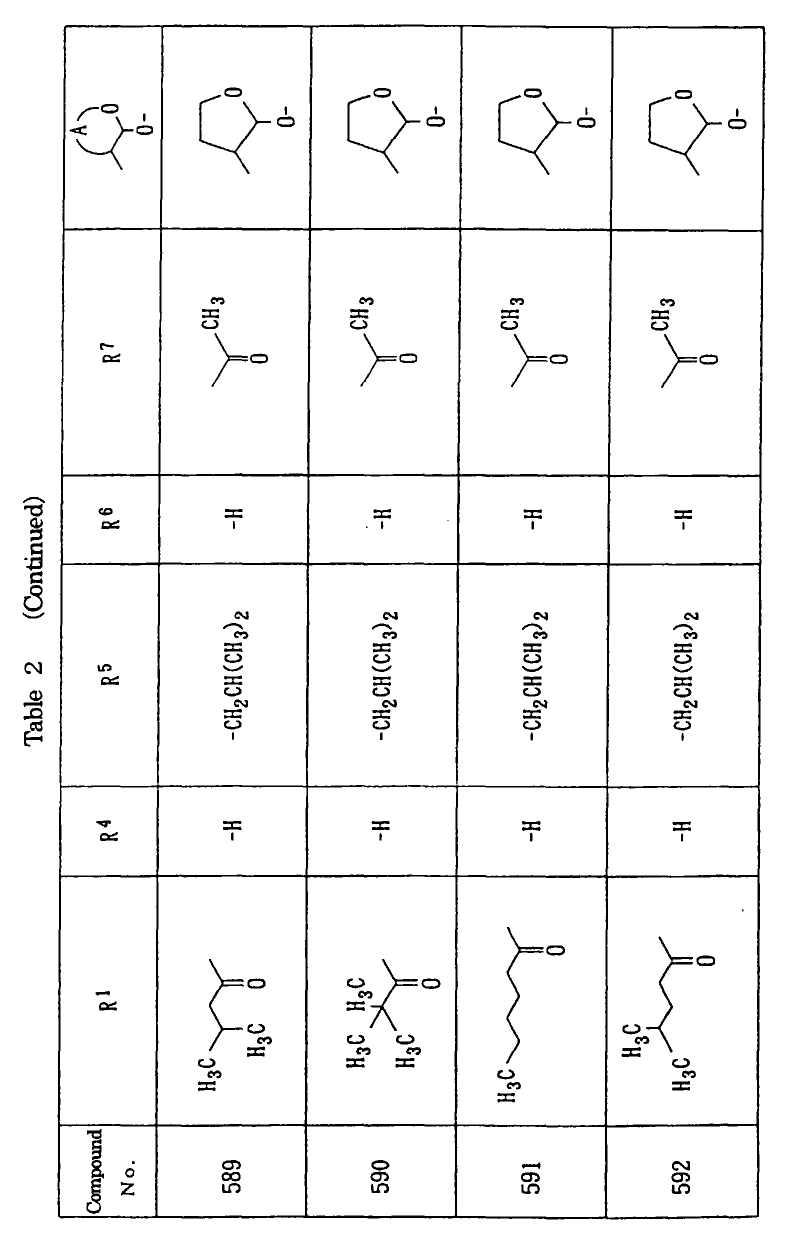

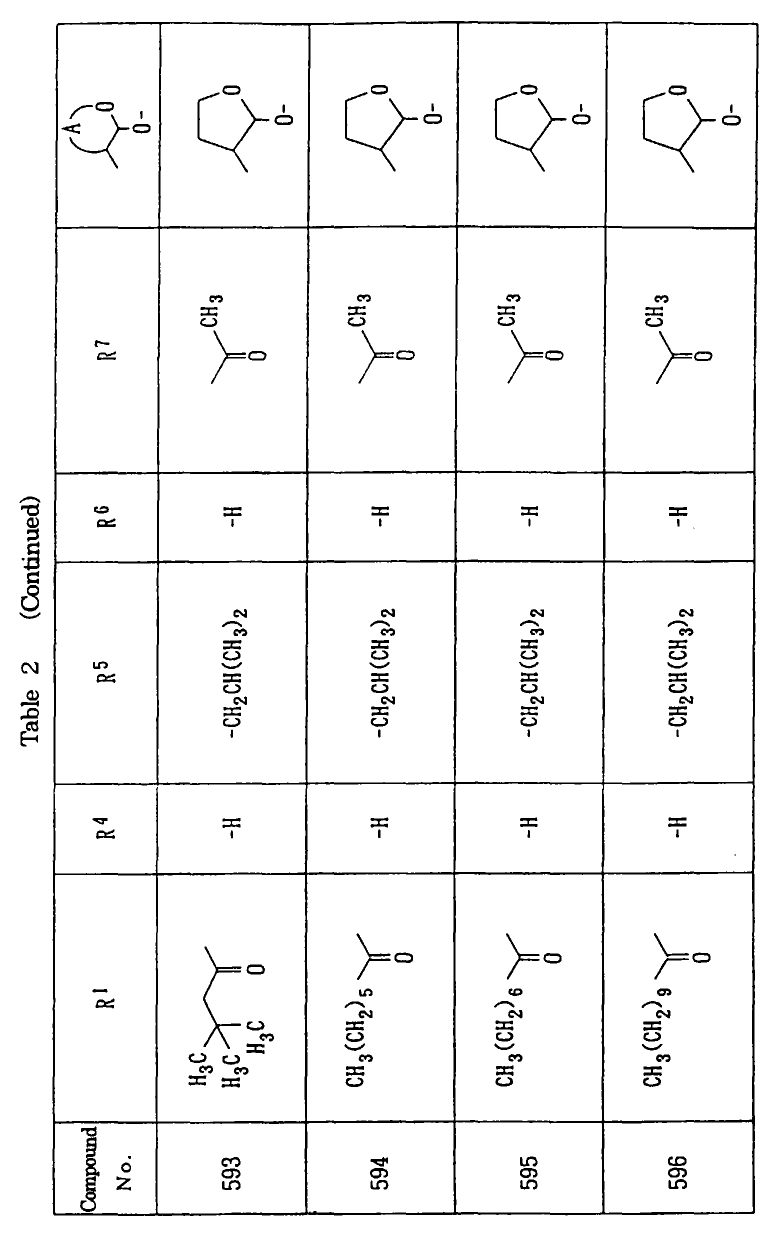

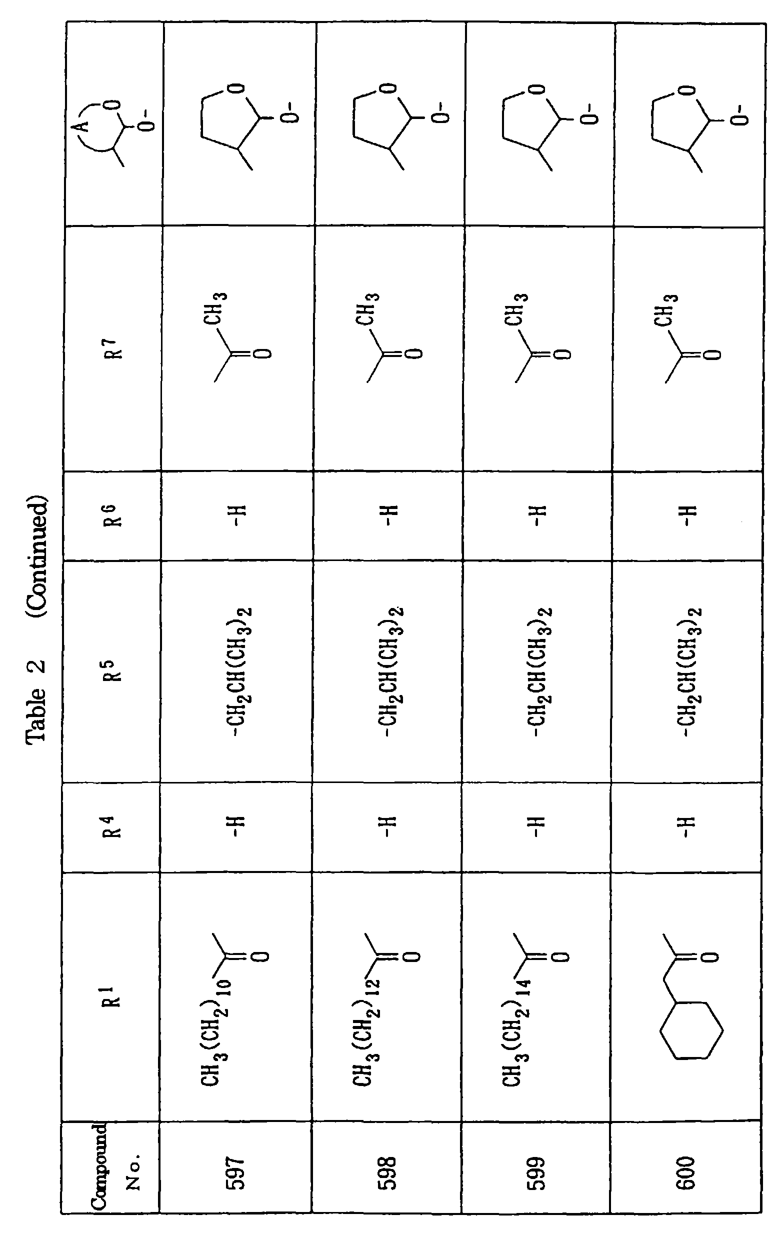

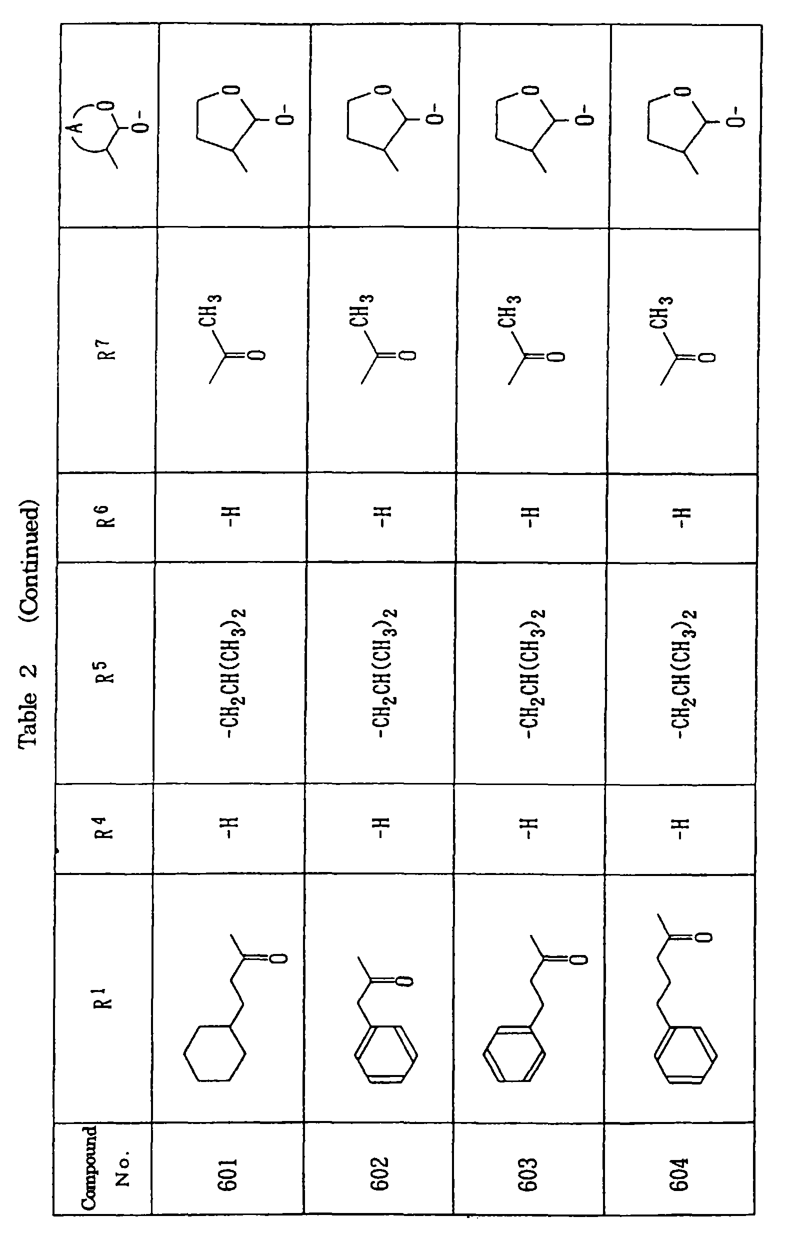

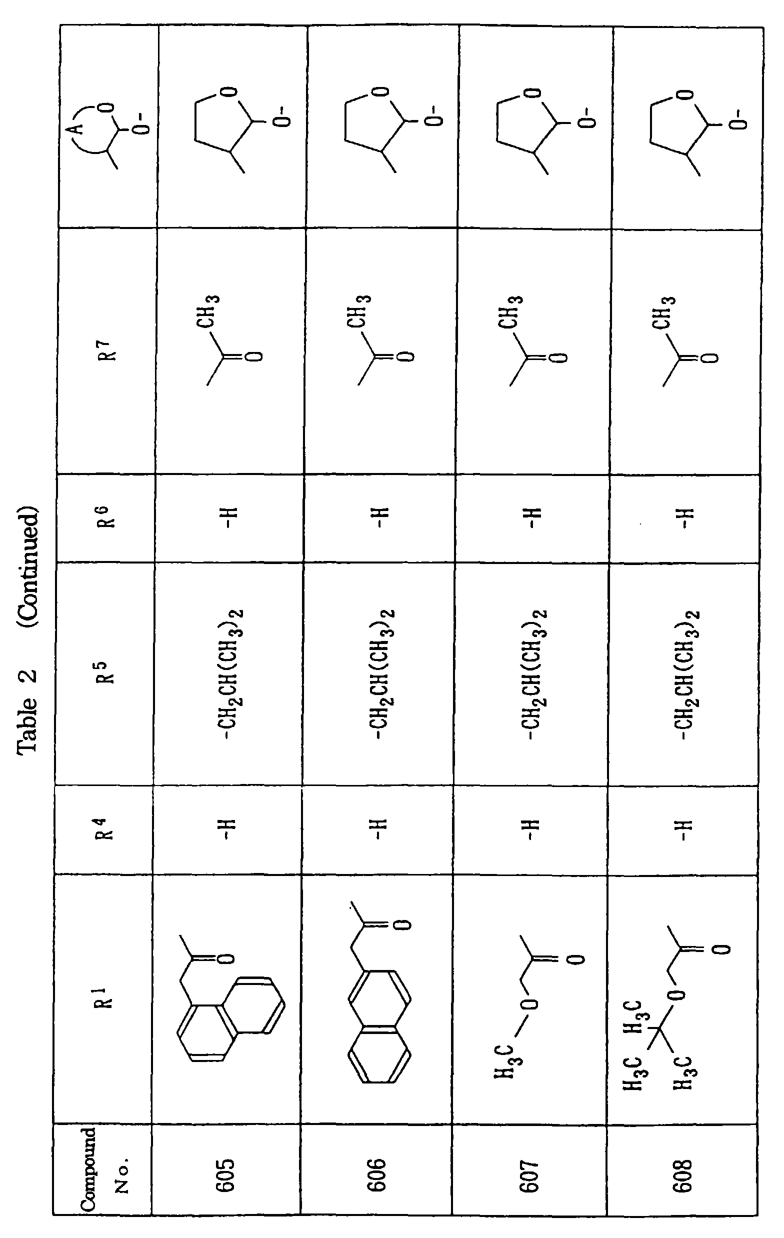

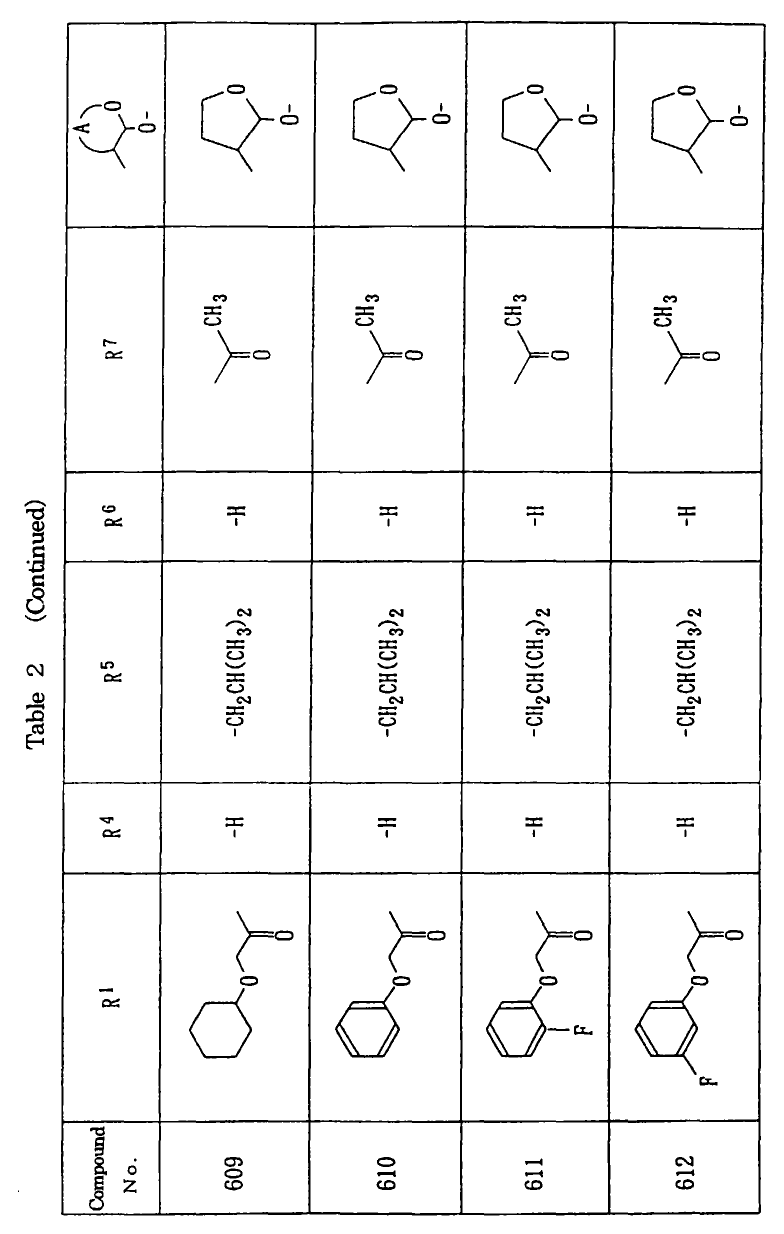

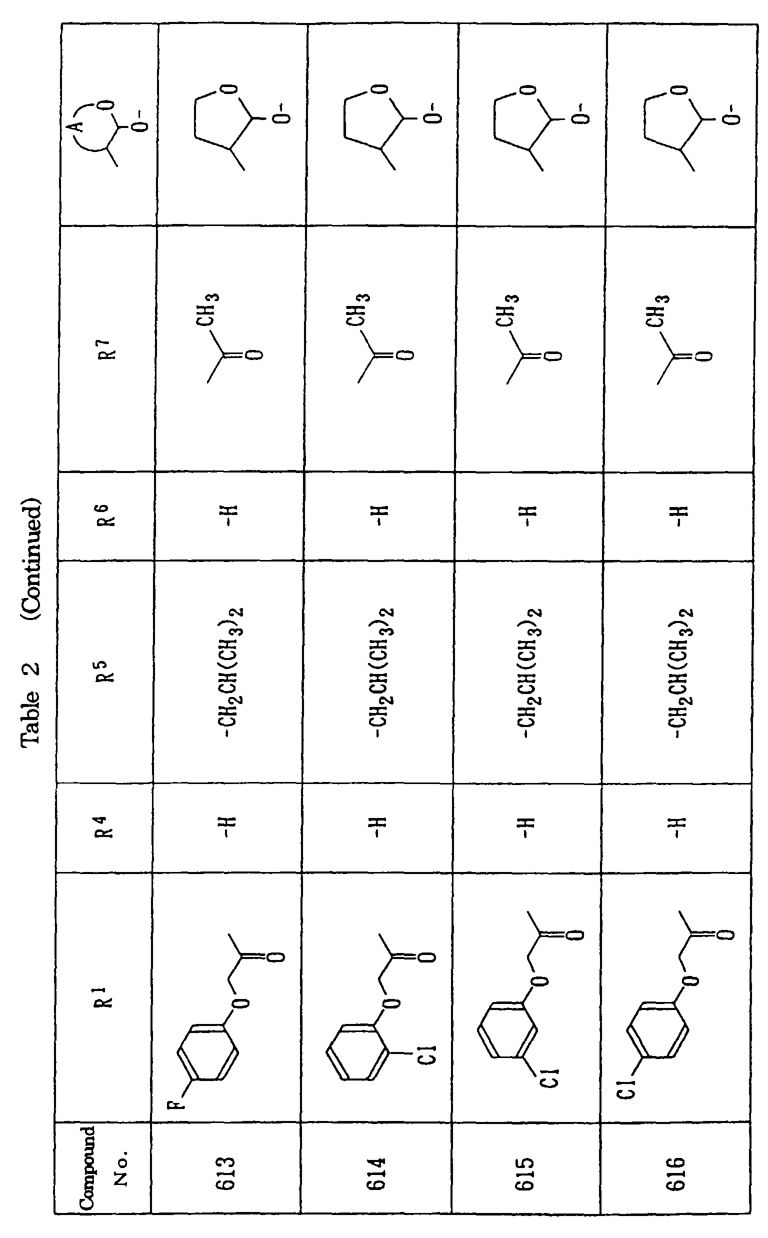

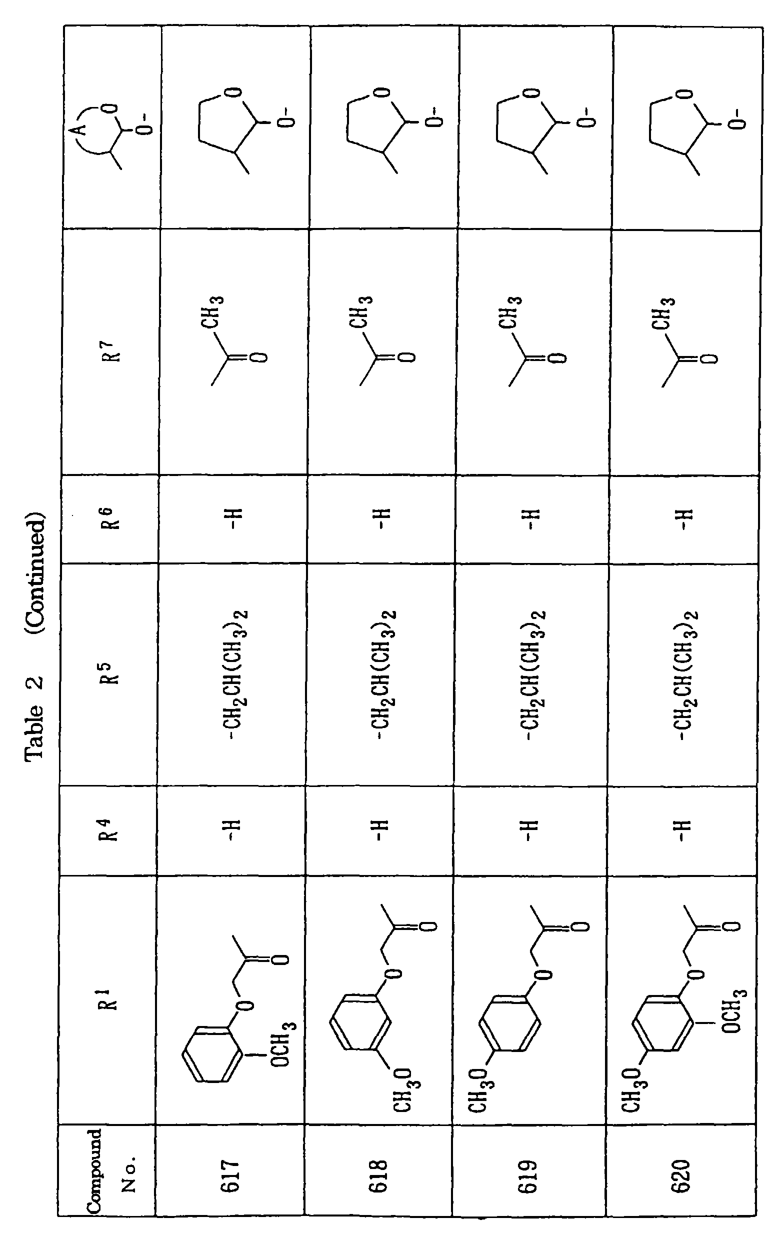

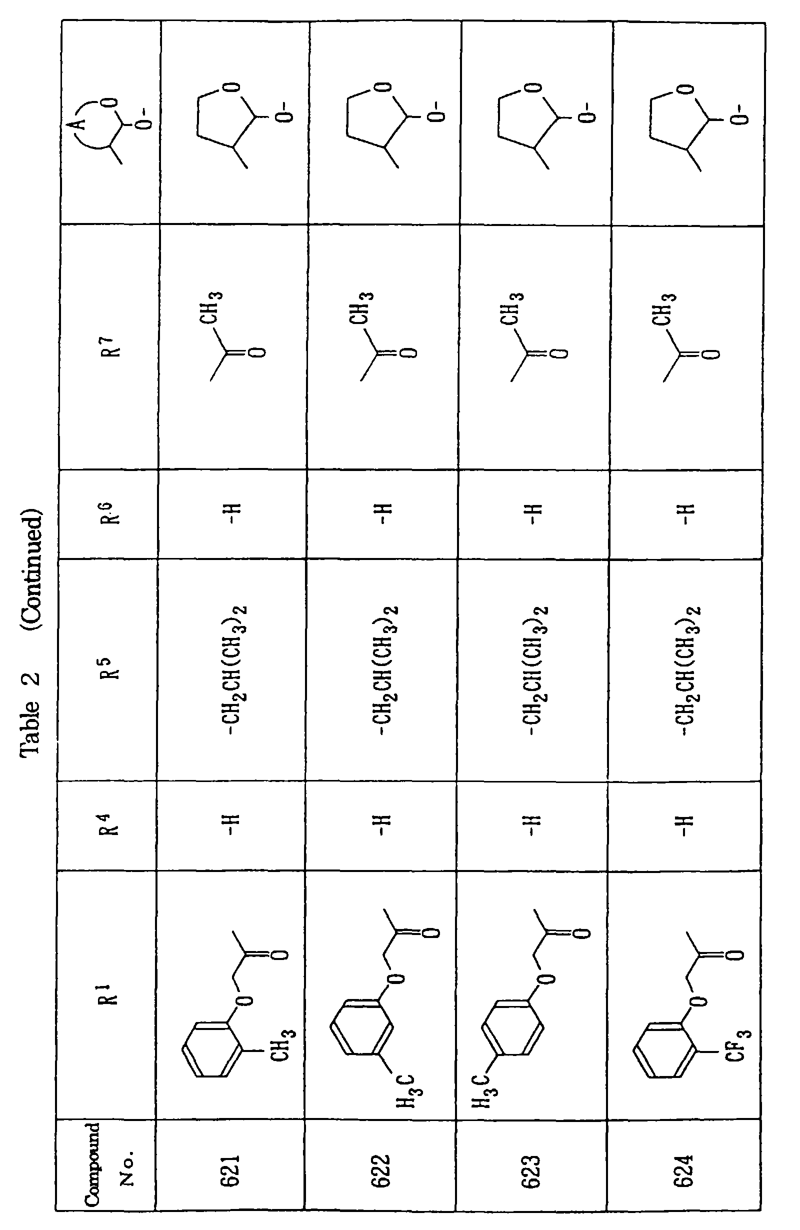

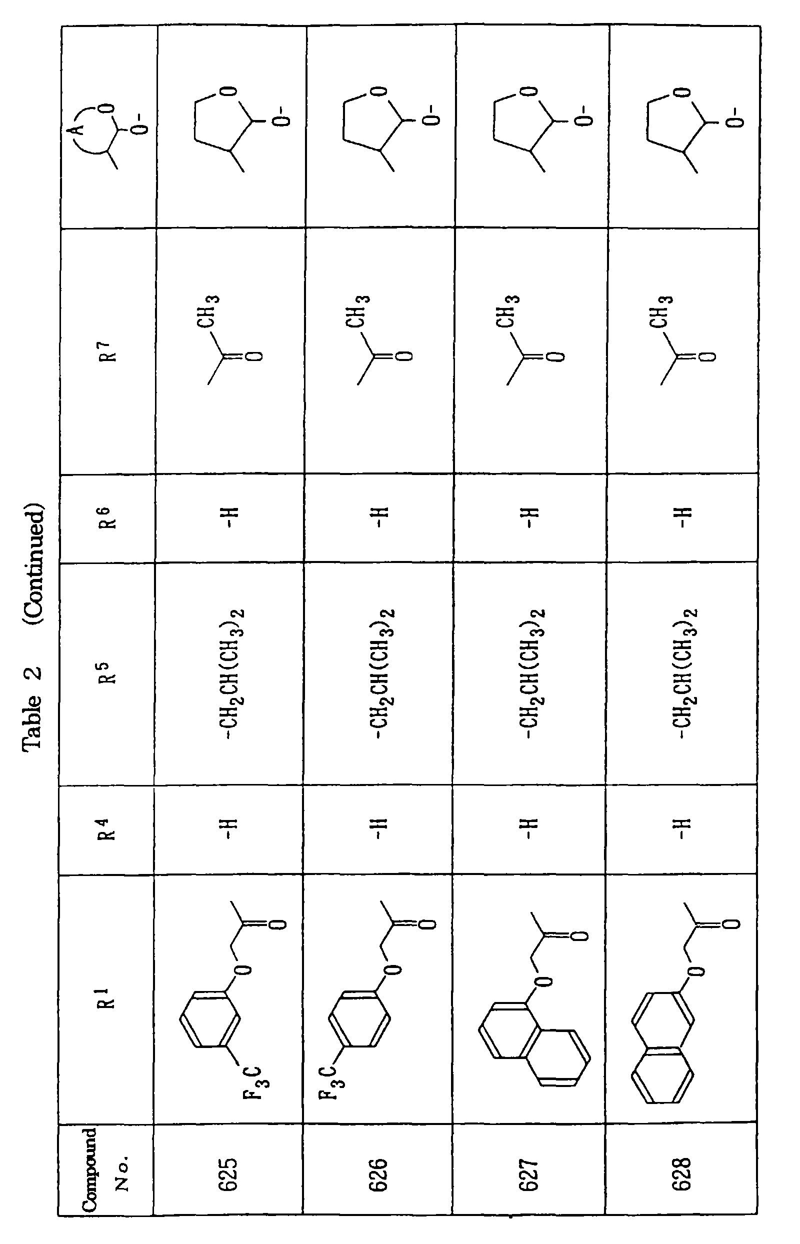

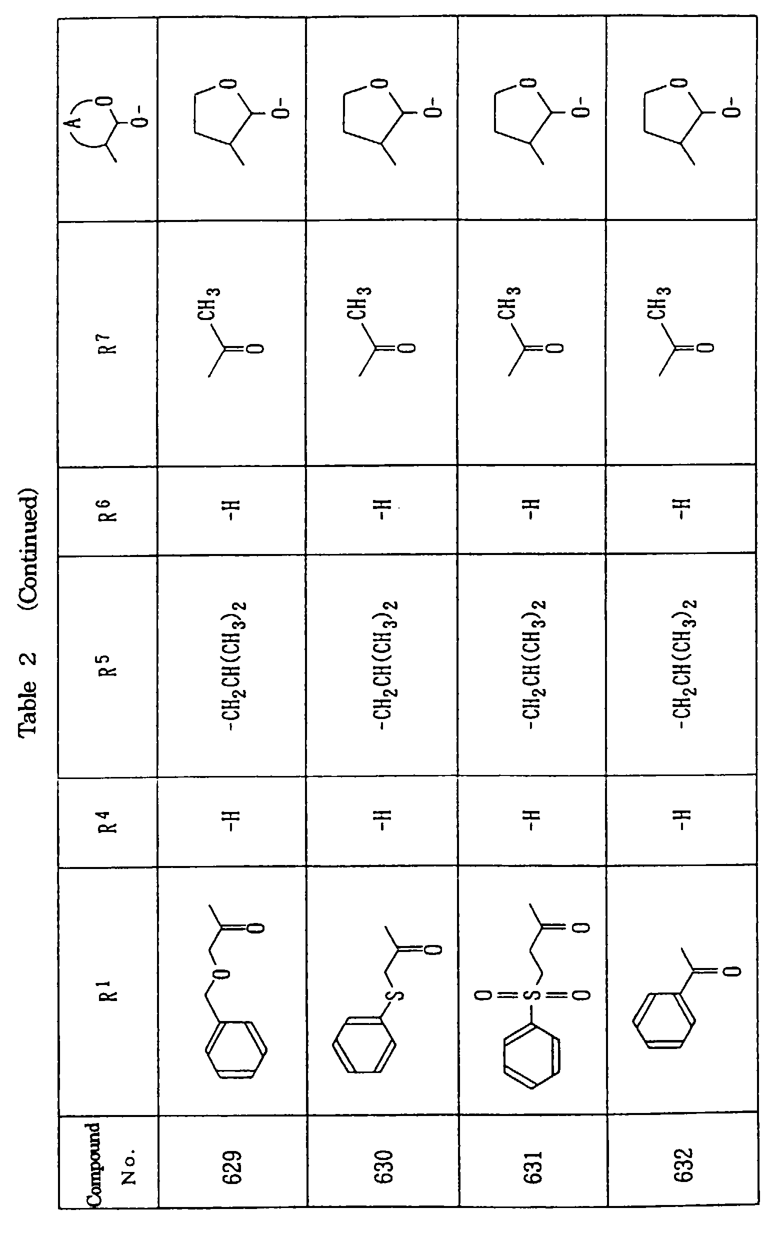

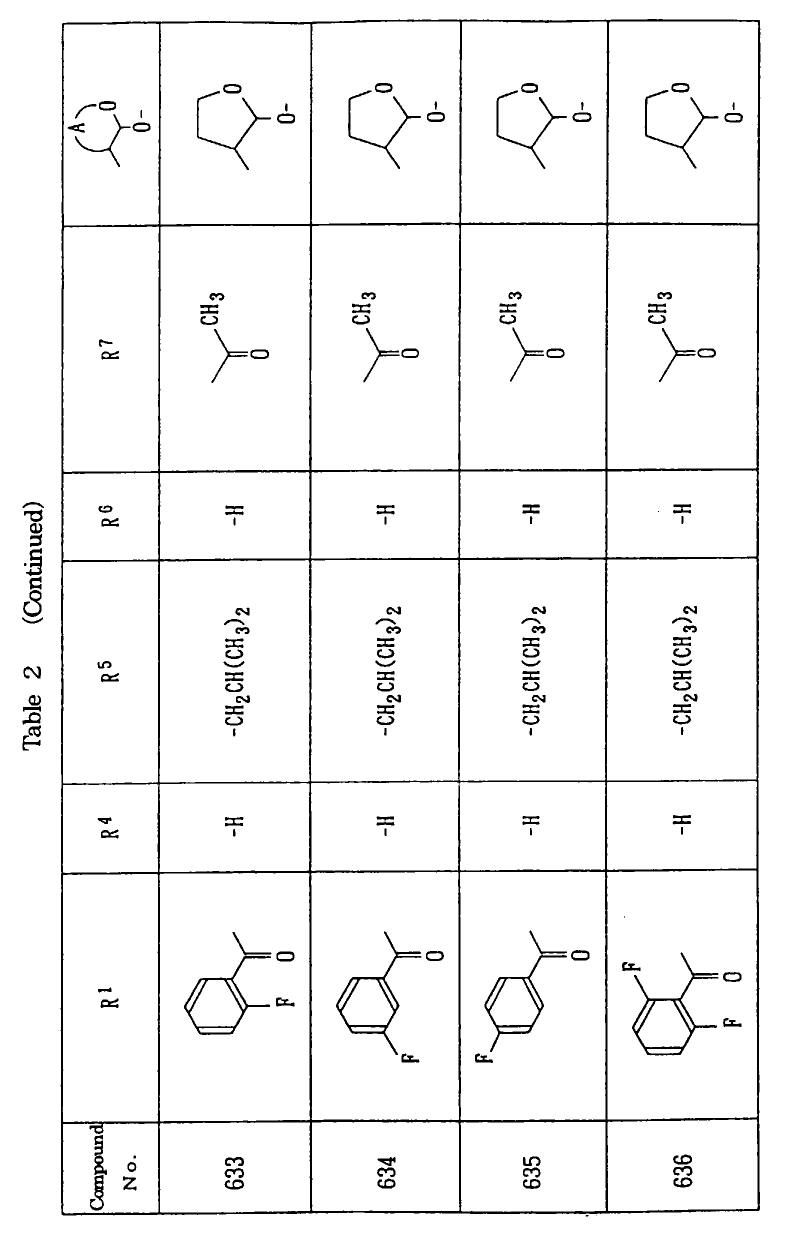

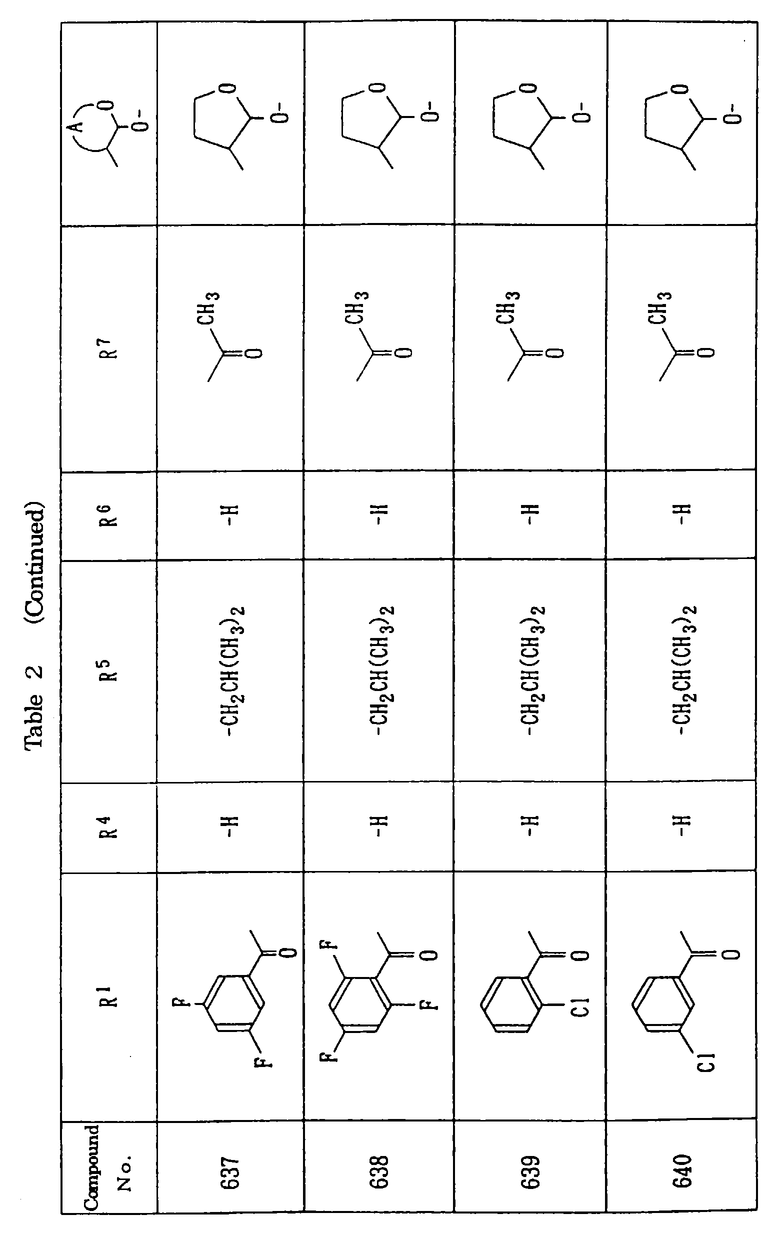

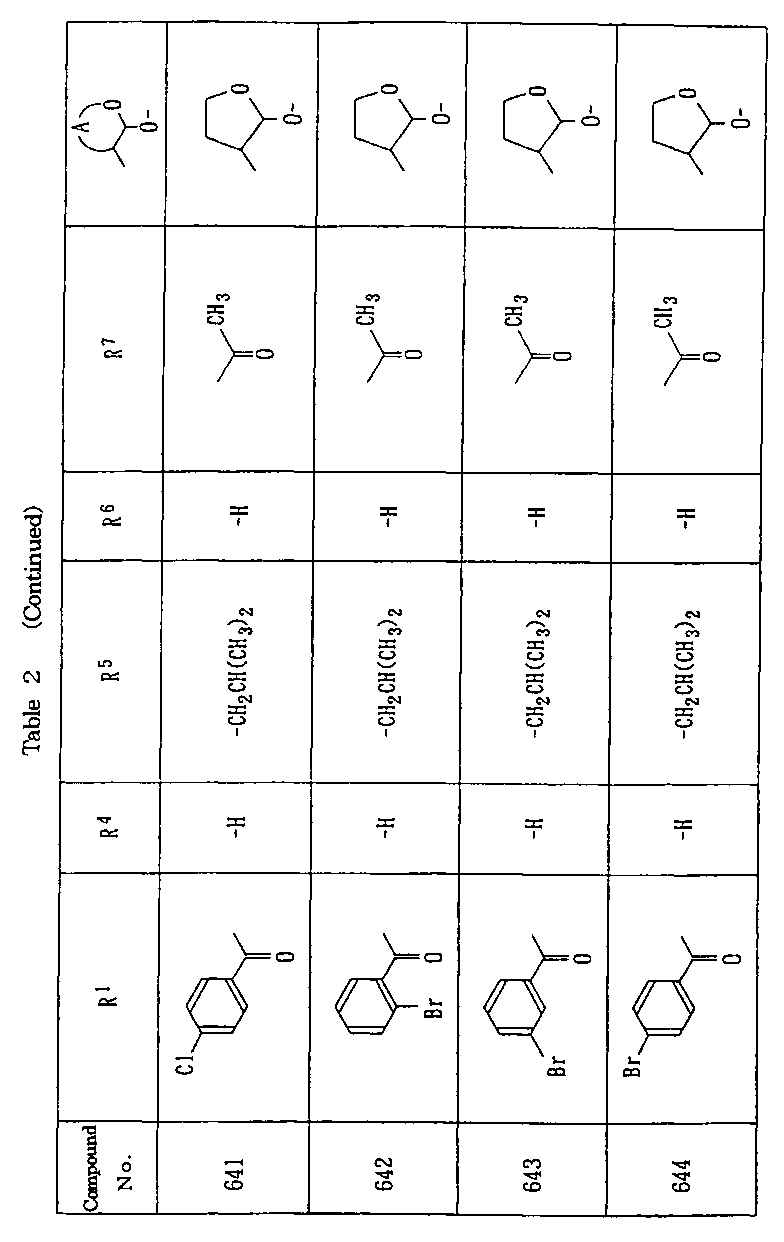

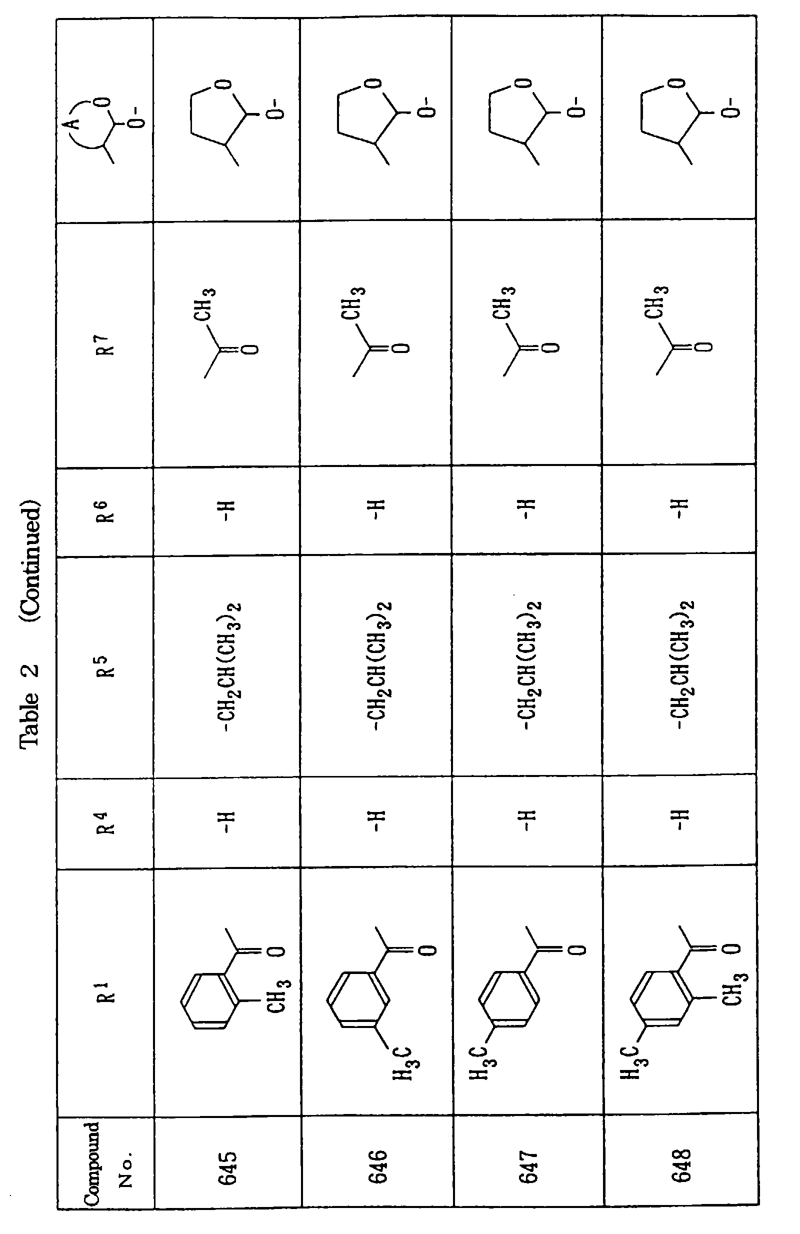

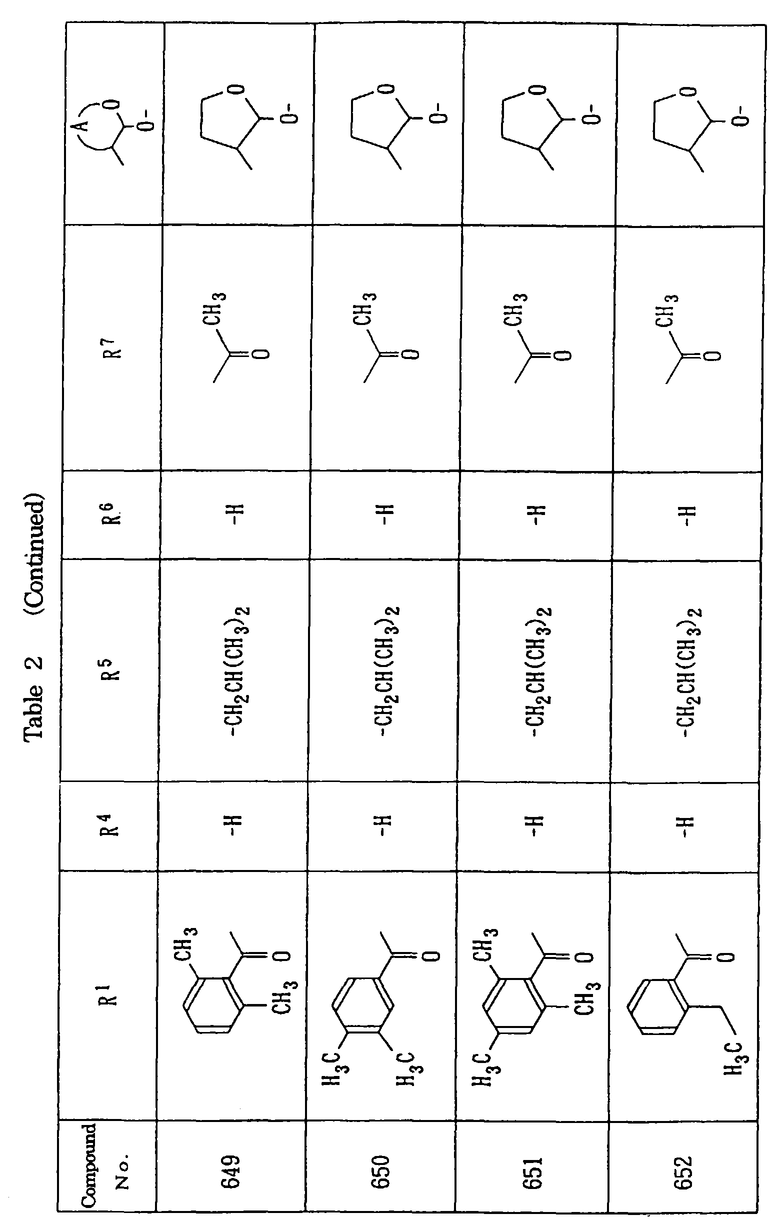

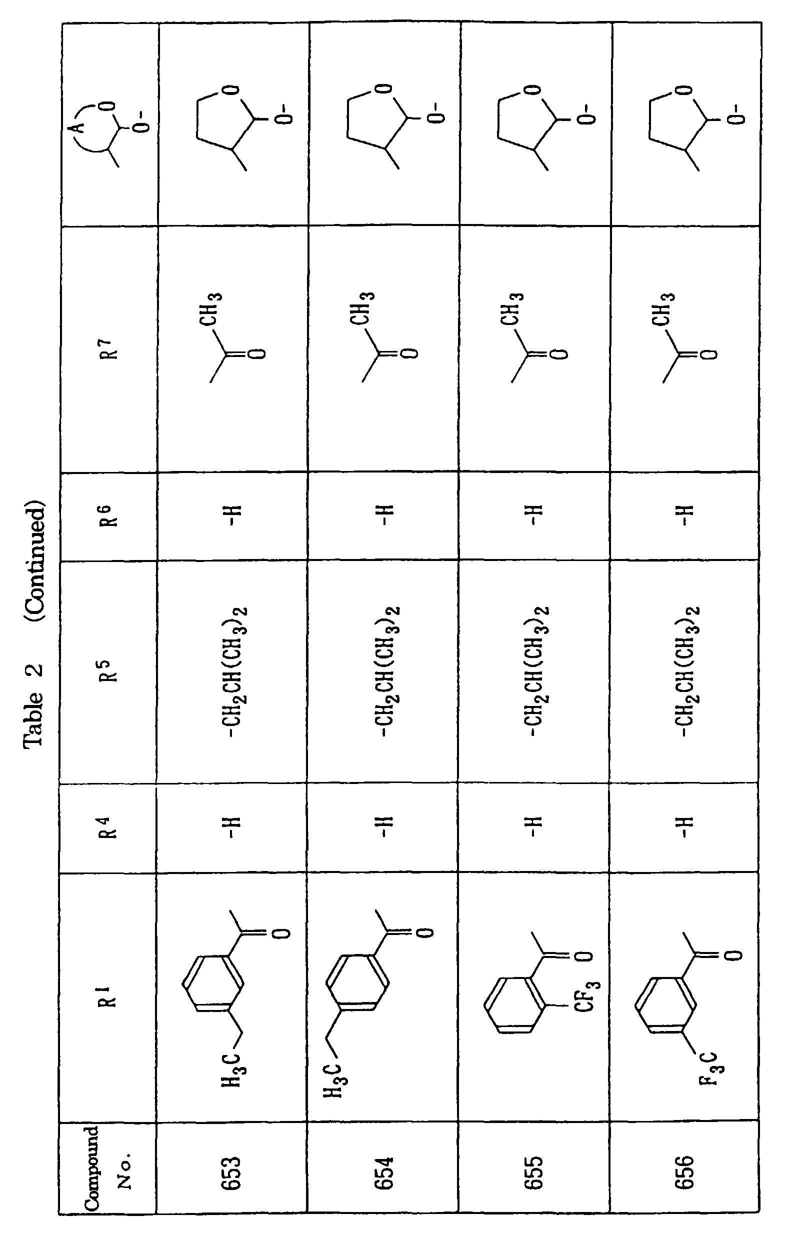

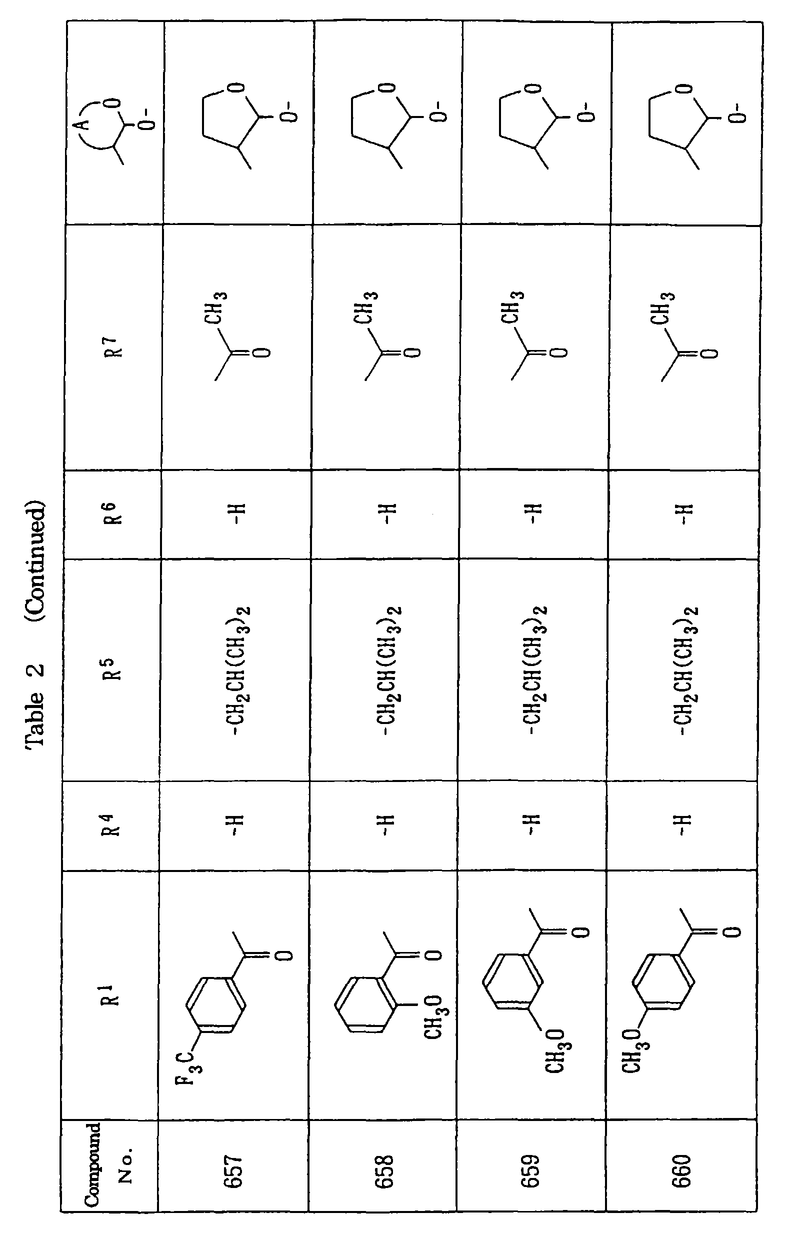

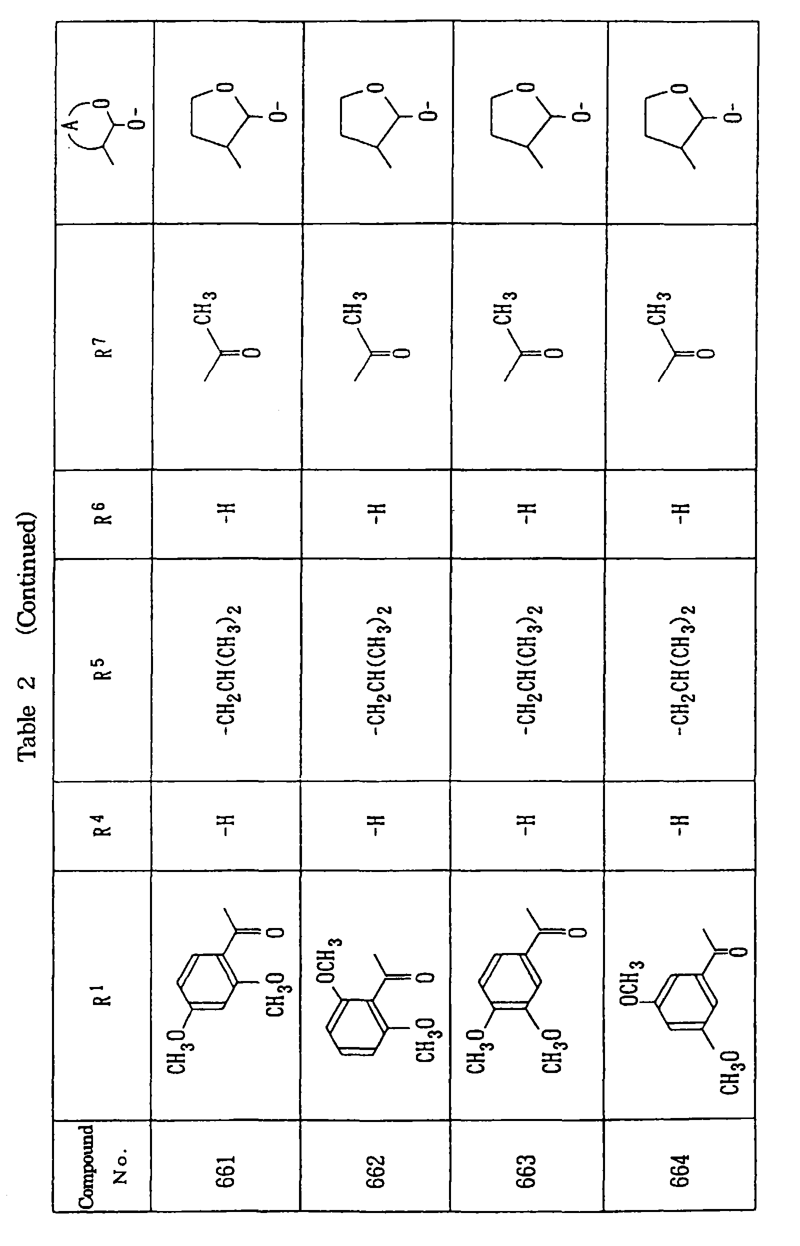

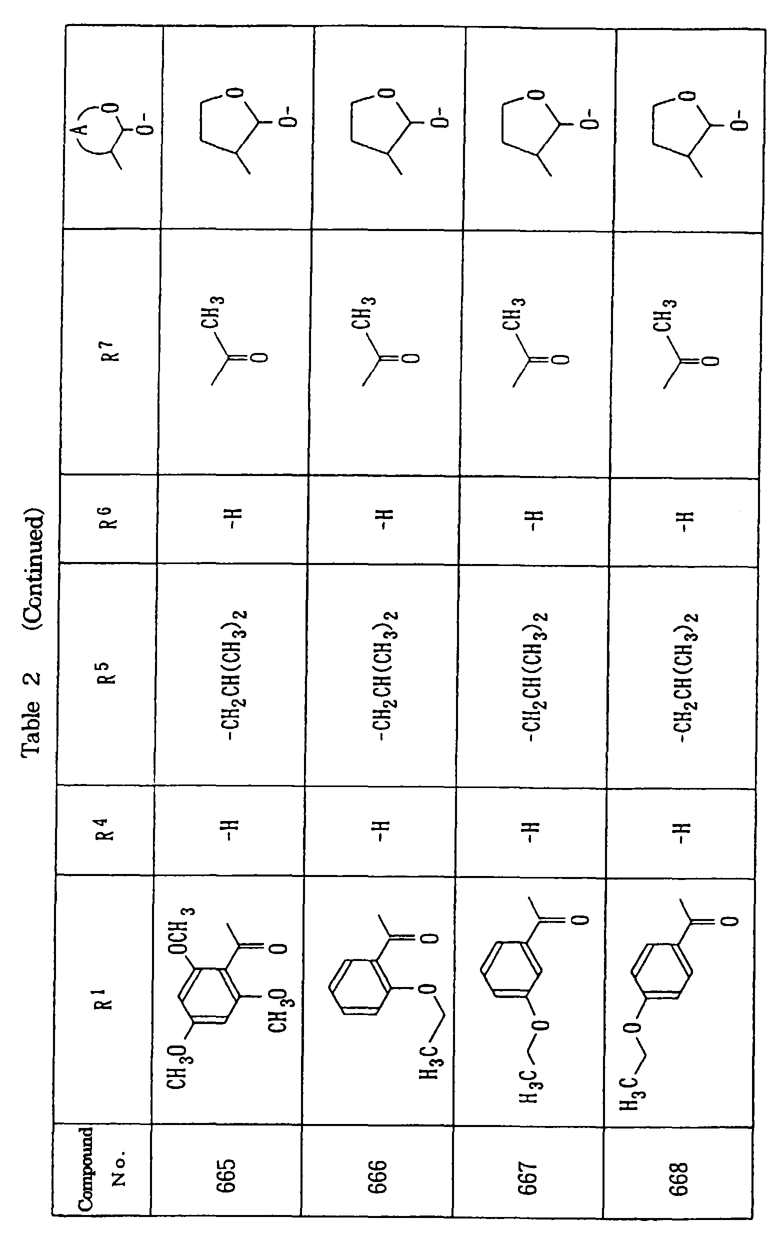

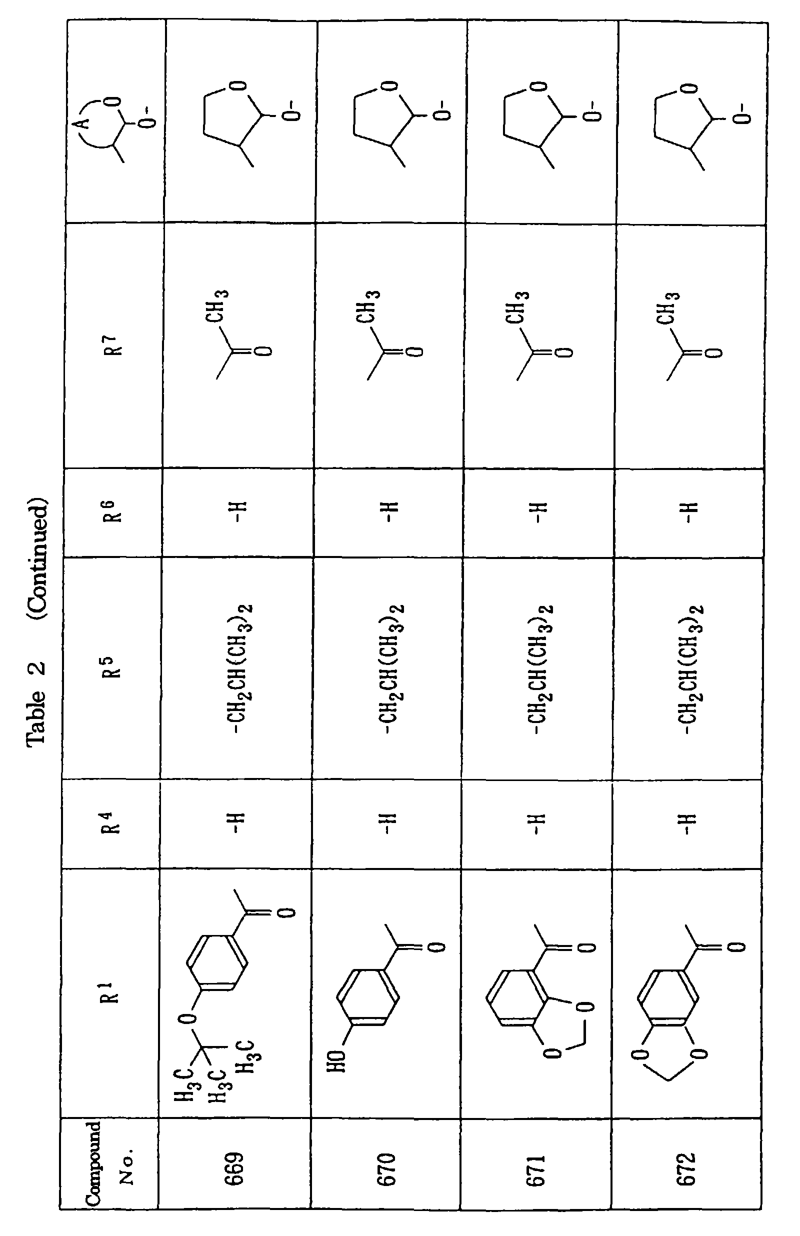

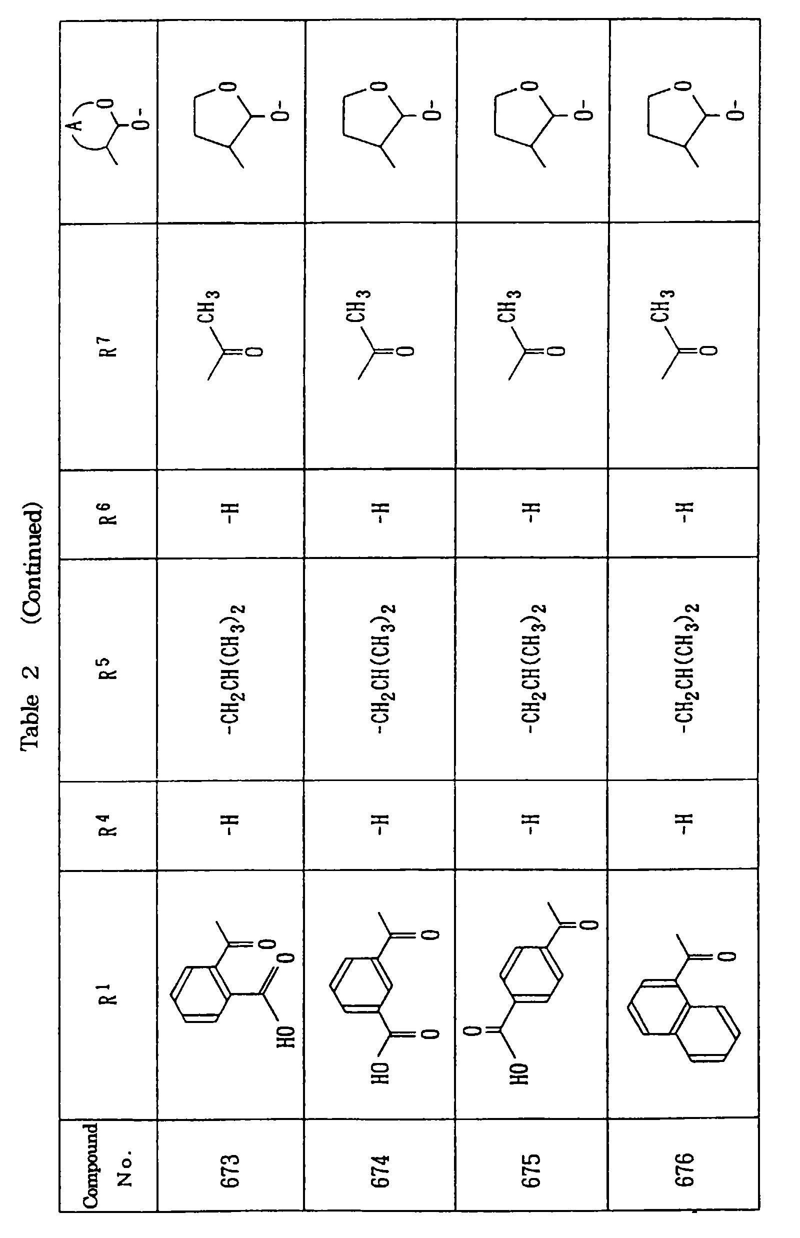

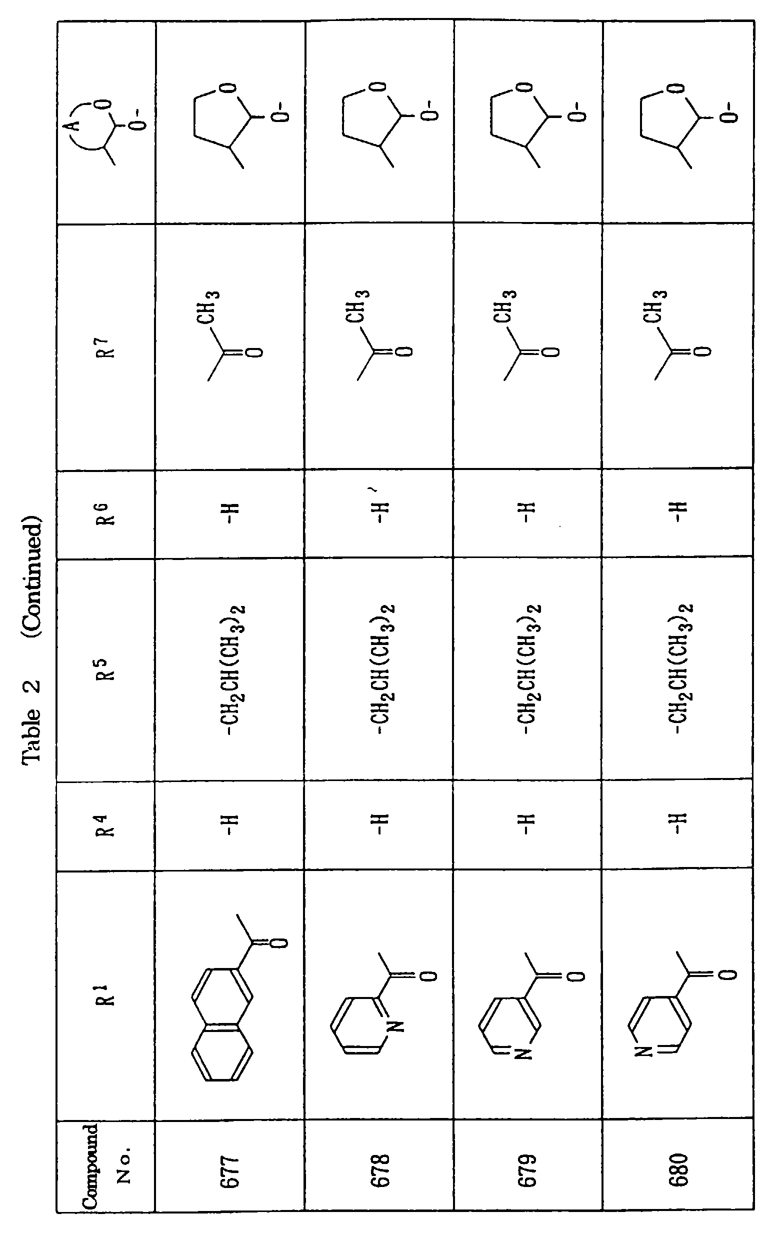

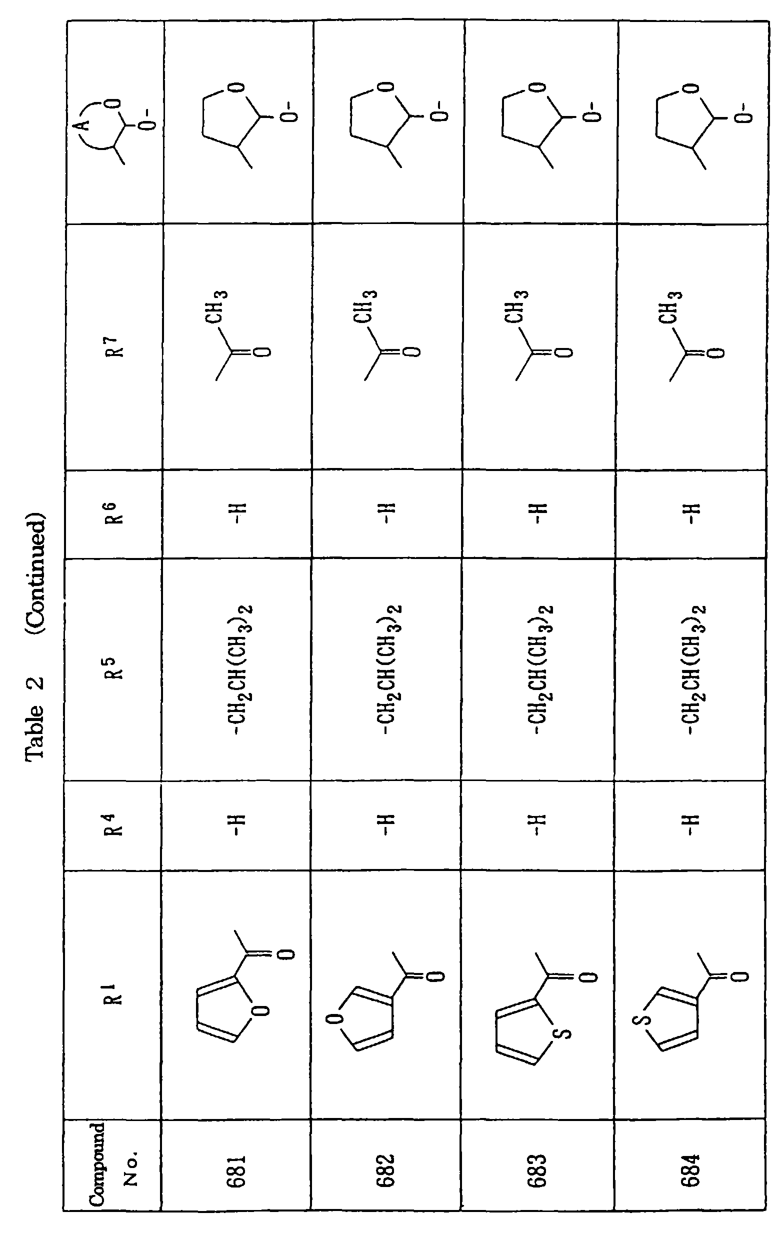

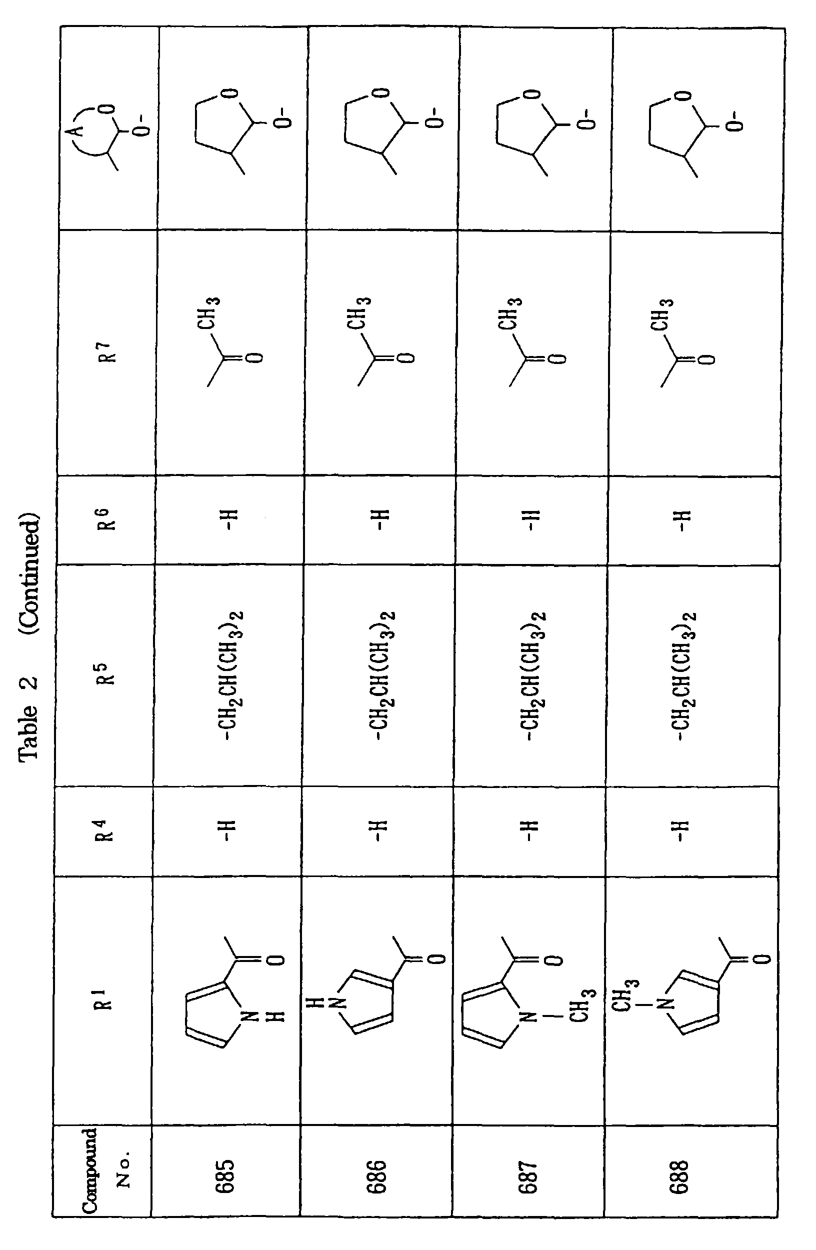

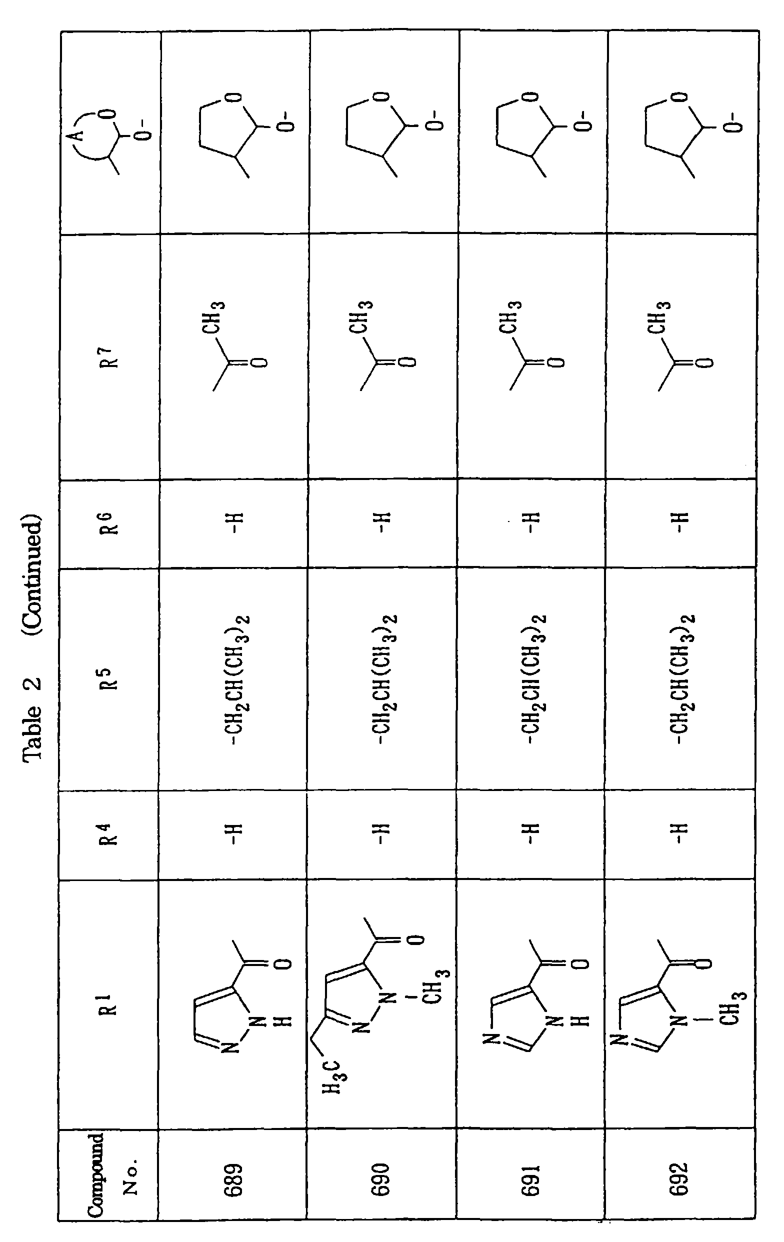

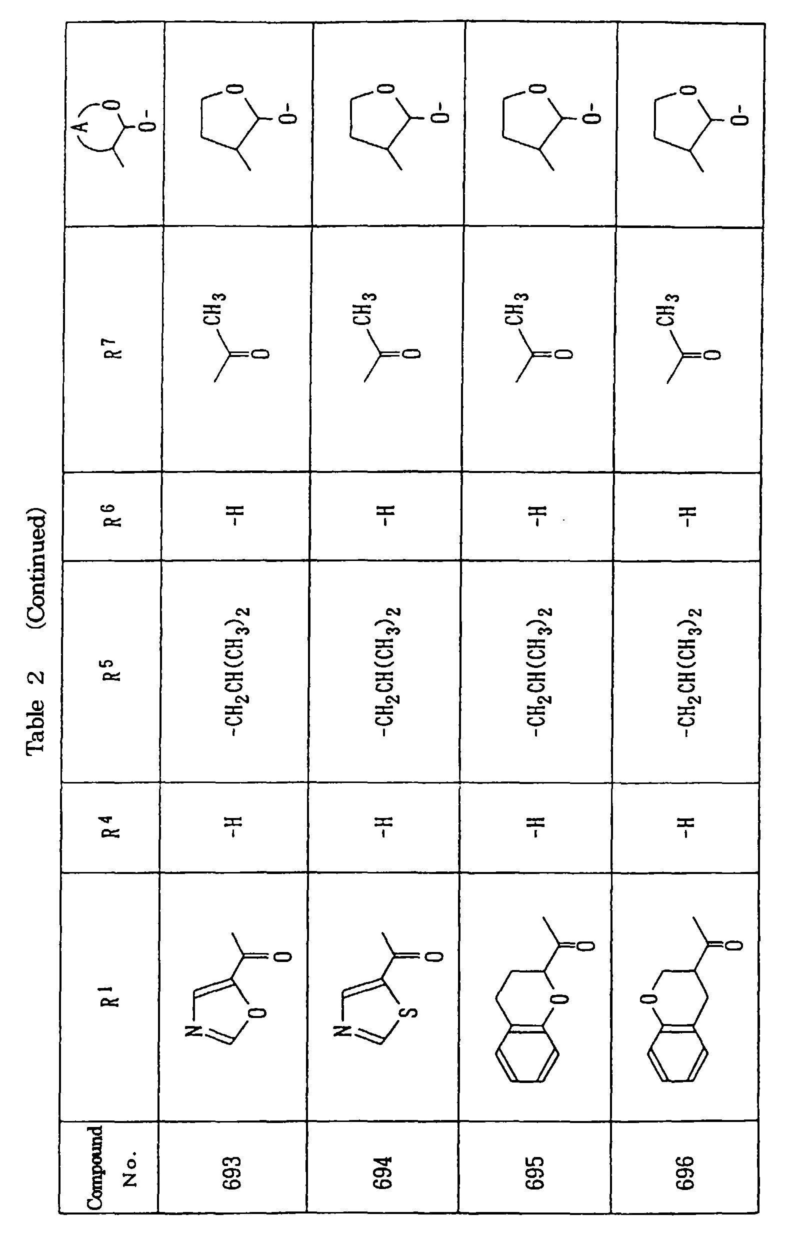

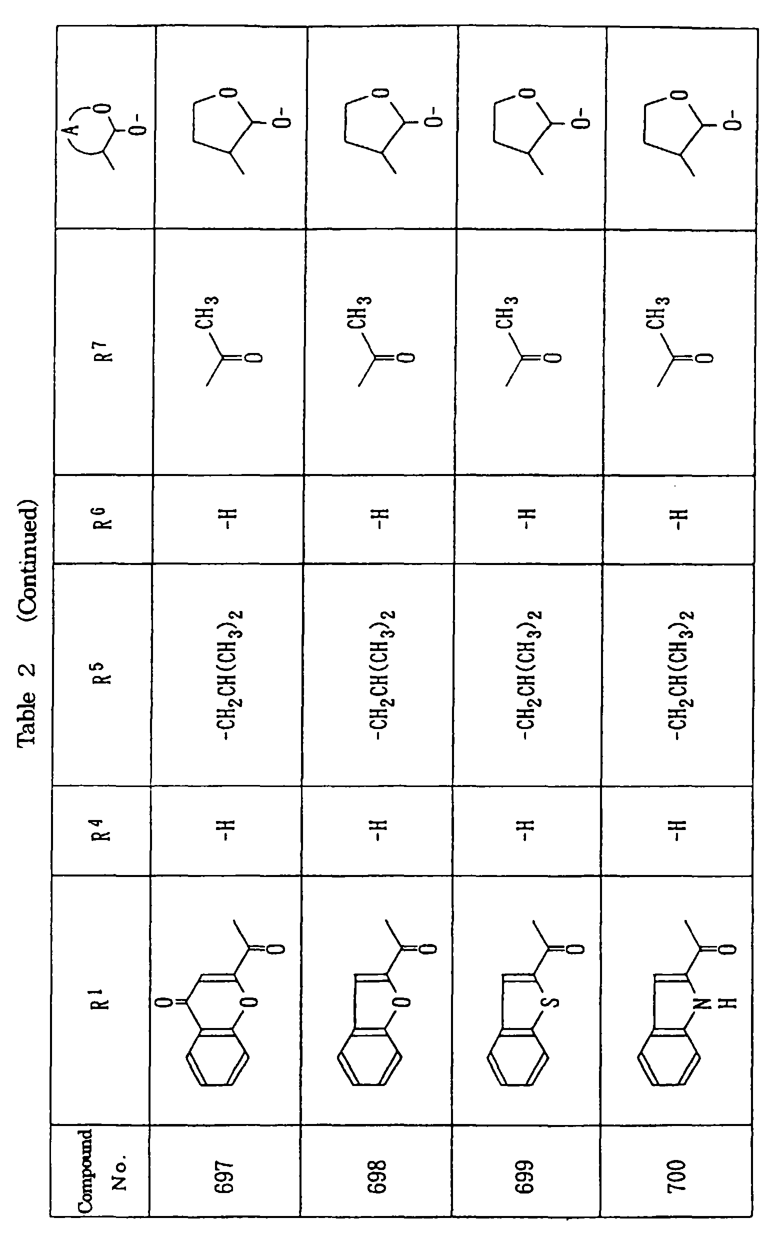

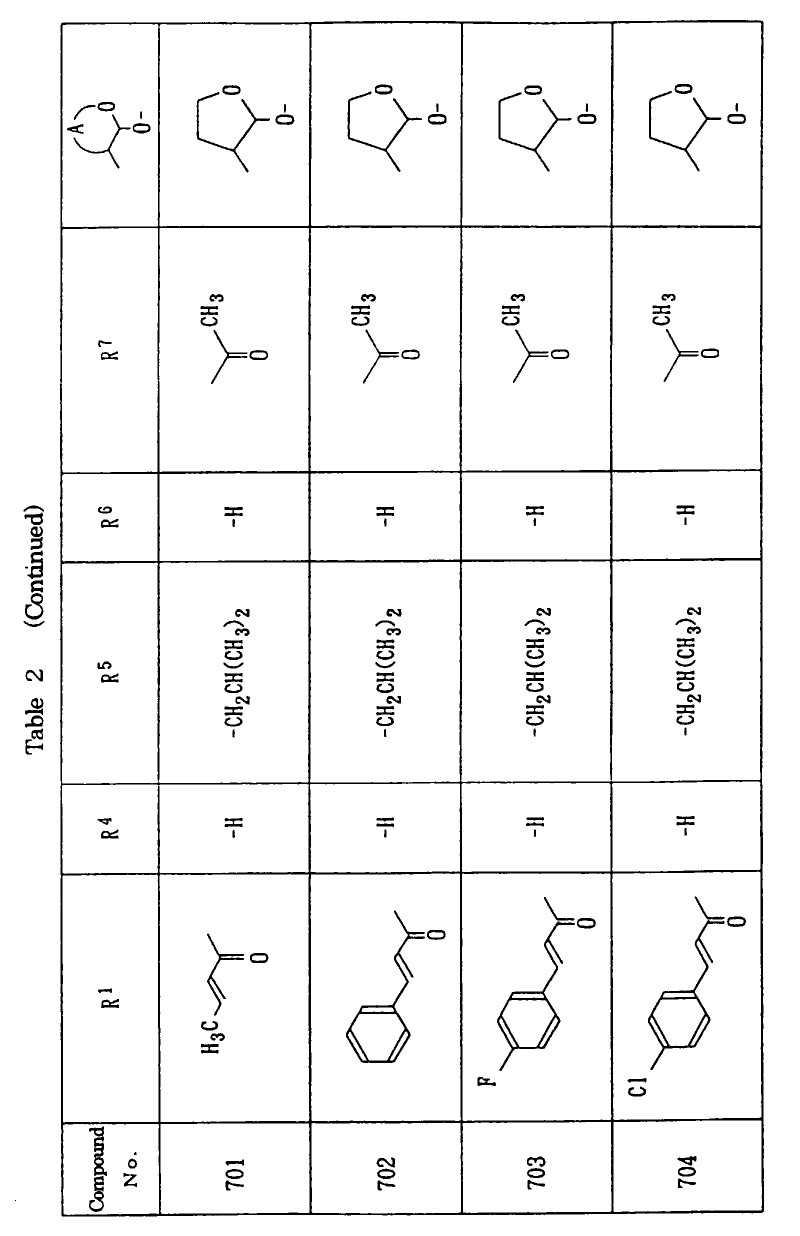

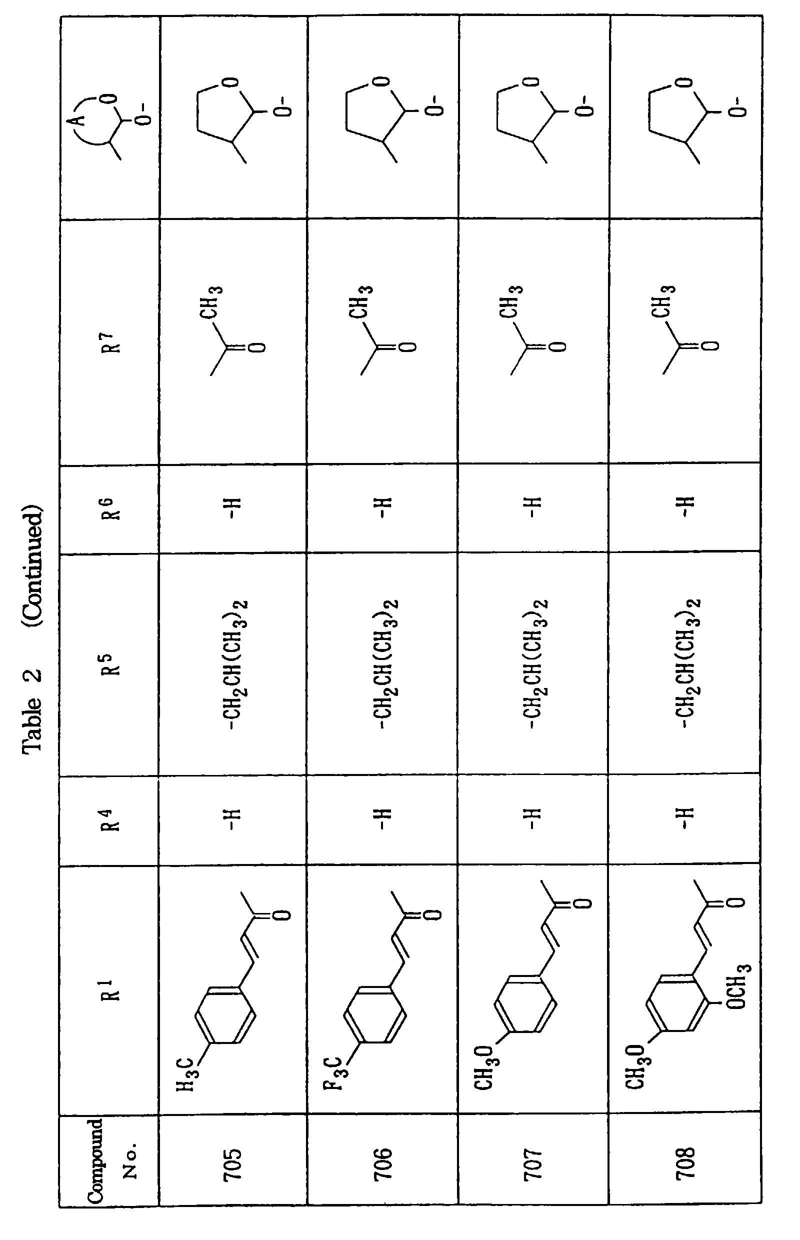

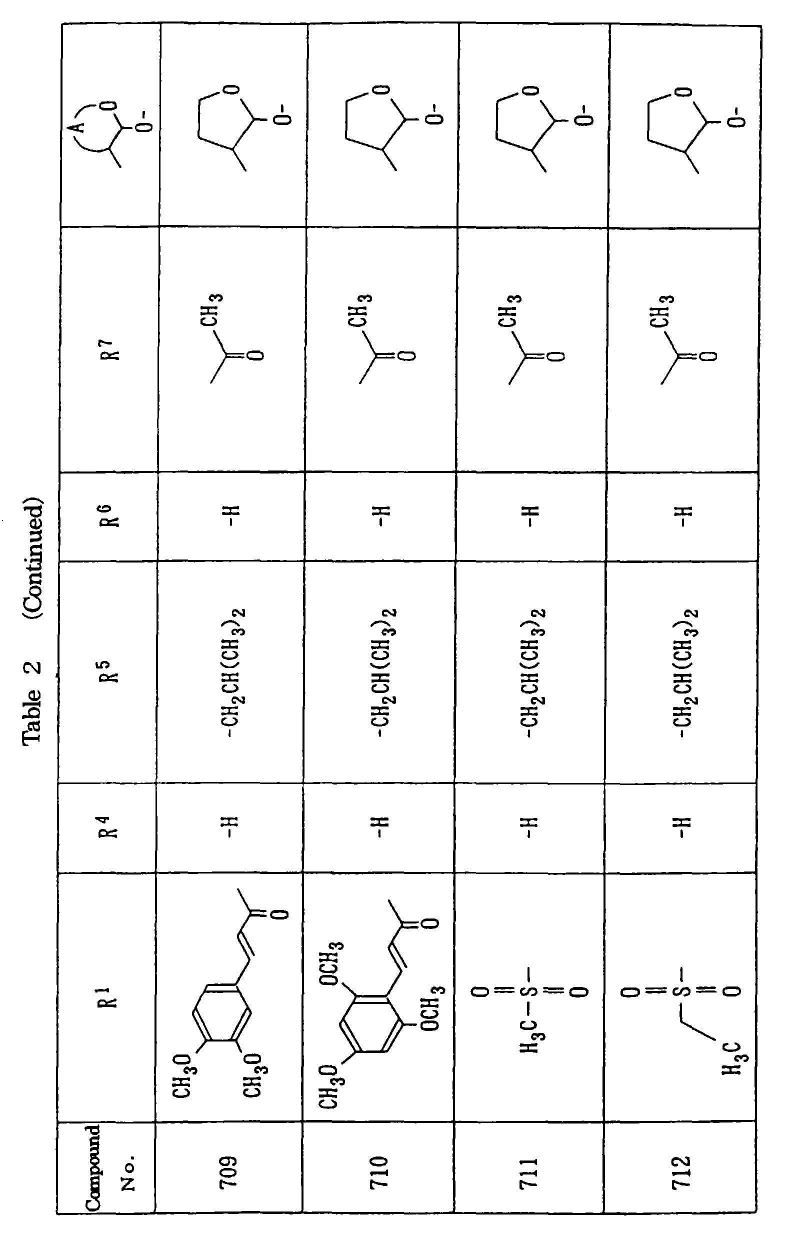

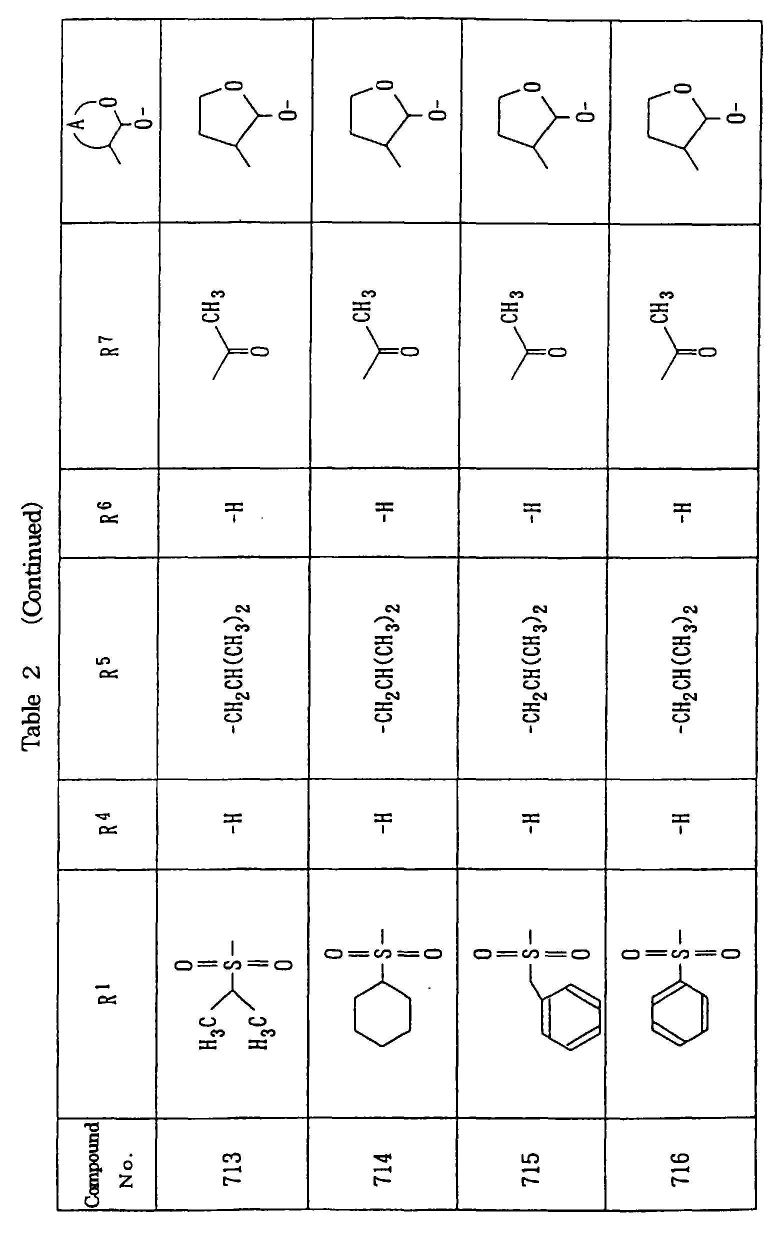

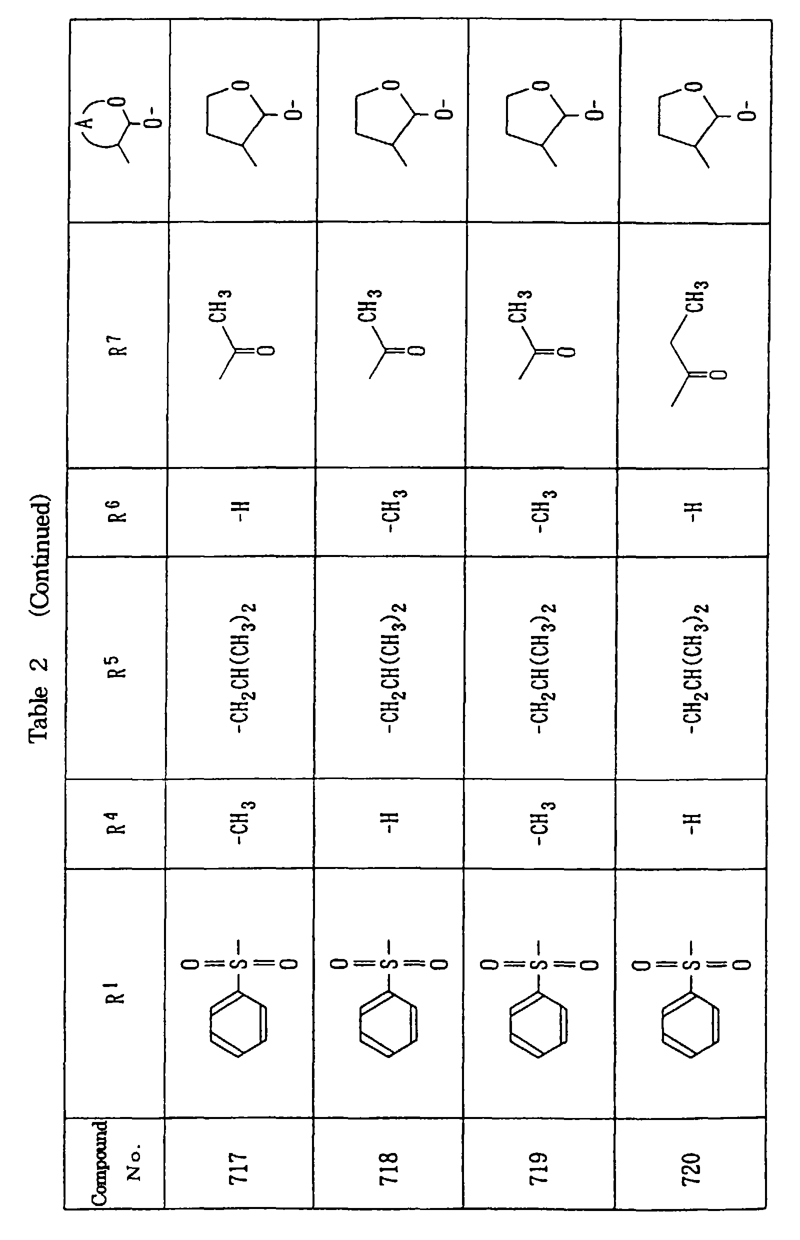

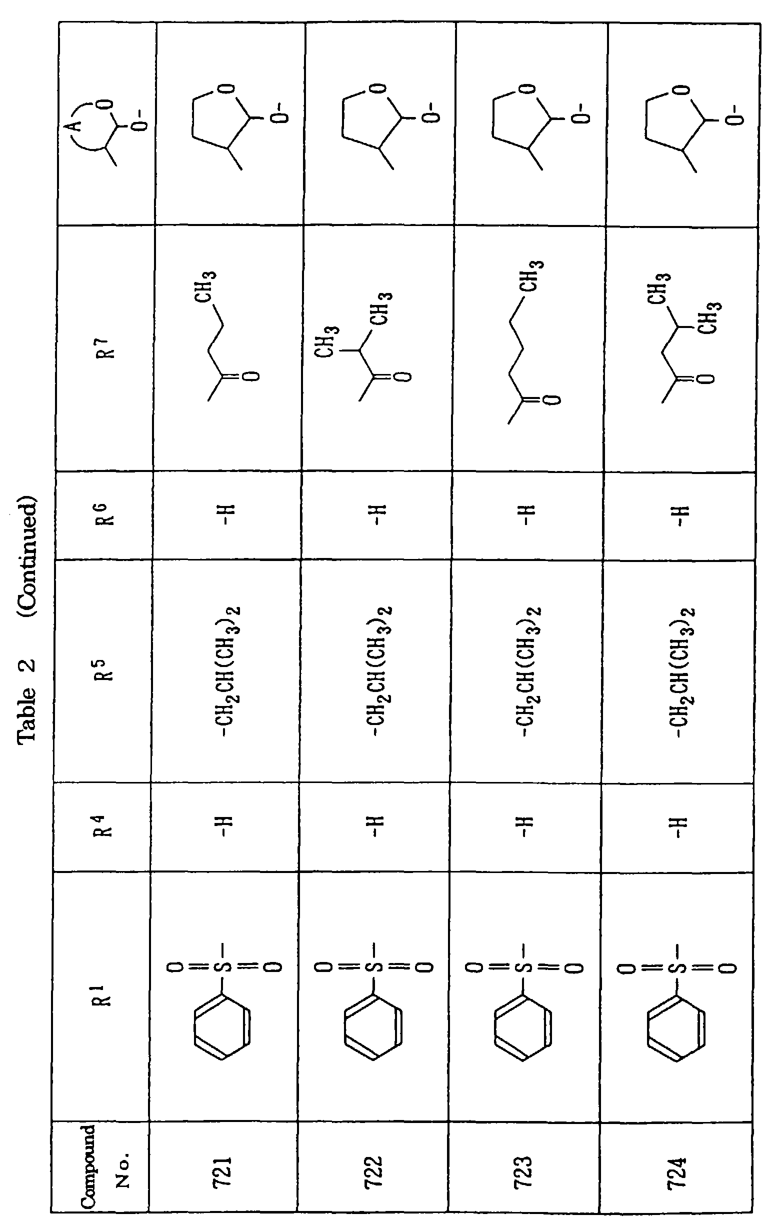

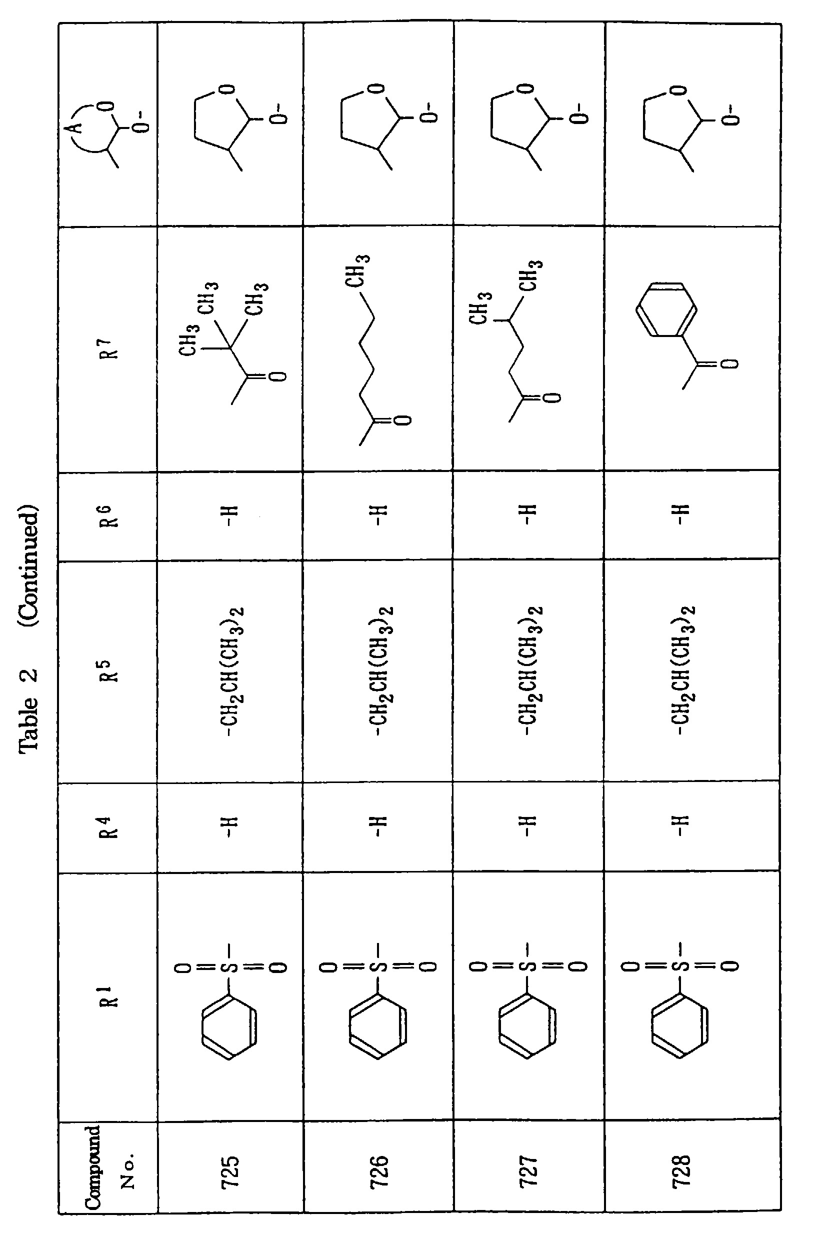

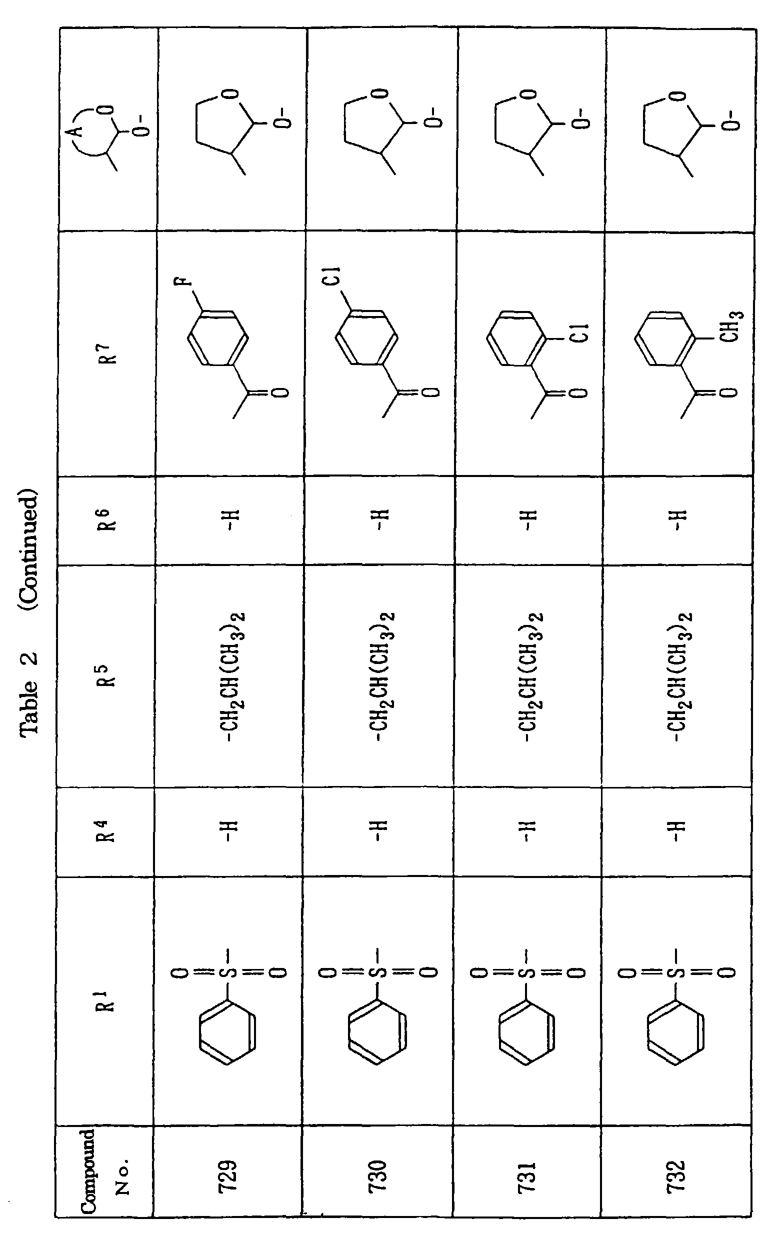

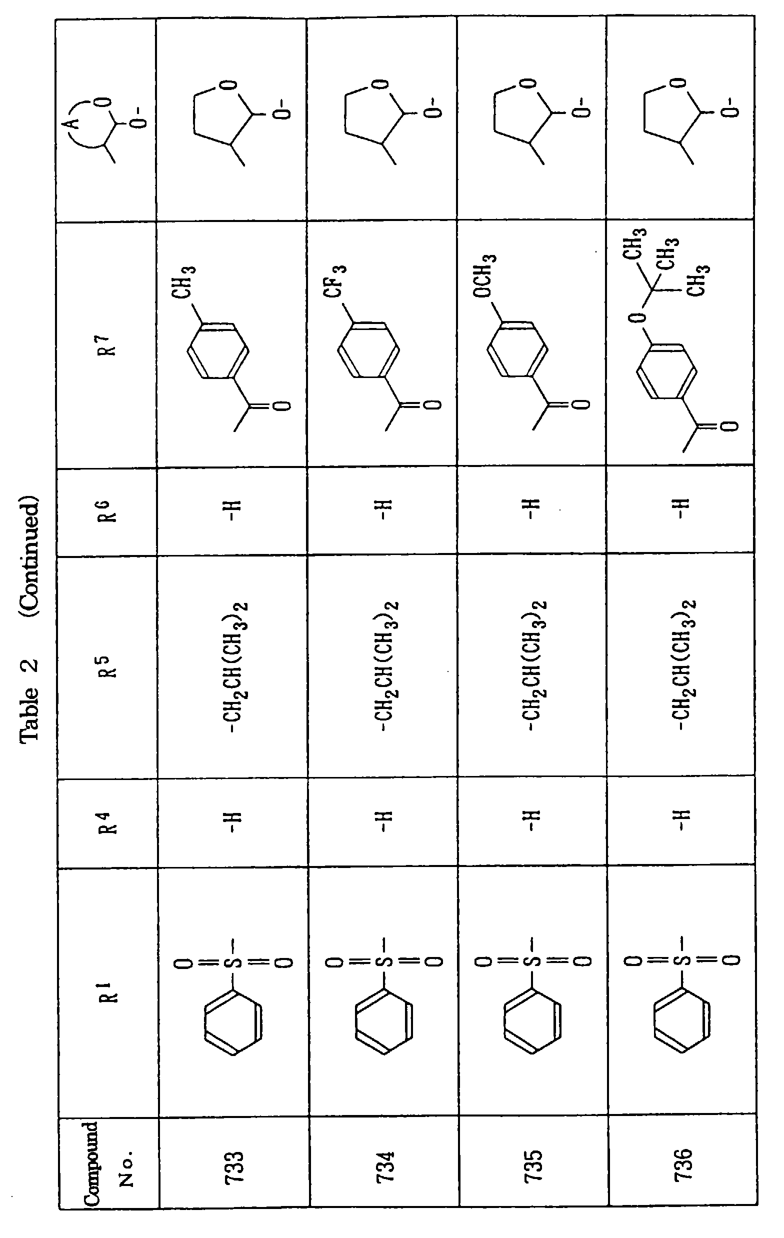

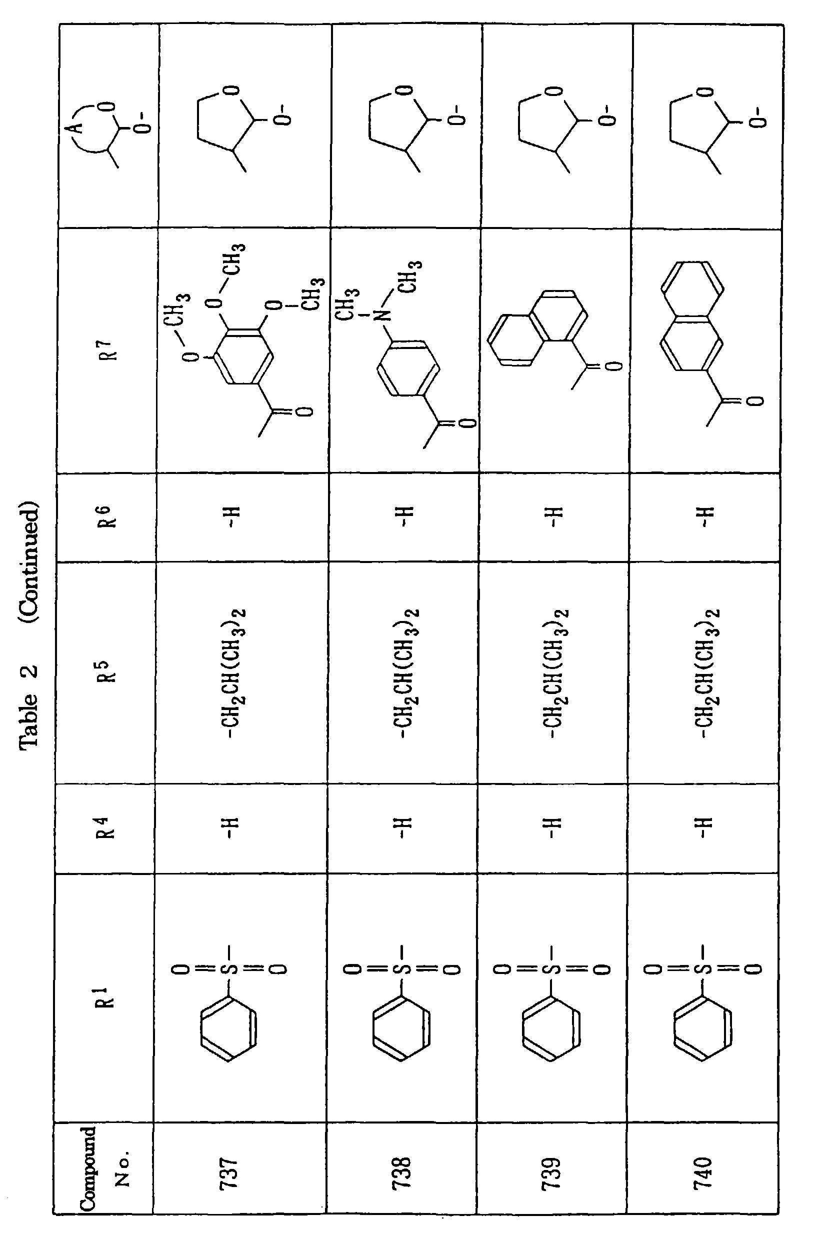

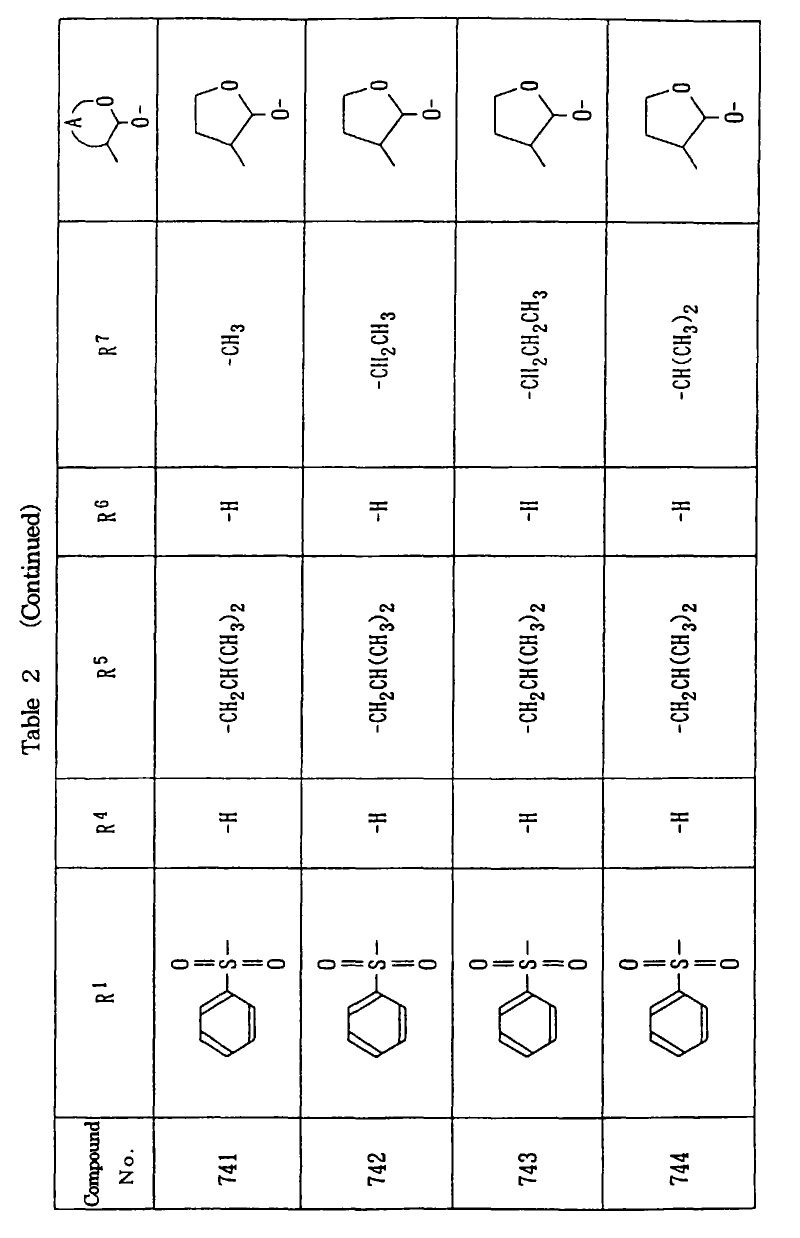

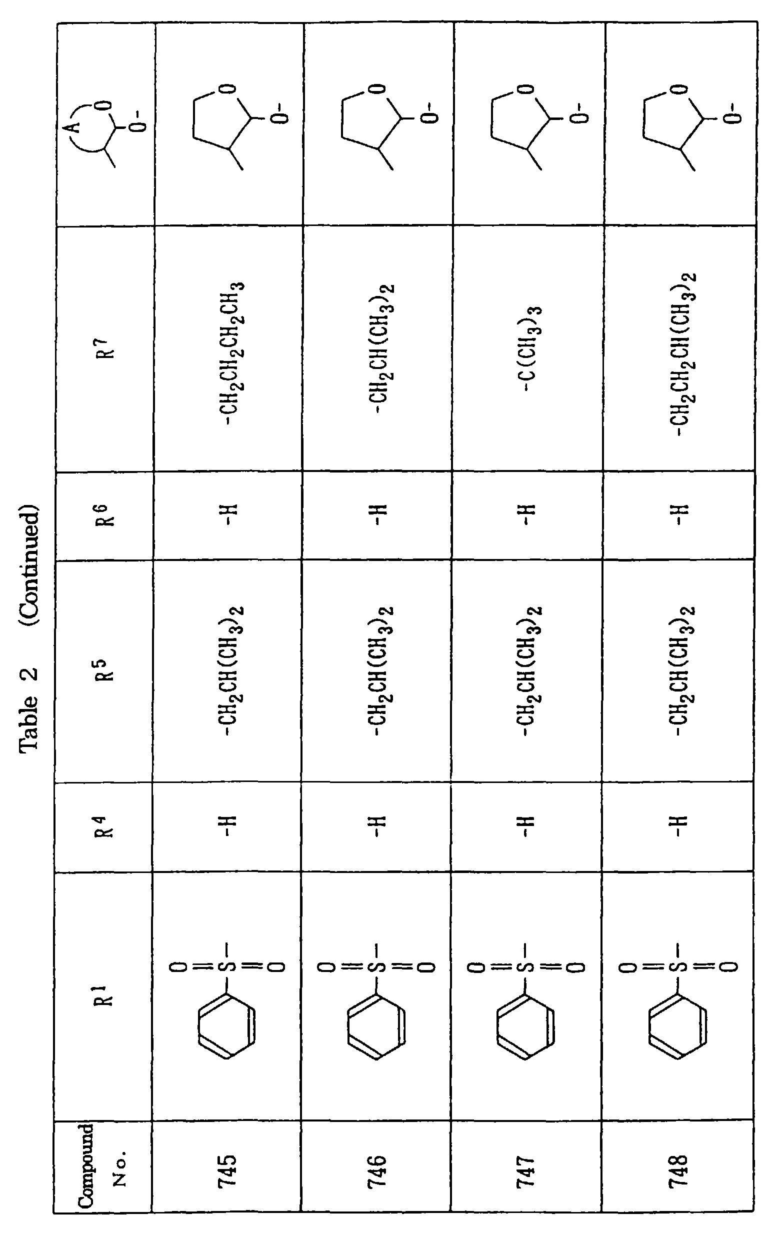

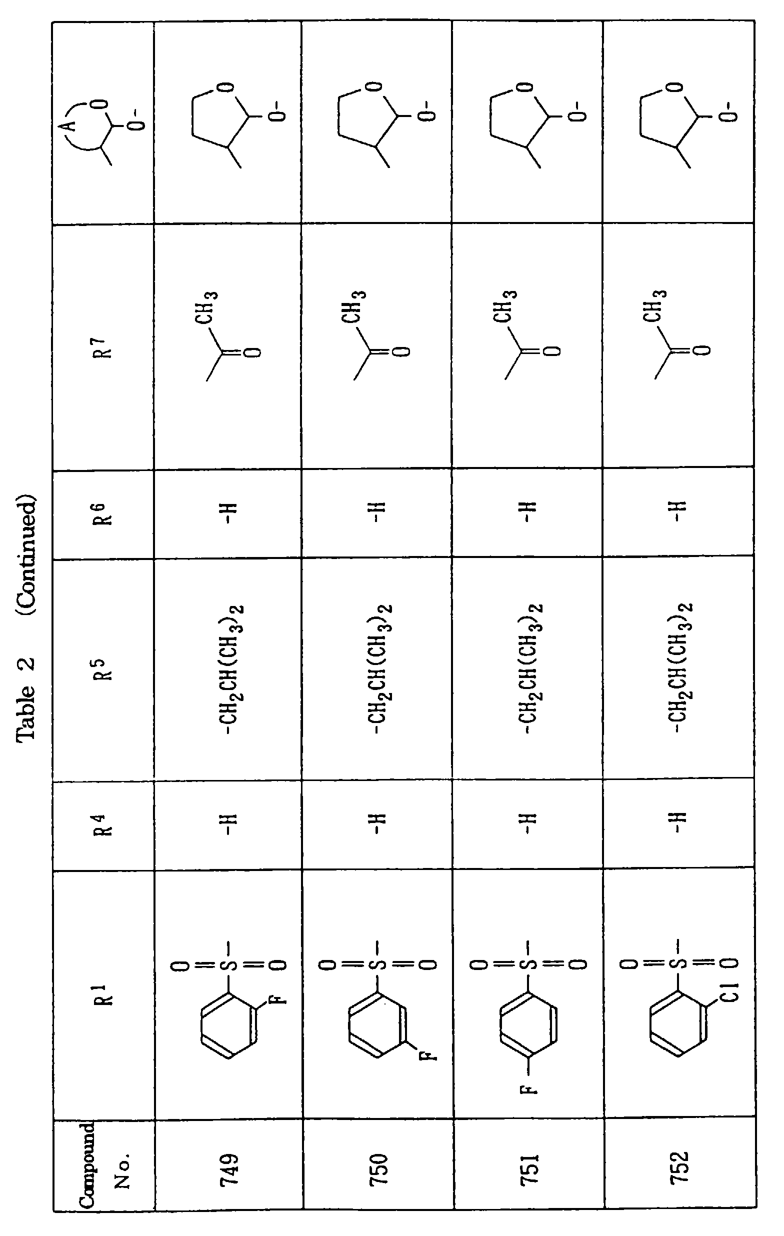

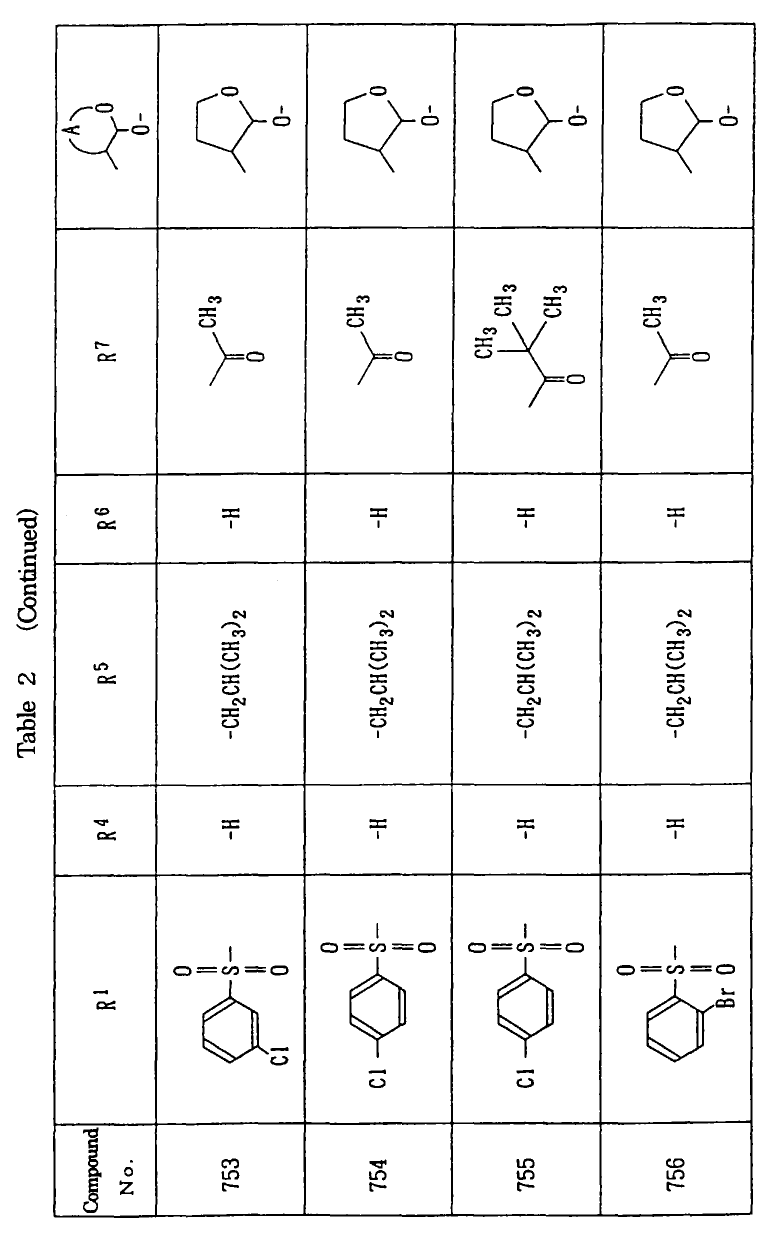

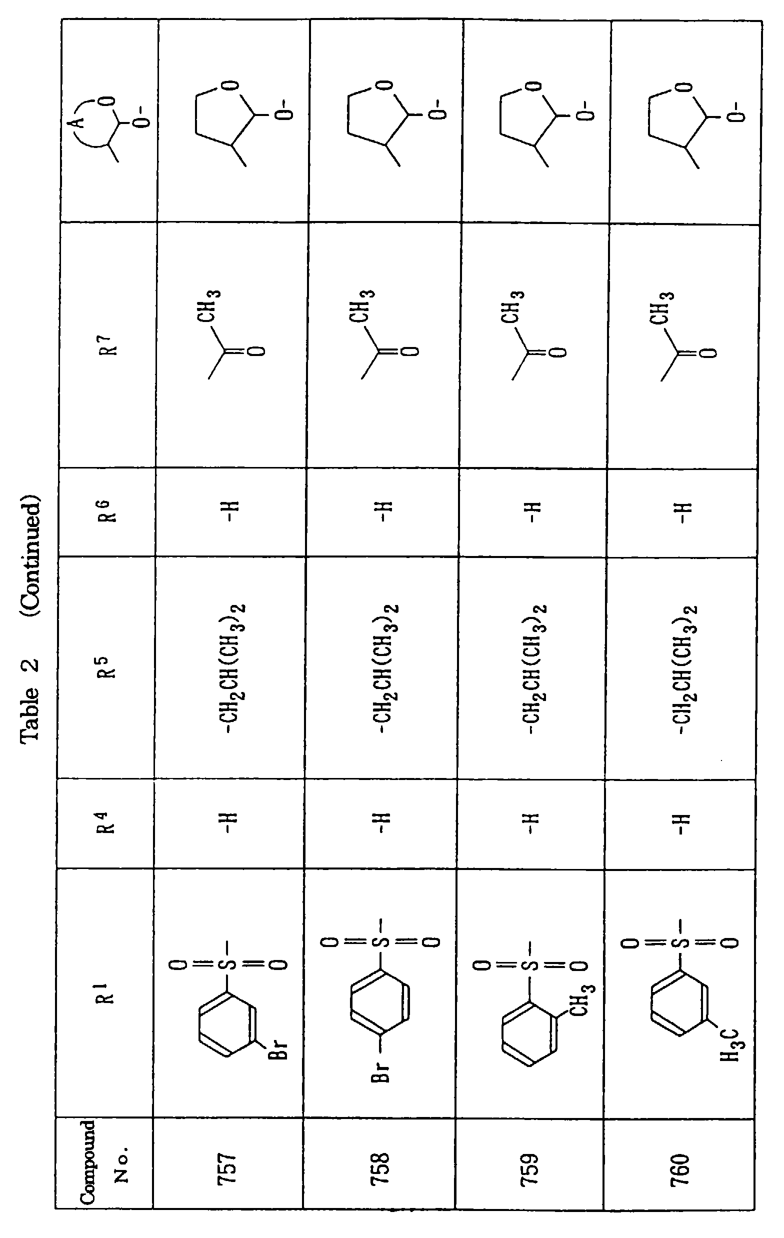

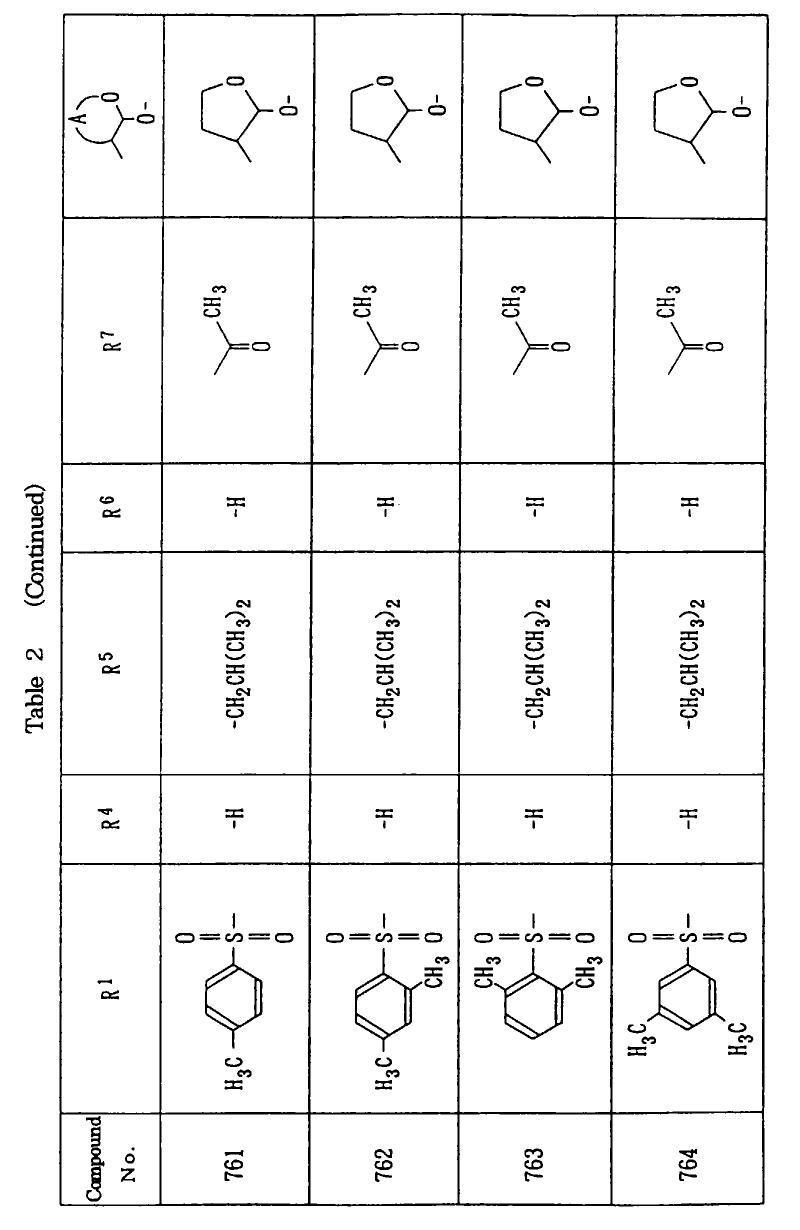

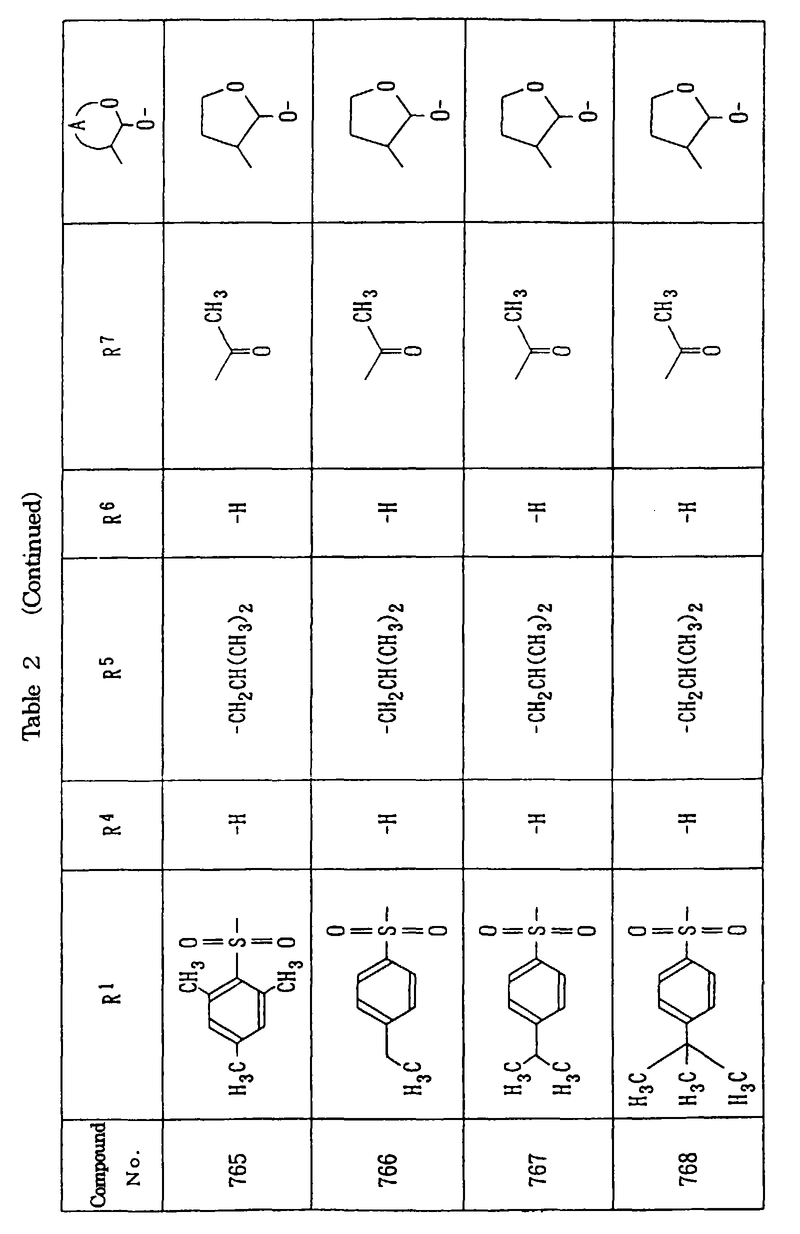

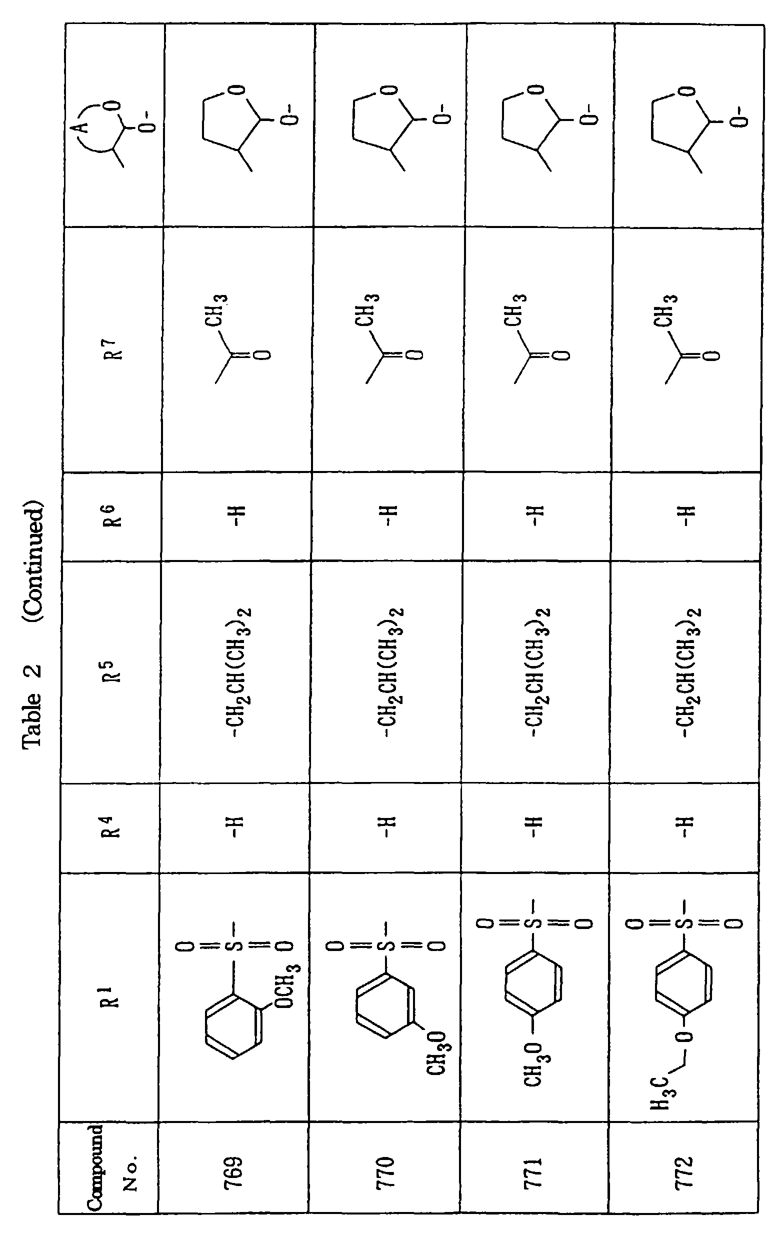

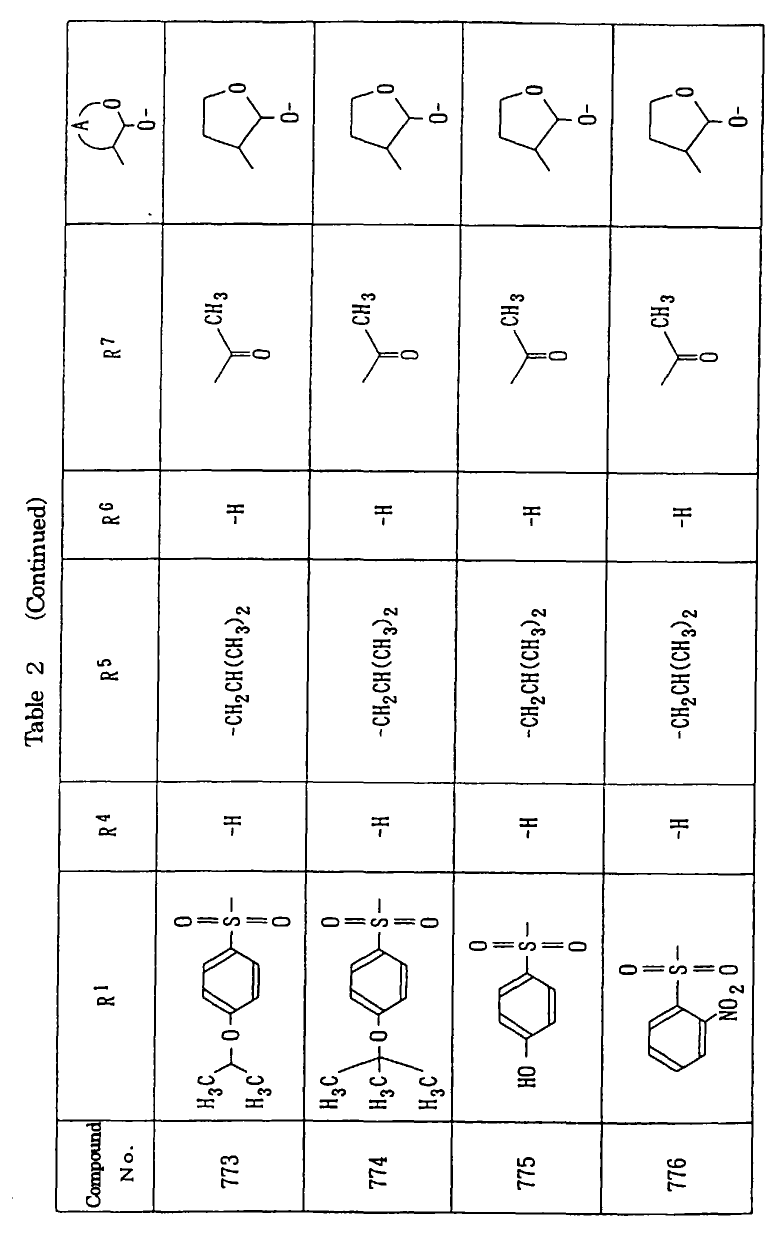

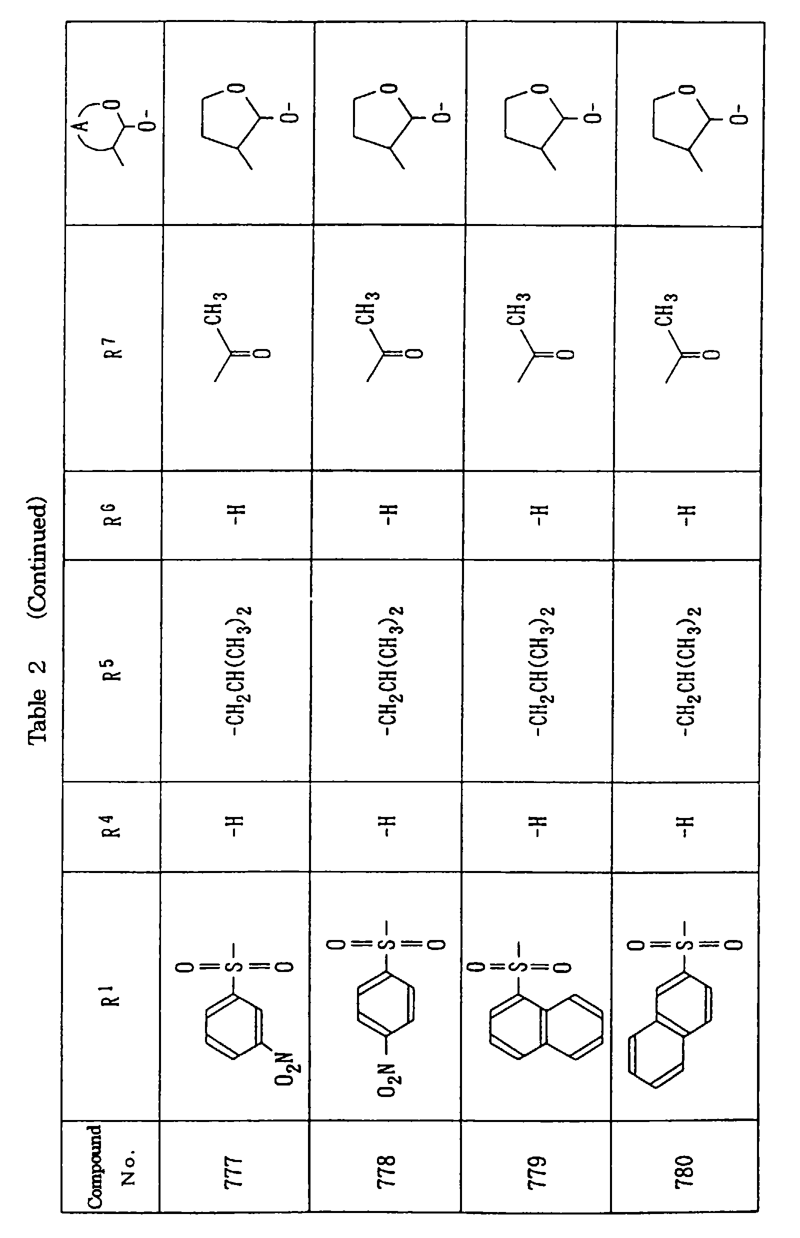

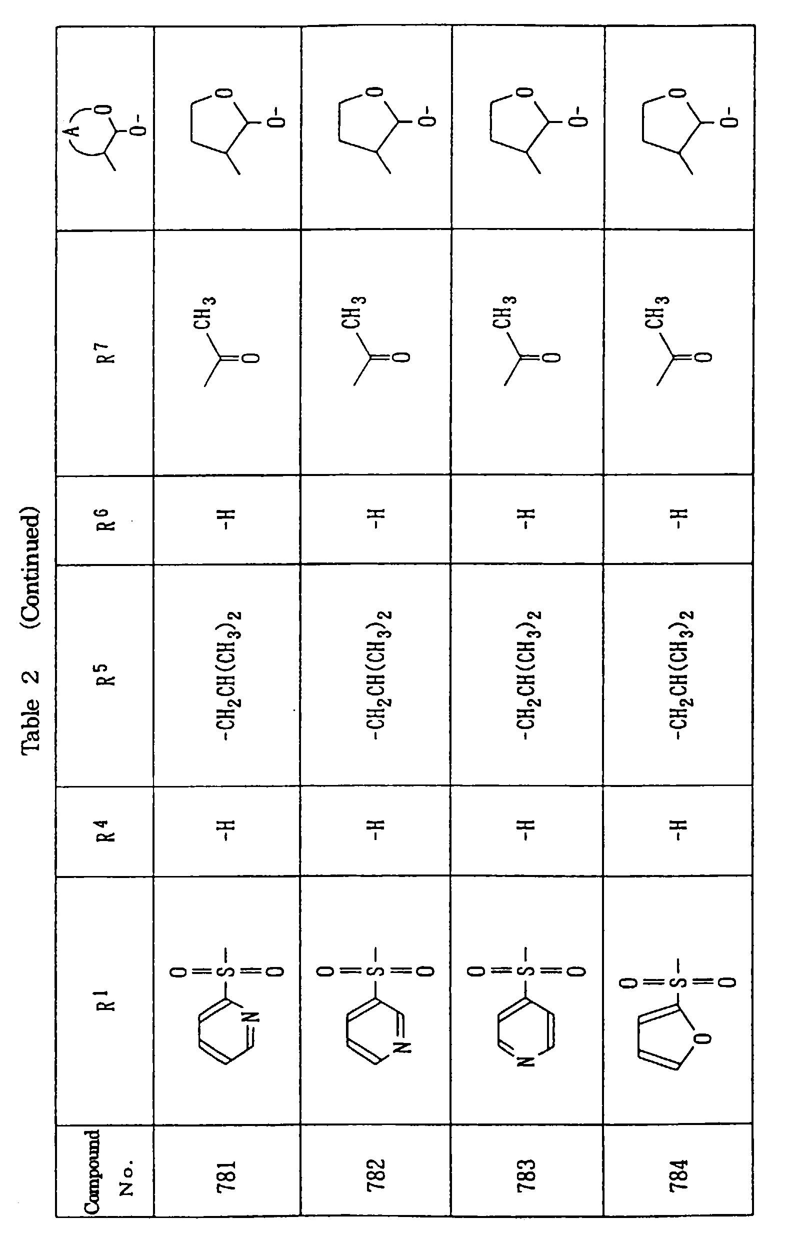

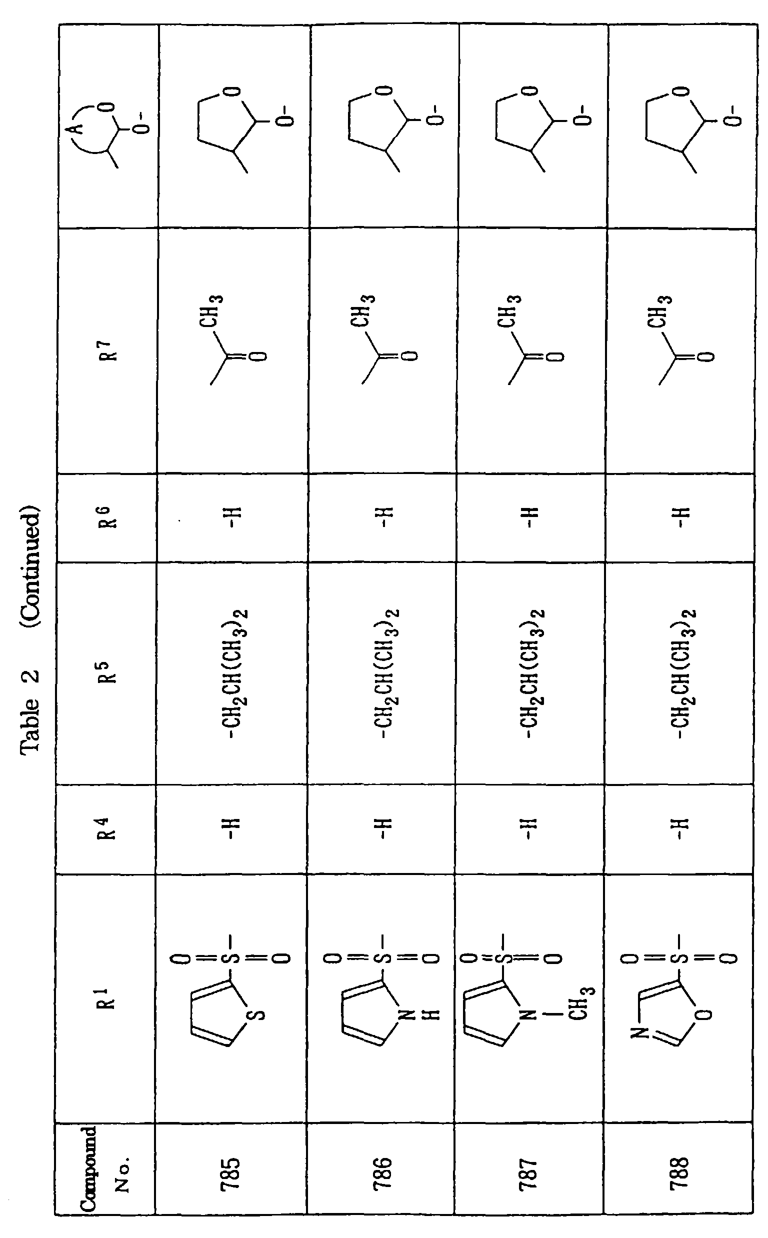

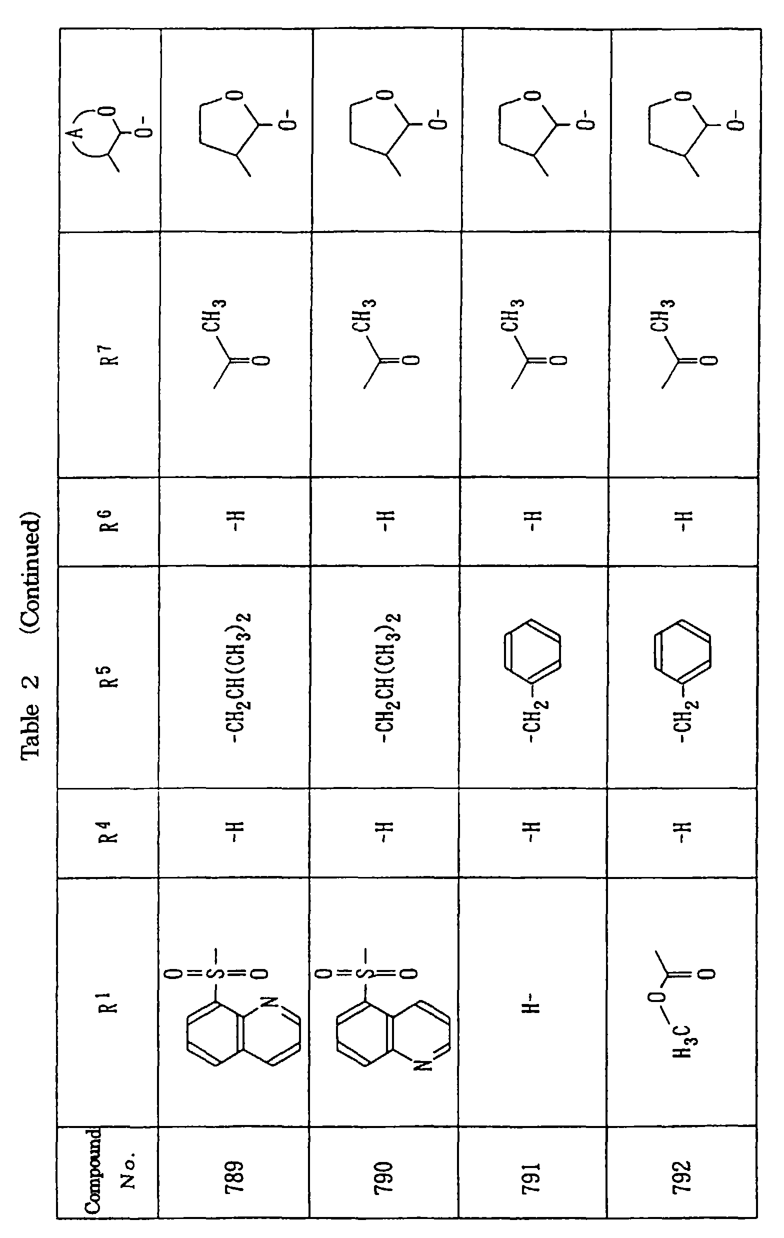

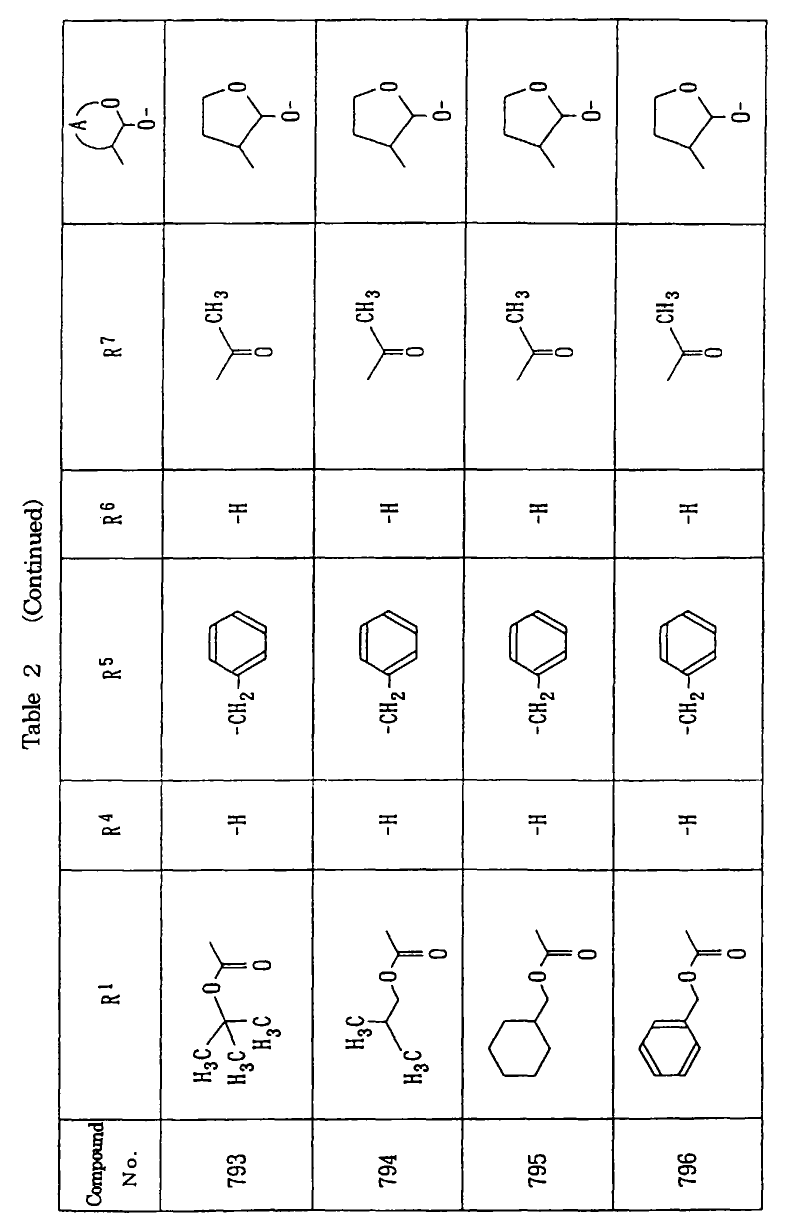

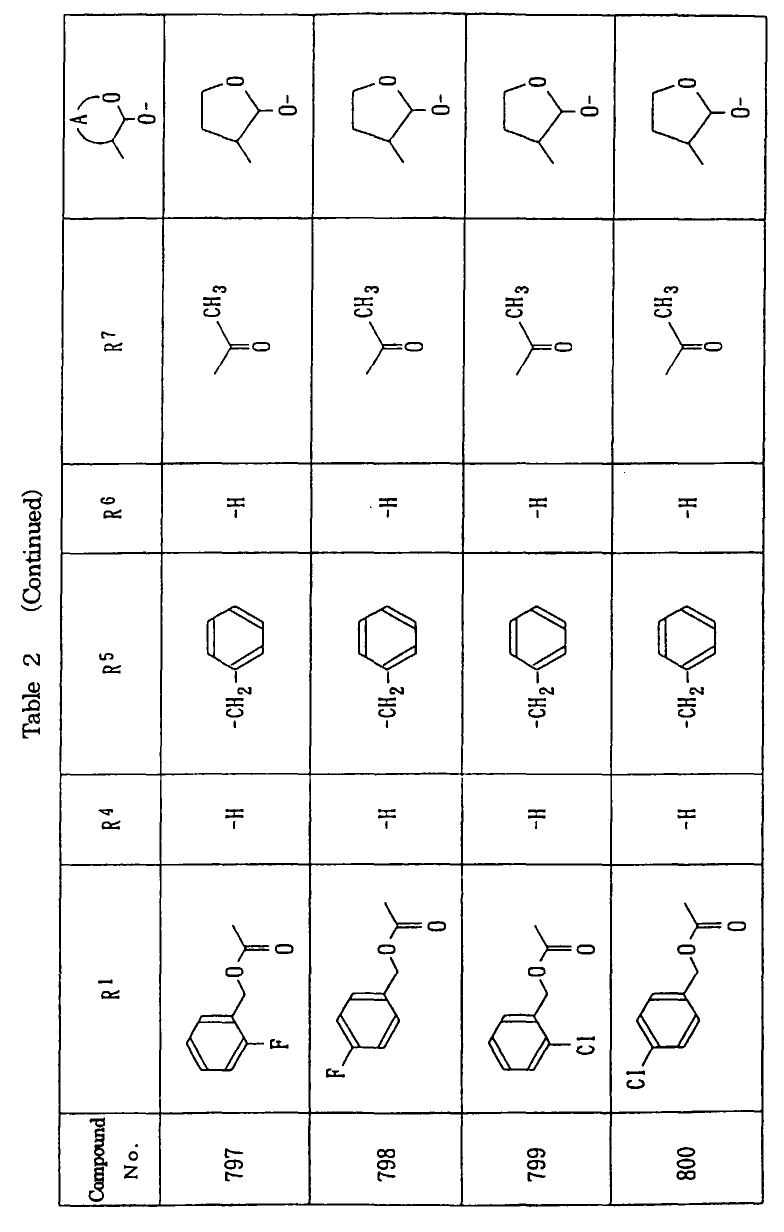

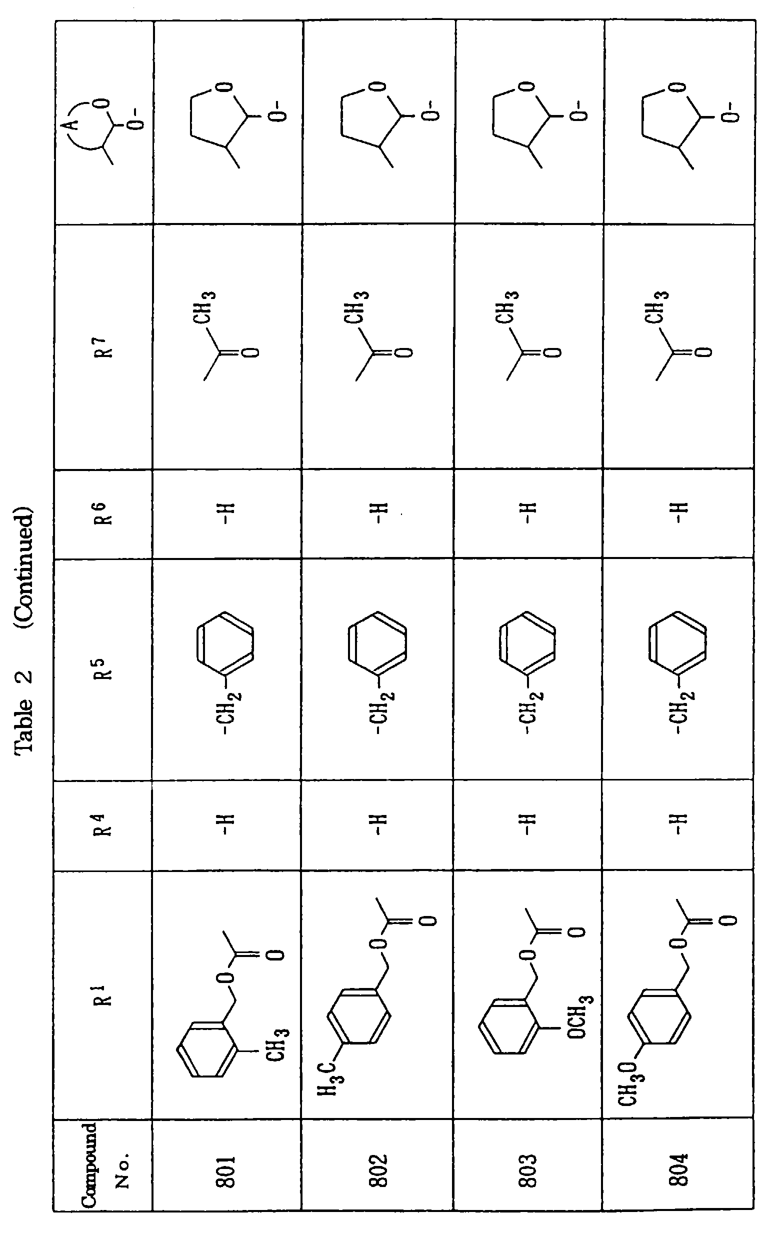

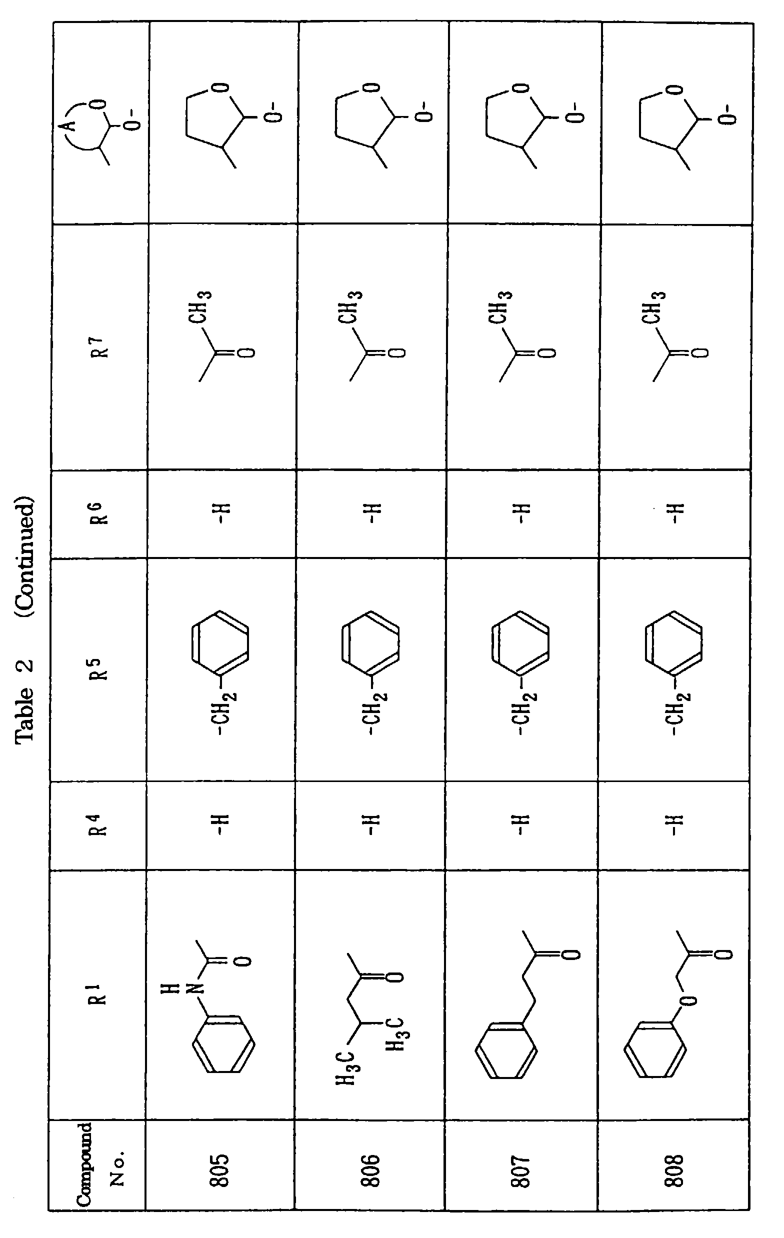

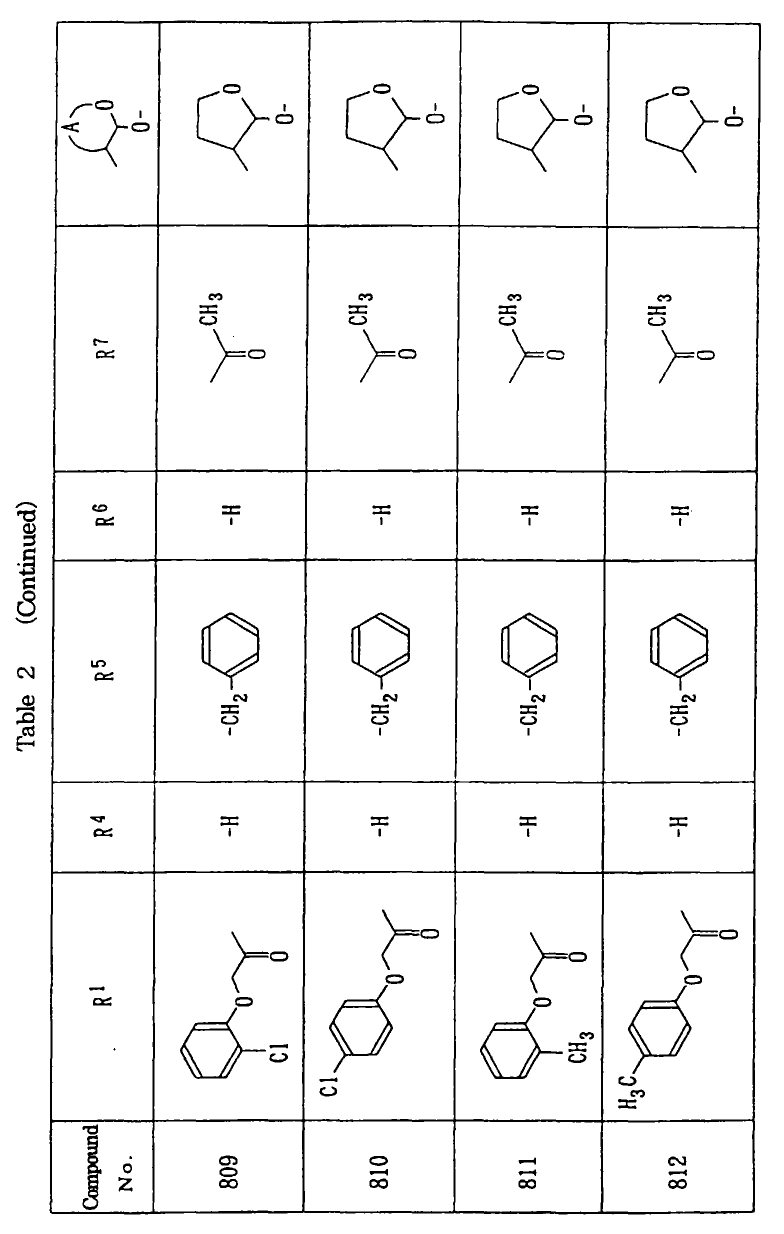

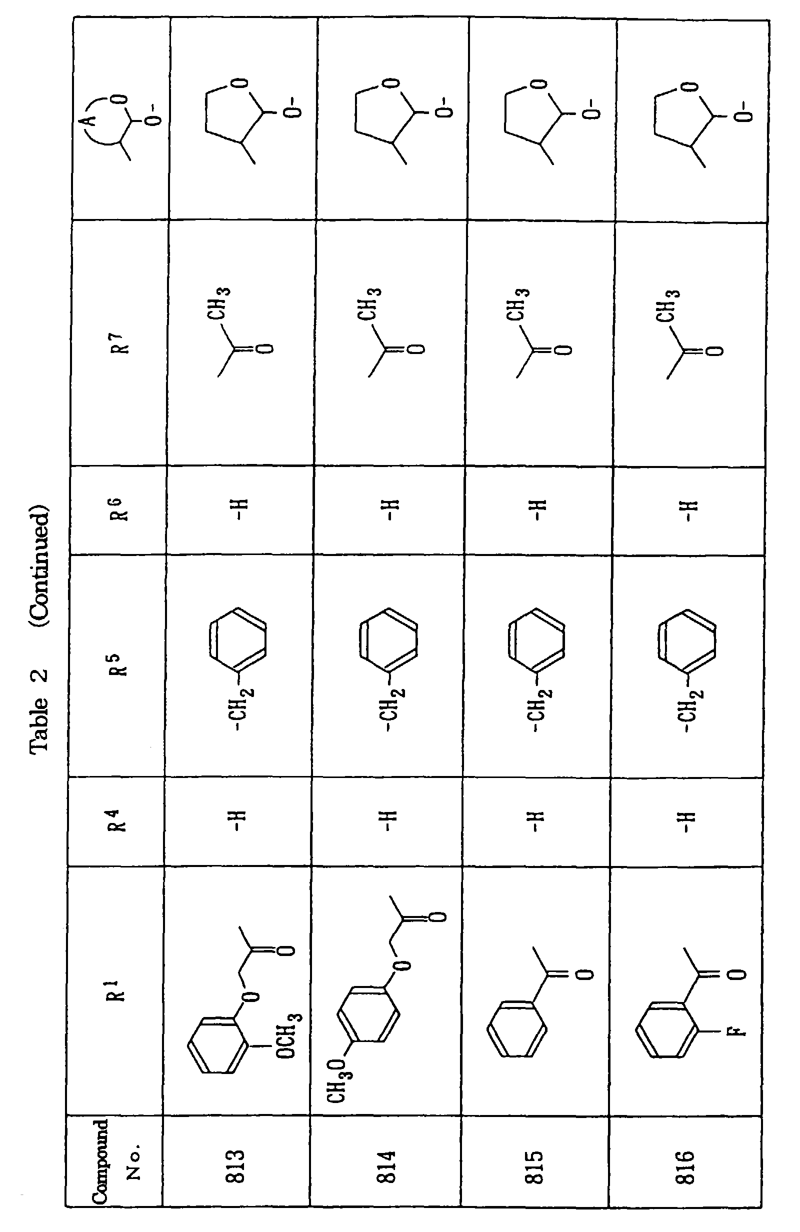

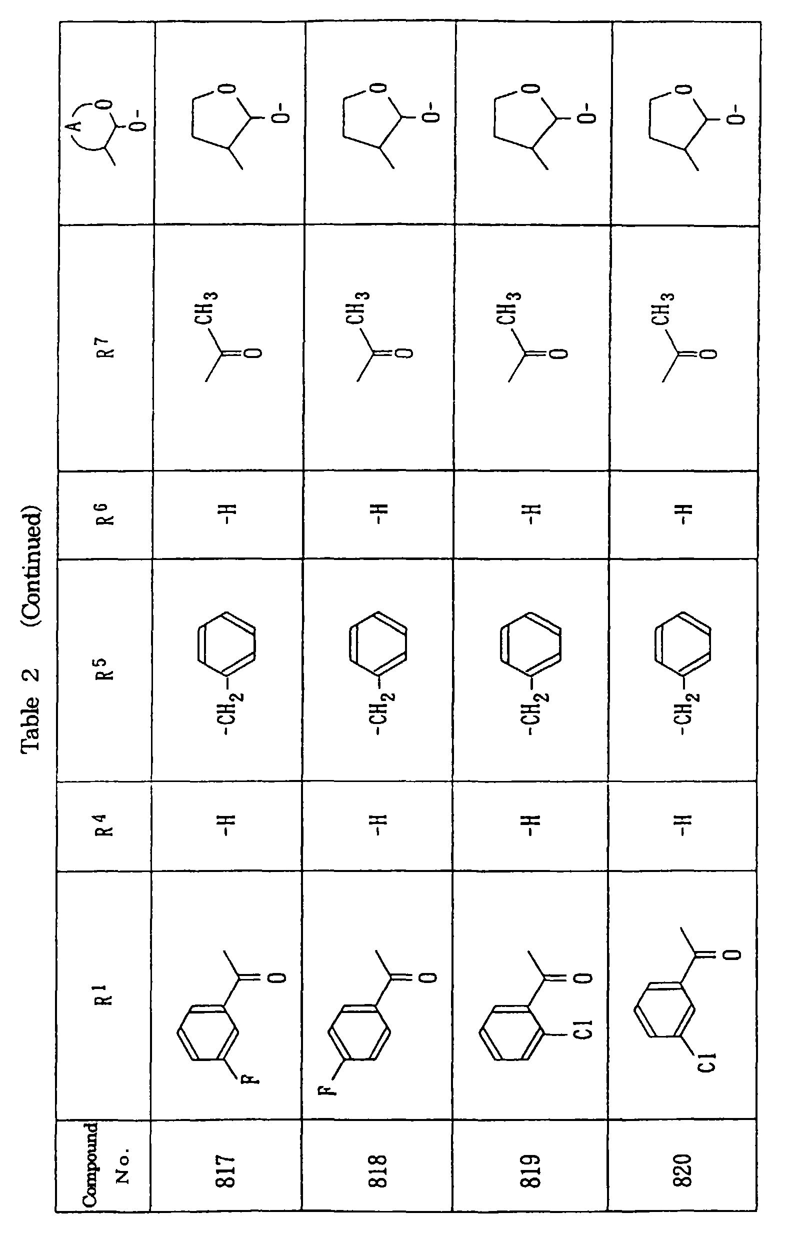

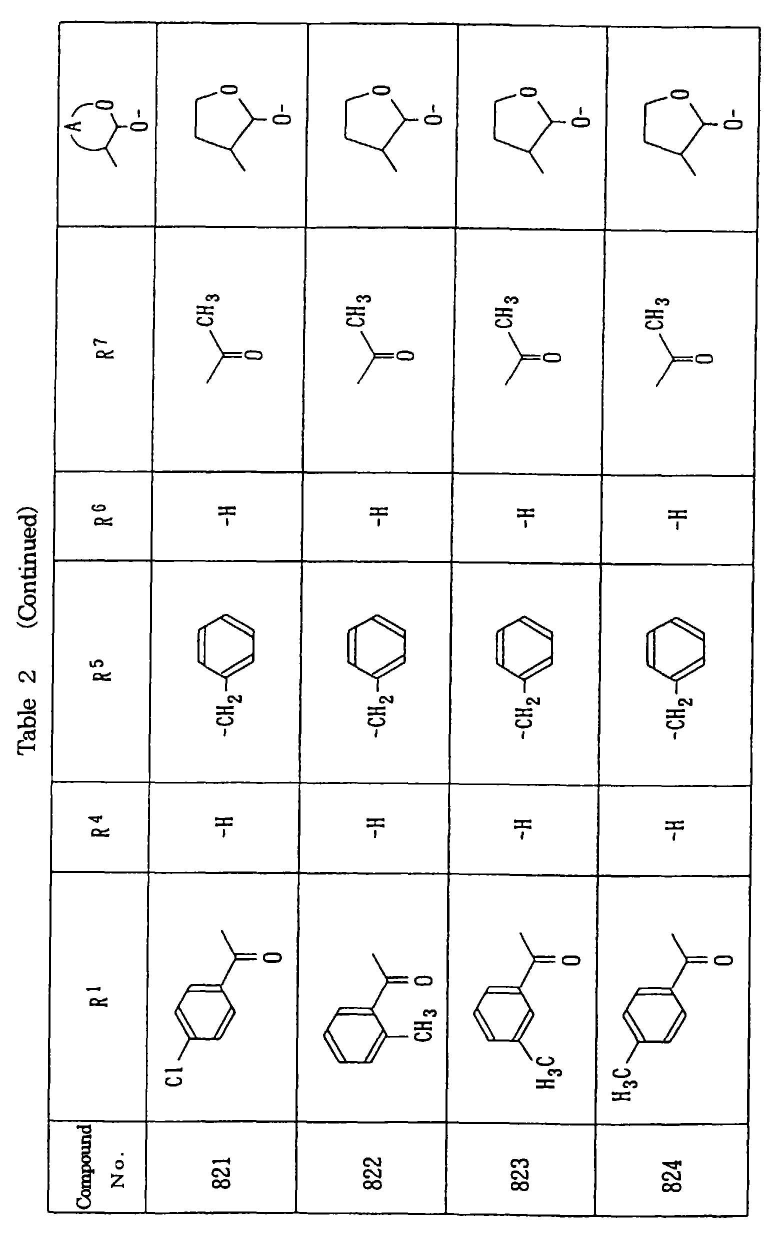

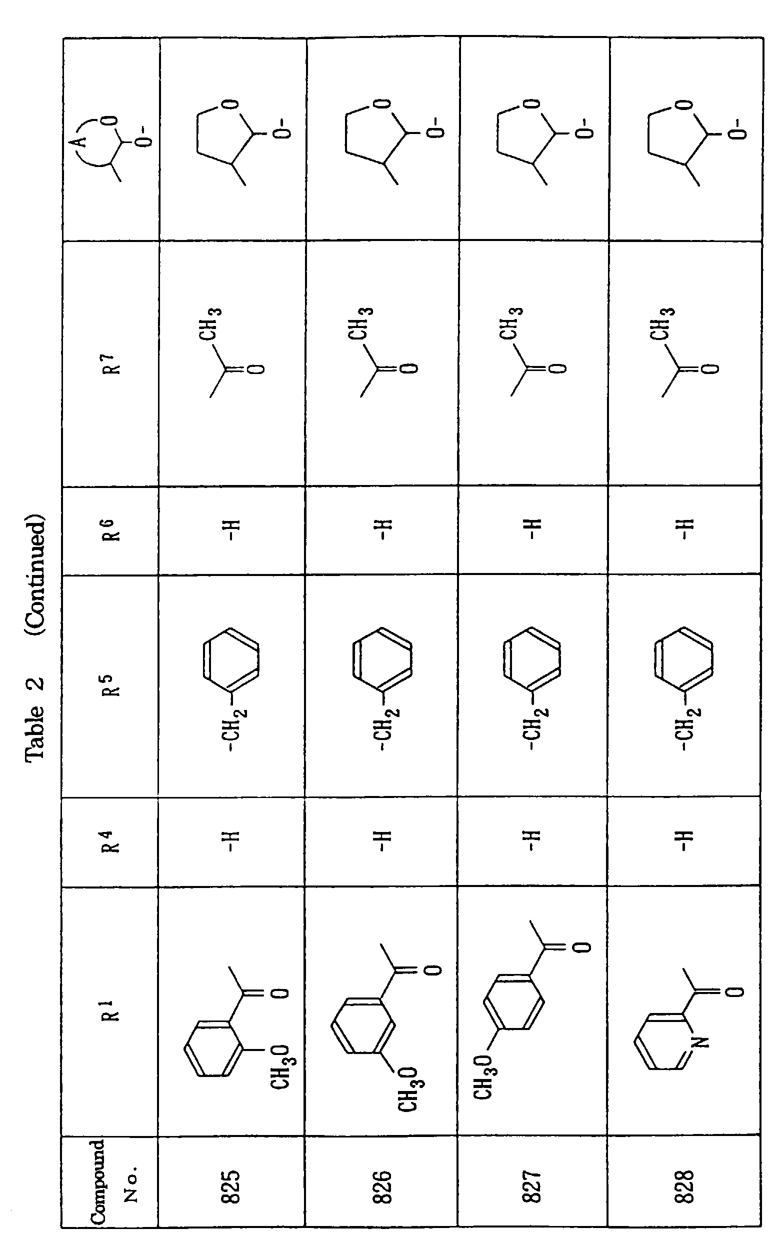

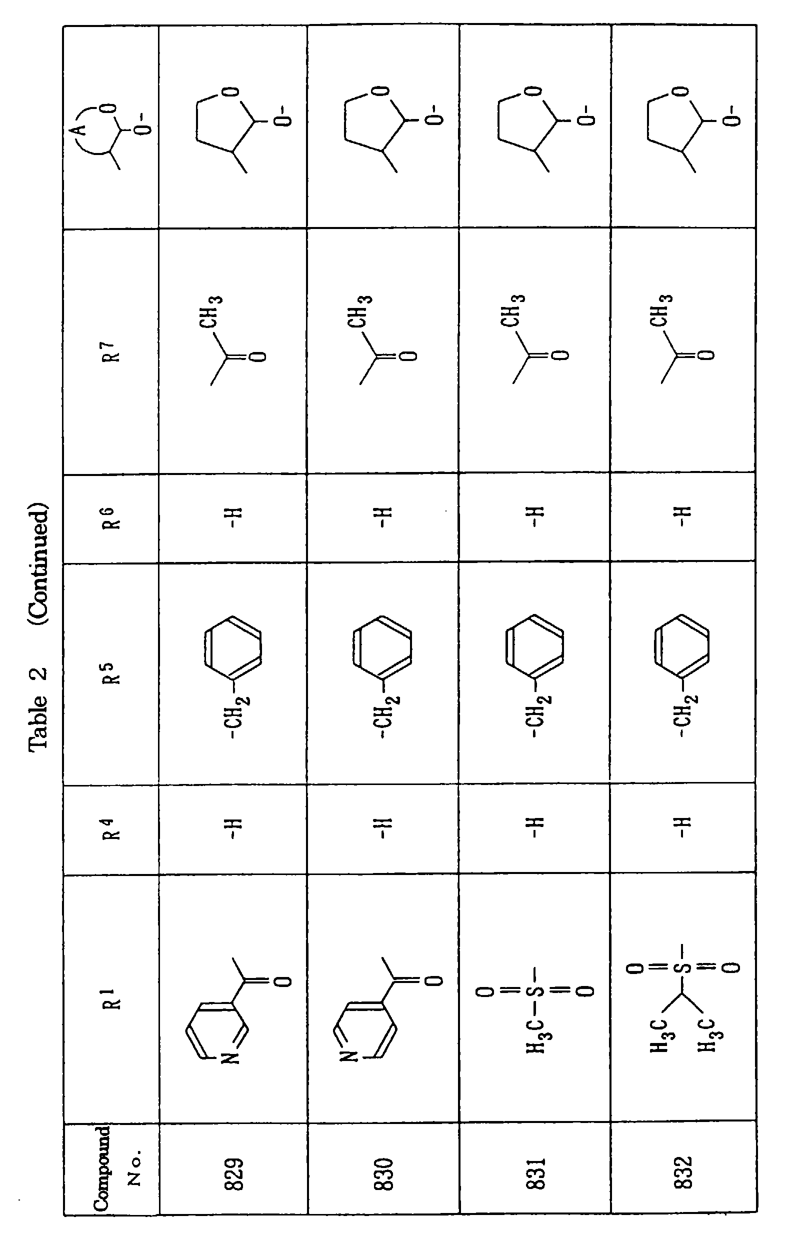

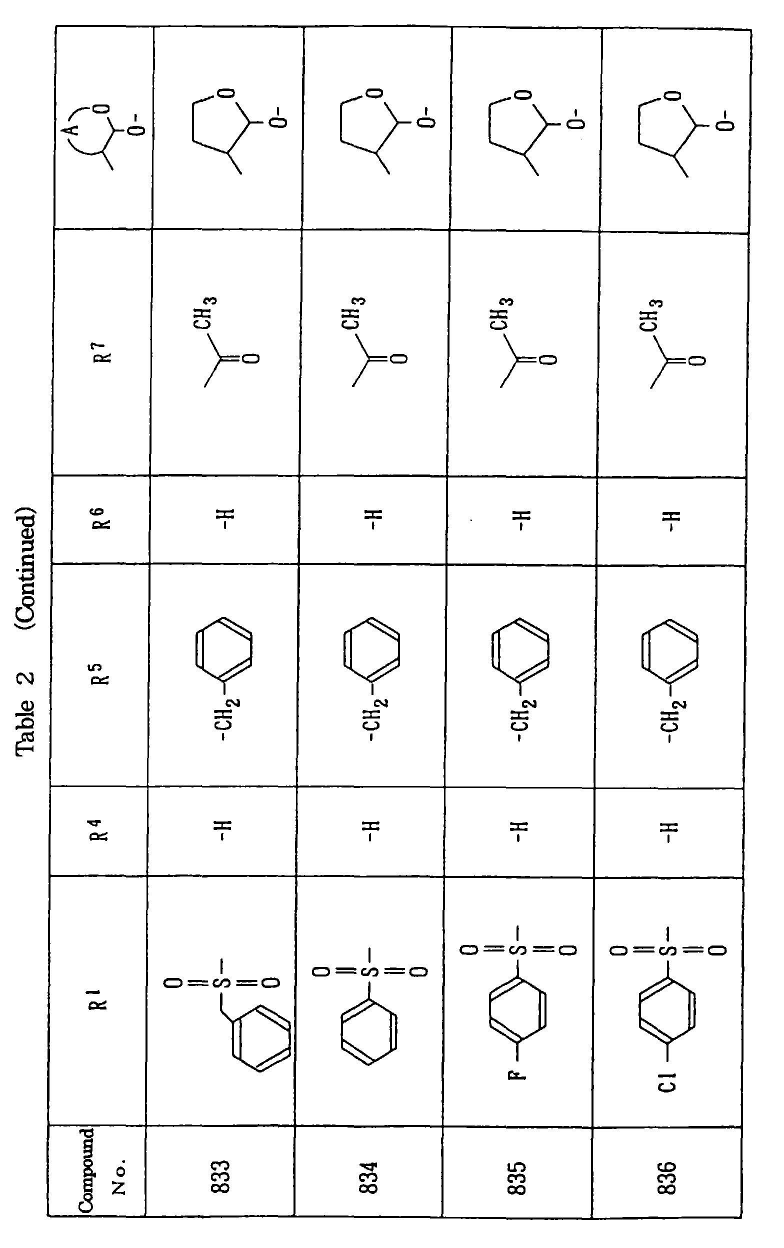

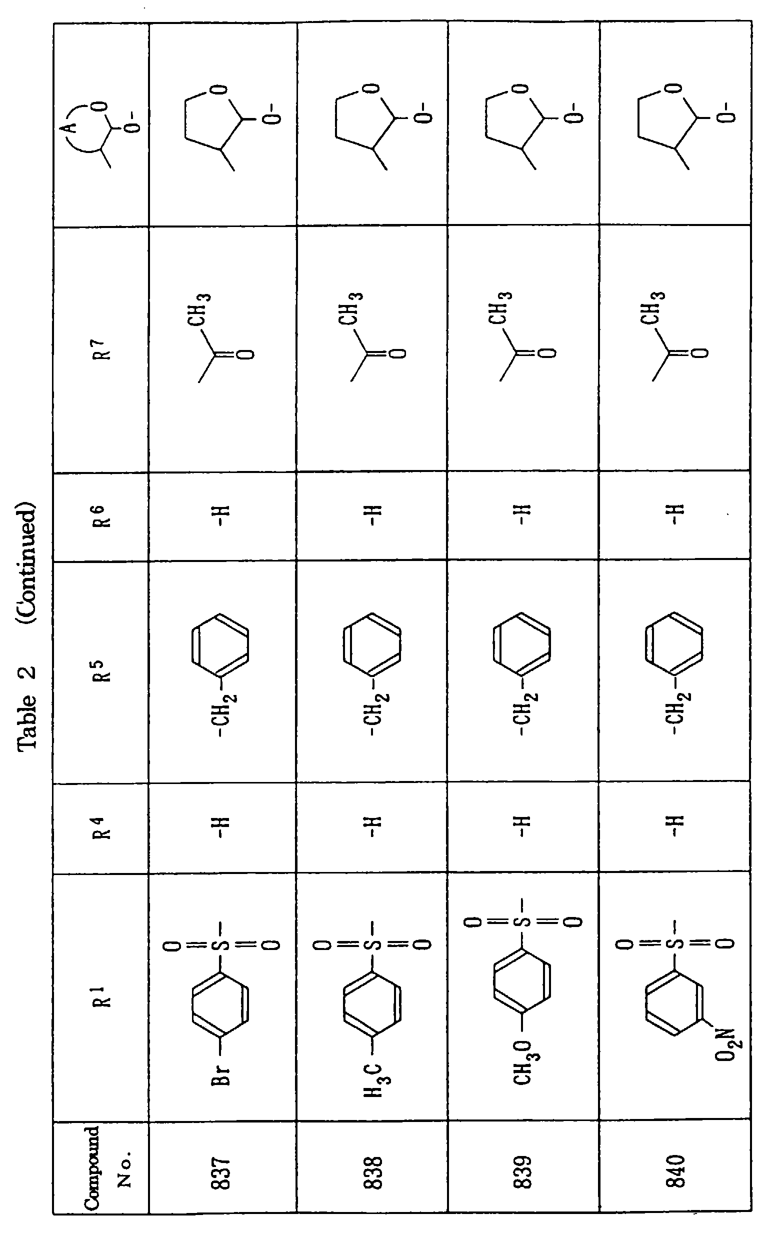

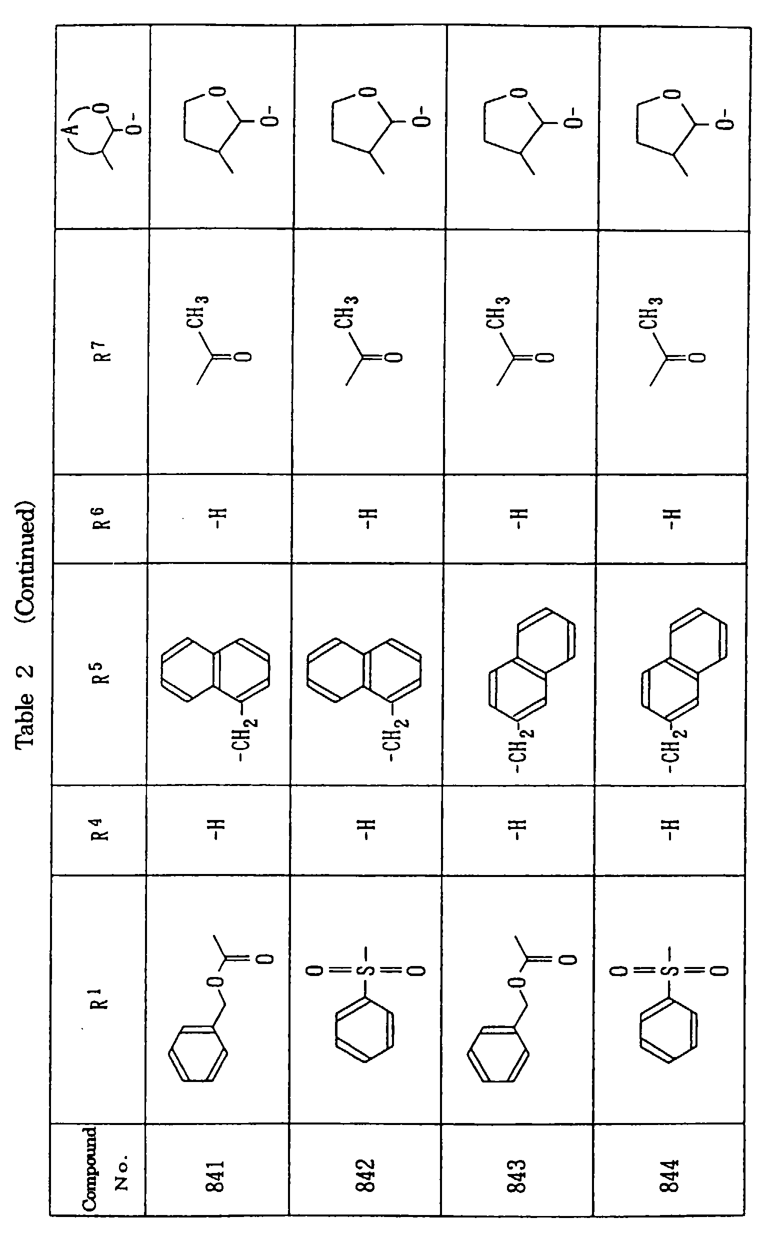

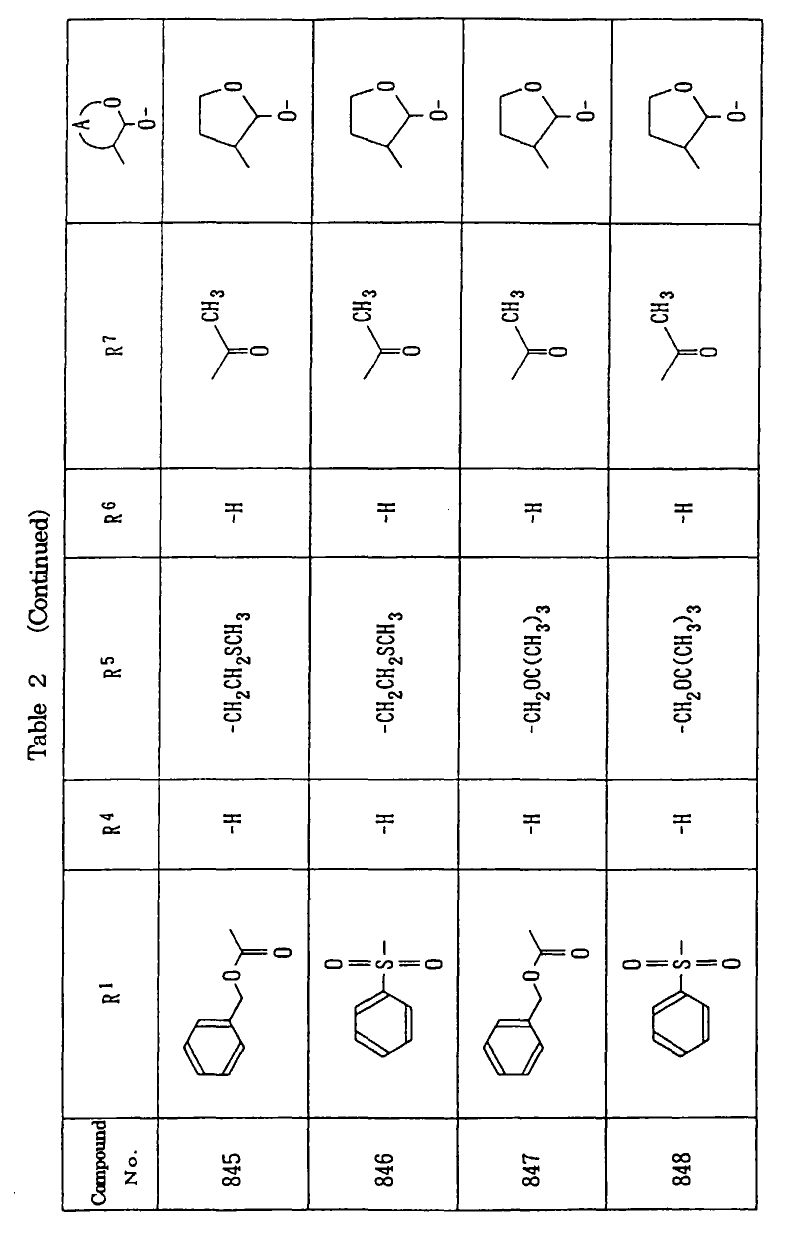

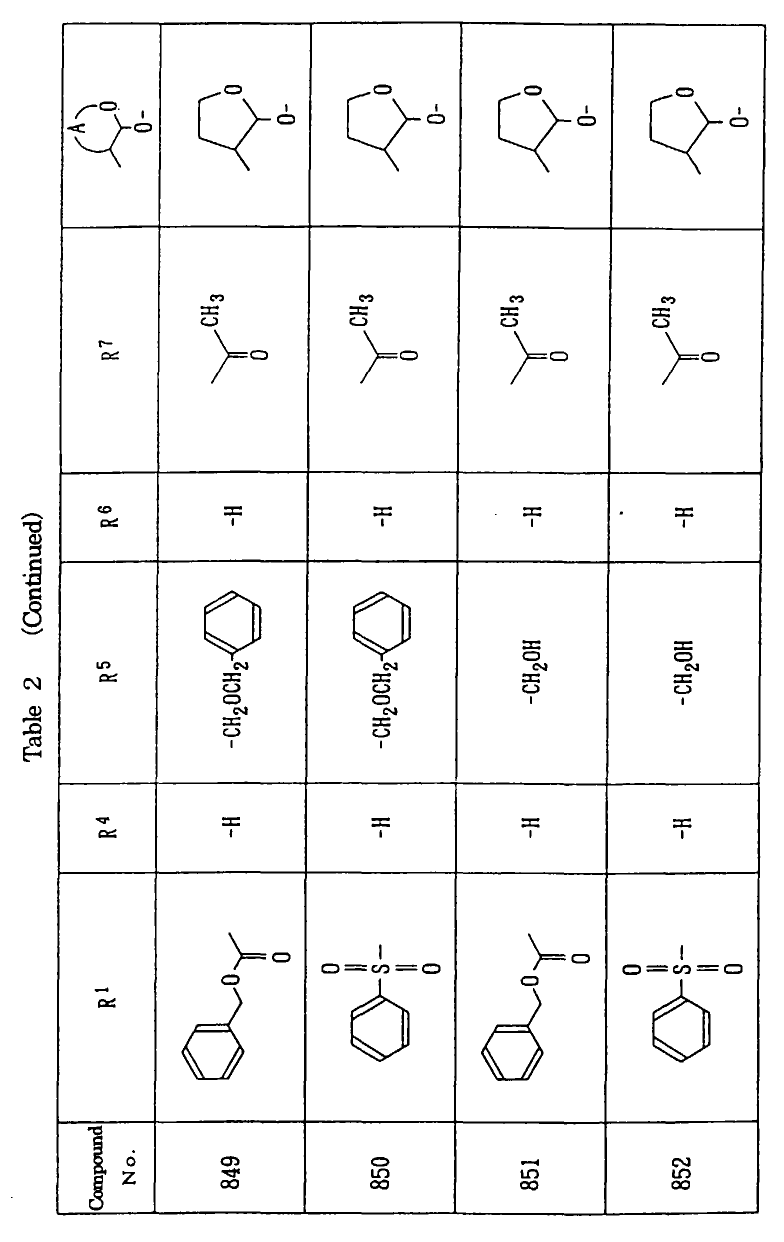

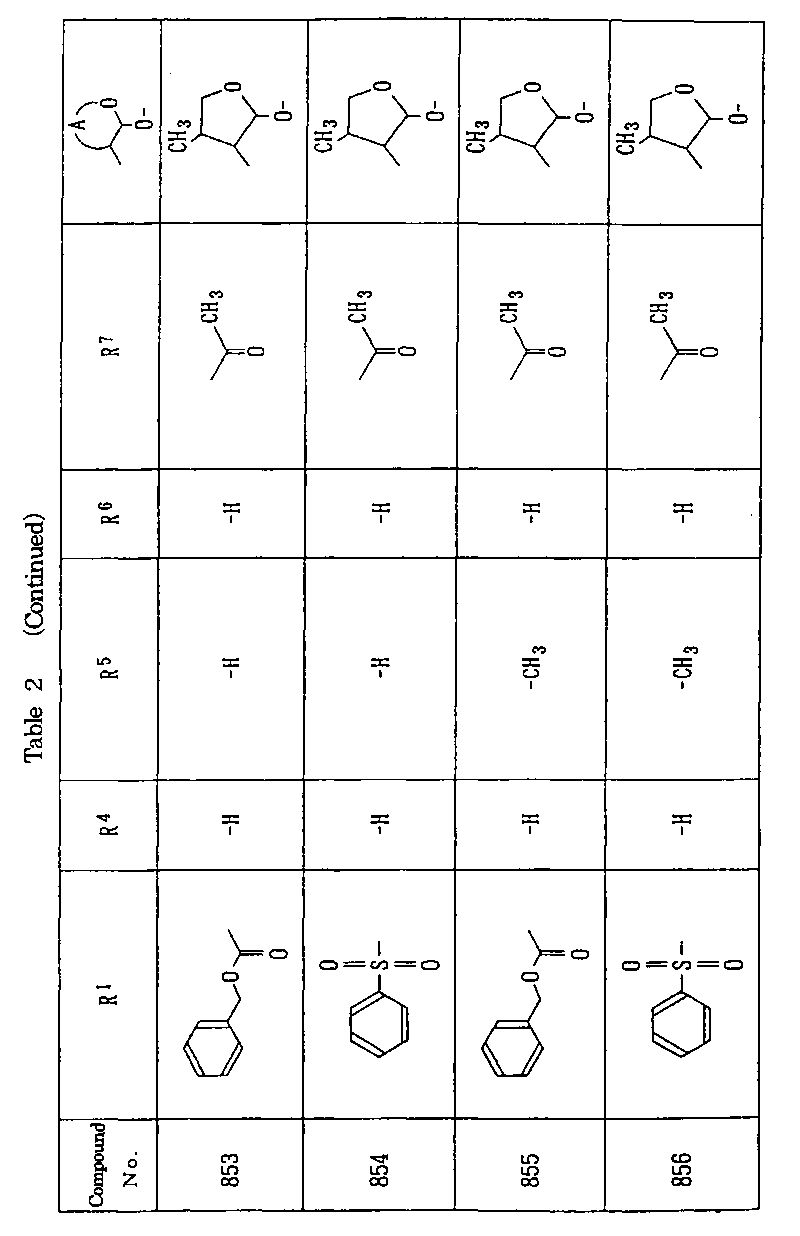

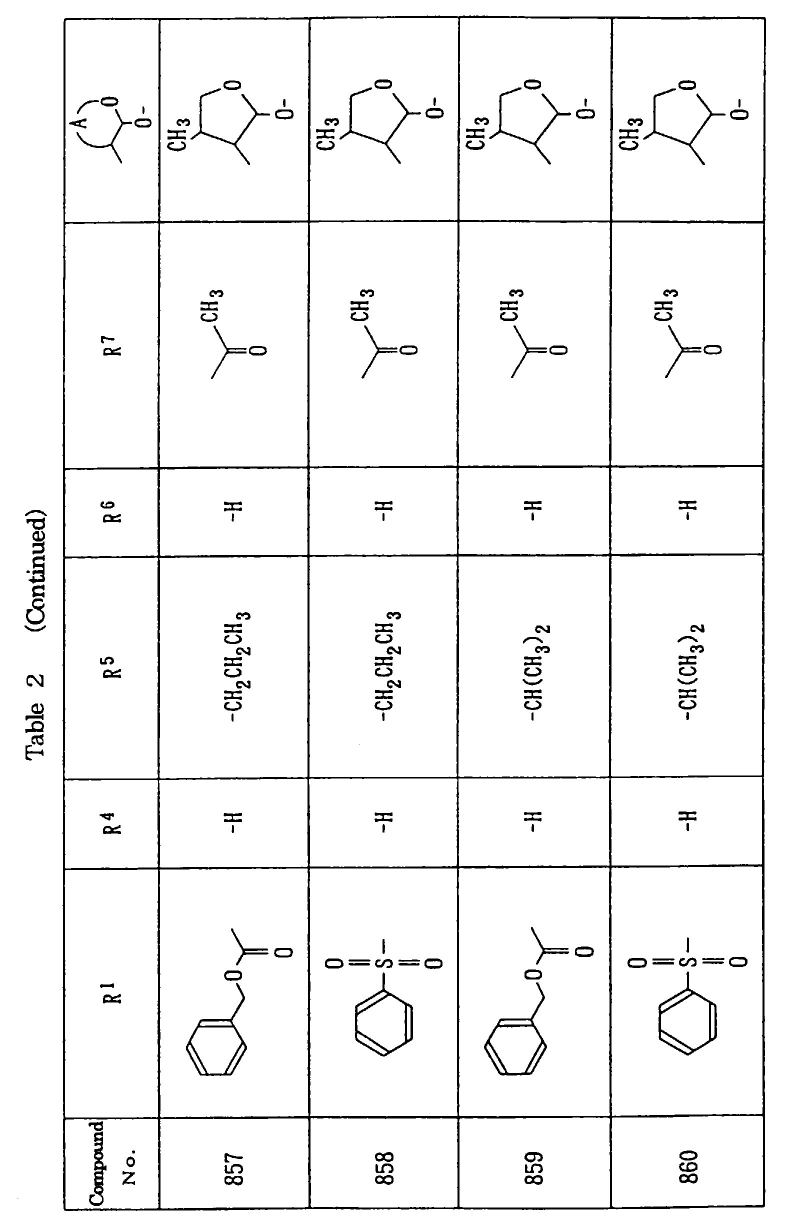

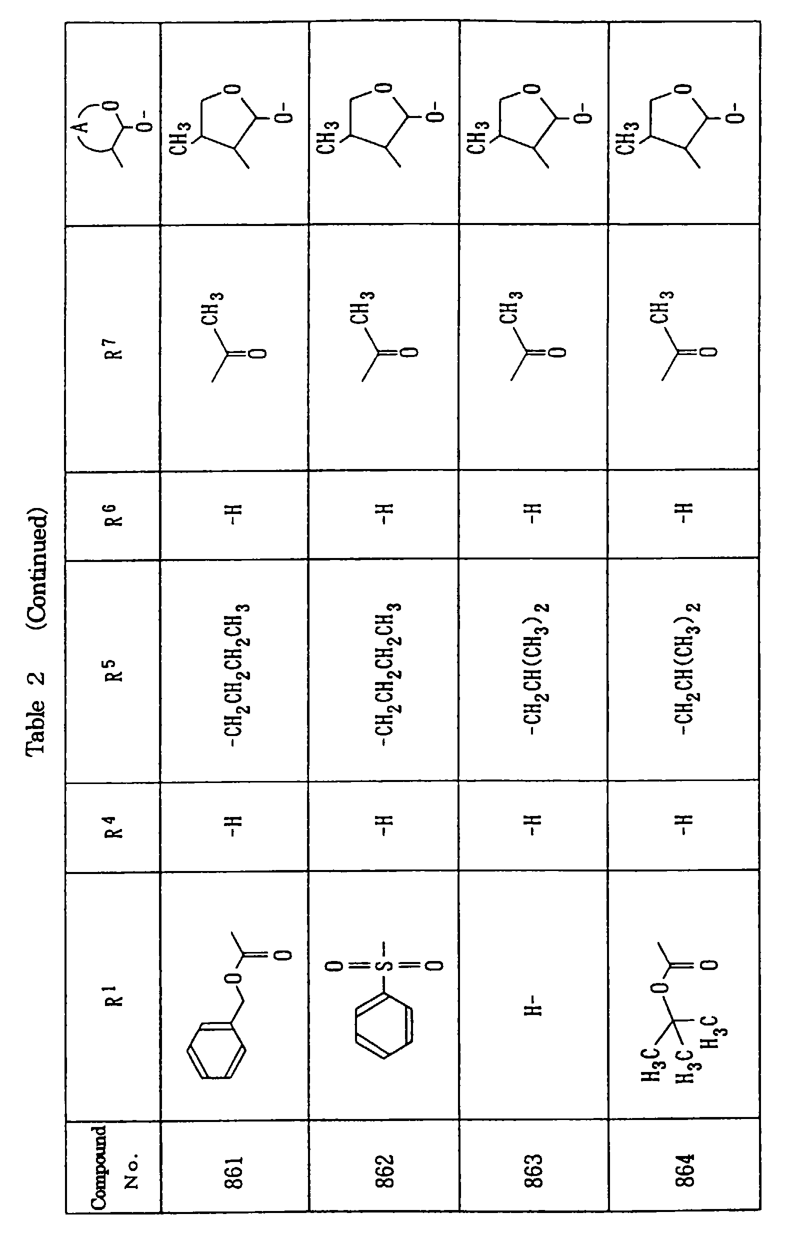

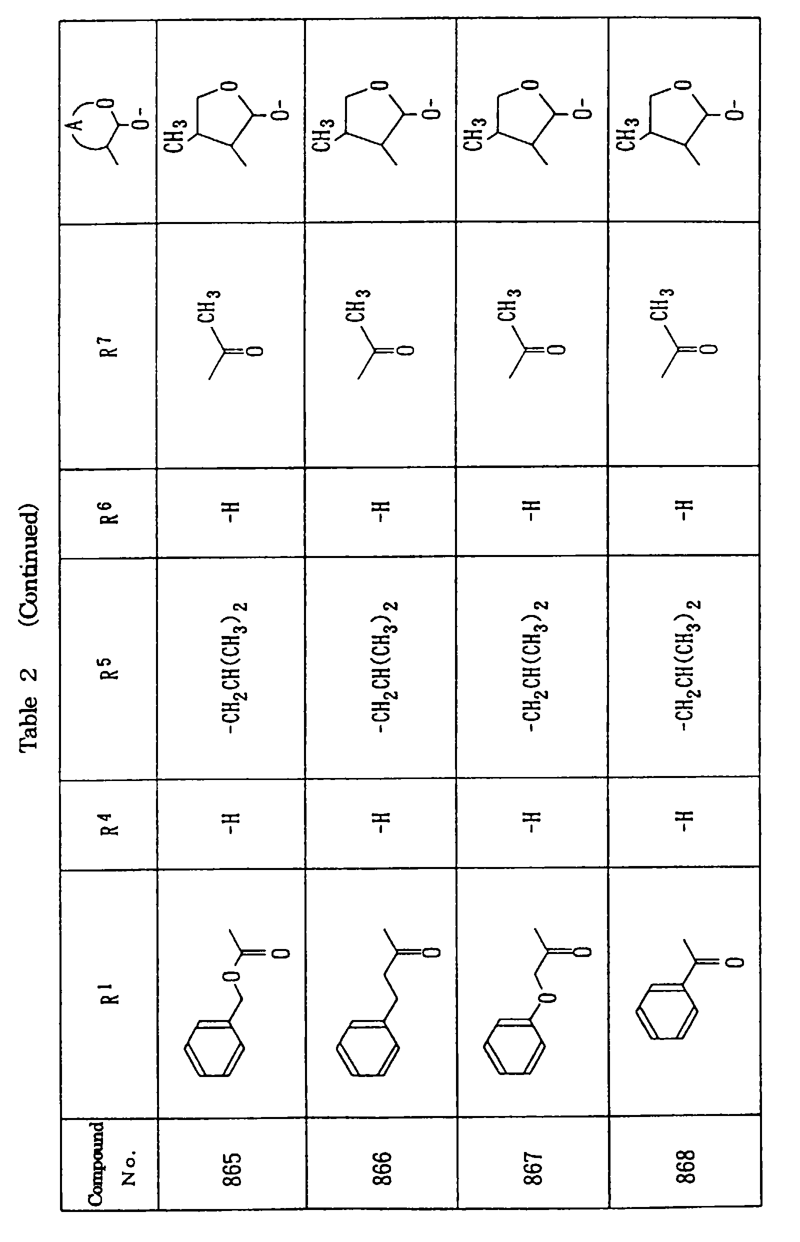

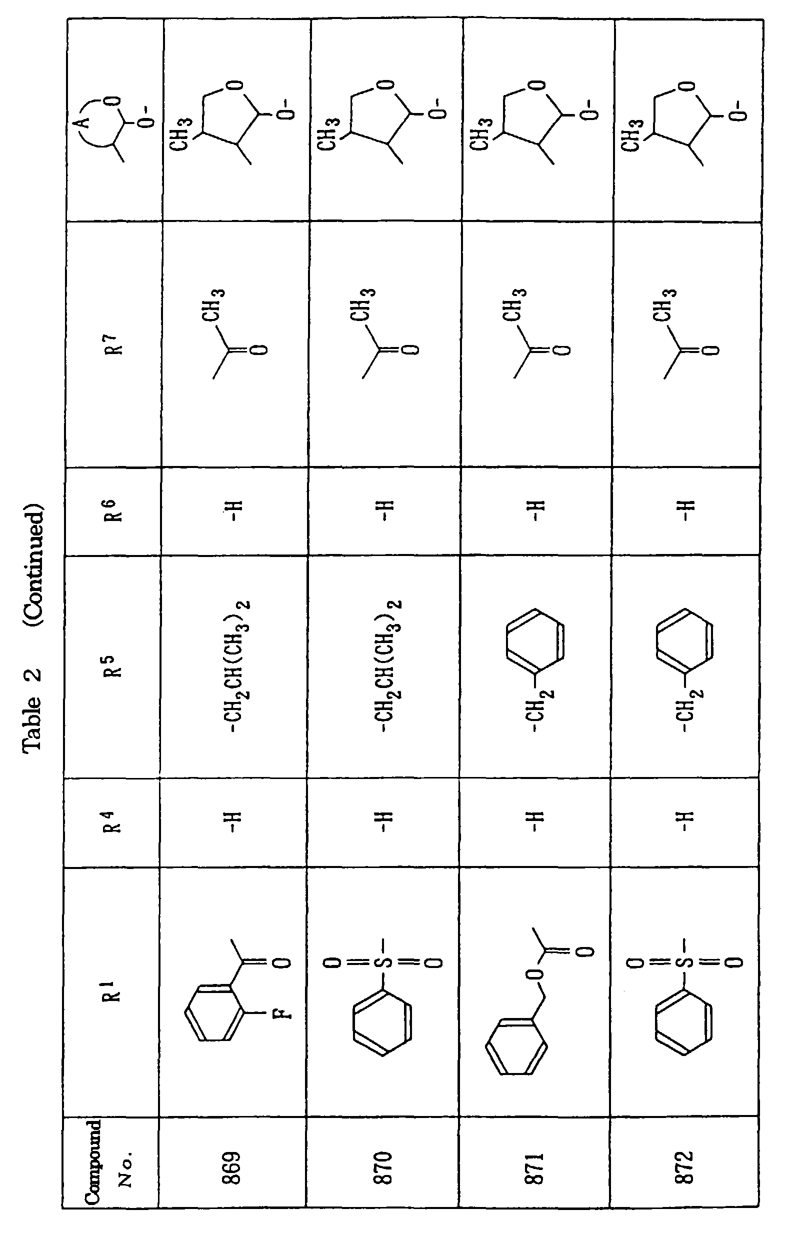

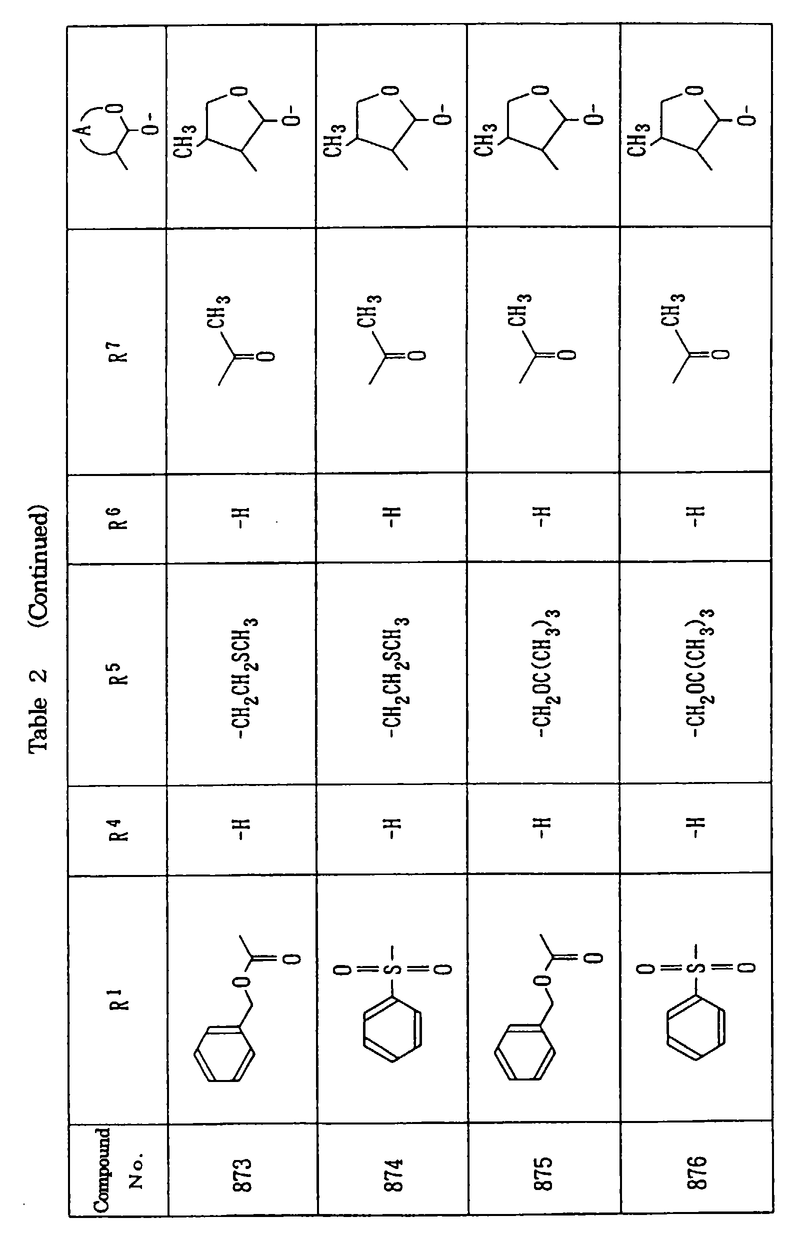

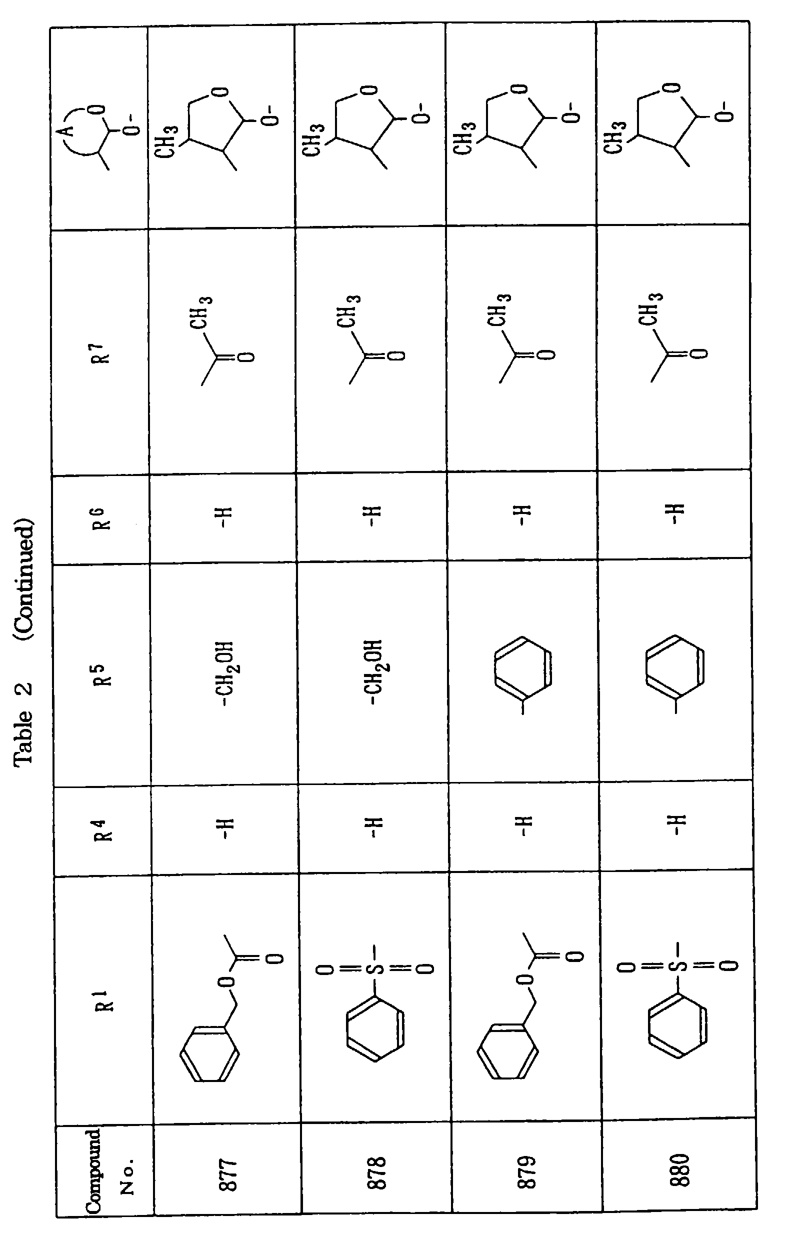

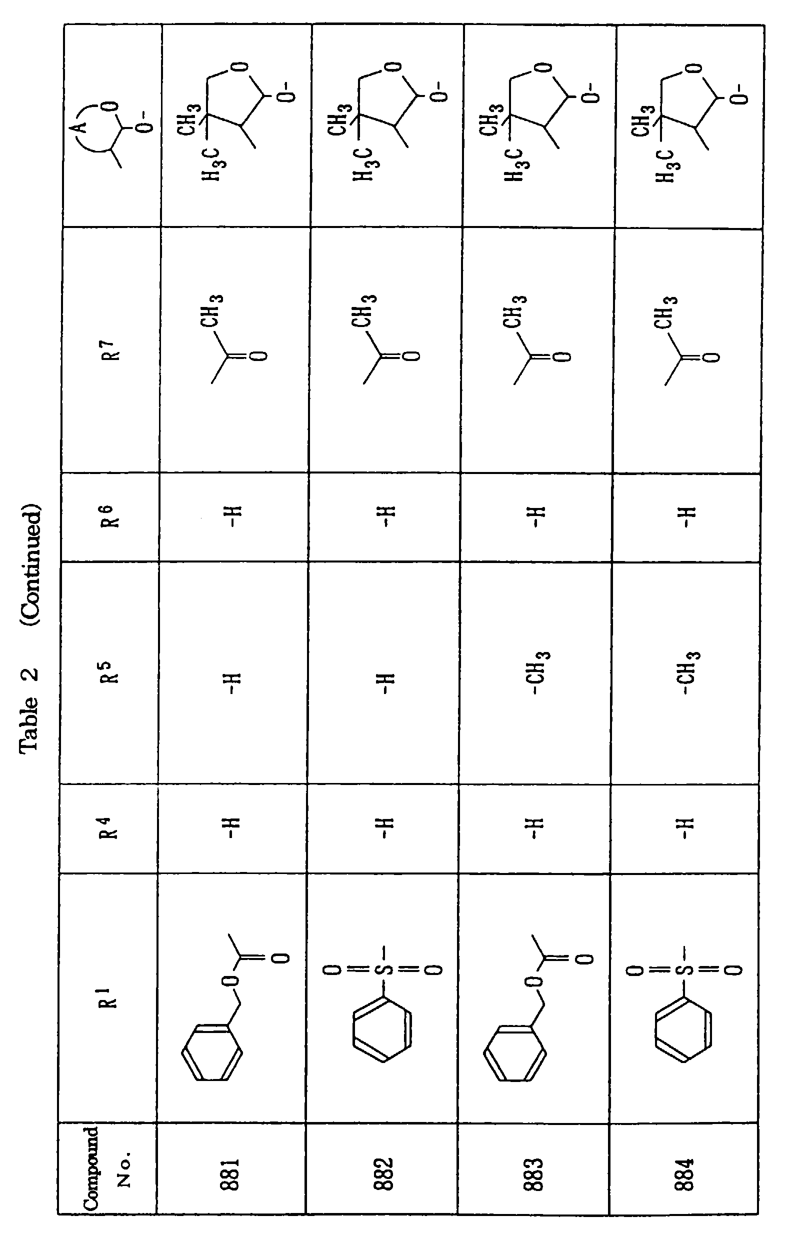

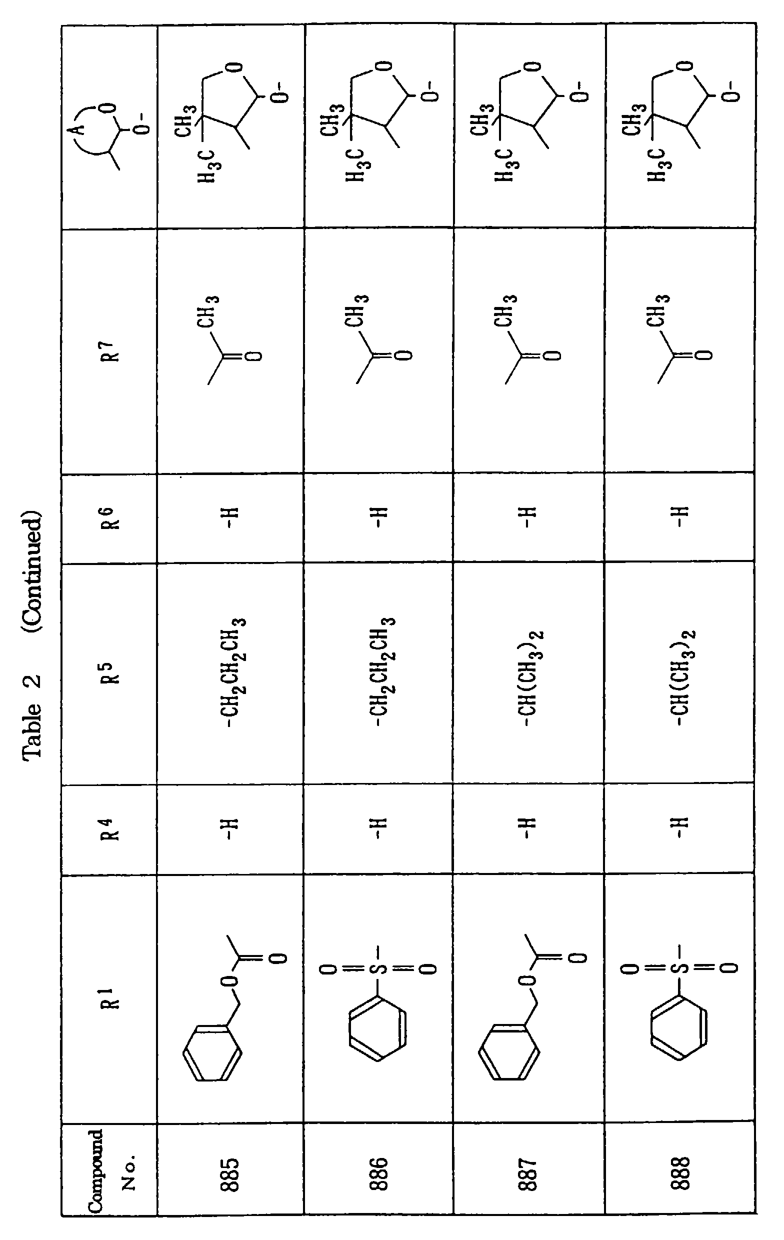

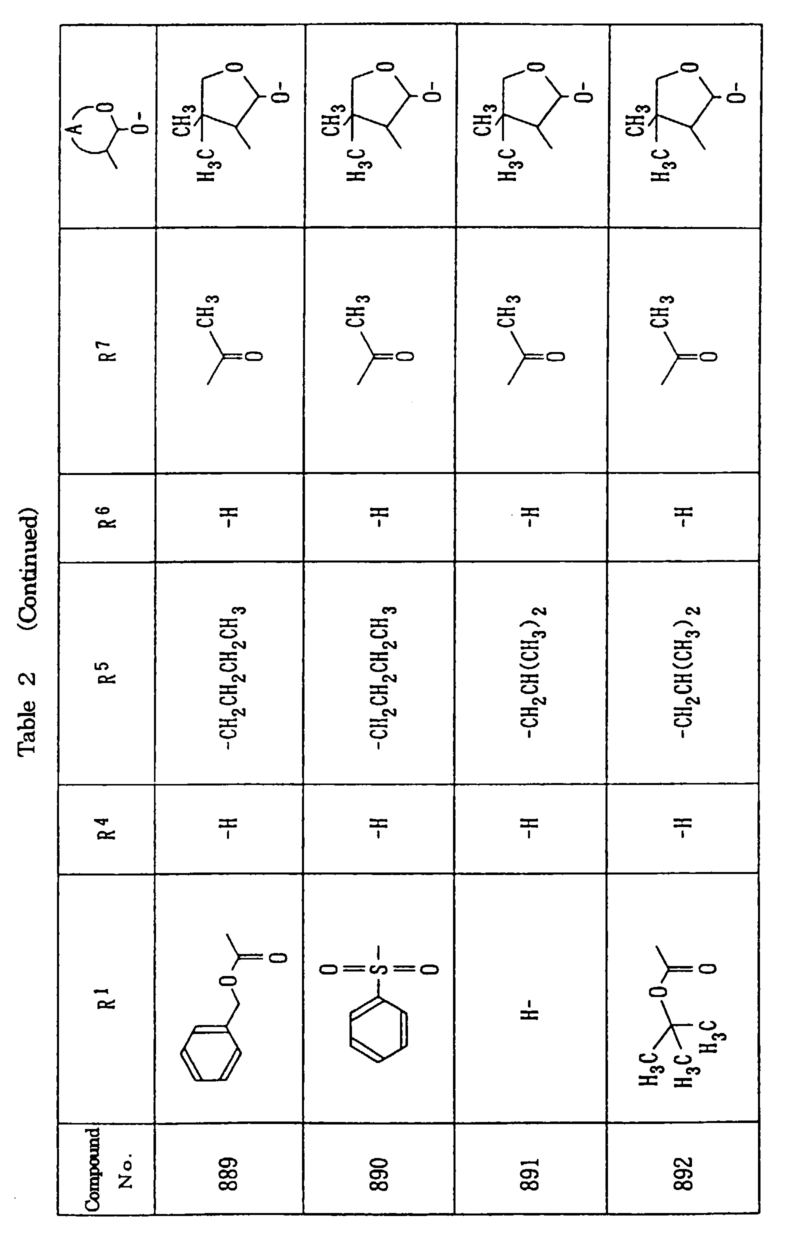

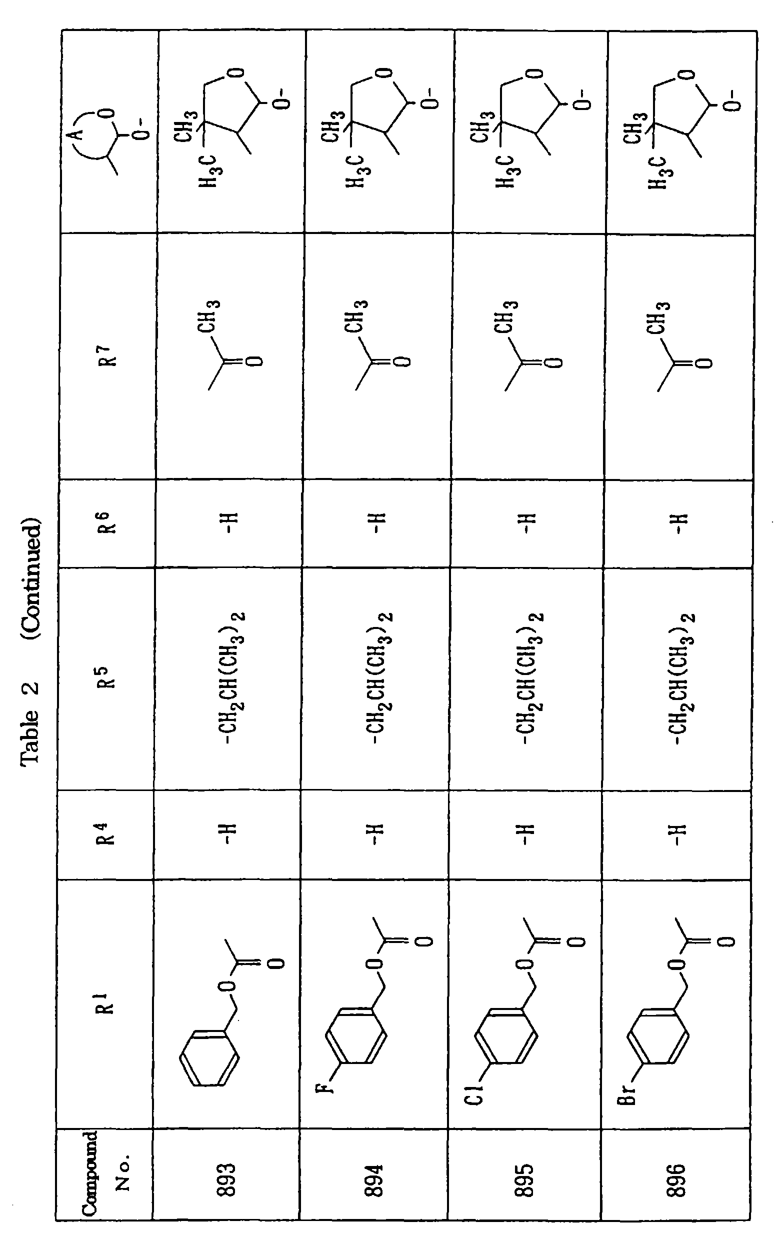

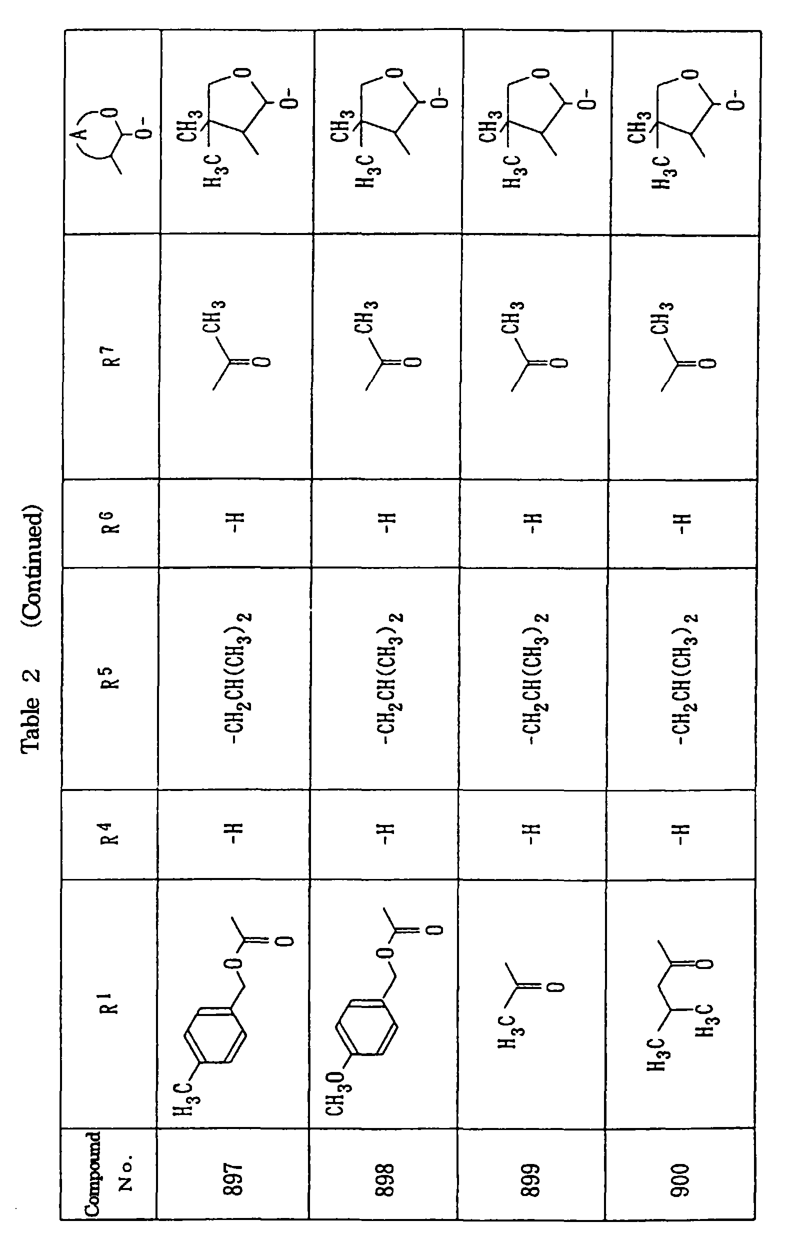

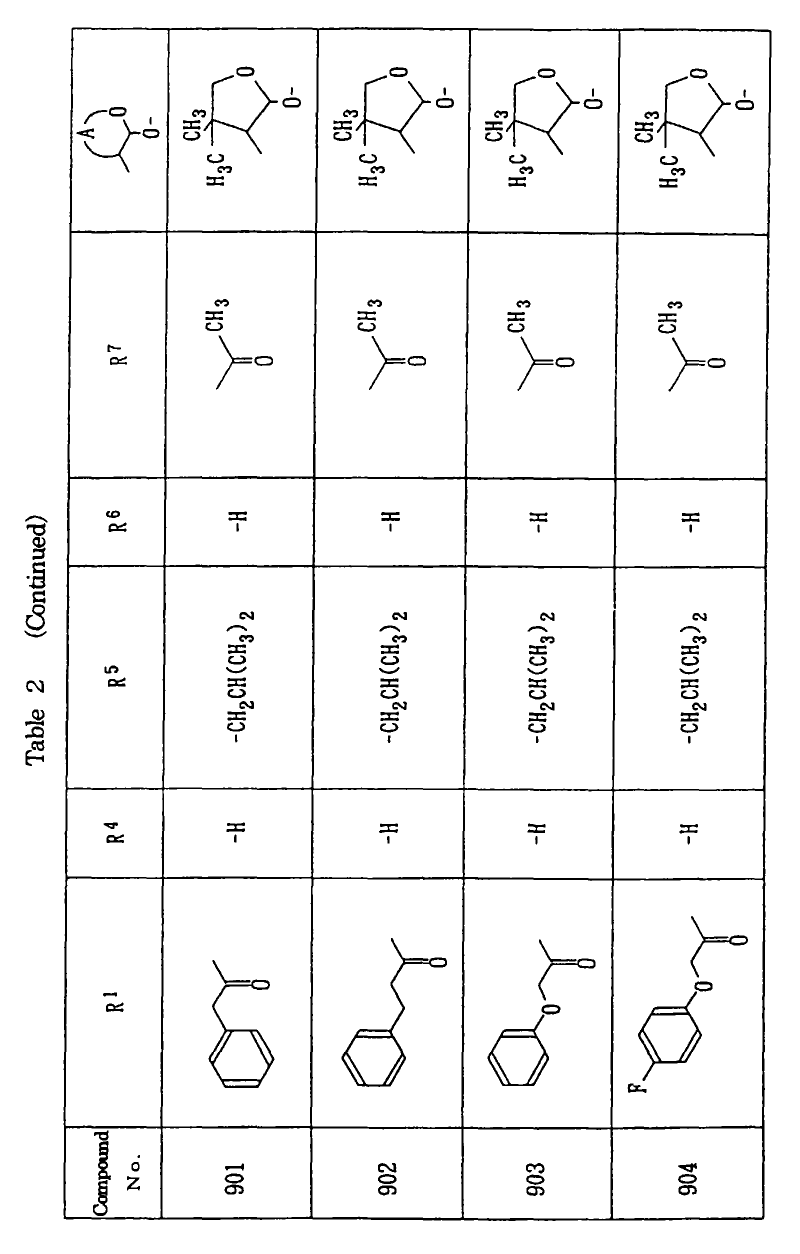

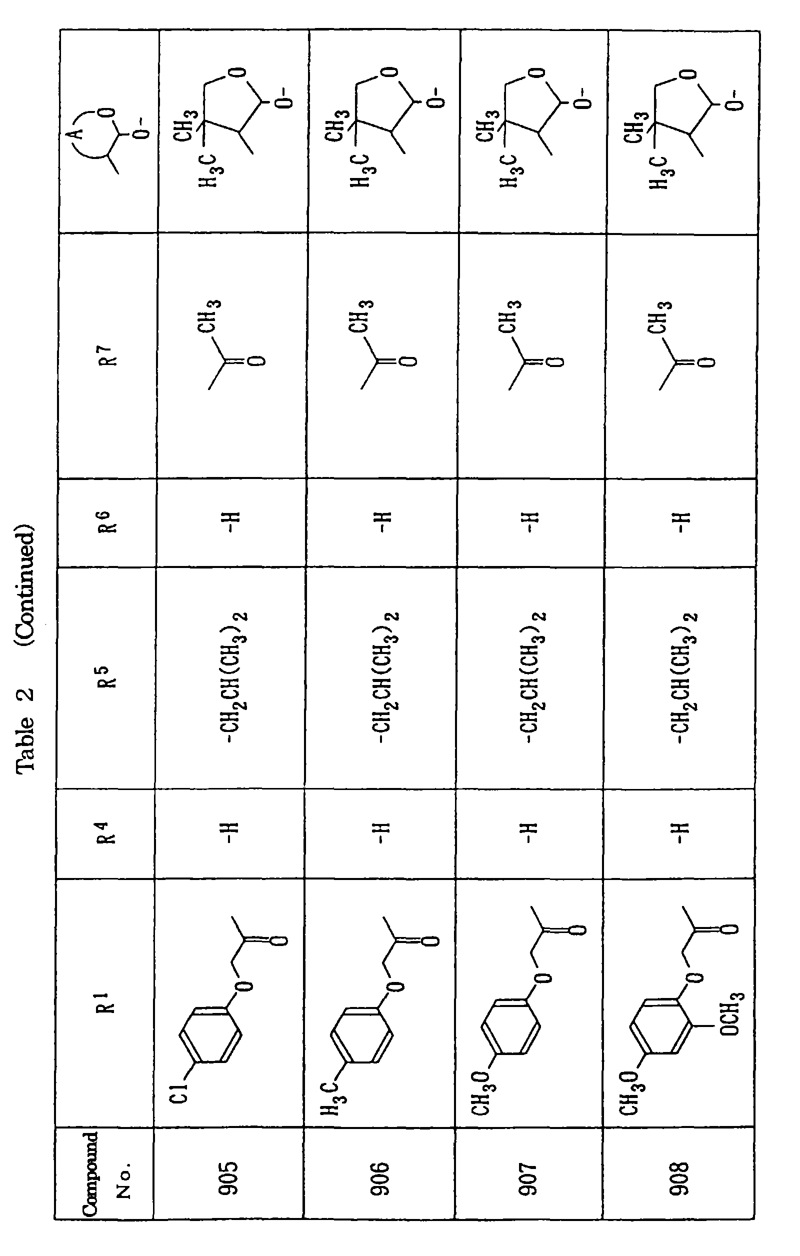

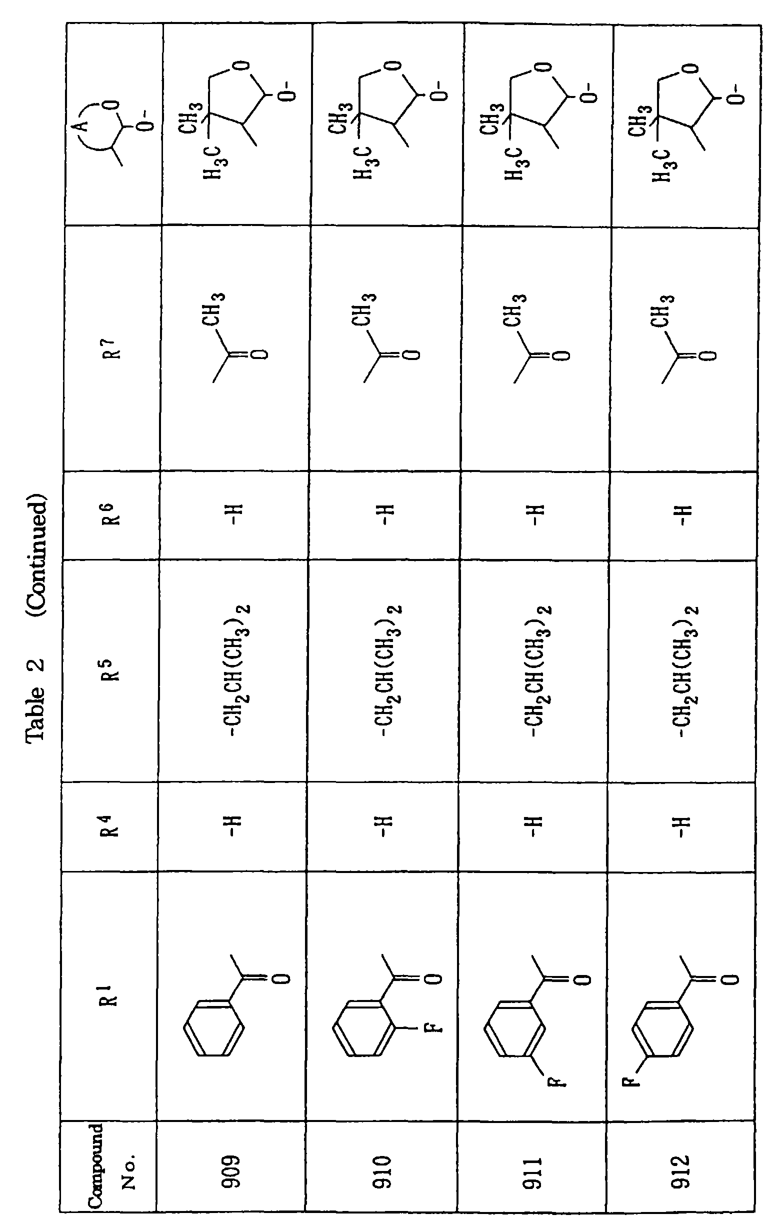

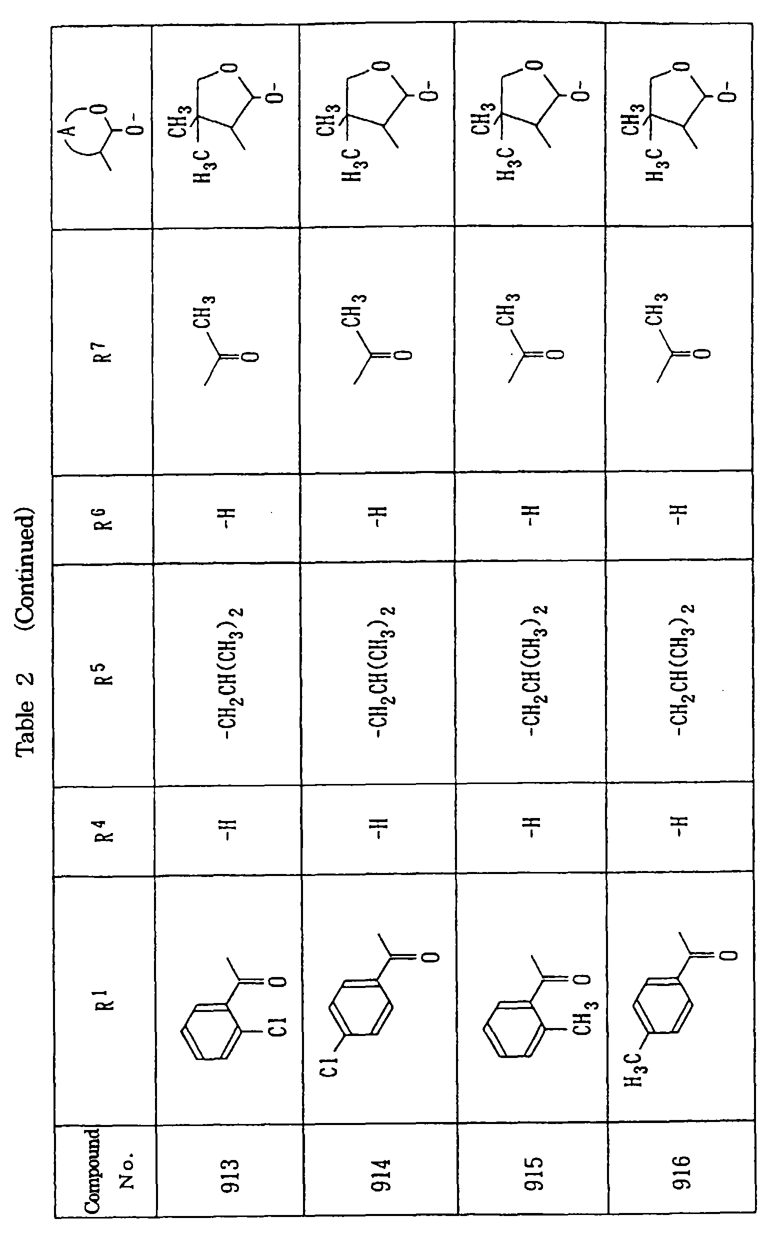

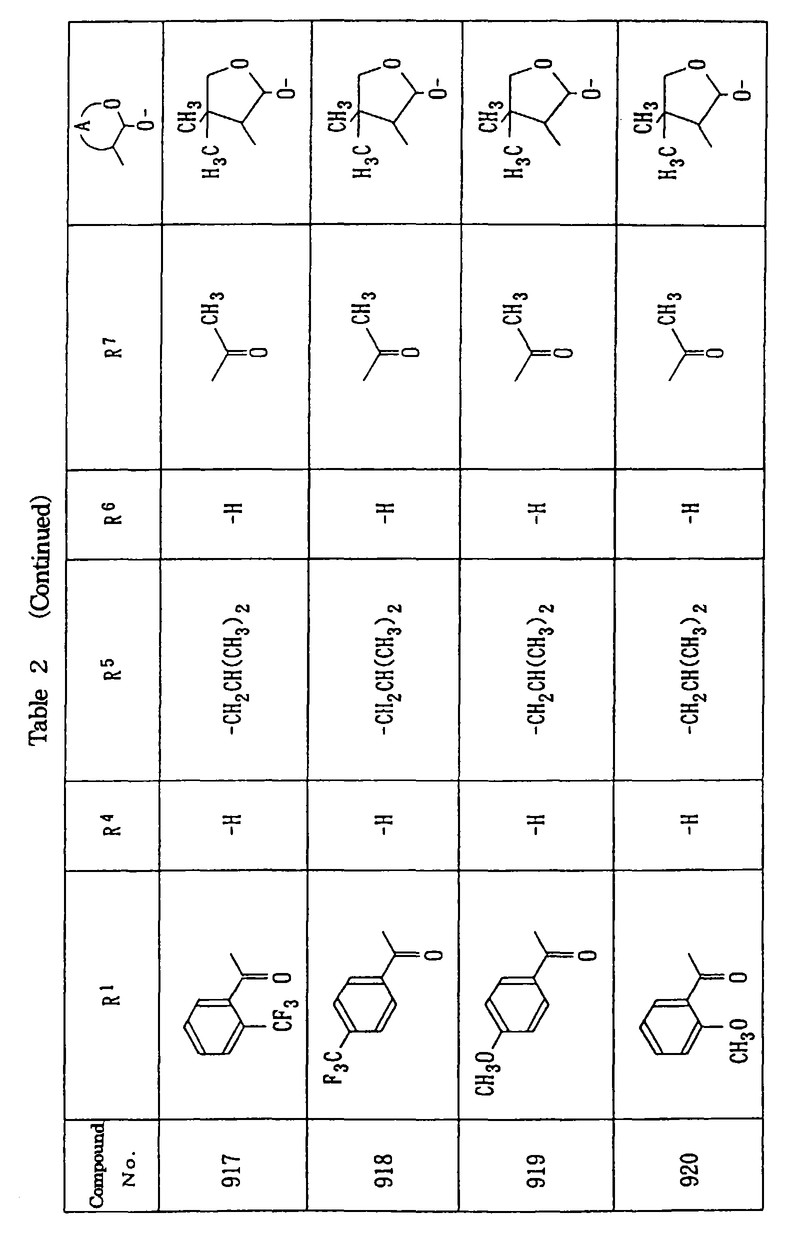

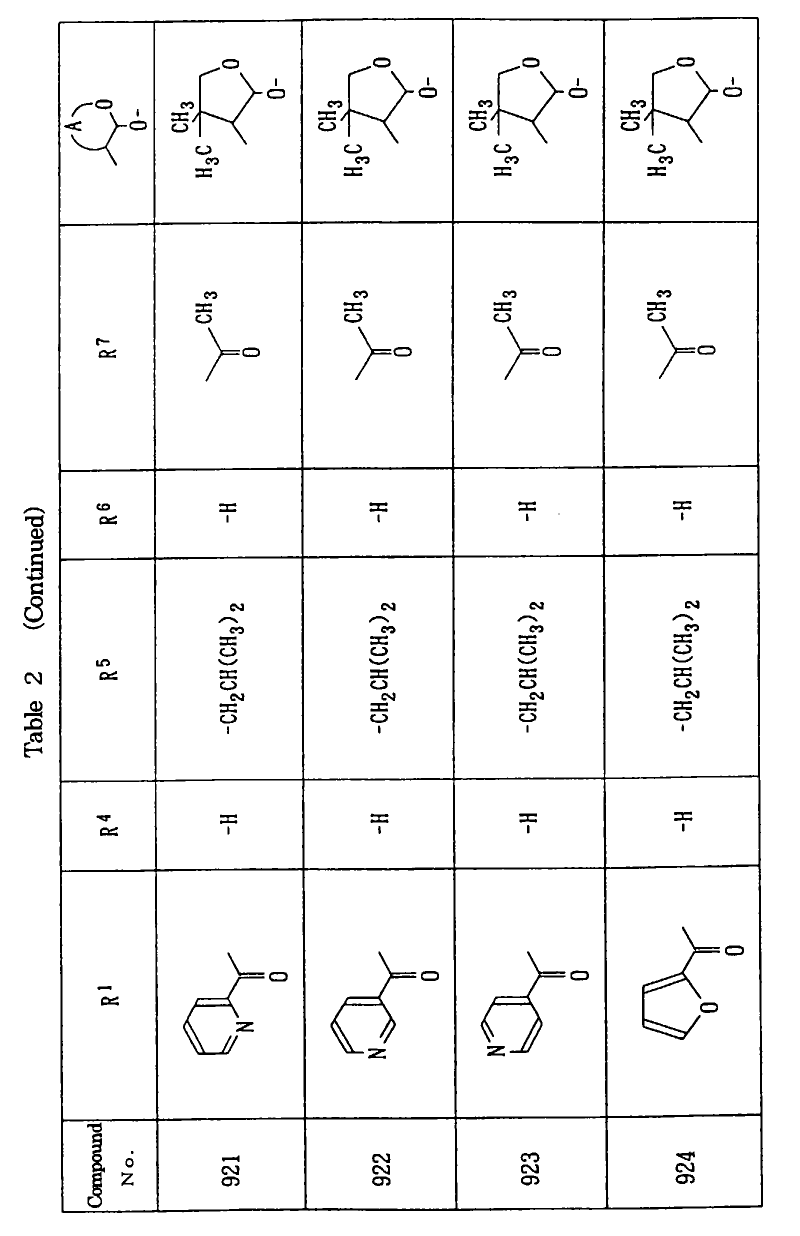

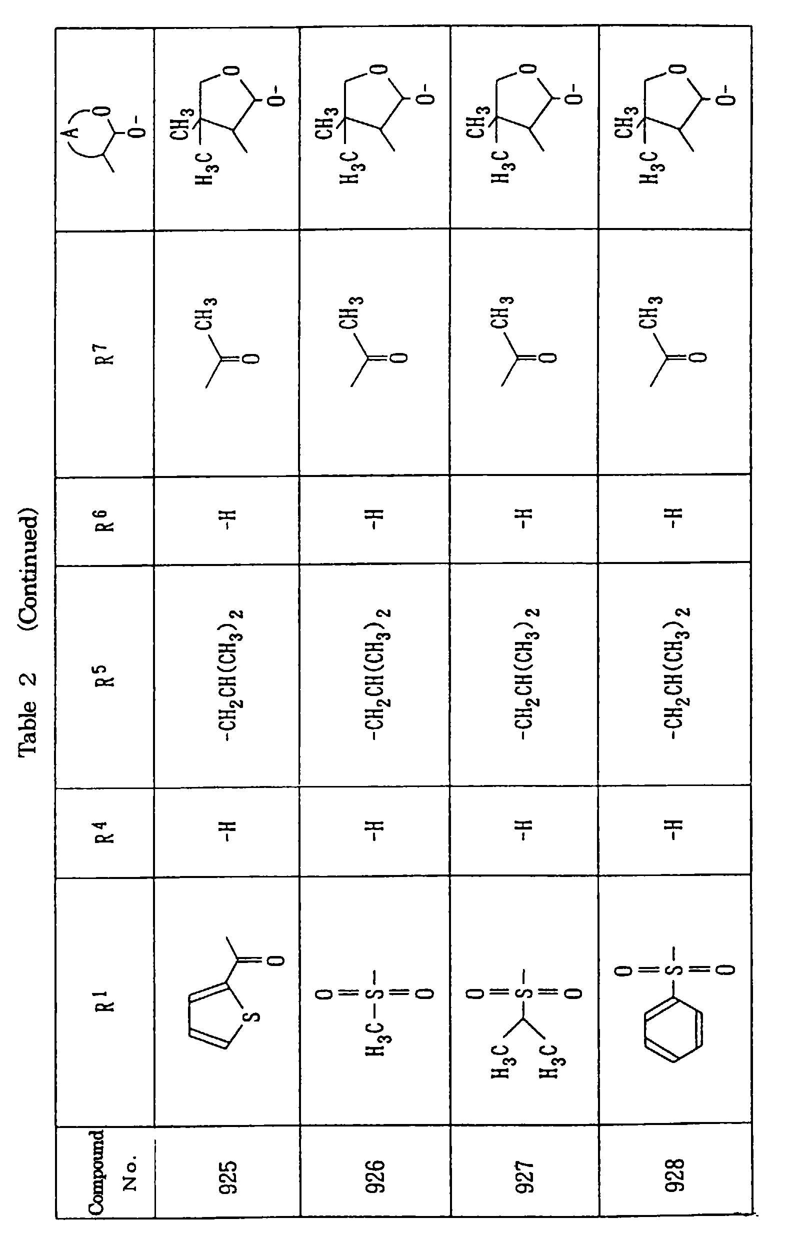

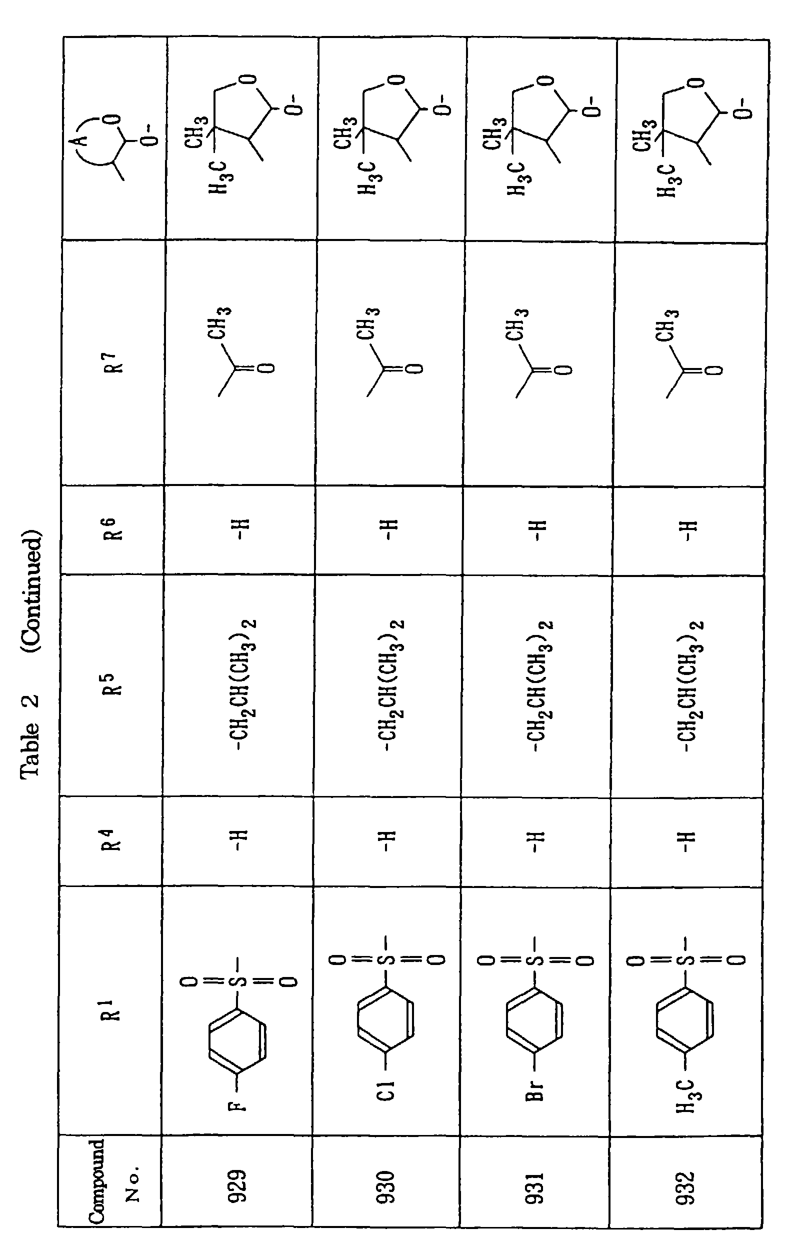

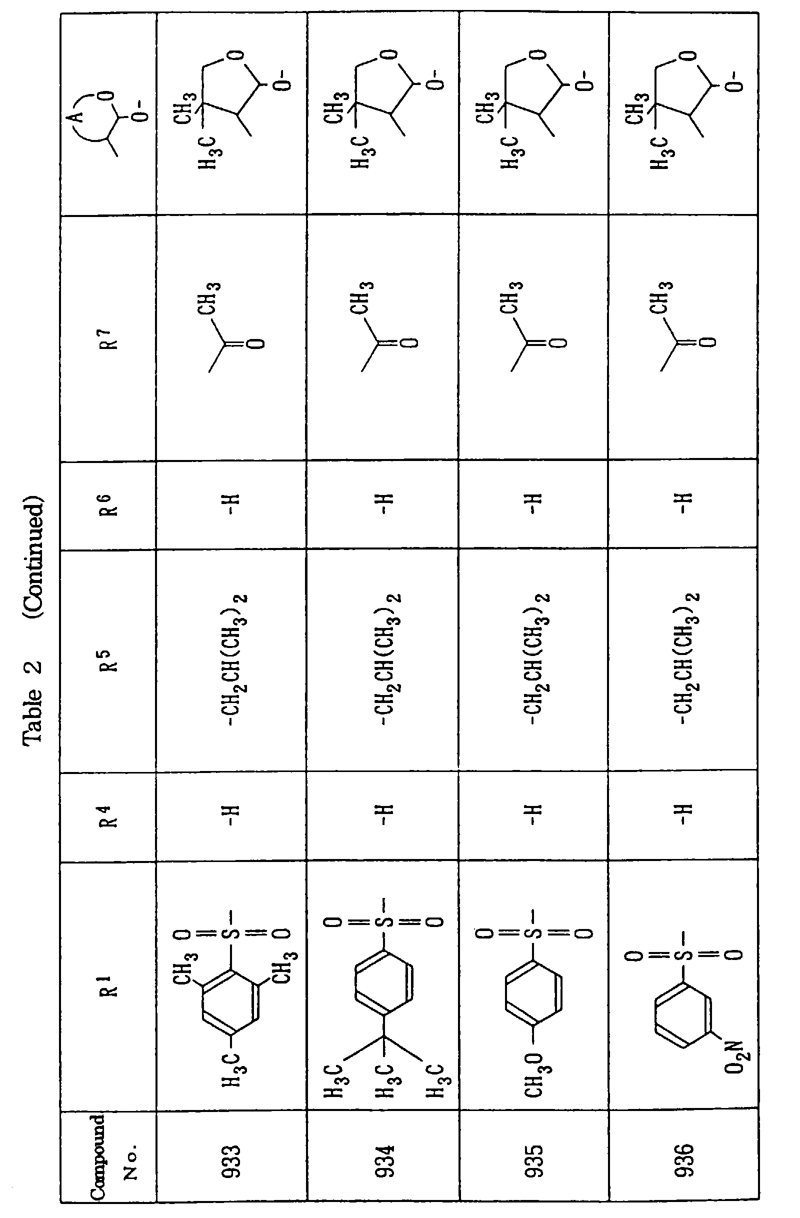

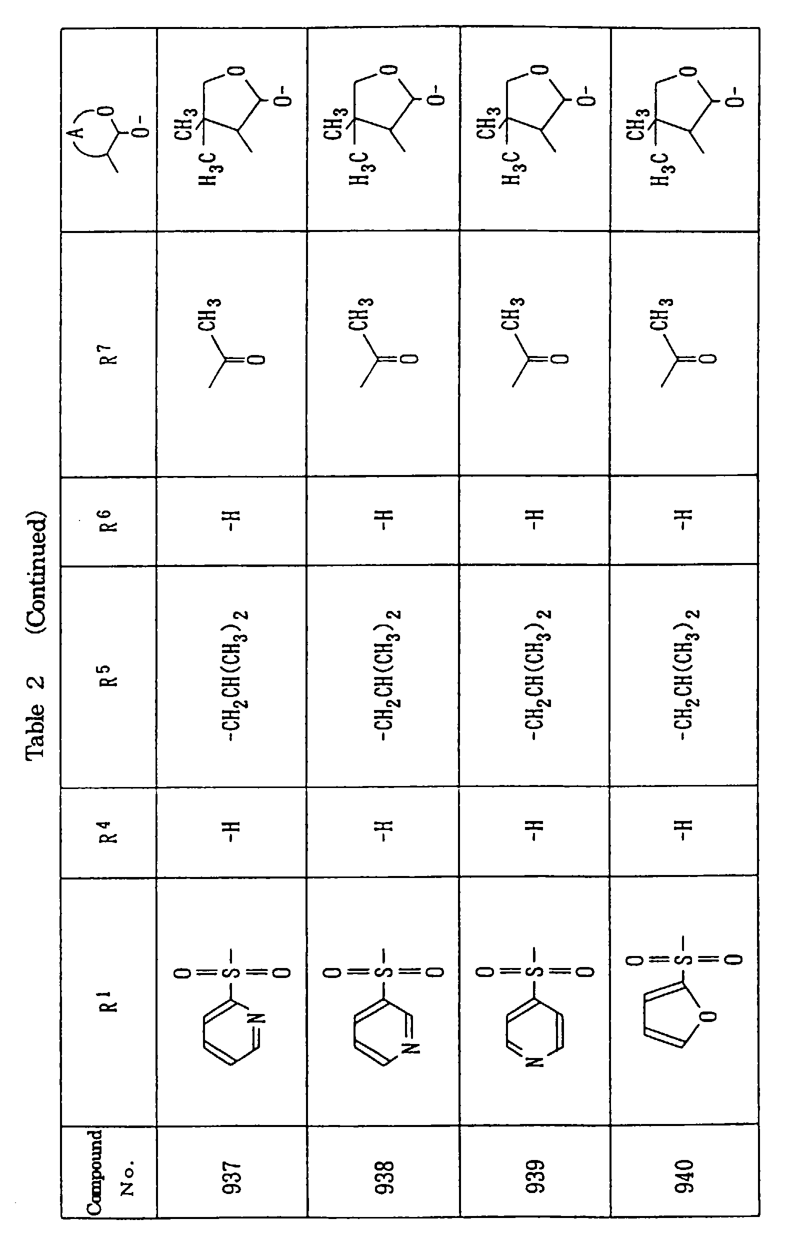

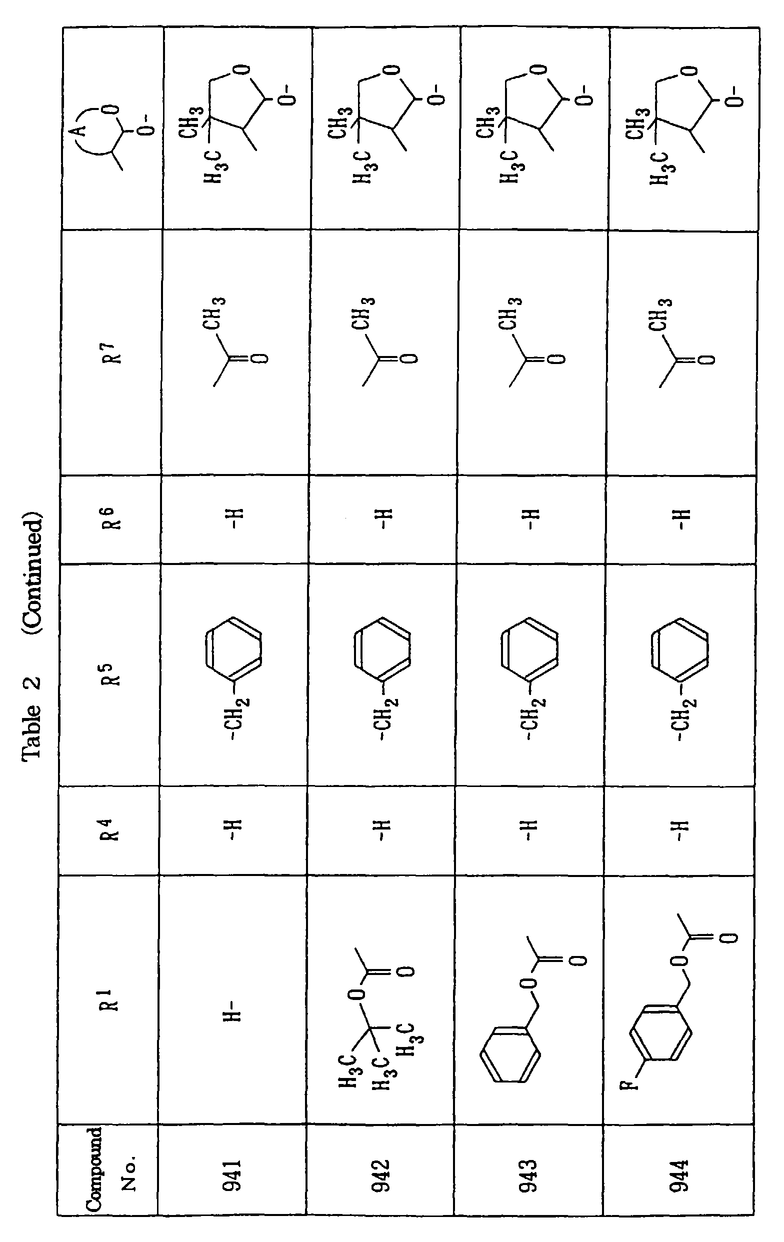

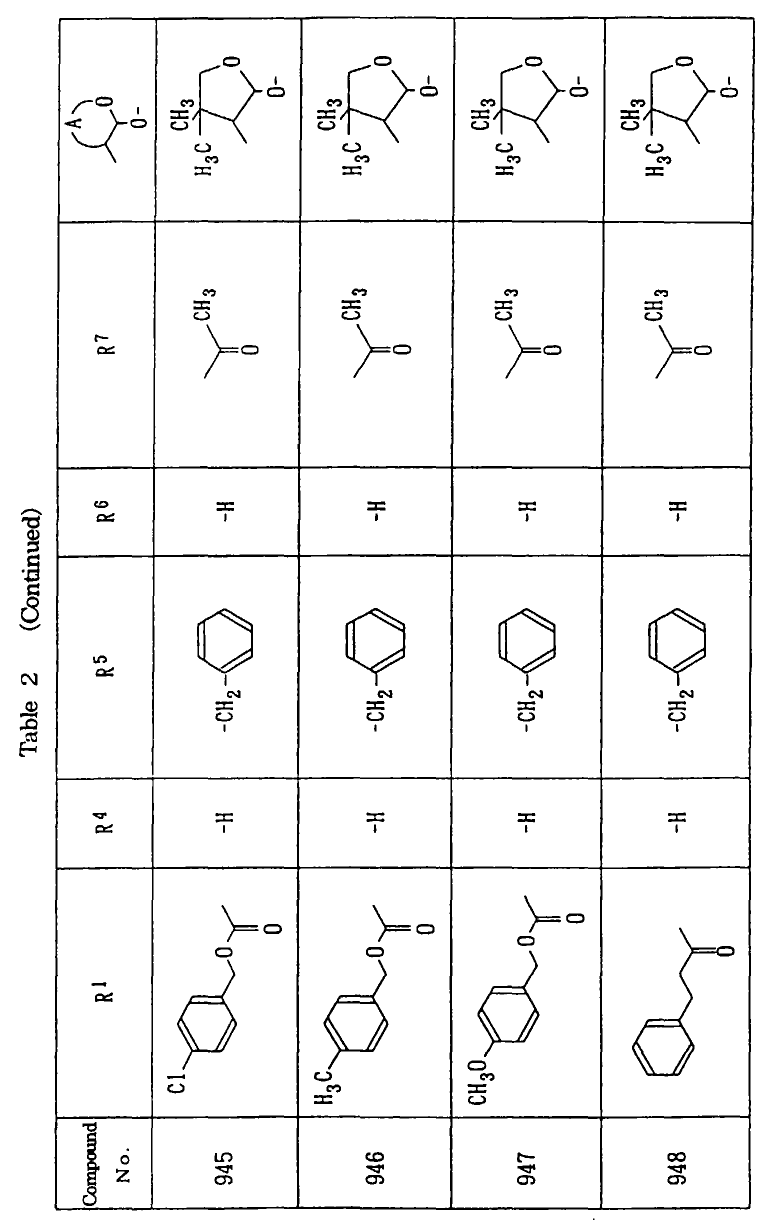

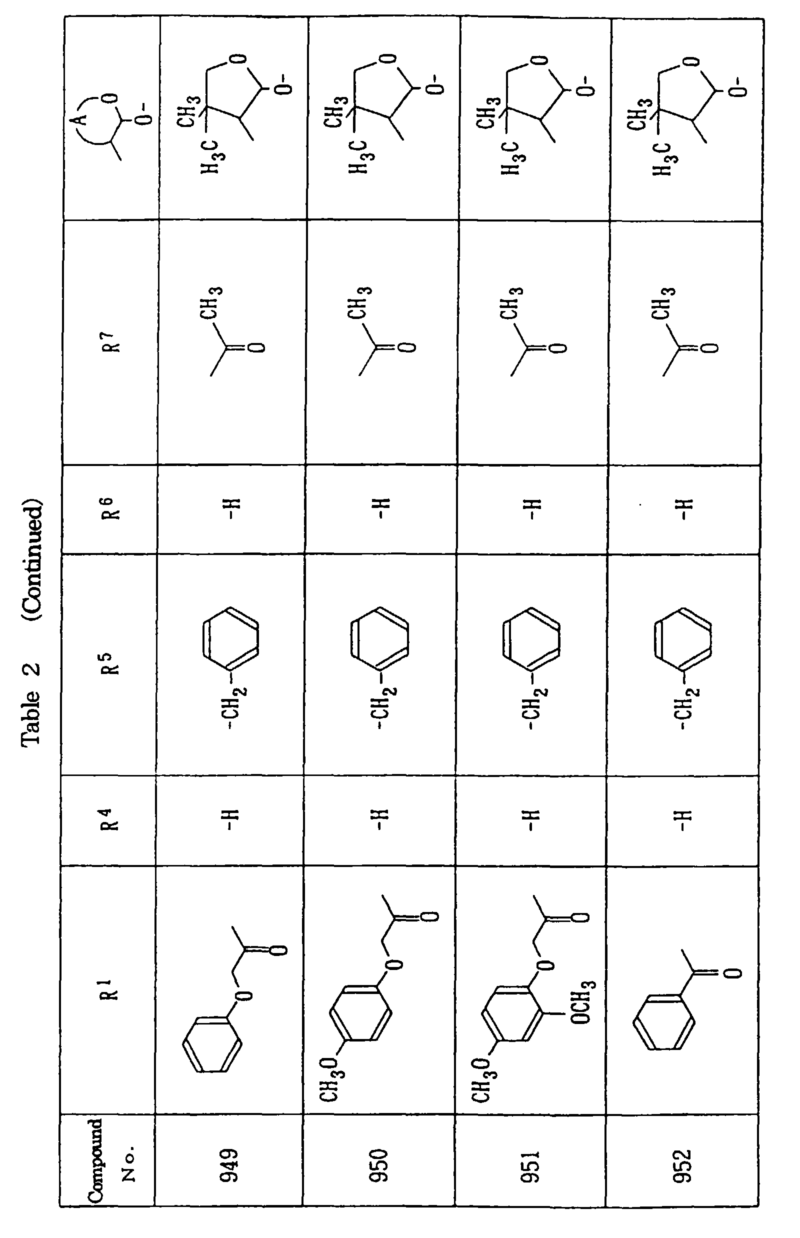

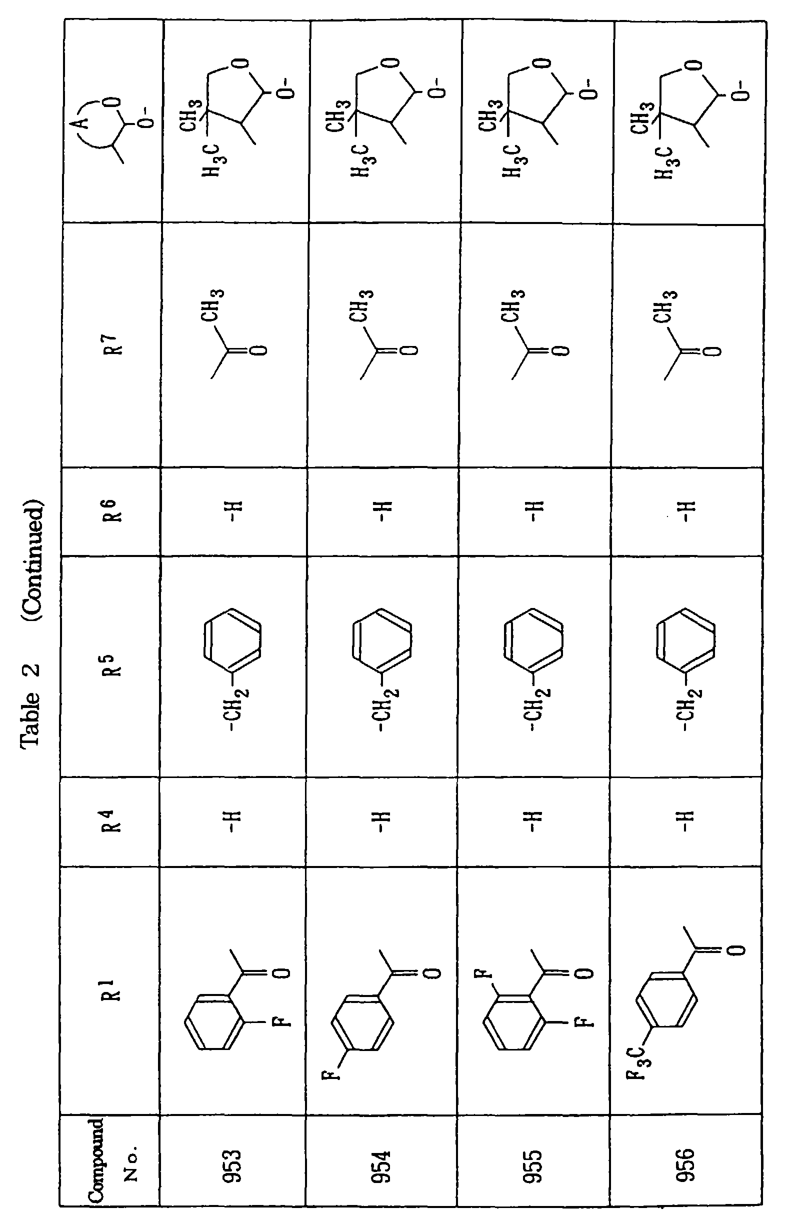

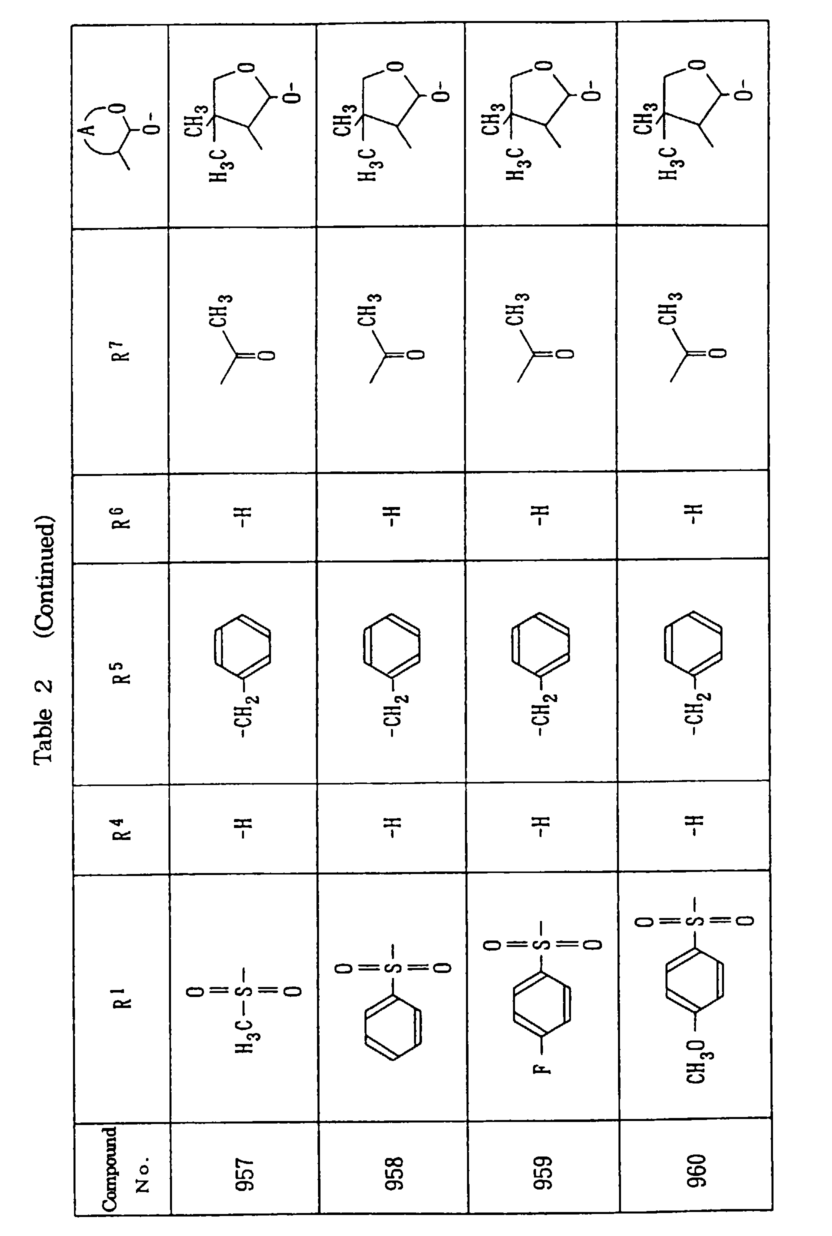

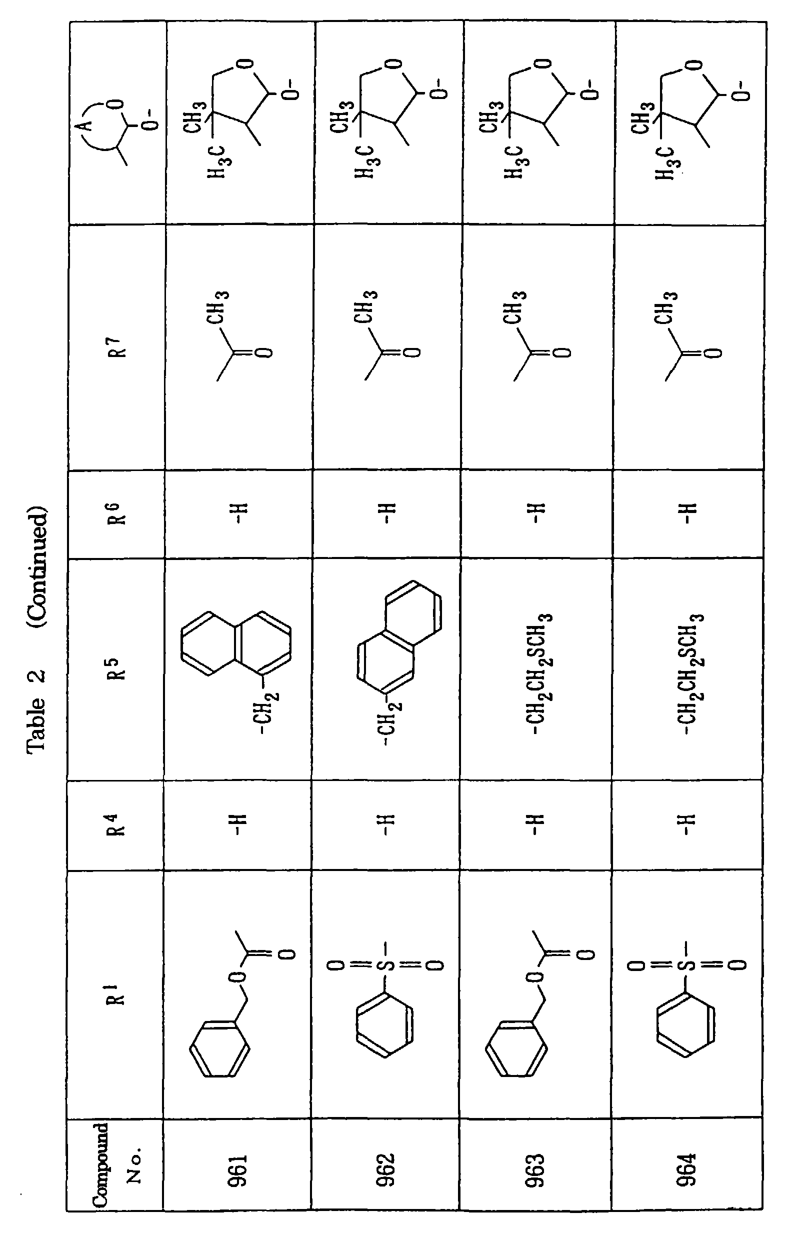

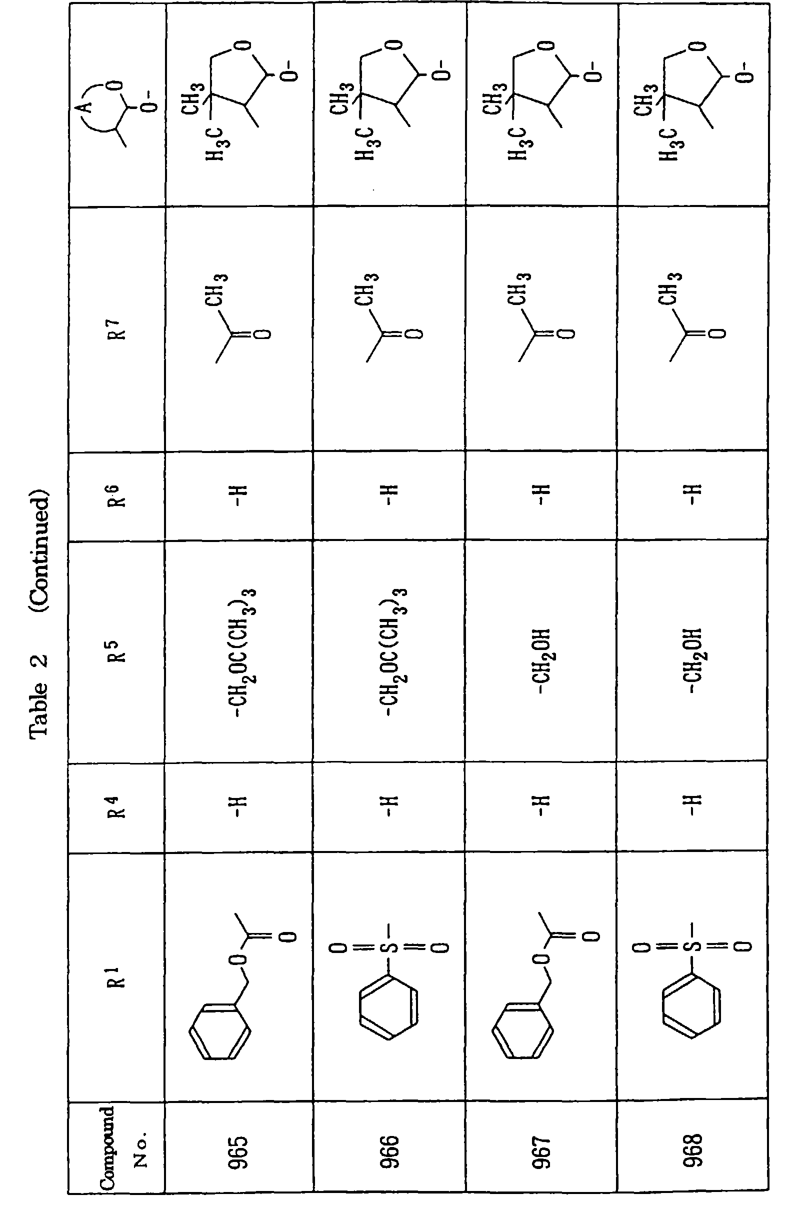

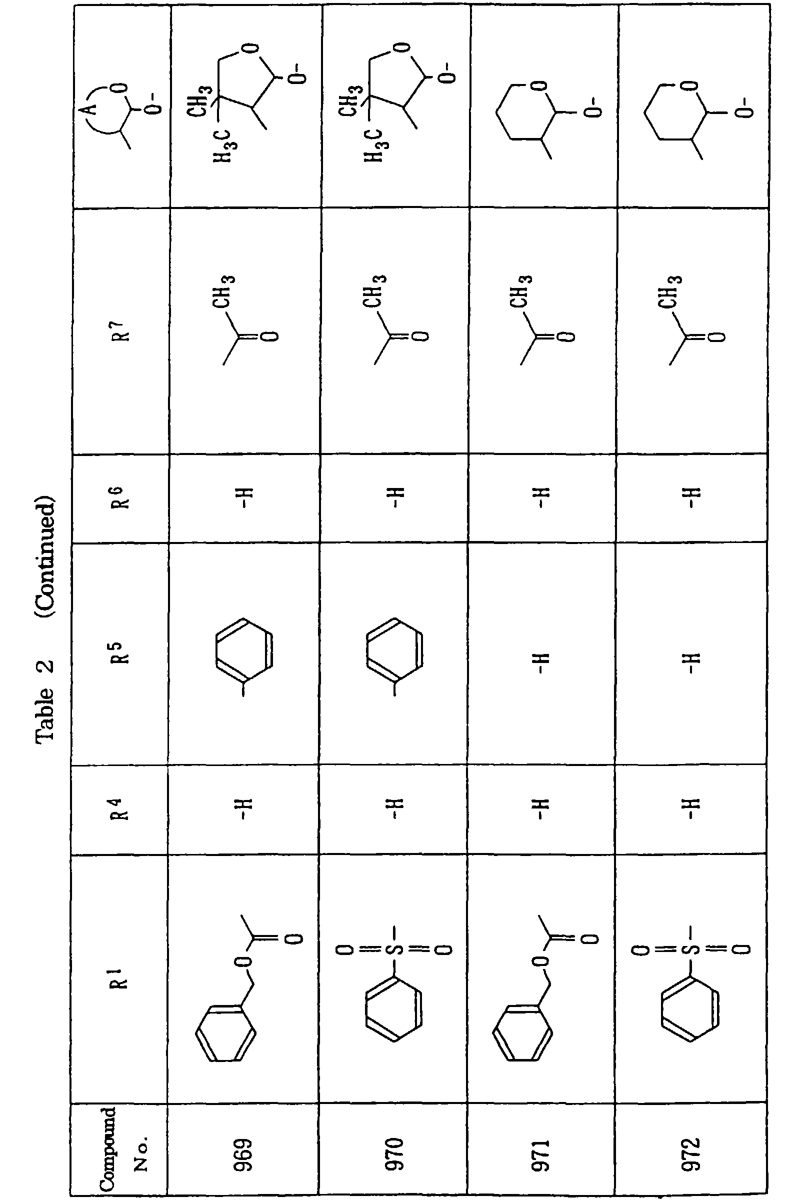

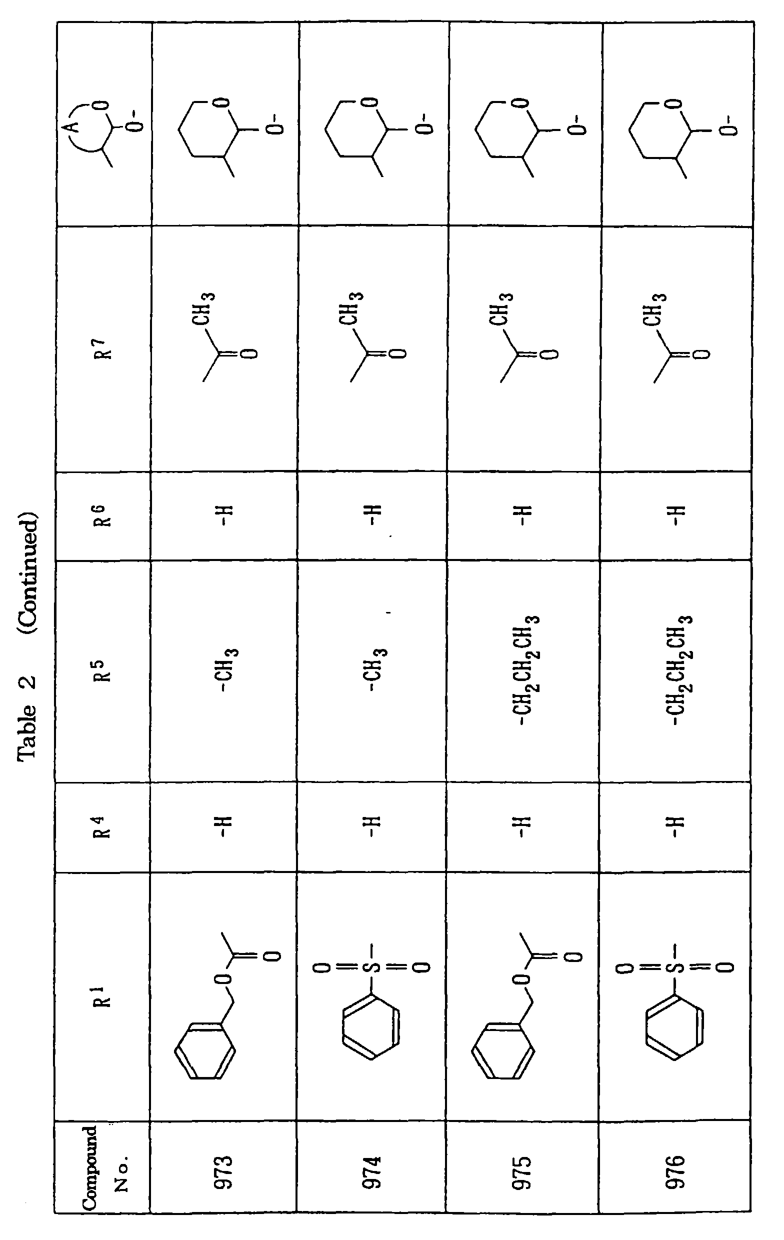

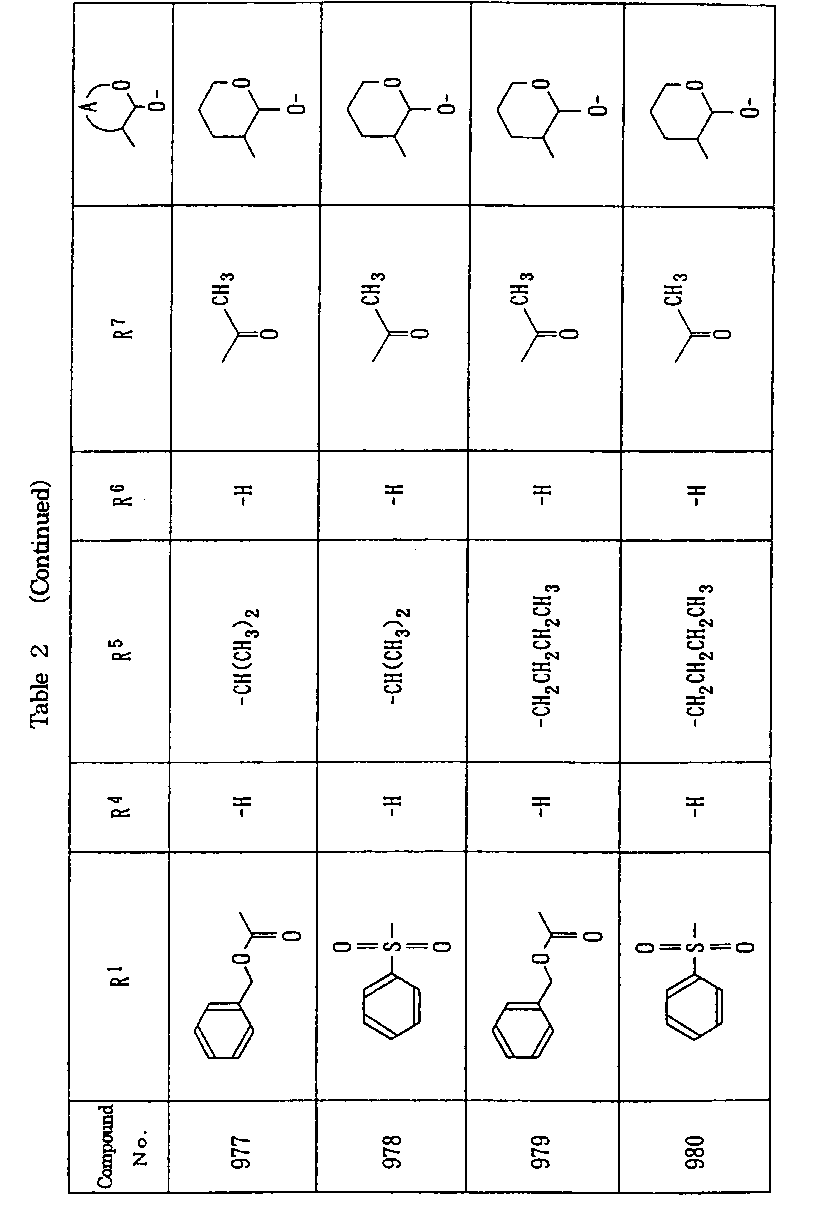

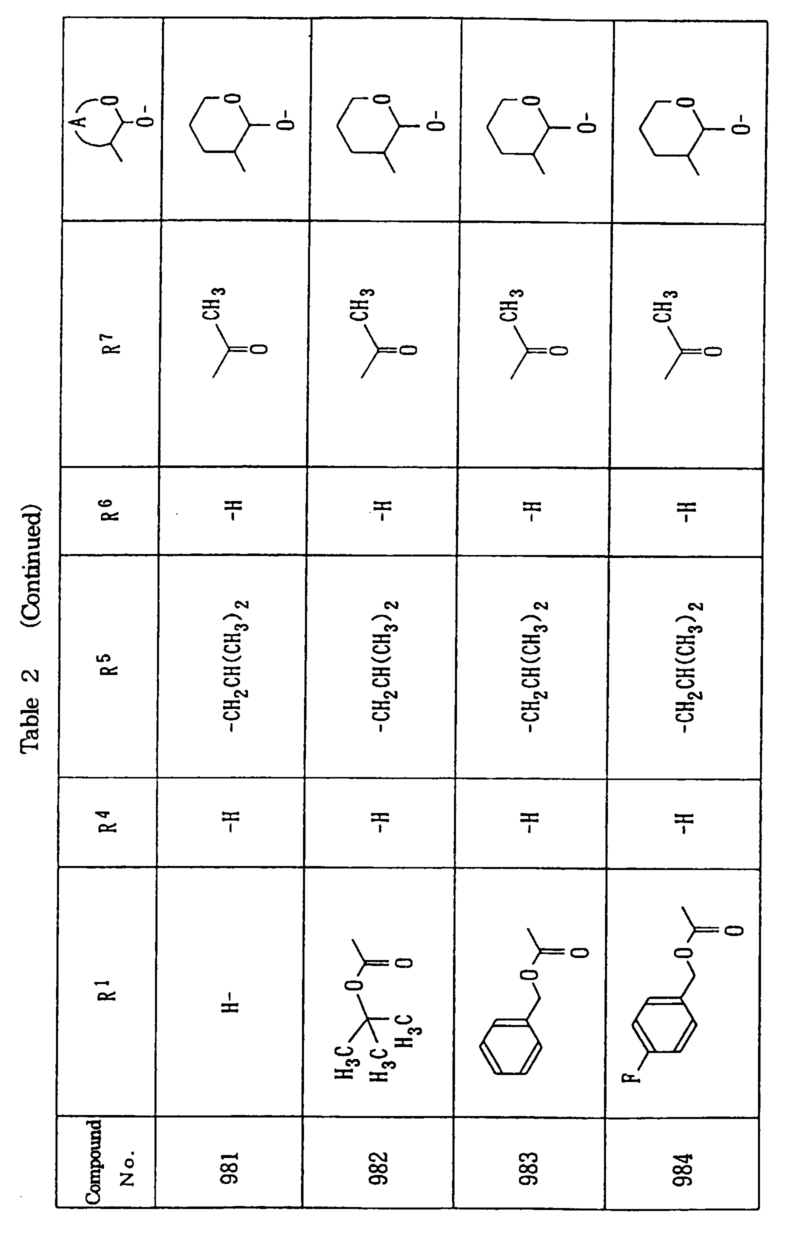

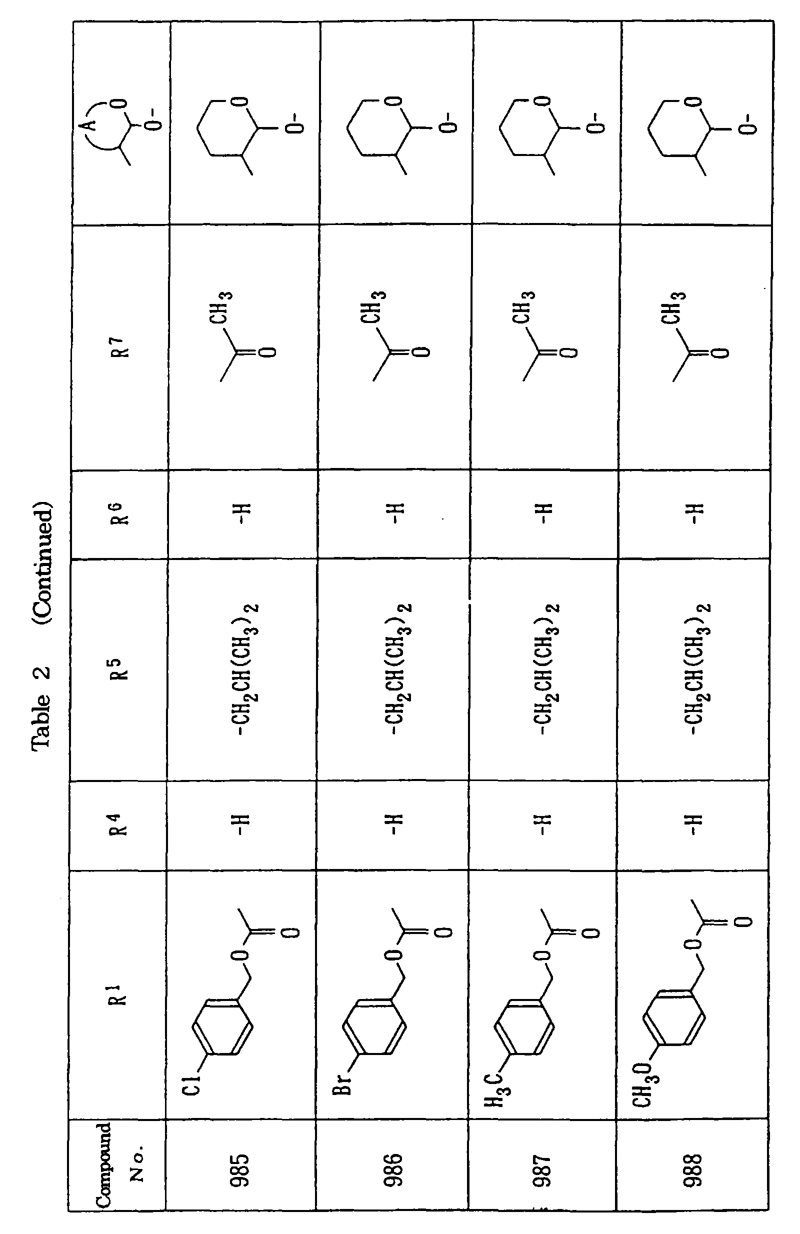

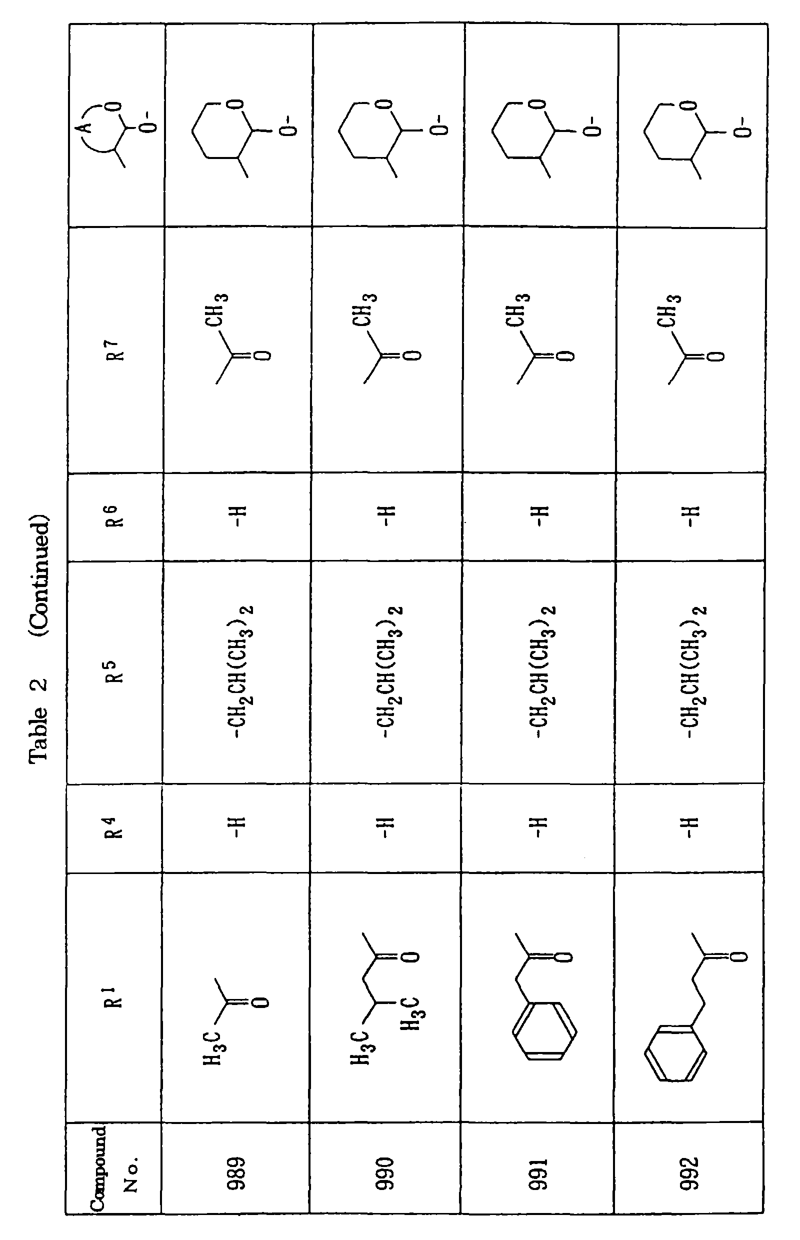

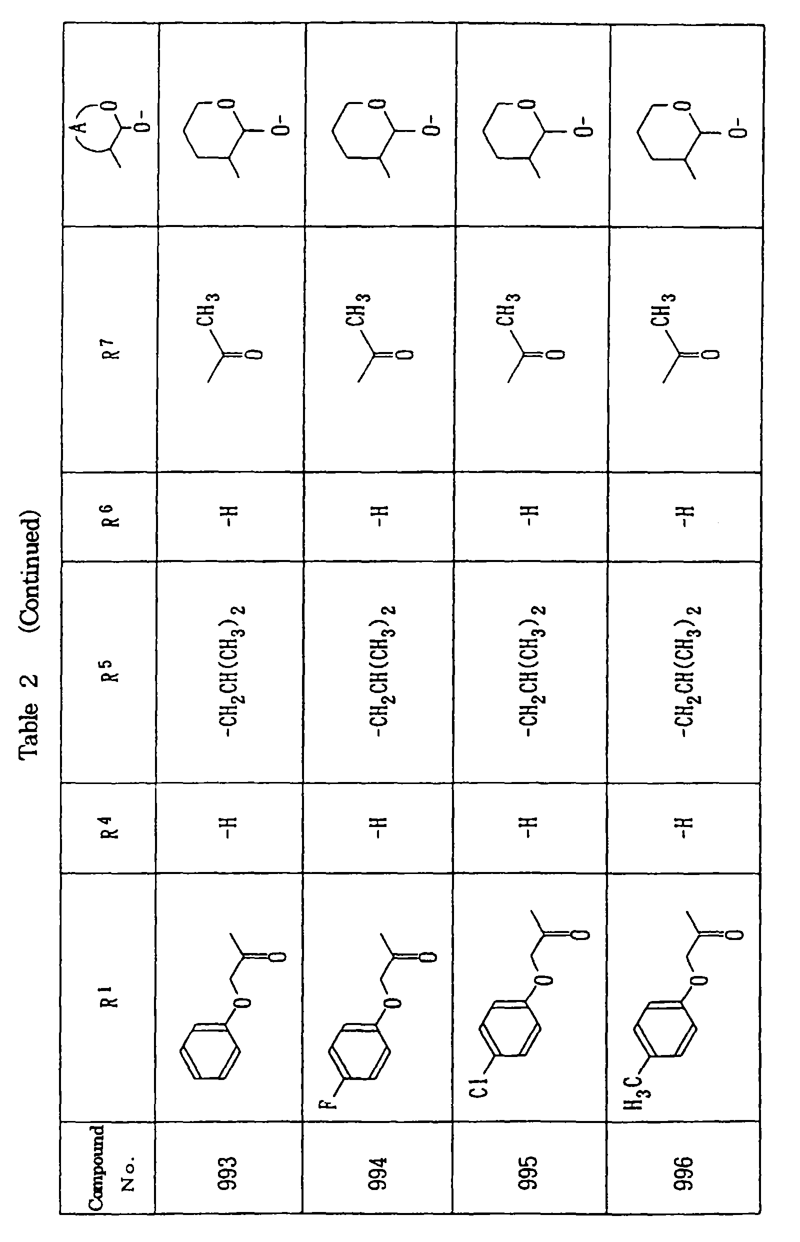

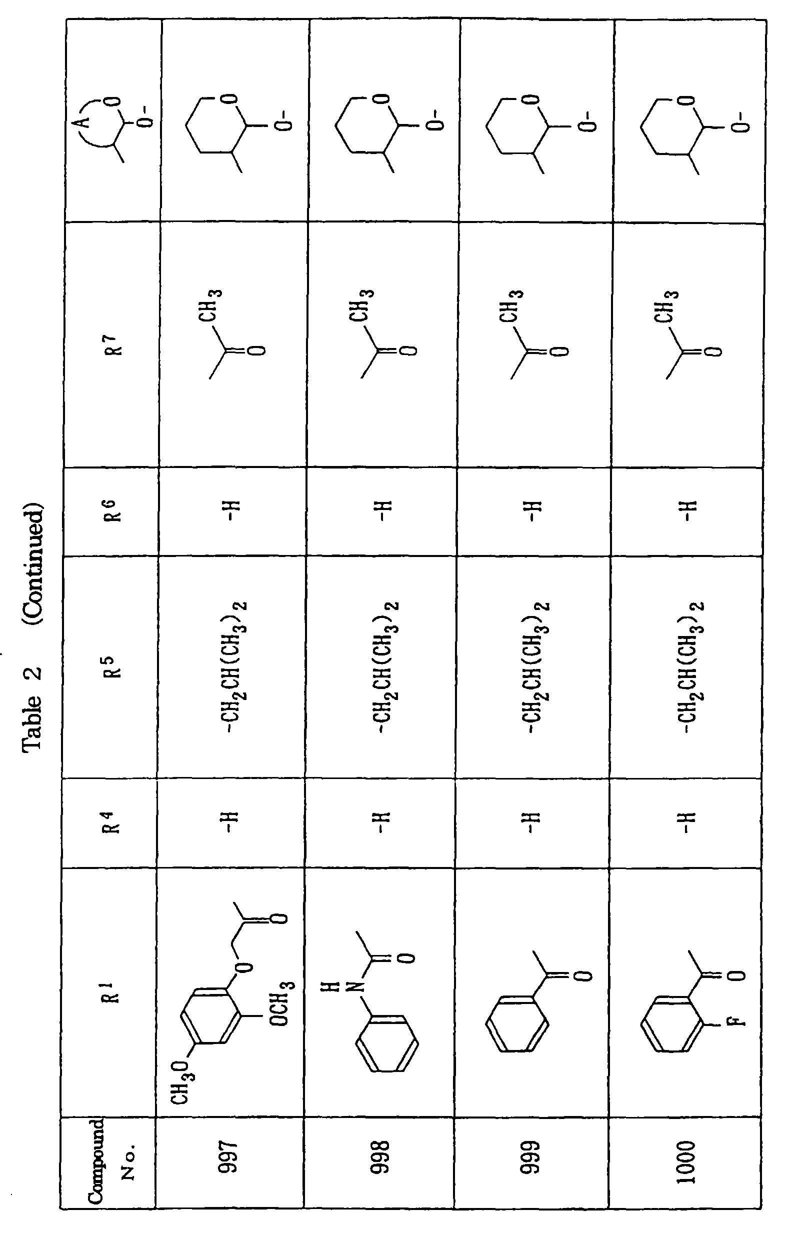

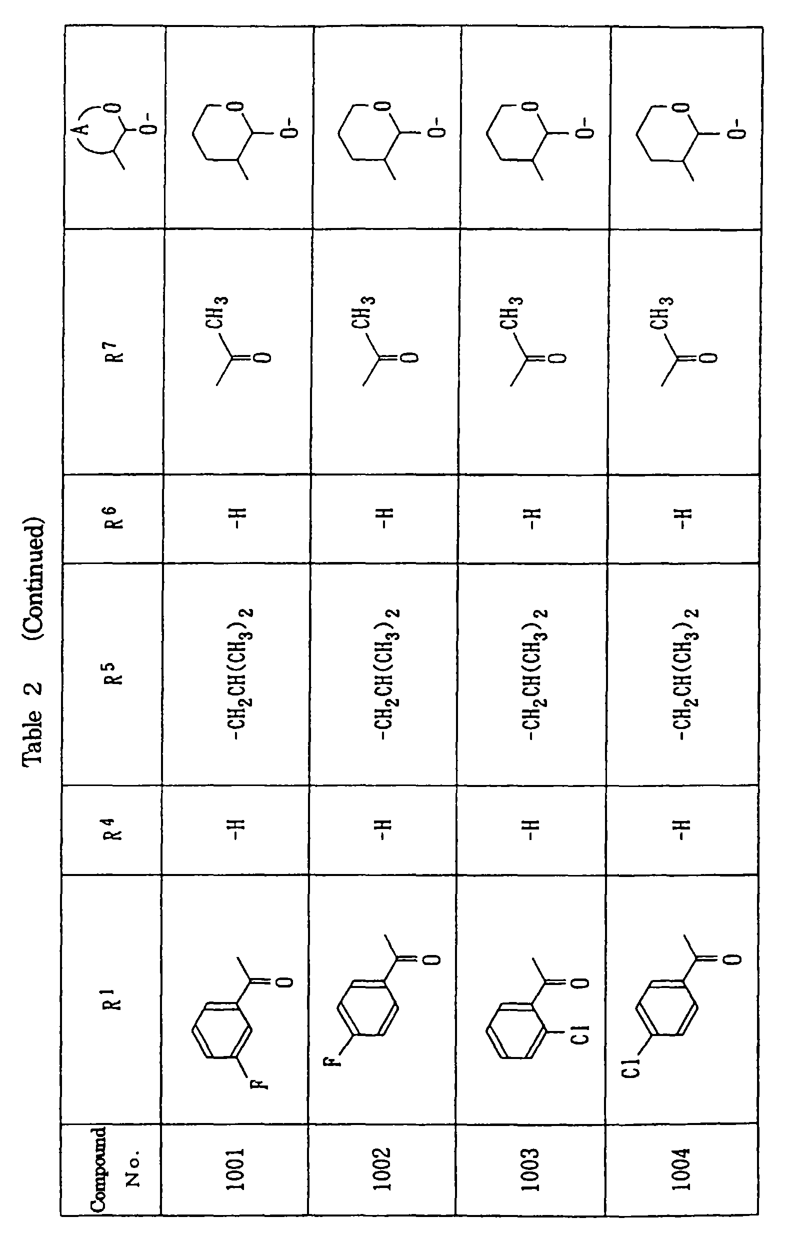

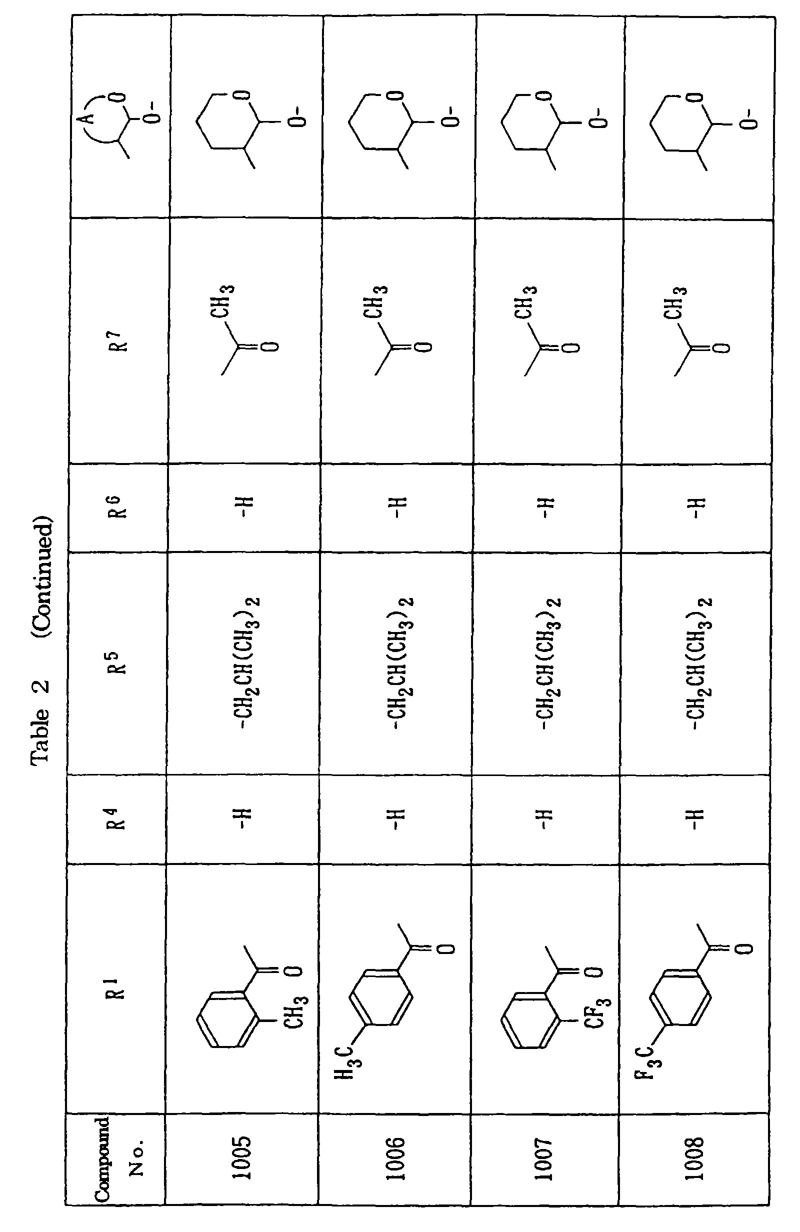

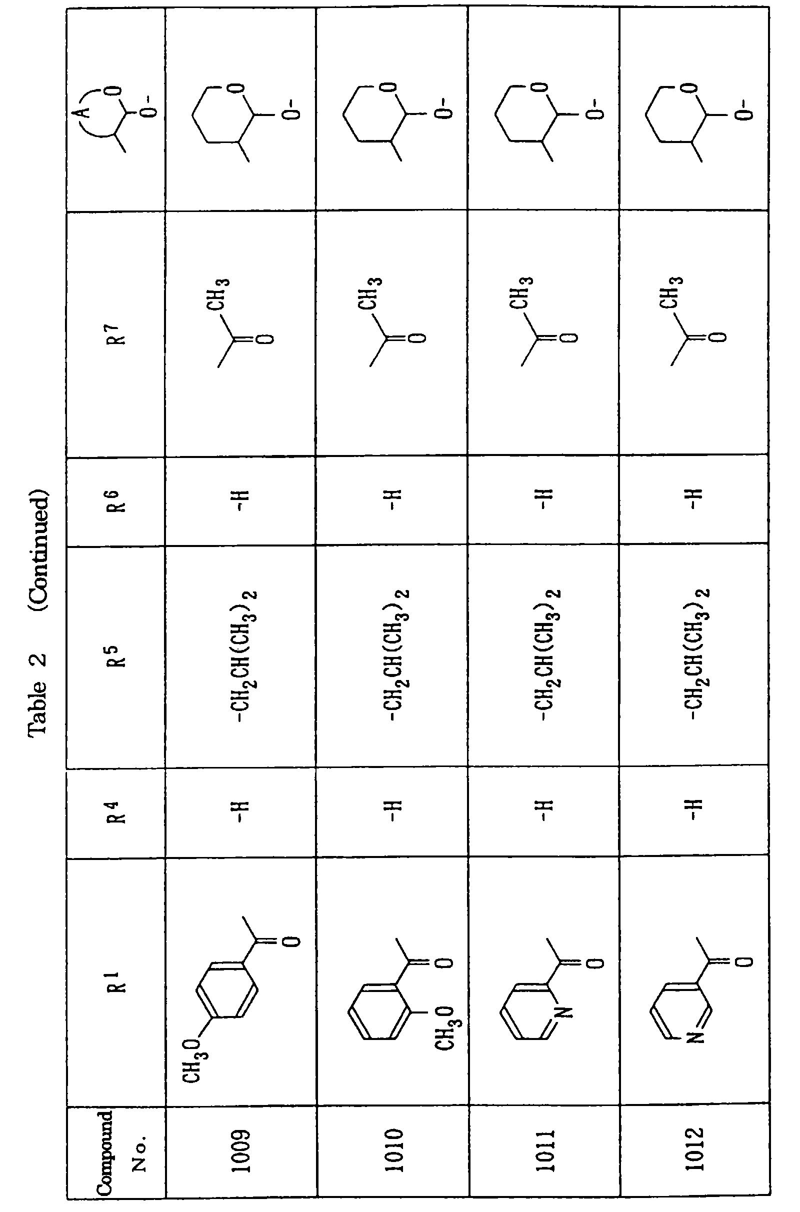

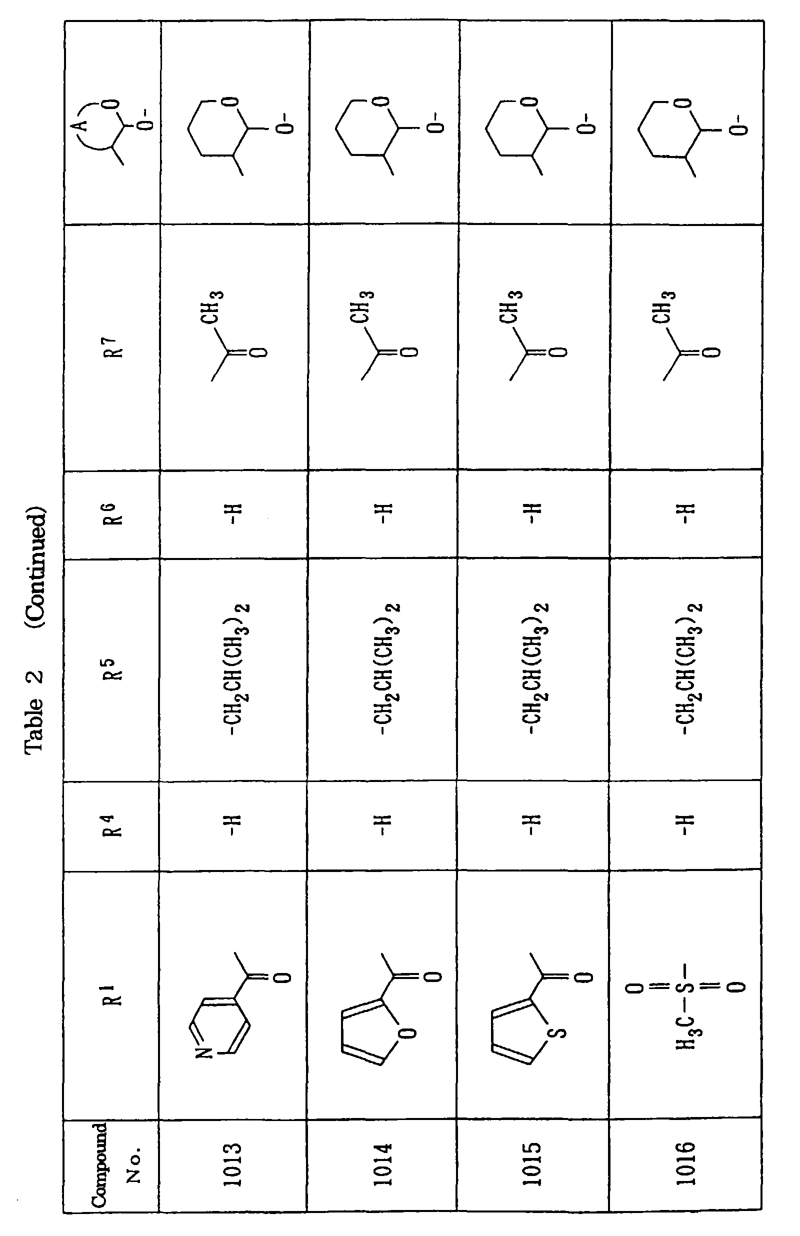

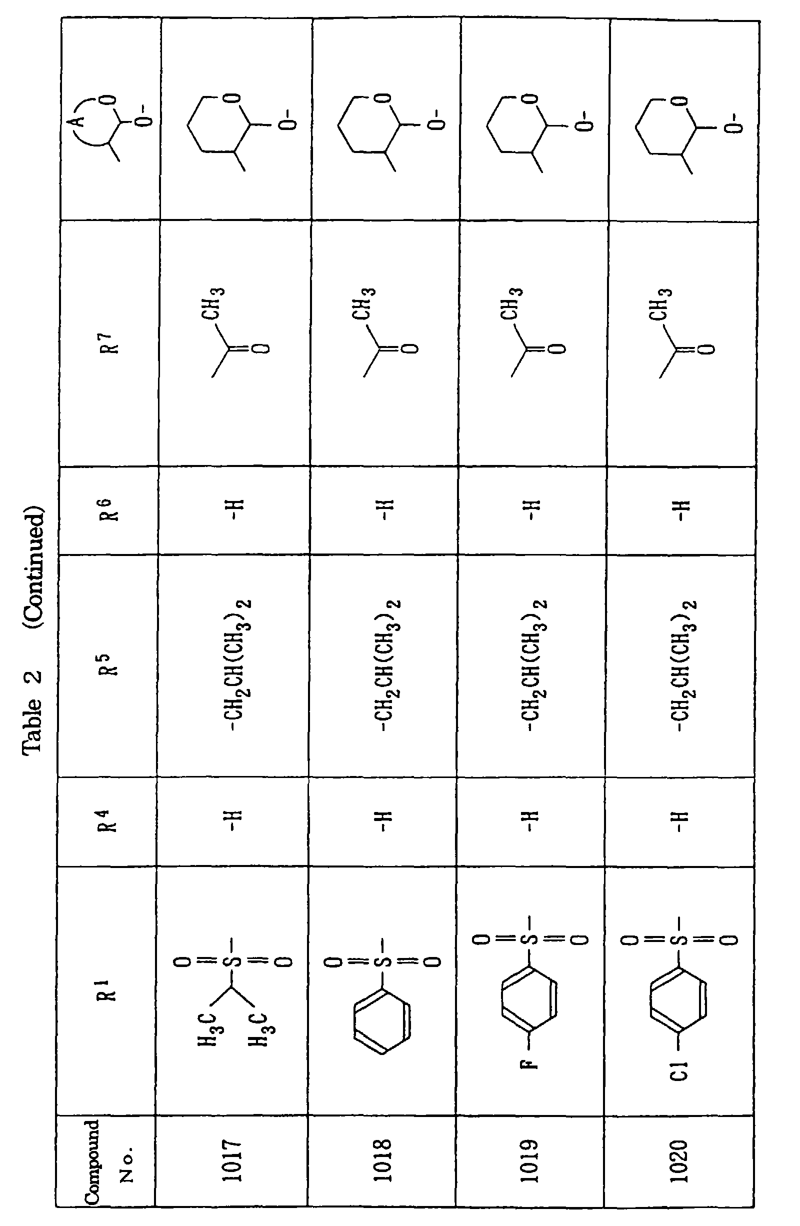

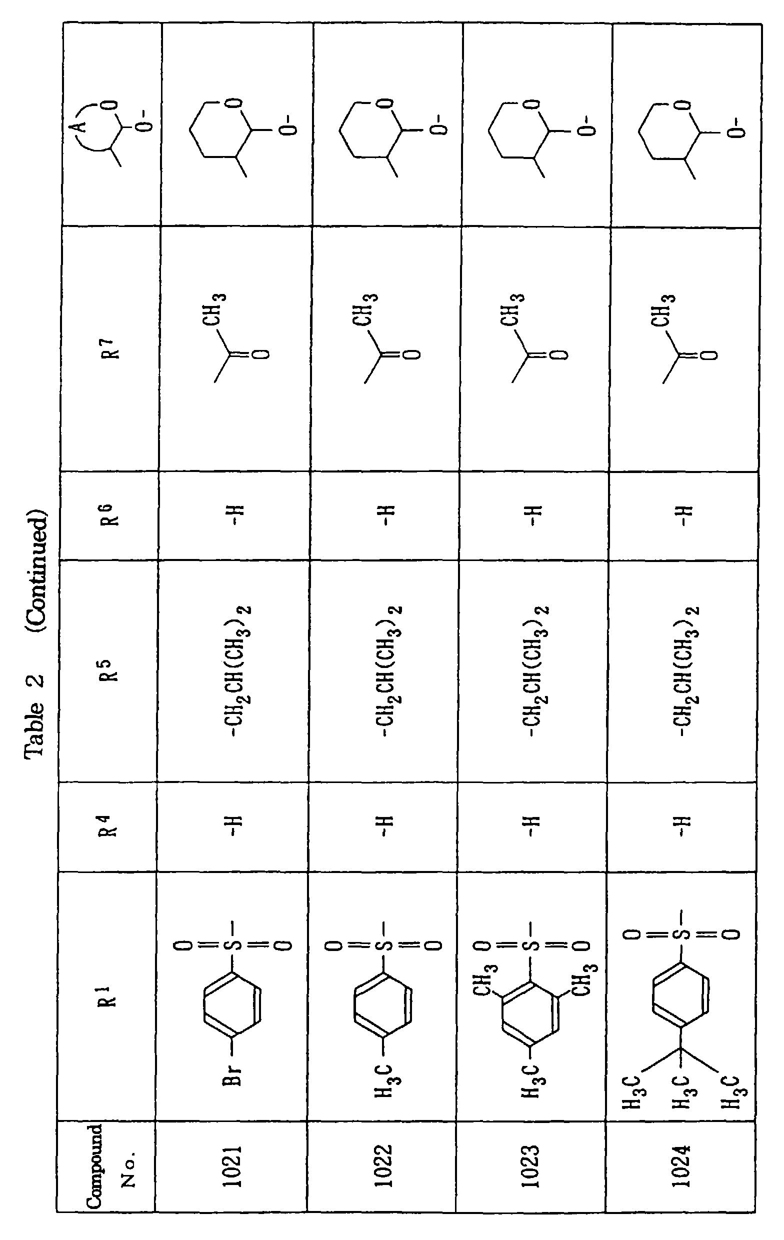

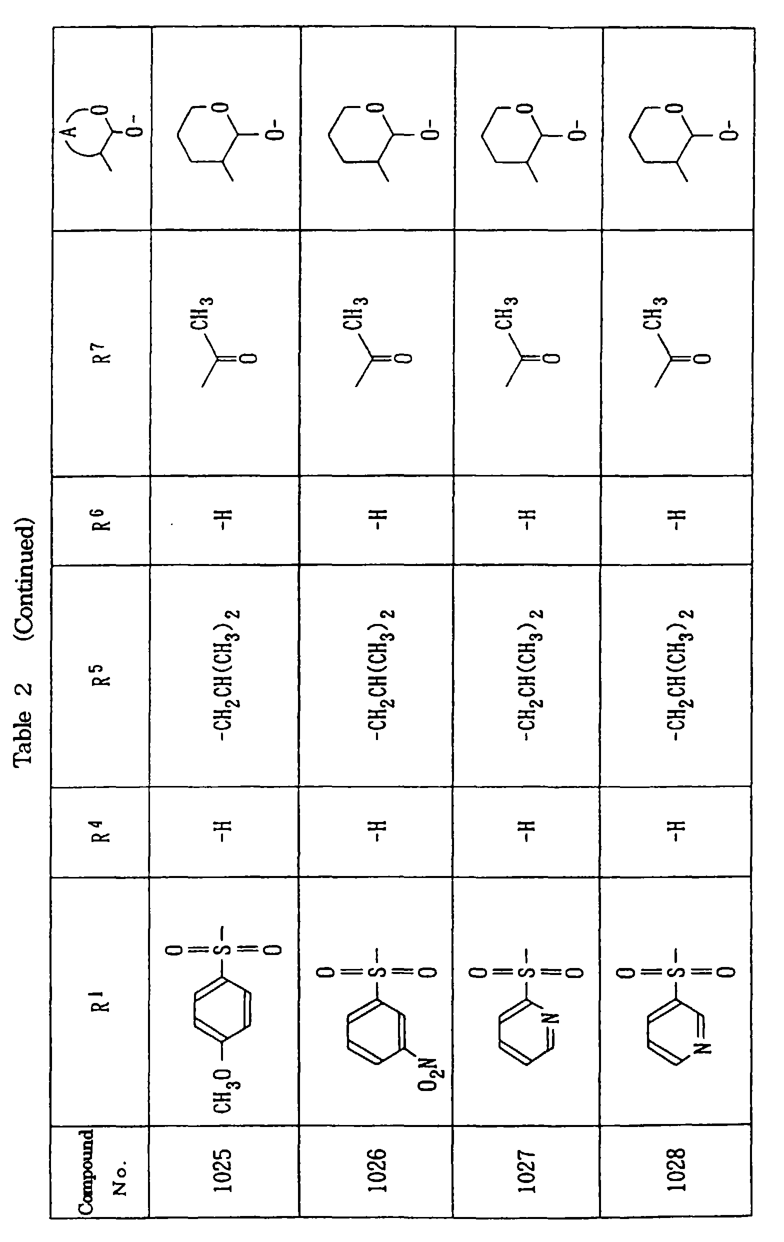

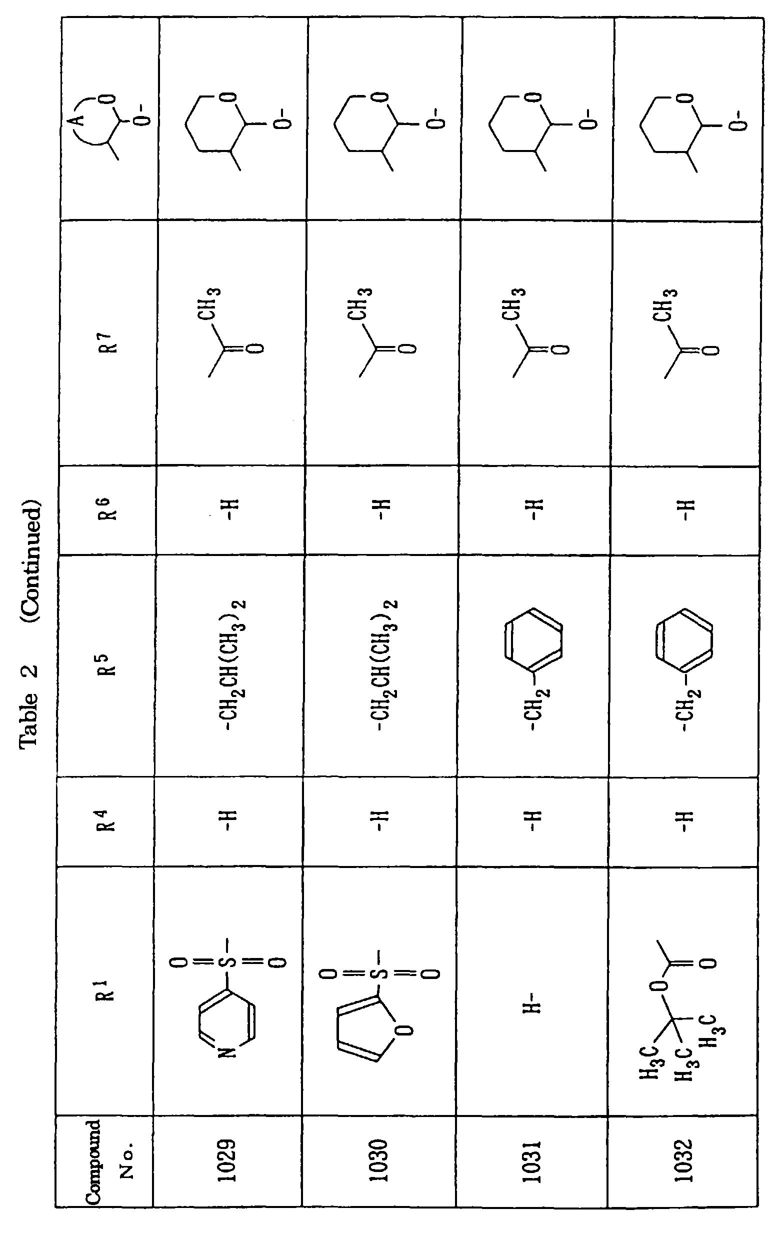

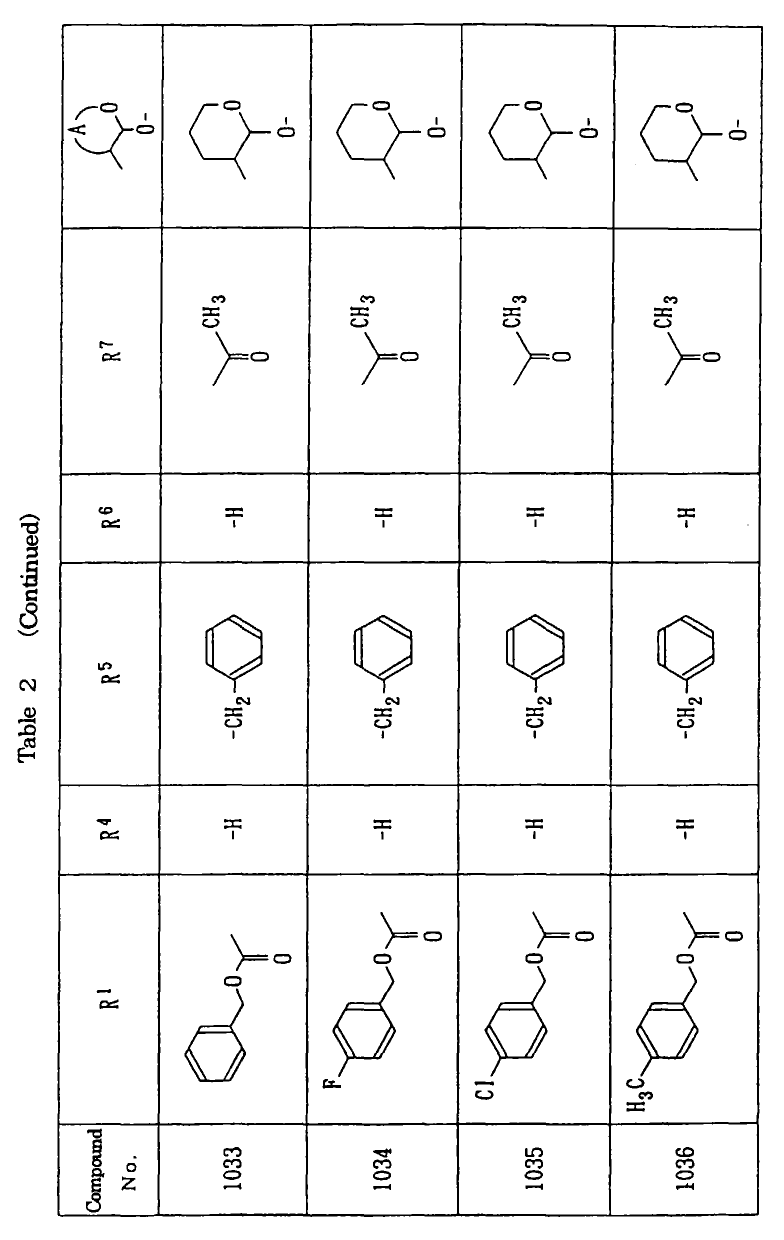

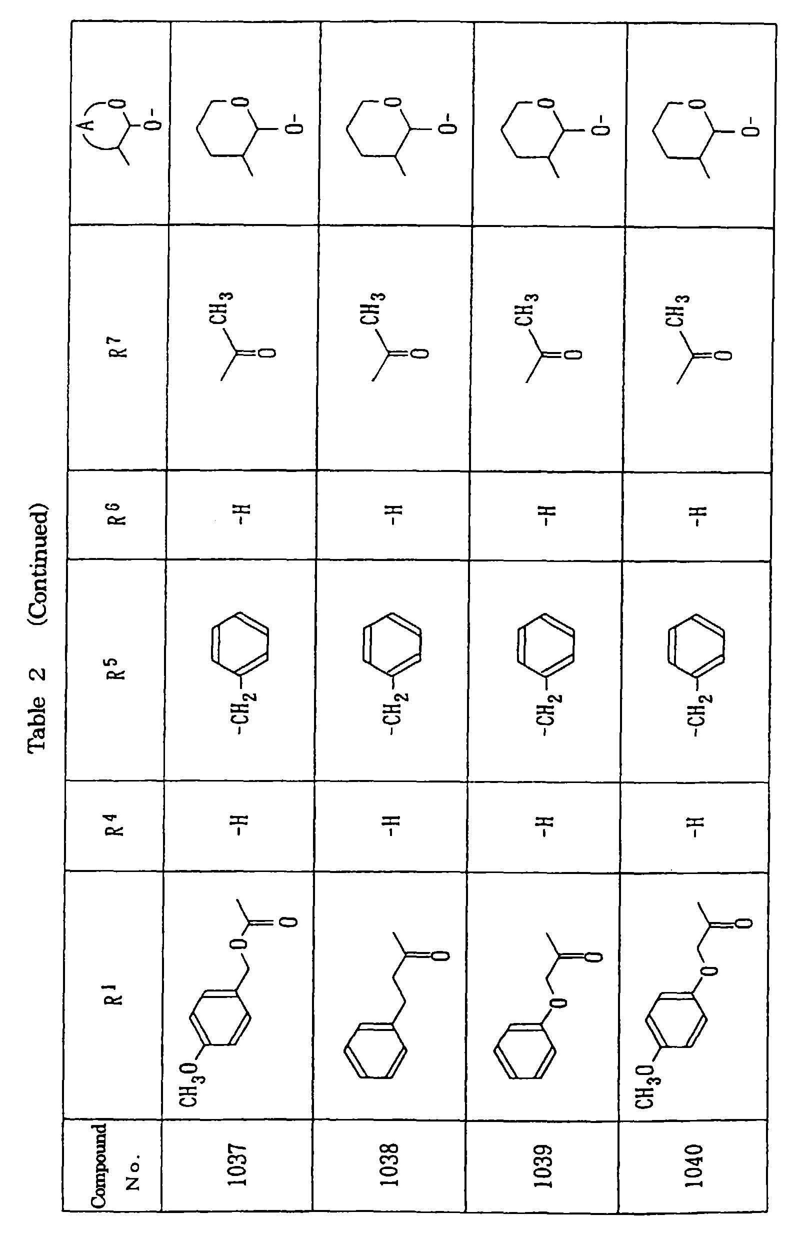

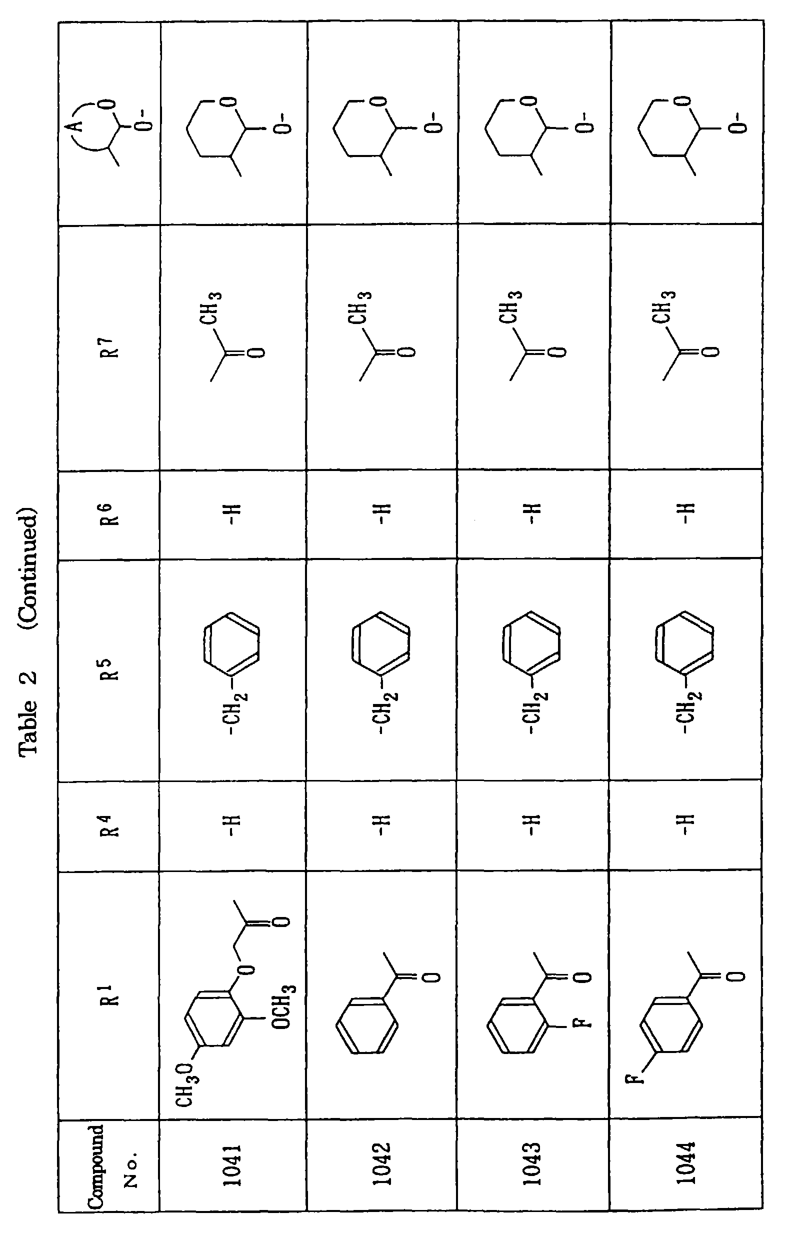

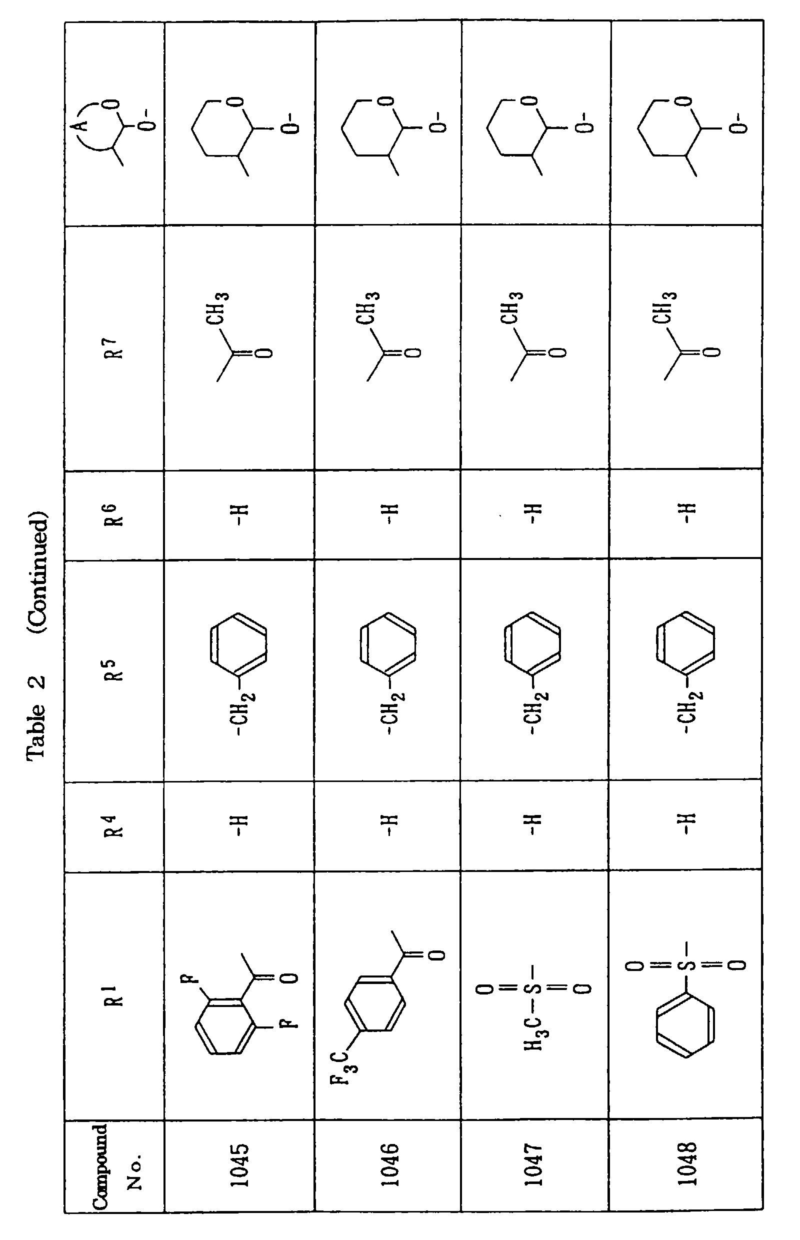

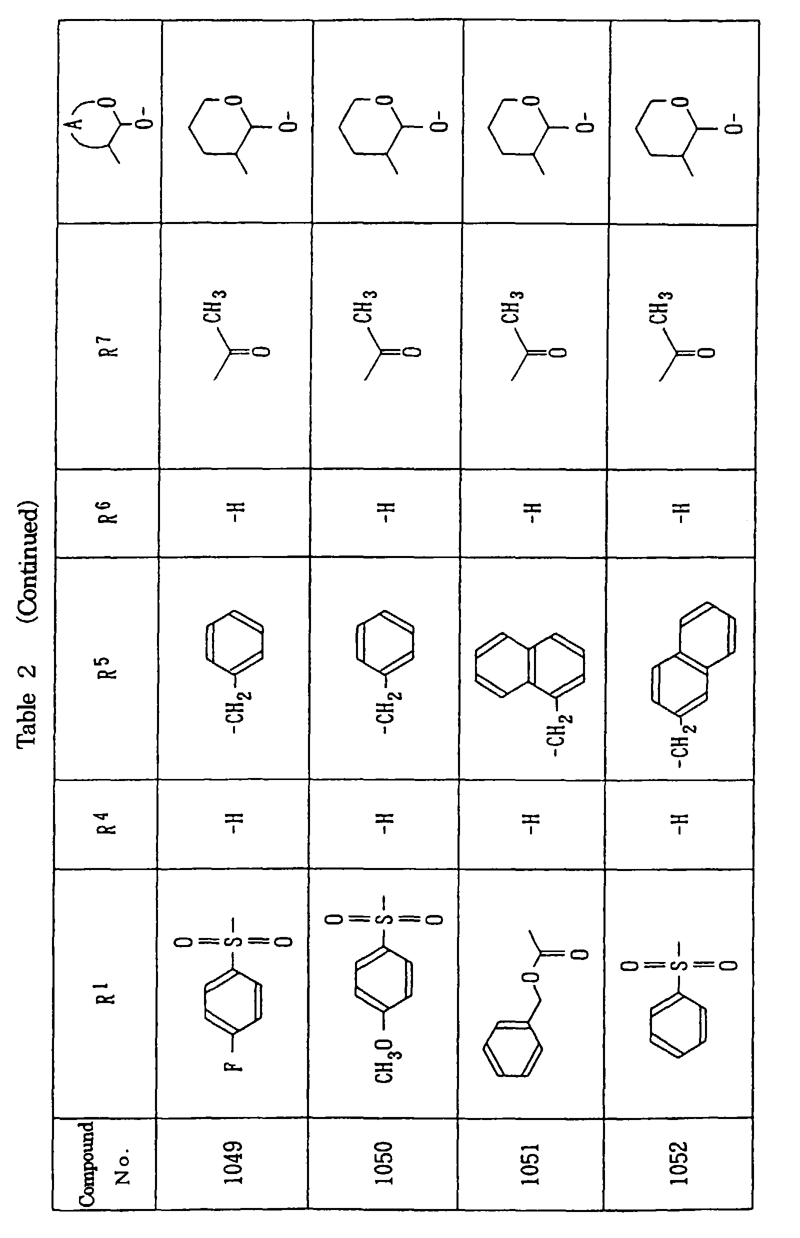

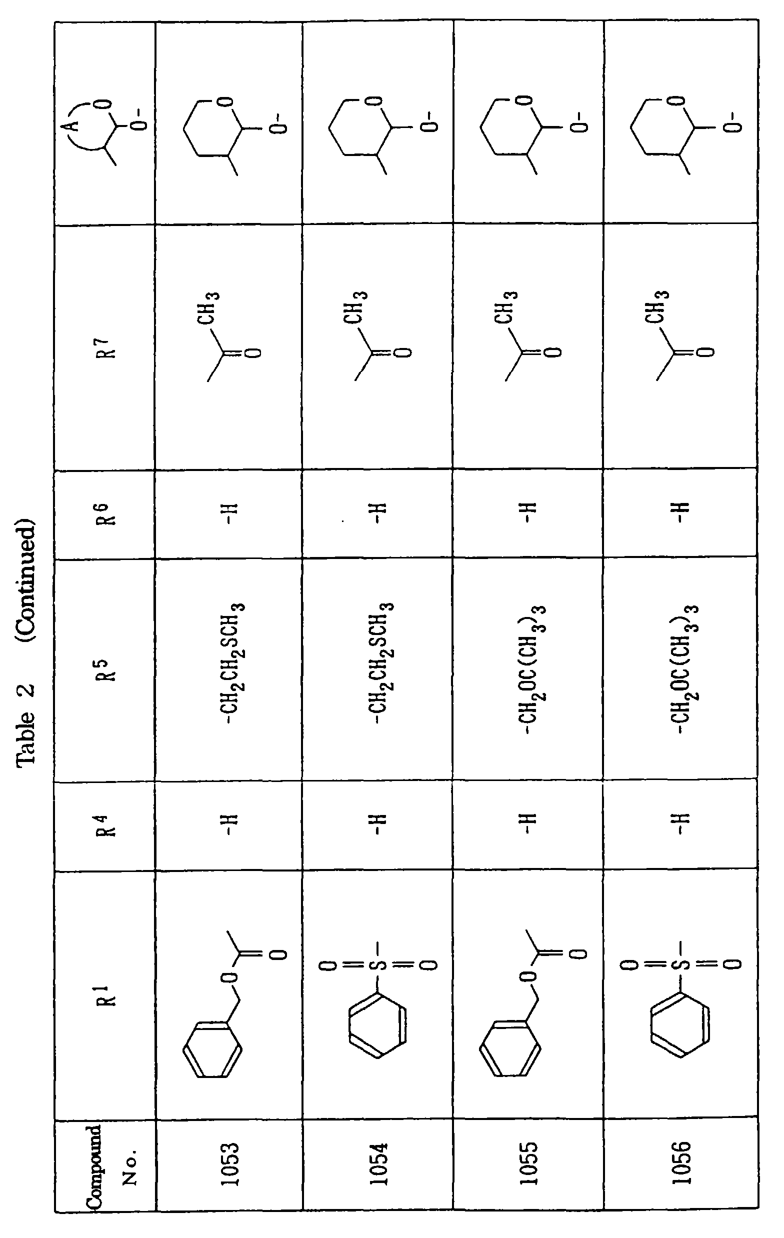

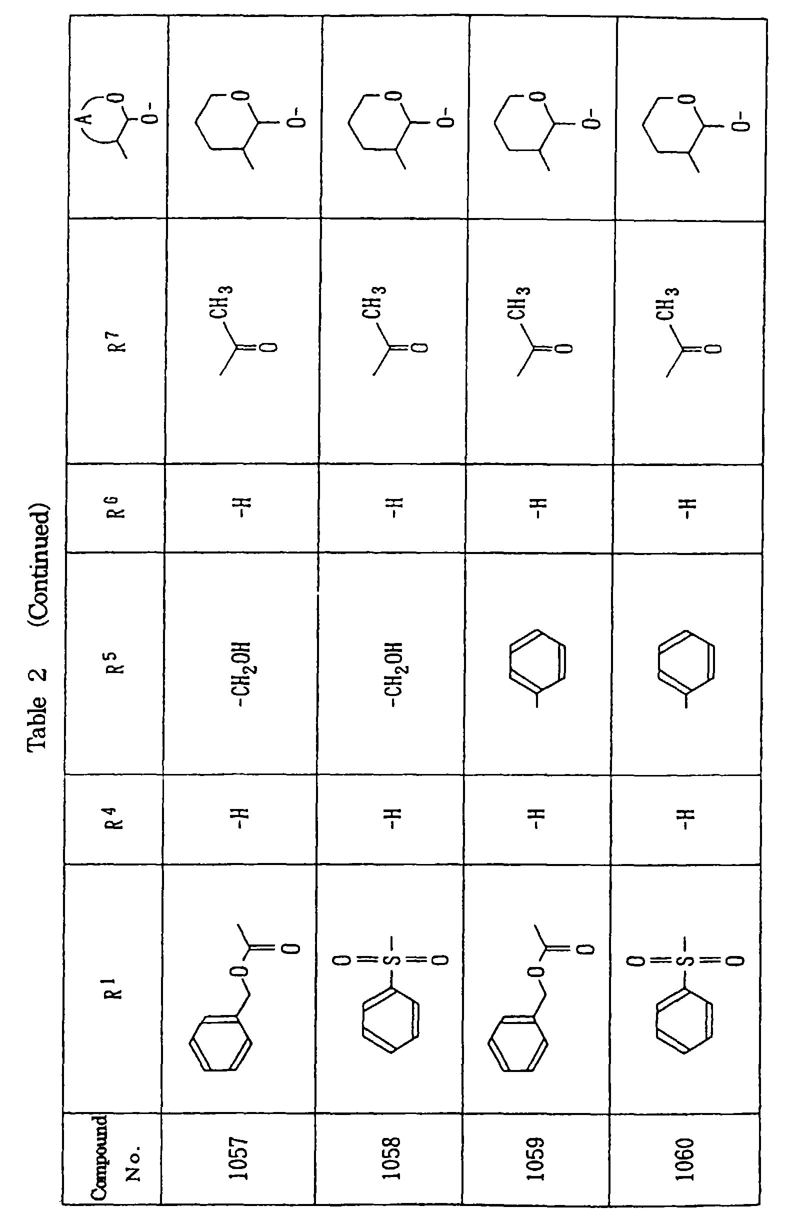

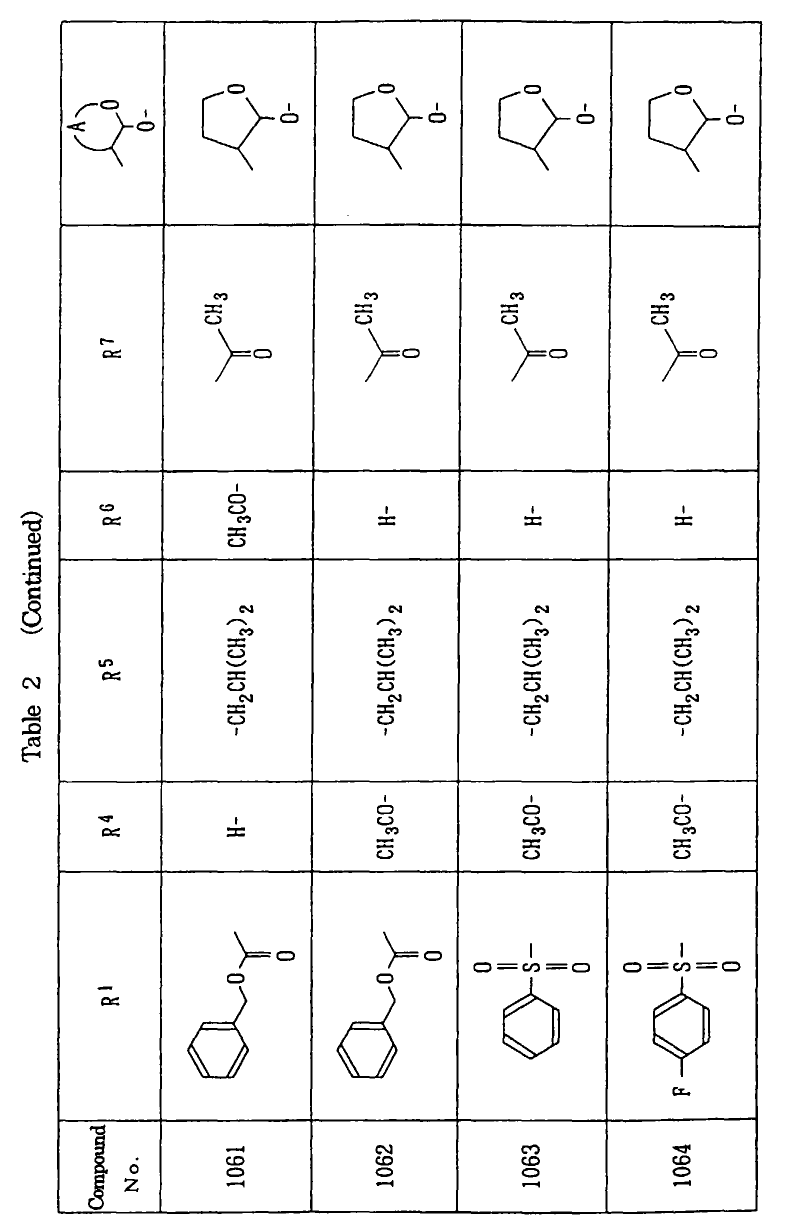

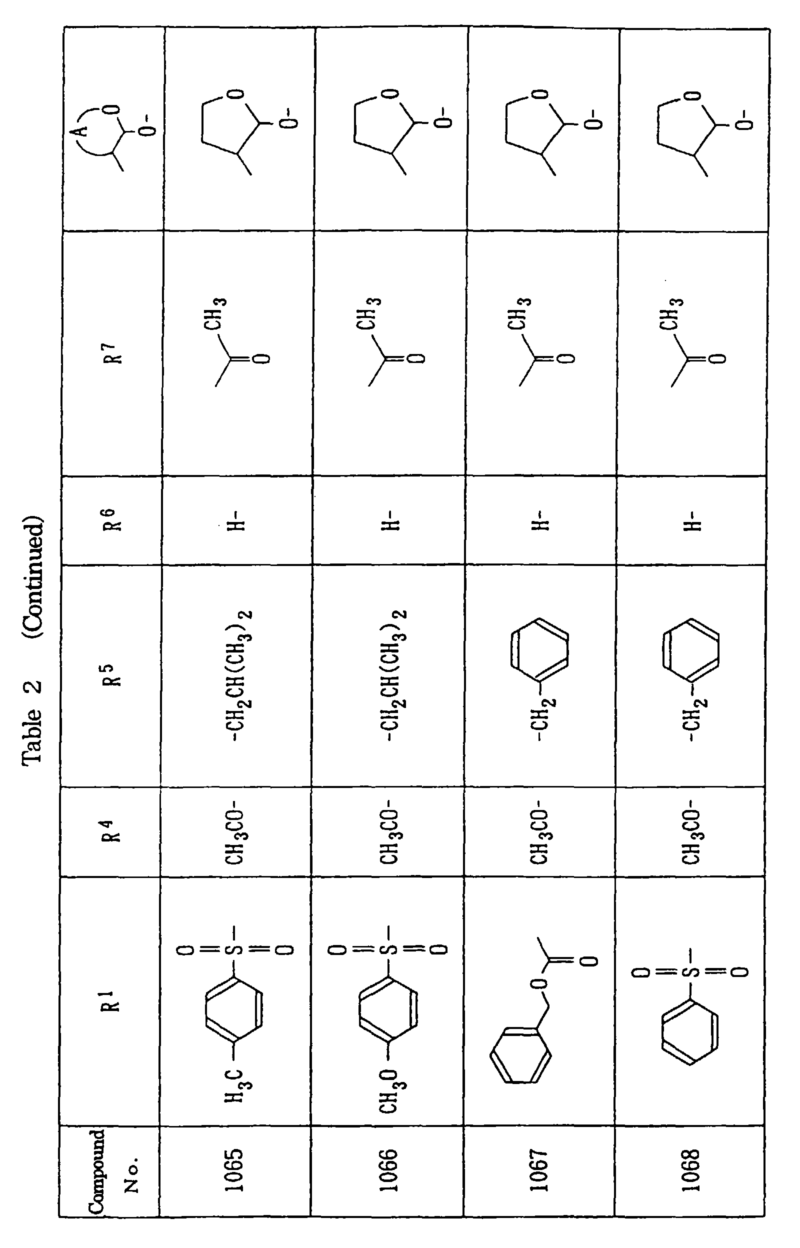

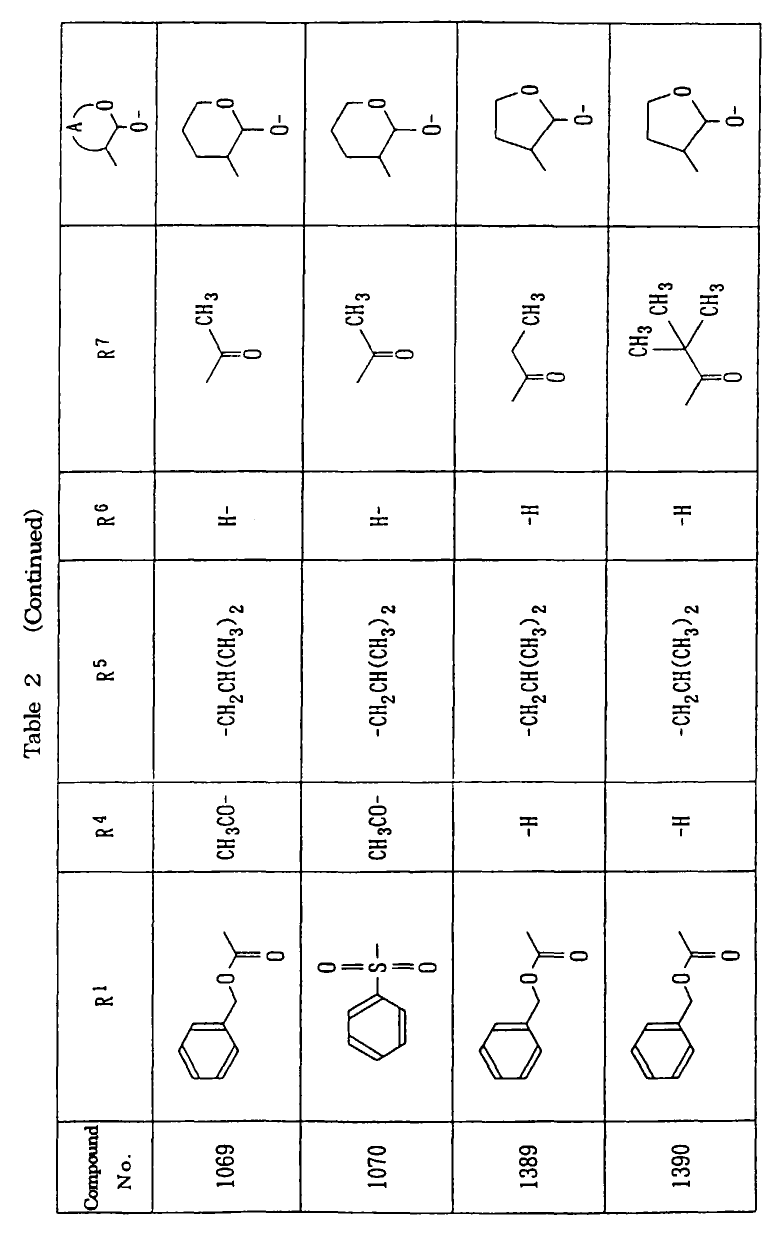

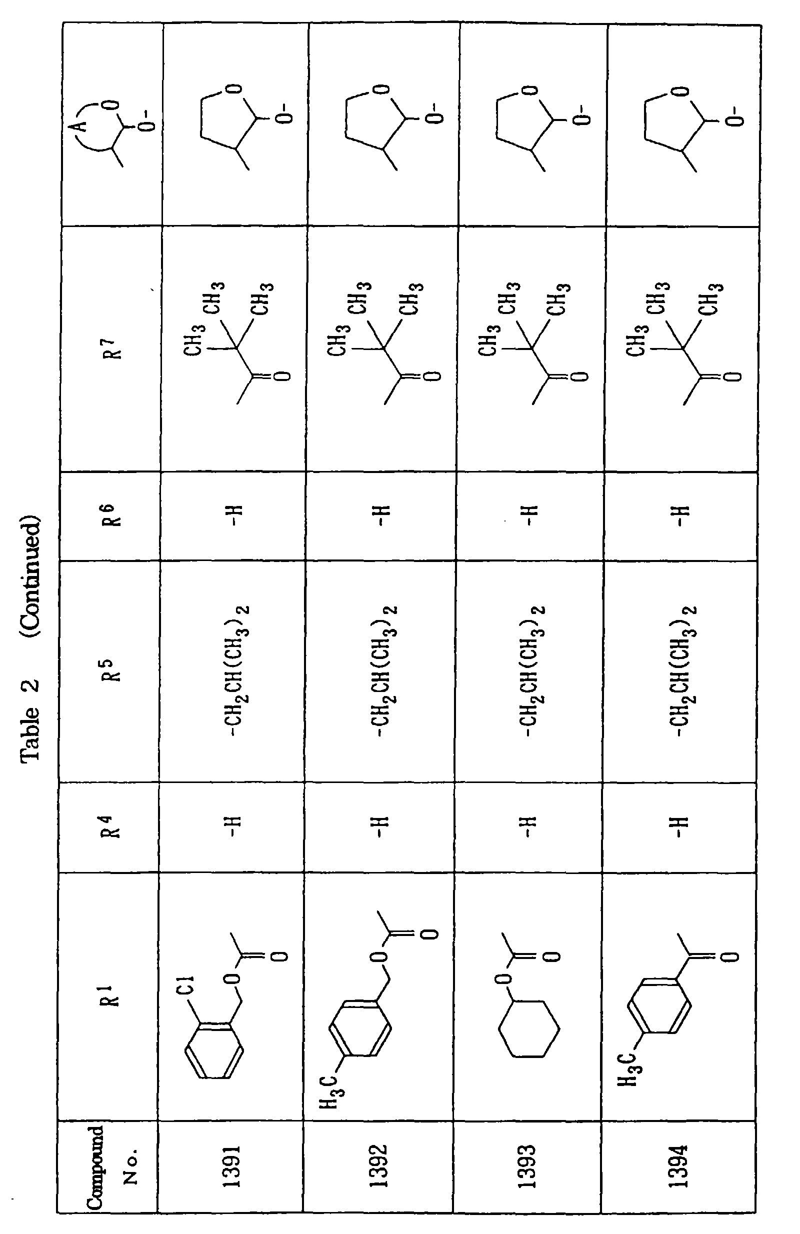

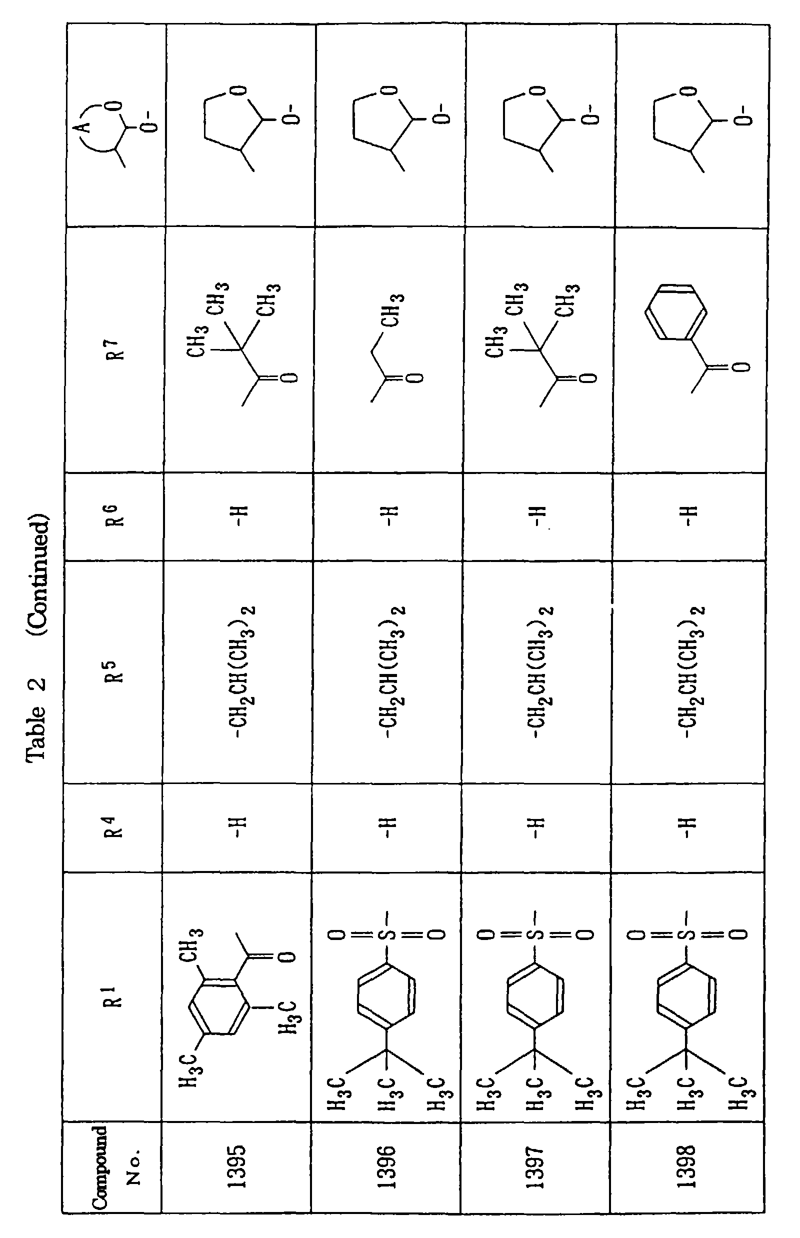

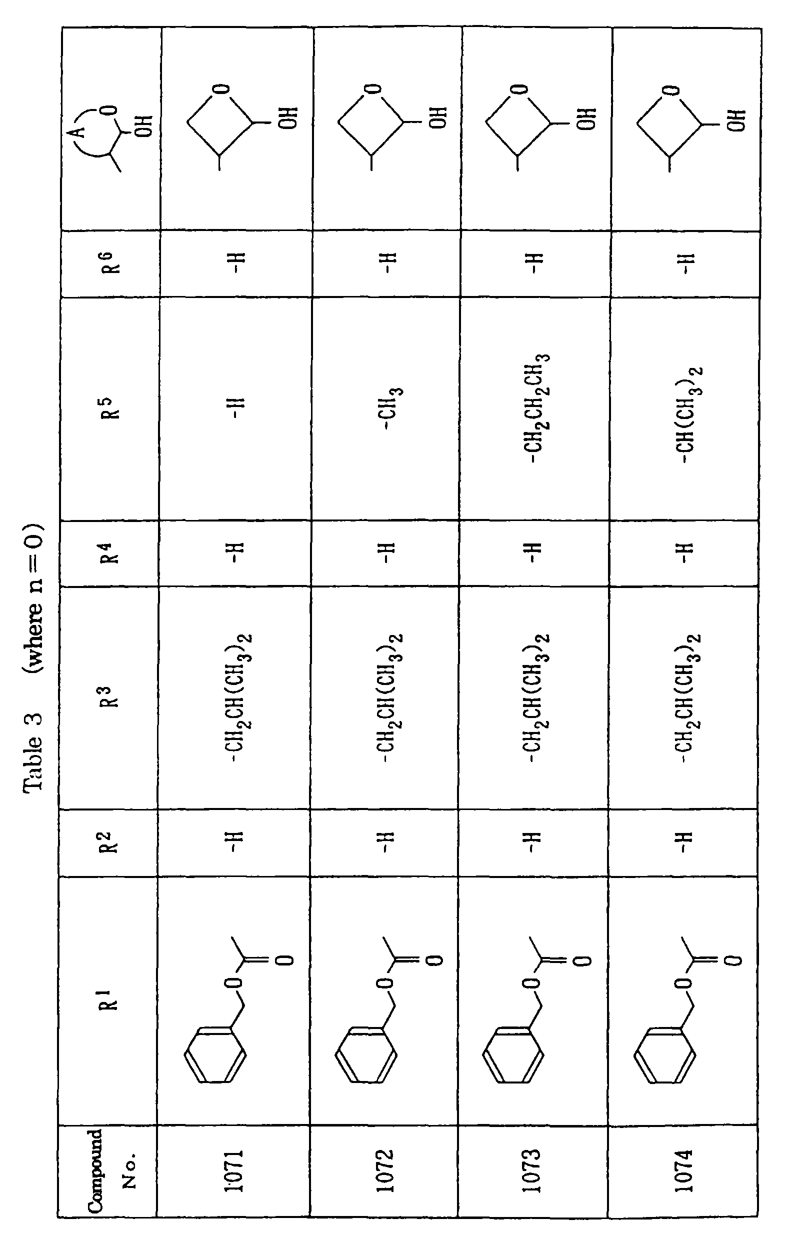

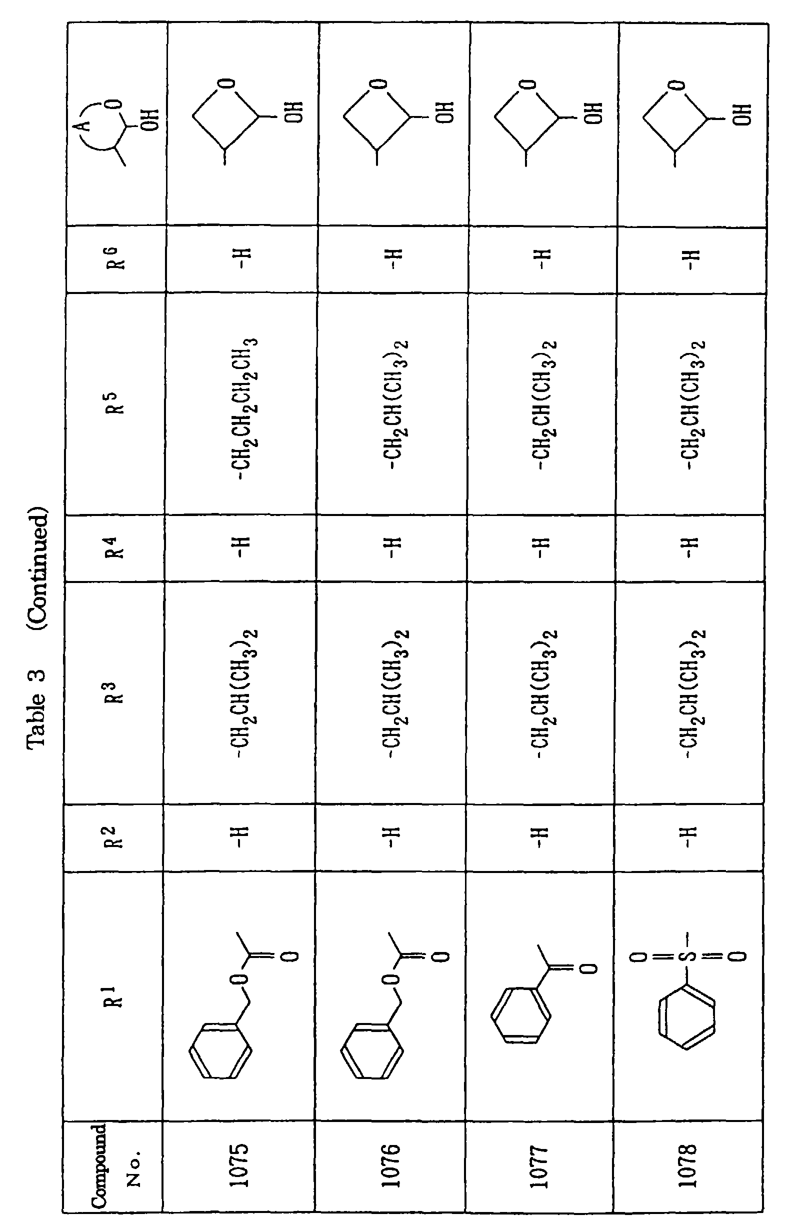

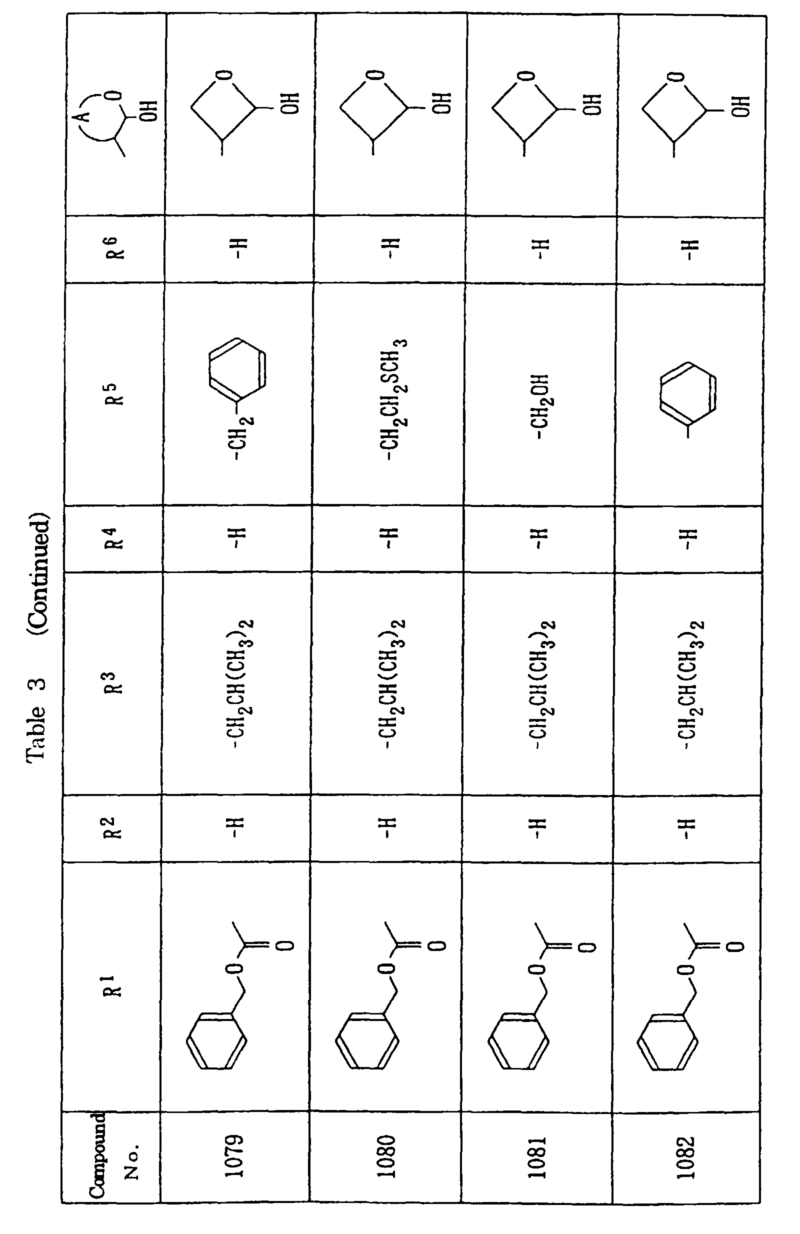

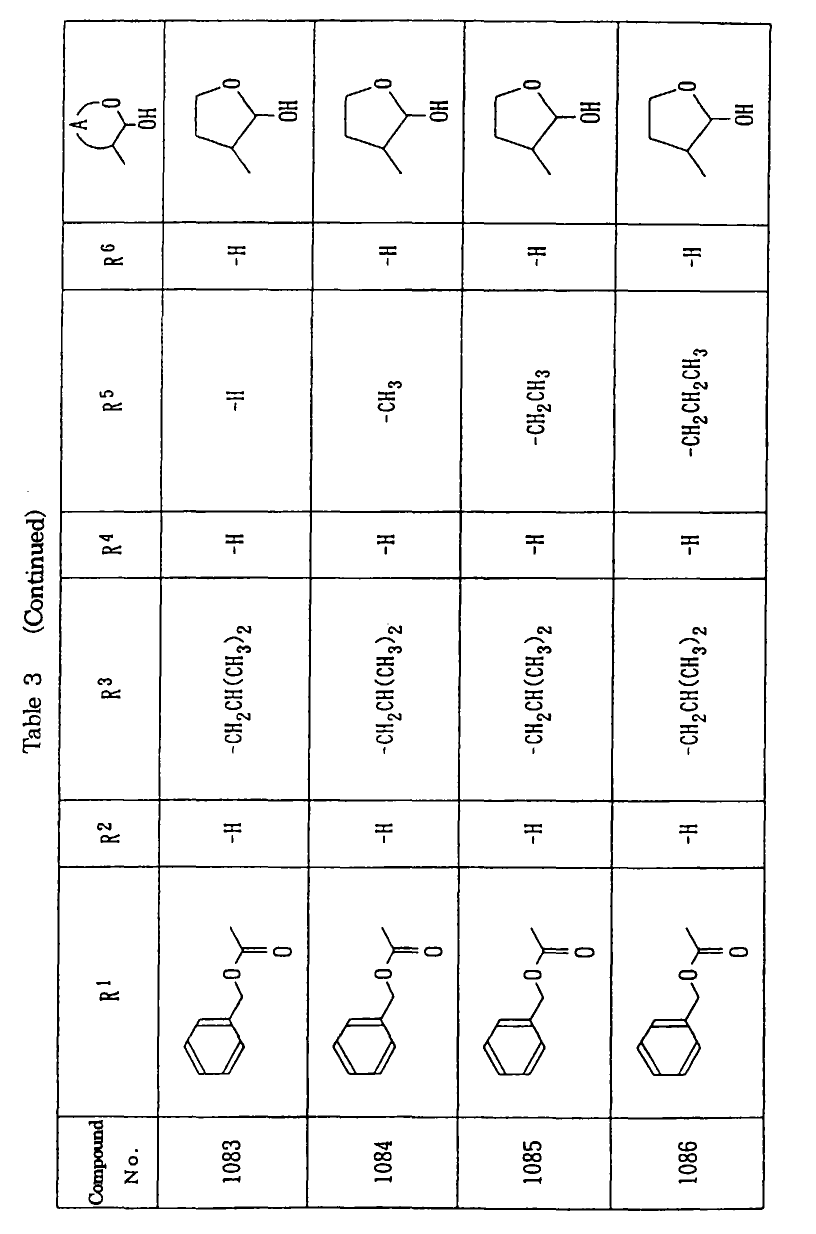

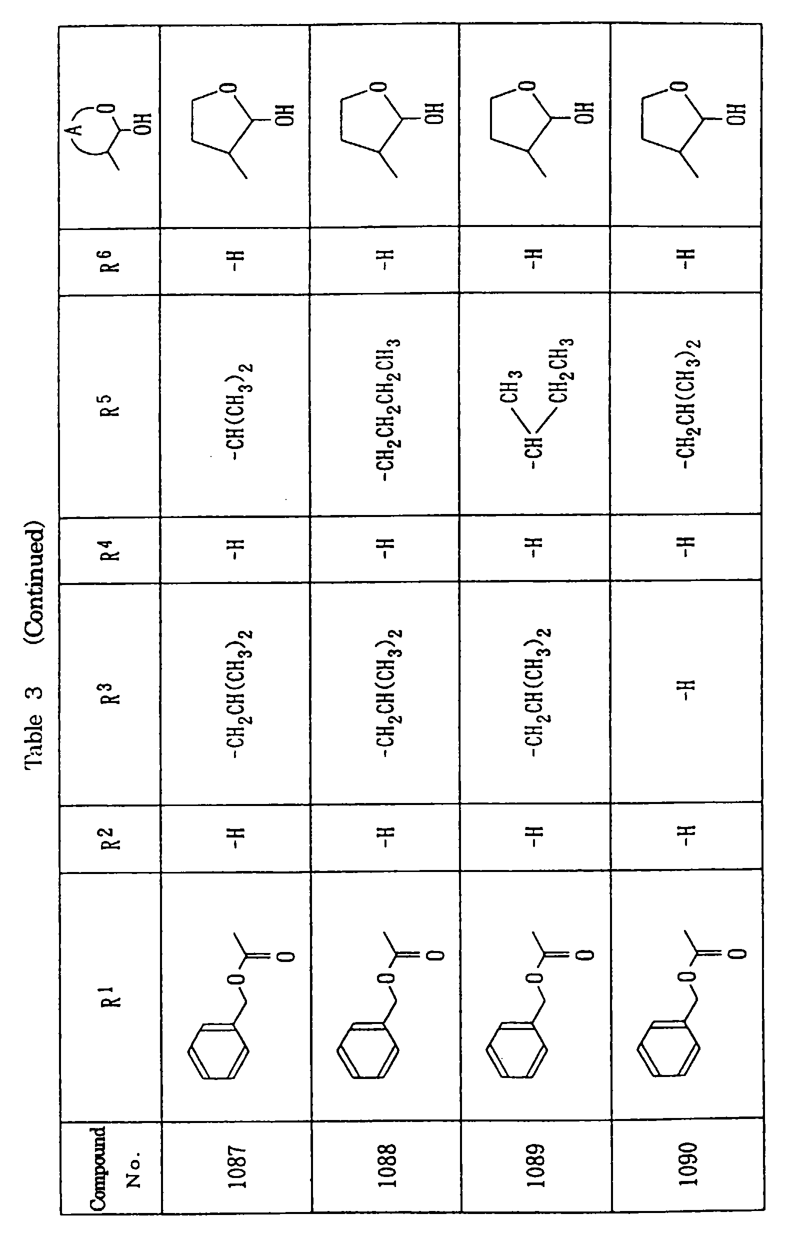

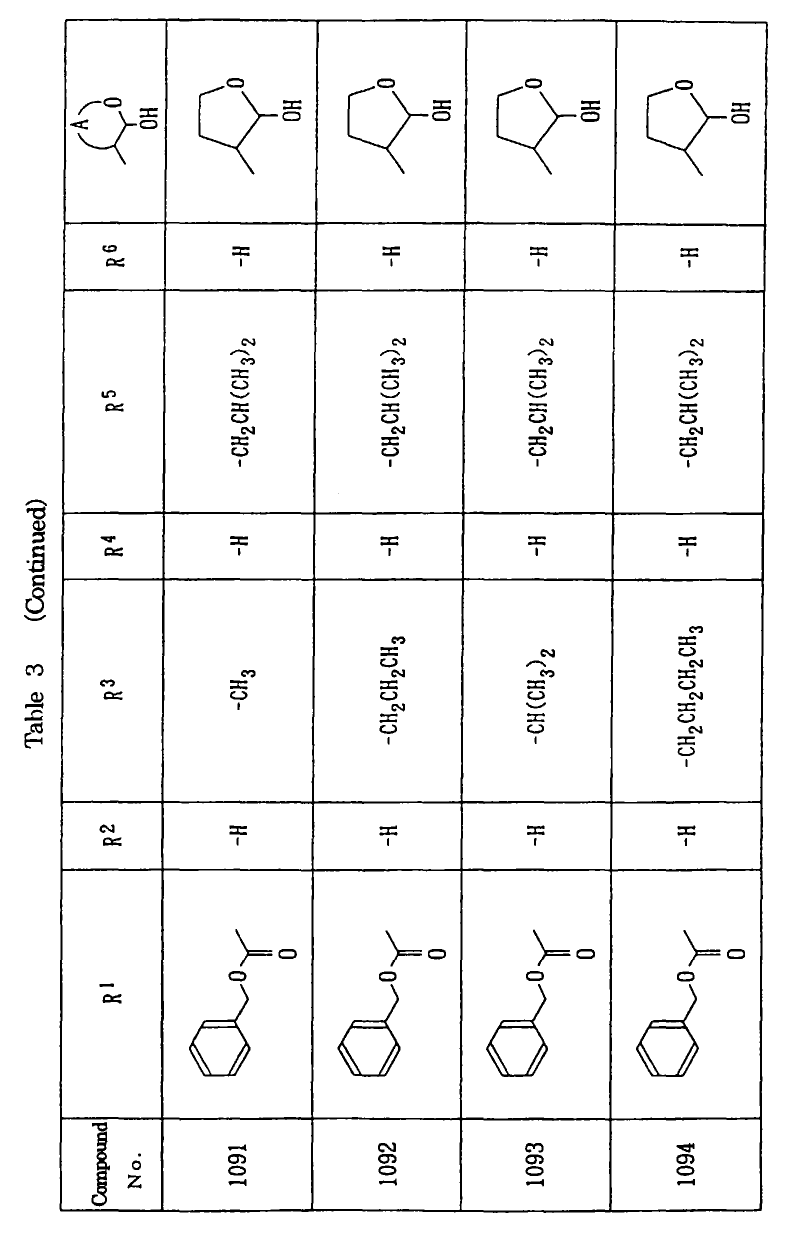

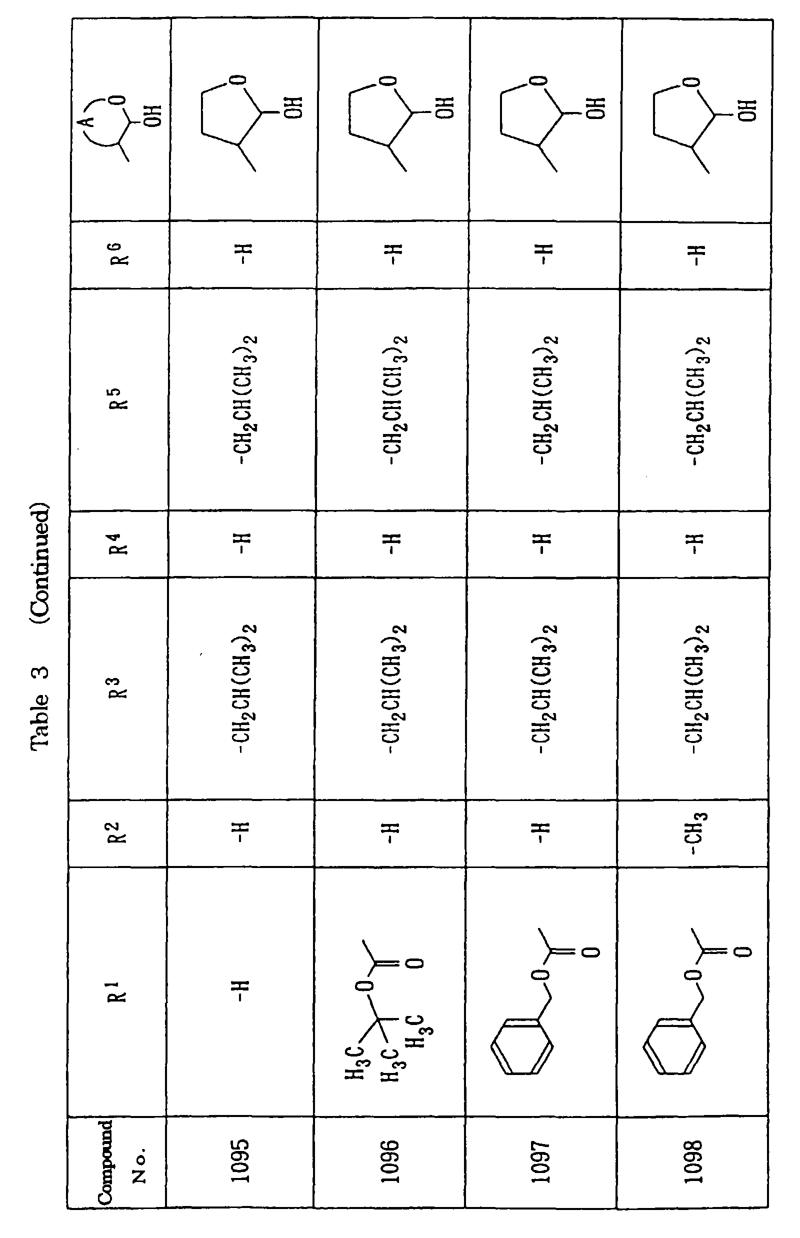

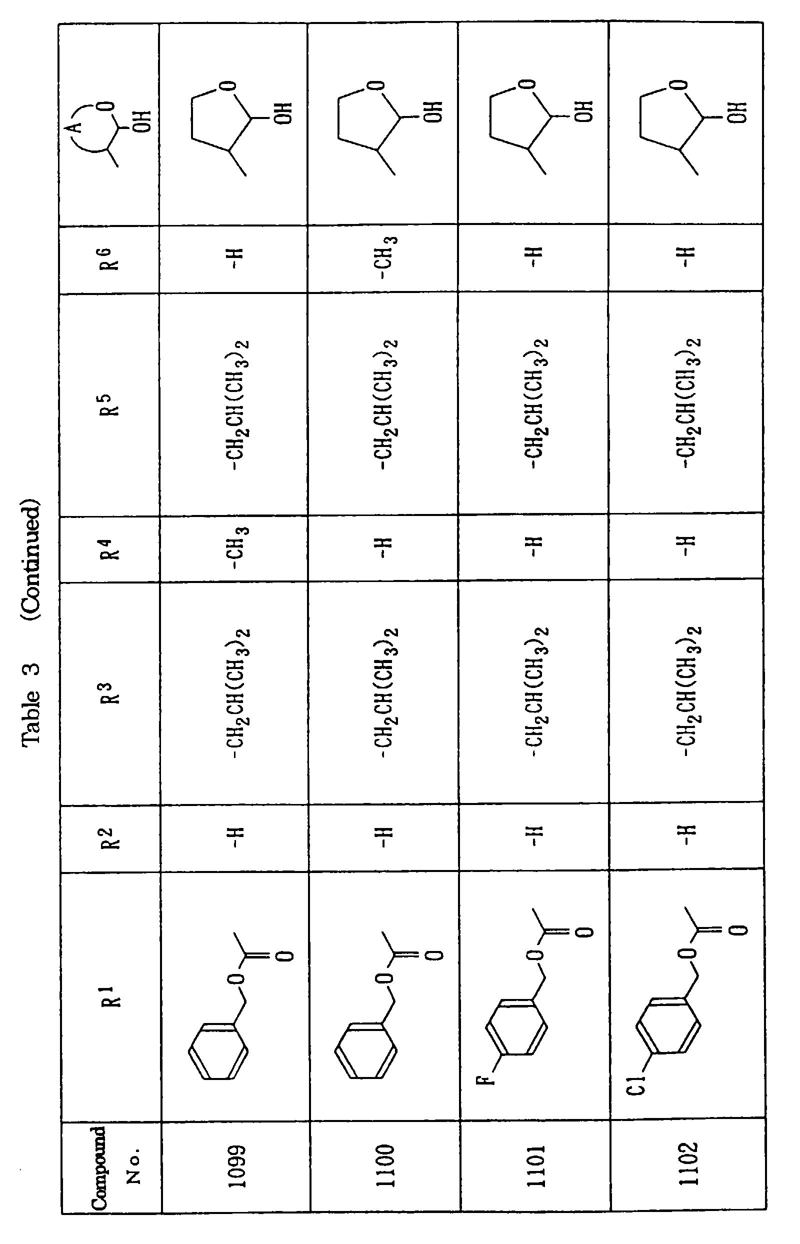

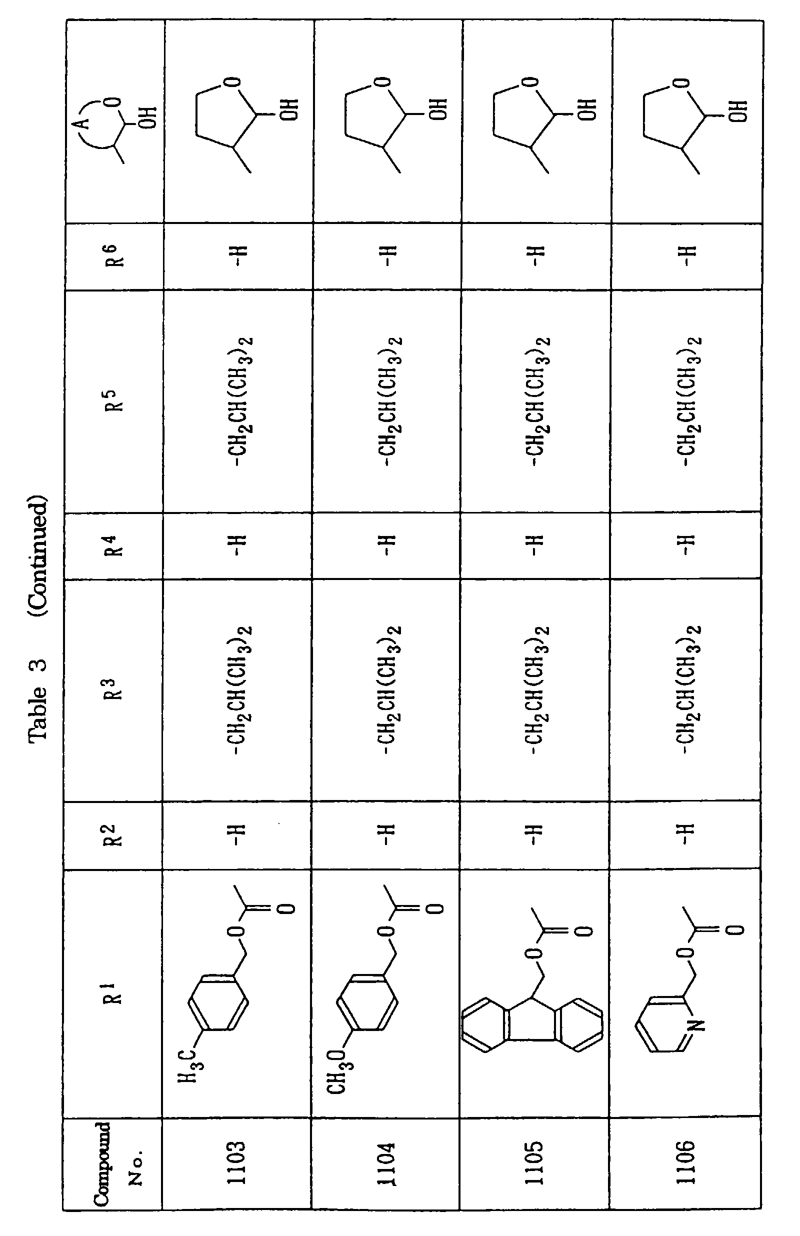

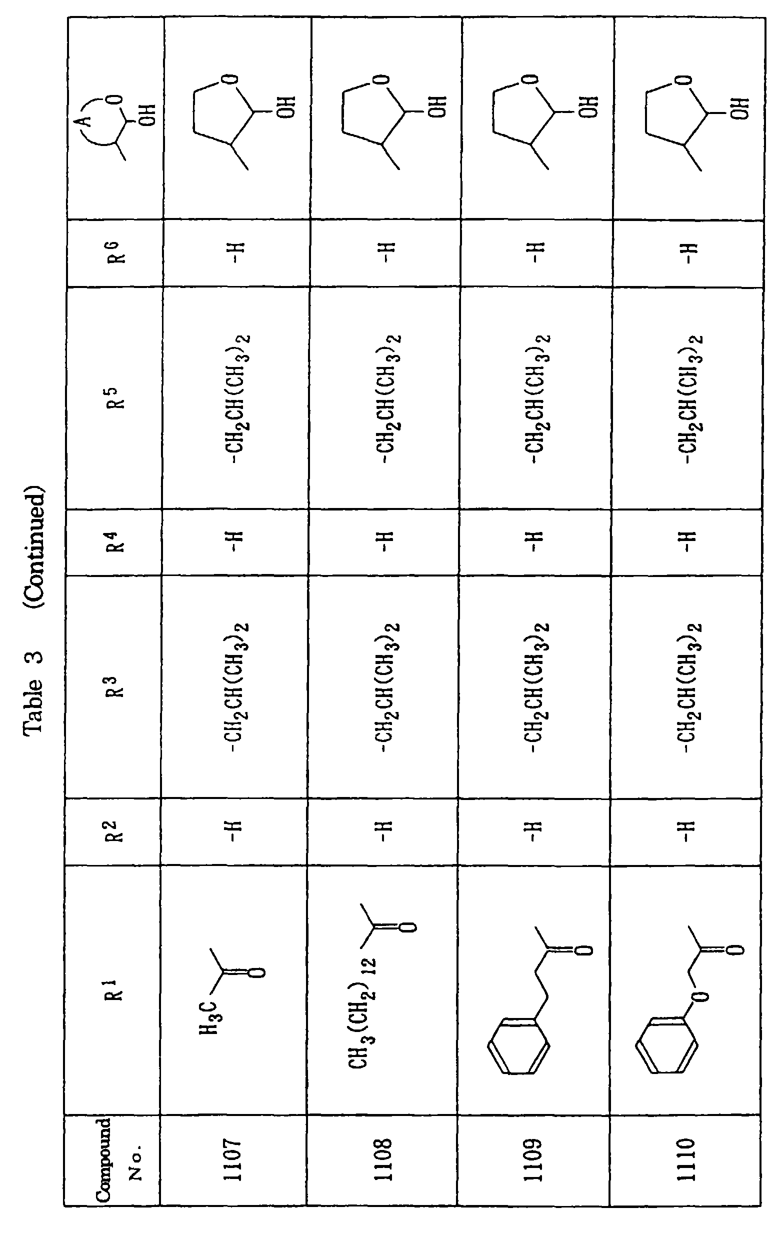

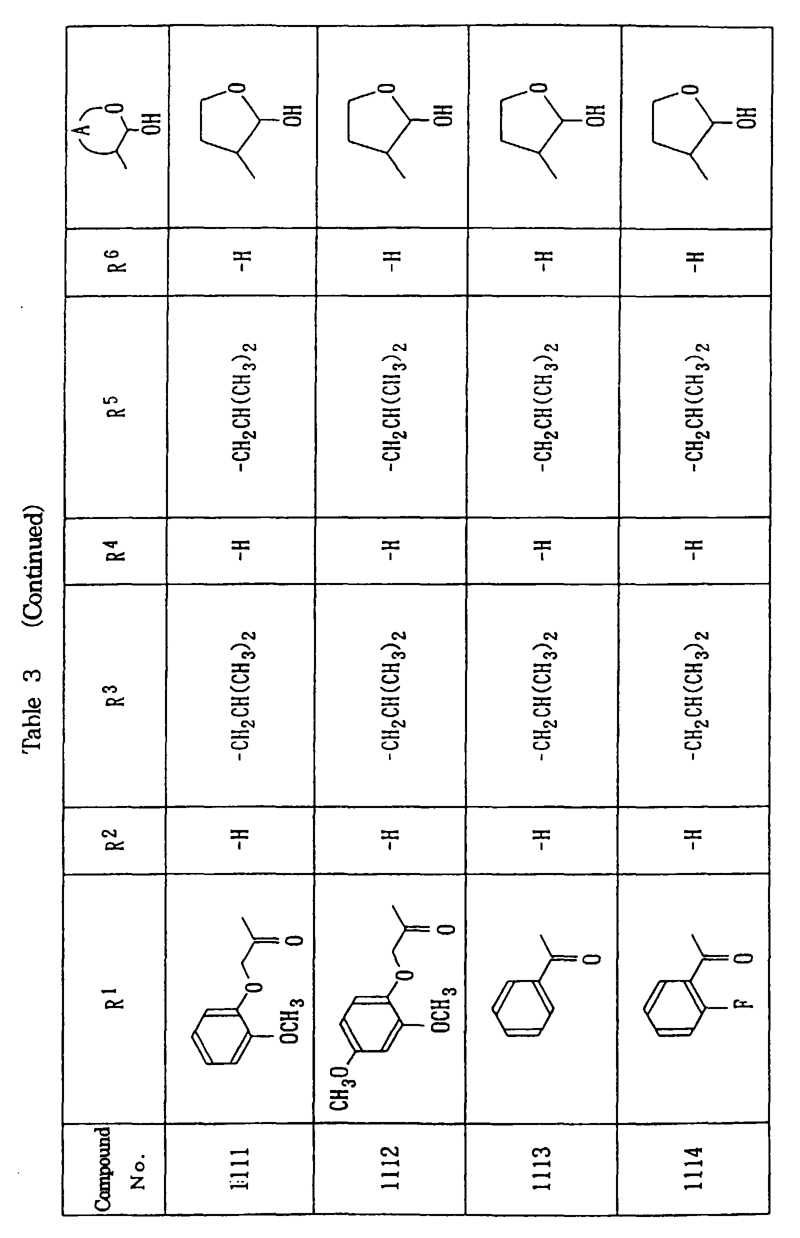

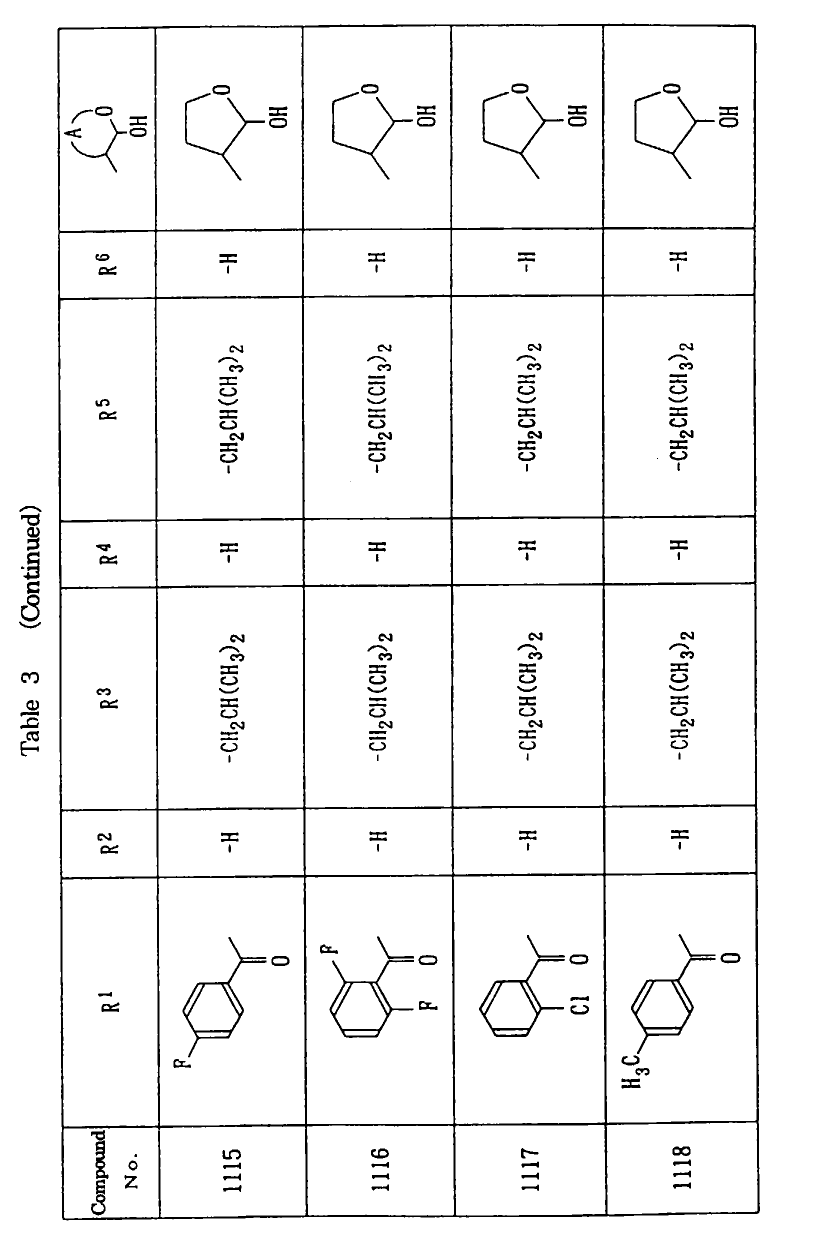

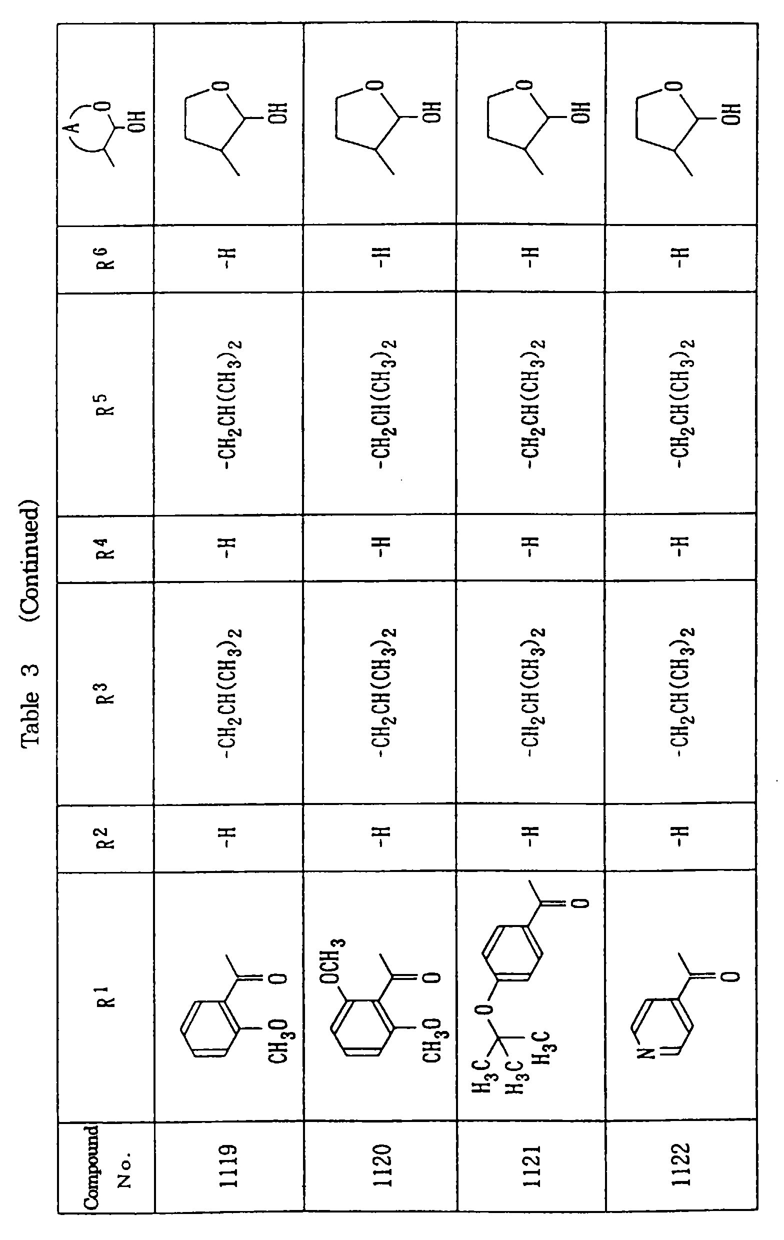

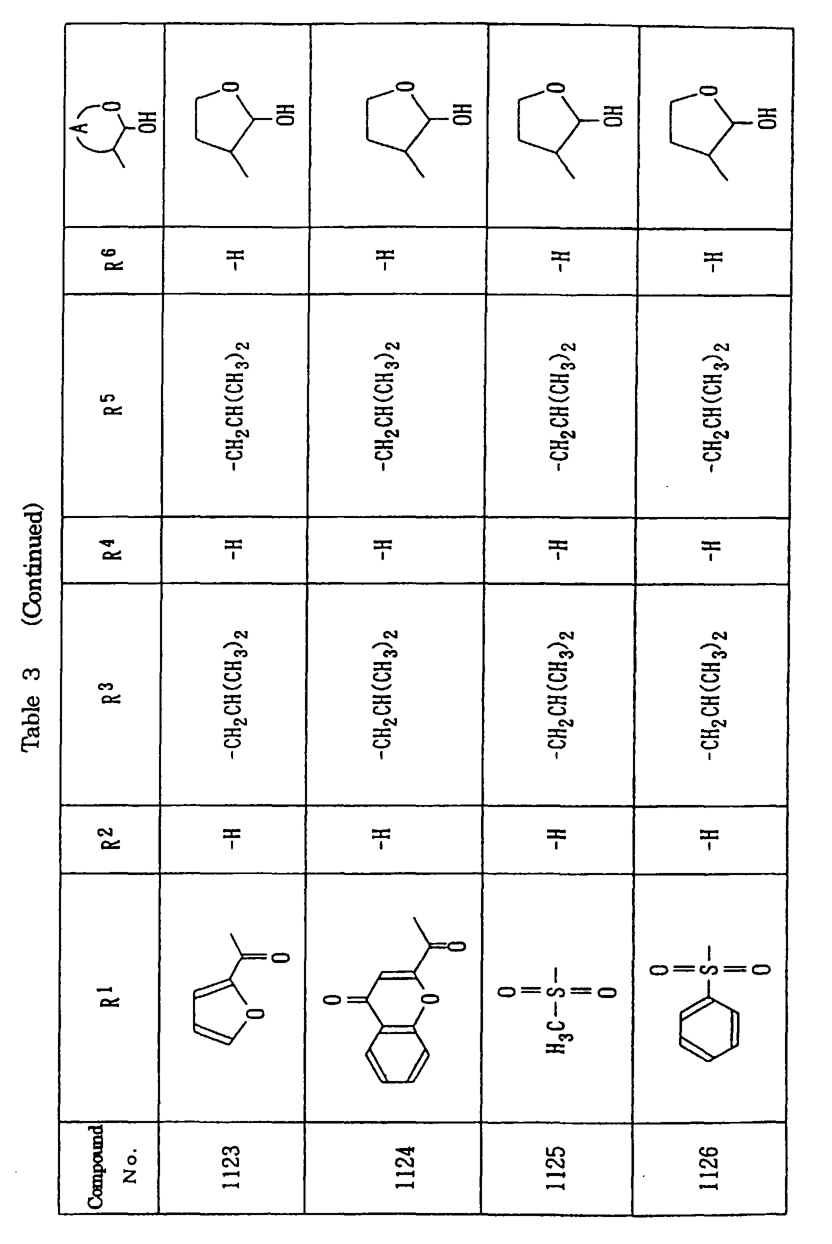

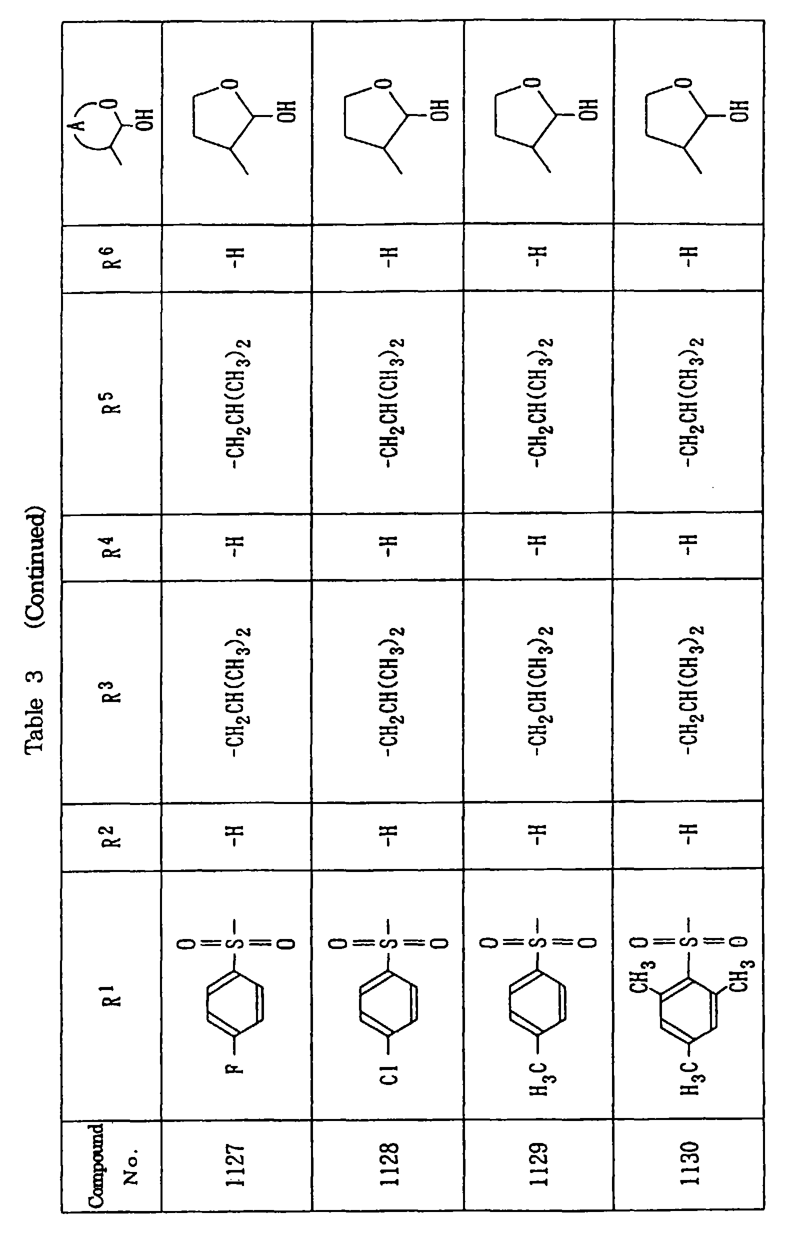

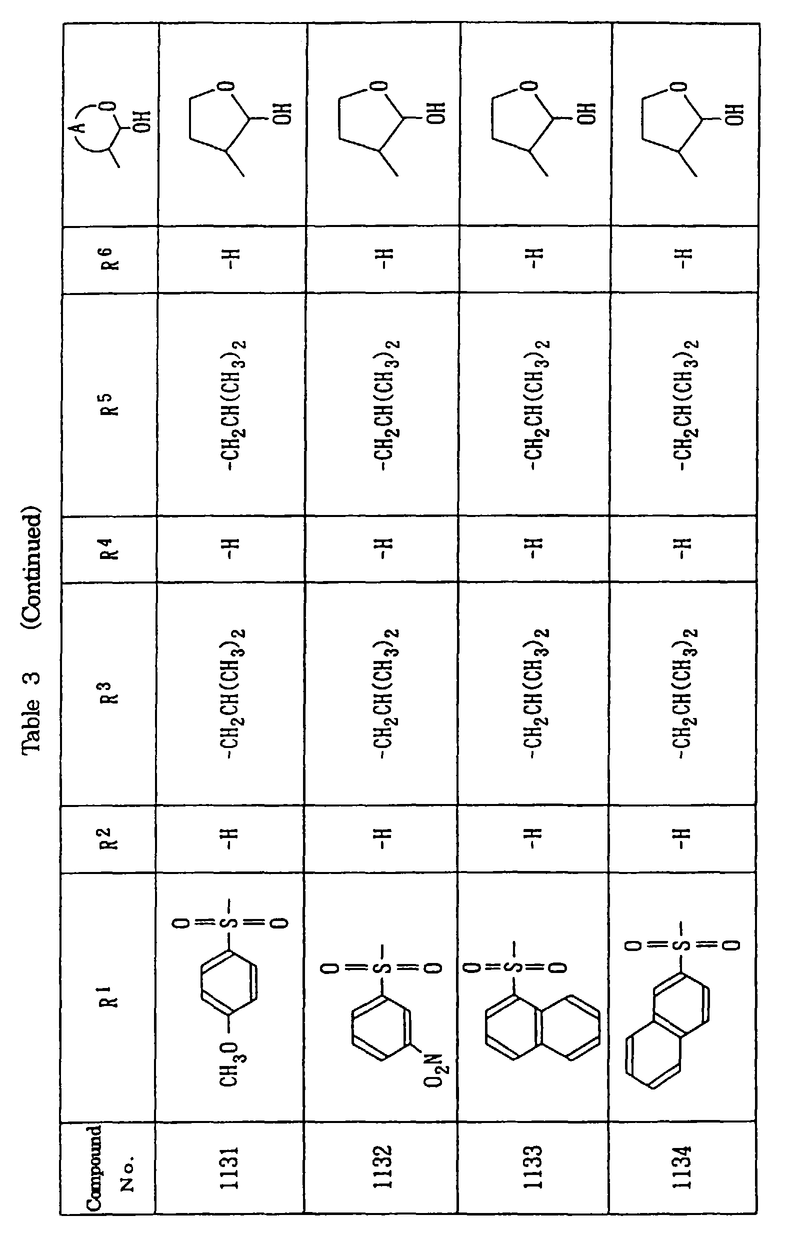

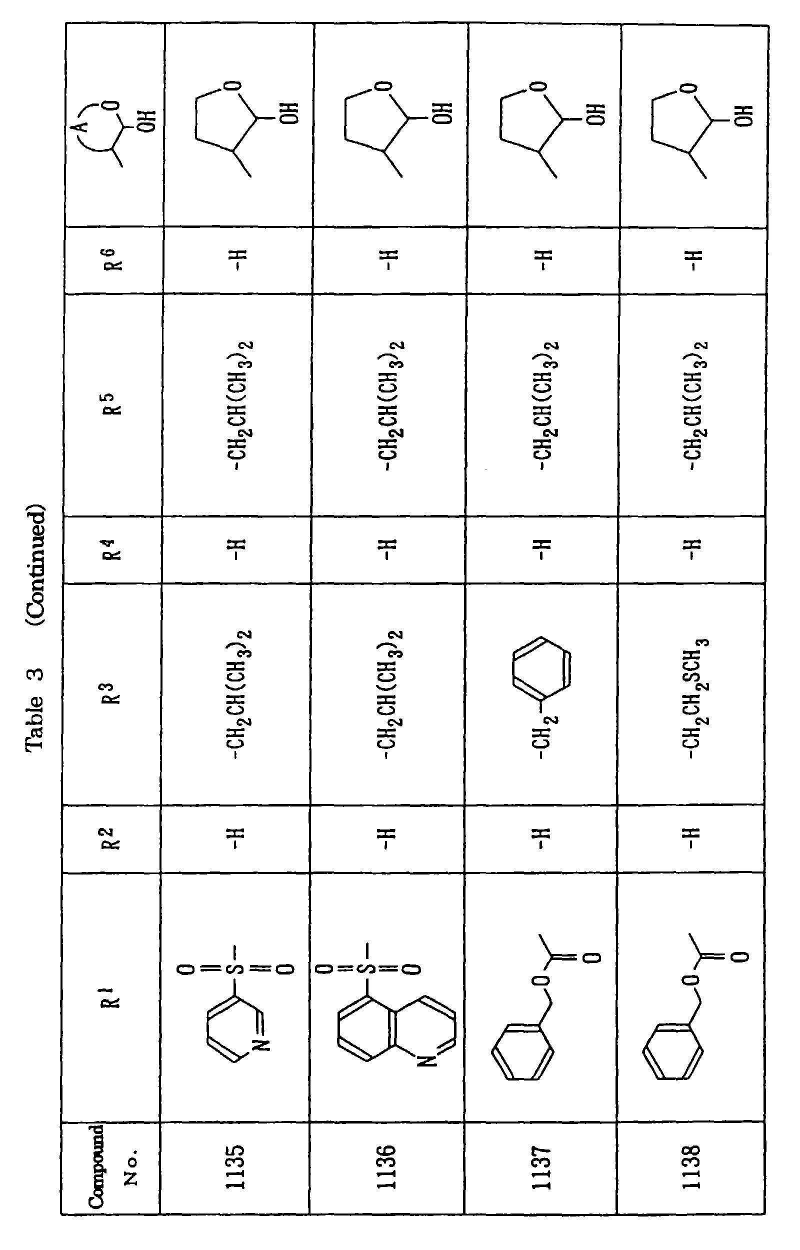

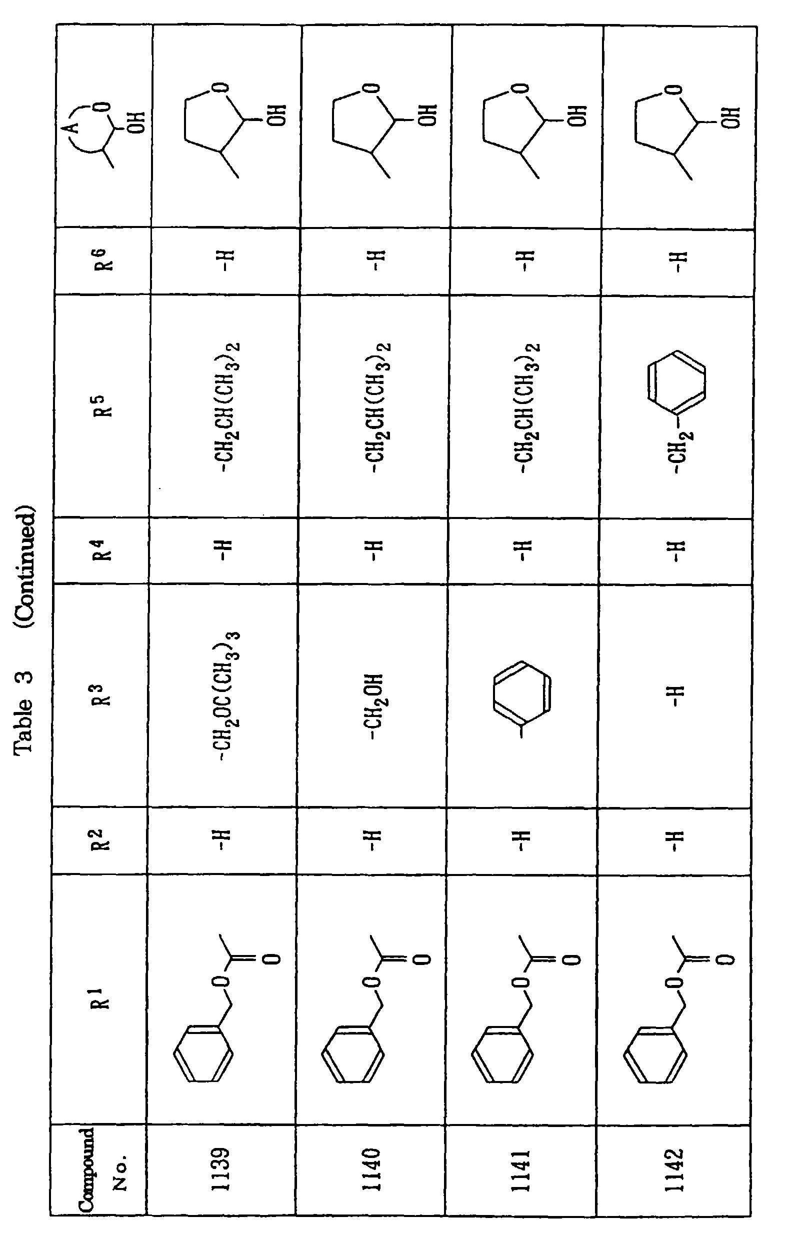

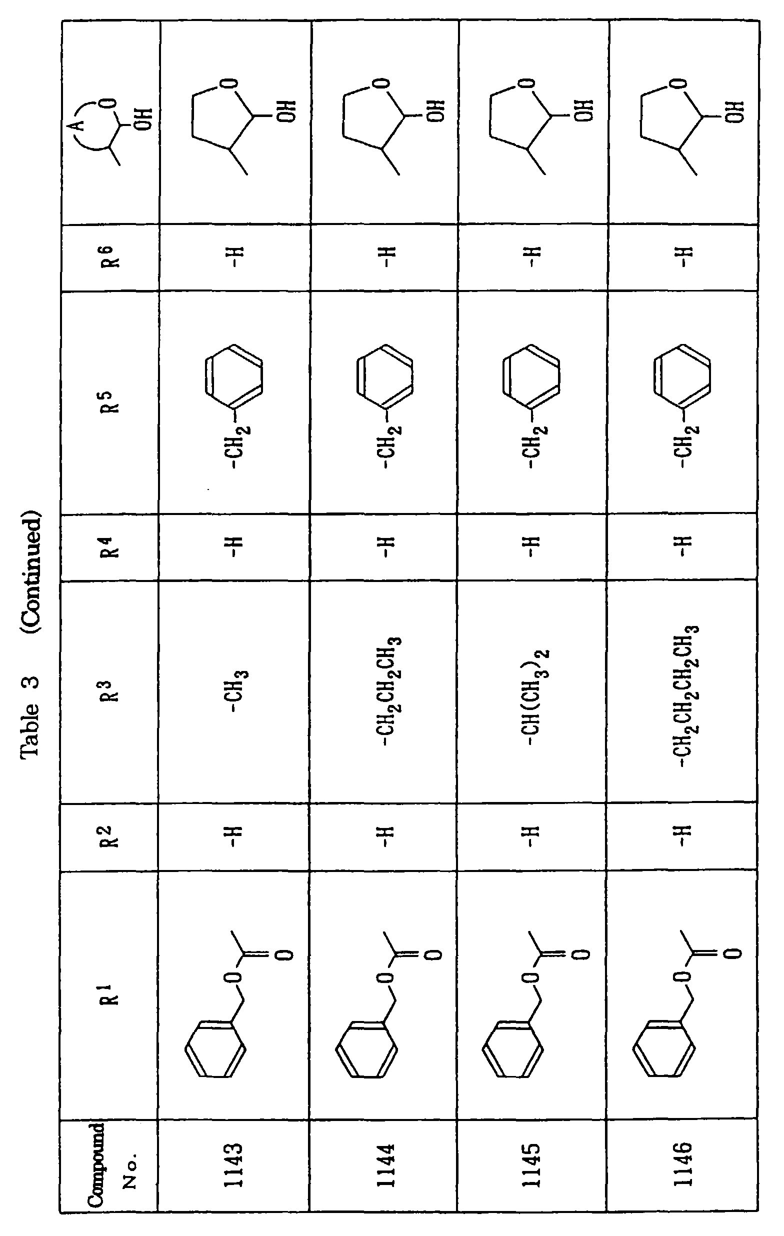

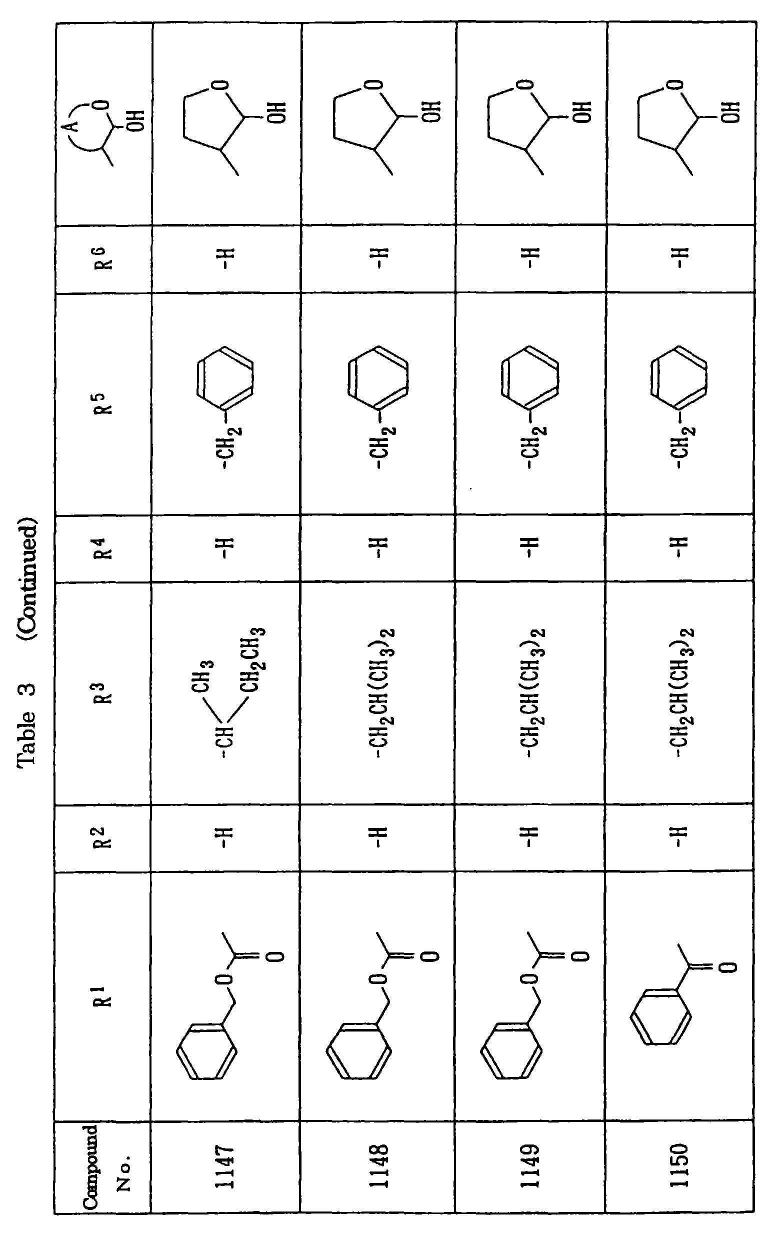

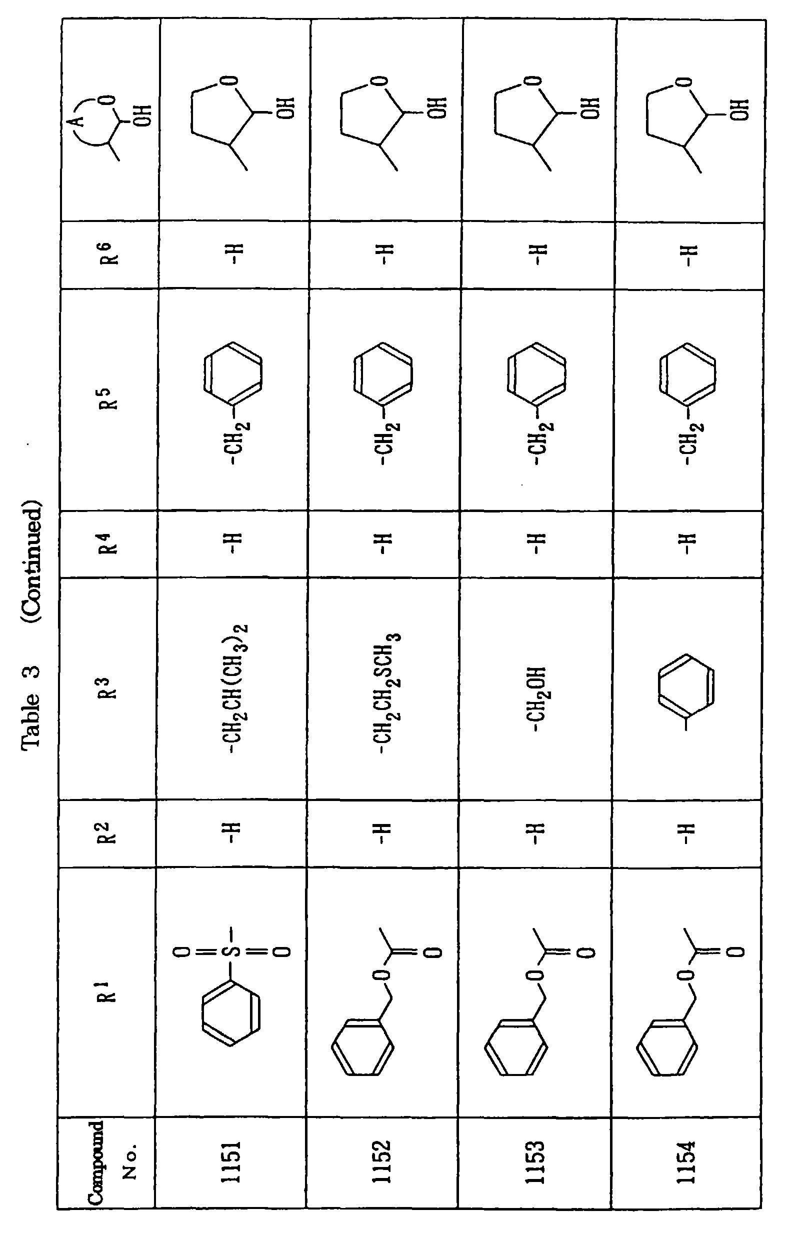

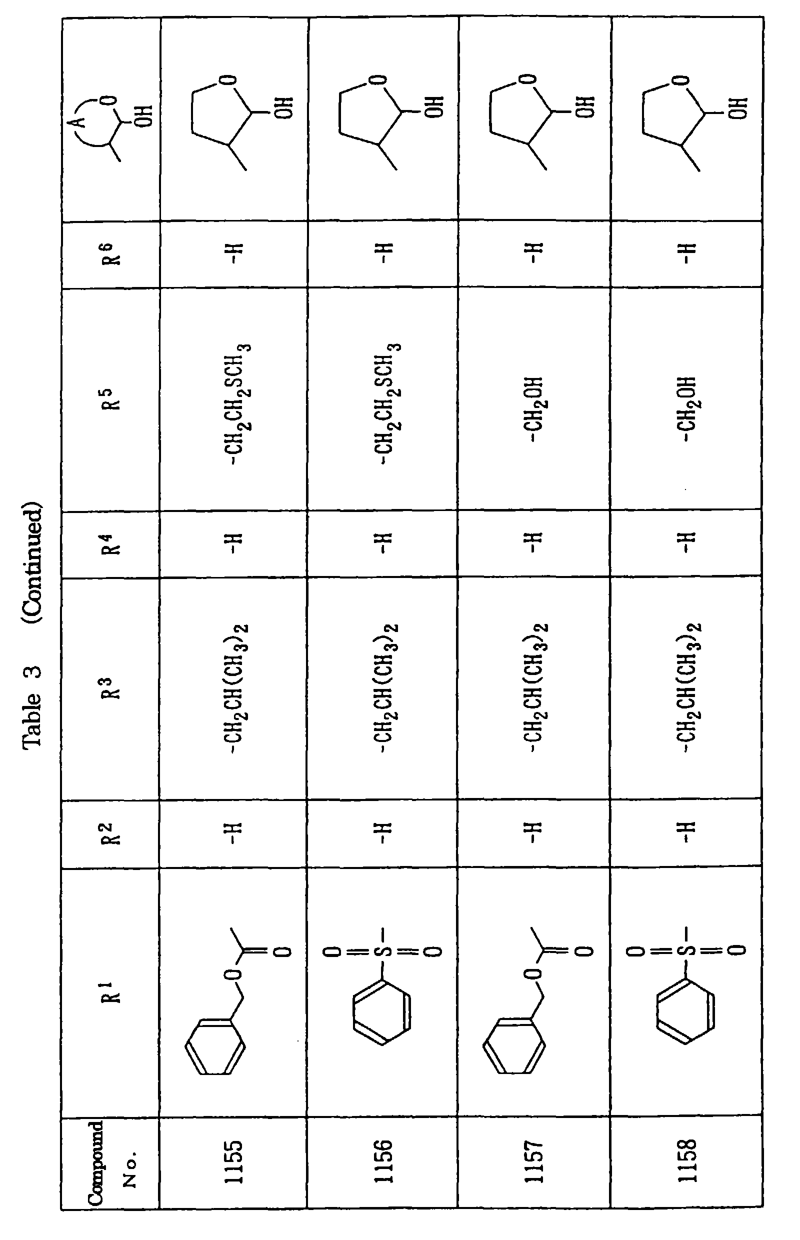

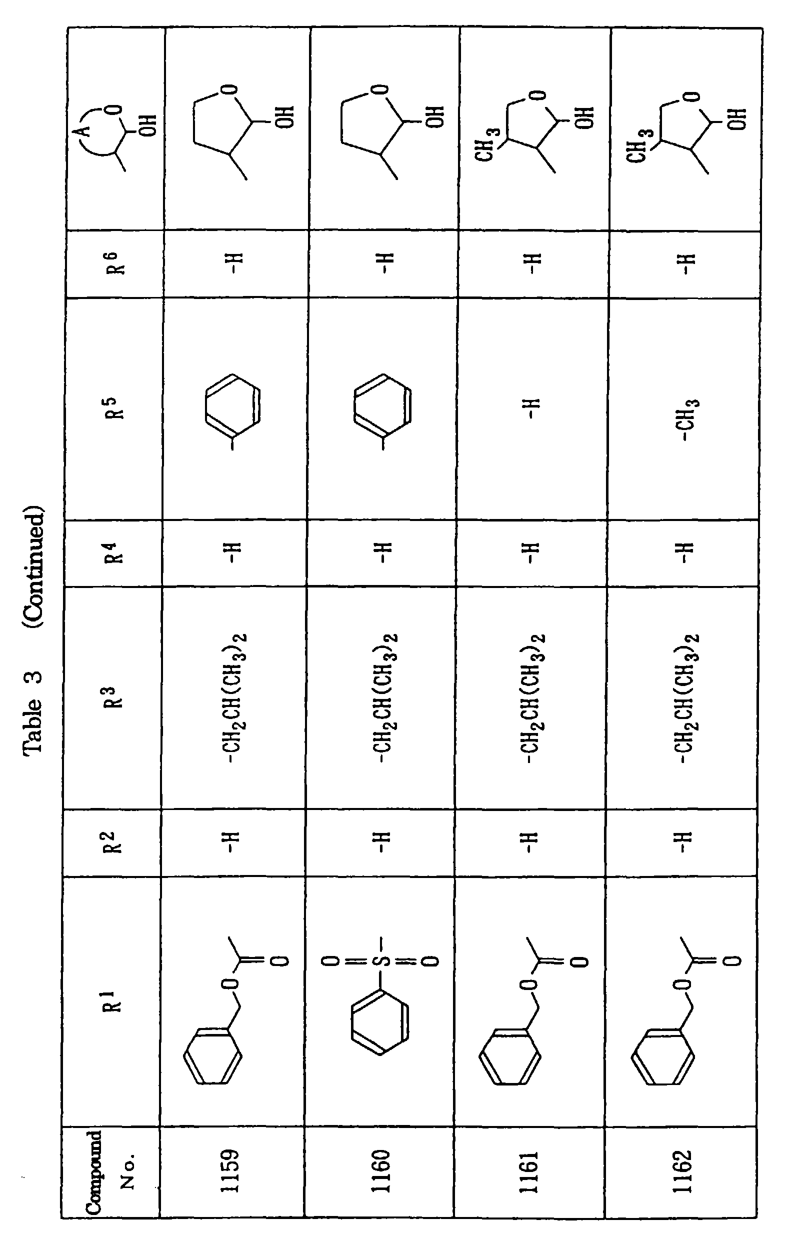

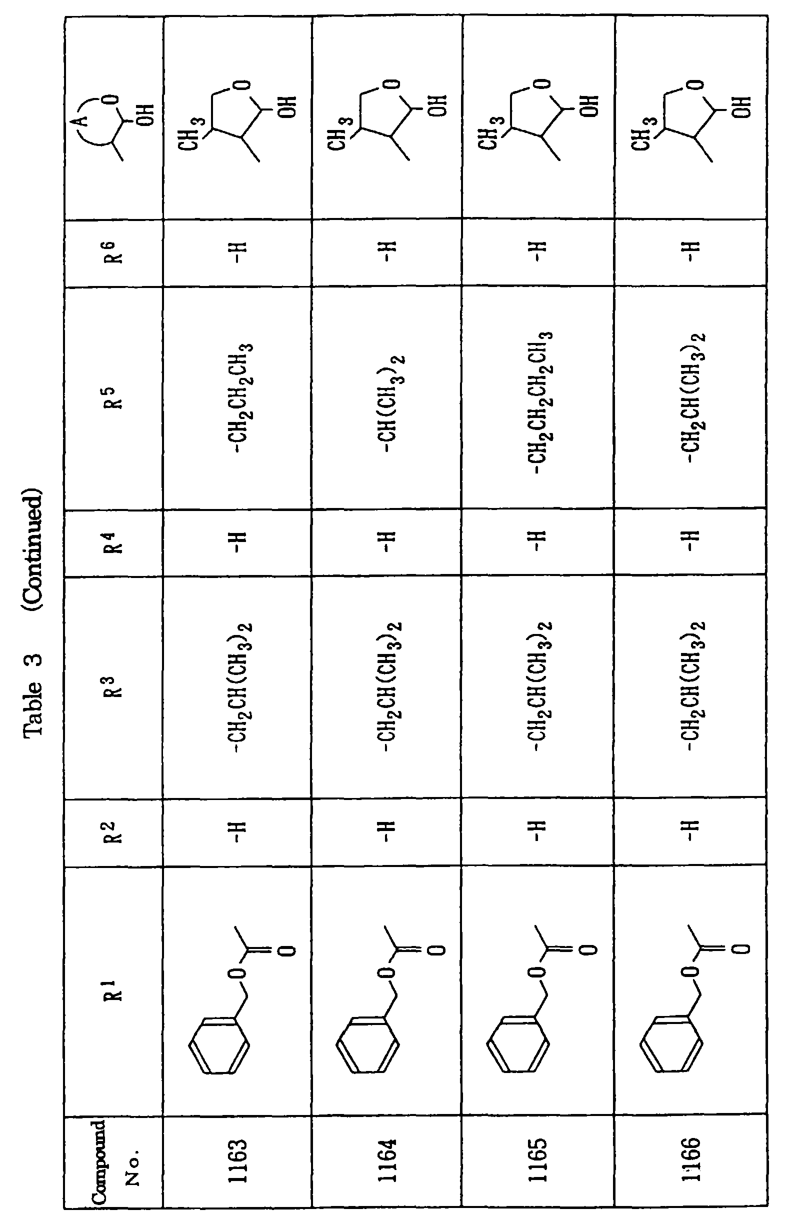

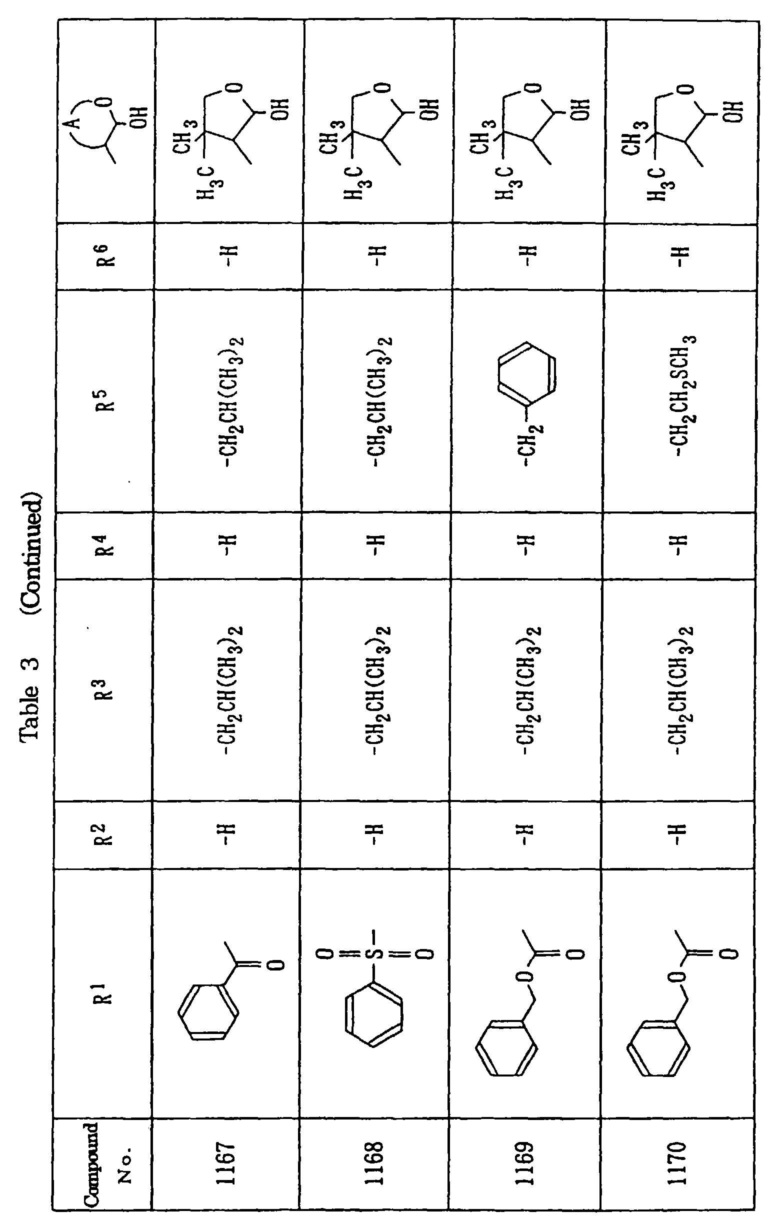

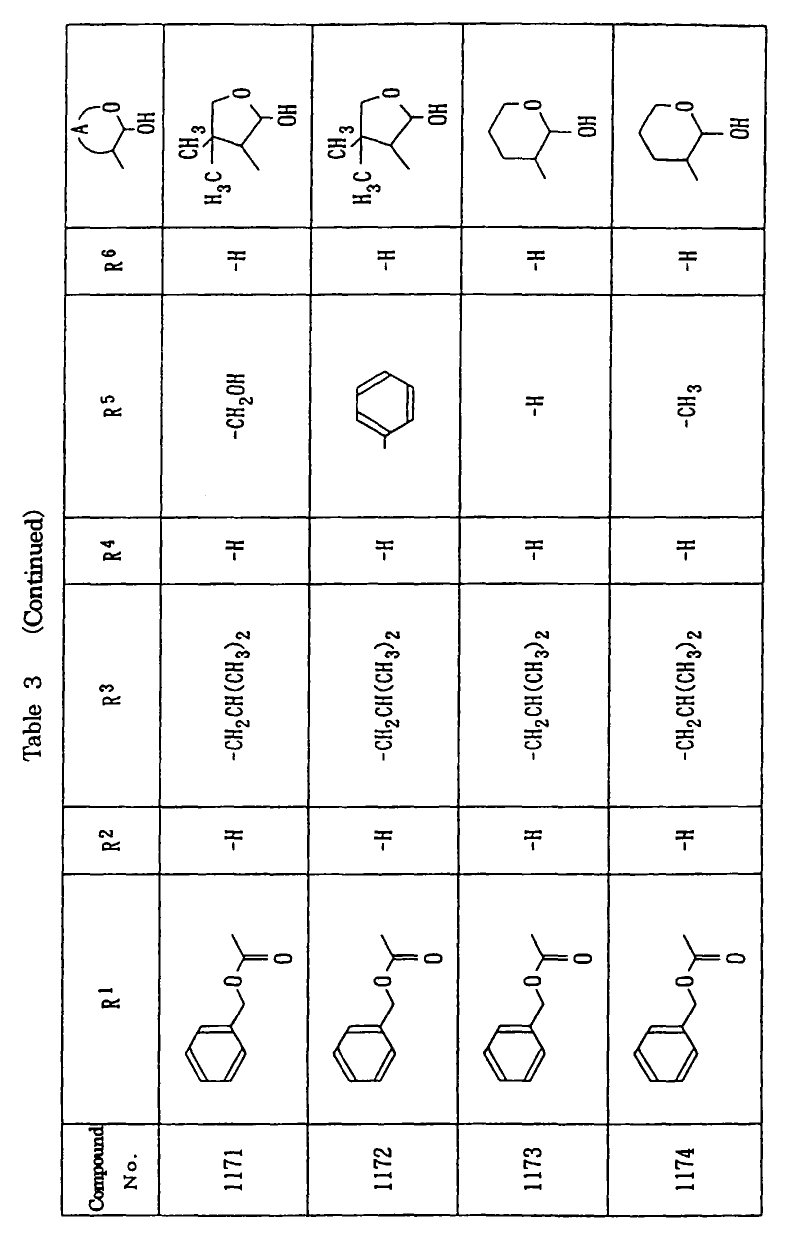

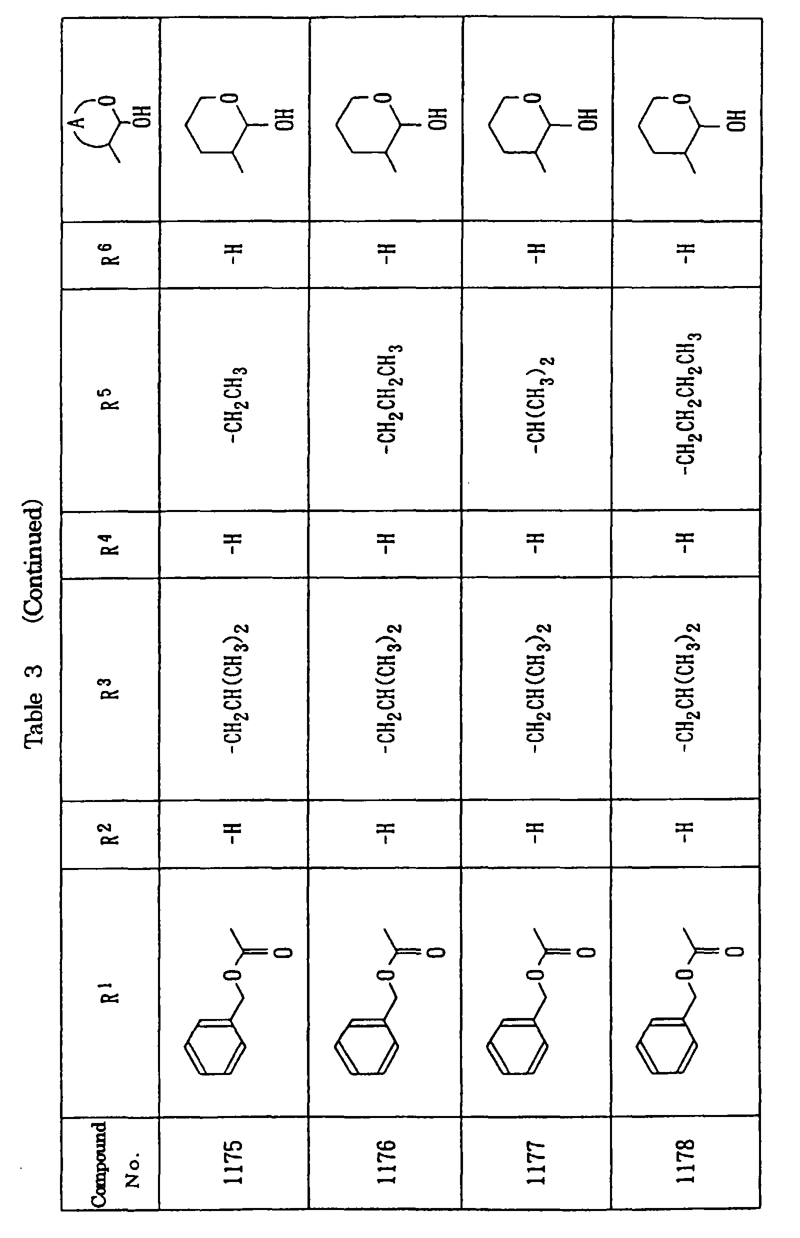

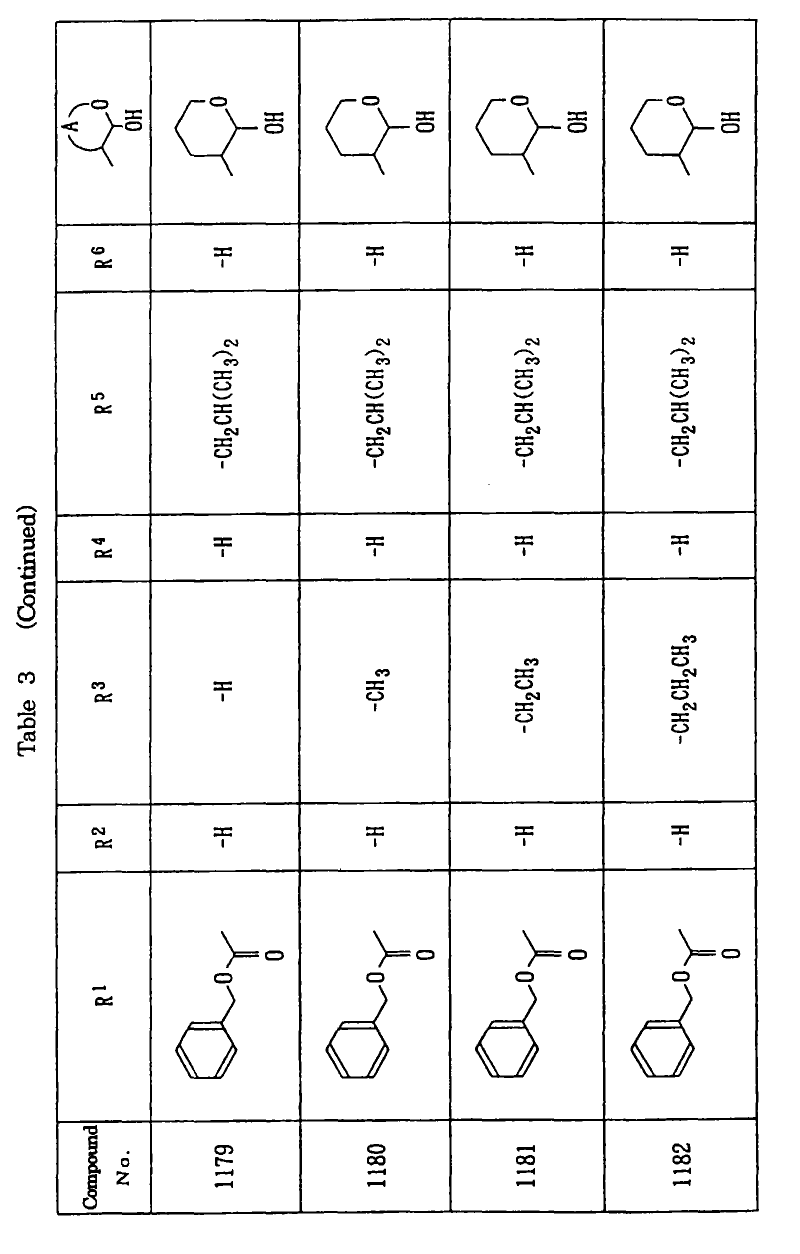

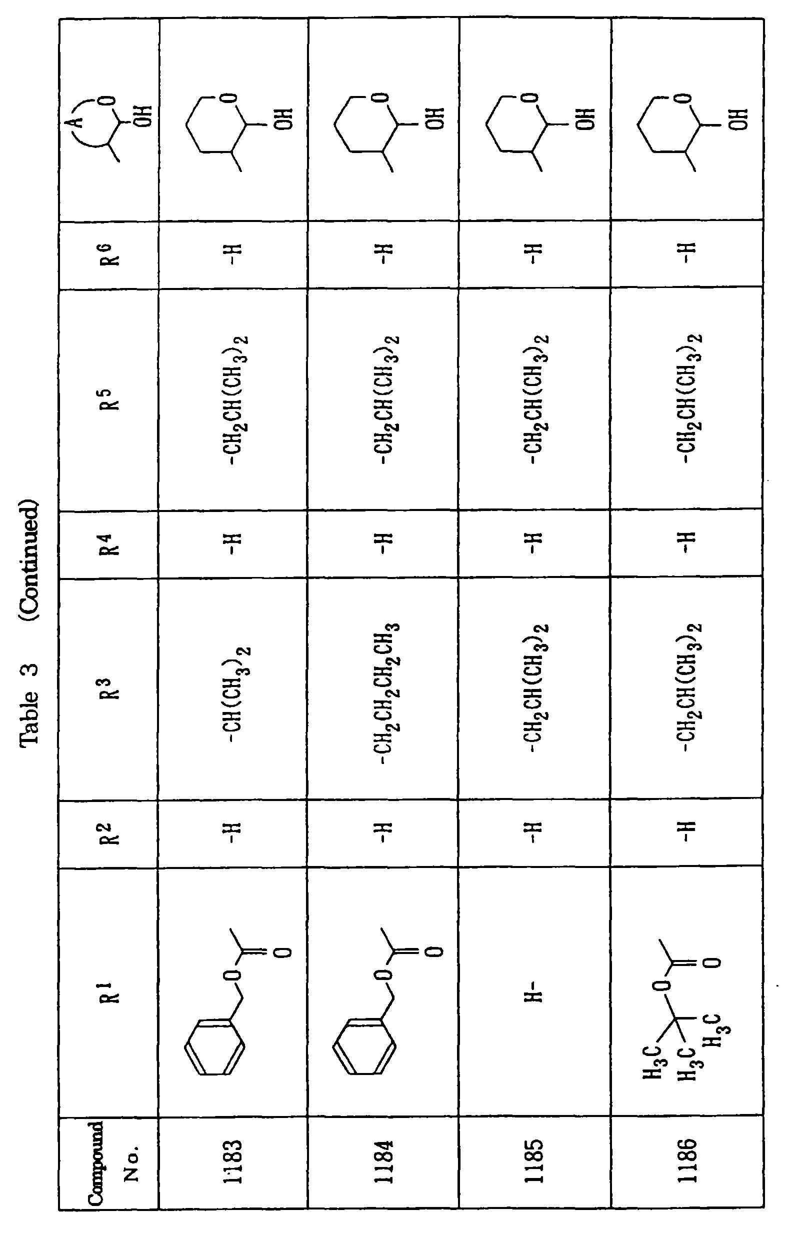

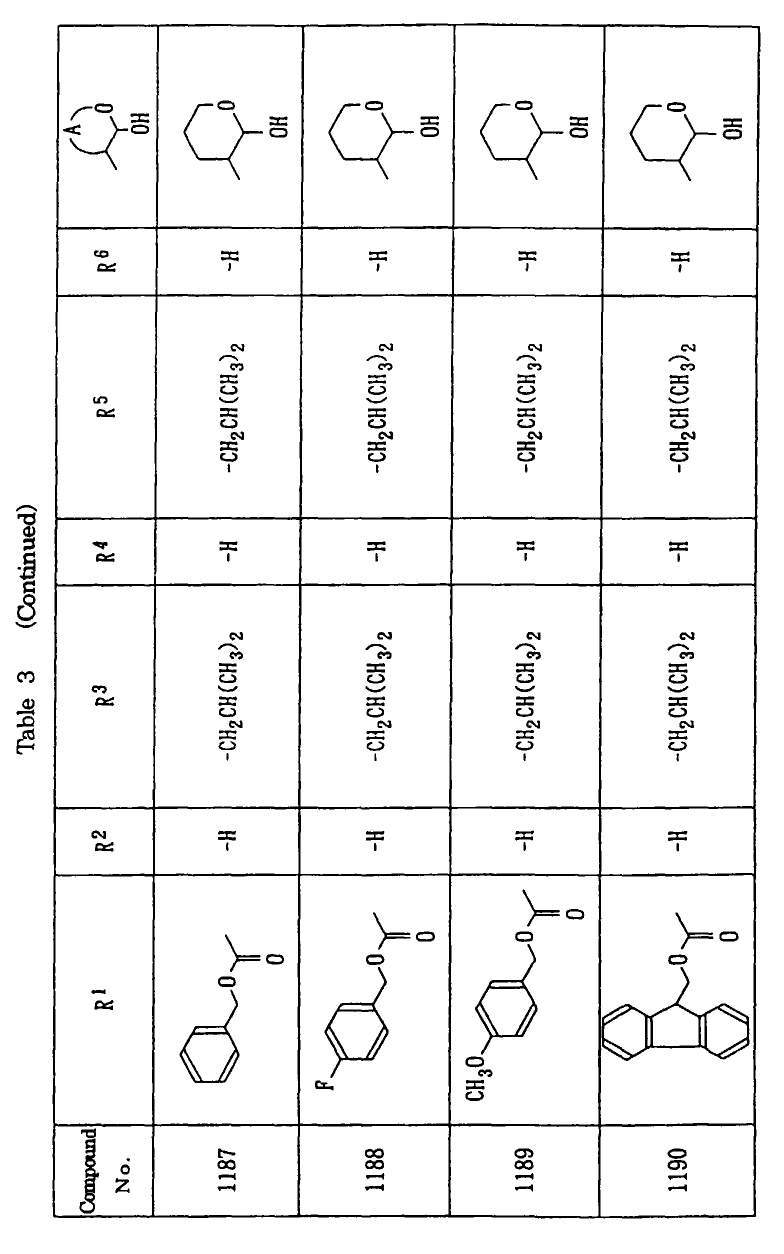

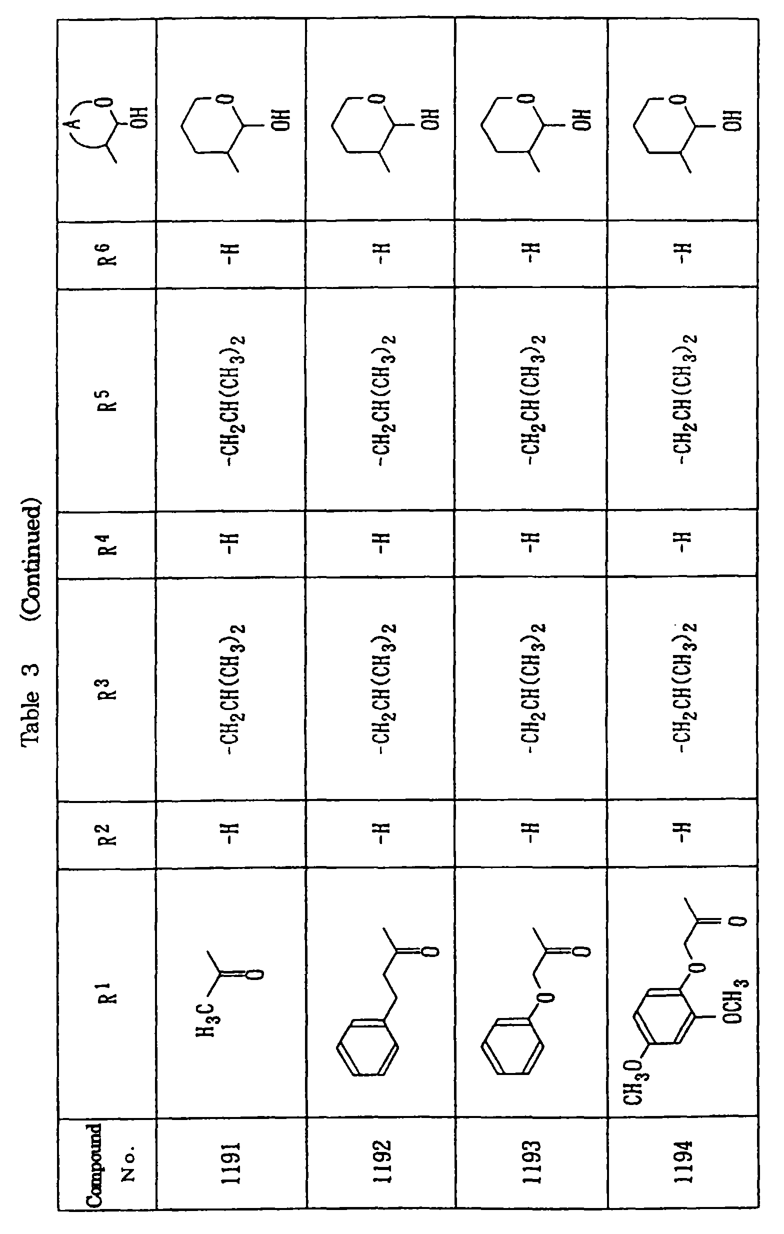

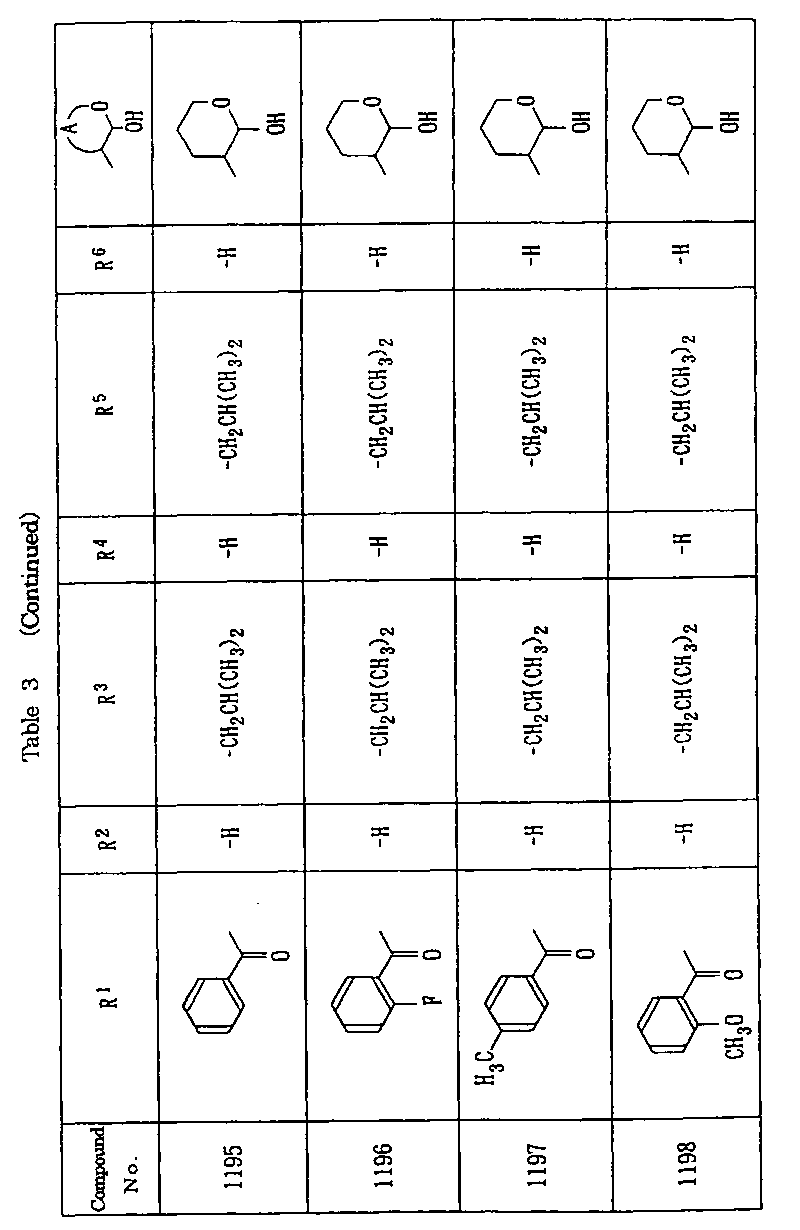

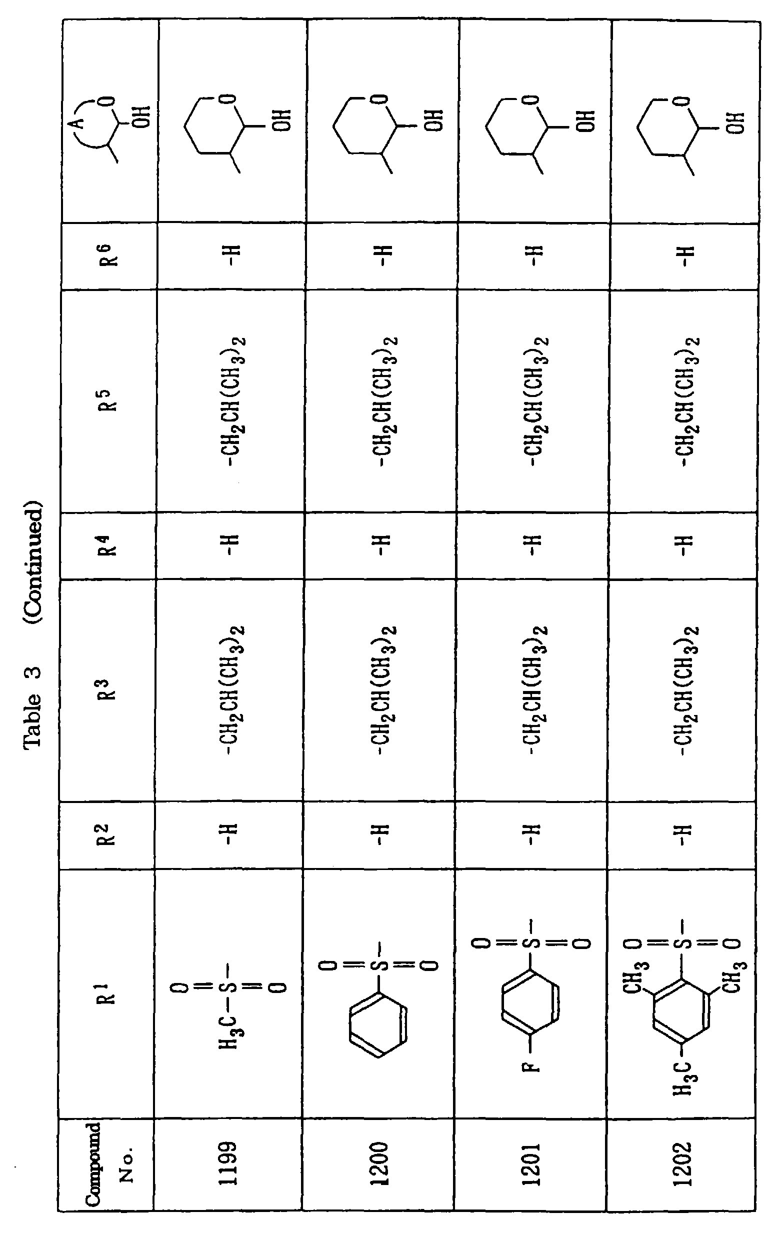

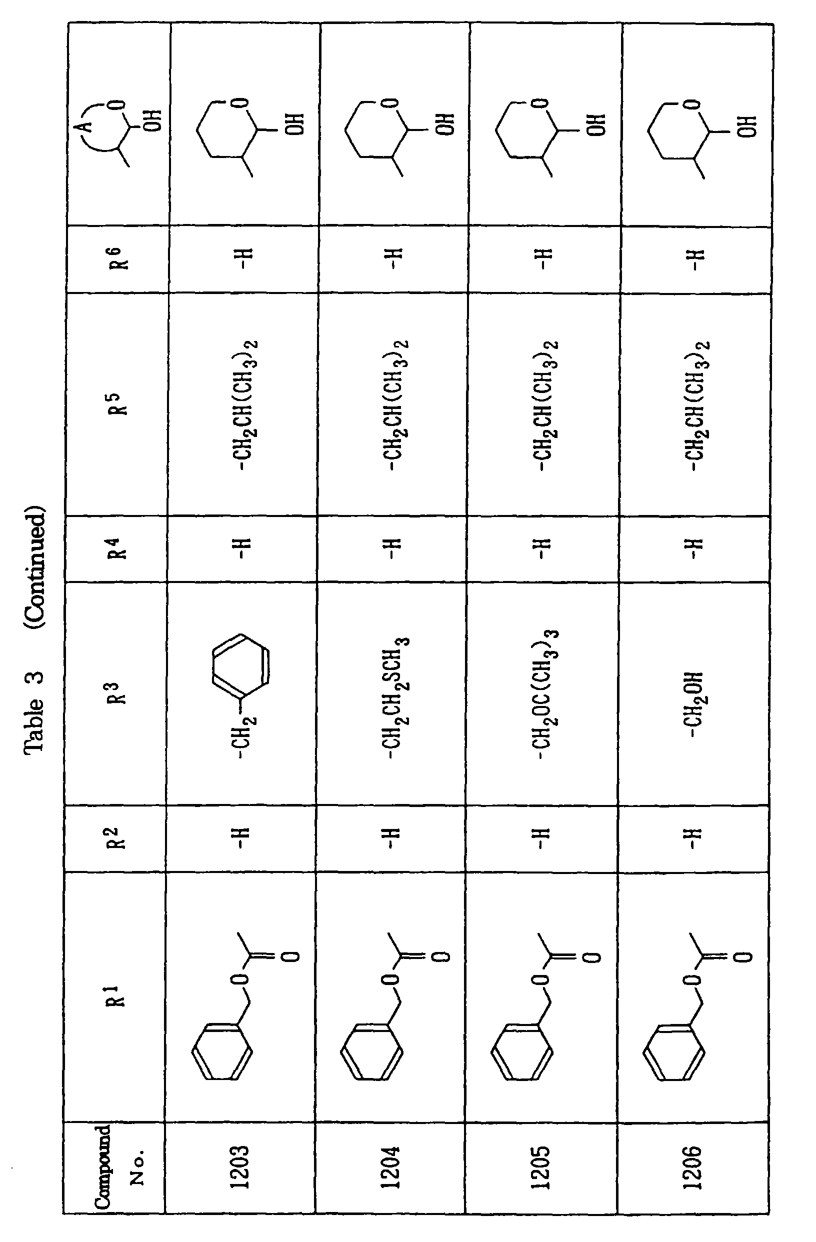

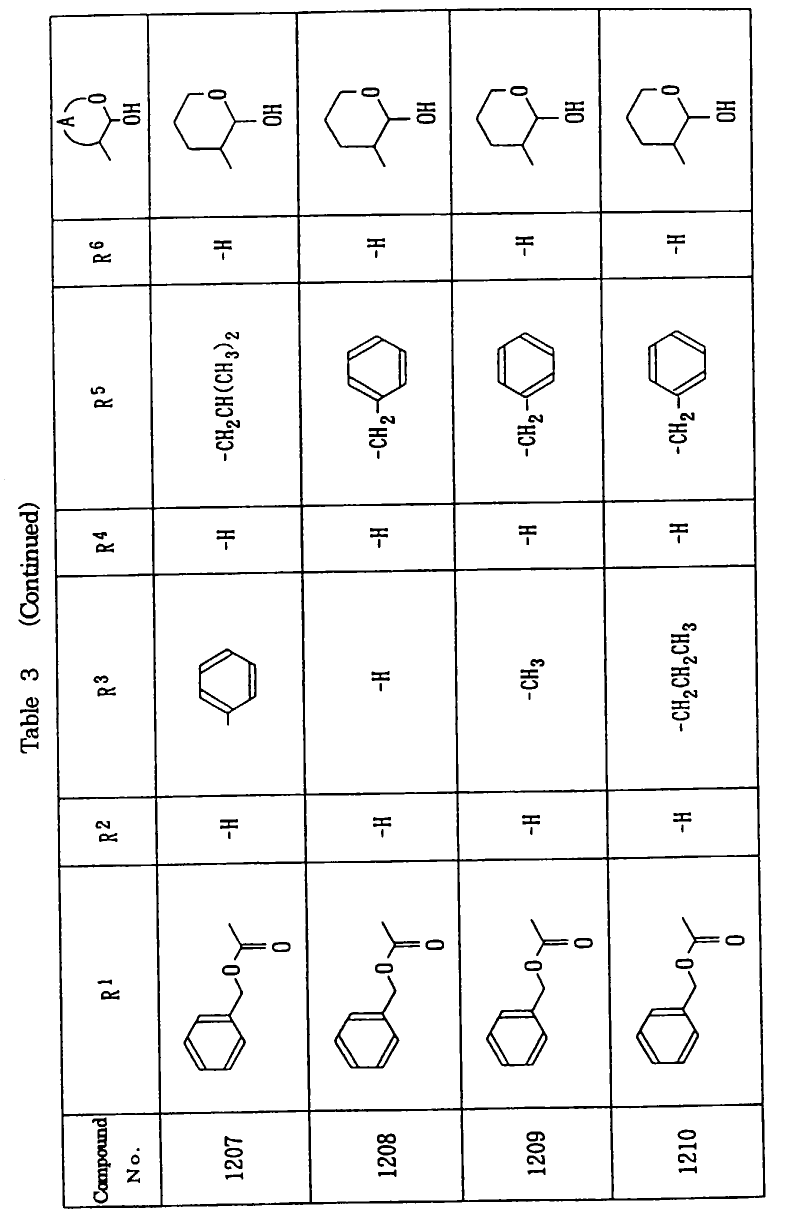

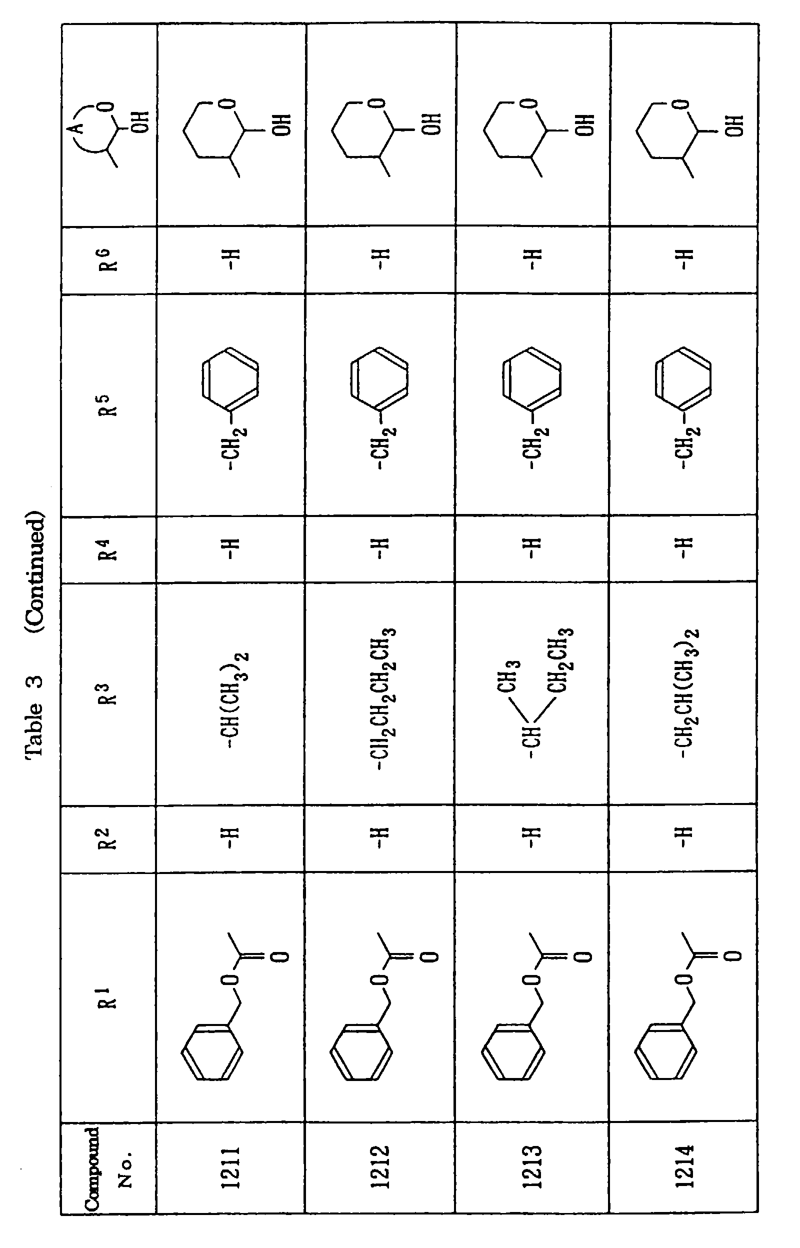

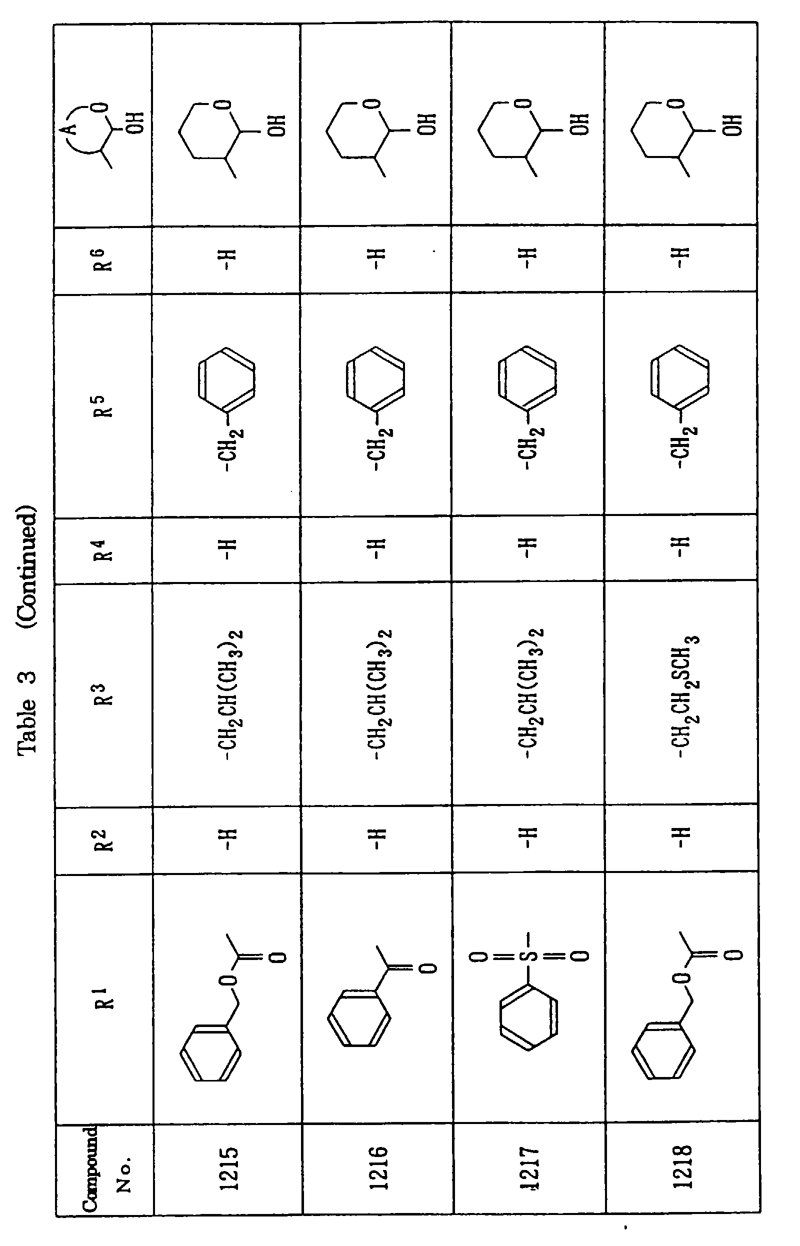

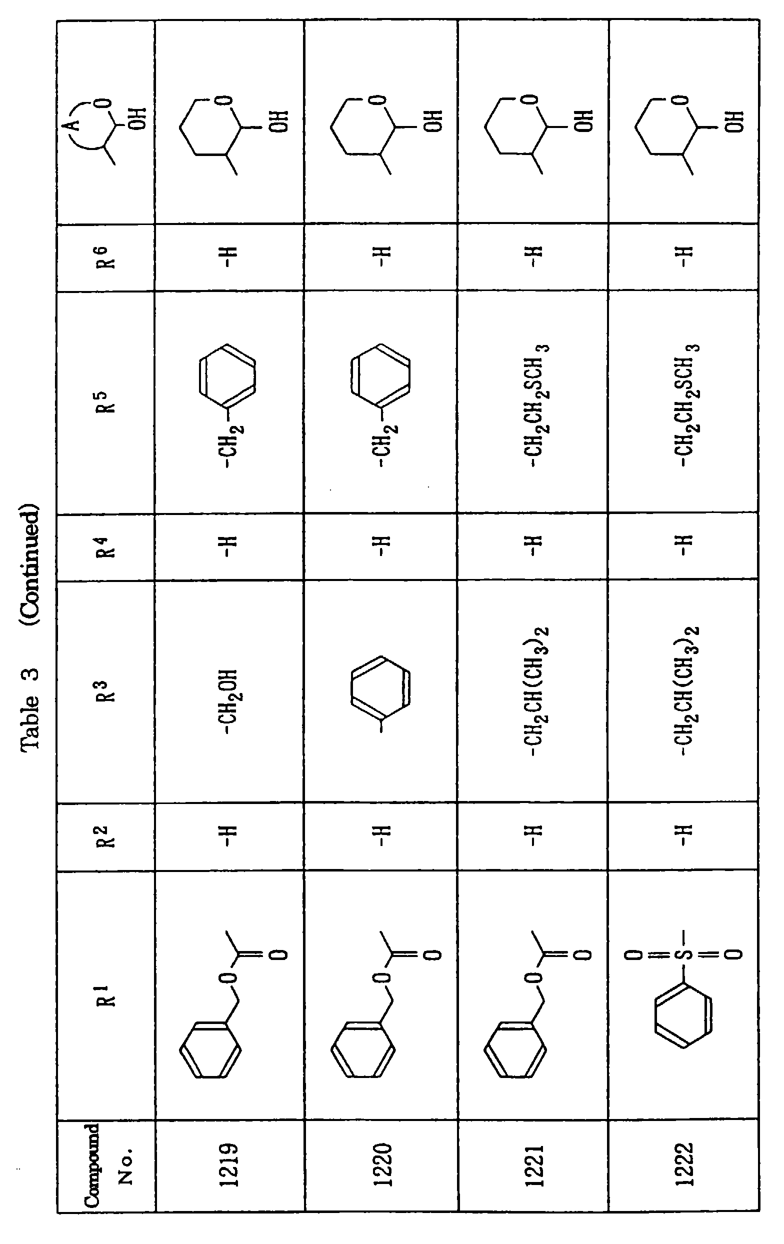

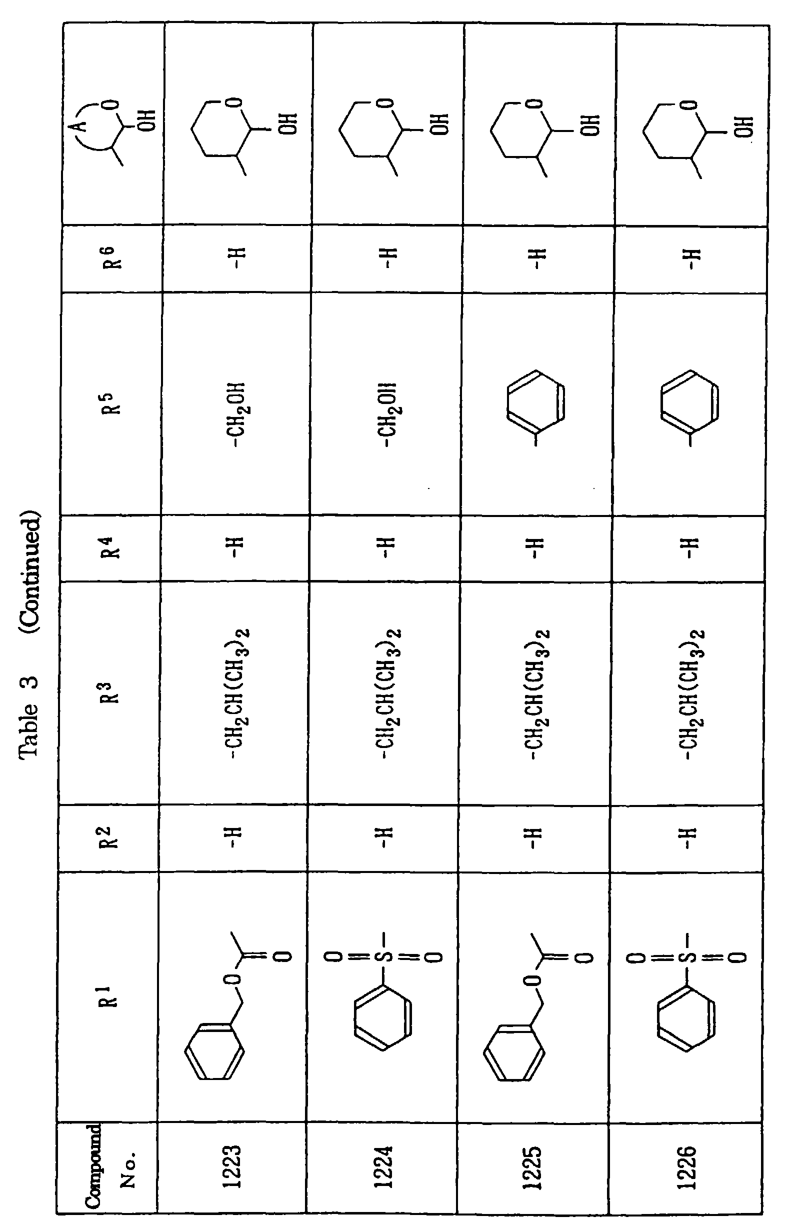

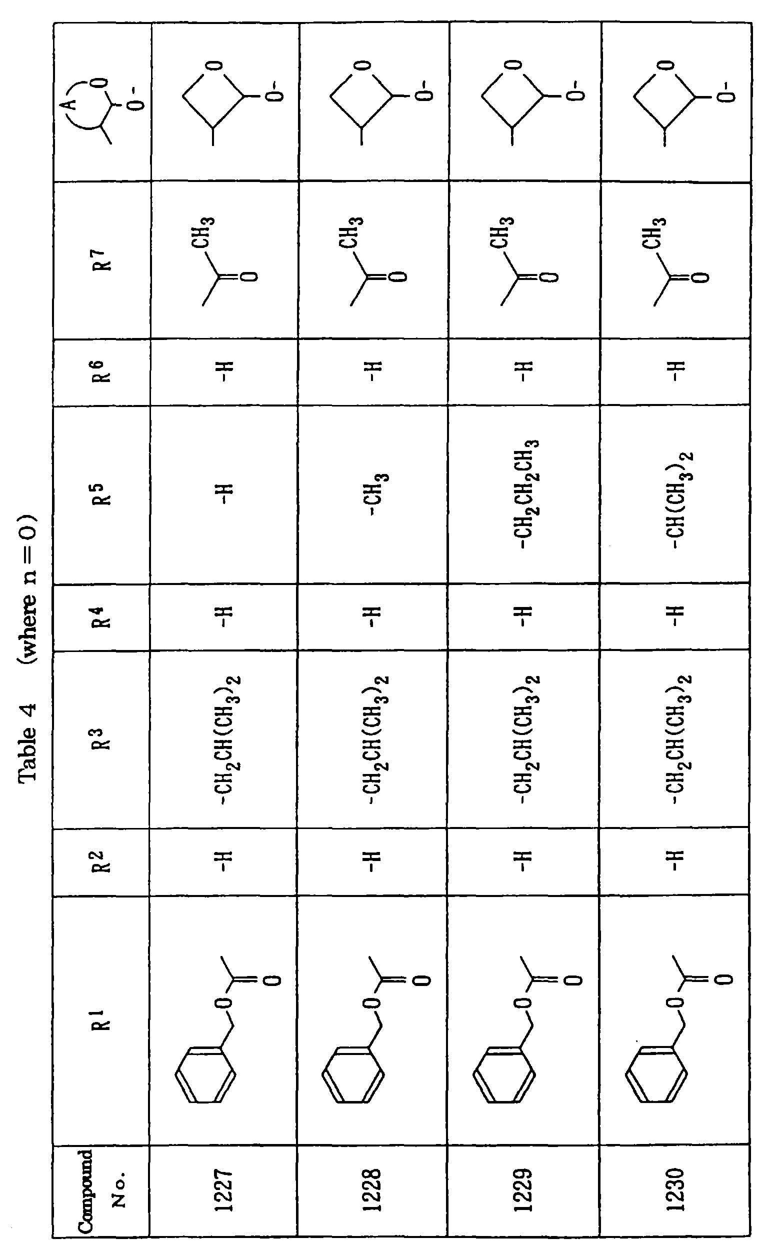

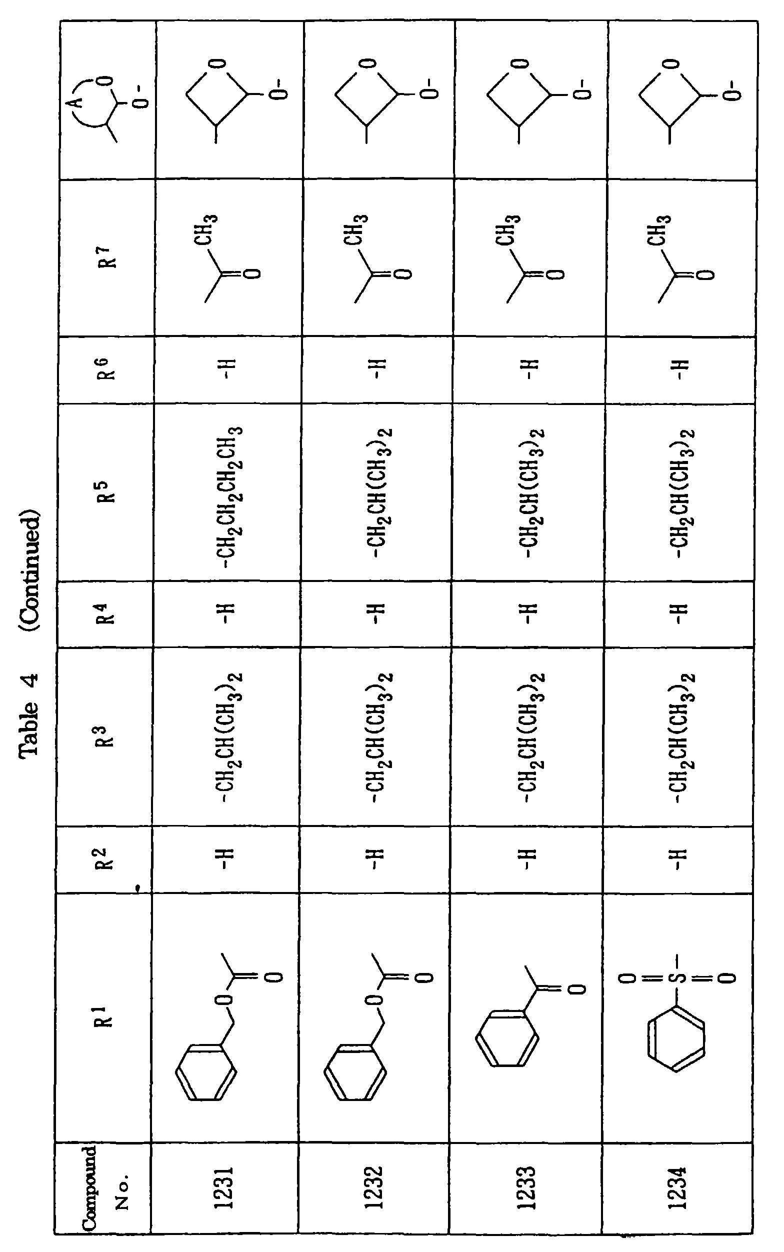

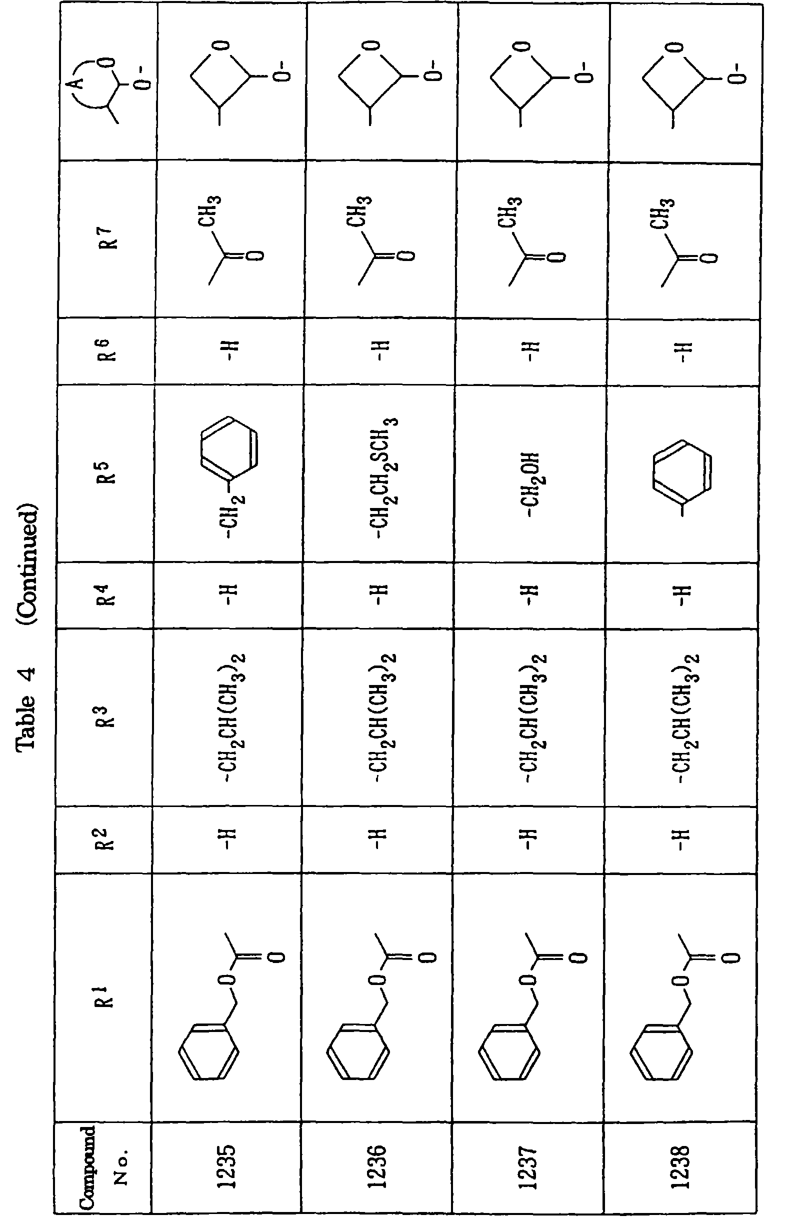

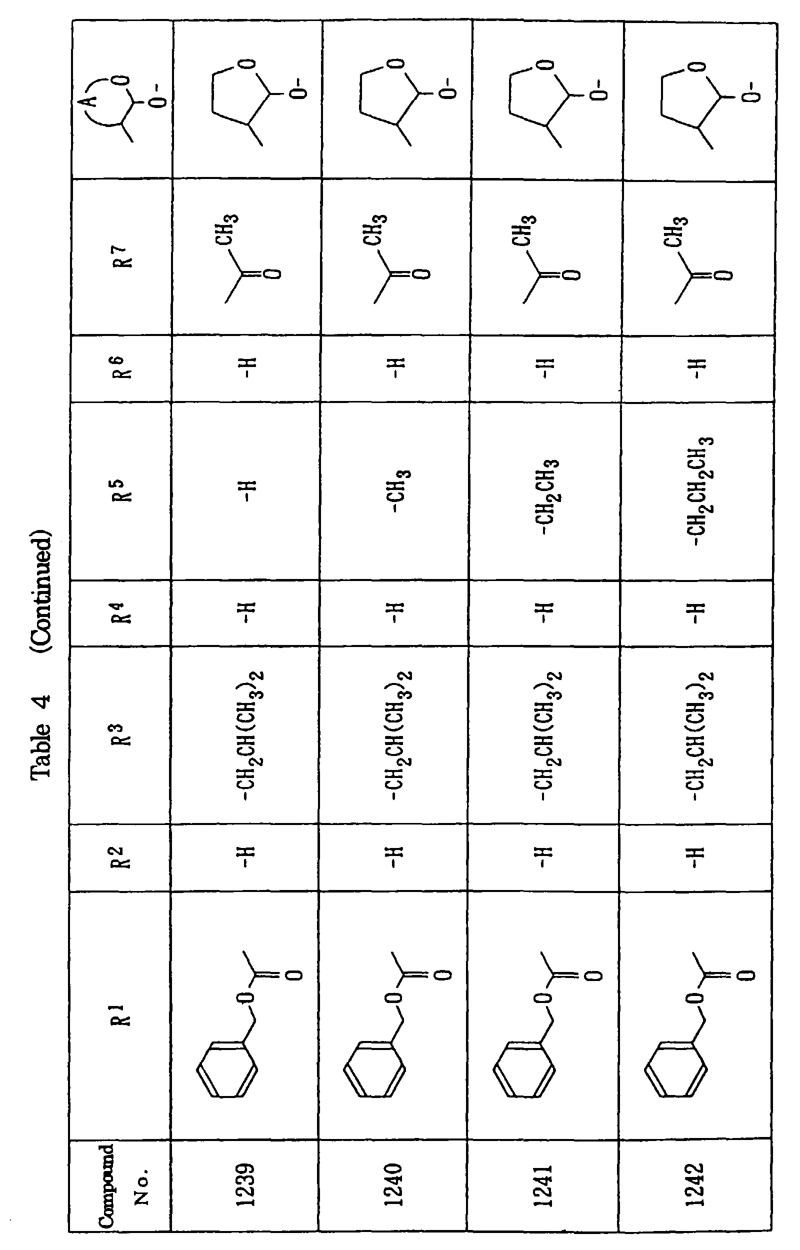

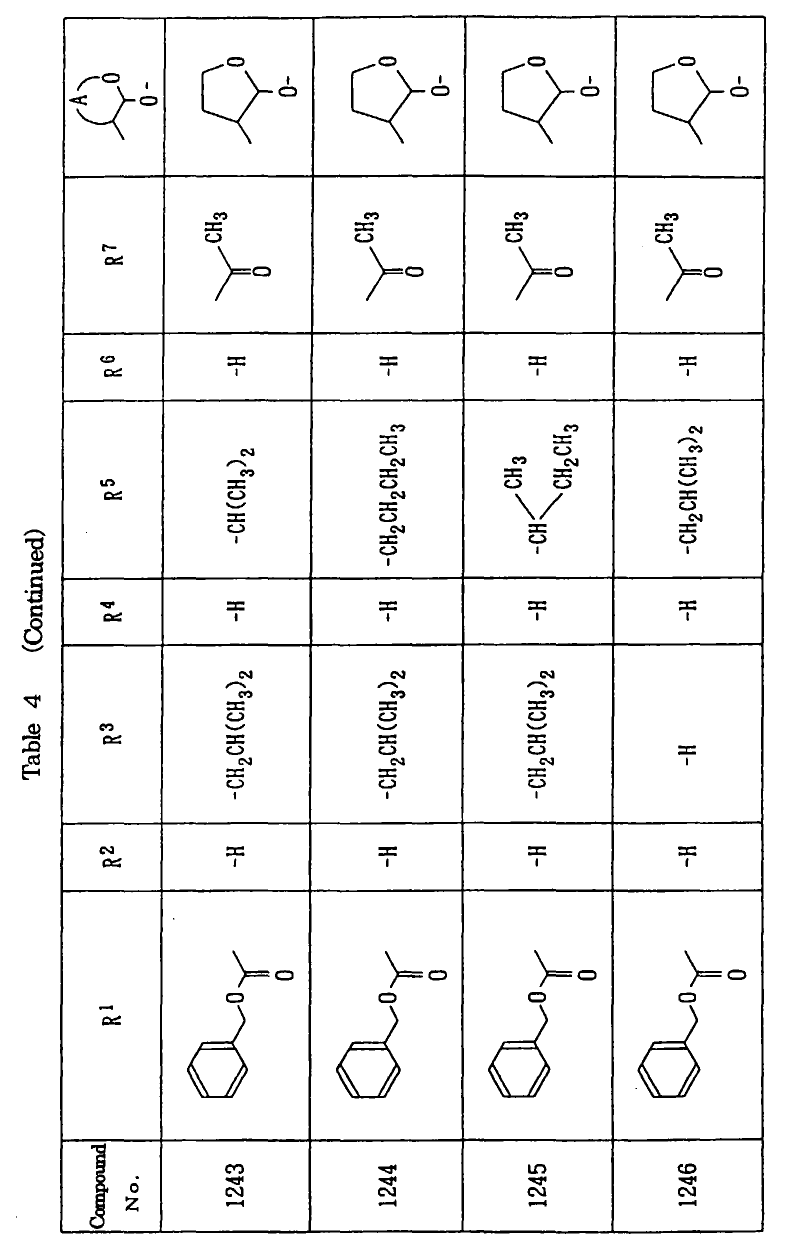

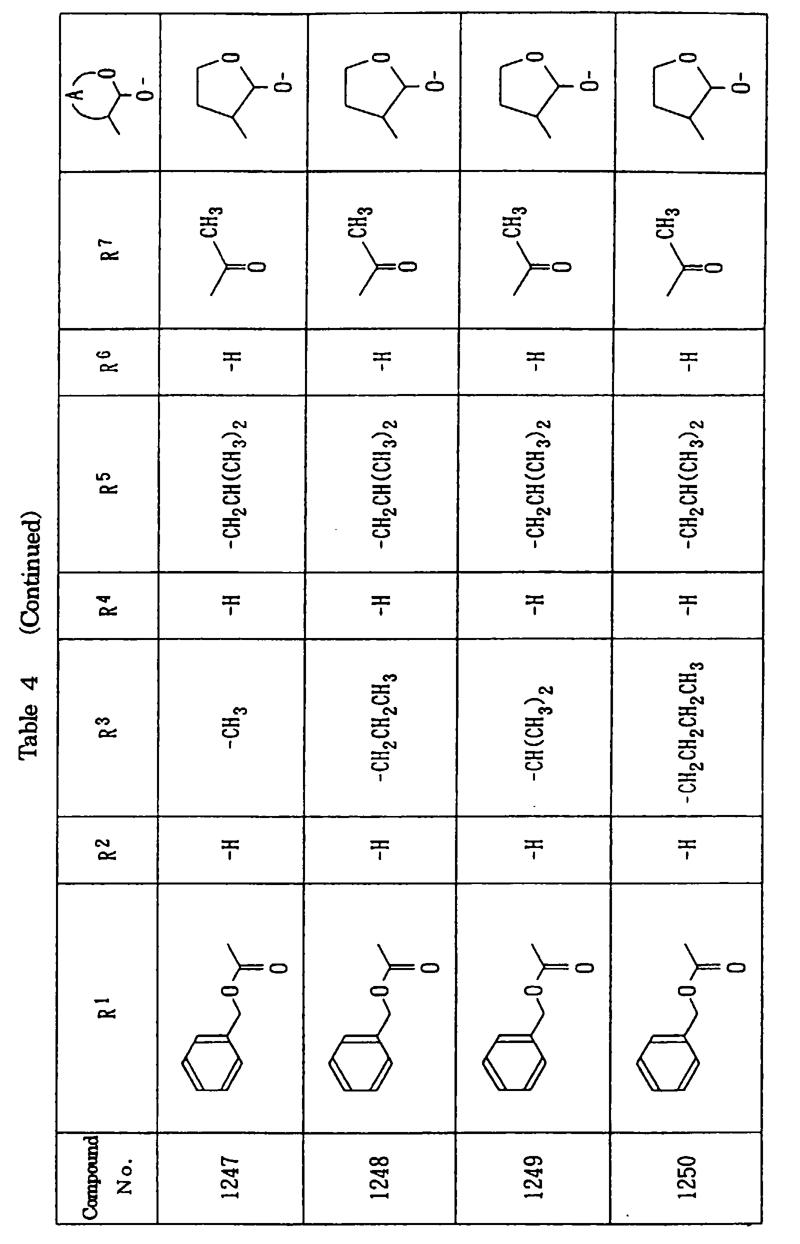

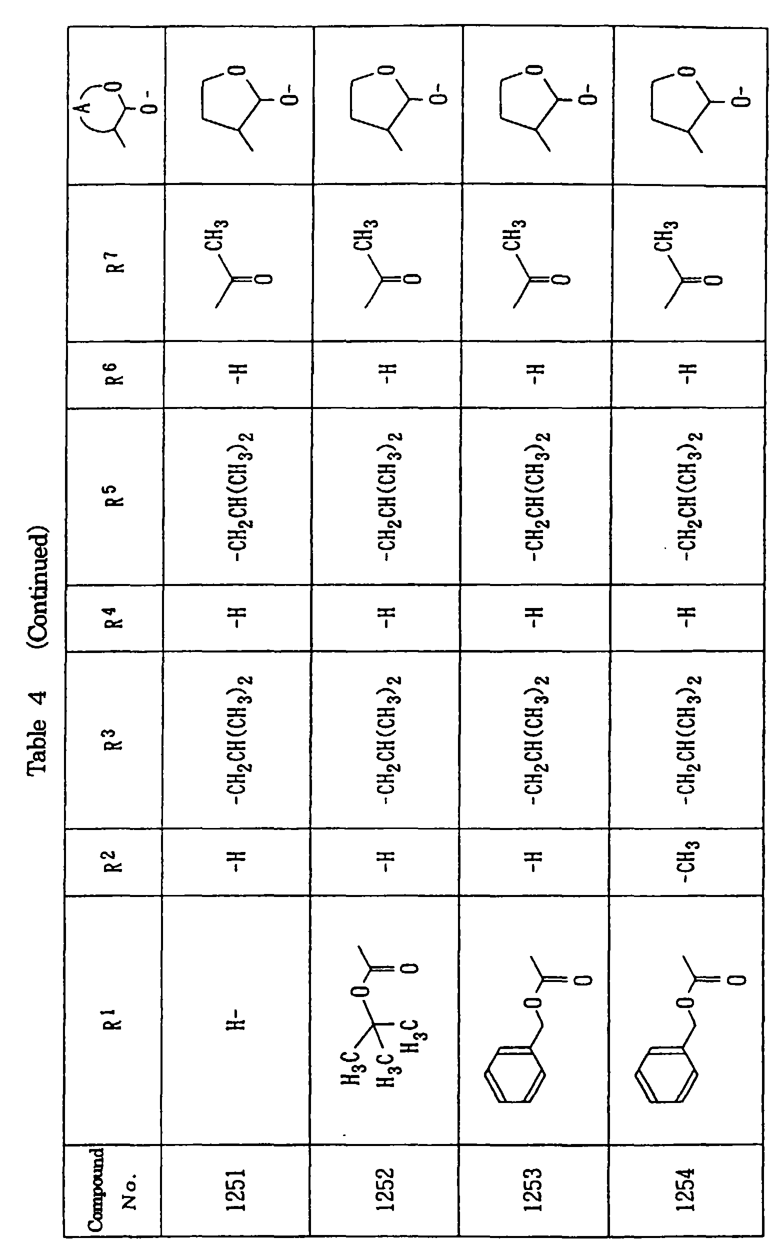

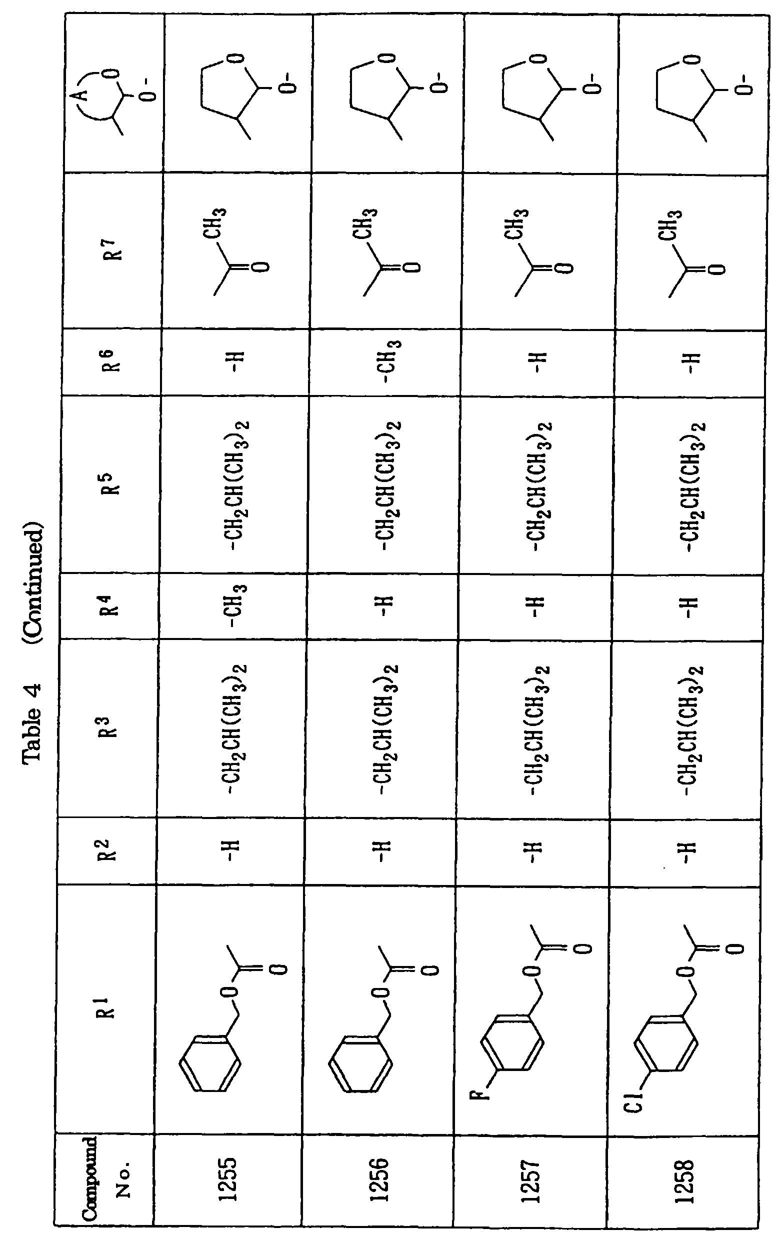

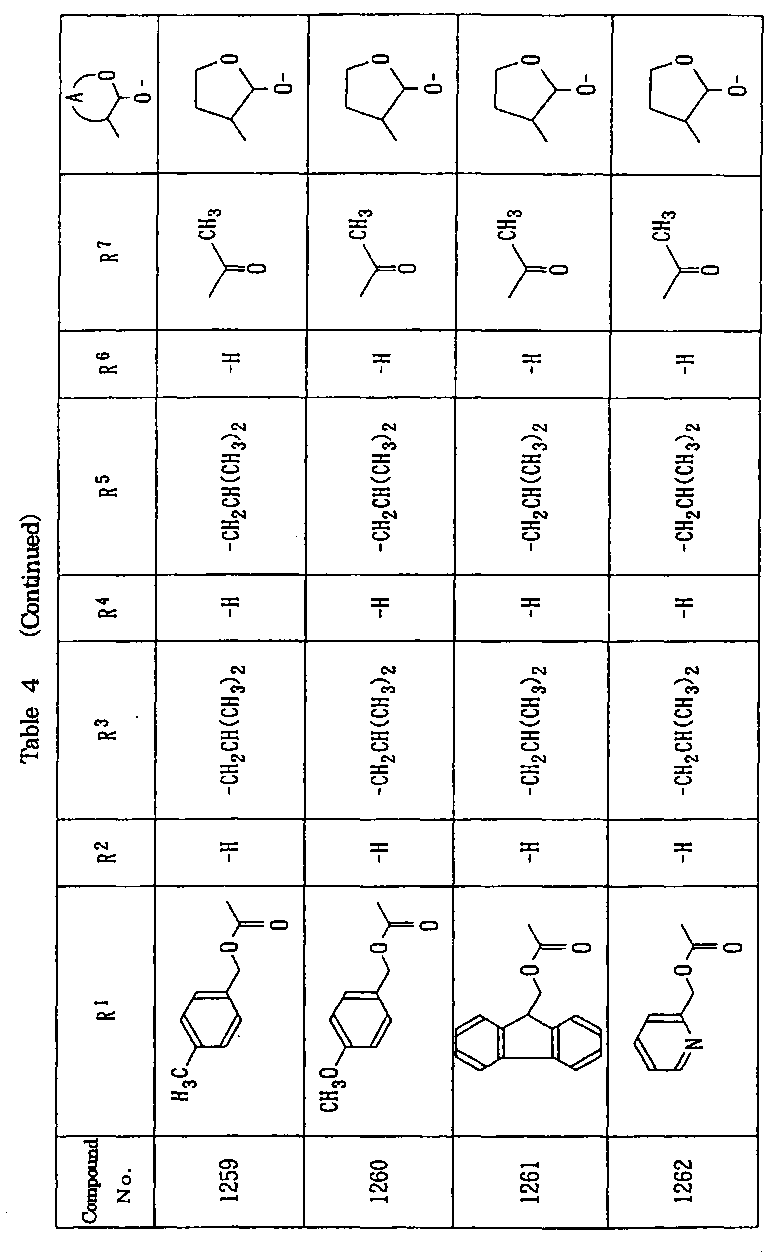

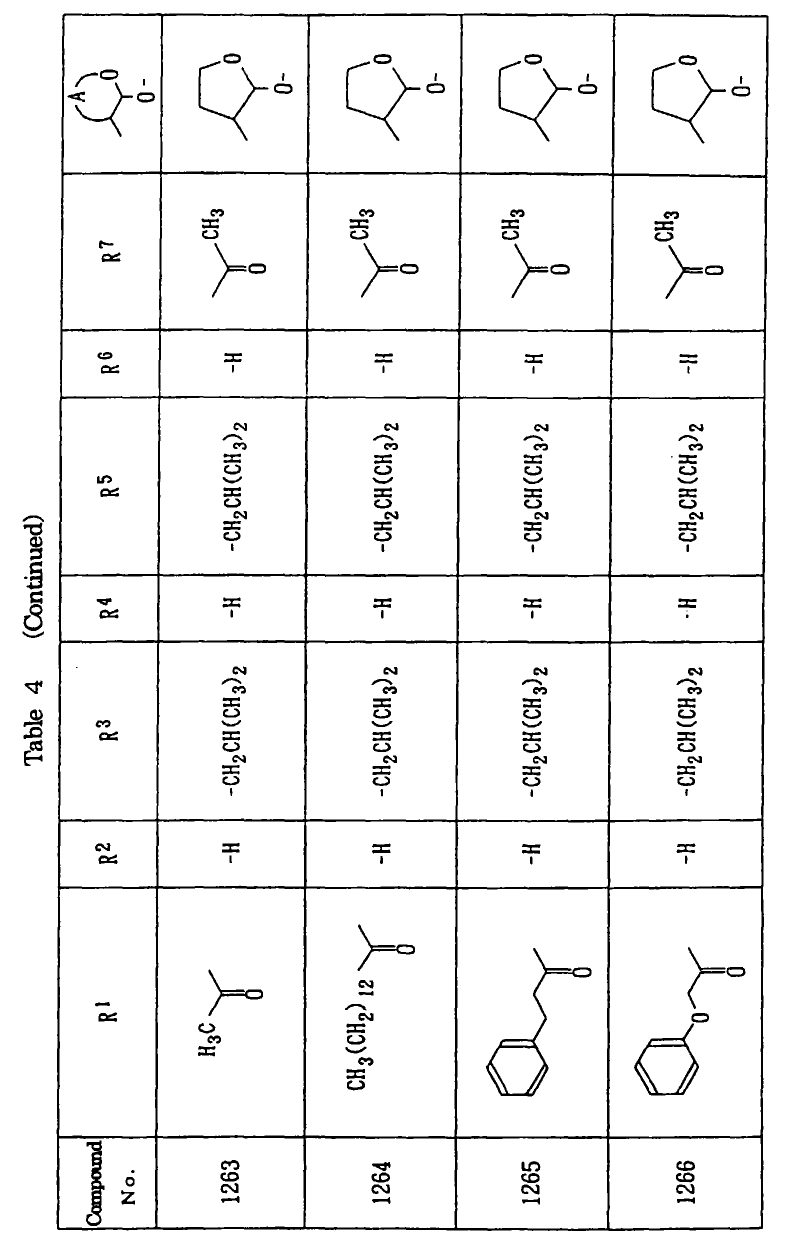

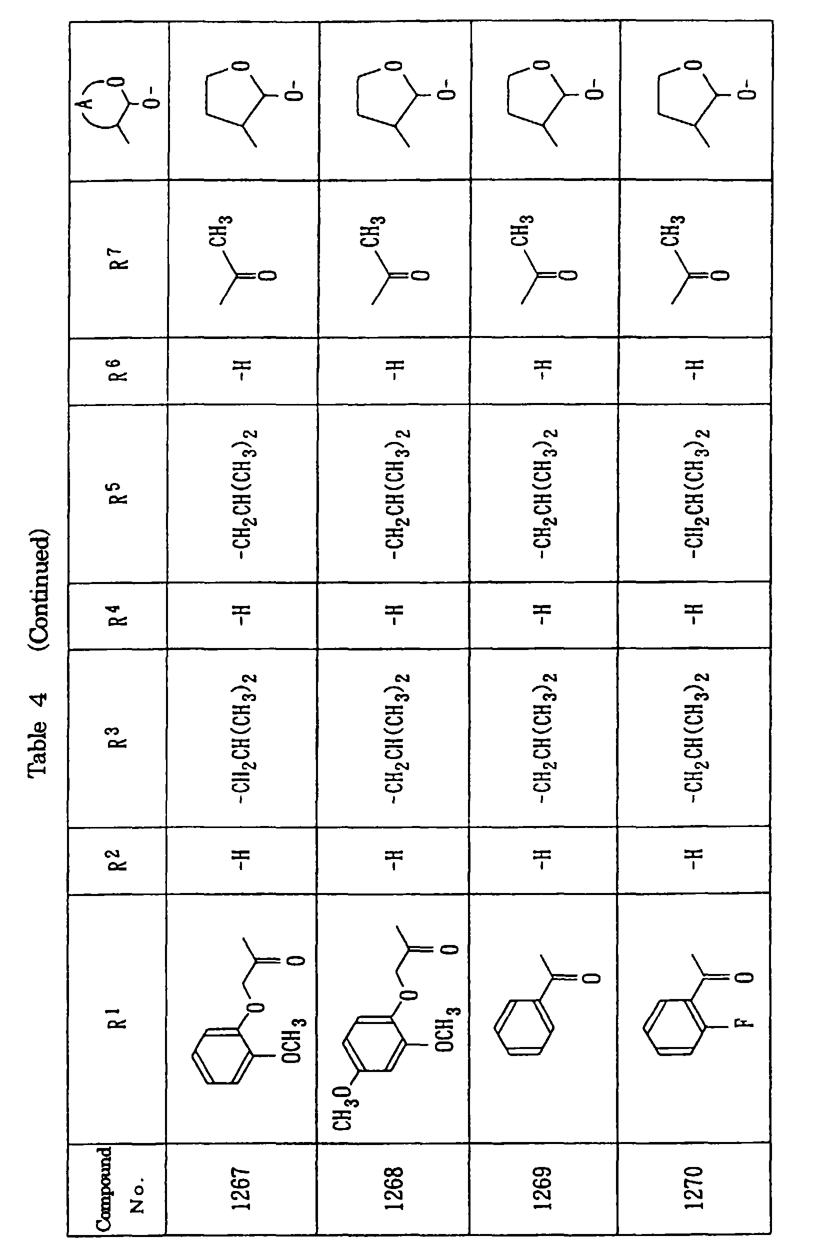

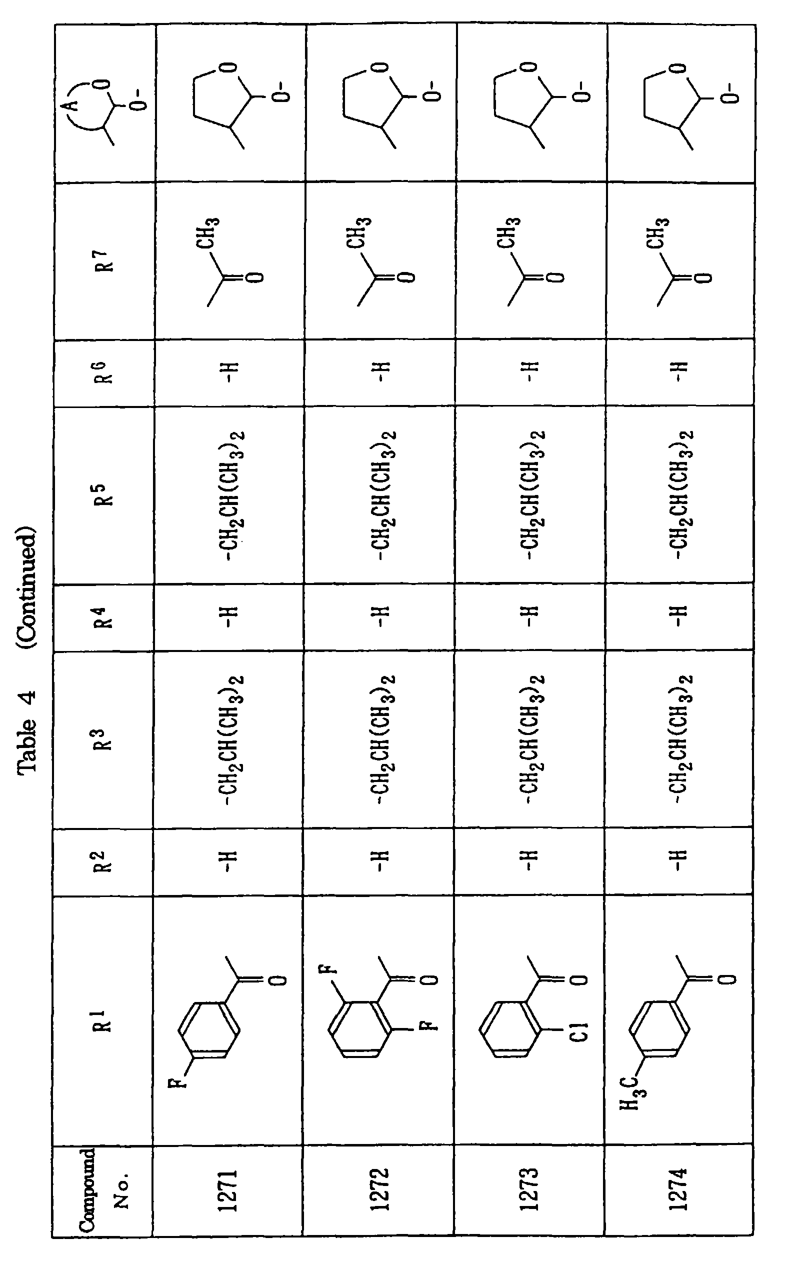

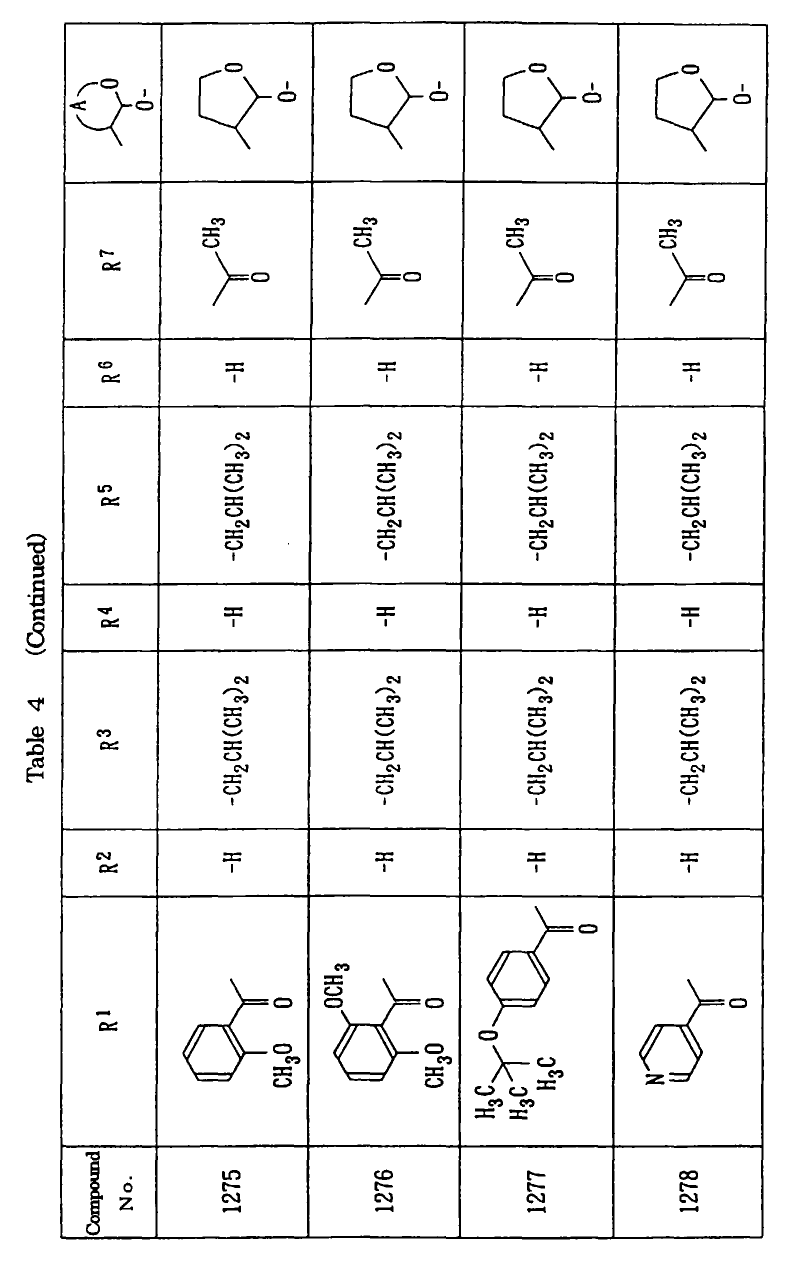

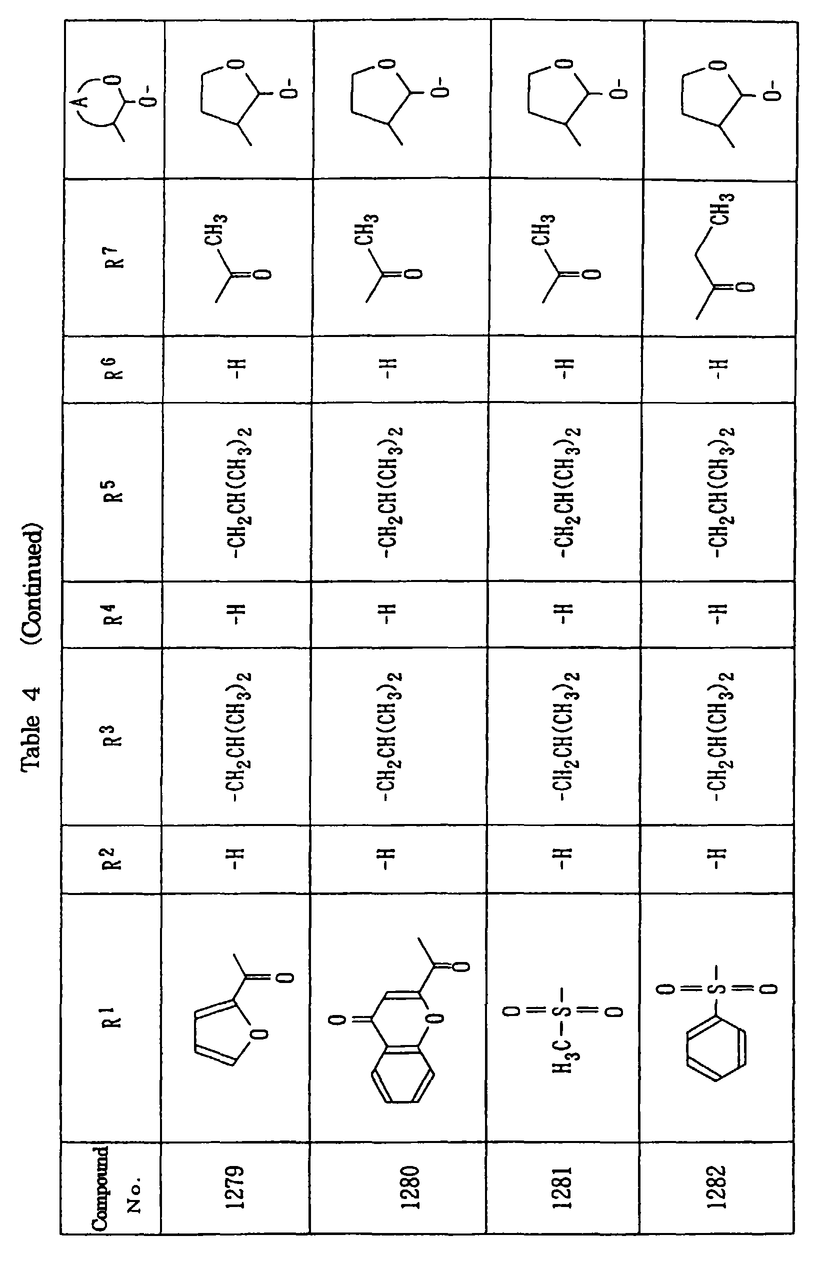

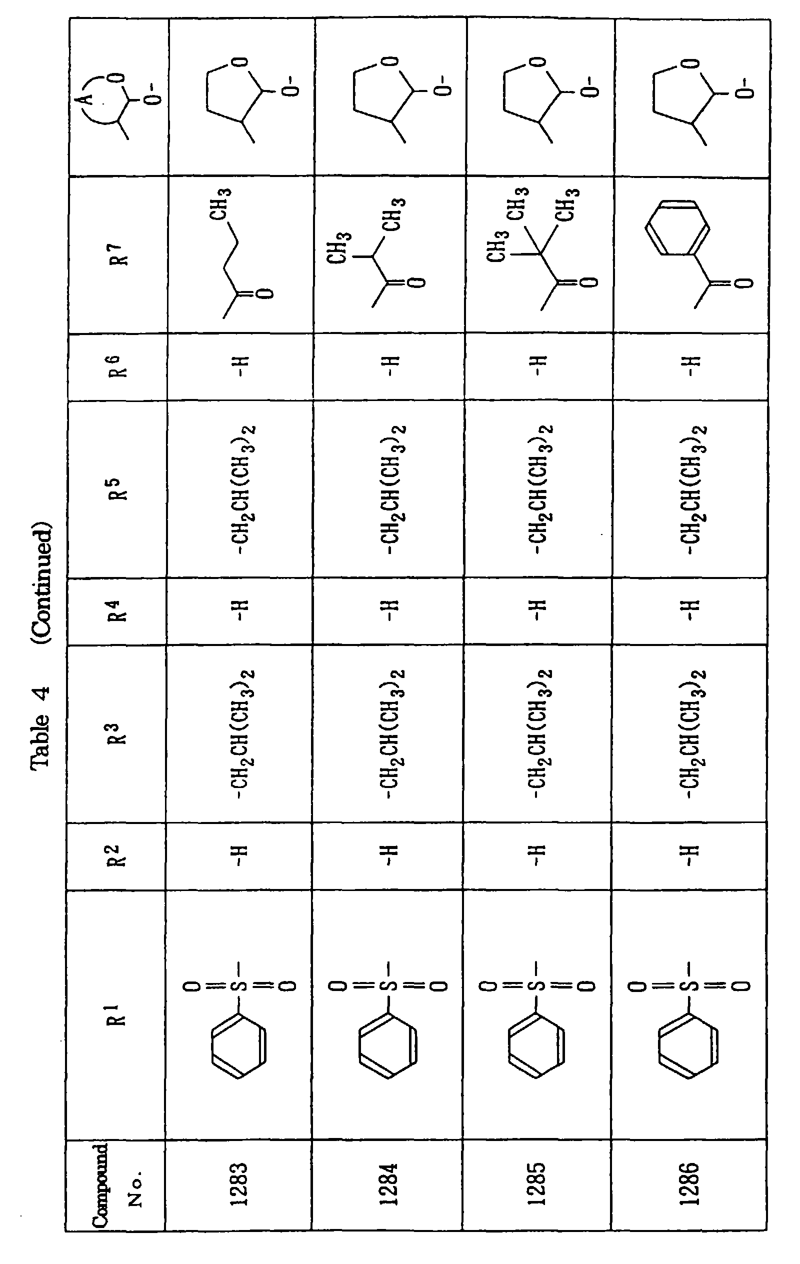

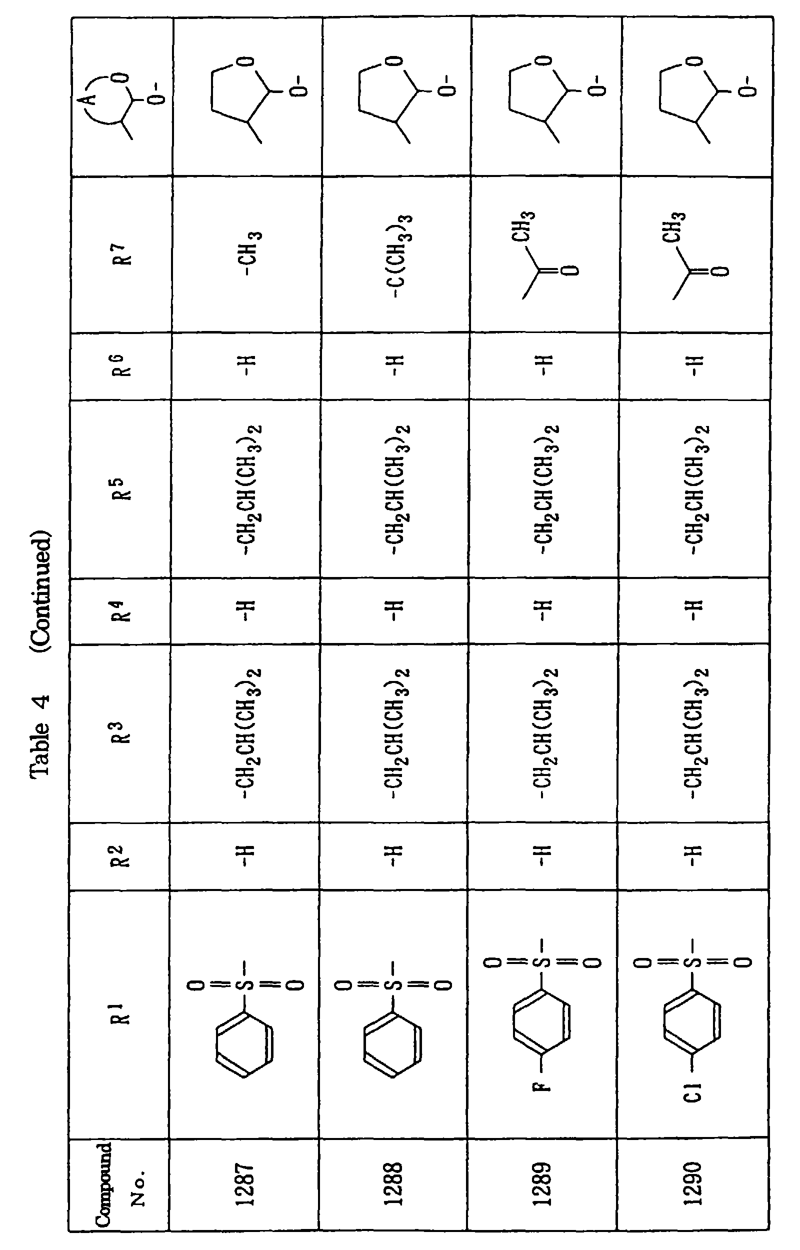

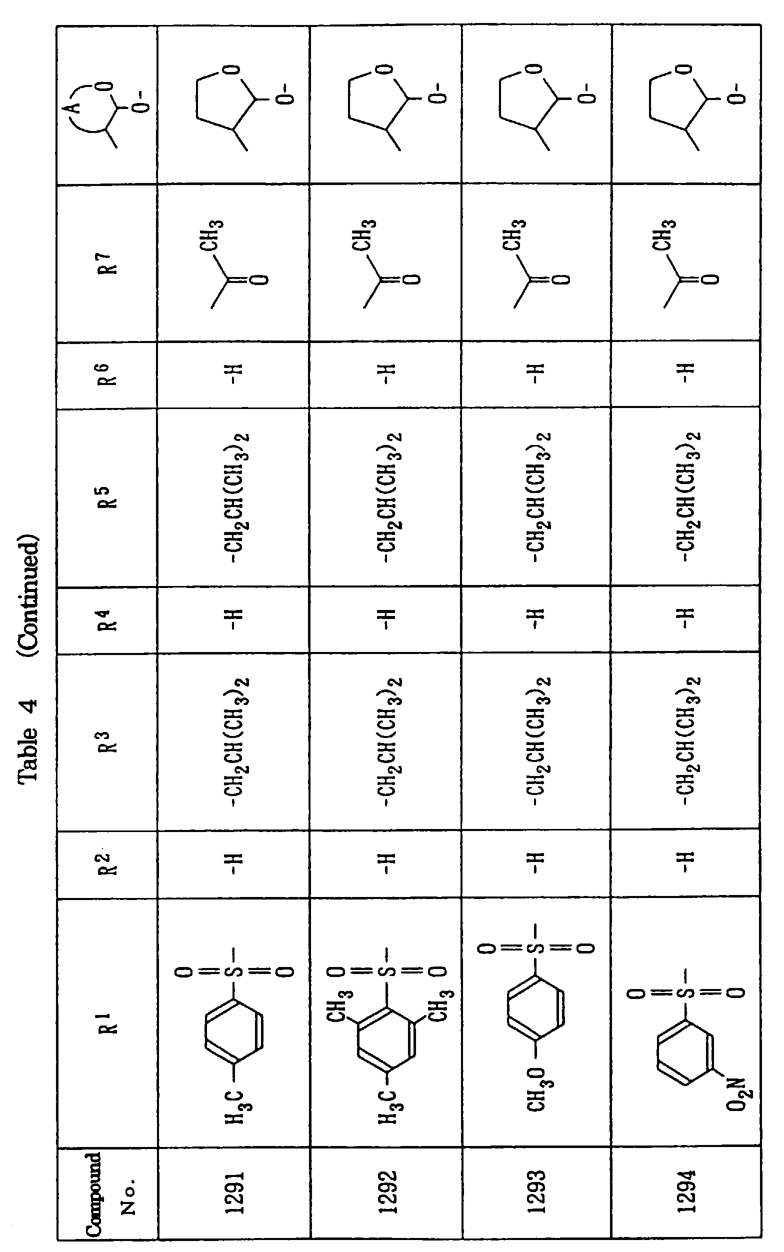

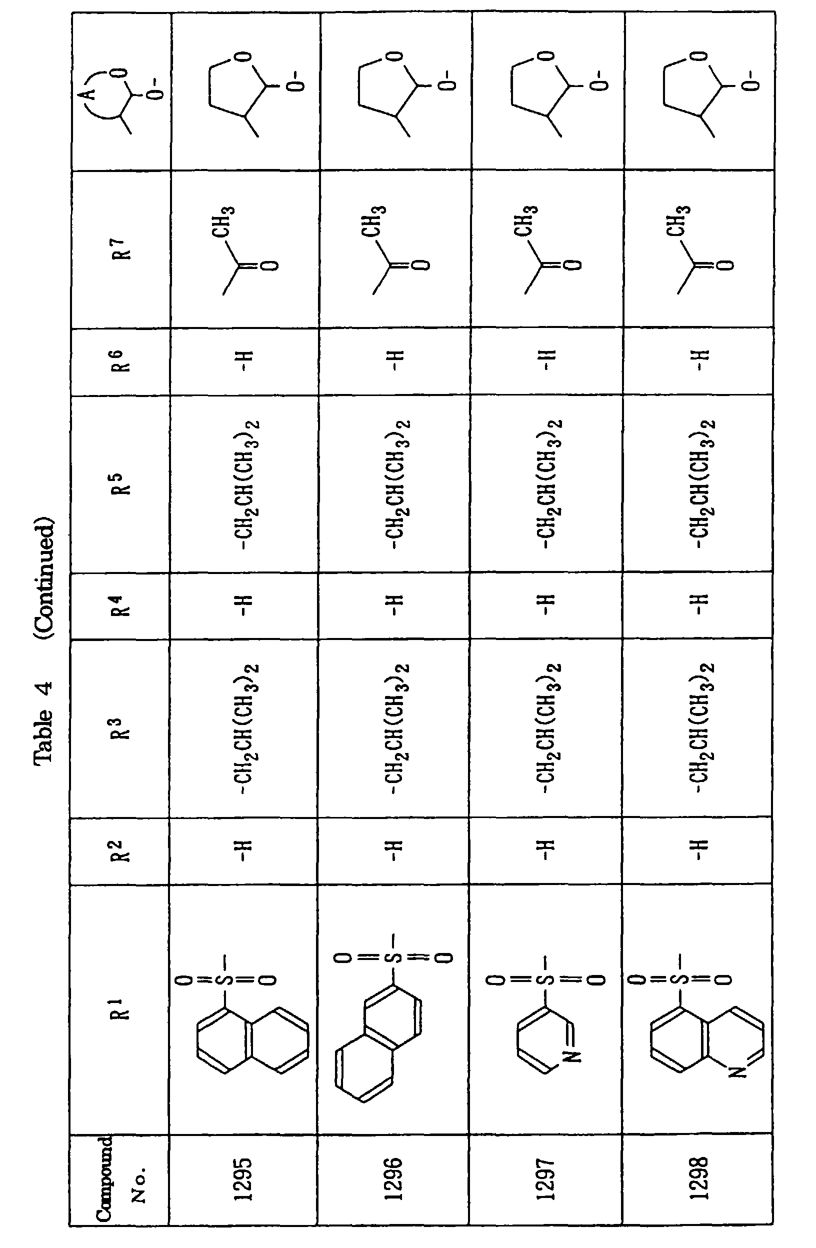

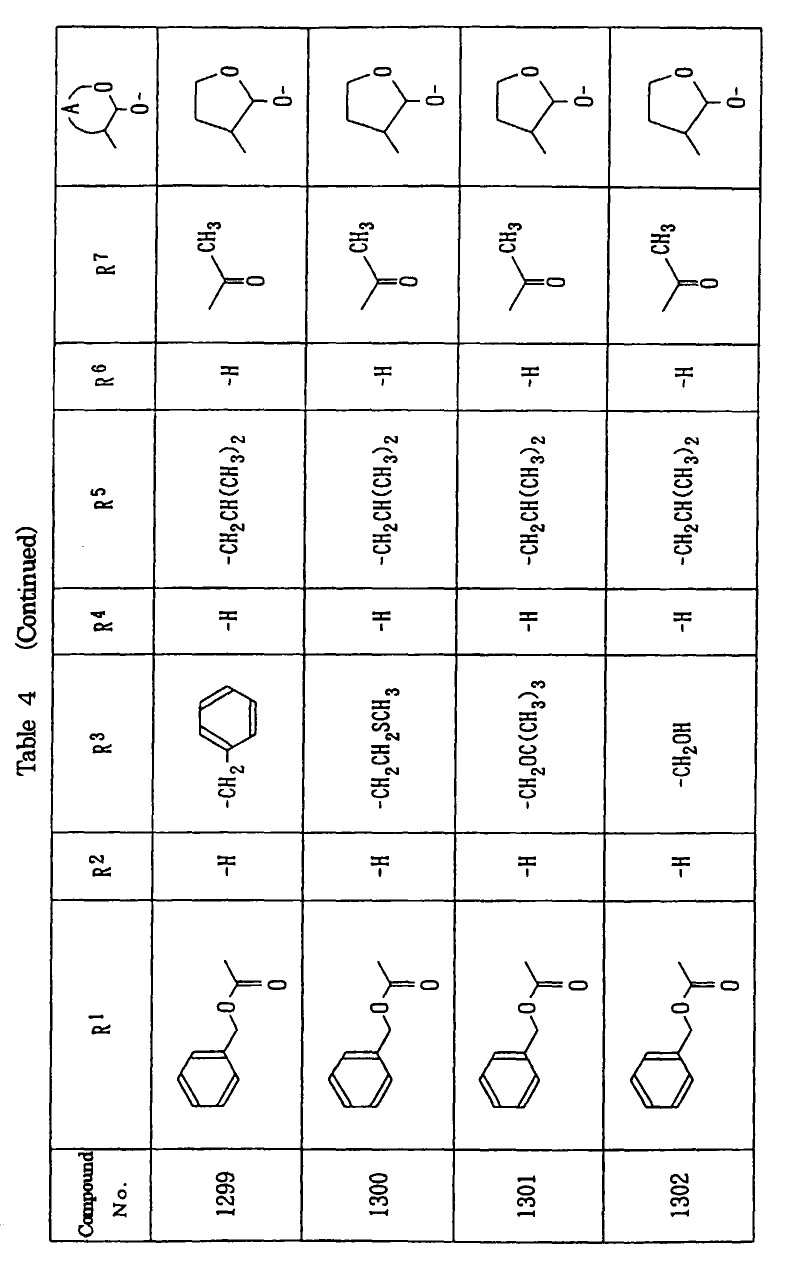

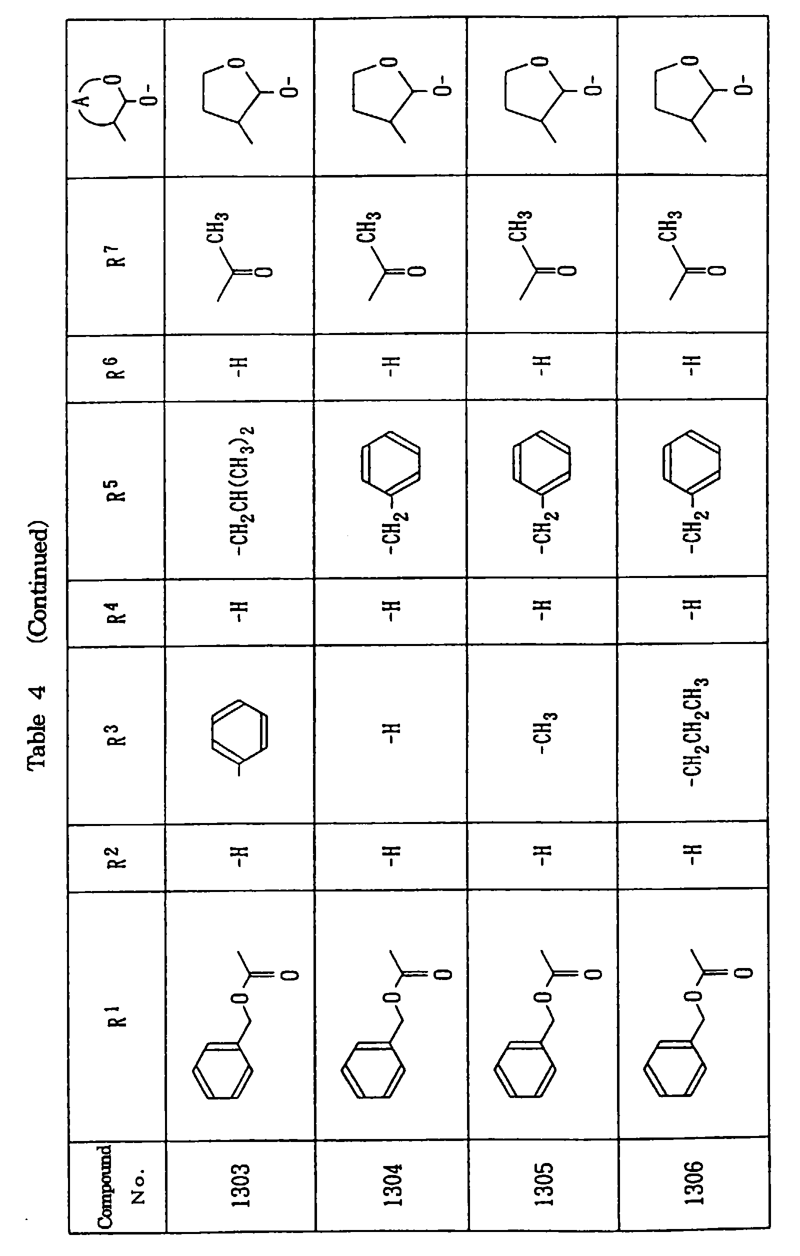

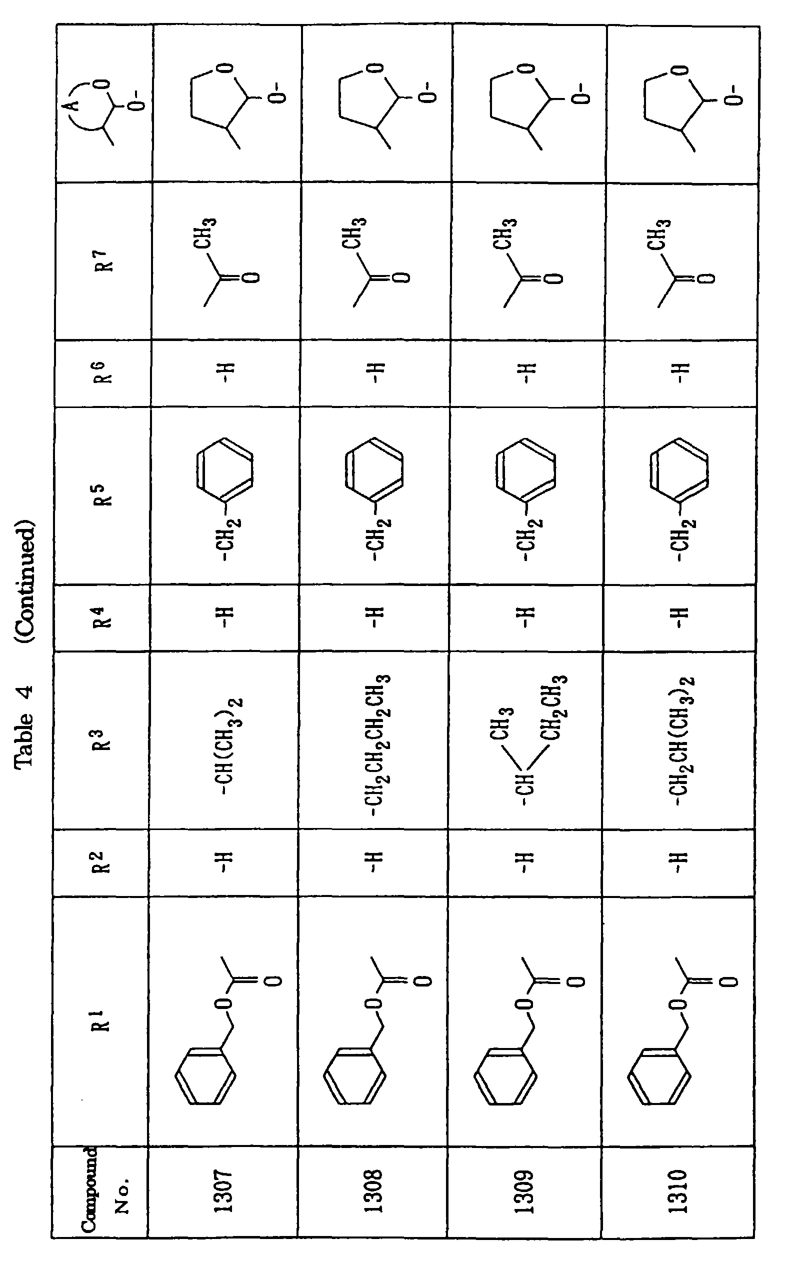

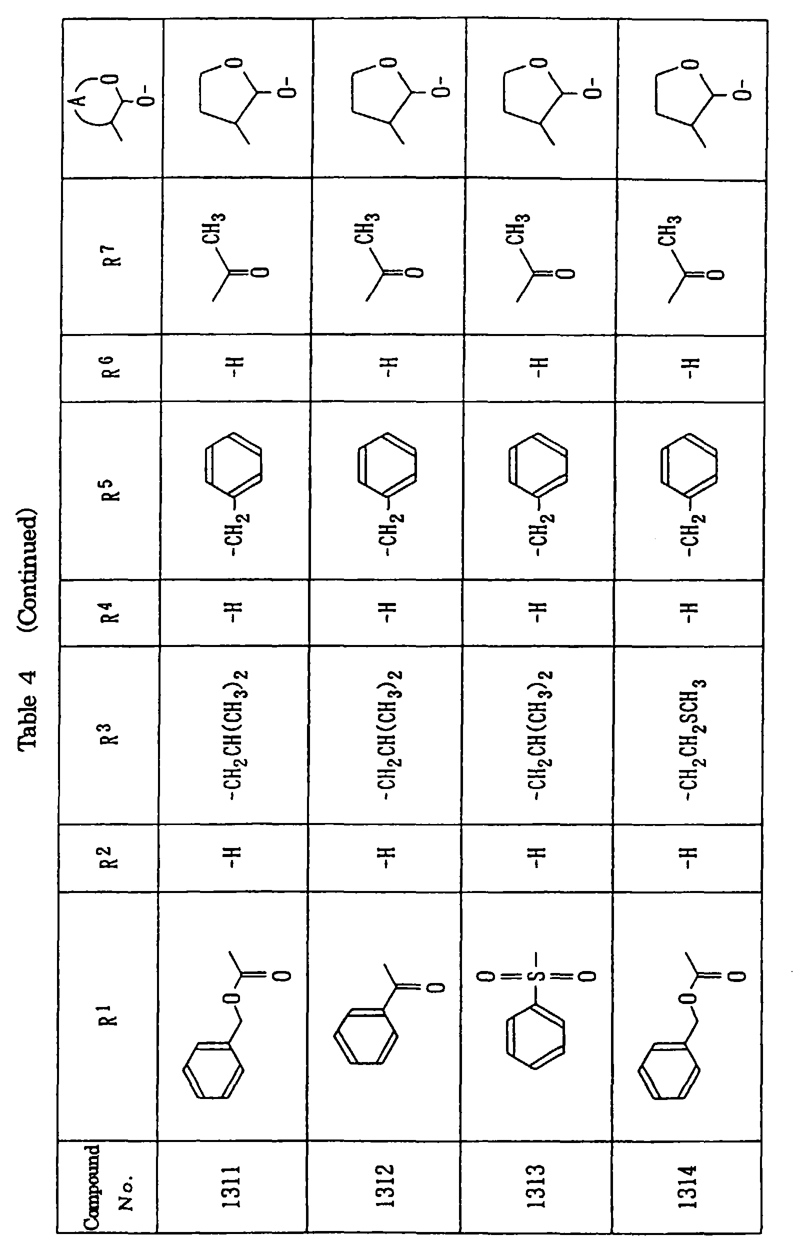

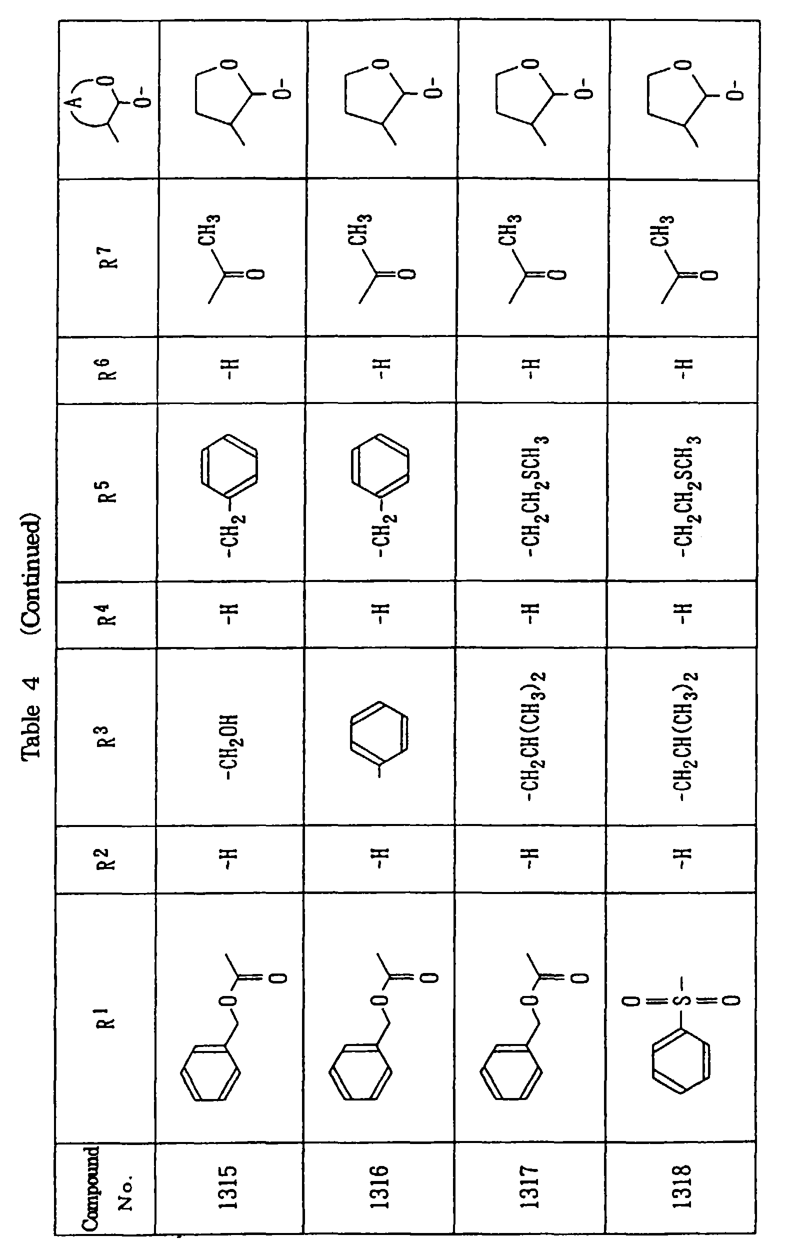

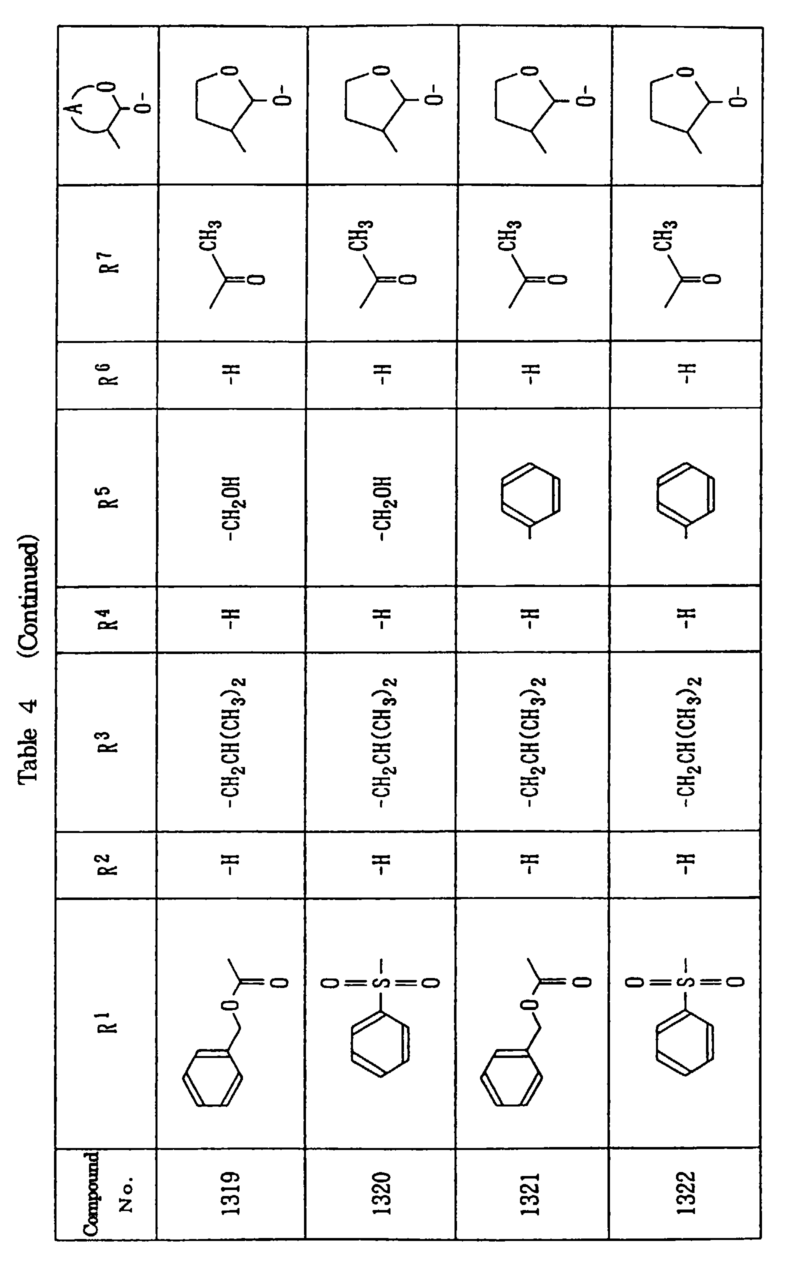

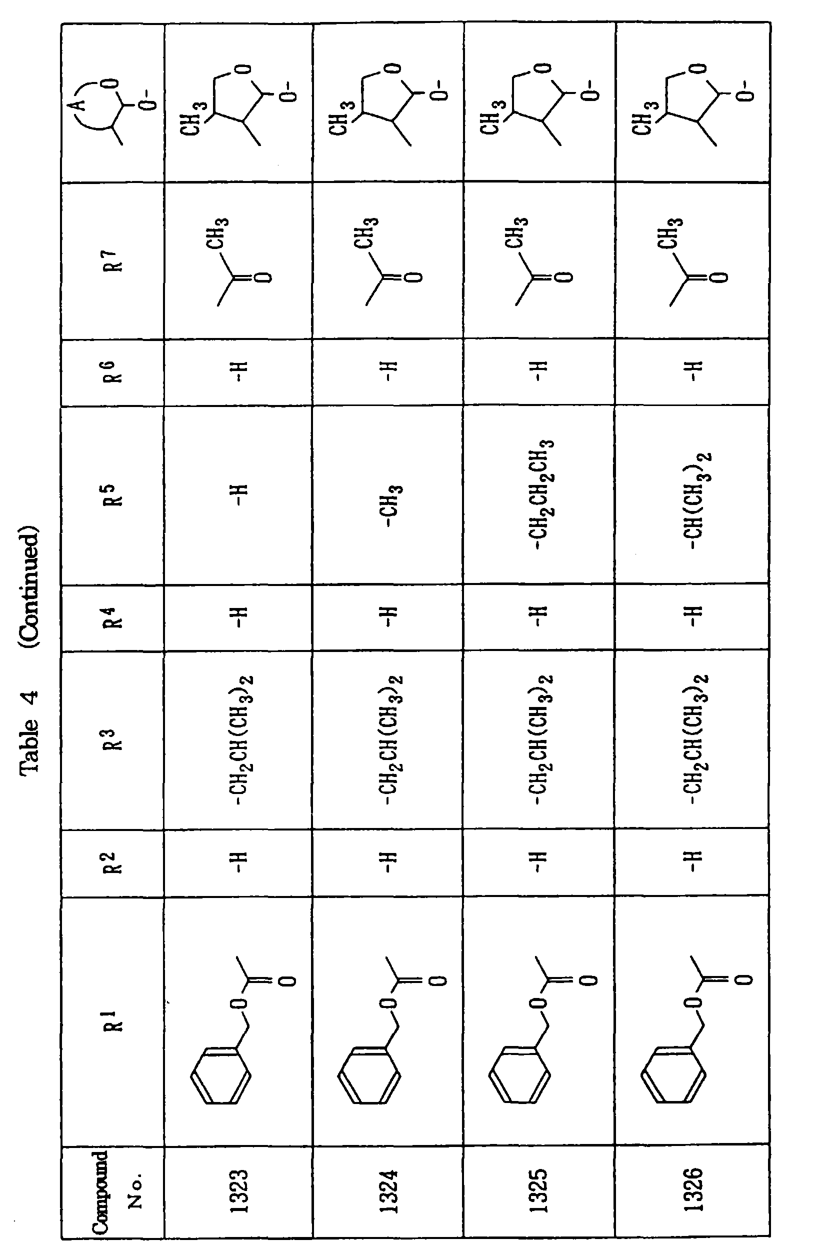

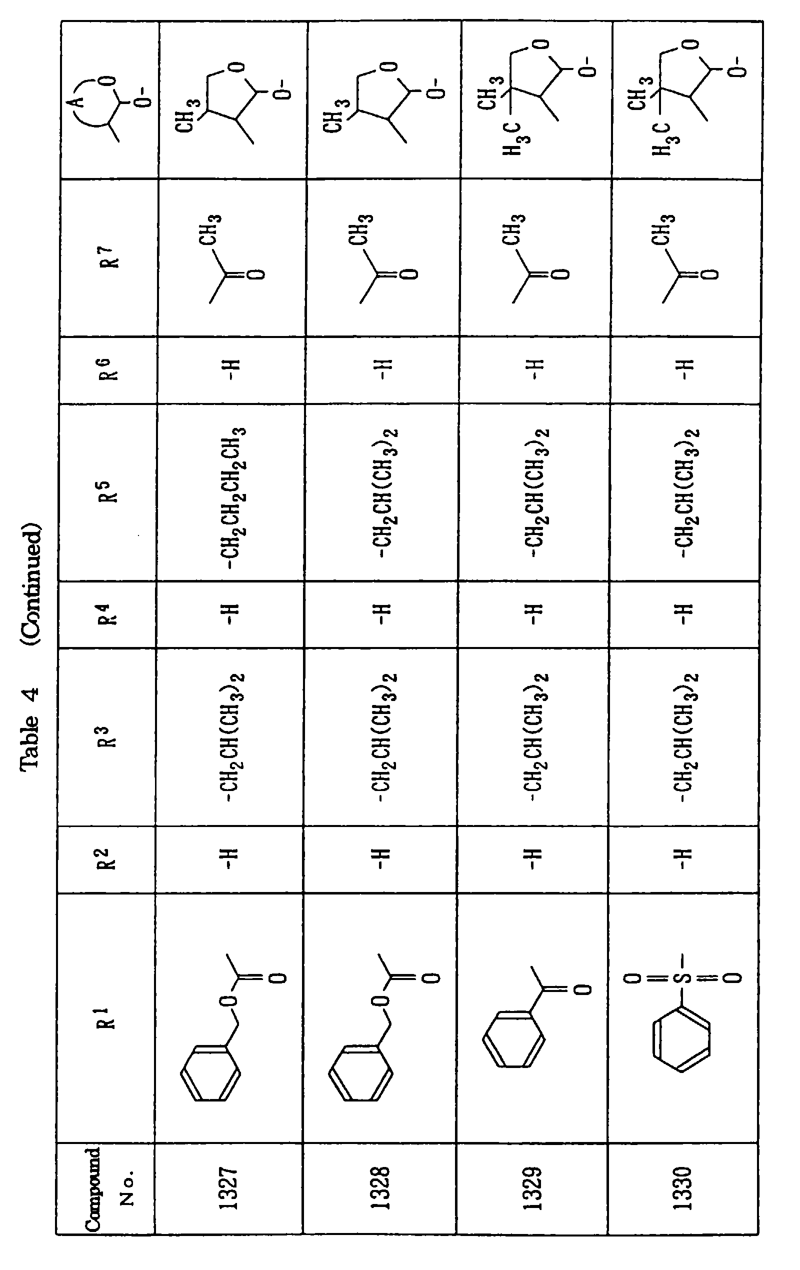

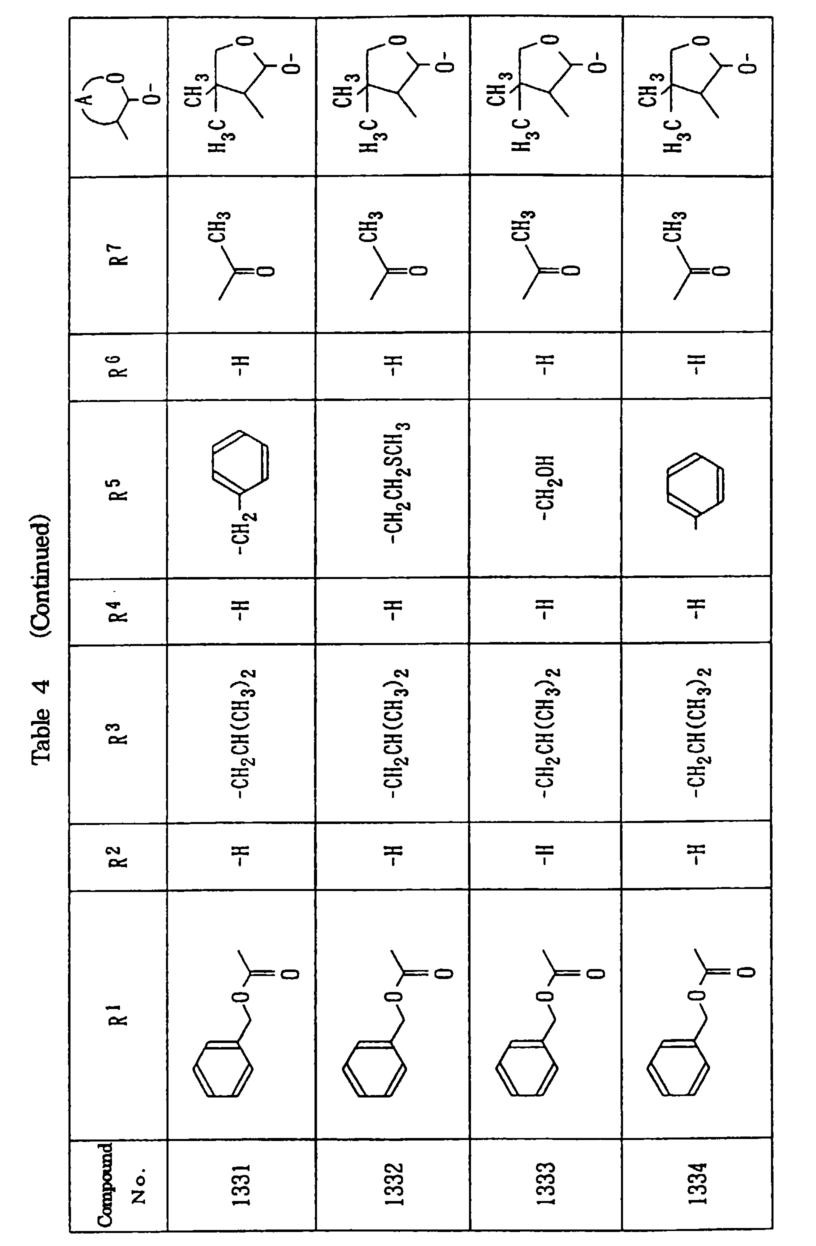

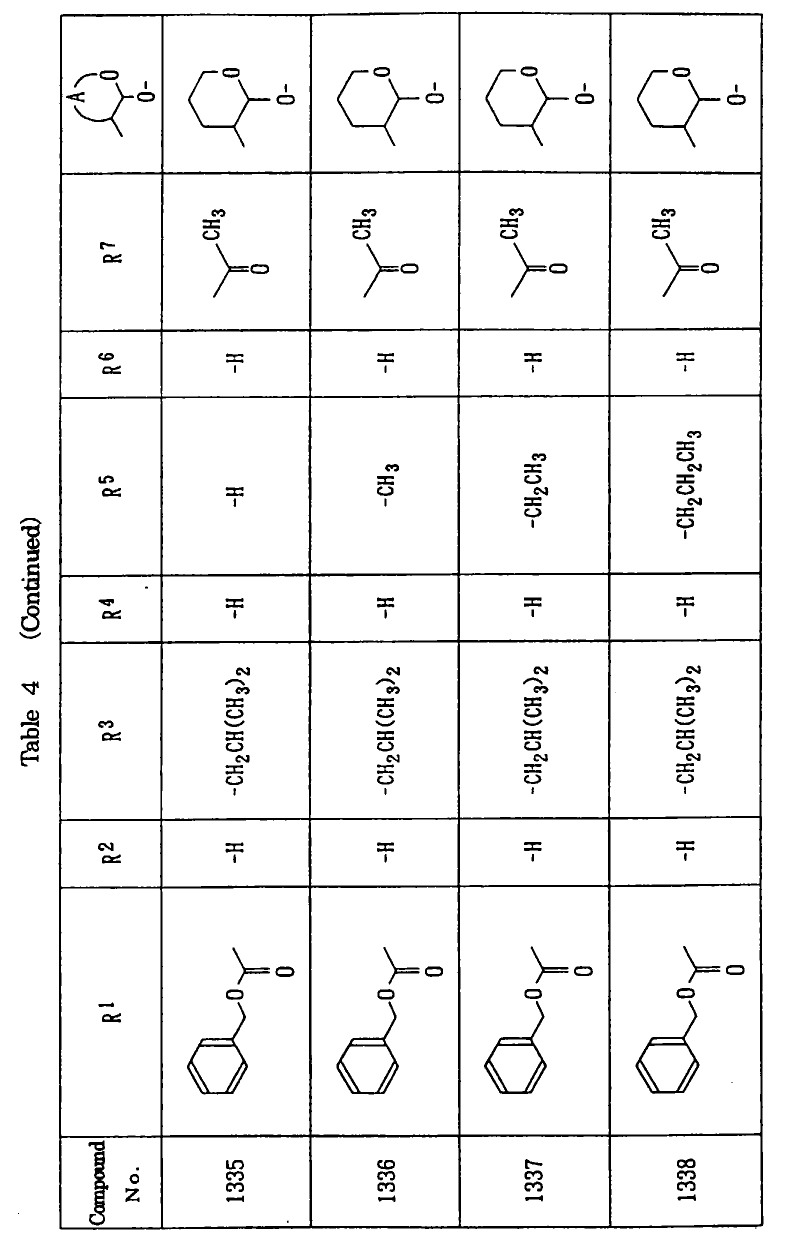

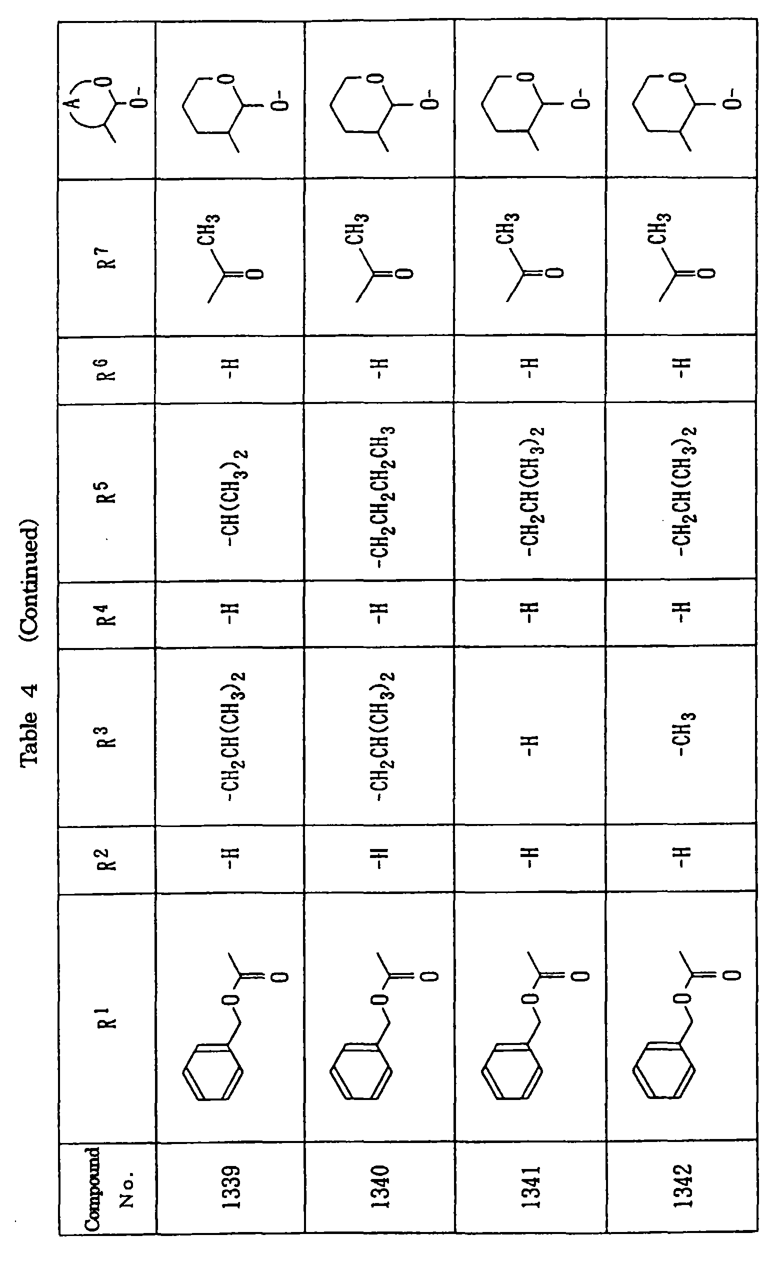

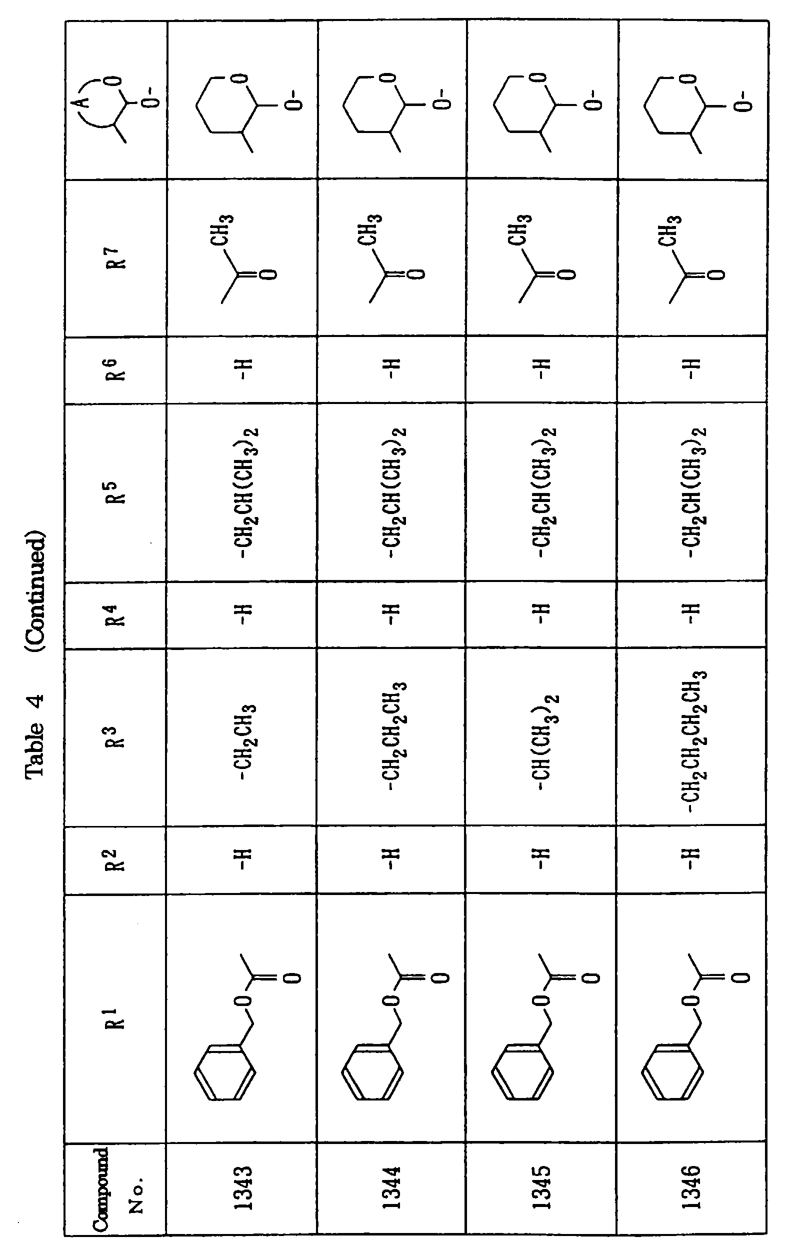

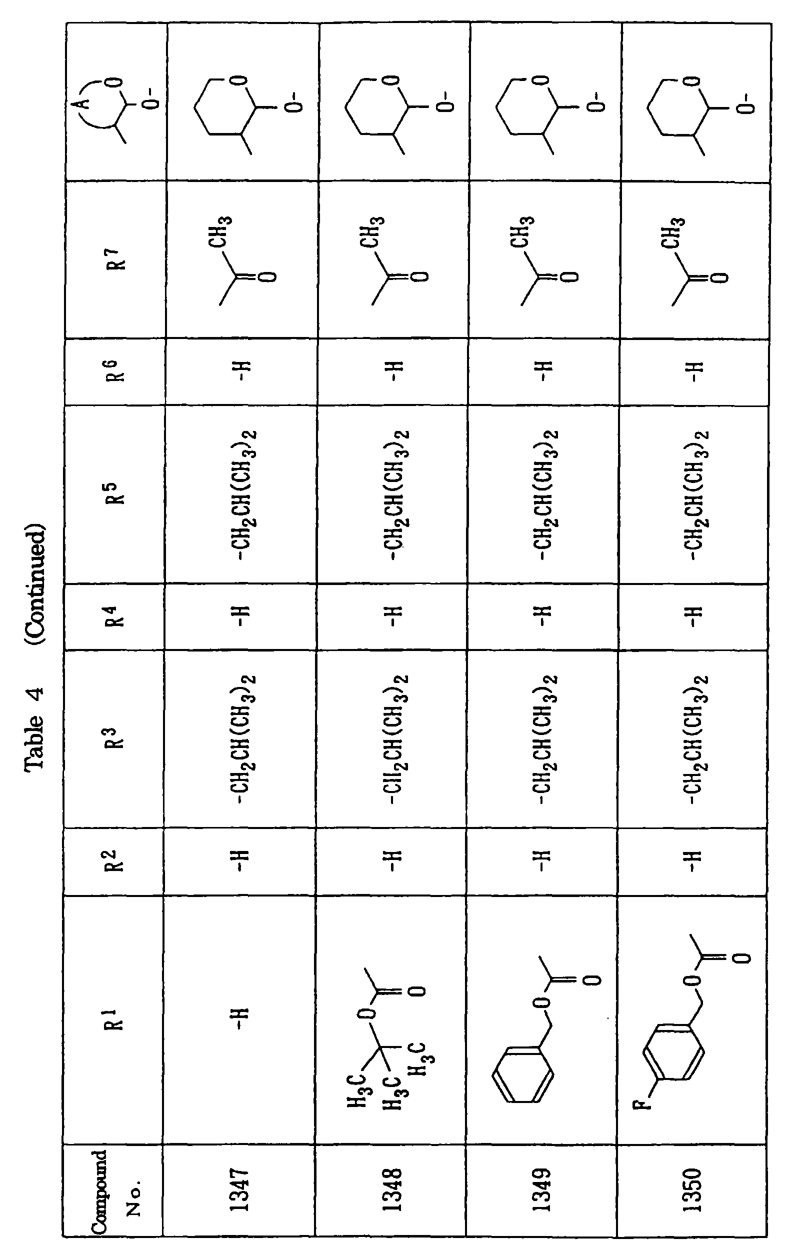

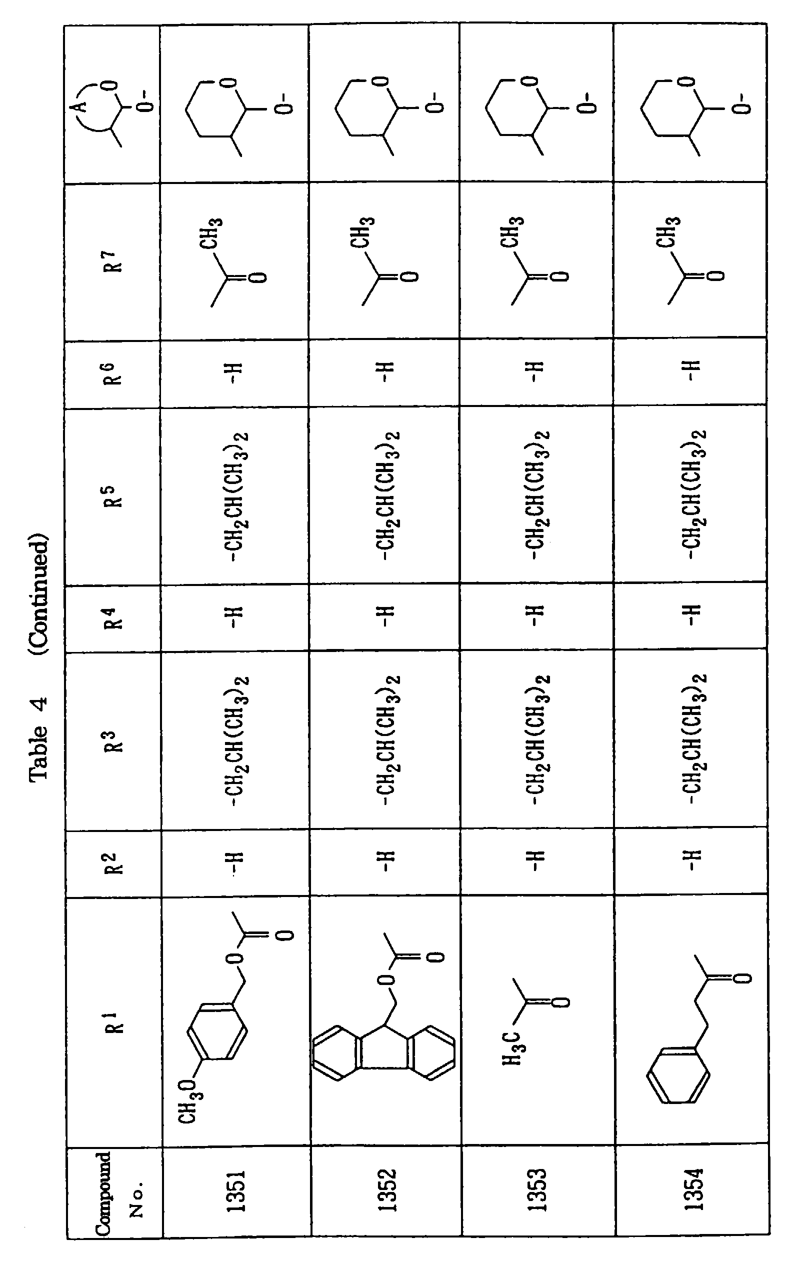

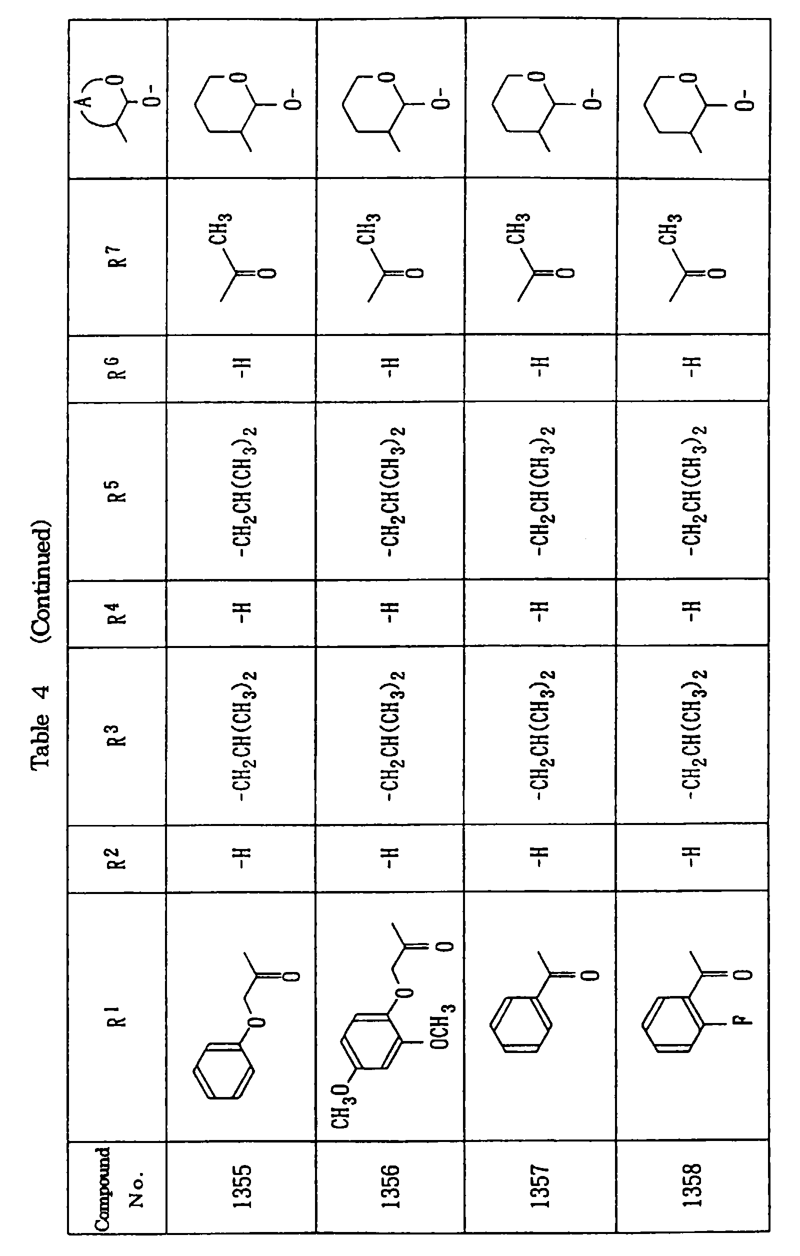

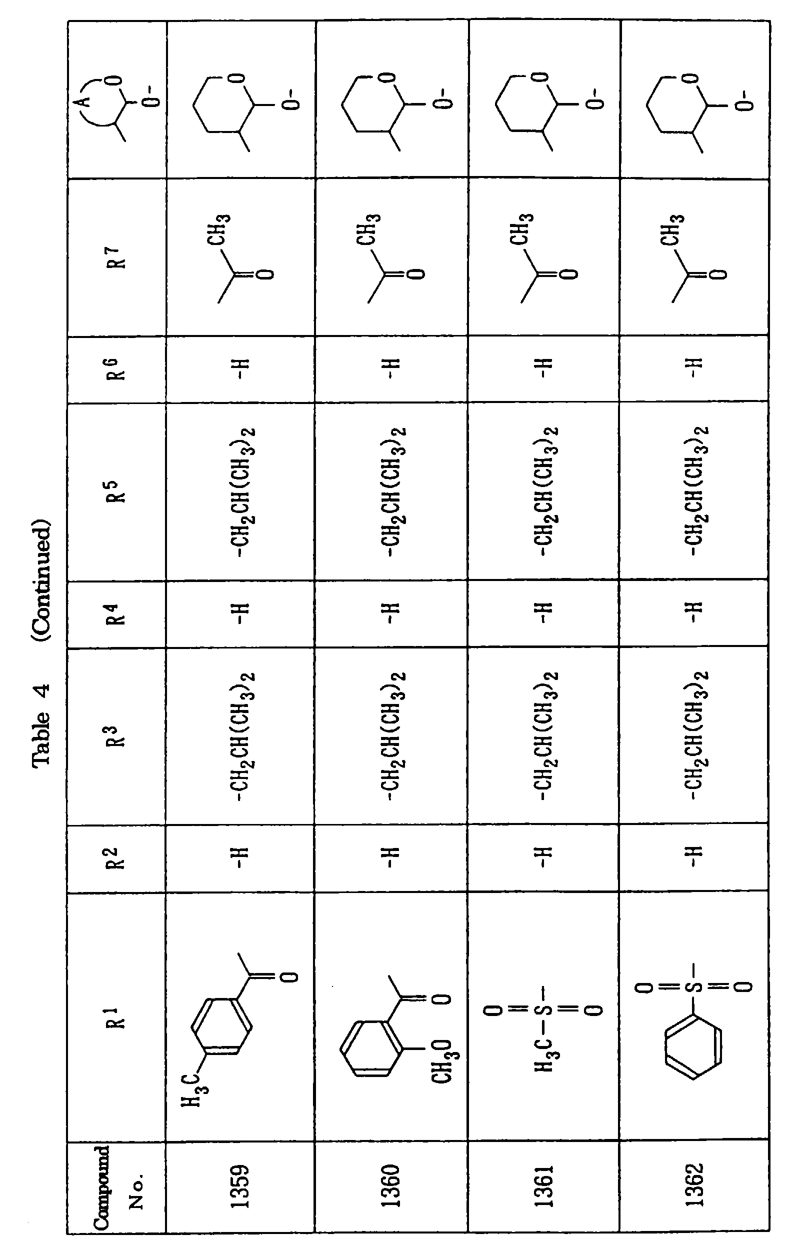

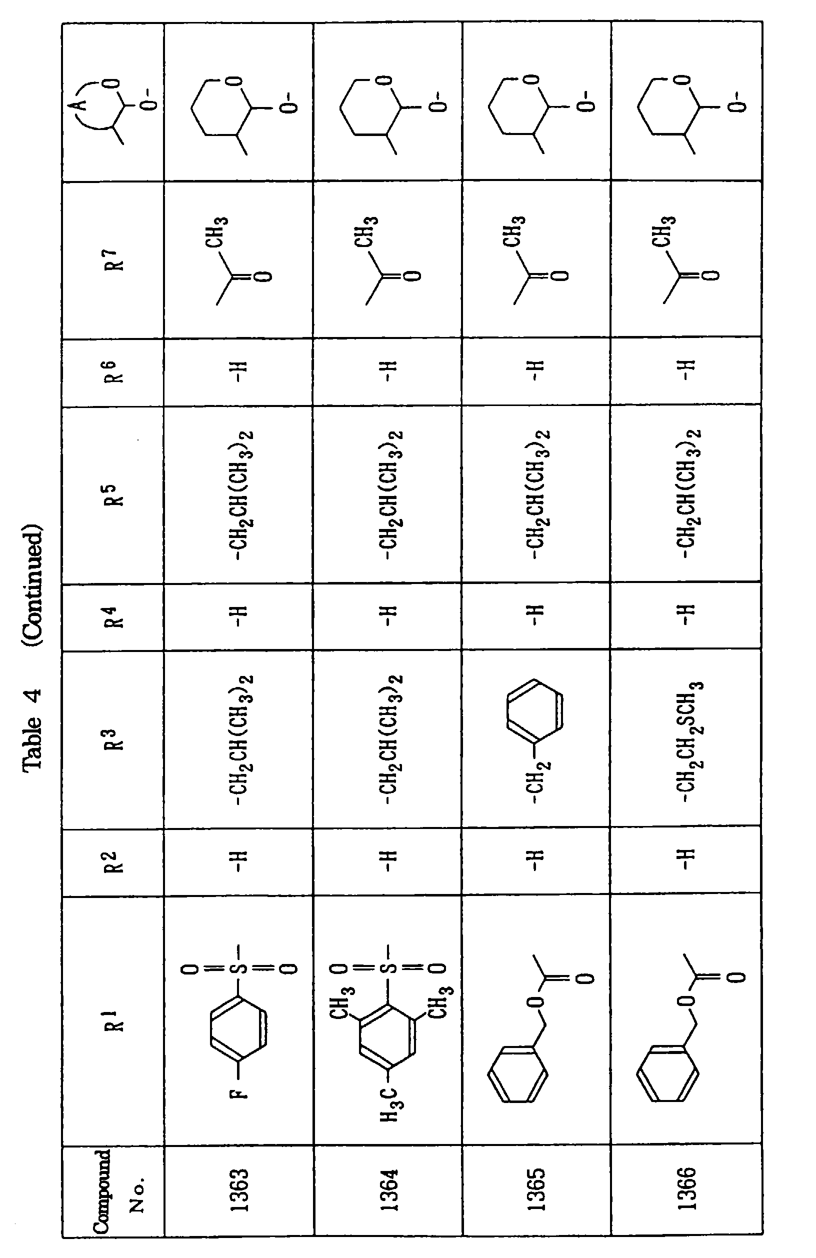

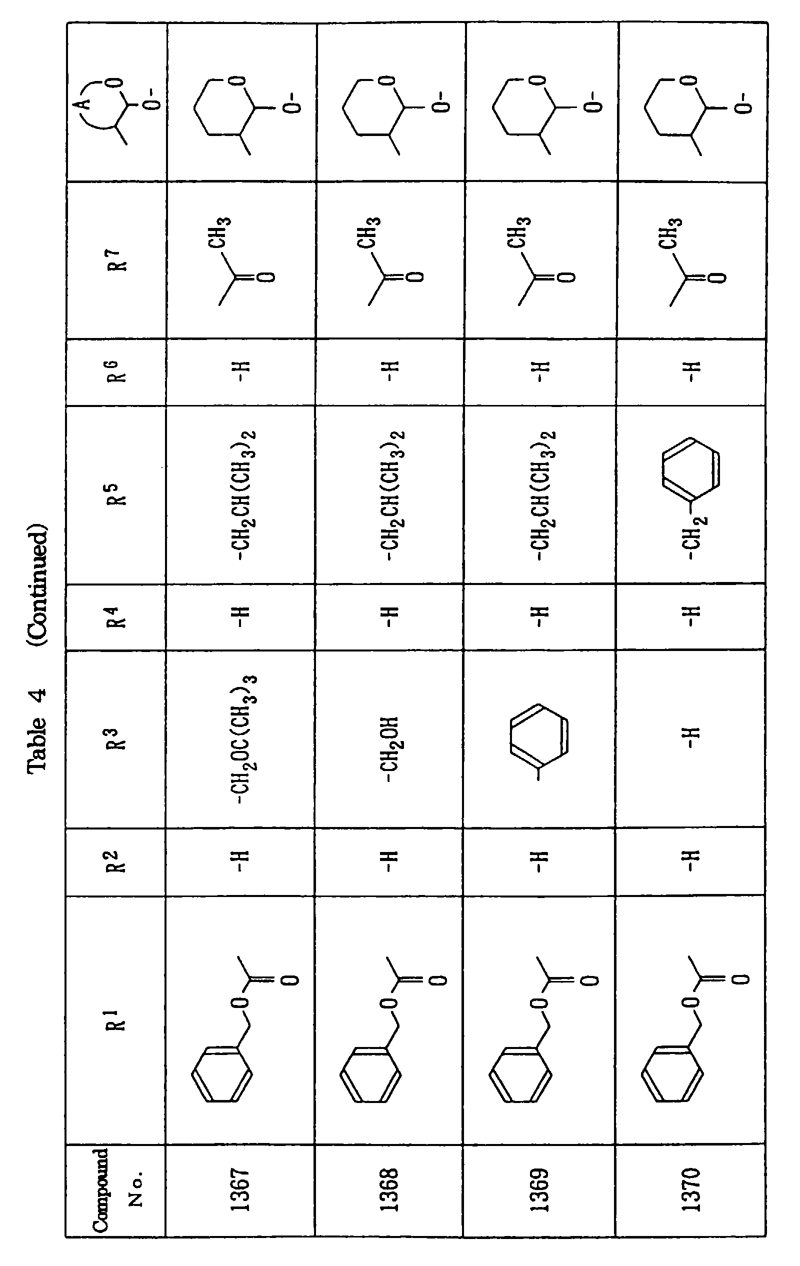

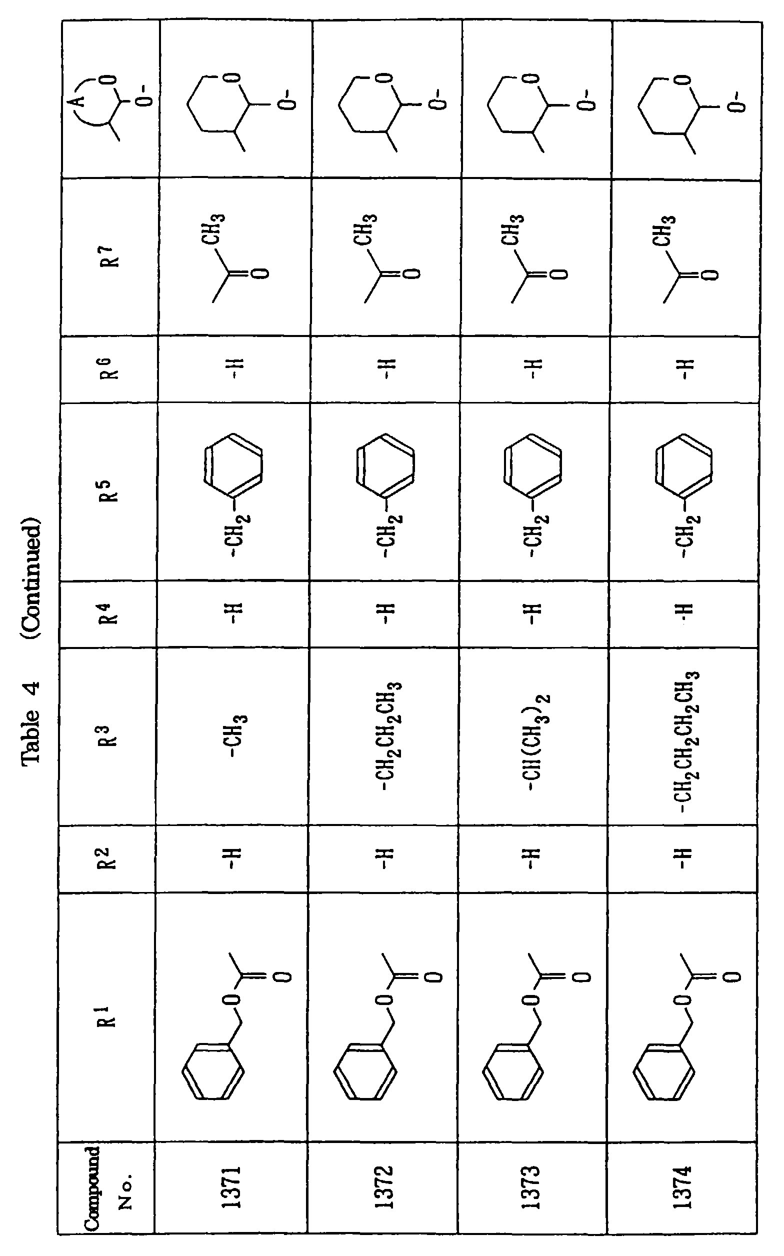

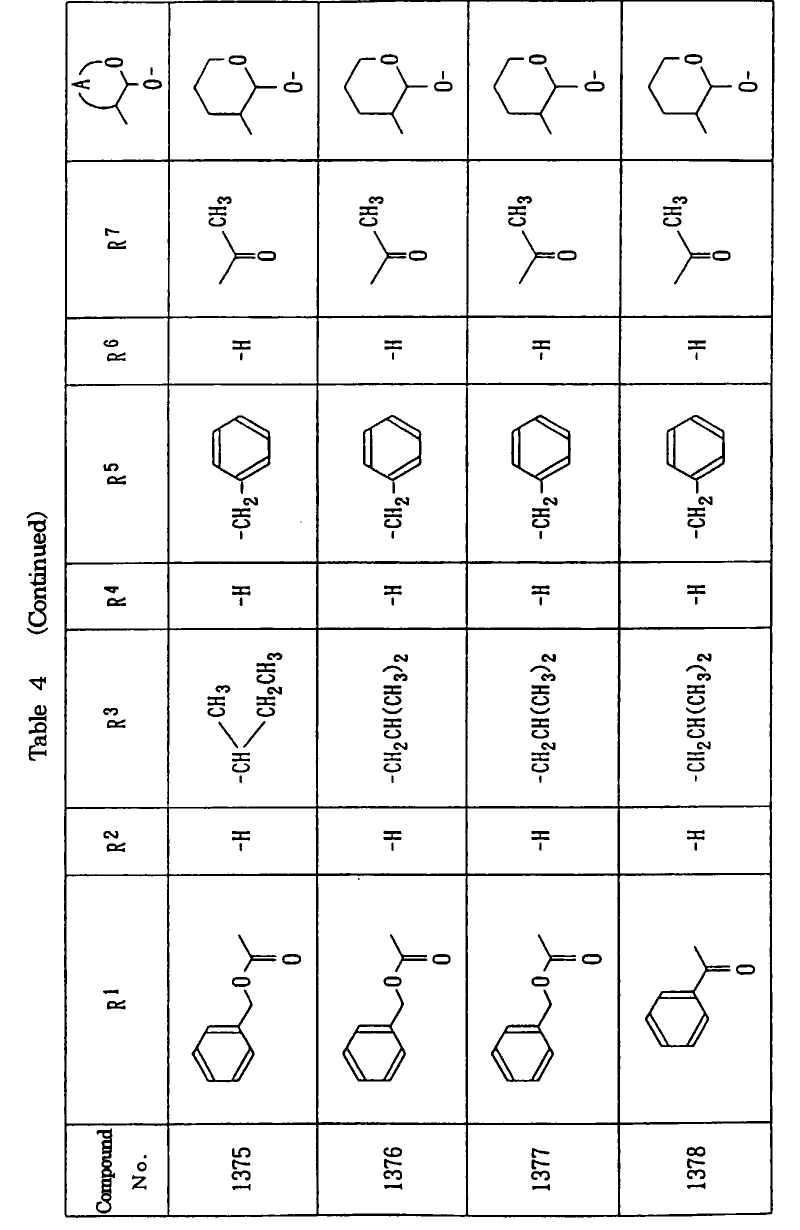

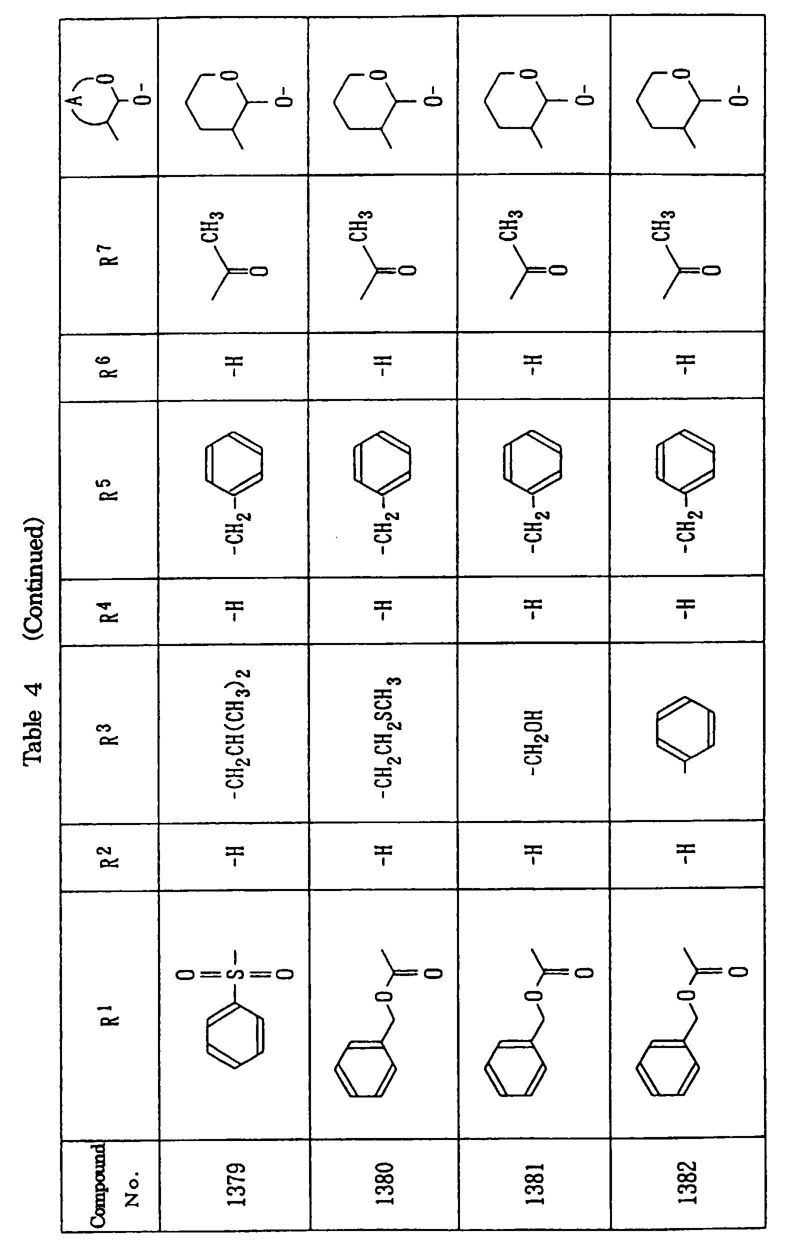

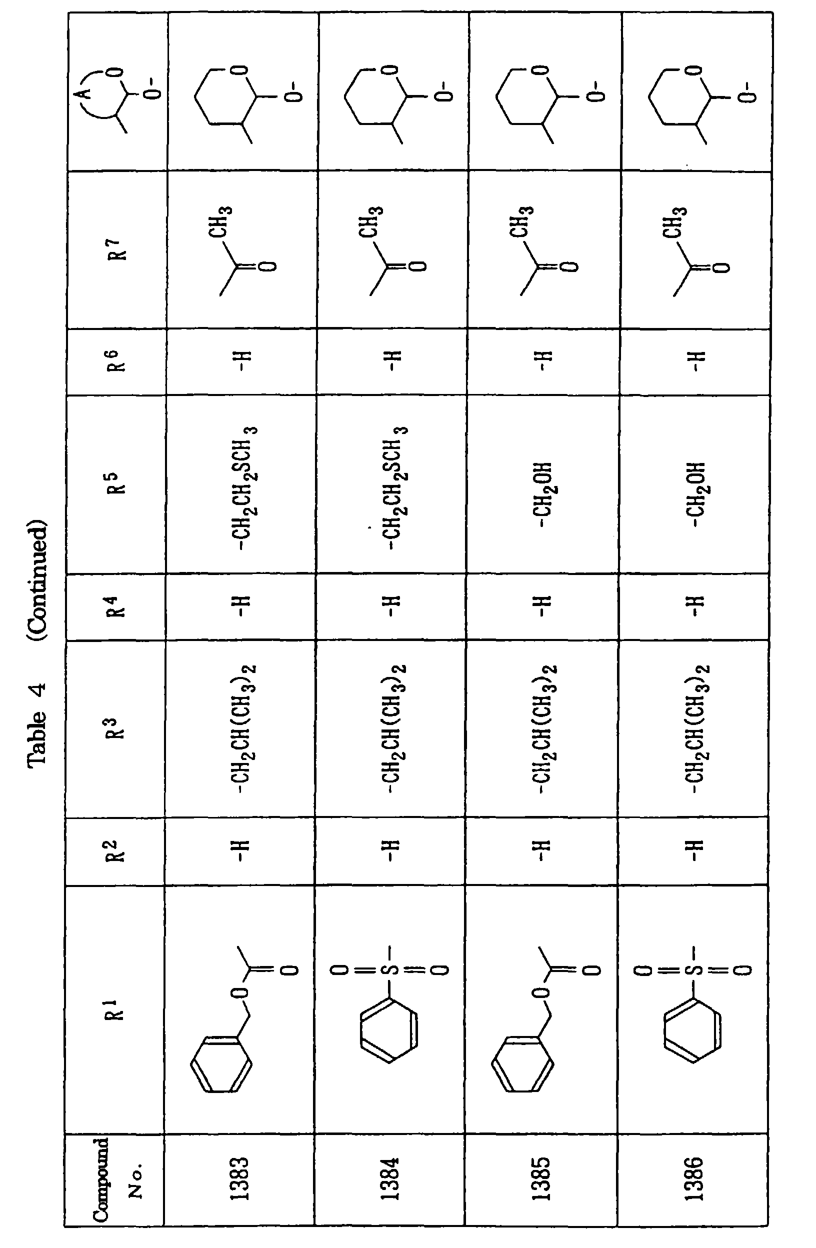

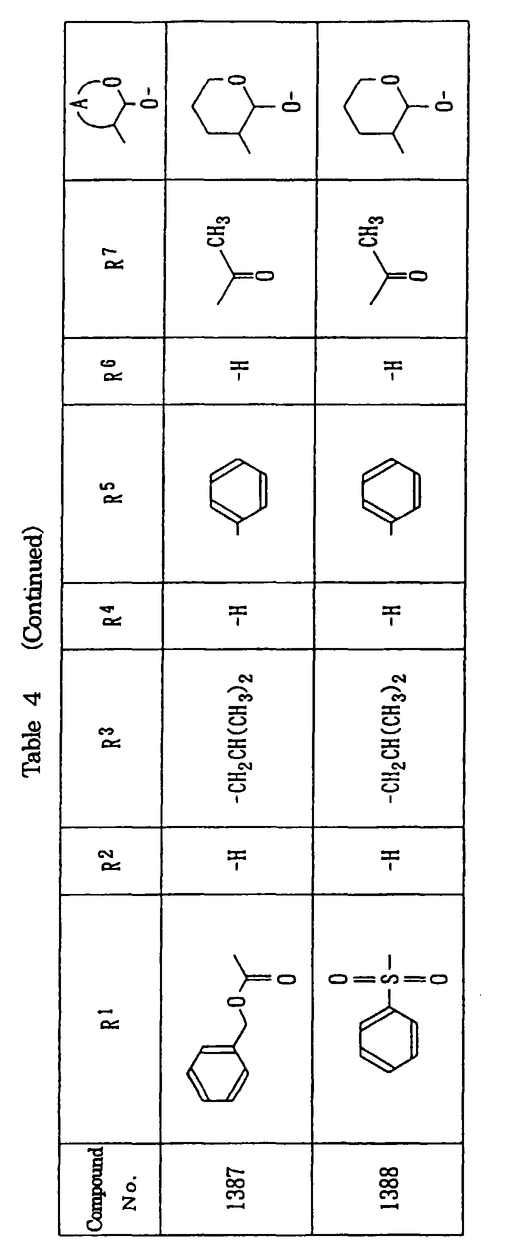

Method for preparation of the compounds of the present invention will be explained. The oxygen-containing heterocyclic derivatives represented by the above formula (I) can be prepared, for example, according to the method described below.

### Preparation method 1: Preparation of the compounds wherein R⁷ is a hydrogen atom

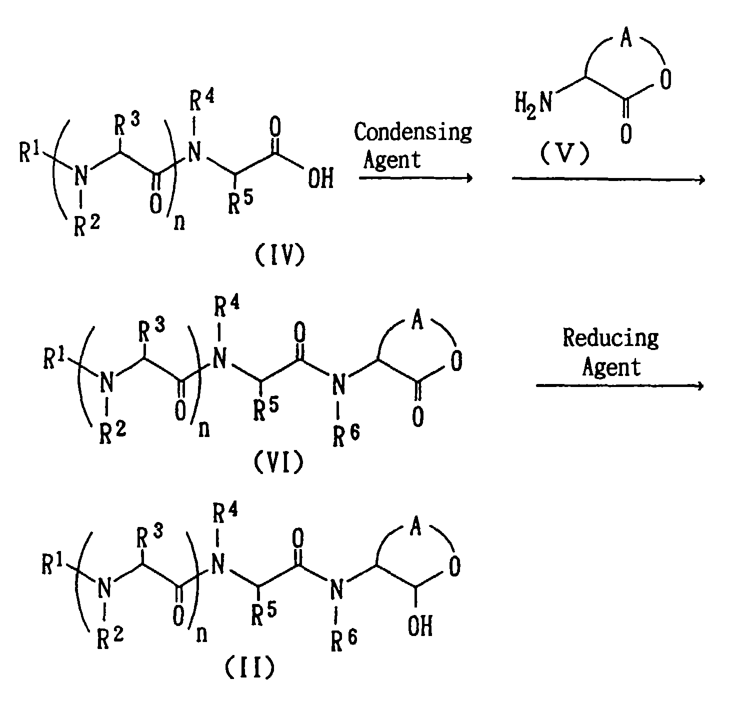

In the above formulas, R¹, R², R³, R⁴, R⁵, R⁶, A, and n have the same meanings as those defined above.

An amino acid derivative represented by the formula (IV) is reacted with a condensing agent such as dicyclohexylcarbodiimide, diphenylphosphoryl azide, carbonyldiimidazole, oxalyl chloride, isobutyl chloroformate, or thionyl chloride optionally in the presence of a base such as triethylamine or pyridine to activate the carboxylic acid. A compound represented by the above formula (VI) can be obtained by reacting the above-obtained product with a lactone derivative represented by the above formula (V). A solvent used for this condensation reaction may be appropriately chosen so as to be suitable for a condensing agent used, and reaction conditions or other may also be applied so as to be suitable for a condensing agent used. The oxygen-containing heterocyclic derivative represented by the formula (II) can be obtained by treating the above-obtained compound of the formula (VI) with a reducing agent such as diisobutyl aluminium hydride or sodium borohydride/cerium chloride.

### Preparation method 2: Preparation of the compounds wherein R⁷ is R⁹-CO-

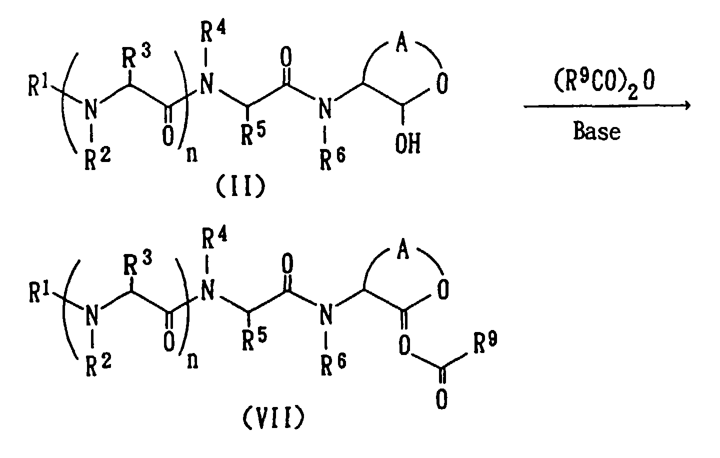

In the above formulas, R¹, R², R³, R⁴, R⁵, R⁶, R⁹, A, and n have the same meanings as those defined above.

The oxygen-containing heterocyclic derivative represented by the above formula (VII) can be obtained by dissolving the oxygen-containing heterocyclic derivative produced by Preparation method 1 in an organic solvent such as methylene chloride, 1,2-dichloroethane, dimethylformamide, N-methylpyrrolidone, tetrahydrofuran, ethyl acetate, acetonitrile, or toluene, and then the solution is allowed to react with an acid anhydride represented by the formula of (R⁹CO)₂O in the presence of a base such as pyridine, triethylamine, or 4-dimethylaminopyridine. This reaction can also be carried out without a solvent.

### Preparation method 3: Preparation of the compounds wherein R⁷ is a C₁-C₅ alkyl group

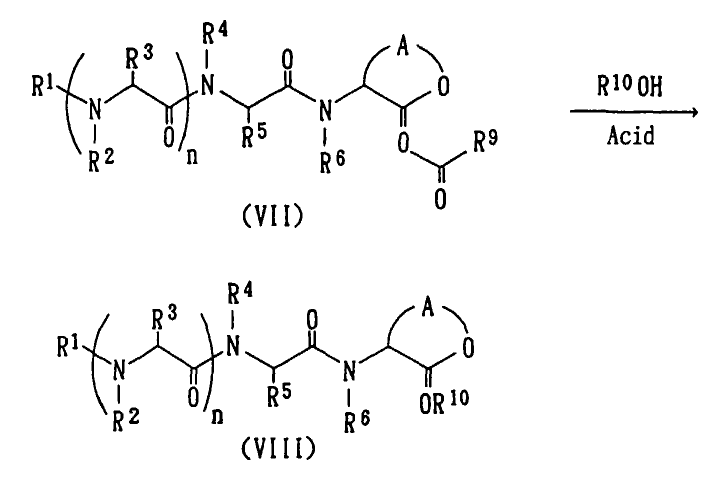

In the above formulas, R¹, R², R³, R⁴, R⁵, R⁶, R⁹, A, and n have the same meaning as those defined above and R¹⁰ represents a C₁-C₅ alkyl group.

The compound represented by the above formula (VIII) can be obtained by dissolving the compound of the formula (VII) obtained in Preparation method 2 in an alcohol compound represented by the formula R¹⁰OH, and then adding a catalytic amount of an acid such as hydrochloric acid or sulfuric acid and stirring the mixture.

In the above-mentioned series of reaction steps, some reactions may require protection and deprotection of one or more functional groups. For that purpose, any protective group suitable for the functional group may be chosen, and any known method described in literatures may be applied as manufacturing procedures.

Among the oxygen-containing heterocyclic derivatives of the present invention obtained as described above, the compounds of the formula (II) wherein R⁷ is hydrogen atom have potent inhibitory activity against a cysteine protease. The compounds of formula (VIII) having a C₁-C₅ alkyl group as R⁷ and the compounds of formula (VII) having R⁹-CO- (R⁹ represents a C₁-C₁₀ alkyl group or a C₆-C₁₂ aryl group which may optionally be substituted) as R⁷ can be used as pro-drugs of the oxygen-containing heterocyclic derivatives (II) that have potent inhibitory activity against a cysteine protease. More specifically, when the compound of formula (VII) or (VIII) is orally administered, the compound is absorbed from the intestinal tract or other, and then the oxygen-containing heterocyclic derivative of the formula (II) as an active form is rapidly released by the functions of enzymes and other in a living body.

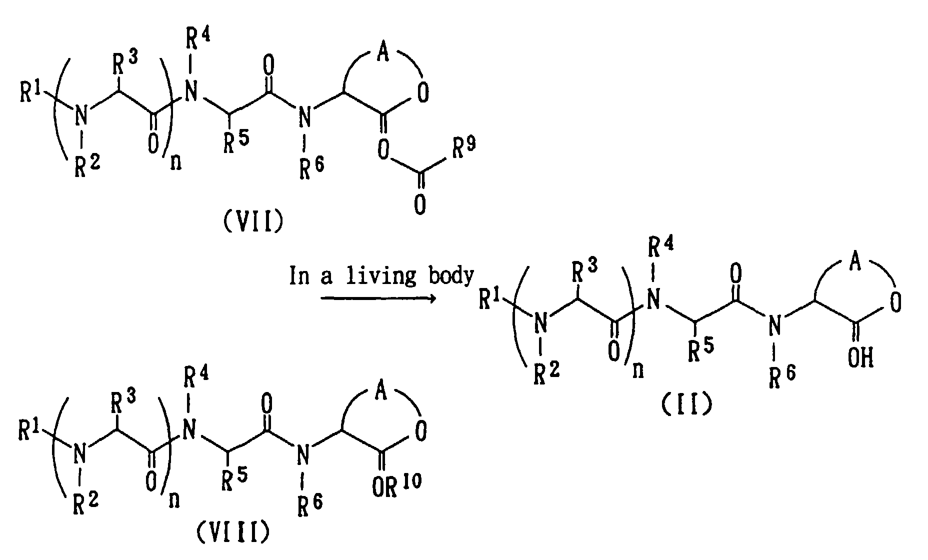

When the compound of the present invention is clinically used, a ratio of the therapeutically useful ingredient based on one or more carrier components may vary in a range of 1-90% by weight. For example, the compounds of the present invention can be orally administered as formulations in the form of granules, fine granules, powders, hard capsules, soft capsules, syrups, emulsions, suspensions, liquid preparations or the like, or alternatively, administered intravenously, intramuscularly, or subcutaneously as injections. They may be used as suppositories. They may also be formulated as powders for injection and used as injections prepared before use. Organic or inorganic and solid or liquid carriers or diluents for the preparation of formulations suitable for oral, intestinal, or parenteral administration may be used for the preparation of the medicament of the present invention. For example, as excipients used for the preparation of solid formulations, lactose, sucrose, starch, talc, cellulose, dextrin, kaolin, calcium carbonate or other may be used. Liquid formulations for oral administration such as emulsions, syrups, suspensions, solutions or other may contain a conventional inert diluent such as water or vegetable oil. These pharmaceutical preparations may contain, in addition to the inert diluent, auxiliaries such as moistening agents, suspension aids, sweeteners, aromatics, colorants, or preservatives. The medicament may be formulated as a liquid preparation and filled in capsules made of an absorbable material such as gelatin. As solvents or suspension mediums used for formulations for parenteral administration, i.e., injection, suppositories and other, for example, propylene glycol, polyethylene glycol, benzyl alcohol, ethyl oleate, lecithin and the like may be used. As base materials used for suppositories, for example, cacao butter, emulsified cacao butter, lauric lipid, witepsol and the like may be used. The pharmaceutical preparations may be prepared according to ordinary methods.

Clinical dose may generally be in the range of 0.01-1,000 mg per day, when orally administered, for an adult as the weight of the compound of the present invention . However, it is further preferred that the dose may appropriately be increased or decreased depending the age, conditions, and symptom of a patient. The daily dose of the medicament of the present invention may be administered once a day, or twice o or three times a day with appropriate intervals, or alternatively, administered intermittently.

When the medicament is used as an injection, it is desirable that 0.001-100 mg per day for an adult as the weight of the compound of the present invention is administered continuously or intermittently.

### Brief Description of the Drawings

Figure 1 is a HPLC chart obtained after 5 minutes' incubation of the compound of Example 88 in rat serum.

Figure 2 is a HPLC chart of a sample obtained by dissolving the compound of Example 1 and the compound of Example 88 in the absence of serum.

### Best Mode for Carrying Out the Invention

The present invention will be further detailed by referring to reference examples and examples. However, these reference examples and examples are non-limiting so far that the scope of the present invention falls within the gist of the present invention.

### Reference Example 1: Preparation of (S)-3-((S)-4-methyl-2-phenylsulfonylaminovalerylamino)-2-tetrahydrofuranone

6 ml of thionyl chloride was cooled to -5°C, and the reagent was added with 998 mg of N-phenylsulfonyl-L-leucine. The reaction mixture was stirred at -5°C for 10 minutes, then warmed up to room temperature and stirring was further continued for 3 hours. The reaction mixture was then concentrated under reduced pressure, and the resulting residue was added with 10 ml of toluene and further concentrated to obtain crude N-phenylsulfonyl-L-leucyl chloride as a residue. The resulting crude N-phenylsulfonyl-L-leucyl chloride was dissolved in 20 ml of methylene chloride, and the solution was added with 443 mg of L-homoserinelactone hydrochloride and 0.946 ml of triethylamine under ice cooling. The reaction mixture was stirred for 15 minutes under ice cooling and then stirring was further continued for 1.5 hours at room temperature. After the completion of the reaction, the reaction mixture was added with diluted hydrochloric acid and extracted with methylene chloride. The extract was washed successively with water, saturated aqueous sodium hydrogencarbonate, and saturated brain, dried over magnesium sulfate, and then filtered. The filtrate was concentrated and the resulting residue was added with 10 ml of ethyl acetate and 20 ml of hexane and stirred. Precipitated crystals were collected by filtration to obtain the desired compound (861 mg).
Yield: 76%
Melting point: 183-184°C
IR (KBr, cm⁻¹): 3331, 3256, 1772, 1649.
NMR (CDCl₃, δ): 0.67 (d, J=6.0Hz, 3H), 0.84 (d, J=6.3Hz, 3H), 1.45-1.56 (m, 3H), 2.01 (m, 1H), 2.63 (m, 1H), 4.26 (m, 1H), 4.36 (m, 1H), 4.45 (ddd, J=9.3Hz, 9.3Hz, 1.8Hz, 1H), 5.28 (d, J=8.1 Hz, 1H), 6.67 (d, J=6.0Hz, 1H), 7.52 (m, 2H), 7.60 (m, 1H), 7.88 (dd, J=7.2Hz, 1.5Hz, 2H).

### Example 1: Preparation of (3S)-3-((S)-4-methyl-2-phenylsulfonylaminovalerylamino)-2-tetrahydrofuranol (Compound No. 196 in Table-1)

413 mg of (S)-3-((S)-2-phenylsulfonylamino-4-methylvalerylamino)-2-tetrahydrofuranone obtained in Reference Example 1 was dissolved in 60 ml of methylene chloride and the solution was cooled to -78 °C . 3.81 ml of a solution of diisobutylalminium hydride in toluene (1.01 mol/L) was added to the reaction solution. After three hours, the reaction mixture was added with a saturated aqueous solution of ammonium chloride and ethyl acetate, and then warmed up to room temperature and filtered through celite. The celite was washed thoroughly with ethyl acetate. The filtrate was washed with saturated brain, dried over magnesium sulfate, and then filtered. The filtrate was concentrated, and the resulting residue was purified by silica gel column chromatography (eluent: ethyl acetate containing 30% hexane) to give 191 mg of the desired compound.
Yield: 46%
Melting point: 162°C
IR (KBr, cm⁻¹): 3337, 3260, 1649.
NMR (CDCl₃+DMSO-d₆, δ): 0.72 (d, J=6.6Hz, 2.7H), 0.78 (d, J=6.3Hz, 0.3H), 0.84 (d, J=6.6Hz, 2.7H), 0.86 (d, J=6.3Hz, 0.3H), 1.46 (t, J=7.2Hz, 2H), 1.63 (m, 2H), 2.09 (m, 0.9H), 2.25 (m, 0.1H), 3.68-3.81 (m, 2H), 3.99-4.12 (m, 2H), 4.95 (br s, 0.1H), 5.03 (d, J=3.6Hz, 0.1H), 5.15 (dd, J=3.9Hz, 3.9Hz, 0.9H), 5.63 (d, J=3.9Hz, 0.9H), 6.68 (d, J=9.3Hz, 0.1H), 6.81 (d, J=8.1Hz, 0.9H), 6.89 (d, J=7.8Hz, 0.9H), 7.14 (d, J=7.2Hz, 0.1H), 7.46-7.58 (m, 3H), 7.85 (m, 2H).

The ratio of the isomers in this solvent was about 9:1.
NMR (CD₃OD, δ): 0.74 (d, J=6.5Hz, 3H), 0.80 (d, J=6.5Hz, 3H), 0.86 (d, J=7.1Hz, 3H), 0.88 (d, J=6.8Hz, 3H), 1.34-1.50 (m, 3H), 1.64 (m, 1H), 2.01 (m, 0.4H), 2.17 (m, 0.6H), 3.73-3.97 (m, 4H), 4.96 (s, 0.6H), 5.11 (d, J=4Hz, 0.4H), 7.53-7.61 (m, 3H), 7.85 (m, 2H).

The ratio of the isomers in this solvent was about 6:4.

Compounds of Example 2 to Example 87 were prepared in the same manners as those of Reference Example 1 and Example 1. Physicochemical data of the compounds will be described below.

### Example 2: Preparation of (3S)-3-benzyloxycarbonylaminoacethylamino-2-tetrahydrofuranol (Compound No. 17 in Table-1)

Melting point: 119-121°C
IR (KBr, cm⁻¹): 3314, 1692, 1649, 1541.
NMR (CDCl₃, δ): 1.84 (m, 1H), 2.22-2.50 (m, 1H), 2.86 (s, 0.3H), 2.97 (s, 0.7H), 3.79-4.00 (m, 3H), 4.11 (m, 1H), 4.37 (m, 1H), 5.14 (s, 2H), 5.27 (m, 1.3H), 5.39 (s, 0.7H), 6.12 (s, 0.3H), 6.42 (s, 0.7H), 7.36 (m, 5H).

### Example 3: Preparation of (3S)-3-((S)-2-bezyloxycarbonylaminopropionylamino)-2-tetrahydrofuranol (Compound No. 18 in Table-1)

Melting point: 161-163°C
IR (KBr, cm⁻¹): 3312, 1688, 1647, 1561, 1530.
NMR (CDCl₃, δ): 1.36 (d, J=7.2Hz, 0.75H), 1.39 (d, 7.2Hz, 2.25H), 1.81 (m, 1H), 2.34 (m, 0.75H), 2.43 (m, 0.25H), 2.99 (s, 0.25H), 3.09 (s, 0.75H), 3.87 (ddd, J=7.8Hz, 7.8Hz, 7.8Hz, 0.75H), 4.00 (m, 0.25H), 4.11 (m, 1H), 4.22 (m, 1H), 4.37 (m, 1H), 5.11 (s, 2H), 5.29 (m, 2H), 6.28 (s, 0.25H), 6.45 (s, 0.75H), 7.35 (m, 5H).

### Example 4: Preparation of (3S)-3-((S)-2-benzyloxycarbonylaminovalerylamino)-2-tetrahydrofuranol (Compound No. 20 in Table-1)

Melting point: 148-149°C
IR (KBr, cm⁻¹): 3299, 1694, 1645, 1539.
NMR (CDCl₃, δ): 0.92 (m, 3H), 1.38 (m, 2H), 1.62 (m, 1H), 1.78 (m, 2H), 2.31 (m, 0.7H), 2.42 (m, 0.3H), 3.27 (s, 0.3H), 3.42 (s, 0.7H), 3.86 (ddd, J=7.8Hz, 7.8Hz, 7.8Hz, 0.7H), 3.98 (m, 0.3H), 4.11 (m, 2H), 4.33 (m, 1H), 5.10 (s, 2H), 5.27 (m, 1.3H), 5.38 (d, J=7.1Hz, 0.7H), 6.30 (s, 0.3H), 6.46 (d, J=8.0Hz, 0.7H), 7.35 (m, 5H).

### Example 5: Preparation of 3-((S)-2-benzyloxycarbonylamino-3-methylbutyrylamino)-2-tetrahydrofuranol (Compound No. 21 in Table-1)

Melting point: 122-123°C
IR (KBr, cm⁻¹): 3302, 1694, 1647, 1537.
NMR (CDCl₃, δ): 0.94 (m, 6H), 1.83 (m, 1H), 2.12 (m, 1H), 2.30 (m, 0.6H), 2.44 (m, 0.4H), 3.40 (s, 0.4H), 3.49 (s, 0.6H), 3.82-4.06 (m, 2H), 4.10 (m, 1H), 4.38 (m, 1H), 5.09 (m, 2H), 5.26 (s, 0.4H), 5.32 (s, 0.6H), 5.50 (m, 1H), 6.32 (s, 0.2H), 6.45 (s, 0.4H), 6.54 (s, 0.2H), 6.68 (s, 0.2H), 7.34 (m, 5H).

### Example 6: Preparation of (3S)-3-((S)-2-benzyloxycarbonylaminohexanoylamino)-2-tetrahydrofuranol (Compound No. 22 in Table-1)

Melting point: 165-166°C
IR (KBr, cm⁻¹): 3304, 1694, 1645, 1539.
NMR (CDCl₃, δ): 0.87 (m, 3H), 1.32 (m, 3H), 1.64 (m, 2H), 1.78 (m, 2H), 2.29 (m, 0.8H), 2.42 (m, 0.2H), 3.22 (s, 0.2H), 3.39 (s, 0.8H), 3.86 (ddd, J=7.8Hz, 7.8Hz, 7.8Hz, 0.8H), 3.99 (m, 0.2H), 4.11 (m, 2H), 4.33 (m, 1H), 5.10 (s, 2H), 5.17-5.44 (m, 2H), 6.27 (s, 0.2H), 6.47 (d, J=7.8Hz, 0.8H), 7.34 (m, 5H).

### Example 7: Preparation of (3S)-3-((2S)-2-benzyloxycarbonylamino-3-methylvalerylamino)-2-tetrahydrofuranol (Compound No. 23 in Table-1)

Melting point: 169-171°C
IR (KBr, cm⁻¹): 3299, 1694, 1649, 1539.
NMR (CDCl₃, δ): 0.91 (m, 6H), 1.12 (m, 1H), 1.50 (m, 1H), 1.84 (m, 2H), 2.31 (m, 0.7H), 2.44 (m, 0.3H), 3.40 (s, 1H), 3.82-4.16 (m, 3H), 4.35 (m, 1H), 5.10 (s, 2H), 5.26 (m, 1H), 5.41 (s, 1H), 6.17 (s, 0.3H), 6.40 (s, 0.7H), 7.38 (m, 5H).

### Example 8: Preparation of (3S)-3-((S)-2-amino-4-methylvalerylamino)-2-tetrahydrofuranol hydrochloride (Compound No. 24 in Table-1)

NMR (CD₃OD, δ): 1.00 (d, J=5.6Hz, 6H), 1.69-1.83 (m, 3H), 1.98 (m, 1H), 2.24 (m, 0.5H), 2.34 (m, 0.5H), 3.84-4.07 (m, 4H), 4.95 (s, 0.5H), 4.99 (d, J=4.0Hz, 0.5H).

### Example 9: Preparation of 3-((S)-2-methoxycarbonylamino-4-methylvalerylamino)-2-tetrahydrofuranol (Compound No. 25 in Table-1)

Melting point: 139-141°C
IR (KBr, cm⁻¹): 3287, 3081, 1686, 1653, 1553.
NMR (CDCl₃, δ): 0.94 (m, 6H), 1.50-1.74 (m, 3H), 1.81 (m, 1H), 2.36 (m, 0.6H), 2.48 (m, 0.4H), 3.44 (s, 0.4H), 3.58 (s, 0.6H), 3.68 (s, 3H), 3.99 (m, 0.6H), 4.01 (m, 0.4H), 4.13 (m, 2H), 4.34 (m, 1H), 5.27 (d, J=3.0Hz, 0.6H), 5.34 (m, 1.4H), 6.49 (s, 0.4H), 6.57 (d, J=7.8Hz, 0.4H), 6.68 (s, 0.2H).

### Example 10: Preparation of (3S)-3-((S)-2-tert-butoxycarbonylamino-4-methylvalerylamino)-2-tetrahydrofuranol (Compound No. 27 in Table-1)

Melting point: 65-70°C
IR (KBr, cm⁻¹): 3310, 1698, 1657.
NMR (CDCl₃, δ): 0.95 (m, 6H), 1.44 (s, 4.5H), 1.44 (s, 4.5H), 1.48 (m, 1H), 1.66 (m, 2H), 1.81 (m, 1H), 2.33 (m, 0.5H), 2.46 (m, 0.5H), 3.88 (ddd, J=7.5Hz, 7.5Hz, 7.5Hz, 0.5H), 4.01 (ddd, J=8.4Hz, 8.4Hz, 8.4Hz, 0.5H), 4.12 (m, 2H), 4.36 (m, 1H), 4.96 (d, J=7.8Hz, 0.5H), 5.03 (m, 0.5H), 5.26 (s, 0.5H), 5.33 (s, 0.5H), 6.47 (m, 0.5H), 6.61 (m, 0.5H).

### Example 11: Preparation of (3S)-3-((S)-2-isobutoxycarbonylamino-4-methylvalerylamino)-2-tetrahydrofuranol (Compound No. 28 in Table-1)

Melting point: 31-33°C
IR (KBr, cm⁻¹): 3310, 1699, 1657, 1543.
NMR (CDCl₃, δ): 0.94 (m, 12H), 1.47-2.00 (m, 5H), 2.36 (m, 0.5H), 2.45 (m, 0.5H), 2.96 (s, 0.5H), 3.17 (s, 0.5H), 3.90 (m, 1.5H), 4.02 (m, 0.5H), 4.15 (m, 2H), 4.36 (m, 1H), 5.08 (m, 1H), 5.27 (s, 0.5H), 5.35 (s, 0.5H), 6.24 (s, 0.5H), 6.50 (s, 0.5H).

### Example 12: Preparation of (3S)-3-((S)-2-cyclohexylmethoxycarbonylamino-4-methylvalerylamino)-2-tetrahydrofuranol (Compound No. 29 in Table-1)

Melting point: 52-54°C
IR (KBr, cm⁻¹): 3310, 1703, 1659, 1545.
NMR (CDCl₃, δ): 0.93 (m, 8H), 1.26-1.33 (m, 4H), 1.43-1.92 (m, 9H), 2.36 (m, 0.5H), 2.50 (m, 0.5H), 3.22 (s, 0.5H), 3.49 (s, 0.5H), 3.87-4.23 (m, 5H), 4.34 (m, 1H), 5.13 (m, 1H), 5.26 (d, J=2.7Hz, 0.5H), 5.32 (dd, J=3.9Hz, 3.9Hz, 0.5H), 5.34 (s, 0.5H), 6.53 (s, 0.5H).

### Example 13: Preparation of (3S)-3-((S)-2-benzyloxycarbonylamino-4-methylvalerylamino)-2-tetrahydrofuranol (Compound No. 31 in Table-1)

Melting point: 40-43°C
IR (KBr, cm⁻¹): 3306, 1705, 1657.
NMR (CDCl₃, δ): 0.92 (d, J=6.1Hz, 3H), 0.94 (d, J=5.9Hz, 3H), 1.52 (m, 1H), 1.64 (m, 2H), 1.78 (m, 1H), 2.29 (m, 0.5H), 2.41 (m, 0.5H), 3.51 (s, 0.5H), 3.74 (s, 0.5H), 3.85 (ddd, J=8.0Hz, 8.0Hz, 8.0Hz, 0.5H), 3.97 (m, 0.5H), 4.10 (m, 2H), 4.32 (m, 1H), 5.09 (s, 1H), 5.10 (s, 1H), 5.24 (s, 0.5H), 5.29 (s, 0.5H), 5.35 (d, J=6.5Hz, 0.5H), 5.38 (d, J=8.2Hz, 0.5H), 6.45 (d, J=6.0Hz, 0.5H), 6.57 (d, J=6.0Hz, 0.5H), 7.33 (m, 5H).

### Example 14: Preparation of (3S)-3-{(S)-2-(N-benzyloxycarbonyl-N-methyl)amino-4-methylvalerylamino}-2-tetrahydrofuranol (Compound No. 32 in Table-1)

IR (neat): 3335, 1669.
NMR (CDCl₃, δ): 0.93 (m, 6H), 1.49 (m, 1H), 1.69 (m, 3H), 2.28 (m, 0.5H), 2.39 (m, 0.5H), 2.85 (s, 1.5H), 2.86 (s, 1.5H), 3.12 (s, 0.5H), 3.30 (s, 0.5H), 3.85 (m, 1H), 4.07 (m, 1H), 4.29 (m, 1H), 4.60 (m, 0.5H), 4.70 (m, 0.5H), 5.09-5.26 (m, 3H), 6.24 (s, 0.5H), 6.53 (s, 0.5H), 7.36 (m, 5H).

### Example 15: Preparation of (3S)-3-{(S)-2-(4-fluorobenzyloxycarbonylamino)-4-methylvalerylamino}-2-tetrahydrofuranol (Compound No. 37 in Table-1)

Melting point: 50-52°C
IR (KBr, cm⁻¹): 3310, 1705, 1657, 1607, 1541, 1514.
NMR (CDCl₃, δ): 0.93 (m, 6H), 1.47-1.90 (m, 4H), 2.32 (m, 0.5H), 2.44 (m, 0.5H), 3.10 (s, 0.5H), 3.35 (s, 0.5H), 3.87 (ddd, J=7.8Hz, 7.8Hz, 7.8Hz, 0.5H), 3.98 (m, 0.5H), 4.11 (m, 2H), 4.33 (m, 1H), 5.04 (s, 1H), 5.06 (s, 1H), 5.25 (m, 2H), 6.20 (bs, 0.5H), 6.46 (d, J=8.1Hz, 0.5H), 7.03 (dd, J=8.7Hz, 2H), 7.33 (m, 2H).

### Example 16: Preparation of (3S)-3-{(S)-2-(2-chlorobenzyloxycarbonylamino)-4-methylvalerylamino}-2-tetrahydrofuranol (Compound No. 38 in Table-1)

Melting point: 46-49°C
IR (KBr, cm⁻¹): 3308, 1707, 1657, 1541.
NMR (CDCl₃, δ): 0.94 (m, 6H), 1.48-1.86 (m, 4H), 2.32 (m, 0.6H), 2.48 (m, 0.4H), 2.90 (s, 0.4H), 3.09 (s, 0.6H), 3.88 (ddd, J=7.8Hz, 7.8Hz, 7.8Hz, 0.6H), 4.01 (m, 0.4H), 4.11 (m, 2H), 4.34 (m, 1H), 5.23 (s, 2H), 5.25 (m, 2H), 6.18 (s, 0.4H), 6.45 (d, J=7.6Hz, 0.6H), 7.26 (m, 2H), 7.40 (m, 2H).

### Example 17: Preparation of (3S)-3-{(S)-2-(4-chlorobenzyloxycarbonylamino)-4-methylvalerylamino}-2-tetrahydrofuranol (Compound No. 40 in Table-1)

Melting point: 47-49°C
IR (KBr, cm⁻¹): 3308, 1705, 1657, 1541.
NMR (CDCl₃, δ): 0.94 (m, 6H), 1.50-1.86 (m, 4H), 2.32 (m, 0.7H), 2.44 (m, 0.3H), 2.88 (s, 0.3H), 3.03 (s, 0.7H), 3.88 (ddd, J=7.8Hz, 7.8HZ, 7.8Hz, 0.7H), 3.95 (m, 0.3H), 4.12 (m, 2H), 4.35 (m, 1H), 5.06 (s, 0.6H), 5.07 (s, 1.4H), 5.22 (m, 1.3H), 5.30 (s, 0.7H), 6.09 (s, 0.3H), 6.39 (d, J=8.7Hz, 0.7H), 7.31 (m, 4H).

### Example 18: Preparation of (3S)-3-{(S)-4-methyl-2-(2-methylbenzyloxycarbonylamino)valerylamino}-2-tetrahydrofuranol (Compound No. 44 in Table-1)

Melting point: 120-122°C
IR (KBr, cm⁻¹): 3302, 1694, 1645, 1541.
NMR (CDCl₃, δ): 0.93 (m, 6H), 1.52 (m, 1H), 1.59 (m, 3H), 2.16-2.49 (m, 1H), 2.34 (s, 3H), 3.28 (s, 0.5H), 3.52 (s, 0.5H), 3.86 (dd, J=7.8Hz, 7.8Hz, 7.8Hz, 0.5H), 3.98 (m, 0.5H), 4.13 (m, 2H), 4.31 (m, 1H), 5.12 (s, 2H), 5.27 (m, 2H), 6.34 (s, 0.5H), 6.51 (s, 0.5H), 7.19 (m, 2H), 7.31 (m, 2H).

### Example 19: Preparation of (3S)-3-{(S)-4-methyl-2-(4-methylbenzyloxycarbonylamino)valerylamino}-2-tetrahydrofuranol (Compound No. 46 in Table-1)

IR (KBr, cm⁻¹): 3310, 1703, 1657, 1539.
NMR (CDCl₃, δ): 0.93 (m, 6H), 1.52 (m, 1H), 1.58-1.86 (m, 3H), 2.29 (m, 0.5H), 2.35 (s, 3H), 2.43 (m, 0.5H), 2.91 (s, 0.5H), 3.03 (s, 0.5H), 3.87 (ddd, J=7.8Hz, 7.8Hz, 7.8Hz, 0.5H), 3.97 (m, 0.5H), 4.10 (m, 2H), 4.33 (m, 1H), 5.06 (s, 2H), 5.14 (m, 1H), 5.26 (m, 1H), 6.20 (s, 0.5H), 6.44 (s, 0.5H), 7.16 (m, 2H), 7.23 (m, 2H).

### Example 20: Preparation of (3S)-3-{(S)-2-(2-methoxybenzyloxycarbonylamino)-4-methylvalerylamino)-2-tetrahydrofuranol (Compound No. 47 in Table-1)

Melting point: 36-38°C
IR (KBr, cm⁻¹): 3308, 1703, 1657, 1539.
NMR (CDCl₃, δ): 0.93 (m, 6H), 1.51 (m, 1H), 1.60-1.91 (m, 3H), 2.30 (m, 0.6H), 2.42 (m, 0.4H), 3.15 (s, 0.4H), 3.31 (d, J=3.0Hz, 0.6H), 3.83 (s, 3H), 3.87 (m, 0.6H), 3.91 (m, 0.4H), 4.13 (m, 2H), 4.32 (m, 1H), 5.16 (s, 0.8H), 5.18 (s, 1.2H), 5.23 (m, 2H), 6.65 (s, 0.4H), 6.53 (d, J=7.5Hz, 0.6H), 6.91 (m, 2H), 7.31 (m, 2H).

### Example 21: Preparation of (3S)-3-{(S)-2-(4-methoxybenzyloxycarbonylamino)-4-methylvalerylamino}-2-tetrahydrofuranol (Compound No. 49 in Table-1)

Melting point: 30-33°C
IR (KBr, cm⁻¹): 3310, 1701, 1657, 1516.
NMR (CDCl₃, δ): 0.92 (m, 6H), 1.50 (m, 1H), 1.59-1.88 (m, 3H), 2.29 (m, 0.5H), 2.42 (m, 0.5H), 3.01 (s, 0.5H), 3.20 (d, J=3.0Hz, 0.5H), 3.80 (s, 3H), 3.88 (ddd, J=7.8Hz, 7.8Hz, 7.8Hz, 0.5H), 3.96 (m, 0.5H), 4.11 (m, 2H), 4.33 (m, 1H), 5.03 (s, 2H), 5.15 (m, 1H), 5.27 (s, 0.5H), 5.32 (s, 0.5H), 6.22 (s, 0.5H), 6.66 (s, 0.5H), 6.87 (m, 2H), 7.28 (m, 2H).

### Example 22: Preparation of (3S)-3-{(S)-2-(9-fluorenylmethoxycarbonylamino)-4-methylvalerylamino}-2-tetrahydrofuranol (Compound No. 51 in Table-1)

Melting point: 150-153°C
IR (KBr, cm⁻¹): 3314, 1725, 1696, 1653, 1534.
NMR (CDCl₃, δ): 0.93 (m, 6H), 1.65 (m, 3H), 1.91 (m, 1H), 2.32 (m, 0.7H), 2.42 (m, 0.3H) 3.08 (s, 0.3H), 3.28 (d, J=3Hz, 0.7H), 3.86 (ddd, J=7.8Hz, 7.8Hz, 7.8Hz, 0.7H), 4.01 (m, 0.3H), 4.08-4.24 (m, 3H), 4.32-4.52 (m, 3H), 5.27 (m, 2H), 6.21 (s, 0.3H), 6.38 (s, 0.7H), 7.31 (m, 2H), 7.40 (m, 2H), 7.56 (dd, J=7.4Hz, 2H), 7.76 (d, J=7.4Hz, 2H).

### Example 23: Preparation of (3S)-3-((S)-4-methyl-2-tetrahydrofurfuryloxycarbonylaminovalerylamino)-2-tetrahydrofuranol (Compound No. 52 in Table-1)

Melting point: 40-43°C
IR (KBr, cm⁻¹): 3308, 1705, 1659, 1543.
NMR (CDCl₃, δ): 0.94 (m, 6H), 1.46-2.07 (m, 8H), 2.32 (m, 0.5H), 2.46 (m, 0.5H), 3.36 (s, 0.5H), 3.39 (s, 0.5H), 3.78-4.25 (m, 8H), 4, 29-4.42 (m, 1H), 5.28 (m, 1.5H), 5.40 (s, 0.5H), 6.34 (s, 0.5H), 5.56 (s, 0.5H).

### Example 24: Preparation of (3S)-3-{(S)-4-methyl-2-(2-tetrahydropyranylmethoxycarbonylamino)valerylamino}-2-tetrahydrofuranol (Compound No. 53 in Table-1)

IR (KBr, cm⁻¹): 3306, 1705, 1659, 1539.
NMR (CDCl₃, δ): 0.94 (m, 6H), 1.30 (m, 1H), 1.53 (m, 3H), 1.68 (m, 4H), 1.84 (m, 2H), 2.32 (m, 0.5H), 2.47 (m, 0.5H), 3.44 (m, 1H), 3.55 (m, 1H), 3.65 (s, 0.5H), 3.74 (s, 0.5H), 3.98 (ddd, J=7.8Hz, 7.8Hz, 7.8Hz, 0.5H), 4.00 (m, 3.5H), 4.12 (m, 2H), 4.35 (m, 1H), 5.28 (d, J=3.0Hz, 0.5H), 5.31 (m, 1H), 5.46 (m, 0.5H), 6.45 (s, 0.5H), 6.61 (m, 0.5H).

### Example 25: Preparation of (3S)-3-{(S)-4-methyl-2-(2-pyridylmethoxycarbonylamino)valerylamino}-2-tetrahydrofuranol (Compound No. 54 in Table-1)

Melting point: 54-56°C
IR (KBr, cm⁻¹): 3306, 1711, 1657, 1541.
NMR (CDCl₃, δ): 0.95 (m, 6H), 1.53 (m, 2H), 1.68 (m, 3H), 2.28 (m, 0.5H), 2.47 (m, 0.5H), 3.85 (ddd, J=8.1Hz, 8.1Hz, 8.1Hz, 0.5H), 3.97 (ddd, J=8.1Hz, 8.1Hz, 8.1Hz, 0.5H), 4.08-4.44 (m, 3H), 5.10-5.36 (m, 3H), 5.75 (s, 1H), 6.67 (s, 0.5H), 6.69 (s, 0.5H), 7.19-7.40 (m, 2H), 7.69 (m, 1H), 8.50 (d, J=3.9Hz, 0.5H), 8.56 (d, J=3.9Hz, 0.5H).

### Example 26: Preparation of 3-{(S)-4-methyl-2-(2-pyridylmethoxycarbonylamino)valerylamino)-2-tetrahydrofuranol N-oxide (Compound No. 57 in Table-1)

NMR (CDCl₃, δ): 0.93 (m, 6H), 1.50-1.92 (m, 4H), 2.10-2.48 (m, 1H), 2.86 (s, 0.8H), 3.30 (s, 0.2H), 3.84 (m, 1H), 4.08 (m, 1H), 4.36 (m, 2H), 5.10-5.68 (m, 3H), 5.99 (s, 0.5H), 6.12 (s, 0.3H), 6.30 (m, 0.2H), 6.82-7.07 (m, 1H), 7.35 (m, 3H), 8.28 (d, J=8.6Hz, 1H).

### Example 27: Preparation of (3S)-3-((S)-2-cyclohexyloxycarbonylamino-4-methylvalerylamino)-2-tetrahydrofuranol (Compound No. 60 in Table-1)

Melting point: 28-30°C
IR (KBr, cm⁻¹): 3310, 1696, 1657, 1539.
NMR (CDCl₃, δ): 0.95 (m, 6H), 1.29 (m, 2H), 1.36 (m, 4H), 1.53 (m, 2H), 1.69 (m, 4H), 1.85 (m, 2H), 2.36 (m, 0.5H), 2.48 (m, 0.5H), 2.85 (s, 0.5H), 3.06 (s, 0.5H), 3.89 (ddd, J=7.8Hz, 7.8Hz, 7.8Hz, 0.5H), 4.02 (m, 0.5H), 4.14 (m, 2H), 4.36 (m, 1H), 4.63 (m, 1H), 4.99 (m, 1H), 5.26 (m, 0.5H), 5.32 (m, 0.5H), 6.22 (s, 0.5H), 6.47 (s, 0.5H).

### Examle28: Preparation of (3S)-3-((S)-4-methyl-2-phenoxycarbonylaminovalerylamino)-2-tetrahydrofuranol (Compound No. 61 in Table-1)

Melting point: 68-70°C
IR (KBr, cm⁻¹): 3308, 1723, 1659, 1539.
NMR (CDCl₃, δ): 0.98 (m, 6H) 1.52 (m, 1H), 1.58-1.86 (m, 3H), 2.30-2.52 (m, 1H), 2.98 (s, 0.6H), 3.23 (s, 0.4H), 3.89 (ddd, J=7.8Hz, 7.8Hz, 7.8Hz, 0.6H), 4.01 (ddd, J=7.8Hz, 7.8Hz, 7.8Hz, 0.4H), 4.09-4.27 (m, 2H), 4.38 (m, 1H), 5.27 (d, J=2.7Hz, 0.4H), 5.33 (dd, J=3.6Hz, 3.6Hz, 0.6H), 5.60 (s, 1H), 6.10 (s, 0.4H), 6.24 (s, 0.6H), 7.12 (m, 2H), 7.20 (m, 1H), 7.35 (m, 2H).

### Example 29: Preparation of (3S)-3-((S)-4-methyl-2-phenylureidovalerylamino)-2-tetrahydrofuranol (Compound No. 63 in Table-1)

Melting point: 181-182°C
IR (KBr, cm⁻¹): 3291, 1638, 1555.
NMR (CDCl₃, δ): 0.96 (m, 6H), 1.50 (m, 2H), 1.67 (m, 1H), 1.86 (m, 1H), 2.26 (m, 0.5H), 2.41 (m, 0.5H), 2.88 (s, 0.5H), 3.40 (s, 0.5H), 3.82 (ddd, J=7.8Hz, 7.8Hz, 7.8Hz, 0.5H), 4.07 (m, 1.5H), 4.29 (m, 2H), 5.20 (s, 0.5H), 5.26 (d, J=4.5Hz, 0.5H), 5.98 (m, 1H), 7.02 (m, 1H), 7.24 (m, 5H), 7.73 (s, 0.5H), 7.91 (s, 0.5H).

### Example 30: Preparation of (3S)-3-{(S)-2-(3,3-dimethylbutyrylamino)-4-methylvlerylamino}-2-tetrahydrofuranol (Compound No. 73 in Table-1)

Melting point: 152-153°C
IR (KBr, cm⁻¹): 3293, 1642, 1549.
NMR (CDCl₃, δ): 0.93 (m, 6H), 1.02 (s, 9H), 1.49-1.94 (m, 4H), 2.07 (s, 1H), 2.08 (s, 1H), 2.32 (m, 0.5H), 2.45 (m, 0.5H), 3.17 (d, J=3.0Hz, 0.5H), 3.73 (d, J=3.0Hz, 0.5H), 3.87 (ddd, J=7.8Hz, 7.8Hz, 7.8Hz, 0.5H), 4.02 (m, 0.5H), 4.10 (m, 1H), 4.23-4.54 (m, 3H), 5.28 (d, J=2.4Hz, 0.5H), 5.31 (dd, J=2.4Hz, 2.4Hz, 0.5H), 6.62 (s, 0.5H), 6.69 (s, 0.5H).

### Eample31: Preparation of (3S)-3-((S)-4-methyl-2-tetradecanoylaminovalerylamino)-2-tetrahydrofuranol (Compound No. 78 in Table-1)

Melting point: 96-98°C
IR (KBr, cm⁻¹): 3292, 1636, 1543.
NMR (CDCl₃, δ): 0.88 (t, J=5.3Hz, 3H), 0.94 (m, 6H), 1.25 (m, 22H), 1.51-1.94 (m, 4H), 2.20 (m, 2H), 2.31 (m, 0.5H), 2.43 (m, 0.5H), 3.10 (d, J=2.7Hz, 0.5H), 3.65 (d, J=2.7Hz, 0.5H), 3.87 (ddd, J=7.8Hz, 7.8Hz, 7.8Hz, 0.5H), 4.00 (ddd, J=7.8Hz, 7.8Hz, 7.8Hz, 0.5H), 4.11 (m, 1H), 4.26-4.55 (m, 2H), 5.27 (d, J=2.7Hz, 0.5H), 5.31 (dd, J=4.1Hz, 4.1Hz, 0.5H), 5.97 (s, 1H), 6.52 (s, 0.5H), 6.62 (s, 0.5H).

### Example 32: Preparation of (3S)-3-{(S)-4-methyl-2-(3-phenylpropionylamino)valerylamino}-2-tetrahydrofuranol (Compound No. 83 in Table-1)

Melting point: 60-62°C
IR (KBr, cm⁻¹): 3291, 1644, 1549.
NMR (CDCl₃, δ): 0.89 (m, 6H), 1.81 (m, 1H), 1.44-1.62 (m, 3H), 2.28-2.46 (m, 1H), 2.50 (m, 2H), 2.77 (s, 0.6H), 2.93 (m, 2H), 3.08 (s, 0.4H), 3.87 (ddd, J=7.8Hz, 7.8Hz, 7.8Hz, 0.6H), 4.05 (ddd, J=7.8Hz, 7.8Hz, 7.8Hz, 0.4H), 4.12 (m, 1H), 4.24-4.48 (m, 2H), 5.24 (s, 0.4H), 5.29 (m, 0.6H), 5.79 (m, 1H), 6.28 (d, J=7.5Hz, 0.4H), 6.45 (d, J=7.5Hz, 0.6H), 7.23 (m, 5H).

### Example 33: Preparation of (3S)-3-{(S)-4-methyl-2-(1-naphthylacetylamino)valerylamino}-2-tetrahydrofuranol (Compound No. 85 in Table-1)

Melting point: 164-167°C
IR (KBr, cm⁻¹): 3279, 1638.
NMR (CDCl₃+DMSO-d₆, δ): 0.76 (d, J=6.0Hz, 3H), 0.78 (d, J=5.8Hz, 3H), 1.35 (m, 2H), 1.48 (m, 1H), 1.72 (m, 1H), 2.19 (m, 0.7H), 2.31 (m, 0.3H), 3.78 (ddd, J=8.0Hz, 8.0Hz, 8.0Hz, 0.7H), 3.85 (ddd, J=8.0Hz, 8.0Hz, 8.0Hz, 0.3H), 3.97-4.09 (m, 3H), 4.20 (m, 1H), 4.41 (m, 1H), 5.09 (d, J=4.0Hz, 0.3H), 5.15 (d, J=3.7Hz, 0.3H), 5.22 (dd, J=4.4Hz, 4.4Hz, 0.7H), 5.38 (d, J=4.3Hz, 0.7H), 6.42 (d, J=7.8Hz, 0.7H), 6.56 (d, J=9.0Hz, 0.3H), 6.71 (d, J=7.4Hz, 0.7H), 6.97 (d, J=7.2Hz, 0.3H), 7.42-7.53 (m, 4H), 7.55-7.88 (m, 2H), 7.98 (d, J=7.4Hz, 1H).

### Example 34: Preparation of (3S)-3-((S)-4-methyl-2-phenoxyacetylaminovalerylamino)-2-tetrahydrofuranol (Compound No. 90 in Table-1)

Melting point: 30°C
IR (KBr, cm⁻¹): 3297, 1653.
NMR (CDCl₃ δ): 0.92 (d, J=6.0Hz, 3H), 0.92 (d, J=5.7Hz, 3H), 1.55-1.70 (m, 2H), 1.84 (m, 2H), 2.32 (m, 0.6H), 2.45 (m, 0.4H), 3.87 (ddd, J=7.8Hz, 7.8Hz, 7.8Hz, 0.6H), 4.01 (ddd, J=7.8Hz, 7.8Hz, 7.8Hz, 0.4H), 4.11 (m, 1H), 4.33 (m, 1H), 4.51 (s, 0.8H), 4.52 (s, 1.2H), 4.55 (m, 1H), 5.28 (s, 0.4H), 5.33 (d, J=4.5Hz, 0.6H), 6.63 (d, J=7.5Hz, 0.4H), 6.68 (d, J=8.1Hz, 0.6H), 6.93 (m, 2H), 7.03 (m, 2H), 7.31 (m, 2H).

### Example 35: Preparation of (3S)-3-{(S)-2-(2-chlorophenoxyacetylamino)-4-methylvalerylamino}-2-tetrahydrofuranol (Compound No. 94 in Table-1)

Melting point: 49-52°C
IR (KBr, cm⁻¹): 3302, 3074, 1655, 1537.
NMR (CDCl₃, δ): 0.94 (m, 6H), 1.60-1.91 (m, 4H), 2.31 (m, 0.7H), 2.45 (m, 0.3H), 3.27 (d, J=2.7Hz, 0.3H), 3.72 (d, J=2.7Hz, 0.7H), 3.87 (ddd, J=7.8Hz, 7.8Hz, 7.8Hz, 0.7H), 4.06 (ddd, J=7.8Hz,7.8Hz, 7.8Hz, 0.3H), 4.11 (m, 1H), 4.40 (m, 1H), 4.52 (m, 1H), 4.52 (s, 0.6H), 4.57 (s, 1.4H), 5.29 (s, 0.3H), 5.33 (dd, J=3.6Hz, 3.6Hz, 0.7H), 6.49 (s, 0.3H), 6.64 (s, 0.7H), 6.90 (m, 1H), 7.02 (m, 1H), 7.26 (m, 2H), 7.41 (m, 1H).

### Example 36: Preparation of (3S)-3-{(S)-2-(4-chlorophenoxyacetylamino)-4-methylvalerylamino}-2-tertahydrofuranol (Compound No. 96 in Table-1)

Melting point: 53-55°C
IR (KBr, cm⁻¹): 3301, 1653, 1541.
NMR (CDCl₃, δ): 0.94 (m, 6H), 1.69-1.88 (m, 4H), 2.32 (m, 0.5H), 2.58 (m, 0.5H), 2.91 (d, J=2.8Hz, 0.5H), 3.28 (d, J=3.0Hz, 0.5H), 3.88 (ddd, J=7.8Hz, 7.8Hz, 7.8Hz, 0.5H), 4.06 (ddd, J=7.8Hz, 7.8Hz, 7.8Hz, 0.5H), 4.12 (m, 1H), 4.34 (m, 1H), 4.47 (s, 1H), 4.49 (s, 1H), 4.53 (m, 1H), 5.28 (d, J=2.6Hz, 0.5H), 5.33 (dd, J=4.5Hz, 4.5Hz, 0.5H), 6.29 (s, 0.5H), 6.47 (s, 0.5H), 6.88 (m, 3H), 7.27 (m, 2H).

### Example 37: Preparation of (3S)-3-((S)-4-methyl-2-phenylthioacetylaminovalerylamino)-2-tetrahydrofuranol (Compound No. 110 in Table-1)

Melting point: 45-47°C
IR (KBr, cm⁻¹): 3287, 1645, 1551.
NMR (CDCl₃, δ): 0.81 (m, 6H), 1.35 (m, 1H), 1.52 (m, 2H), 1.70 (m, 1H), 2.25 (m, 0.5H), 2.39 (m, 0.5H), 3.23 (s, 0.5H), 3.66 (m, 2.5H), 3.85 (ddd, J=7.8Hz, 7.8Hz, 7.8Hz, 0.5H), 3.96 (ddd, J=7.8Hz, 7.8Hz, 7.8Hz, 0.5H), 4.09 (m, 1H), 4.40-4.42 (m, 2H), 5.23 (d, J=2.3Hz, 0.5H), 5.28 (dd, J=3.9Hz, 3.9Hz, 0.5H), 6.39 (s, 0.5H), 6.53 (s, 0.5H), 7.10 (m, 1H), 7.21 (m, 1H), 7.29 (m, 3H), 7.30 (s, 1H).

### Example 38: Preparation of (3S)-3-{(S)-4-methyl-2-(3-pheylsulfonylpropionylamino)valerylamino)-2-tetrahydorofuranol (Compound No. 111 in Table-1)

IR (KBr, cm⁻¹): 3301, 1649, 1547.
NMR (CDCl₃, δ): 0.93 (m, 6H), 1.48-1.74 (m, 5H), 1.80-1.92 (m, 1H), 2.30 (m, 0.5H), 2.43 (m, 0.5H), 2.72 (m, 2H), 3.41 (m, 1H), 3.52 (m, 1H), 3.86 (ddd, J=8.1Hz, 8.1Hz, 8.1Hz, 0.5H), 4.02 (m, 0.5H), 4.09 (m, 2H), 4.28-4.48 (m, 2H), 5.29 (s, 0.5H), 5.33 (dd, J=4.2Hz, 4.2Hz, 0.5H), 6.27 (s, 1H), 6.46 (s, 0.5H), 6.58 (s, 0.5H), 7.59 (m, 2H), 7.69 (m, 1H), 7.93 (dd, J=7.2Hz, 5.4Hz, 2H).

### Example 39: Preparation of (3S)-3-((S)-2-benzoylamino-4-methylvalerylamino)-2-tetrahydrofuranol (Compound No. 112 in Table-1)

Melting point: 154- 156°C
IR (KBr, cm⁻¹): 3300, 1665, 1636.
NMR (CDCl₃, δ): 0.98 (d, J=5.5Hz, 6H), 1.71 (m, 3H), 1.87 (m, 1H), 2.31 (m, 0.7H), 2.42 (m, 0.3H), 3.16 (s, 0.3H), 3.60 (s, 0.7H), 3.87 (ddd, J=8.2Hz, 8.2Hz, 8.2Hz, 0.7H), 4.03 (ddd, J=7.5Hz, 7.5Hz, 7.5Hz, 0.3H), 4.12 (ddd, J=8.6Hz, 8.6Hz, 3.5Hz, 1H), 4.36 (m, 1H), 4.68 (m, 1H), 5.30 (s, 0.3H), 5.35 (d, J=4.7Hz, 0.7H), 6.67-6.75 (m, 2H), 7.43 (m, 2H), 7.52 (m, 1H), 7.79 (m, 2H).

### Example 40: Preparation of (3S)-3-{(S)-2-(2-fluorobenzoylamino)-4-metylvalerylamino}-2-tetrahydrofuranol (Compound No. 113 in Table-1)

Melting point: 62-64°C
IR (KBr, cm⁻¹): 3306, 1644, 1534.
NMR (CDCl₃, δ): 0.80-1.07 (m, 6H), 1.59-2.01 (m, 4H), 2.40 (m, 1H), 3.82 (m, 1H), 3.97-4.20 (m, 1.6H), 4.24-4.45 (m, 1.4H), 4.68 (m, 1H), 5.31 (d, J=2.5Hz, 0.6H), 5.35 (dd, J=4.2Hz, 4.1Hz, 0.4H), 6.87 (m, 1H), 7.02-7.20 (m, 2H), 7.25 (m, 1H), 7.50 (m, 1H), 8.00 (m, 1H).

### Example 41: Preparation of (3S)-3-{(S)-2-(3-fluorobenzoylamino)-4-methylvalerylamino}-2-tetrahydrofuranol (Compound No. 114 in Table-1)

Melting point: 159-161°C
IR (KBr, cm⁻¹): 3304, 3076, 1638, 1588, 1547.
NMR (CDCl₃, δ): 0.87-1.07 (m, 6H), 1.61-1.98 (m, 4H), 2.39 (m, 1H), 3.39 (d, J=2.6Hz, 0.5H), 3.87 (m, 1H), 3.98-4.18 (m, 1.5H), 4.35 (m, 1H), 4.67 (m, 1H), 5.31 (d, J=2.6Hz, 0.5H), 5.35 (dd, J=4.1Hz, 4.1Hz, 0.5H), 6.71 (d, J=7.6Hz, 0.5H), 6.76 (d, J=7.6Hz, 0.5H), 6.90 (d, J=8.2Hz, 0.5H), 6.99 (d, J=8.2Hz, 0.5H), 7.20 (ddd, J=8.2Hz, 8.2Hz, 2.7Hz, 1H), 7.40 (m, 1H), 7.43-7.60 (m, 2H).

### Example 42: Preparation of (3S)-3-{(S)-2-(4-fluorobenzoylamino)-4-methylvalerylamino}-2-tetrahydrofuranol (Compound No. 115 in Table-1)

Melting point: 151-153°C
IR (KBr, cm⁻¹): 3422, 3301, 1640, 1545, 1503.
NMR (CDCl₃, δ): 0.85-1.17 (m, 6H), 1.60-1.97 (m, 4H), 2.37 (m, 1H), 3.79 (d, J=2.7Hz, 0.55H), 3.87 (m, 0.55H), 4.06 (m, 0.45H), 4.10 (m, 1H), 4.27 (ddd, J=6.8Hz, 6.8Hz, 2.1Hz, 0.45H), 4.39 (m, 1H), 5.31 (d, J=2.7Hz, 0.55H), 5.35 (d, J=4.1Hz, 4.1Hz, 0.45H), 6.28 (d, J=8.3Hz, 0.45H), 6.96 (d, J=8.3Hz, 0.55H), 7.02-7.19 (m, 3H), 7.75-7.89 (m, 2H).

### Example 43: Preparation of (3S)-3-{(S)-2-(4-chlorobenzoylamino)-4-methylvalerylamino}-2-tetrahydrofuranol (Compound No. 121 in Table-1)

Melting point: 93-96°C
IR (KBr, cm⁻¹): 3295, 1636.
NMR (CDCl₃, δ): 0.95 (d, J=6.0Hz, 3H), 0.97 (d, J=5.7Hz, 3H), 1.73 (m, 3H), 1.86 (m, 1H), 2.31 (m, 0.7H), 2.42 (m, 0.3H), 3.41 (s, 0.3H), 3.86 (s, 0.7H), 3.87 (ddd, J=8.4Hz, 8.4Hz, 8.4Hz, 0.7H), 4.02 (ddd, J=7.8Hz, 7.8Hz, 7.8Hz, 0.3H), 4.12 (m, 1H), 4.35 (m, 1H), 4.68 (m, 1H), 5.30 (s, 0.3H), 5.35 (d, J=4.5Hz, 0.7H), 6.71 (d, J=8.1Hz, 0.7H), 6.76 (d, J=6.9Hz, 0.3H), 6.87 (d, J=6.4Hz, 0.7H), 6.95 (d, J=8.1Hz, 0.3H), 7.39 (dd, J=8.4Hz, 1.8Hz, 2H), 7.73 (dd, J=8.4Hz, 2.1Hz, 2H).

### Example 44: Preparation of (3S)-3-{(S)-4-methyl-2-(2-methylbenzoylamino)valerylamino}-2-tetrahydrofuranol (Compound No. 125 in Table-1)

Melting point: 73-74°C
IR (KBr, cm⁻¹): 3298, 1638, 1541.
NMR (CD₃OD, δ): 0.98 (d, J=6.1Hz, 6H), 1.50-1.99 (m, 4H), 2.30 (m, 1H), 2.38 (s, 1.65H), 2.39 (s, 1.35H), 3.85 (m, 0.55H), 3.98-4.17 (m, 1.45H), 4.23 (m, 1H), 4.60 (m, 1H), 5.16 (s, 0.55H), 5.25 (d, J=4.7Hz, 0.45H), 7.18-7.29 (m, 2H), 7.30-7.42 (m, 2H).

### Example 45: Preparation of (3S)-3-{(S)-4-methyl-2-(3-methylbenzoylamino)valerylamino}-2-tetrahydrofuranol (Compound No. 126 in Table-1)

Melting point: 85-87°C
IR (KBr, cm⁻¹): 3299, 1638, 1586, 1541.
NMR (CDCl₃, δ): 0.83-1.07 (m, 6H), 1.60-1.97 (m, 3H), 2.07 (m, 1H), 2.30 (m, 1H), 2.35 (s, 1.8H), 2.36 (s, 1.2H), 3.82 (m, 0.6H), 3.96-4.18 (m, 1.6H), 4.18-4.42 (m, 1.4H), 4.75 (m, 1H), 4.93 (m, 0.4H), 5.31 (d, J=2.9Hz, 0.6H), 5.35 (dd, J=4.4Hz, 4.3Hz, 0.4H), 7.01 (m, 1H), 7.14 (m, 1H), 7.32-7.40 (m, 2H), 7.57-7.67 (m, 2H).

### Example 46: Preparation of (3S)-3-{(S)-4-methyl-2-(4-methylbenzoylamino)valerylamino}-2-tetrahydorofuranol (Compound No. 127 in Table-1)

Melting point: 101-102°C
IR (KBr, cm⁻¹): 3304, 1634, 1545, 1504.
NMR (CDCl₃, δ): 0.87-1.07 (m, 6H), 1.60-1.95 (m, 4H), 2.37 (m, 1H), 2.39 (s, 3H), 3.45 (d, J=2.9Hz, 0.6H), 3.90 (m, 0.4H), 3.97-4.19 (m, 2H), 4.38 (m, 1H), 4.71 (m, 1H), 5.30 (d, J=2.9Hz, 0.6H), 5.35 (dd, J=6.7Hz, 6.7Hz, 0.6H), 6.74 (d, J=8.5Hz, 0.4H), 6.80 (d, J=8.5Hz, 1H), 6.89 (d, J=6.7Hz, 0.6H), 7.22 (d, J=8.2Hz, 2H), 7.68 (d, J=8.2Hz, 2H).

### Example 47: Preparation of (3S)-3-{(S)-2-(2,6-dimethylbenzoylamino)-4-methylvalerylamino)-2-tetrahydrofuranol (Compound No. 129 in Table-1)

Melting point: 89-91°C
IR (KBr, cm⁻¹): 3389, 1638, 1539.
NMR (CDCl₃, δ): 0.83-1.10 (m, 6H), 1.58-1.99 (m, 4H), 2.25 (s, 3H), 2.27 (s, 3H), 2.32 (m, 1H), 3.65-3.93 (m, 1.55H) 4.07 (m, 1H), 4.22-4.45 (m, 1.45H), 4.70 (m, 1H), 5.25 (d, J=3.1Hz, 0.55H), 5.31 (dd, J=4.3Hz, 4.2Hz, 0.45H), 6.36 (d, J=8.2Hz, 1H), 6.85-7.07 (m, 3H), 7.14 (dd, J=7.8Hz, 7.3Hz, 1H).

### Example 48: Preparation of (3S)-3-{(S)-2-(3,4-dimethylbenzoylamino)-4-methylvalerylamino}-2-tetrahydrofuranol (Compound No. 130 in Table-1)

Melting point: 92-94°C
IR (KBr, cm⁻¹): 3299, 1636, 1541.
NMR (CDCl₃, δ): 0.83-1.10 (m, 6H), 1.57-1.99 (m, 4H), 2.28 (s, 3H), 2.29 (s, 3H), 2.34 (m, 1H), 3.57 (s, 0.5H), 3.86 (m, 0.5H), 3.97-4.20 (m, 1.5H), 4.21-4.45 (m, 1.5H), 4.70 (m, 1H), 5.31 (s, 0.5H), 5.35 (d, J=2.1Hz, 0.5H), 6.74 (d, J=8.3Hz, 0.5H), 6.80 (d, J=8.2Hz, 0.5H), 6.85 (d, J=8.5Hz, 0.5H), 6.93 (d, J=7.1Hz, 0.5H), 7.17 (d, J=7.8Hz, 1H), 7.43-7.60 (m, 2H).

### Example 49: Preparation of (3S)-3-{(S)-4-methyl-2-(2,4,6-trimethylbenzoylamino)valerylamino}-2-tetrahydrofuranol (Compound No. 131 in Table-1)

Melting point: 150-152°C
IR (KBr, cm⁻¹): 3295, 1638, 1522.
NMR (CDCl₃, δ): 0.87-1.07 (m, 6H), 1.58-1.97 (m, 4H), 2.18-2.35 (m, 9H), 2.36 (m, 1H), 3.09 (s, 0.45H), 3.48 (s, 0.55H), 3.85 (m, 0.55H), 3.97-4.20 (m, 1.45H), 4.35 (m, 1H), 4.65 (m, 1H), 5.29 (d, J=2.5Hz, 0.45H), 5.34 (dd, J=4.1Hz, 3.8Hz, 0.55H), 6.08 (d, J=5.2Hz, 1H), 6.75 (m, 1H), 6.83 (s, 2H).

### Example 50: Preparation of (3S)-3-{(S)-2-(4-ethylbenzoylamino)-4-methylvalerylamino}-2-tetrahydrofuranol (Compound No. 134 in Table-1)

Melting point: 98-99°C
IR (KBr, cm⁻¹): 3304, 1672, 1634, 1545, 1505.
NMR (CDCl₃, δ): 0.83-1.05 (m, 6H), 1.23 (t, J=7.6Hz, 3H), 1.60-1.99 (m, 4H), 2.38 (m, 1H), 2.68 (q, J=7.6Hz, 2H), 3.34 (s, 0.4H), 3.87 (m, 1H), 4.00-4.20 (m, 1.6H), 4.35 (m, 1H), 4.68 (m, 1H), 5.30 (s, 0.6H), 5.34 (d, J=3.7Hz, 0.4H), 6.65-6.90 (m, 2H), 7.21-7.27 (m, 2H), 7.69-7.74 (m, 2H).

### Example 51: Preparation of (3S)-3-{(S)-4-methyl-2-(4-trifluoromethylbenzoylamino)valerylamino}-2-tetrahydrofuranol (Compound No. 137 in Table-1)

Melting point: 135-136°C
IR (KBr, cm⁻¹): 3310, 1640, 1548, 1508.
NMR (CDCl₃, δ): 0.87-1.07 (m, 6H), 1.61-1.97 (m, 4H), 2.37 (m, 1H), 3.43 (s, 0.4H), 3.82-4.08 (m, 1.6H), 4.17 (m, 1H), 4.29 (m, 1H), 4.70 (m, 1H), 5.31 (d, J=2.6Hz, 0.4H), 5.36 (d, J=4.2Hz, 4.2Hz, 0.6H), 6.71 (d, J=7.9Hz, 0.6H), 6.73 (d, J=7.9Hz, 0.4H), 7.03 (d, J=8.3Hz, 0.6H), 7.14 (d, J=8.3Hz, 0.4H), 7.61-7.86 (m, 2H), 7.87-7.93 (m, 2H).

### Example 52: Preparation of (3S)-3-{(S)-2-(2-methoxybenzoylamino)-4-methylvalerylamino)-2-tetrahydrofuranol (Compound No. 138 in Table-1)

Melting point: 65-66°C
IR (KBr, cm⁻¹): 3376, 1640, 1601, 1532.
NMR (CDCl₃, δ): 0.83-1.05 (m, 6H), 1.60-1.95 (m, 4H), 2.31 (m, 1H), 3.85 (m, 0.6H), 3.98 (s, 3H), 4.00-4.20 (m, 1.6H), 4.24-4.45 (m, 1.4H), 4.69 (m, 1H), 5.00 (m, 0.4H), 5.34 (m, 1H), 6.93-7.17 (m, 2.4H), 7.22 (m, 0.6H), 7.50 (m, 1H), 8.15 (m, 1H), 8.30 (m, 1H).

### Example 53: Preparation of (3S)-3-{(S)-2-(4-methoxybenzoylamino)-4-methylvalerylamino}-2-tetrahydrofuranol (Compound No. 140 in Table-1)

Melting point: 85-88°C
IR (KBr, cm⁻¹): 3295, 1632.
NMR (CDCl₃, δ): 0.95 (d, J=6.0Hz, 3H), 0.96 (d, J=4.8Hz, 3H), 1.72 (m, 3H), 1.86 (m, 1H), 2.29 (m, 0.5H), 2.40 (m, 0.5H), 3.84 (s, 3H), 3.86 (ddd, J=8.1Hz, 8.1Hz, 8.1Hz, 0.5H), 4.01 (ddd, J=8.1Hz, 8.1Hz, 8.1Hz, 0.5H), 4.08 (m, 1H), 4.28 (m, 0.5H), 4.35 (m, 0.5H), 4.68 (m, 1H), 5.30 (s, 0.5H), 5.34 (d, J=4.8Hz, 0.5H), 6.71 (d, J=8.4Hz, 0.5H), 6.78 (d, J=8.4Hz, 0.5H), 6.82 (d, J=8.1Hz, 0.5H), 6.91 (dd, J=9.0Hz, 2.4Hz, 2H), 6.94 (d, J=8.7Hz, 0.5H), 7.76 (m, 2H).

### Example 54: Preparation of (3S)-3-{(S)-2-(2,4-dimethoxybenzoylamino)-4-methylvalerylamino}-2-tetrahydrofuranol (Compound No. 141 in Table-1)

Melting point: 65-67°C
IR (KBr, cm⁻¹): 3382, 1638, 1604, 1534.
NMR (CDCl₃, δ): 0.83-1.05 (m, 6H), 1.59-1.98 (m, 4H), 2.40 (m, 1H), 3.83 (m, 0.6H), 3.84 (s, 1.2H), 3.86 (s, 1.8H), 3.95 (s, 1.8H), 3.97 (s, 1.2H), 4.00-4.21 (m, 2H), 4.35 (m, 1H), 4.65 (m, 1H), 4.80 (m, 0.4H), 5.32 (d, J=3.2Hz, 0.6H), 5.35 (dd, J=4.6Hz, 4.6Hz, 0.5H), 6.48 (s, 0.4H), 6.49 (s, 0.6H), 6.59 (m, 1H), 6.99 (d, J=8.1Hz, 0.6H), 7.15 (d, J=6.7Hz, 0.4H), 8.07-8.22 (m, 2H).

### Example 55: Preparation of (3S)-3-{(S)-2-(2,6-dimethoxybenzoylamino)-4-methylvalerylamino}-2-tetrahydrofuranol (Compound No. 142 in Table-1)

Melting point: 166-168°C
IR (KBr, cm⁻¹): 3299, 3279, 1645, 1597, 1508.
NMR (DMSO-d₆, δ): 0.87 (d, J=6.2Hz, 6H), 1.38-1.60 (m, 2H), 1.60-1.83 (m, 2H), 2.15 (m, 1H), 3.65 (m, 1H), 3.71 (s, 2.4H), 3.72 (s, 3.6H), 3.93 (m, 1H), 4.10 (m, 1H), 4.39 (m, 1H), 5.14 (m, 1H), 6.55 (m, 1H), 6.58-6.70 (m, 2H), 7.18 (d, J=5.7Hz, 1H), 7.29 (m, 1H), 8.33 (d, J=8.4Hz, 1H).

### Example 56: Preparation of (3S)-3-{(S)-2-(3,5-dimethoxybenzoylamino)-4-methylvalerylamino}-2-tetrahydrofuranol (Compound No. 144 in Table-1)

Melting point: 88-90°C
IR (KBr, cm⁻¹): 3407, 1639, 1595, 1539.
NMR (CDCl₃, δ): 0.83-1.05 (m, 6H), 1.60-1.98 (m, 4H), 2.40 (m, 1H), 3.41 (s, 0.6H), 3.70-3.93 (m, 0.8H), 3.79 (s, 6H), 3.95-4.08 (m, 0.6H), 4.10 (m, 1H), 4.25 (m, 1H), 4.65 (m, 1H), 5.30 (d, J=2.2Hz, 0.6H), 5.34 (m, 0.4H), 6.58 (dd, J=1.9Hz, 1.9Hz, 1H), 6.67-6.87 (m, 2H), 6.87-6.97 (m, 2H).

### Example 57: Preparation of (3S)-3-{(S)-2-(4-ethoxybenzoylamino)-4-methylvalerylamino}-2-tetrahydrofuranol (Compound No. 148 in Table-1)

Melting point: 84-85°C
IR (KBr, cm⁻¹): 3299, 1634, 1609, 1547, 1504.
NMR (CDCl₃, δ): 0.83-1.07 (m, 6H), 1.43 (t, J=7.0Hz, 3H), 1.60-1.99 (m, 4H), 2.38 (m, 1H), 3.82 (m, 1H), 3.92-4.20 (m, 3.5H), 4.55 (s, 0.5H), 4.69 (m, 1H), 5.31 (d, J=2.8Hz, 0.5H), 5.35 (dd, J=4.3Hz, 4.1Hz, 0.5H), 6.80 (d, J=8.3Hz, 0.5H), 6.84-7.00 (m, 3H), 7.14 (d, J=7.0Hz, 0.5H), 7.76 (d, J=8.7Hz, 2H).

### Example 58: Preparation of (3S)-3-{(S)-4-methyl-2-(3,4-methylenedioxybenzoylamino)valerylamino}-2-tetrahydrofuranol (Compound No. 152 in Table-1)

Melting point: 94-96°C
IR (KBr, cm⁻¹): 3410, 3111, 1753, 1659.
NMR (CDCl₃, δ): 0.95 (d, J=6.0Hz, 3H), 0.96 (d, J=5.1Hz, 3H), 1.69-1.81 (m, 3H), 1.86 (m, 1H), 2.30 (m, 0.5H), 2.41 (m, 0.5H), 3.89 (ddd, J=8.1Hz, 8.1Hz, 8.1Hz, 0.5H), 4.05 (ddd, J=7.6Hz, 7.6Hz, 7.6Hz, 0.5H), 4.11 (m, 1H), 4.30 (m, 0.5H), 4.36 (m, 0.5H), 4.64 (m, 1H), 5.30 (s, 0.5H), 5.32 (d, J=4.6Hz, 0.5Hz), 6.02 (s, 2H), 6.61-6.80 (m, 2H), 6.82 (d, J=7.8Hz, 1H), 7.28 (dd, J=2.7Hz, 2.7Hz, 1H), 7.33 (ddd, J=8.0Hz, 2.0Hz, 2.0Hz, 1H).

### Example 59: Preparation of (3S)-3-{(S)-4-methyl-2-(1-naphthoylamino)valerylamino}-2-tetrahydrofuranol (Compound No. 156 in Table-1)

Melting point: 85-86°C
IR (KBr, cm⁻¹): 3293, 1638, 1535.
NMR (CDCl₃, δ): 0.83-1.05 (m, 6H), 1.59-1.99 (m, 4H), 2.38 (m, 1H), 3.47 (s, 0.45H), 3.88 (m, 1H), 3.94-4.20 (m, 1.55H), 4.38 (m, 1H), 4.80 (m, 1H), 5.29 (d, J=2.9Hz, 0.55H), 5.35 (dd, J=4.4Hz, 3.9Hz, 0.45H), 6.64 (d, J=8.2Hz, 0.55H), 6.73 (d, J=8.1Hz, 0.45H), 6.89 (m, 1H), 7.44 (m, 1H), 7.47-7.68 (m, 3H), 7.80-7.97 (m, 2H), 8.25 (m, 1H).

### Example 60: Preparation of (3S)-3-{(S)-4-methyl-2-(2-naphthoylamino)valerylamino}-2-tetrahydrofuranol (Compound No. 157 in Table-1)

Melting point: 98-100°C
IR (KBr, cm⁻¹): 3293, 1640, 1539, 1512.
NMR (CDCl₃, δ): 0.83-1.13 (m, 6H), 1.60-1.99 (m, 4H), 2.36 (m, 1H), 3.53 (s, 0.3H), 3.85 (m, 0.7H), 3.99-4.21 (m, 2H), 4.40 (m, 1H), 4.80 (m, 1H), 5.34 (dd, J=4.4Hz, 4.3Hz, 0.3H), 5.38 (d, J=2.7Hz, 0.7H), 6.88 (d, J=8.5Hz, 0.7H), 6.96 (d, J=7.2Hz, 0.3H), 7.04 (d, J=8.3Hz, 0.7H), 7.12 (d, J=8.2Hz, 0.3H), 7.43-7.64 (m, 2H), 7.79-7.97 (m,4H), 8.32 (s, 1H).

### Example 61: Preparation of (3S)-3-{(S)-2-(2-furoylamino)-4-methylvalerylamino}-2-tetrahydrofuranol (Compound No. 161 in Table-1)

Melting point: 92-95°C
IR (KBr, cm⁻¹): 3420, 3300, 1645, 1595, 1529.
NMR (CDCl₃, δ): 0.85-1.05 (m, 6H), 1.58-2.00 (m, 4H), 2.25-2.55 (m, 1H), 3.52 (d, J=2.7Hz, 0.55H), 3.87 (dt, J=7.8Hz, 7.8Hz, 0.55H), 3.95-4.17 (m, 1.9H), 4.27-4.45 (m, 1H), 4.45-4.73 (m, 1H), 5.31 (d, J=2.7Hz, 0.55H), 5.35 (dd, J=4.0Hz, 4.0Hz, 0.45H), 6.51 (dd, J=3.1Hz, 1.4Hz, 1H), 6.77 (d, J=7.7Hz, 1H), 6.80-6.93 (m, 1H), 7.14 (dd, J=3.1Hz, 1.9Hz, 1H), 7.46 (dd, J=1.9Hz, 1.4Hz, 1H).

### Example 62: Preparation of (3S)-3-{(S)-2-(3-ethyl-1-methyl-5-pyrazole)carbonylamino-4-methylvalerylamino}-2-tetrahydrofuranol (Compound No. 170 in Table-1)

Melting point: 89-91°C
IR (KBr, cm⁻¹): 3301, 1643.
NMR (CDCl₃, δ): 0.97 (d, J=5.0Hz, 6H), 1.23 (t, J=7.7Hz, 2.1H), 1.23 (t, J=7.6Hz, 0.9H), 1.60-1.90 (m, 4H), 2.34 (m, 0.7H), 2.56 (m, 0.3H), 2.62 (q, J=7.6Hz, 2H), 3.89 (ddd, J=7.8Hz, 7.8Hz, 7.8Hz, 1H), 4.01-4.17 (m, 2H), 4.08 (s, 0.9H), 4.08 (s, 2.1H), 4.35 (m, 1H), 4.58 (m, 1H), 5.29 (s, 0.3H), 5.35 (d, J=3.9Hz, 0.7H), 6.39 (s, 1H), 6.44 (d, J=6.8Hz, 0.3H), 6.55 (d, J=8.4Hz, 0.7H), 6.60 (d, J=8.4Hz, 1H).

### Example 63: Preparation of (3S)-3-{(S)-2-(2-chromancarbonylamino-4-methylvalerylamino}-2-tetrahydrofuranol (Compound No. 175 in Table-1)

Melting point: 84-85°C
IR (KBr, cm⁻¹): 3299, 1786, 1532.
NMR (CDCl₃, δ): 0.78-0.93 (m, 3H), 0.95-1.05 (m, 3H), 1.35-2.07 (m, 5H), 2.20-2.54 (m, 2H), 2.70-3.00 (m, 2H), 3.80-4.20 (m, 2H), 4.24-4.60 (m, 3H), 5.15 (m, 1H), 5.31 (m, 1H), 6.40 (m, 1H), 6.65 (m, 1H), 6.83-6.97 (m, 2H), 6.98-7.20 (m, 2H).

### Example 64: Preparation of (3S)-3-((S)-2-cinnamoylamino-4-methylvalerylamino)-2-tetrahydrofuranol (Compound No. 182 in Table-1)

Melting point: 102-104°C
IR (KBr, cm⁻¹): 3293, 1651, 1624, 1543.
NMR (CDCl₃, δ): 0.96 (m, 6H), 1.63-1.92 (m, 4H), 2.32 (m, 0.5H), 2.47 (m, 0.5H), 3.35 (d, J=2.7Hz, 0.5H), 3.43 (d, J=2.7Hz, 0.5H), 3.92 (m, 1H), 4.02-4.18 (m, 2H), 4.34 (m, 1H), 4.62 (m, 1H), 5.31 (s, 0.5H), 5.35 (m, 0.5H), 6.43 (d, J=15.0Hz, 1H), 6.44 (m, 1H), 6.90 (m, 1H), 7.32 (m, 2H), 7.51 (m, 2H), 7.62 (m, 1H).

### Example 65: Preparation of (3S)-3-{(S)-2-(4-methoxycinnamoylamino)-4-methylvalerylamino}-2-tetrahydrofuranol (Compound No. 187 in Table-1)

Melting point: 101-103°C
IR (KBr, cm⁻¹): 3283, 1651, 1602, 1543, 1512.
NMR (CDCl₃, δ): 0.85-1.05 (m, 6H), 1.60-1.99 (m, 4H), 2.30 (m, 1H), 3.77 (s, 1.5H), 3.79 (s, 1.5H), 3.82 (m, 0.5H), 4.10 (m, 1H), 4.30 (m, 0.5H), 4.41 (m, 1H), 4.72 (m, 1H), 5.35 (m, 1H), 6.38 (d, J=15.6Hz, 0.5H), 6.41 (d, J=15.6Hz, 0.5H), 6.65-6.85 (m, 2H), 6.90 (d, J=8.5Hz, 0.5H), 7.15 (d, J=8.5Hz, 0.5H), 7.20 (d, J=8.5Hz, 0.5H), 7.43 (dd, J=8.8Hz, 3.1Hz, 2H), 7.49 (d, J=8.5Hz, 0.5H), 7.58 (d, J=15.6Hz, 0.5H), 7.59 (d, J=15.6Hz, 0.5H).

### Example 66: Preparation of (3S)-3-{(S)-2-(4-fluorophenylsulfonylamino)-4-methylvalerylamino}-2-tetrahydrofuranol (Compound No. 202 in Table-1)

Melting point: 156-158°C
IR (KBr, cm⁻¹): 3347, 3256, 1649, 1593, 1541.
NMR (DMSO-d₆, δ): 0.71 (d, J=6.5Hz, 3H), 0.80 (d, J=6.6Hz, 3H), 1.28 (m, 2H), 1.45-1.68 (m, 2H), 1.86 (m, 1H), 3.60-3.95 (m, 4H), 4.98 (dd, J=4.4Hz, 4.1Hz, 1H), 6.37 (d, J=4.1Hz, 1H), 7.39 (dd, J=8.8Hz, 8.3Hz, 2H), 7.75 (d, J=7.5Hz, 1H), 7.81 (dd, J=8.8Hz, 5.3Hz, 2H), 8.00 (d, J=9.0Hz, 1H).

### Example 67: Preparation of (3S)-3-{(S)-2-(2-chlorophenylsulfonylamino)-4-methylvalerylamino}-2-tetrahydrofuranol (Compound No. 203 in Table-1)

IR (KBr, cm⁻¹): 3365, 1657, 1541.
NMR (CDCl₃, δ): 0.65 (d, J=6.2Hz, 1.2H), 0.67 (d, J=6.4Hz, 1.8H), 0.82 (d, J=6.1Hz, 1.2H), 0.84 (d, J=6.4Hz, 1.8H), 1.43-1.72 (m, 4H), 2.13 (m, 0.6H), 2.36 (m, 0.4H), 3.62-4.28 (m, 5H), 5.24 (d, J=3.0Hz, 0.4H), 5.31 (dd, J=3.8Hz, 3.8Hz, 0.6H), 5.91 (d, J=8.8Hz, 0.4H), 5.94 (d, J=7.9Hz, 0.6H), 6.47 (d, J=7.5Hz, 0.4H), 6.65 (d, J=8.1Hz, 0.6H), 7.38-7.45 (m, 1H), 7.45-7.60 (m, 2H), 8.06 (dd, J=7.3Hz, 1.1Hz, 1H).

### Example 68: Preparation of (3S)-3-{(S)-2-(4-chlorophenylsulfonylamino)-4-methylvalerylamino}-2-tetrahydrofuranol (Compound No. 205 in Table-1)

Melting point: 112-115°C
IR (KBr, cm⁻¹): 3335, 3264, 1649.
NMR (CDCl₃, δ): 0.76 (d, J=6.3Hz, 0.6H), 0.80 (d, J=6.3Hz, 2.4H), 0.87 (d, J=6.9Hz, 0.6H), 0.89 (d, J=6.6Hz, 2.4H), 1.48 (m, 3H), 1.68 (m, 1H), 2.11 (m, 0.8H), 2.40 (m, 0.2H), 3.67 (ddd, J=6.9Hz, 6.9Hz, 6.9Hz, 1H), 3.85 (m, 0.8H), 3.94 (m, 0.2H), 4.05-4.21 (m, 2H), 5.18 (s, 0.2H), 5.25 (d, J=4.5Hz, 0.8H), 5.31 (d, J=9.9Hz, 0.2H), 5.35 (d, J=8.4Hz, 0.8H), 5.94 (d, J=7.8Hz, 0.2H), 6.23 (d, J=7.8Hz, 0.8H), 7.48 (d, J=8.4Hz, 2H), 7.80 (d, J=8.4Hz, 2H).

### Example 69: Preparation of (3S)-3-{(S)-2-(4-bromophenylsulfonylamino)-4-methylvalerylamino}-2-tetrahydrofuranol (Compound No. 208 in Table-1)

Melting point: 139-140°C
IR (KBr, cm⁻¹): 3478, 3362, 3264, 1647, 1576, 1537.
NMR (DMSO-d₆, δ): 0.63-0.90 (m, 6H), 1.15-1.49 (m, 2H), 1.42-1.66 (m, 2H), 1.83 (m, 0.65H), 2.02 (m, 0.35H), 3.45-3.92 (m, 4H), 4.80 (d, J=4.1Hz, 0.35H), 4.96 (dd, J=4.4Hz, 4.4Hz, 0.65H), 6.10 (d, J=4.1Hz, 0.35H), 6.36 (d, J=4.4Hz, 0.65H), 7.60-7.85 (m, 4.65H), 7.95-8.15(m, 1.35H).

### Example 70: Preparation of (3S)-3-{(S)-4-methyl-2-(4-methylphenylsulfonylamino)valerylamino}-2-tetrahydrofuranol (Compound No. 211 in Table-1)

Melting point: 137-138°C
IR (KBr, cm⁻¹): 3343, 3264, 1649, 1541.
NMR (DMSO-d₆, δ): 0.66 (d, J=6.5Hz, 0.9H), 0.74 (d, J=6.7Hz, 3H), 0.80 (d, J=6.7Hz, 2.1H), 1.10-1.40 (m, 3H), 1.53 (m, 1H), 1.85 (m, 0.3H), 1.98 (m, 0.7H), 2.35 (s, 3H), 3.51 (m, 0.7H), 3.58-3.85 (m, 3.3H), 4.78 (d, J=4.4Hz, 0.7H), 4.96 (m, 0.3H), 6.07 (d, J=4.4Hz, 0.7H), 6.39 (d, J=3.9Hz, 0.3H), 7.31 (d, J=7.9Hz, 2H), 7.61 (d, J=7.9Hz, 2H), 7.66 (d, J=6.2Hz, 0.3H), 7.80 (m, 1H), 7.92 (d, J=6.4Hz, 0.7H).

### Example 71: Preparation of (3S)-3-{(S)-4-methyl-2-(2,4,6-trimethylphenylsulfonylamino)valerylamino}-2-tetrahydrofuranol (Compound No. 215 in Table-1)

Melting point: 75-77°C
IR (KBr, cm⁻¹): 3328, 1657, 1605, 1541.
NMR (CDCl₃, δ): 0.67 (d, J=6.3Hz, 1.35H), 0.68 (d, J=6.3Hz, 1.65H), 0.83 (d, J=6.4Hz, 1.35H), 0.84 (d, J=6.4Hz, 1.65H), 1.38-1.72 (m, 4H), 2.11 (m, 0.65H), 2.29 (s, 3H), 2.31 (m, 0.45H), 2.63 (s, 6H), 3.61 (m, 1H), 3.72 (d, J=3.0Hz, 0.45H), 3.74-3.98 (m, 1H), 3.98-4.24 (m, 2.55H), 5.23 (d, J=3.0Hz, 0.45H), 5.28 (d, J=3.9Hz, 3.9Hz, 0.55H), 5.46 (d, J=8.6Hz, 0.45H), 5.60 (d, J=8.0Hz, 0.55H), 6.38 (d, J=7.4Hz, 0.45H), 6.54 (d, J=8.0Hz, 0.55H), 6.95 (s, 2H).

### Example 72: Preparation of (3S)-3-{(S)-2-(4-tert-butylphenylsulfonylamino)-4-methylvalerylamino}-2-tetrahydrofuranol (Compound No. 218 in Table-1)

Melting point: 140- 141°C
IR (KBr, cm⁻¹): 3362, 3161, 1647, 1535.
NMR (DMSO-d₆, δ): 0.74 (d, J=6.5Hz, 3H), 0.81 (d, J=6.6Hz, 3H), 1.15-1.41 (m, 3H), 1.29 (s, 9H), 1.54 (m, 1H), 1.95 (m, 1H), 3.46 (m, 1H), 4.62-4.82 (m, 3H), 4.80 (d, J=4.3Hz, 1H), 6.06 (d, J=4.3Hz, 1H), 7.53 (d, J=8.5Hz, 2H), 7.66 (d, J=8.5Hz, 2H), 7.82 (d, J=9.4Hz, 1H), 7.95 (d, J=6.7Hz, 1H).

### Example 73: Preparation of (3S)-3-{(S)-2-(4-methoxyphenylsulfonylamino)-4-methylvalerylamino}-2-tetrahydrofuranol (Compound No. 221 in Table-1)

Melting point: 153-155°C
IR (KBr, cm⁻¹): 3362, 3150, 1647, 1597, 1535, 1501.
NMR (DMSO-d₆, δ): 0.69 (m, 0.6H), 0.76 (d, J=6.5Hz, 2.7H), 0.82 (d, J=6.7Hz, 2.7H), 1.15-1.45 (m, 3H), 1.56 (m, 1H), 2.01 (m, 1H), 3.54 (m, 1H), 3.60-3.90 (m, 3H), 3.81 (s, 3H), 4.89 (d, J=4.6Hz, 0.9H), 4.99 (m, 0.1H), 6.09 (d, J=4.6Hz, 0.9H), 6.41 (d, J=2.7Hz, 0.1H), 7.04 (d, J=8.8Hz, 2H), 7.60-7.80 (m, 3.1H), 7.94 (d, J=6.9Hz, 0.9H).

### Example 74: Preparation of (3S)-3-{(S)-4-methyl-2-(3-nitrophenylsulfonylamino)valerylamino}-2-tetrahydrofuranol (Compound No. 227 in Table-1)

Melting point: 164-165°C
IR (KBr, cm⁻¹): 3358, 3264, 1649, 1537.
NMR (DMSO-d₆, δ): 0.73-0.95 (m, 6H), 1.20-1.80 (m, 4.75H), 1.95 (m, 0.25H), 3.47-3.65 (m, 2H), 3.77 (m, 1H), 3.94 (m, 1H), 4.73 (d, J=2.7Hz, 0.25H), 4.92 (dd, J=4.1Hz, 4.1Hz, 0.75H), 6.06 (d, J=2.7Hz, 0.25H), 6.29 (d, J=4.1Hz, 0.75H), 7.80-7.95 (m, 2H), 8.05-8.21 (m, 1.25H), 8.35 (d, J=8.4Hz, 0.75H), 8.46 (d, J=7.9Hz, 1H), 8.50 (s, 1H).

### Example 75: Preparation of (3S)-3-{(S)-4-methyl-2-(1-naphtylsulfonylamino)valerylamino}-2-tetrahydrofuranol (Compound No. 229 in Table-1)

Melting point: 89-91°C
IR (KBr, cm⁻¹): 3580, 3520, 3470, 3281, 1647, 1553.
NMR (DMSO-d₆, δ): 0.34 (d, J=6.0Hz, 1.95H), 0.51 (d, J=6.2Hz, 1.05H), 0.62 (d, J=6.1Hz, 1.95H), 0.71 (d, J=6.4Hz, 1.05H), 1.10-1.60 (m, 4H), 1.75-1.95 (m, 1H), 3.47-3.83 (m, 4H), 4.73 (d, J=4.5Hz, 0.35H), 4.94 (dd, J=4.3Hz, 4.3Hz, 0.65H), 6.03 (d, J=4.5Hz, 0.35H), 6.34 (d, J=4.3Hz, 0.65H), 7.50-7.75 (m, 4H), 7.86 (d, J=8.2Hz, 0.35H), 7.98-8.32 (m, 3.65H), 8.66 (d, J=7.4Hz, 1H).

### Example 76: Preparation of (3S)-3-{(S)-4-methyl-2-(2-naphtylsulfonylamino)valerylamino}-2-tetrahydrofuranol (Compound No. 230 in Table-1)

Melting point: 102- 104°C
IR (KBr, cm⁻¹): 3351, 1655, 1541.
NMR (DMSO-d₆, δ): 0.65 (d, J=6.4Hz, 1.5H), 0.65-0.90 (m, 4.5H), 1.14-1.68 (m, 5H), 3.25 (m, 0.5H), 3.57-3.91 (m, 3.5H), 4.73 (s, 0.5H), 4.89 (dd, J=4.3Hz, 4.3Hz, 0.5H), 5.98 (s, 0.5H), 6.32 (d, J=4.3Hz, 0.5H), 7.60-7.80 (m, 3.5H), 7.93 (d, J=6.7Hz, 0.5H), 7.98-8.17 (m, 4H), 8.38 (d, J=8.3Hz, 1H).

### Example 77: Preparation of (3S)-3-{(S)-4-methyl-2-(3-pyridylsulfonylamino)valerylamino}-2-tetrahydrofuranol (Compound No. 232 in Table-1)

Melting point: 127-130°C
IR (KBr, cm⁻¹): 3268, 1647.
NMR (CDCl₃, δ): 0.79 (d, J=6.3Hz, 0.6H), 0.84 (d, J=6.6Hz, 2.4H), 0.86 (d, J=6.0Hz, 0.6H), 0.90 (d, J=6.6Hz, 2.4H), 1.49-1.61 (m, 3H), 1.71 (m, 1H), 2.09 (m, 0.8H), 2.37 (m, 0.2H), 3.78-3.87 (m, 1.8H), 3.96 (q, J=6.6Hz, 0.2H), 4.05-4.15 (m, 2H), 5.17 (s, 0.2H), 5.22 (d, J=4.8Hz, 0.8Hz), 5.75 (d, J=8.7Hz, 0.8H), 5.79 (d, J=9.3Hz, 0.2H), 6.13 (d, J=6.6Hz, 0.2H), 6.36 (d, J=8.1Hz, 0.8H), 7.45 (dd, J=7.8Hz, 4.8Hz, 1H), 8.17 (ddd, J=7.8Hz, 1.8Hz, 1.8Hz, 1H), 8.80 (dd, J=4.8Hz, 1.2Hz, 1H), 9.07 (d, J=2.1Hz, 1H).

### Example 78: Preparation of (3S)-3-((S)-2-benzyloxycarbonylamino-3-phenylpropionylamino)-2-tetrahydrofuranol (Compound No. 246 in Table-1)

Melting point: 144-146°C
IR (KBr, cm⁻¹): 3302, 1696, 1649, 1537.
NMR (CDCl₃, δ): 1.69 (m, 1H), 2.20-2.41 (m, 1H), 2.60 (s, 0.8H), 2.82 (s, 0.2H), 2.97 (dd, J=14.7Hz, 7.8Hz, 1H), 3.13 (m, 1H), 3.81 (ddd, J=7.8Hz, 7.8Hz, 7.8Hz, 0.8H), 4.02 (m, 1.2H), 4.26 (m, 1H), 4.37 (m, 1H), 5.09 (m, 3H), 5.40 (s, 1H), 5.70 (s, 0.2H), 6.08 (s, 0.8H), 7.33 (m, 10H).

### Example 79: Preparation of (3S)-3-((S)-2-benzyloxycarbonylamino-3-tert-butoxypropionylamino)-2-tetrahydrofuranol (Compound No. 297 in Table-1)

IR (KBr, cm⁻¹): 3322, 1719, 1661, 1534.
NMR (CDCl₃, δ): 1.17 (s, 9H), 1.69-1.98 (m, 1H), 2.30 (m, 0.6H), 2.44 (m, 0.4H), 3.40 (m, 1H), 3.62-3.98 (m, 3H), 4.11 (m, 1H), 4.22 (m, 1H), 4.35 (m, 1H), 5.11 (s, 2H), 5.22 (s, 0.4H), 5.29 (s, 0.6H), 5.76 (s, 1H), 6.80 (s, 0.4H), 7.08 (bs, 0.6H), 7.35 (m, 5H),

### Example 80: Preparation of (3S)-4-methyl-3-((S)-4-methyl-2-phenylsulfonylaminovalerylamino)-2-tetrahydrofuranol (Compound No. 320 in Table-1)

Melting point: 116-121°C
IR (KBr, cm⁻¹): 3356, 3272, 1655.
NMR (CDCl₃, δ): 0.70 (d, J=6.0Hz, 2.1H), 0.71 (d, J=6.0Hz, 0.9H), 0.86 (d, J=6.6Hz, 3H), 0.95 (d, J=6.6Hz, 2.1H), 1.04 (d, J=6.6Hz, 0.9H), 1.50 (m, 2H), 1.62 (m, 1H), 2.12 (m, 1H), 3.43 (m, 1H), 3.70 (m, 1H), 3.91 (m, 1H), 4.17 (dd, J=8.4Hz, 8.4Hz, 1H), 5.12 (d, J=4.5Hz, 0.3H), 5.25 (d, J=4.5Hz, 0.7H), 5.35 (d, J=7.2Hz, 0.7H), 5.40 (d, J=7.2Hz, 0.3H), 6.34 (d, J=8.7Hz, 0.3H), 6.37 (d, J=8.7Hz, 0.7H), 7.49-7.62 (m, 3H), 7.88 (m, 2H).

### Example 81: Preparation of (3S)-3-((S)-2-benzyloxycarbonylamino-4-methylvalerylamino)-2-tetrahydropyrazole (Compound No. 433 in Table-1)

IR (KBr, cm⁻¹): 3298, 1691, 1649, 1541.
NMR (CDCl₃, δ): 0.90-0.92 (m, 6H), 1.57-1.70 (m, 7H), 3.43-3.70 (m, 2H), 3.87-3.99 (m, 2H), 4.08-4.12 (m, 1H), 5.02 (s, 0.4H), 5.08 (s, 1.6H), 5.66 (s, 1H), 6.57 (s, 0.8H), 6.88 (s, 0.2H), 7.31 (s, 5H).

### Example 82: Preparation of (3S)-3-{(S)-2-(2-fluorobenzoylamino)-4-methylvalerylamino}-2-tetrahydropyrazole (Compound No. 450 in Table-1)

IR (KBr, cm⁻¹): 3408, 1600, 1495.
NMR (CDCl₃, δ): 0.92-0.98 (m, 6H), 1.64-1.82 (m, 7H), 3.48-3.59 (m, 2H), 3.84-4.13 (m, 2H), 4.68-4.70 (m, 1H), 5.01 (m, 0.3H), 5.05 (m, 0.7H), 6.55 (d, J=8.3Hz, 0.7H), 6.82 (d, J=8.2Hz, 0.3H), 7.07-7.28 (m, 2H), 7.41-7.52 (m, 1H), 7.99-8.05 (m, 1H).

### Example 83: Preparation of (3S)-3-((S)-4-methyl-2-phenylsufonylaminovalerylamino)-2-tetrahydropyranole (Compound No. 468 in Table-1)

Melting point: 156-157°C
IR (KBr, cm⁻¹): 3335, 3261, 1649, 1545.
NMR (CDCl₃, δ): 0.67 (d, J=8.5Hz, 3H), 0.85 (d, J=6.0Hz, 3H), 1.21-2.05 (m, 7H), 3.21 (d, J=4.1Hz, 0.8H), 3.41-3.78 (m, 2H), 3.80-4.07 (m, 2H), 4.19 (m, 0.2H), 4.95 (s, 1H), 5.23 (d, J=6.8Hz, 1H), 6.26 (m, 1H), 7.42-7.68 (m, 3H), 7.87 (d, J=8.5Hz, 2H).

### Example 84: Preparation of (3S)-3-{(S)-4-methyl-2-(2,4,6-trimethylphenylsulfonylamino)valerylamino}-2-tetrahydropyranol (Compound No. 473 in Table-1)

IR (KBr, cm⁻¹): 3331, 1655, 1541.
NMR (CDCl₃, δ): 0.66-0.72 (m, 3H), 0.83-0.85 (m, 3H), 1.44-1.97 (m, 7H), 2.28 (s, 3H), 2.63 (s, 6H), 3.42-3.75 (m, 2H), 3.84-3.98 (m, 2H), 4.40-4.47 (m, 1H), 4.93 (s, 1H), 5.47 (d, J=8.4Hz, 0.3H), 5.53 (d, J=7.8Hz, 0.7H), 6.34 (m, 1H), 6.94 (s, 2H).

### Example 85: Preparation of (3S)-3-{(S)-2-(N-acetyl-N-4-methylphenylsulfonyl)amino-4-methylvalerylamino}-2-tetrahydorofuranol (Compound No. 515 in Table-1)

Melting point: 49-51°C
IR (KBr, cm⁻¹): 3414, 1701, 1674, 1524.
NMR (CDCl₃, δ): 0.81-1.04 (m, 6H), 1.54-2.02 (m, 4H), 2.19-2.55 (m, 1H), 2.25 (s, 1.95H), 2.29 (s, 1.05H), 2.46 (s, 3H), 2.85 (d, J=2.9Hz, 0.35H), 3.23 (d, J=3.2Hz, 0.65H), 3.80-4.20 (m, 2H), 4.27-4.44 (m, 1H), 4.86 (t, J=7.7Hz, 0.35H), 4.97 (dd, J=7.7Hz, 6.3Hz, 0.65H), 5.24 (d, J=2.9Hz, 0.35H), 5.34 (dd, J=3.2Hz, 3.2Hz, 0.65H), 6.02 (d, J=7.4Hz, 0.35H), 6.41 (d, J=7.9Hz, 0.65H), 7.37 (d, J=8.4Hz, 2H), 7.98 (d, J=8.4Hz, 0.7H), 8.05 (d, J=8.4Hz, 1.3H).

### Example 86: Preparation of (3S)-3-{(S)-2-(N-acetyl-N-4-methoxyphenylsulfonyl)amino-4-methylvalerylamino}-2-tetrahydrofuranol (Compound No. 516 in Table-1)

Melting point: 48-51°C
IR (KBr, cm⁻¹): 3414, 1701, 1595, 1522, 1501.
NMR (CDCl₃, δ): 0.84-1.06 (m, 6H), 1.57-2.01 (m, 4H), 2.24 (s, 1.95H), 2.29 (s, 1.05H), 2.26-2.58 (m, 1H), 3.04 (d, J=2.6Hz, 0.35H), 3.42 (d, J=3.1Hz, 0.65H), 3.80-4.18 (m, 2H), 3.89 (s, 1.95H), 3.90 (s, 1.05H), 4.24-4.43 (m, 1H), 4.87 (dd, J=7.7Hz, 6.0Hz, 0.35H), 4.95 (t, J=6.9Hz, 0.65H), 5.24 (d, J=2.6Hz, 0.35H), 5.34 (dd, J=3.1Hz, 3.1Hz, 0.65H), 6.04 (d, J=7.1Hz, 0.35H), 6.42 (d, J=8.0Hz, 0.65H), 7.04 (d, J=9.0Hz, 2H), 8.04 (d, J=9.0Hz, 0.7H), 8.12 (d, J=9.0Hz, 1.3H).

### Example 87: Preparation of (3S)-3-{(S)-2-((S)-4-methyl-2-phenylsulfonylaminovalerylamino)-4-methylvalerylamino}-2-tetrahydrofuranol (Compound No. 1126 in Table-3)

Melting point: 186-188°C
IR (KBr, cm⁻¹): 3285, 1644, 1549.
NMR (CDCl₃, δ): 0.40 (d, J=6.3Hz, 1.2H), 0.52 (d, J=5.6Hz, 1.8H), 0.75-1.00 (m, 9H), 1.32-2.07 (m, 7H), 2.25 (m, 0.6H), 2.40 (m, 0.4H), 3.48 (d, J=2.8Hz, 0.4H), 3.59 (m, 1H), 3.73-3.92 (m, 1.2H), 4.02-4.20 (m, 1.4H), 4.21-4.56 (m, 2H), 5.30-5.38 (m, 1H), 5.59 (d, J=4.8Hz, 0.4H), 5.68 (d, J=5.9Hz, 0.6H), 6.82 (d, J=8.3Hz, 0.6H), 6.95 (d, J=9.7Hz, 0.4H), 6.99 (d, J=8.6Hz, 0.6H), 7.08 (d, J=7.0Hz, 0.4H), 7.47-7.72 (m, 3H), 7.91 (d, J=8.5Hz, 2H).

### Example 88: Preparation of (2S,3S)-2-acetoxy-3-((S)-4-methyl-2-phenylsulfonylaminovalerylamino)tetrahydrofuran (Compound No. 716 in Table-2)

244 mg of (S)-3-((S)-4-methyl-2-phenylsulfonylaminovalerylamino)-2-tetrahydrofuranone obtained in Reference Example 1 was dissolved in 35 ml of methylene chloride and the solution was cooled to -78°C. 1.91 ml of a solution of diisobutylalminium hydride in toluene (1.01 mol/L) was added to the reaction solution. After stirring for 3 hours at -78°C, the reaction mixture was added with a saturated aqueous solution of ammonium chloride and ethyl acetate. The mixture was warmed up to room temperature and then filtered through celite. The celite was thoroughly washed with ethyl acetate. The filtrate was washed with saturated brine, dried over magnesium sulfate, and then filtered. The filtrate was concentrated to obtain crude (3S)-3-((S)-4-methyl-2-phenylsulfonylaminovalerylamino)-2-tetrahydrofuranol (compound of Example 1). This compound was dissolved in 1 ml of pyridine and added with 1.5 ml of acetic anhydride under ice cooling, and then, the mixture was stirred for 9 hours under ice cooling and further added with 1.5 ml of methanol and concentrated. The resulting residue was dissolved in ethyl acetate, and the solution was washed successively with diluted hydrochloric acid, water, saturated aqueous solution of sodium hydrogencarbonate, and saturated brine, and then dried over magnesium sulfate and filtered. The filtrate was concentrated, and the resulting residue was added with 2.5 ml of ethyl acetate and 2.5 ml of hexane, and then the mixture was stirred. Precipitated crystals were collected by filtration to obtain the desired compound (120 mg).
Yield: 44%
Melting point: 177-178°C
IR (KBr, cm⁻¹): 3409, 3100, 1753, 1659.
NMR (CDCl₃, δ): 0.54 (d, J=6.3Hz, 3H), 0.80 (d, J=6.3Hz, 3H), 1.37 (m, 2H), 1.59 (m, 1H), 1.81 (m, 1H), 2.16 (s, 3H), 2.24 (m, 1H), 3.61 (m, 1H), 3.95 (ddd, J=9.3Hz, 9.0Hz, 7.5Hz, 1H), 4.14 (ddd, J=9.3Hz, 9.3Hz, 3.0Hz, 1H), 4.53 (m, 1H), 4.87 (d, J=6.6Hz, 1H), 6.16 (d, J=4.5Hz, 1H), 6.56 (d, J=8.7Hz, 1H), 7.54 (m, 2H), 7.62 (m, 1H), 7.86 (dd, J=7.2Hz, 1.5Hz, 2H).

Compounds of Example 89 to Example 117 were prepared in the same manners as that of Example 88. Physicochemical data of the compounds will be described below.

### Example 89: Preparation of (2S,3S)-2-acetoxy-3-((S)-2-tert-butoxycarbonylamino-4-methylvalerylamino)tetrahydrofuran (Compound No. 547 in Table-2)

Melting point: 143-145°C
IR (KBr, cm⁻¹): 3297, 1748, 1659.
NMR (CDCl₃, δ): 0.93 (d, J=6.0Hz, 3H), 0.94 (d, J=6.0Hz, 3H), 1.45 (s, 9H), 1.49 (m, 1H), 1.67 (m, 2H), 1.83 (m, 1H), 2.11 (s, 3H), 2.36 (m, 1H), 3.96 (ddd, J=9.3Hz, 9.3Hz, 9.3Hz, 1H), 4.06 (m, 1H), 4.14 (ddd, J=9.3Hz, 9.3Hz, 3.0Hz, 1H), 4.57 (m, 1H), 4.84 (s, 1H), 6.17 (s, J=4.8Hz, 1H), 6.45 (s, 1H).

### Example 90: Preparation of (2S,3S)-2-acetoxy-3-((S)-2-benzyloxycarbonylamino-4-methylvalerylamino)tetrahydrofuran (Compound No. 551 in Table-2)

Melting point: 162-164°C
IR (KBr, cm⁻¹): 3310, 1687, 1655, 1535.
NMR (CDCl₃, δ): 0.94 (d, J=6.2Hz, 3H), 0.95 (d, J=6.2Hz, 3H), 1.83 (m, 1H), 2.07 (s, 3H), 2.11 (m, 3H), 2.36 (m, 1H), 3.93 (ddd, J=7.8Hz, 7.8Hz, 7.8Hz, 1H), 4.09 (m, 2H), 4.79 (m, 1H), 5.12 (s, 2H), 5.32 (s, 1H), 6.04 (d, J=4.2Hz, 1H), 6.17 (d, J=4.4Hz, 1H), 7.35 (m, 5H).

### Example 91: Preparation of (2S,3S)-2-acetoxy-3-{(S)-2-(2-chlorobenzyloxycarbonylamino)-4-methylvalerylamino}tetrahydrofuran (Compound No. 558 in Table-2)

Melting point: 144-147°C
IR (KBr, cm⁻¹): 3314, 3076, 1699, 1655, 1535.
NMR (CDCl₃, δ): 0.92-0.95 (m, 6H), 1.51-1.84 (m, 4H), 2.08 (s, 3H), 2.34 (m, 1H), 3.95 (dd, J=8.8Hz, 7.4Hz, 1H), 4.09-4.16 (m, 2H), 4.57 (m, 1H), 5.19 (d, J=13.0Hz, 1H), 5.23 (s, 1H), 5.26 (d, J=13.0Hz, 1H), 6.16 (d, J=4.3Hz, 1H), 6.29 (s, 1H), 7.25-7.29 (m, 2H), 7.37-7.41 (m, 2H).

### Example 92: Preparation of (2S,3S)-2-acetoxy-3-{(S)-4-methyl-2-(4-methylbenzyloxycarbonylamino)valerylamino}tetrahydrofuran (Compound No. 566 in Table-2)

Melting point: 161-163°C
IR (KBr, cm⁻¹): 3314, 1691, 1651, 1539.
NMR (CDCl₃, δ): 0.91-0.93 (m, 6H) 1.46 (m, 4H), 2.07 (s, 3H), 2.32 (s, 3H), 2.33 (m, 1H), 3.94 (q, J=7.5Hz, 1H), 4.08-4.15 (m, 2H), 4.57 (m, 1H), 5.05 (d, J=12.0Hz, 1H), 5.06 (s, 1H), 5.09 (d, J=12.0Hz, 1H), 6.15 (d, J=4.2Hz, 1H), 6.32 (s, 1H), 7.15 (d, J=7.8Hz, 2H), 7.23 (d, J=7.8Hz, 2H).

### Example 93: Preparation of (2S,3S)-2-acetoxy-3-{(S)-2-(9-fluorenylmethoxycarbonylamino)-4-methylvalerylamino}tetrahydrofuran (Compound No. 571 in Table-2)

Melting point: 158-159°C
IR (KBr, cm⁻¹): 3308, 1746, 1692, 1657, 1537.
NMR (CDCl₃, δ): 0.94 (m, 6H), 1.52-1.74 (m, 3H), 1.64 (m, 1H), 2.06 (s, 3H), 2.36 (m, 1H), 3.96 (ddd, J=7.8Hz, 7.8Hz, 7.8Hz, 1H), 4.11 (m, 2H), 4.19 (t, J=7.5Hz, 1H), 4.43 (m, 2H), 4.56 (m, 1H), 5.15 (s, 1H), 6.06 (d, J=4.2Hz, 1H), 6.22 (s, 1H), 7.31 (dd, J=7.5Hz, 7.5Hz, 2H), 7.41 (dd, J=7.5Hz, 7.5Hz, 2H), 7.58 (d, J=7.5Hz, 2H), 7.77 (d, J=7.5Hz, 2H).

### Example 94: Preparation of (2S,3S)-2-acetoxy-3-((S)-2-cyclohexyloxycarbonylamino-4-methylvalerylamino)tetrahydrofuran (Compound No. 580 in Table-2)

Melting point: 135- 137°C
NMR (CDCl₃, δ): 0.92-0.95 (m, 6H), 1.26-1.88 (m, 14H), 2.10 (s, 3H), 2.35 (m, 1H), 3.97 (m, 1H), 4.10-4.16 (m, 2H), 4.51-4.64 (m, 2H), 5.03 (s, 1H), 6.16 (d, J=4.5Hz, 1H), 6.40 (s, 1H).

### Example 95: Preparation of (2S,3S)-2-acetoxy-3-{(S)-4-methyl-2-(1-naphtylacetylamino)valerylamino}tetrahydrofuran (Compound No. 605 in Table-2)

Melting point: 182-184°C
IR (KBr, cm⁻¹): 3308, 1745, 1644, 1551.
NMR (CDCl₃, δ): 0.72 (d, J=6.4Hz, 3H), 0.74 (d, J=6.4Hz, 3H), 1.10-1.35 (m, 2H), 1.47 (m, 1H), 1.67 (m, 1H), 2.13 (s, 3H), 2.20 (m, 1H), 3.93 (ddd, J=8.9Hz, 8.9Hz, 8.9Hz, 1H), 3.98-4.15 (m, 3H), 4.31 (m, 1H), 4.46 (m, 1H), 5.66 (d, J=8.1Hz, 1H), 6.12 (d, J=4.6Hz, 1H), 6.53 (d, J=8.6Hz, 1H), 7.05-7.57 (m, 4H), 7.80-7.95 (m, 3H).

### Example 96: Preparation of (2S,3S)-2-acetoxy-3-{(S)-2-(2-fluorobenzoylamino)-4-methylvalerylamino}tetrahydrofuran (Compound No. 633 in Table-2)

Melting point: 165-166°C.
IR (KBr, cm⁻¹): 3343, 3304, 1748, 1694, 1640, 1551.
NMR (CDCl₃, δ): 0.96 (d, J=5.7Hz, 3H), 0.98 (d, J=5.7Hz, 3H), 1.58-1.95 (m, 4H), 2.10 (s, 3H), 2.39 (m, 1H), 3.95 (ddd, J=9.0Hz, 9.0Hz, 7.4Hz, 1H), 4.14 (ddd, J=9.0Hz, 9.0Hz, 2.9Hz, 1H), 4.55-4.75 (m, 2H), 6.19 (d, J=4.8Hz, 1H), 6.61 (d, J=8.2Hz, 1H), 7.00 (d, J=7.4Hz, 0.5H), 7.09 (d, J=7.4Hz, 0.5H), 7.15 (dd, J=8.2Hz, 7.5Hz, 1H), 7.29 (ddd, J=6.8Hz, 6.8Hz, 0.9Hz, 1H), 7.55 (m, 1H), 8.06 (ddd, J=7.9Hz, 7.9Hz, 1.9Hz, 1H).

### Example 97: Preparation of (2S,3S)-2-acetoxy-3-{(S)-2-(4-chlorobenzoylamino)-4-methylvalerylamino}tetrahydrofuran (Compound No. 641 in Table-2)

Melting point: 204-205°C
IR (KBr, cm⁻¹): 3304, 1746, 1674, 1635.
NMR (CDCl₃, δ): 0.96 (d, J=6.1Hz, 3H), 0.97 (d, J=6.0Hz, 3H), 1.58-1.78 (m, 3H), 1.85 (m, 1H), 2.13 (s, 3H), 2.31 (m, 1H), 3.95 (m, 1H), 4.14 (ddd, J=8.6Hz, 8.6Hz, 2.9Hz, 1H), 4.50-4.70 (m, 2H), 6.20 (d, J=4.6Hz, 1H), 6.62 (d, J=8.3Hz, 1H), 6.81 (d, J=7.8Hz, 1H), 7.41 (d, J=6.7Hz, 2H), 7.73 (d, J=6.7Hz, 2H).

### Example 98: Preparation of (2S,3S)-2-acetoxy-3-{(S)-4-methyl-2-(2-methylbenzoylamino)valerylamino}tetrahydrofuran (Compound No. 645 in Table-2)

Melting point: 185-186°C
IR (KBr, cm⁻¹): 3264, 1753, 1674, 1626.
NMR (CDCl₃, δ): 0.99 (d, J=5.6Hz, 6H), 1.55-1.98 (m, 4H), 2.11 (s, 3H), 2.30 (m, 1H), 2.49 (s, 3H), 3.98 (m, 1H), 4.15 (ddd, J=6.3Hz, 6.3Hz, 2.9Hz, 1H), 4.47-4.70 (m, 2H), 6.19 (d, J=4.6Hz, 1H), 6.28 (d, J=8.2Hz, 1H), 6.81 (d, J=8.4Hz, 1H), 7.16-7.29 (m, 2H), 7.31-7.40 (m, 2H).

### Example 99: Preparation of (2S,3S)-2-acetoxy-3-{(S)-4-methyl-2-(4-methylbenzoylamino)valerylamino}tetrahydrofuran (Compound No. 647 in Table-2)

Melting point: 199-200°C
IR (KBr, cm⁻¹): 3318, 1746, 1663, 1630, 1534.
NMR (CDCl₃, δ): 0.96 (d, J=6.0Hz, 3H), 0.97 (d, J=6.0Hz, 3H), 1.59-1.95 (m, 4H), 2.12 (s, 3H), 2.29 (m, 1H), 2.40 (s, 3H), 3.95 (m, 1H), 4.13 (ddd, J=6.3Hz, 6.3Hz, 2.9Hz, 1H), 4.49-4.70 (m, 2H), 6.19 (d, J=4.6Hz, 1H), 6.58 (d, J=7.6Hz, 1H), 6.70 (d, J=7.6Hz, 1H), 7.24 (d, J=7.8Hz, 2H), 7.69 (d, J=7.8Hz, 2H).

### Example 100: Preparation of (2S,3S)-2-acetoxy-3-{(S)-4-methyl-2-(2,4,6-trimethylbenzoylamino)valerylamino}tetrahydrofuran (Compound No. 651 in Table-2)

NMR (CDCl₃, δ): 0.97 (d, J=5.0Hz, 6H), 1.59-1.79 (m, 3H), 1.89 (m, 1H), 2.11 (s, 3H), 2.27 (s, 6H), 2.32 (s, 3H), 2.35 (m, 1H), 3.95 (m, 1H), 4.14 (ddd, J=9.0Hz, 9.0Hz, 2.8Hz, 1H), 4.55-4.70 (m, 2H), 5.98 (d, J=8.1Hz, 1H), 6.09 (d, J=4.6Hz, 1H), 6.84 (s, 2H), 6.86 (d, J=7.5Hz, 1H).

### Example 101: Preparation of (2S,3S)-3-((S)-4-methyl-2-phenylsulfonylaminovalerylamino)-2-poropionyloxytetrahydrofuran (Compound No. 720 in Table-2)

Melting point: 154-156°C
IR (KBr, cm⁻¹): 3355, 3274, 1711, 1678.
NMR (CDCl₃, δ): 0.56 (d, J=6.3Hz, 3H), 0.80 (d, J=6.6Hz, 3H), 1.17 (t, J=7.5Hz, 3H), 1.39 (m, 2H), 1.57 (m, 1H), 1.79 (m, 1H), 2.23 (m, 1H), 2.40 (qd, J=7.5Hz, 16.8Hz, 1H), 2.49 (qd, J=7.5Hz, 16.8Hz, 1H), 3.62 (m, 1H), 3.94 (ddd, J=9.0Hz, 9.0Hz, 9.0Hz, 1H), 4.13 (ddd, J=9.3Hz, 9.0Hz, 3.0Hz, 1H), 4.92 (d, J=6.9Hz, 1H), 6.17 (d, J=4.8Hz, 1H), 6.49 (d, J=8.7Hz, 1H), 7.53 (m, 2H), 7.62 (m, 1H), 7.86 (d, J=6.0Hz, 2H).

### Example 102: Preparation of (2S,3S)-3-((S)-4-methyl-2-phenylsulfonylaminovalerylamino)-2-pivaloyloxytetrahydrofuran (Compound No. 725 in Table-2)

Melting point: 165-166°C
IR (KBr, cm⁻¹): 3293, 1640.
NMR (CDCl₃, δ): 0.64 (d, J=5.9Hz, 3H), 0.81 (d, J=6.1Hz, 3H), 1.26 (s, 9H), 1.48 (m, 3H), 1.72 (m, 1H), 2.23 (m, 1H), 3.64 (m, 1H), 3.94 (ddd, J=9.1Hz, 9.1Hz, 9.1Hz, 1H), 4.10 (ddd, J=9.1Hz, 9.1Hz, 3.1Hz, 1H), 4.45 (m, 1H), 5.07 (d, J=7.7Hz, 1H), 6.10 (d, J=4.5Hz, 1H), 6.21 (d, J=8.4Hz, 1H), 7.51 (m, 2H), 7.61 (m, 1H), 7.86 (d, J=7.1Hz, 2H).

### Example 103: Preparation of (2S,3S)-2-benzoyloxy-3-((S)-4-methyl-2-phenylsulfonylaminovalerylamino)tetrahydrofuran (Compound No. 728 in Table-2)

Melting point: 184-185°C
IR (KBr, cm⁻¹): 3353, 3260, 1698, 1678.
NMR (CDCl₃, δ): 0.55 (d, J=6.0Hz, 3H), 0.69 (d, J=6.0Hz, 3H), 1.30-1.48 (m, 3H), 1.90 (m, 1H), 2.30 (m, 1H), 3.64 (m, 1H), 4.00 (ddd, J=9.3Hz, 9.0Hz, 7.5Hz, 1H), 4.19 (ddd, J=9.3Hz, 9.3Hz, 3.0Hz, 1H), 4.51 (m, 1H), 5.13 (d, J=8.1Hz, 1H), 6.37 (d, J=6.6Hz, 1H), 6.39 (d, J=4.2Hz, 1H), 7.26-7.50 (m, 4H), 7.58 (m, 2H), 7.80 (dd, J=7.5Hz, 1.8Hz, 2H), 8.06 (dd, J=7.8Hz, 0.9Hz, 2H).

### Example 104: Preparation of (2S,3S)-2-acetoxy-3-{(S)-2-(4-chlorophenylsulfonylamino)-4-methylvalerylamino)tetrahydrofuran (Compound No. 754 in Table-2)

Melting point: 136-137°C
IR (KBr, cm⁻¹): 3335, 3258, 1744, 1651, 1535.
NMR (CDCl₃, δ): 0.61 (d, J=6.2Hz, 3H), 0.83 (d, J=6.3Hz, 3H), 1.35-1.62 (m, 3H), 1.80 (m, 1H), 2.15 (s, 3H), 2.21 (m, 1H), 3.61 (m, 1H), 3.97 (ddd, J=9.1Hz, 9.1Hz, 9.1Hz, 1H), 4.14 (ddd, J=9.1Hz, 9.1Hz, 2.9Hz, 1H), 4.50 (m, 1H), 5.09 (d, J=7.3Hz, 1H), 6.15 (d, J=4.6Hz, 1H), 6.42 (d, J=8.8Hz, 1H), 7.51 (d, J=8.6Hz, 2H), 7.80 (d, J=8.6Hz, 2H).

### Example 105: Preparation of (2S,3S)-3-{(S)-2-(4-chlorophenylsulfonylamino)-4-methylvalerylamino}-2-pivaloyloxytetrahydrofuran (Compound No. 755 in Table-2)

Melting point: 155-156°C
IR (KBr, cm⁻¹): 3366, 3229, 1726, 1684, 1664, 1543.
NMR (CDCl₃, δ): 0.70 (d, J=6.3Hz, 3H), 0.83 (d, J=6.4Hz, 3H), 1.25 (s, 9H), 1.40-1.82 (m, 4H), 2.23 (m, 1H), 3.62 (dd, J=12.5Hz, 6.4Hz, 1H), 3.96 (ddd, J=8.9Hz, 8.9Hz, 8.9Hz, 1H), 4.11 (ddd, J=8.9Hz, 8.9Hz, 2.9Hz, 1H), 4.45 (m, 1H), 5.21 (d, J=8.2Hz, 1H), 6.04 (d, J=8.6Hz, 1H), 6.10 (d, J=4.5Hz, 1H), 7.45-7.55 (m, 2H), 7.75-7.85 (m, 2H).

### Example 106: Preparation of (2S,3S)-2-acetoxy-3-{(S)-4-methyl-2-(4-methylphenylsulfonylamino)valerylamino}tetrahydrofuran (Compound No. 761 in Table-2)

Melting point: 159-160°C
IR (KBr, cm⁻¹): 3372, 1721, 1674, 1535.
NMR (CDCl₃, δ): 0.52 (d, J=6.2Hz, 3H), 0.80 (d, J=6.3Hz, 3H), 1.36 (m, 2H), 1.56 (m, 1H), 1.83 (m, 1H), 2.16 (s, 3H), 2.22 (m, 1H), 2.44 (s, 3H), 3.59 (m, 1H), 3.94 (m, 1H), 4.14 (m, 1H), 4.56 (m, 1H), 4.81 (d, J=6.6Hz, 1H), 6.15 (d, J=4.6Hz, 1H), 6.35 (d, J=7.8Hz, 1H), 7.33 (d, J=8.0Hz, 2H), 7.74 (d, J=8.0Hz, 2H).

### Example 107: Preparation of (2S,3S)-2-acetoxy-3-{(S)-4-methyl-2-(2,4,6-trimethylphenylsulfonylamino)valerylamino}tetrahydrofuran (Compound No. 765 in Table-2)

Melting point: 158-159°C
IR (KBr, cm⁻¹): 3416, 3191, 1755, 1661, 1605, 1535.
NMR (CDCl₃, δ): 0.56 (d, J=6.3Hz, 3H), 0.79 (d, J=6.3Hz, 3H), 1.39 (m, 2H), 1.58 (m, 1H), 1.81 (m, 1H), 2.16 (s, 3H), 2.24 (m, 1H), 2.31 (s, 3H), 2.62 (s, 6H), 3.56 (m, 1H), 3.94 (m, 1H), 4.14 (m, 1H), 4.52(m, 1H), 5.00 (d, J=7.1Hz, 1H), 6.15 (d, J=4.6Hz, 1H), 6.61 (d, J=8.7Hz, 1H), 6.97(s, 2H).

### Example 108: Preparation of (2S,3S)-2-acetyl-3-{(S)2-(4-tert-butylphenylsulfonylamino)-4-methylvalerylamino)tetrahydrofuran (Compound No. 768 in Table-2)

NMR (CDCl₃, δ): 0.44 (d, J=6.2Hz, 3H), 0.77 (d, J=6.2Hz, 3H), 1.23-1.43 (m, 2H), 1.34 (s, 9H), 1.55 (m, 1H), 1.85 (m, 1H), 2.17 (s, 3H), 2.20 (m, 1H), 3.59 (m, 1H), 3.95 (m, 1H), 4.13 (ddd, J=9.0Hz, 9.0Hz, 2.9Hz, 1H), 4.57 (m, 1H), 4.95 (d, J=6.4Hz, 1H), 6.17 (d, J=4.6Hz, 1H), 6.77(d, J=8.7Hz, 1H), 7.53 (d, J=8.6Hz, 2H), 7.78 (d, J=8.6Hz, 2H).

### Example 109: Preparation of (2S,3S)-2-acetyl-3-{(S)-2-(4-methoxyphenylsulfonylamino)-4-methylvalerylamino)tetrahydrofuran (Compound No. 771 in Table-2)

Melting point: 157-158°C
IR (KBr, cm⁻¹): 3329, 3273, 1746, 1659, 1597, 1544, 1501.
NMR (CDCl₃, δ): 0.54 (d, J=6.1Hz, 3H), 0.81 (d, J=6.3Hz, 3H), 1.38 (m, 2H), 1.56 (m, 1H), 1.84 (m, 1H), 2.16 (s, 3H), 2.22 (m, 1H), 3.56 (m, 1H), 3.88 (s, 3H), 3.95 (m, 1H), 4.15 (m, 1H), 4.56 (m, 1H), 4.81 (d, J=6.4Hz, 1H), 6.16 (d, J=4.5Hz, 1H), 6.68 (d, J=9.2Hz, 1H), 6.99 (d, J=8.6Hz, 2H), 7.79 (d, J=8.6Hz, 2H).

### Example 110: Preparation of (2S,3S)-2-acetoxy-3-{(S)-4-methyl-2-(2-naphtylsulfonylamino)valerylamino}tetrahydrofuran (Compound No. 780 in Table-2)

Melting point: 158-159°C
IR (KBr, cm⁻¹): 3337, 3275, 1723, 1676, 1543.
NMR (CDCl₃, δ): 0.48 (d, J=6.1Hz, 3H), 0.76 (d, J=6.2Hz, 3H), 1.33-1.75 (m, 4H), 1.97 (m, 1H), 2.15 (s, 3H), 3.68 (m, 1H), 3.87 (m, 1H), 4.05 (m, 1H), 4.42 (m, 1H), 5.19 (d, J=7.1Hz, 1H), 6.12 (d, J=4.6Hz, 1H), 6.53 (d, J=8.7Hz, 1H), 7.58-7.71 (m, 2H), 7.82 (dd, J=8.7Hz, 1.9Hz, 1H), 7.87-8.03 (m, 3H), 8.44 (s, 1H).

### Example 111: Preparation of (2S,3S)-2-acetoxy-3-{(S)-4-methyl-2-(3-pyridylsulfonylamino)valerylamino}tetrahydrofuran (Compound No. 782 in Table-2)

Melting point: 160-161°C
IR (KBr, cm⁻¹): 3410, 3075, 1753, 1657, 1535, 1342, 1172.
NMR (CDCl₃, δ): 0.68 (d, J=5.9Hz, 3H), 0.85 (d, J=5.9Hz, 3H), 1.40-1.62 (m, 3H), 1.80 (m, 1H), 2.14 (s, 3H), 2.22 (m, 1H), 3.73 (m, 1H), 3.96 (ddd, J=9.1Hz, 8.9Hz, 8.9Hz, 1H), 4.03 (ddd, J=9.1Hz, 9.1Hz, 3.0Hz, 1H), 4.45 (m, 1H), 5.46 (m, 1H), 6.13 (d, J=4.6Hz, 1H), 6.32 (d, J=8.6Hz, 1H), 7.48 (m, 1H), 8.16 (ddd, J=8.2Hz, 1.9Hz, 1.9Hz, 1H), 8.83 (dd, J=4.9Hz, 1.5Hz, 1H), 9.07 (d, J=2.3Hz, 1H).

### Example 112: Preparation of (2S,3S)-2-acetoxy-3-{(S)-2-[N-acetyl-N-(4-methylphenylsulfonyl)amino]-4-methylvalerylamino}tetrahydrofuran (Compound No. 1065 in Table-2)

Melting point: 154°C
IR (KBr, cm⁻¹): 3387, 1746, 1674, 1522.
NMR (CDCl₃, δ): 0.84 (d, J=6.3Hz, 3H), 0.93 (d, J=6.3Hz, 3H), 1.58 (m, 2H), 1.84 (m, 1H), 2.09 (m, 1H), 2.12 (s, 3H), 2.33 (s, 3H), 2.38 (m, 1H), 2.47 (s, 3H), 3.96 (m, 1H), 4.14 (m, 1H), 4.55 (m, 1H), 4.78 (t, J=6.8Hz, 1H), 6.14 (d, J=4.6Hz, 1H), 6.50 (d, J=8.3Hz, 1H), 7.39 (d, J=8.1Hz, 2H), 7.91 (d, J=8.1Hz, 2H).

### Example 113: Preparation of (2S,3S)-2-acetoxy-3-{(S)-2-[N-acetyl-N-(4-methoxyphenylsulfonyl)amino]-4-methylvalerylamino}tetrahydrofuran (Compound No. 1066 in Table-2)

Melting point: 64-66°C
IR (KBr, cm⁻¹): 3397, 1748, 1595, 1530.
NMR (CDCl₃, δ): 0.85 (d, J=6.2Hz, 3H), 0.94 (d, J=6.3Hz, 3H), 1.59 (m, 2H), 1.85 (m, 1H), 2.08 (m, 1H), 2.12 (s, 3H), 2.32 (s, 1H), 2.37 (m, 1H), 3.90 (s, 3H), 3.96 (m, 1H), 4.15 (m, 1H), 4.56 (m, 1H), 4.79 (t, J=6.8Hz, 1H), 6.15 (d, J=4.7Hz, 1H), 6.51 (d, J=8.4Hz, 1H), 7.04 (d, J=9.0Hz, 2H), 7.97 (d, J=9.0Hz, 2H).

### Example 114: Preparation of (2S,3S)-2-acetoxy-3-((S)-2-benzyloxycarbonylamino-4-methylvalerylamino)-2-tetrahydrofuran (Compound No. 983 in Table-2)

IR (KBr, cm⁻¹): 3310, 1693, 1653, 1537.
NMR (CDCl₃, δ): 0.90-0.93 (m, 6H), 1.43-1.77 (m, 7H), 2.11 (s, 3H), 3.65-3.74 (m, 2H), 4.08-4.17 (m, 2H), 5.09 (s, 2H), 5.30 (d, J=8.1Hz, 1H), 5.96 (d, J=2.7Hz, 1H), 6.17 (s, 1H), 7.34 (m, 5H).

### Example 115: Preparation of (2S,3S)-2-acetoxy-3-{(S)-2-(2-fluorobenzoylamino)-4-methylvalerylamino}-2-tetrahydropyran (Compound No. 1000 in Table-2)

IR (KBr, cm⁻¹): 3298, 2947, 1633, 1545.
NMR (CDCl₃, δ): 0.91-0.98 (m, 6H), 1.65-1.77 (m, 7H), 2.13 (s, 3H), 3.64-3.77 (m, 2H), 3.90 (m, 1H), 4.20 (m, 1H), 4.60 (s, 1H), 6.00 (d, J=3.3Hz, 1H), 6.42 (s, 1H), 7.13 (m, 1H), 7.26 (m, 1H), 7.48 (m, 1H), 8.02 (m, 1H).

### Example 116: Preparation of (2S,3S)-2-acetoxy-3-{(S)-4-methyl-2-(2,4,6-trimethylphenylsulfonylamino)valerylamino}-2-tetrahydoropyran (Compound No. 1023 in Table-2)

IR (KBr, cm⁻¹): 3418, 1658, 1606, 1523. NMR (CDCl₃, δ): 0.57 (d, J=6.3Hz, 3H), 0.79 (d, J=6.3Hz, 3H), 1.32-1.78 (m, 7H), 2.16 (s, 3H), 2.29 (s, 3H), 2.60 (s, 6H), 3.49 (m, 1H), 3.61-3.78 (m, 2H), 4.08 (m, 1H), 5.08 (d, J=7.2Hz, 1H), 5.94 (d, J=3.0Hz, 1H), 6.33 (d, J=8.7Hz, 1H), 6.95 (s, 2H).

### Example 117: Preparation of (2S,3S)-3-{(S)-2-(4-tert-butylphenylsulfonylamino)-4-methylvalerylamino}-2-pivaloyloxytetrahydrofuran (Compound No. 1397 in Table-2)

Melting point: 166-167°C
IR (KBr, cm⁻¹): 3360, 1728, 1682, 1665, 1547.
NMR (CDCl₃, δ): 0.55 (d, J=6.0Hz, 3H), 0.78 (d, J=6.0Hz, 3H), 1.25 (s, 9H), 1.32 (s, 9H), 1.38-1.82 (m, 4H), 2.25 (m, 1H), 3.60 (m, 1H), 3.95 (m, 1H), 4.12 (ddd, J=9.0Hz, 9.0Hz, 3.0Hz, 1H), 4.46 (m, 1H), 5.05 (d, J=7.1Hz, 1H), 6.10 (d, J=4.5Hz, 1H), 6.41 (d, J=8.3Hz, 1H), 7.52 (d, J=8.6Hz, 2H), 7.78 (d, J=8.6Hz, 2H).

### Example 118: Preparation of (3S)-2-methoxy-3-((S)-4-methyl-2-phenylsulfonylaminovalerylamino)tetrahydrofuran (Compound No. 741 in Table-2)

43 mg of (2S,3S)-2-acetoxy-3-((S)-4-methyl-2-phenylsulfonylaminovalerylamino)tetrahydrofuran obtained in Example 88 was dissolved in 40 ml of methanol, and the solution was added with 1 ml of ethyl acetate containing 4N hydrochloric. After stirring overnight at room temperature, 7 ml of saturated aqueous solution of sodium hydrogencarbonate was added to the reaction mixture, and then the solvent was evaporated. The resulting residue was added with a saturated aqueous solution of sodium hydrogencarbonate and the mixture was extracted with ethyl acetate. The extract was washed with saturated brine and dried over magnesium sulfate. The drying agent was removed by filtration and the filtrate was concentrated to obtain a crude product. The crude product was added with hexane and diethyl ether and then stirred. After filtration, the desired compound (29 mg) was obtained.
Yield: 65%
Melting point: 84-90°C
IR (KBr, cm⁻¹): 3268, 1647, 1618.
NMR (CDCl₃, δ): 0.71 (d, J=6.6Hz, 2.1H), 0.80 (d, J=6.6Hz, 0.9H), 0.84 (d, J=6.6Hz, 2.1H), 0.88 (d, J=6.6Hz, 0.9H), 1.42-1.49 (m, 3H), 1.61 (m, 1H), 2.08 (m, 0.3H), 2.27 (m, 0.7H), 3.30 (s, 2.1H), 3.37 (s, 0.9H), 3.63 (m, 1H), 3.69 (m, 0.3H), 3.81-4.11 (m, 1.7H), 4.13 (m, 1H), 4.66 (s, 0.7H), 4.71 (d, J=4.8Hz, 0.3H), 4.66 (d J=7.8Hz, 0.7H), 5.23 (d, J=8.4Hz, 0.3H), 5.96 (d, J=7.8Hz, 0.7H), 6.17 (d, J=8.7Hz, 0.3H), 7.47-7.62 (m, 3H), 7.86 (d, J=7.5Hz, 2H).

### Test Example 1: Measurement of inhibitory activity against cysteine proteases

Inhibitory activity against cathepsin B (Sigma, C-6286) was determined according to a method described in a literature (Biochemical Journal, Vol. 201, p. 189, 1982). The results are shown in Table-5.

m-Calpain was purified from brains of rats according to a method described in a literature (Journal of Biological Chemistry, Vol. 259, p. 3210, 1984), and the inhibitory activity was determined in a manner according to a published method (Journal of Biological Chemistry, Vol. 259, p. 12489, 1984). The results are shown in Table-6.

From the results shown in Table-5 and Table-6, it is apparent that the compounds of the present invention have potent inhibitory activity against cystein proteases such as papain, cathepsin B, cahepsin L, and calpain.

**Table-5**

| Inhibitory activity against cathepsin B | | | |
|---|---|---|---|
| Example No. (Compound No. in Table-1) | IC₅₀ (µ M) | Example No. (Compound No. in Table-1) | IC₅₀ (µ M) |
| 1(196) | 0.42 | 40(113) | 1.00 |
| 4(20) | 0.70 | 41(114) | 0.87 |
| 5(21) | 0.98 | 42(115) | 0.55 |
| 6(22) | 1.35 | 43(121) | 0.14 |
| 7(23) | 0.90 | 44(125) | 1.45 |
| 10(27) | 0.37 | 45(126) | 0.49 |
| 11(28) | 2.15 | 46(127) | 0.27 |
| 12(29) | 1.20 | 48(130) | 0.086 |
| 13(31) | 0.38 | 50(134) | 0.20 |
| 15(37) | 0.70 | 51(137) | 0.21 |
| 16(38) | 1.00 | 53(140) | 0.15 |
| 17(40) | 0.64 | 55(142) | 1.65 |
| 18(44) | 0.58 | 56(144) | 0.90 |
| 19(46) | 0.89 | 57(148) | 0.24 |
| 20(47) | 1.05 | 58(152) | 0.46 |
| 21(49) | 1.17 | 59(156) | 0.80 |
| 24(53) | 2.90 | 60(157) | 0.034 |
| 25(54) | 2.90 | 61(161) | 1.20 |
| 27(60) | 1.20 | 62(170) | 0.50 |
| 28(61) | 1.70 | 65(187) | 0.19 |
| 33(85) | 0.95 | 75(229) | 2.40 |
| 34(90) | 1.35 | 77(232) | 0.40 |
| 39(112) | 0.20 | 78(246) | 0.30 |

| Example No. (Compound No. in Table-3) | IC₅₀ (µ M) | | |
|---|---|---|---|
| 87(1126) | 1.30 | | |

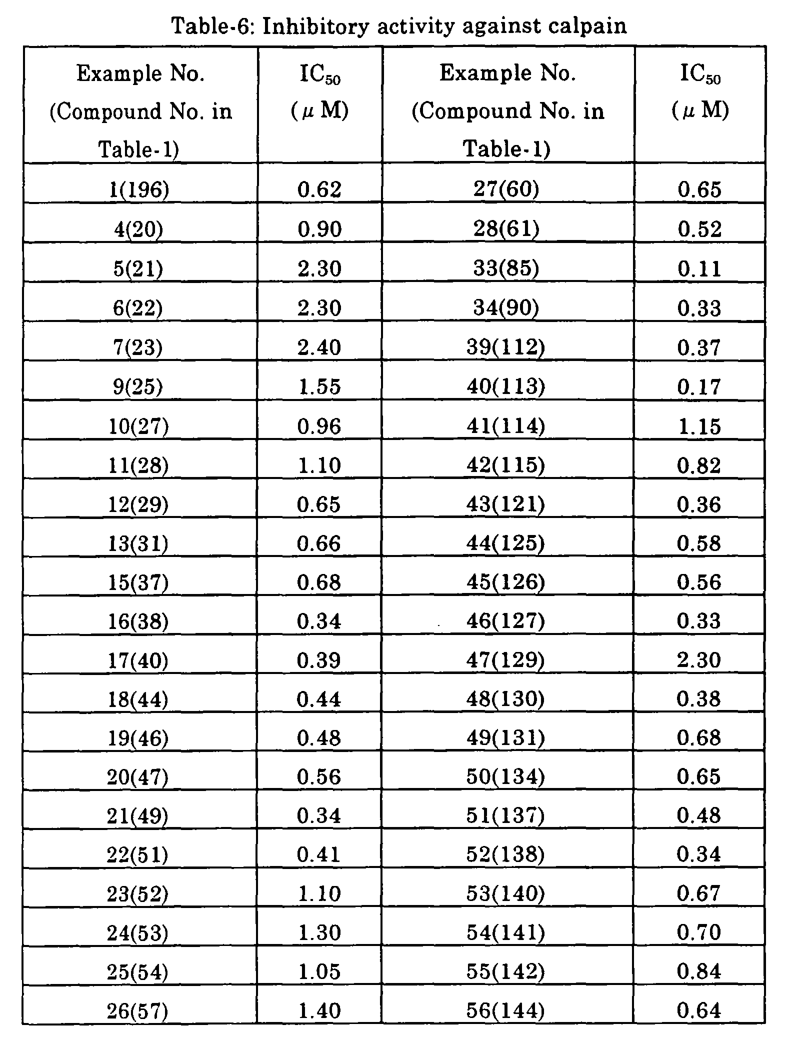

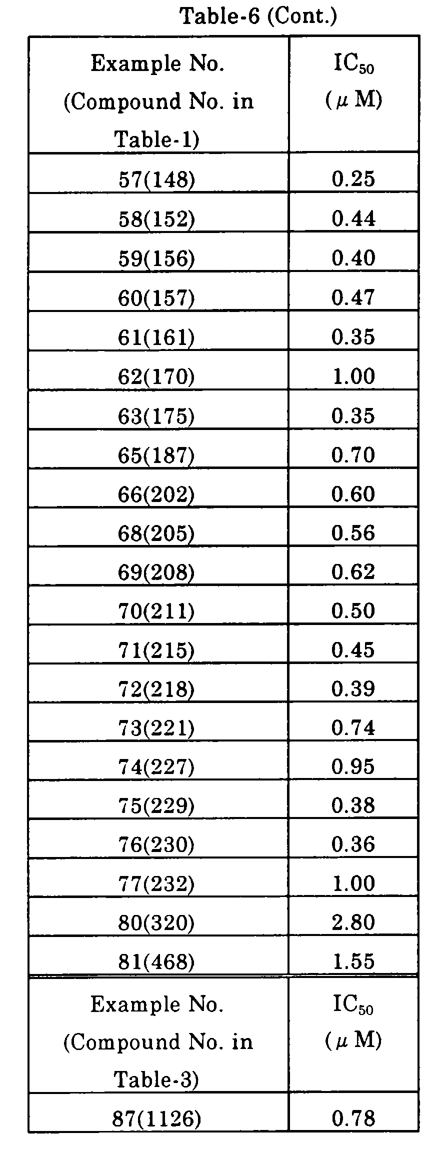

### Test Example 2: Release of an active compound in blood

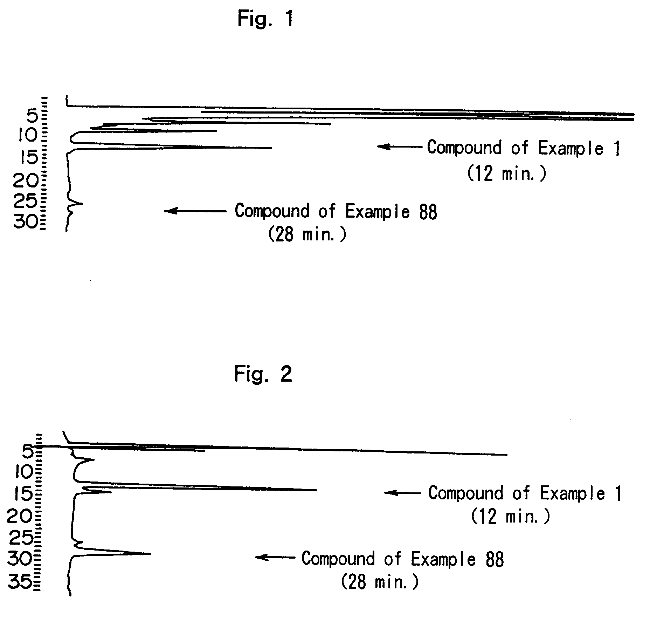

(2S,3S)-2-acetoxy-3-((S)-4-methyl-2-phenylsulfonylaminovalerylamino)tetrahydrofuran obtained in Example 88 was dissolved in acetonitrile and the solution was added to rat serum so that its final concentration was adjusted to 100 µ M. The mixture was incubated at 37°C for 5 minutes. The mixture was then added with acetonitrile and centrifuged, and the resulting solution portion was applied to HPLC for analysis. The result is shown in Figure 1. For comparison, solutions obtained by dissolving (3S)-3-((S)-4-methyl-2-phenylsulfonylaminovalerylamino)-2-tetrahydrofuranol obtained in Example 1 and (2S,3S)-2-acetoxy-3-((S)-4-methyl-2-phenylsulfonylaminovalerylamino)tetrahydrofuran obtained in Example 88 in acetonitrile respectively were examined by HPLC. The results are shown in Figure 2. As seen from the results shown in Figures 1 and 2, it was found that 97% of (2S,3S)-2-acetoxy-3-((S)-4-methyl-2-phenylsulfonylaminovalerylamino)tetrahydrofuran added in the serum was hydrolyzed and converted into the active form, i.e., (3S)-3-((S)-4-methyl-2-phenylsulfonylaminovalerylamino)-2-tetrahydrofuranol (compound of Example 1). The same procedures were repeated by using human serum and dog serum, and the formations of active form were also observed.

The HPLC conditions were as follows:
- Column:: Nucleosil 100₅ C_{18,} 4.6×250 mm (Nagel)
- Column temperature:: 50°C
- Mobile phase:: CH₃CN:H₂O:PIC A Low UV (Waters) = 22:78:1
- Flow rate:: 1 ml/min
- Detection wavelength:: UV 222 nm

From these results, it can be readily understood that the oxygen-containing heterocyclic derivative of the present invention is rapidly converted into the active form, i.e., the lactol derivative in a living body.

### Test Example 3: Acute toxicity test

Compounds of the present invention was suspended in 0.5% aqueous solution of CMC-Na and the suspensions were orally administered to SD male and female rats. Their symptoms were observed for seven days.

All of the compounds of Examples 88, 96, and 106 were revealed to have LD₅₀ values of less than 2,000 mg/kg.

### Test Example 4: Pharmaceutical formulations

### (1) Tablet

The following components were mixed in a conventional manner and compressed as tablets by using an ordinary apparatus.

| | |
|---|---|
| Compound of Example 88 | 30 mg |
| Crystalline cellulose | 60 mg |
| Corn starch | 100 mg |
| Lactose | 200 mg |
| Magnesium stearate | 4 mg |

### (2) Soft capsule

The following components were mixed in a conventional manner and filled into soft capsules.

| | |
|---|---|
| Compound of Example 88 | 30 mg |
| Olive oil | 300 mg |
| Lecithin | 20 mg |

### (3) Pharmaceutical preparation for injection

The following components were mixed in a conventional manner and 1 ml ampoules were prepared.

| | |
|---|---|
| Compound of Example 25 | 3 mg |
| Sodium chloride | 4 mg |
| Distilled water for injection | 1 ml |

### Industrial Applicability

The oxygen-containing heterocyclic derivatives of the present invention have potent inhibitory activity against cysteine proteases such as papain, cathepsin B, cathepsin H, cathepsin L, calpain and interleukin 1 β converting enzyme, and they are excellent in oral absorbability, tissue distribution, and cellular membrane permeability. Therefore, the compounds can be used as therapeutic medicaments for diseases such as muscular dystrophy, amyotrophia, myocardial infarct, cerebral apoplexy, Alzheimer's disease, disturbance of consciousness and movement disorder caused by a head injury, multiple sclerosis, neuropathy of peripheral nerve, cataract, inflammation, allergy, fulminant hepatis, osteoporosis, hypercalcemia, breast cancer, prostatic cancer, prostatic hypertrophy and the like, as well as medicaments for suppression of cancerous growth, prevention of cancerous metastasis, or inhibition of platelet agglomeration.

## Claims

1. An oxygen-containing heterocyclic derivative represented by the following formula (I) and its pharmaceutically acceptable salts, and a hydrate and a solvate thereof: 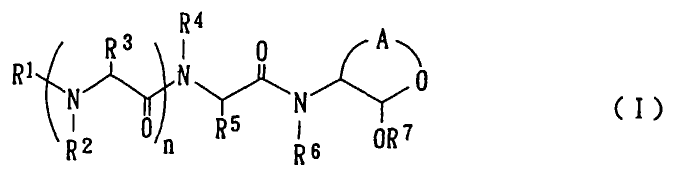 wherein R¹ represents a hydrogen atom, R⁸-CO-, R⁸-O-CO-, R⁸-NH-CO-, or R⁸-SO₂- (R⁸ represents a C₁-C₂₀ alkyl group which may optionally be substituted with one or more substituents selected from the group consisting of a C₃-C₈ cycloalkyl group, a C₃-C₈ cycloalkyloxy group, fluorenyl group, a C₁-C₅ alkoxy group, a C₆-C₁₄ aryl group which may optionally be substituted, a C₆-C₁₄ aryloxy group which may optionally be substituted, a C₆-C₁₄ arylthio group which may optionally be substituted, a C₆-C₁₄ arylsulfonyl group which may optionally be substituted, and a residue of a heterocyclic compound which may optionally be substituted; a C₃-C₈ cycloalkyl group; a C₆-C₁₄ aryl group which may optionally be substituted; a C₂-C₅ alkenyl group which may optionally be substituted with an optionally substituted C₆-C₁₄ aryl group; or a residue of a heterocyclic compound which may optionally be substituted); R², R⁴, and R⁶ independently represent a hydrogen atom, a C₁-C₅ alkyl group, or a C₂-C₆ alkanoyl group; R³ and R⁵ independently represent a hydrogen atom, a C₁-C₂₀ alkyl group which may optionally be substituted with one or more substituents selected from the group consisting of a C₆-C₁₄ aryl group which may optionally be substituted, hydroxyl group, a C₁-C₅ alkoxy group, a C₁-C₅ alkylthio group, and a C₇-C₁₂ aralkyloxy group, or they independently represents a C₆-C₁₄ aryl group which may optionally be substituted; R⁷ represents a hydrogen atom, a C₁-C₅ alkyl group, or R⁹-CO- (R⁹ represents a C₁-C₁₀ alkyl group or a C₆-C₁₂ aryl group which may optionally be substituted); symbol "A" represents a C₁-C₃ alkylene group which may optionally be substituted with a C₁-C₃ alkyl group; and symbol "n" represents 0 or 1.

2. The compound according to claim 1, wherein R¹ represents a hydrogen atom, R⁸-CO-, R⁸-O-CO-, R⁸-NH-CO-, or R⁸-SO₂- (R⁸ represents a C₁-C₂₀ alkyl group which may optionally be substituted with one or more substituents selected from the group consisting of a C₃-C₈ cycloalkyl group, fluorenyl group, a C₆-C₁₄ aryl group which may optionally be substituted, a C₆-C₁₄ aryloxy group which may optionally be substituted, a C₆-C₁₄ arylthio group which may optionally be substituted, a C₆-C₁₄ arylsulfonyl group which may optionally be substituted, and a residue of a heterocyclic compound which may optionally be substituted; a C₃-C₈ cycloalkyl group; a C₆-C₁₄ aryl group which may optionally be substituted; a C₂-C₅ alkenyl group which may optionally be substituted with an optionally substituted C₆-C₁₄ aryl group; or a residue of a heterocyclic compound which may optionally be substituted); R³ and R⁵ independently represent a hydrogen atom, a C₁-C₂₀ alkyl group which may optionally be substituted with one or more substituents selected from the group consisting of a C₆-C₁₄ aryl group which may optionally be substituted and a C₁-C₅ alkoxy group.

3. The compound according to claim 2, wherein R², R⁴, and R⁶ independently represent a hydrogen atom or a C₁-C₅ alkyl group.

4. The compound according to claim 3, wherein symbol "n" represents 0.

5. The compound according to claim 1, wherein R¹ represents R⁸-CO- (R⁸ represents a C₁-C₂₀ alkyl group which may optionally be substituted with one or more substituents selected from the group consisting of a C₆-C₁₄ aryl group which may optionally be substituted, a C₆-C₁₄ aryloxy group which may optionally be substituted, a C₆-C₁₄ arylthio group which may optionally be substituted, and a C₆-C₁₄ arylsulfonyl group which may optionally be substituted; a C₆-C₁₄ aryl group which may optionally be substituted; a C₂-C₅ alkenyl group which may optionally be substituted with an optionally substituted C₆-C₁₄ aryl group; or a residue of a heterocyclic compound which may optionally be substituted); R², R⁴, and R⁶ represent hydrogen atoms; R³ and R⁵ independently represent a C₁-C₂₀ alkyl group; R⁷ represents a hydrogen atom or R⁹-CO-(R³ represents a C₁-C₁₀ alkyl group); symbol "A" represents a C₁-C₃ alkylene group; and symbol "n" represents 0.

6. The compound according to claim 5, wherein R⁷ represents a hydrogen atom.

7. The compound according to claim 5, wherein R⁷ represents R⁹-CO- in which R⁹ represents a C₁-C₁₀ alkyl group.

8. The compound according to claim 1, wherein R¹ represents R⁸-CO- (R⁸ represents a C₁-C₂₀ alkyl group which may optionally be substituted with a C₆-C₁₄ aryl group which may optionally be substituted, or a C₆-C₁₄ aryl group which may optionally be substituted); R², R⁴, and R⁶ represent hydrogen atoms; R³ and R⁵ independently represent a C₁-C₂₀ alkyl group; R⁷ represents R⁹-CO- (R⁹ represents a C₁-C₁₀ alkyl group); symbol "A" represents a C₁-C₃ alkylene group; and symbol "n" represents 0.

9. The compound according to claim 1, wherein R¹ represents R⁸-O-CO- (R⁸ represents a C₁-C₂₀ alkyl group which may optionally be substituted with one or more substituents selected from the group consisting of a C₃-C₈ cycloalkyl group, fluorenyl group, a C₆-C₁₄ aryl group which may optionally be substituted, and a residue of a heterocyclic compound which may optionally be substituted; a C₃-C₈ cycloalkyl group; or a C₆-C₁₄ aryl group which may optionally be substituted); R², R⁴, and R⁶ represent hydrogen atoms; R³ and R⁵ independently represent a hydrogen atom, or a C₁-C₂₀ alkyl group which may optionally be substituted with one or more substituents selected from the group consisting of a C₆-C₁₄ aryl group which may optionally be substituted and a C₁-C₅ alkoxy group; R⁷ represents a hydrogen atom or R⁹-CO- (R⁹ represents a C₁-C₁₀ alkyl group); symbol A" represents a C₁-C₃ alkylene group; and symbol "n" represents 0.

10. The compound according to claim 9, wherein R⁷ represents a hydrogen atom.

11. The compound according to claim 9, wherein R⁷ represents R⁹-CO- in which R⁹ represents a C₁-C₁₀ alkyl group.

12. The compound according to claim 1, wherein R¹ represents R⁸-O-CO- (R⁸ represents a C₁-C₂₀ alkyl group which may optionally be substituted with one or more substituents selected from the group consisting of fluorenyl group and a C₆-C₁₄ aryl group which may optionally be substituted; or a C₃-C₈ cycloalkyl group); R², R⁴, and R⁸ represent hydrogen atoms; R³ and R⁵ independently represent a C₁-C₂₀ alkyl group; R⁷ represents R⁹-CO- (R⁹ represents a C₁-C₁₀ alkyl group); symbol "A" represents a C₁-C₃ alkylene group; and symbol "n" represents 0.

13. The compound according to claim 1, wherein R¹ represents R⁸-NH-CO-(R⁸ represents a C₆-C₁₄ aryl group which may optionally be substituted); R², R⁴, and R⁶ represent hydrogen atoms; R³ and R⁵ independently represent a C₁-C₂₀ alkyl group; R⁷ represents a hydrogen atom or R⁹-CO- (R⁹ represents a C₁-C₁₀ alkyl group or a C₆-C₁₂ aryl group which may optionally be substituted); symbol "A" represents a C₁-C₃ alkylene group; and symbol "n" represents 0.

14. The compound according to claim 13, wherein R⁷ represents a hydrogen atom.

15. The compound according to claim 1, wherein R¹ represents R⁸-SO₂- (R⁸ represents a C₆-C₁₄ aryl group which may optionally be substituted or a residue of a heterocyclic compound which may optionally be substituted); R², R⁴, and R⁶ represent hydrogen atoms; R³ and R⁵ independently represent a C₁-C₂₀ alkyl group; R⁷ represents a hydrogen atom, a C₁-C₅ alkyl group, or R⁹-CO- (R⁹ represents a C₁-C₁₀ alkyl group or a C₆-C₁₂ aryl group which may optionally be substituted); symbol "A" represents a C₁-C₃ alkylene group which may optionally be substituted with a C₁-C₃ alkyl group; and symbol "n" represents 0.

16. The compound according to claim 15, wherein R⁷ represents a hydrogen atom.

17. The compound according to claim 15, wherein R⁷ represents a C₁-C₅ alkyl group.

18. The compound according to claim 15, wherein R¹ represents R⁸-SO₂- (R⁸ represents a C₆-C₁₄ aryl group which may optionally be substituted) and R⁷ represents a C₁-C₅ alkyl group.

19. The compound according to claim 15, wherein R⁷ represents R⁹-CO-wherein R⁹ represents a C₁-C₁₀ alkyl group.

20. The compound according to claim 15, wherein R⁷ represents R⁹-CO-wherein R⁹ represents a C₆-C₁₂ aryl group which may optionally be substituted.

21. The compound according to claim 15, wherein R¹ represents R⁸-SO₂-wherein R⁸ represents a C₆-C₁₄ aryl group which may optionally be substituted and R⁷ represents R⁹-CO- wherein R⁹ represents a C₆-C₁₂ aryl group which may optionally be substituted.

22. The compound according to claim 1, wherein R¹ represents R⁸-CO-, R⁸-O-CO- or R⁸-SO₂- (R⁸ represents (1) a C₆-C₁₄ aryl group which may optionally be substituted with one or more substituents selected from the group consisting of a C₁-C₃ alkyl group, a halogen atom, and a C₁-C₃ alkoxy group; a C₃-C₈ cycloalkyl group; or a C₁-C₆ alkyl group which may optionally be substituted with a heterocyclic residue optionally substituted with one or more substituents selected from the group consisting of C₁-C₃ alkyl groups, (2) a C₃-C₈ cycloalkyl group, (3) a C₆-C₁₄ aryl group which may optionally be substituted with one or more substituents selected from the group consisting of a C₁-C₃ alkyl group, a halogen atom, nitro group, fluorenyl group and a C₁-C₃ alkoxy group, (4) a C₂-C₅ alkenyl group which may optionally be substituted with a C₆-C₁₄ aryl group optionally substituted with one or more substituents selected from the group consisting of a C₁-C₃ alkyl group and a halogen atom, or (5) a residue of a heterocyclic compound which may optionally be substituted with one or more substituents selected from the group consisting of a C₁-C₃ alkyl group and a halogen atom); R², R⁴ and R⁶ represent hydrogen atoms; R³ represents a C₁-C₆ alkyl group; R⁵ represents a C₁-C₆ alkyl group which may optionally be substituted with a C₆-C₁₄ aryl group; and R⁷ represents a hydrogen atom or R⁹-CO- wherein R⁹ represents a C₁-C₆ alkyl group.

23. The compound according to claim 1, wherein R¹ represents R⁸-CO-, R⁸-O-CO-, or R⁸-SO₂- (R⁸ represents (1) a C₆-C₁₄ aryl group which may optionally be substituted with one or more substituents selected from the group consisting of a C₁-C₃ alkyl group, a halogen atom, and a C₁-C₃ alkoxy group; a C₃-C₈ cycloalkyl group; pyridyl group; imidazolyl group which may optionally be substituted with one or more substituents selected from the group consisting of C₁-C₃ alkyl groups; furyl group; tetrahydrofuryl group; or a C₁-C₆ alkyl group which may optionally be substituted with a tetrahydropyranyl group, (2) a C₃-C₈ cycloalkyl group, (3) a C₆-C₁₄ aryl group which may optionally be substituted with one or more substituents selected from the group consisting of a C₁-C₃ alkyl group, a halogen atom, nitro group, fluorenyl group, and a C₁-C₃ alkoxy group, (4) a C₂-C₅ alkenyl group which may optionally be substituted with a C₆-C₁₄ aryl group optionally substituted with a C₁-C₃ alkyl group, (5) pyridyl group, (6) imidazolyl group which may optionally be substituted with one or more substituents selected from the group consisting of C₁-C₃ alkyl groups, (7) chromanyl group, (8) furyl group, (9) tetrahydrofuryl group, or (10) tetrahydropyranyl group); R², R⁴, and R⁶ represent hydrogen atoms, R³ represents a C₁-C₆ alkyl group, R⁵ represents a C₁-C₆ alkyl group which may optionally be substituted with a C₆-C₁₄ aryl group; and R⁷ represents a hydrogen atom or R⁹-CO- wherein R⁹ represents a C₁-C₆ alkyl group.

24. The compound according to claim 23, wherein symbol "A" represents an ethylene group which may optionally be substituted with a C₁-C₃ alkyl group and symbol "n" represents 0.

25. The compound according to claim 1, wherein R¹ represents R⁸-CO-, R⁸-O-CO-, or R⁸-SO₂- (R⁸ represents a C₁-C₃ alkyl group which may optionally be substituted with a C₆-C₁₄ aryl group optionally substituted with a C₁-C₃ alkyl group; or a C₆-C₁₄ aryl group which may optionally be substituted with one or more substituents selected from the group consisting of C₁-C₃ alkyl groups); R², R⁴, and R⁶ represent hydrogen atoms, R³ and R⁵ represent a C₁-C₆ alkyl group; and R⁷ represents a hydrogen atom or R⁹-CO- wherein R⁹ represents a C₁-C₆ alkyl group.

26. The compound according to claim 25, wherein symbol "n" represents 0.

27. The compound according to claim 1, wherein R¹ represents R⁸-CO-, R⁸-O-CO-, or R⁸-SO₂- (R⁸ represents a C₁-C₃ alkyl group substituted with phenyl group optionally substituted with a C₁-C₃ alkyl group; or phenyl group which may optionally be substituted with one or more substituents selected from C₁-C₃ alkyl groups); R⁴ and R⁶ represent hydrogen atoms; R⁵ represents a C₁-C₆ alkyl group; R⁷ represents hydrogen atom or R⁹-CO- wherein R⁹ represents a C₁-C₆ alkyl group; symbol "A" represents an ethylene group which may optionally be substituted with methyl group and symbol "n" represents 0.

28. The compound according to claim 1, wherein R¹ represents 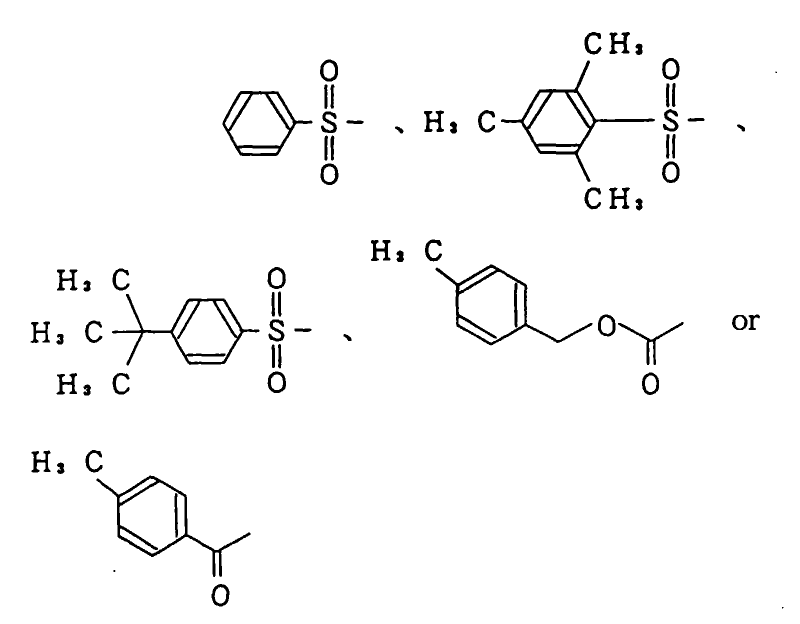 R⁴ and R⁶ represent hydrogen atoms; R⁵ represents a C₁-C₆ alkyl group; R⁷ represents a hydrogen atom or R⁹-CO- wherein R⁹ represents a C₁-C₆ alkyl group; symbol "A" represents an ethylene group which may optionally be substituted with methyl group and symbol "n" represents 0.

29. A pharmaceutical composition comprising a compound according to claim 1 and a pharmaceutically acceptable additive.

30. A pharmaceutical composition comprising a compound according to claim 1 and a pharmaceutically acceptable additive for treatment of a disease caused by abnormal accentuation of a cysteine protease.
